# EUROPEAN PATENT APPLICATION

(11) **EP 4 692 065 A1**
(43) Date of publication of application: **11.02.2026**
(21) Application number: 24780934.6
(22) Date of filing: 01.04.2024
(51) Int. Cl.: C07D 231/14, A01N 43/56, A01N 47/02, A01N 47/12, A01N 47/28, A01N 55/10, A01P 3/00, A01P 5/00, A01P 7/04, A61K 31/415, A61K 31/4155, A61K 31/422, A61K 31/4245, A61K 31/427, A61K 31/4439, A61K 31/4709, A61K 31/695, A61P 17/00, A61P 31/04, A61P 31/10

(54) **PYRAZOLE COMPOUND AND HARMFUL ORGANISM CONTROL AGENT**

(30) Priority: 31.03.2023 JP 2023059146; 28.04.2023 JP 2023075183; 26.06.2023 JP 2023104517; 25.08.2023 JP 2023137545; 06.12.2023 JP 2023206333; 06.12.2023 JP 2023206334
(71) Applicant: Nissan Chemical Corporation, Tokyo 103-6119 (JP)
(72) Inventor: KAWAI, Kentaro, Funabashi-shi, Chiba 274-8507 (JP); SAITO, Katsuya, Funabashi-shi, Chiba 274-8507 (JP); NAKAMURA, Ayu, Funabashi-shi, Chiba 274-8507 (JP); NOTO, Kenkichi, Funabashi-shi, Chiba 274-8507 (JP); KAJI, Motohiro, Funabashi-shi, Chiba 274-8507 (JP); OTANI, Masato, Shiraoka-shi, Saitama 349-0294 (JP)
(74) Representative: Müller-Boré & Partner Patentanwälte PartG mbB
(86) International application number: PCT/JP2024/013460
(87) International publication number: WO 2024/204842

(57) **Abstract**

Provided is a novel fungicide, in particular, agricultural-horticultural fungicide. Provided is a pyrazole compound represented by formula (1) or a salt thereof.

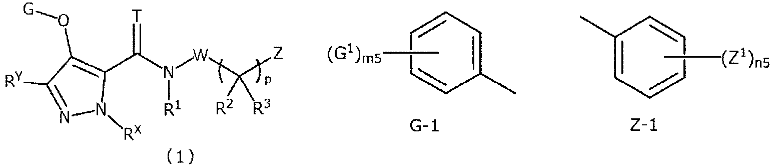

The formula indicates a structure in which G represents G-1, G¹ represents C₁-C₆ haloalkyl, etc., T represents an oxygen atom, R^{X} represents C₁-C₆ alkyl, etc., R^{Y} represents a hydrogen atom, etc., R¹ represents a hydrogen atom, etc., R² represents a hydrogen atom, etc., R³ represents a hydrogen atom, etc., W represents -O- or -N(R^{W})-, R^{W} represents a hydrogen atom, etc., Z represents Z-1, etc., Z¹ represents C₁-C₆ alkyl, etc., m5 and n5 each represent an integer of 0, 1, 2, 3, 4 or 5, and p represents an integer of 0 or 1.

## Description

### Technical Field

The present invention relates to a novel pyrazole compound, a salt thereof, and a pest control agent that contains the compound and a salt thereof as an active ingredient.

### Background Art

Patent Literatures 1 through 3 disclose some type of pyrazole compounds but do not disclose the pyrazole compound in accordance with the present invention at all.

Patent Literatures 4 and 5 indicate that some type of pyrazole compounds are useful as a fungicide but do not disclose the pyrazole compound in accordance with the present invention at all.

### Citation List

### [Patent Literature]

[Patent Literature 1]
   International Publication No. WO2002/085860
[Patent Literature 2]
   International Publication No. WO2006/132197
[Patent Literature 3]
   Specification of European Patent Application Publication No. 289879
[Patent Literature 4]
   International Publication No. WO2009/028280
[Patent Literature 5]
   International Publication No. WO1996/038419

### Summary of Invention

### Technical Problem

However, long-standing use of a chemical agent can cause acquisition of chemical resistance in pathogens. Therefore, development of a novel chemical agent having an excellent control effect is constantly expected.

### Solution to Problem

As a result of diligent studies conducted while aiming at solving the above problem, the inventors of the present invention have found that a novel pyrazole compound represented by a formula (1) below in accordance with the present invention exhibits excellent control activity as a fungicide, in particular, an agricultural-horticultural fungicide, and thus accomplished the present invention.

The pyrazole compound in accordance with the present invention is not disclosed in any literature, and usefulness of the pyrazole compound as a pest control agent has not been known.

That is, the present invention relates to <1> below.

<1> A pyrazole compound represented by a formula (1) below or a salt thereof, 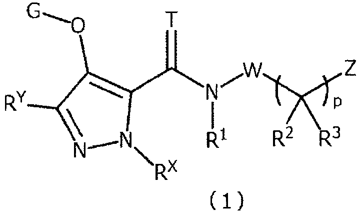
   where G represents G-1,
   G-1 represents a structure indicated by a structural formula below, 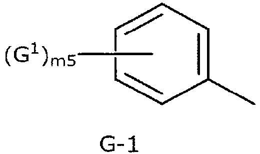
   G¹ represents hydroxy, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   T represents an oxygen atom,
   W represents -O- or -N(R^{W})-,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 represent structures indicated by structural formulae below, 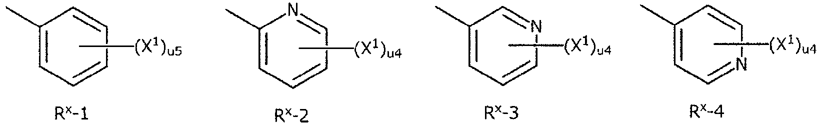
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom, C₁-C₆ alkyl or C₁-C₆ alkyl substituted by Rⁱ,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl, C₁-C₆ alkylaminothiocarbonyl, di(C₁-C₆ alkyl)aminothiocarbonyl, C₁-C₆ alkylsulfonyl, - C(O)OR^{d}, -C(O)C(O)R^{e}, -C(O)SR^{f}, -C(O)R^{g}, Q-1 to Q-53 or Q-54,
   R² represents a hydrogen atom, C₁-C₆ alkyl, C₁-C₆ haloalkyl or C₁-C₆ alkyl substituted by R^{h},
   R³ represents a hydrogen atom, C₁-C₆ alkyl or C₁-C₆ haloalkyl,
   R^{a} represents hydroxy, cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, -C(N=OR^{b})R^{c}, Q-1 to Q-53 or Q-54,
   R^{d} represents C₁-C₆ alkyl,
   R^{c} represents C₁-C₆ alkyl,
   R^{d} represents a structure indicated by a structural formula below, 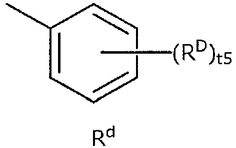
   R^{D} represents a halogen atom, nitro or C₁-C₆ alkyl,
   in a case where t5 represents an integer of 2, 3, 4 or 5 in relationship to R^{D}, the respective R^{D} may be identical with each other or may be different from each other,
   R^{e} represents C₁-C₆ alkyl or C₁-C₆ alkoxy,
   R^{f} represents C₁-C₆ alkyl,
   R^{g} represents Q-1 to Q-53 or Q-54,
   R^{h} represents cyano or C₁-C₆ alkoxy,
   Rⁱ represents cyano or C₁-C₆ alkoxy,
   Z represents Z-1 to Z-49 or Z-50,
   Z-1 through Z-50 respectively represent structures indicated by structural formulae below, 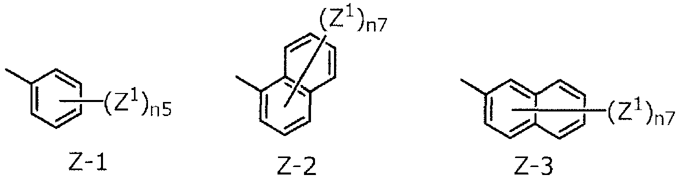 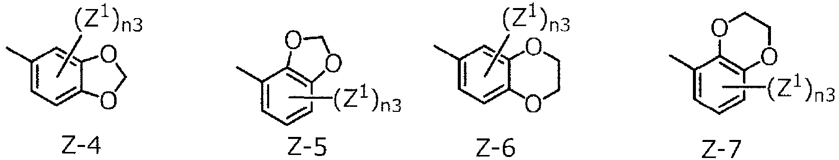 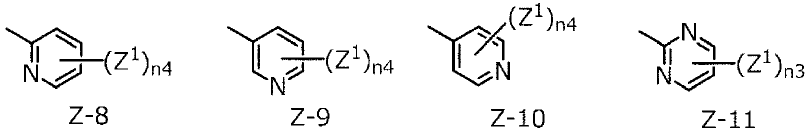 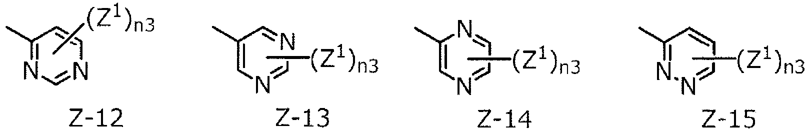 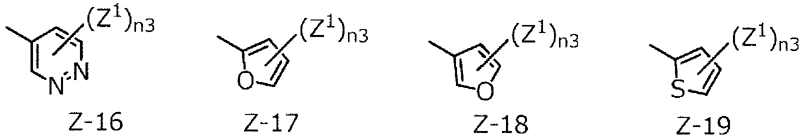 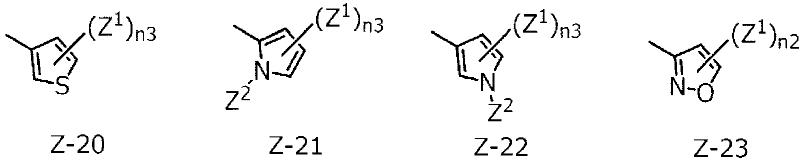 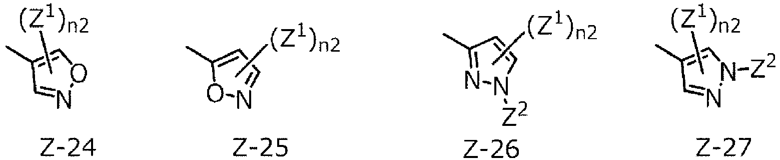 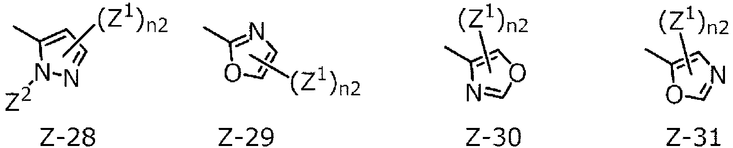 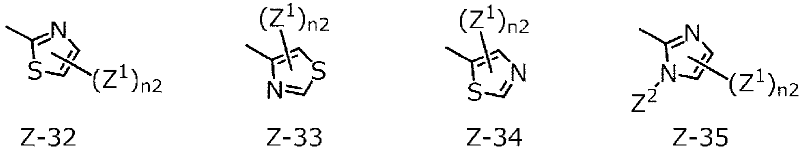 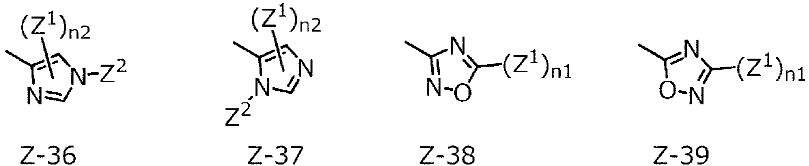 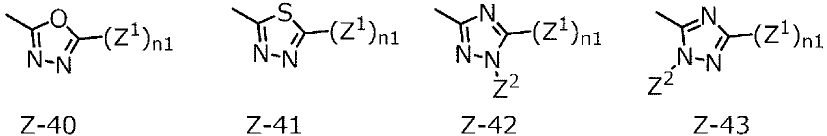 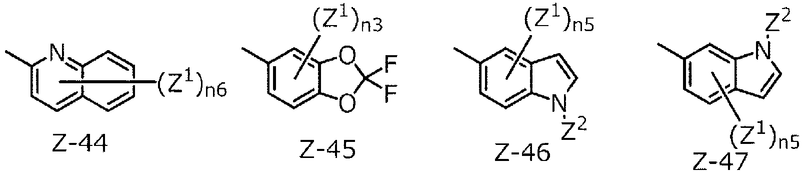 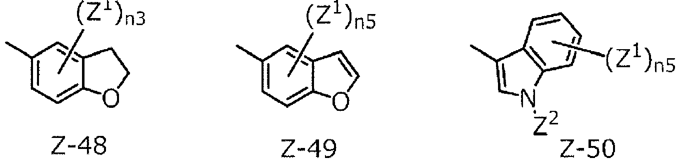
   a substitution position of Z¹ indicates that the substitution occurs on an aromatic ring of each of Z-1 through Z-50,
   Z¹ represents hydroxy, carboxy, amino, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ haloalkylthio, C₁-C₆ alkylsulfinyl, C₁-C₆ alkylsulfonyl, C₁-C₆ alkylcarbonyl, C₁-C₆ alkoxycarbonyl, - OR^{j}, -NHC(O)R^{k}, -C(O)NHR^{m}, -C(=NOR)R', -C(=NOH)NH₂, E-1 to E-54 or E-55,
   in a case where n2 represents an integer of 2 in relationship to Z¹, the respective Z¹ may be identical with each other or may be different from each other,
   in a case where n3 represents an integer of 2 or 3 in relationship to Z¹, the respective Z¹ may be identical with each other or may be different from each other,
   in a case where n4 represents an integer of 2, 3 or 4 in relationship to Z¹, the respective Z¹ may be identical with each other or may be different from each other,
   in a case where n5 represents an integer of 2, 3, 4 or 5 in relationship to Z¹, the respective Z¹ may be identical with each other or may be different from each other,
   in a case where n6 represents an integer of 2, 3, 4, 5 or 6 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   Z² represents C₁-C₆ alkyl, phenyl or pyridin-2-yl,
   R represents a hydrogen atom or C₁-C₆ alkyl,
   R' represents a hydrogen atom or C₁-C₆ alkyl,
   R^{j} represents C₂-C₆ alkenyl, C₂-C₆ alkynyl, C₁-C₆ alkyl substituted by R⁴, C₁-C₆ alkylcarbonyl, C₁-C₆ alkoxycarbonyl, C₁-C₆ alkylaminocarbonyl, E-1 to E-54 or E-55,
   R⁴ represents cyano, C₁-C₆ alkoxy, C₃-C₁₀ cycloalkyl, C₁-C₆ alkylcarbonyl, C₁-C₆ alkoxycarbonyl, di(C₁-C₆ alkyl)aminocarbonyl, E-1 to E-54 or E-55,
   R^{k} represents C₁-C₆ alkyl, C₃-C₁₀ cycloalkyl, C₁-C₆ haloalkyl, C₁-C₆ alkyl substituted by R⁵, C₁-C₆ alkoxy, C₂-C₆ alkenyloxy, C₁-C₆ alkylamino, di(C₁-C₆ alkyl)amino, benzyloxy, E-1 to E-54 or E-55,
   R⁵ represents cyano, C₁-C₆ alkoxy, E-1 to E-53 or E-54,
   R^{m} represents C₁-C₆ alkyl, C₃-C₁₀ cycloalkyl, C₂-C₆ alkenyl, C₁-C₆ alkoxy, benzyl, E-1 to E-54 or E-55,
   E-1 through E-55 respectively represent structures indicated by structural formulae below, 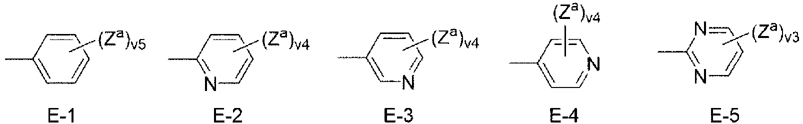 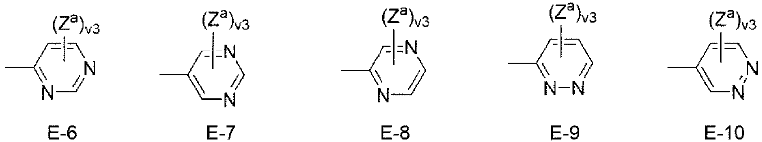 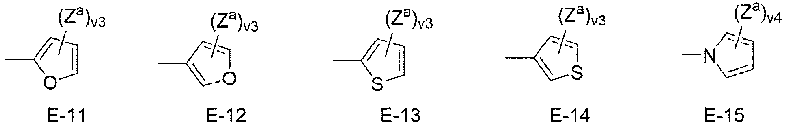 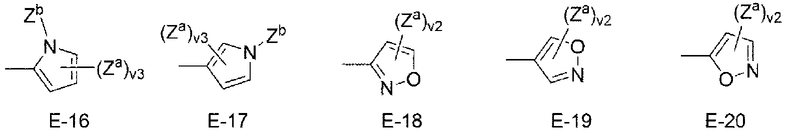 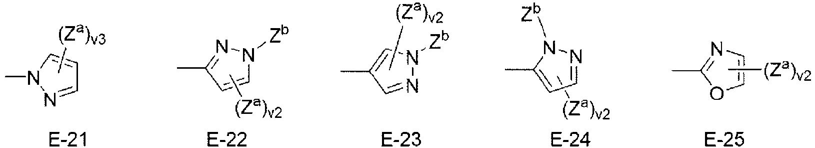 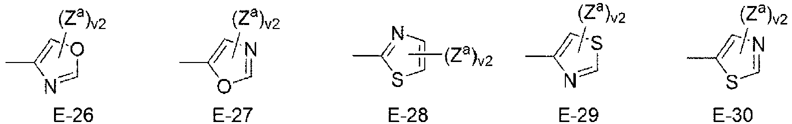 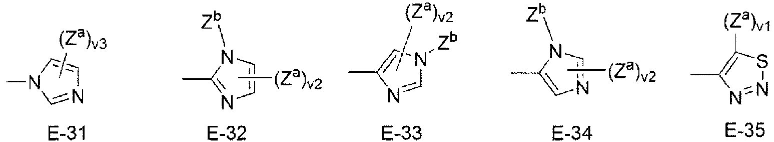 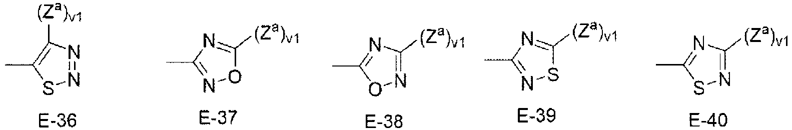 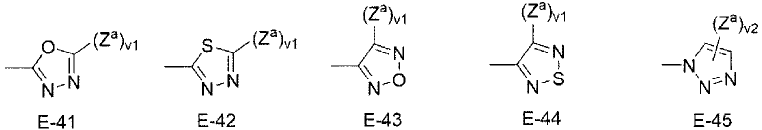 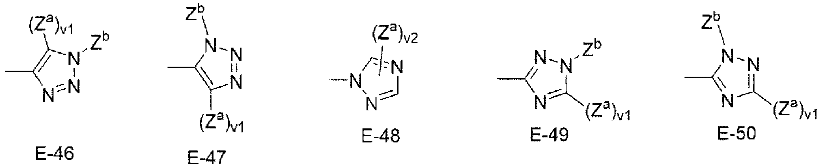 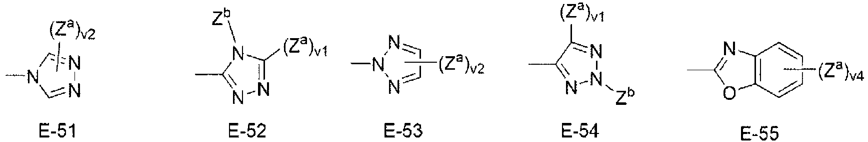
   Z^{a} represents a halogen atom, cyano, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ haloalkoxy, C₁-C₆ alkylcarbonyl, C₁-C₆ alkoxycarbonyl, phenyl, S-1 to S-5 or S-6,
   in a case where v5 represents an integer of 2, 3, 4 or 5 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   in a case where v3 represents an integer of 2 or 3 in relationship to Z^{a}, the respective Z^{a} may be identical with each other or may be different from each other,
   in a case where v2 represents an integer of 2 in relationship to Z^{a}, the respective Z^{a} may be identical with each other or may be different from each other,
   R⁶ represents hydroxy, C₁-C₆ alkoxy or C₁-C₆ alkoxycarbonyl,
   Z^{b} represents C₁-C₆ alkyl,
   S-1 through S-6 respectively represent structures indicated by structural formulae below, 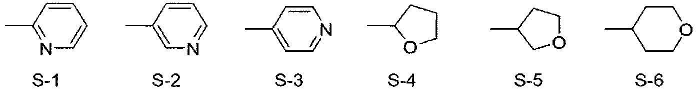
   Q-1 through Q-54 respectively represent structures indicated by structural formulae below, 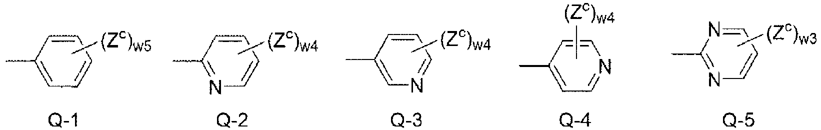 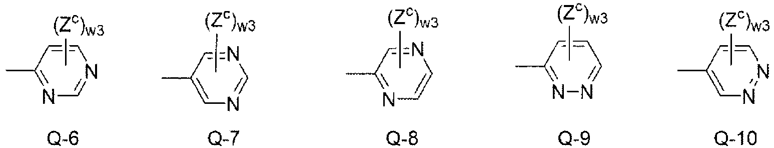 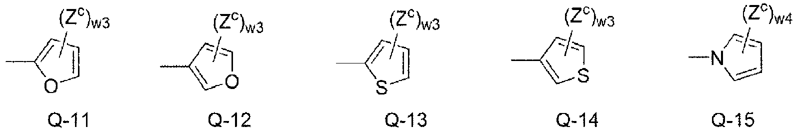 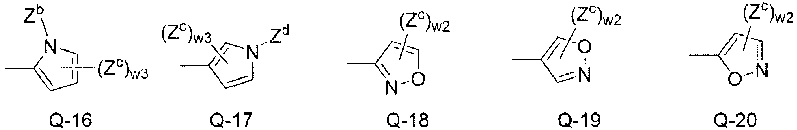 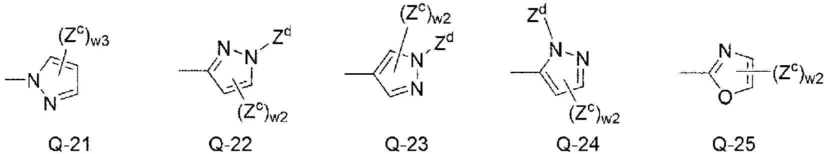 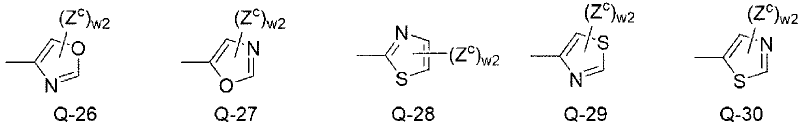 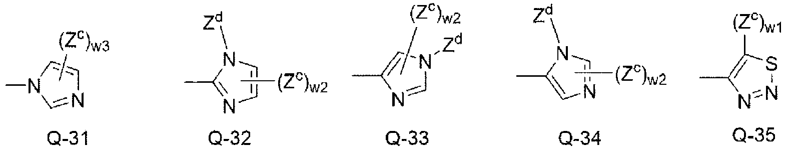 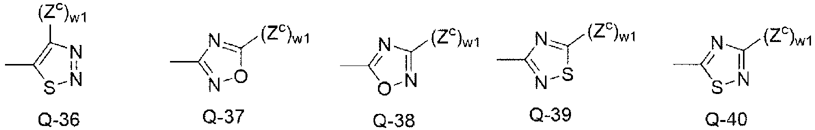 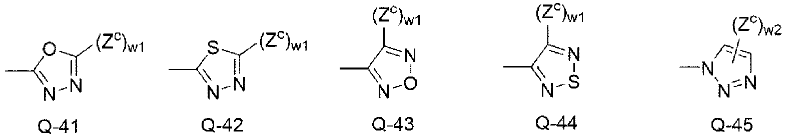 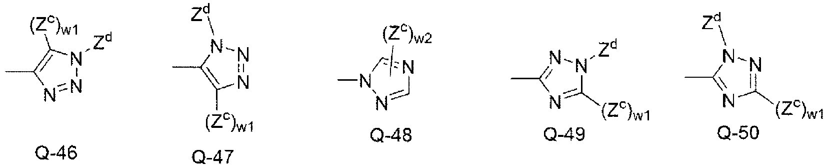 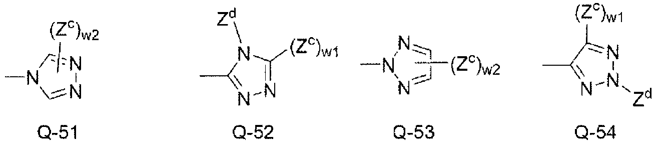
   Z^{c} represents a halogen atom, cyano, C₁-C₆ alkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where w5 represents an integer of 2, 3, 4 or 5 in relationship to Z^{c}, the respective Z^{c} may be identical with each other or may be different from each other,
   in a case where w4 represents an integer of 2, 3 or 4 in relationship to Z^{c}, the respective Z^{c} may be identical with each other or may be different from each other,
   in a case where w3 represents an integer of 2 or 3 in relationship to Z^{c}, the respective Z^{c} may be identical with each other or may be different from each other,
   in a case where w2 represents an integer of 2 in relationship to Z^{c}, the respective Z^{c} may be identical with each other or may be different from each other,
   Z^{d} represents C₁-C₆ alkyl,
   m5 represents an integer of 0, 1, 2, 3, 4 or 5,
   n2 represents an integer of 0, 1 or 2,
   n3 represents an integer of 0, 1, 2 or 3,
   n4 represents an integer of 0, 1, 2, 3 or 4,
   n5 represents an integer of 0, 1, 2, 3, 4 or 5,
   n6 represents an integer of 0, 1, 2, 3, 4, 5 or 6,
   n7 represents an integer of 0, 1, 2, 3, 4, 5, 6 or 7,
   u4 represents an integer of 0, 1, 2, 3 or 4,
   u5 represents an integer of 0, 1, 2, 3, 4 or 5,
   t5 represents an integer of 0, 1, 2, 3, 4 or 5,
   p represents an integer of 0 or 1,
   v1 represents an integer of 0 or 1,
   v2 represents an integer of 0, 1 or 2,
   v3 represents an integer of 0, 1, 2 or 3,
   v4 represents an integer of 0, 1, 2, 3 or 4,
   v5 represents an integer of 0, 1, 2, 3, 4 or 5,
   w1 represents an integer of 0 or 1,
   w2 represents an integer of 0, 1 or 2,
   w3 represents an integer of 0, 1, 2 or 3,
   w4 represents an integer of 0, 1, 2, 3 or 4, and
   w5 represents an integer of 0, 1, 2, 3, 4 or 5.
   Advantageous Effects of Invention

The compound in accordance with the present invention represented by the formula (1) exerts excellent control activity against many pathogens. Thus, the present invention can provide a useful fungicide, in particular, agricultural-horticultural fungicide.

### Description of Embodiments

The following description will discuss details of the present invention.

Depending on a type of substituent, geometric isomers of an E-form and a Z-form may be present in the compound in accordance with the present invention. The present invention encompasses the E-form, the Z-form, and a mixture containing the E-form and the Z-form in any proportions.

In addition, an optically active substance which results from the presence of one or two or more asymmetric carbon atoms or asymmetric sulfur atoms may be present in the compound in accordance with the present invention. The present invention encompasses all optically active substances and racemic bodies.

In addition, a tautomer may be present in the compound in accordance with the present invention depending on a type of substituent. The present invention encompasses all tautomers and a mixture containing tautomers in any proportion.

In addition, the compound in accordance with the present invention may be present as one or two or more rotational isomers due to restricted bond rotation caused by a steric hindrance between substituents. The present invention encompasses all rotational isomers and a mixture containing diastereomers in any proportion.

Specific examples of substituents indicated in this specification are provided below. Here, "n-" represents normal, "i-" represents iso, "s-" represents secondary, "tert" represents tertiary, and "Ph" represents phenyl.

Examples of the "halogen atom" in this specification include a fluorine atom, a chlorine atom, a bromine atom and an iodine atom. The term "halo" in this specification also represents such a halogen atom.

The term "Cₐ-C_{b} alkyl" in this specification indicates a linear or branched saturated hydrocarbon group in which the number of carbon atoms is a to b. Specific examples of "Cₐ-C_{b} alkyl" include methyl, ethyl, n-propyl, i-propyl, n-butyl, i-butyl, s-butyl, tert-butyl, n-pentyl, 1,1-dimethylpropyl, n-hexyl and the like, each of which is selected within a range of the specified number of carbon atoms.

The term "Cₐ-C_{b} haloalkyl" in this specification indicates a linear or branched saturated hydrocarbon group in which the number of carbon atoms is a to b and in which a hydrogen atom bonded to a carbon atom has been arbitrarily substituted by a halogen atom. In a case of substitution by two or more halogen atoms, the halogen atoms may be identical with each other or may be different from each other. Specific examples of "Cₐ-C_{b} haloalkyl" include fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 2-fluoroethyl, 2-chloroethyl, 2-bromoethyl, 2,2-difluoroethyl, 2,2,2-trifluoroethyl, 2-chloro-2,2-difluoroethyl, 2,2,2-trichloroethyl and the like, each of which is selected within a range of the specified number of carbon atoms.

The term "Cₐ-C_{b} alkenyl" in this specification indicates linear or branched unsaturated hydrocarbon in which the number of carbon atoms is a to b and in which a molecule thereof has one or two or more double bonds. Specific examples of "Cₐ-C_{b} alkenyl" include vinyl, 1-propenyl, 2-propenyl (hereinafter also referred to as allyl), 1-methylethenyl, 2-butenyl, 2-methyl-2-propenyl, 3-methyl-2-butenyl, 1,1-dimethyl-2-propenyl and the like, each of which is selected within a range of the specified number of carbon atoms.

The term "Cₐ-C_{b} alkynyl" in this specification indicates linear or branched unsaturated hydrocarbon in which the number of carbon atoms is a to b and in which a molecule thereof has one or two or more triple bonds. Specific examples of "Cₐ-C_{b} alkynyl" include ethynyl, propargyl, 2-butynyl, 3-butynyl, 1-pentynyl, 1-hexynyl and the like, each of which is selected within a range of the specified number of carbon atoms.

The term "Cₐ-C_{b} alkoxy" in this specification indicates alkyl-O- in which alkyl has the foregoing meaning and in which the number of carbon atoms is a to b. Specific examples of "Cₐ-C_{b} alkoxy" include methoxy, ethoxy, n-propyloxy, i-propyloxy, n-butyloxy, i-butyloxy, s-butyloxy, tert-butyloxy, 2-ethylhexyloxy and the like, each of which is selected within a range of the specified number of carbon atoms.

The term "Cₐ-C_{b} haloalkoxy" in this specification indicates haloalkyl-O- in which haloalkyl has the foregoing meaning and in which the number of carbon atoms is a to b. Specific examples of "Cₐ-C_{b} haloalkoxy" include difluoromethoxy, trifluoromethoxy, chlorodifluoromethoxy, bromodifluoromethoxy, 2-fluoroethoxy, 2-chloroethoxy, 2,2,2-trifluoroethoxy, 1,1,2,2,-tetrafluoroethoxy, 2-chloro-1,1,2-trifluoroethoxy, 1,1,2,3,3,3-hexafluoropropyloxy and the like, each of which is selected within a range of the specified number of carbon atoms.

The term "Cₐ-C_{b} alkenyloxy" in this specification indicates alkenyl-O- in which alkenyl has the foregoing meaning and in which the number of carbon atoms is a to b. Specific examples of "Cₐ-C_{b} alkenyloxy" include vinyloxy, 1-propenyloxy, 2-propenyloxy, 1-methylethenyloxy, 2-butenyloxy, 2-methyl-2-propenyloxy, 3-methyl-2-butenyloxy, 1,1-dimethyl-2-propenyloxy and the like, each of which is selected within a range of the specified number of carbon atoms.

The term "Cₐ-C_{b} alkylthio" in this specification indicates alkyl-S- in which alkyl has the foregoing meaning and in which the number of carbon atoms is a to b. Specific examples of "Cₐ-C_{b} alkylthio" include methylthio, ethylthio, n-propylthio, i-propylthio, n-butylthio, i-butylthio, s-butylthio, tert-butylthio and the like, each of which is selected within a range of the specified number of carbon atoms.

The term "Cₐ-C_{b} haloalkylthio" in this specification indicates haloalkyl-S- in which haloalkyl has the foregoing meaning and in which the number of carbon atoms is a to b. Specific examples of "Cₐ-C_{b} haloalkylthio" include difluoromethylthio, trifluoromethylthio, chlorodifluoromethylthio, bromodifluoromethylthio, 2-fluoroethylthio, 2-chloroethylthio, 2,2,2-trifluoroethylthio, 1,1,2,2,-tetrafluoroethylthio, 2-chloro-1,1,2-trifluoroethylthio, 1,1,2,3,3,3-hexafluoropropylthio and the like, each of which is selected within a range of the specified number of carbon atoms.

The term "Cₐ-C_{b} alkylsulfinyl" in this specification indicates alkyl-S(O)- in which alkyl has the foregoing meaning and in which the number of carbon atoms is a to b. Specific examples of "Cₐ-C_{b} alkylsulfinyl" include methylsulfinyl, ethylsulfinyl, n-propylsulfinyl, i-propylsulfinyl, n-butylsulfinyl, i-butylsulfinyl, S-butylsulfinyl, tert-butylsulfinyl and the like, each of which is selected within a range of the specified number of carbon atoms.

The term "Cₐ-C_{b} alkylsulfonyl" in this specification indicates alkyl-SO₂- in which alkyl has the foregoing meaning and in which the number of carbon atoms is a to b. Specific examples of "Cₐ-C_{b} alkylsulfonyl" include methylsulfonyl, ethylsulfonyl, n-propylsulfonyl, i-propylsulfonyl, n-butylsulfonyl, i-butylsulfonyl, s-butylsulfonyl, tert-butylsulfonyl and the like, each of which is selected within a range of the specified number of carbon atoms.

The term "Cₐ-C_{b} cycloalkyl" in this specification indicates a cyclic hydrocarbon group in which the number of carbon atoms is a to b. "Cₐ-C_{b} cycloalkyl" can form a monocyclic or fused ring structure from a 3-membered ring to a 10-membered ring. Each of the rings may be arbitrarily substituted by alkyl to a range of the specified number of carbon atoms. Specific examples of "Cₐ-C_{b} cycloalkyl" include cyclopropyl, 1-methylcyclopropyl, 2-methylcyclopropyl, 2,2-dimethylcyclopropyl, cyclobutyl, cyclopentyl, cyclohexyl and the like, each of which is selected within a range of the specified number of carbon atoms.

The term "Cₐ-C_{b} alkylamino" in this specification indicates amino in which one of hydrogen atoms has been substituted by alkyl which has the foregoing meaning and in which the number of carbon atoms is a to b. Specific examples of "Cₐ-C_{b} alkylamino" include methylamino, ethylamino, n-propylamino, i-propylamino, n-butylamino, i-butylamino, tert-butylamino and the like, each of which is selected within a range of the specified number of carbon atoms.

The term "di(Cₐ-C_{b} alkyl)amino" in this specification indicates amino in which both hydrogen atoms have been substituted by alkyls which have the foregoing meaning, in which the number of carbon atoms is a to b, and which may be identical with each other or different from each other. Specific examples of "di(Cₐ-C_{b} alkyl)amino" include dimethylamino, ethyl(methyl)amino, diethylamino, n-propyl(methyl)amino, i-propyl(methyl)amino, di(n-propyl)amino, di(n-butyl)amino and the like, each of which is selected within a range of the specified number of carbon atoms.

The term "Cₐ-C_{b} alkylcarbonyl" in this specification indicates alkyl-C(O)- in which alkyl has the foregoing meaning and in which the number of carbon atoms is a to b. Specific examples of "Cₐ-C_{b} alkylcarbonyl" include acetyl, propionyl, butyryl, isobutyryl, valeryl, isovaleryl, 2-methylbutanoyl, pivaloyl, hexanoyl, heptanoyl and the like, each of which is selected within a range of the specified number of carbon atoms.

The term "Cₐ-C_{b} haloalkylcarbonyl" in this specification indicates haloalkyl-C(O)- in which haloalkyl has the foregoing meaning and in which the number of carbon atoms is a to b. Specific example of "Cₐ-C_{b} haloalkylcarbonyl" include difluoroacetyl, trichloroacetyl, trifluoroacetyl and the like, each of which is selected within a range of the specified number of carbon atoms.

The term "Cₐ-C_{b} alkenylcarbonyl" in this specification indicates alkenyl-C(O)- in which alkenyl has the foregoing meaning and in which the number of carbon atoms is a to b. Specific examples of "Cₐ-C_{b} alkenylcarbonyl" include vinylcarbonyl, 1-propenylcarbonyl, 2-propenylcarbonyl, 1-methylethenylcarbonyl, 2-butenylcarbonyl, 2-methyl-2-propenylcarbonyl, 3-methyl-2-butenylcarbonyl, 1,1-dimethyl-2-propenylcarbonyl and the like, each of which is selected within a range of the specified number of carbon atoms.

The term "Cₐ-C_{b} cycloalkylcarbonyl" in this specification indicates cycloalkyl-C(O)- in which cycloalkyl has the foregoing meaning and in which the number of carbon atoms is a to b. Specific examples of "Cₐ-C_{b} cycloalkylcarbonyl" include cyclopropylcarbonyl, 1-methylcyclopropylcarbonyl, 2-methylcyclopropylcarbonyl, 2,2-dimethylcyclopropylcarbonyl, cyclobutylcarbonyl, cyclopentylcarbonyl, cyclohexylcarbonyl and the like, each of which is selected within a range of the specified number of carbon atoms.

The term "Cₐ-C_{b} alkoxycarbonyl" in this specification indicates alkyl-O-C(O)- in which alkyl has the foregoing meaning and in which the number of carbon atoms is a to b. Specific examples of "Cₐ-C_{b} alkoxycarbonyl" include methoxycarbonyl, ethoxycarbonyl, n-propyloxycarbonyl, i-propyloxycarbonyl, n-butoxycarbonyl, i-butoxycarbonyl, s-butoxycarbonyl, tert-butoxycarbonyl, 2-ethylhexyloxycarbonyl and the like, each of which is selected within a range of the specified number of carbon atoms.

The term "Cₐ-C_{b} haloalkoxycarbonyl" in this specification indicates haloalkyl-O-C(O)- in which haloalkyl has the foregoing meaning and in which the number of carbon atoms is a to b. Specific examples of "Cₐ-C_{b} haloalkoxycarbonyl" include 2,2,2-trichloroethoxycarbonyl, 2,2,2-trifluoroethoxycarbonyl, 2-chloro-2,2-difluoroethoxycarbonyl, 3,3,3-trifluoropropyloxycarbonyl and the like, each of which is selected within a range of the specified number of carbon atoms.

The term "Cₐ-C_{b} alkenyloxycarbonyl" in this specification indicates alkenyl-O-C(O)- in which alkenyl has the foregoing meaning and in which the number of carbon atoms is a to b. Specific examples of "Cₐ-C_{b} alkenyloxycarbonyl" include vinyloxycarbonyl, 1-propenyloxycarbonyl, 2-propenyloxycarbonyl, 1-methylethenyloxycarbonyl, 2-butenyloxycarbonyl, 2-methyl-2-propenyloxycarbonyl, 3-methyl-2-butenyloxycarbonyl, 1,1-dimethyl-2-propenyloxycarbonyl and the like, each of which is selected within a range of the specified number of carbon atoms.

The term "Cₐ-C_{b} alkylcarbonyloxy" in this specification indicates alkyl-C(O)-O- in which alkyl has the foregoing meaning and in which the number of carbon atoms is a to b. Specific examples of "Cₐ-C_{b} alkylcarbonyloxy" include acetyloxy, propionyloxy, pivaloyloxy and the like, each of which is selected within a range of the specified number of carbon atoms.

The term "Cₐ-C_{b} alkoxycarbonyloxy" in this specification indicates alkyl-O-C(O)-O- in which alkyl has the foregoing meaning and in which the number of carbon atoms is a to b. Specific examples of "Cₐ-C_{b} alkoxycarbonyloxy" include methoxycarbonyloxy, ethoxycarbonyloxy, i-butoxycarbonyloxy and the like, each of which is selected within a range of the specified number of carbon atoms.

The term "Cₐ-C_{b} alkylaminocarbonyl" in this specification indicates carbamoyl in which one of hydrogen atoms has been substituted by alkyl which has the foregoing meaning and in which the number of carbon atoms is a to b. Specific examples of "Cₐ-C_{b} alkylaminocarbonyl" include methylcarbamoyl, ethylcarbamoyl, n-propylcarbamoyl, i-propylcarbamoyl, n-butylcarbamoyl, i-butylcarbamoyl, s-butylcarbamoyl, tert-butylcarbamoyl and the like, each of which is selected within a range of the specified number of carbon atoms.

The term "di(Cₐ-C_{b} alkyl)aminocarbonyl" in this specification indicates carbamoyl in which both hydrogen atoms have been substituted by alkyls which have the foregoing meaning, in which the number of carbon atoms is a to b, and which may be identical with each other or different from each other. Specific examples of "di(Cₐ-C_{b} alkyl)aminocarbonyl" include N,N-dimethylcarbamoyl, N-ethyl-N-methylcarbamoyl, N,N-diethylcarbamoyl, N,N-di(n-propyl)carbamoyl, N,N-di(n-butyl)carbamoyl and the like, each of which is selected within a range of the specified number of carbon atoms.

The term "Cₐ-C_{b} alkylaminothiocarbonyl" in this specification indicates alkylamino-C(=S)-. Alkylamino in this alkylamino-C(=S)- has the foregoing meaning and, in this alkylamino, one of hydrogen atoms of amino is alkyl in which the number of carbon atoms is a to b. Specific examples of "Cₐ-C_{b} alkylaminothiocarbonyl" include methylthiocarbamoyl, ethylthiocarbamoyl, n-propylthiocarbamoyl, i-propylthiocarbamoyl, n-butylthiocarbamoyl, i-butylthiocarbamoyl, s-butylthiocarbamoyl, tertbutylthiocarbamoyl and the like, each of which is selected within a range of the specified number of carbon atoms.

The term "di(Cₐ-C_{b} alkyl)aminothiocarbonyl" in this specification indicates alkylamino-C(=S)-. Alkylamino in this alkylamino-C(=S)- has the foregoing meaning and, in this alkylamino, both hydrogen atoms of amino are alkyls in which the number of carbon atoms is a to b and which may be identical with each other or different from each other. Specific examples of "di(Cₐ-C_{b} alkyl)aminothiocarbonyl" include N,N-dimethylthiocarbamoyl, N-ethyl-N-methylthiocarbamoyl, N,N-diethylthiocarbamoyl, N,N-di(n-propyl)thiocarbamoyl, N,N-di(n-butyl)thiocarbamoyl and the like, each of which is selected within a range of the specified number of carbon atoms.

The term "tri(Cₐ-C_{b} alkyl)silyl" in this specification indicates silyl which has been substituted by alkyls which have the foregoing meaning, in which the number of carbon atoms is a to b, and which may be identical with each other or different from each other. Specific examples of "tri(Cₐ-C_{b} alkyl)silyl" include trimethylsilyl, triethylsilyl, tri(n-propyl)silyl, ethyldimethylsilyl, n-propyldimethylsilyl, n-butyldimethylsilyl, i-butyldimethylsilyl, tertbutyldimethylsilyl and the like, each of which is selected within a range of the specified number of carbon atoms.

The term "Cₐ-C_{b} alkyl substituted by R⁴", "Cₐ-C_{b} alkyl substituted by R^{5"}, "Cₐ-C_{b} alkyl substituted by R^{6"}, "Cₐ-C_{b} alkyl substituted by R^{a"}, "Cₐ-C_{b} alkyl substituted by R^{h"} or "Cₐ-C_{b} alkyl substituted by R^{i"} in this specification indicates alkyl which has the foregoing meaning, in which the number of carbon atoms is a to b, and in which any hydrogen atoms bonded to carbon atoms have partially or all substituted by one or more substituents R⁴, R⁵, R⁶, R^{a}, R^{h} or Rⁱ. Each of the alkyls is selected within a range of the specified number of carbon atoms. In the presence of two or more substituents R⁴, R⁵, R⁶, R^{a}, R^{h} or Rⁱ, the substituents R⁴, R⁵, R⁶, R^{a}, R^{h} or Rⁱ may be identical with each other or may be different from each other.

The term "Cₐ-C_{b} alkylcarbonyl substituted by R^{a"} in this specification indicates alkyl-C(O)- in which alkyl has the foregoing meaning, in which the number of carbon atoms is a to b, and in which any hydrogen atoms bonded to carbon atoms have partially or all substituted by one or more substituents R^{a}. Alkyl is selected within a range of the specified number of carbon atoms. In the presence of two or more substituents R^{a}, the substituents R^{a} may be identical with each other or may be different from each other.

The term "Cₐ-C_{b} alkenylcarbonyl substituted by R^{a"} in this specification indicates alkenyl-C(O)- in which alkenyl has the foregoing meaning, in which the number of carbon atoms is a to b, and in which any hydrogen atoms bonded to carbon atoms have partially or all substituted by one or more substituents R^{a}. Alkenyl is selected within a range of the specified number of carbon atoms. In the presence of two or more substituents R^{a}, the substituents R^{a} may be identical with each other or may be different from each other.

The term "Cₐ-C_{b} alkoxycarbonyl substituted by R^{a"} in this specification indicates alkyl-O-C(O)- in which alkyl has the foregoing meaning, in which the number of carbon atoms is a to b, and in which any hydrogen atoms bonded to carbon atoms have partially or all substituted by one or more substituents R^{a}. Alkyl is selected within a range of the specified number of carbon atoms. In the presence of two or more substituents R^{a}, the substituents R^{a} may be identical with each other or may be different from each other.

The term "benzyl" in this specification indicates - CH₂C₆H₅.

The term "benzyloxy" in this specification indicates - O-CH₂C₆H₅.

The following description will discuss a method for producing the compound in accordance with the present invention represented by the formula (1). The compound in accordance with the present invention represented by the formula (1) can be produced by, for example, a method below. The descriptions below are merely an example, and the compound in accordance with the present invention represented by the formula (1) may be produced with other methods. Hereinafter, the "compound in accordance with the present invention represented by the formula (1)" will be referred to also as a "compound (1) in accordance with the present invention", and a "compound represented by a formula (2)" will be referred to also as a "compound (2)". The other compounds will be described similarly in accordance with this description.

### (Production Example 1)

The compound (1) in accordance with the present invention can be produced by, for example, the following production method.

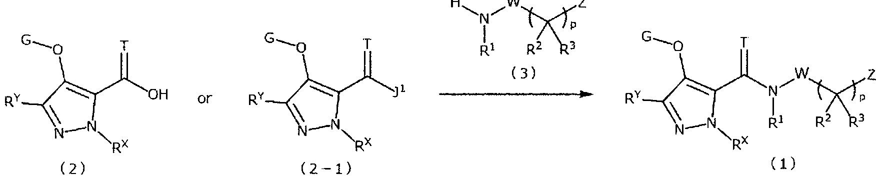

In the formula, J¹ represents a chlorine atom, a bromine atom, C₁-C₄ alkylcarbonyloxy or C₁-C₄ alkoxycarbonyloxy, and G, T, R^{X}, R^{Y}, R¹, R², R³, W, Z and p have the same meanings as above.

The compound (1) in accordance with the present invention can be produced by causing a compound (2), a compound (3) or a salt thereof (e.g., hydrochloride, hydrobromide, hydroiodide, sulfate, trifluoroacetate, oxalate, para-toluenesulfonate or the like) and a dehydration condensation agent to react with each other in a solvent or without a solvent and optionally in the presence of one of or both of a base and an additive.

Alternatively, the compound (1) in accordance with the present invention can be produced by causing a compound (2-1) and the compound (3) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of one of or both of a base and an additive.

An equivalent of the compound (3) or a salt thereof used can be an equivalent of 0.5 to 50 with respect to an equivalent of 1 of the compound (2) or compound (2-1).

Some of compounds (3) are known compounds, and can be obtained as commercially available products. The other compounds can also be produced in accordance with a method for synthesizing a general known compound disclosed in a literature.

An equivalent of the dehydration condensation agent used can be an equivalent of 0.5 to 50 with respect to an equivalent of 1 of the compound (2).

Examples of the dehydration condensation agent include 1H-benzotriazol-1-yloxytris(dimethylamino)phosphonium hexafluorophosphate, N,N'-dicyclohexylcarbodiimide, 1-ethyl-3-(3-dimethylaminopropyl)carbodiimide hydrochloride, 1-[bis(dimethylamino)methylene]-1H-1,2,3-triazolo[4,5-b]pyridinium 3-oxide hexafluorophosphate and the like.

In a case where a solvent is used, the solvent used only needs to be inert with respect to reaction. Examples of the solvent include: water; alcohol solvents such as methanol, ethanol and tert-butyl alcohol; ether solvents such as diethyl ether, tetrahydrofuran, 1,4-dioxane, 1,2-dimethoxyethane and diglyme; aromatic hydrocarbon solvents such as benzene, xylene and toluene; aliphatic hydrocarbon solvents such as n-pentane, n-hexane and cyclohexane; halogenated hydrocarbon solvents such as dichloromethane, chloroform and 1,2-dichloroethane; nitrile solvents such as acetonitrile and propionitrile; amide solvents such as N,N-dimethylformamide, N,N-dimethylacetamide, N-methylpyrrolidone and N,N'-dimethylimidazolidinone; dimethyl sulfoxide; pyridine; mixed solvents of these; and the like.

Examples of the base that can be used in the reaction include: organic bases such as pyridine, 2,6-lutidine, 4-dimethylaminopyridine, triethylamine, diisopropylethylamine, tributylamine, N,N-dimethylaniline, 1,4-diazabicyclo[2.2.2]octane (DABCO), 1,8-diazabicyclo[5.4.0]-7-undecene (DBU) and 1,5-diazabicyclo[4.3.0]-5-nonene (DBN); inorganic bases such as sodium hydroxide, potassium hydroxide, sodium hydride, sodium hydrogen carbonate, potassium carbonate, cesium carbonate and potassium phosphate; and metal alkoxides such as sodium methoxide, sodium ethoxide and potassium tert-butoxide. A use amount (equivalent) of the base can be an equivalent of 0.1 to 100 with respect to an equivalent of 1 of the compound (2) or compound (2-1).

Examples of the additive that can be used in the reaction include 1-hydroxybenzotriazole, 1-hydroxy-7-azabenzotriazole, 4-(dimethylamino)pyridine and the like. A use amount (equivalent) of the additive can be an equivalent of 0.005 to 100 with respect to an equivalent of 1 of the compound (2) or compound (2-1).

A reaction temperature can be set to an arbitrary temperature from -78°C to a reflux temperature of the reaction mixture. A reaction time varies in accordance with a concentration of a reactive substrate or a reaction temperature. The reaction time can be set arbitrarily, usually in a range of 5 minutes to 100 hours.

### (Production Example 2)

A compound (1-1-1) in accordance with the present invention can be produced by, for example, the following production method.

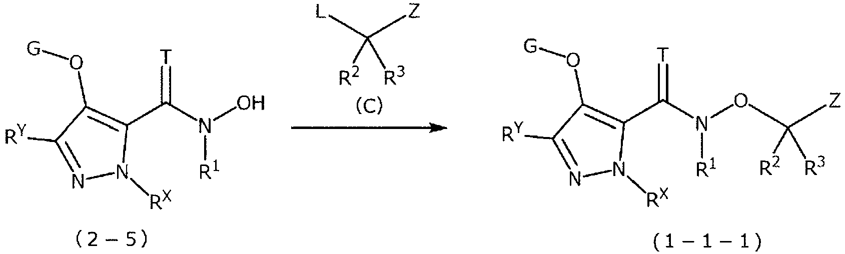

In the formula, L represents a leaving group such as a chlorine atom, a bromine atom, an iodine atom, C₁-C₄ alkylsulfonyloxy (e.g., methanesulfonyloxy or the like), halosulfonyloxy (e.g., fluorosulfonyloxy or the like), C₁-C₄ haloalkylsulfonyloxy (e.g., trifluoromethanesulfonyloxy or the like), arylsulfonyloxy (e.g., para-toluenesulfonyloxy or the like), C₁-C₄ alkylcarbonyloxy or C₁-C₄ alkoxycarbonyloxy, and G, T, R^{X}, R^{Y}, R¹, R², R³ and Z have the same meanings as above.

The compound (1-1-1) in accordance with the present invention can be produced by causing a compound (2-5) and a compound (C) to react with each other in a solvent or without a solvent and optionally in the presence of one of or both of a base and an additive.

An equivalent of the compound (C) used can be an equivalent of 0.5 to 50 with respect to an equivalent of 1 of the compound (2-5).

Some of compounds (C) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-5).

Examples of the additive that can be used in the reaction include sodium iodide, potassium iodide, and the like. A use amount (equivalent) of the additive can be an equivalent of 0.005 to 100 with respect to an equivalent of 1 of the compound (2-5).

A reaction temperature and a reaction time are within the temperature range and the time range described in Production Example 1.

### (Production Example 3)

A compound (1-1-3) in accordance with the present invention can be produced by, for example, the following production method.

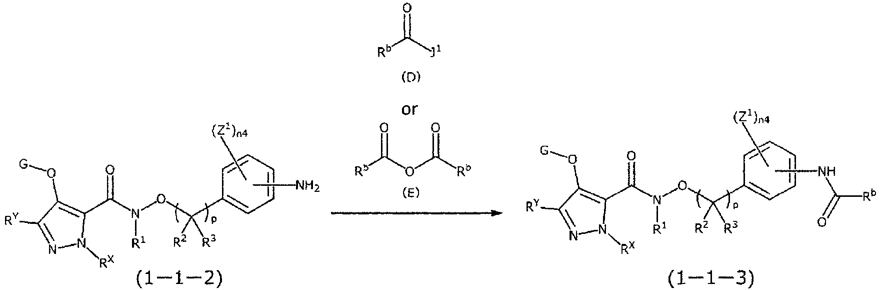

In the formula, G, J¹, R^{b}, R^{X}, R^{Y}, R¹, R², R³, Z¹, n4 and p have the same meanings as above.

The compound (1-1-3) in accordance with the present invention can be produced by causing a compound (1-1-2) in accordance with the present invention and a compound (D) or compound (E) to react with each other in a solvent or without a solvent and optionally in the presence of one of or both of a base and an additive.

An equivalent of the compound (D) or compound (E) used can be an equivalent of 0.5 to 50 with respect to an equivalent of 1 of the compound (1-1-2) in accordance with the present invention.

Some of compounds (D) and compounds (E) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-1-2) in accordance with the present invention.

Examples of the additive that can be used in the reaction include the additives exemplified in Production Example 1. A use amount (equivalent) of the additive can be an equivalent of 0.005 to 100 with respect to an equivalent of 1 of the compound (1-1-2) in accordance with the present invention.

A reaction temperature and a reaction time are within the temperature range and the time range described in Production Example 1.

The compound (1-1-2) in accordance with the present invention can be synthesized in accordance with the method of production method 1.

### (Production Example 4)

A compound (1-1-5) in accordance with the present invention can be produced by, for example, the following production method.

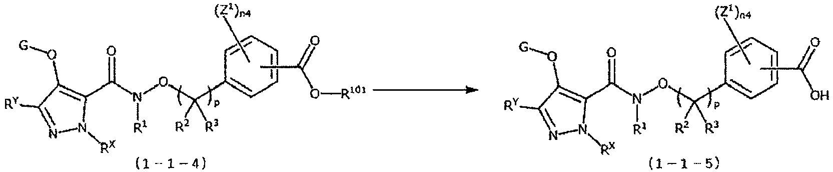

In the formula, R¹⁰¹ represents C₁-C₆ alkyl, and G, R^{X}, R^{Y}, R¹, R², R³, Z¹, n4 and p have the same meanings as above.

The compound (1-1-5) in accordance with the present invention can be obtained by causing a compound (1-1-4) in accordance with the present invention to be hydrolyzed in a solvent or without a solvent and optionally in the presence of a base.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-1-4) in accordance with the present invention.

A reaction temperature and a reaction time are within the temperature range and the time range described in Production Example 1.

The compound (1-1-4) in accordance with the present invention can be synthesized in accordance with the method of production method 1.

### (Production Example 5)

A compound (1-1-6) in accordance with the present invention can be produced by, for example, the following production method.

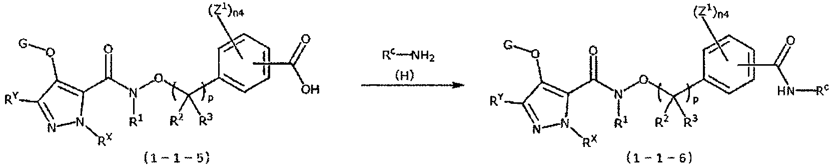

In the formula, G, R^{c}, R^{X}, R^{Y}, R¹, R², R³, Z¹, n4 and p have the same meanings as above.

The compound (1-1-6) in accordance with the present invention can be produced by causing the compound (1-1-5) in accordance with the present invention, a compound (H) or a salt thereof (e.g., hydrochloride, hydrobromide, hydroiodide, sulfate, trifluoroacetate, oxalate, para-toluenesulfonate or the like), and a dehydration condensation agent to react with each other in a solvent or without a solvent and optionally in the presence of one of or both of a base and an additive.

An equivalent of the compound (H) or a salt thereof used can be an equivalent of 0.5 to 50 with respect to an equivalent of 1 of the compound (1-1-5) in accordance with the present invention.

Some of compounds (H) are known compounds, and can be obtained as commercially available products. The other compounds can also be produced in accordance with a method for synthesizing a general known compound disclosed in a literature.

Examples of the dehydration condensation agent that can be used in the reaction include the dehydration condensation agents exemplified in Production Example 1. A use amount (equivalent) of the dehydration condensation agent can be an equivalent of 0.1 to 100 with respect to an equivalent of 1 of the compound (1-1-5) in accordance with the present invention.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-1-5) in accordance with the present invention.

Examples of the additive that can be used in the reaction include the additives exemplified in Production Example 1. A use amount (equivalent) of the additive can be an equivalent of 0.1 to 100 with respect to an equivalent of 1 of the compound (1-1-5) in accordance with the present invention.

A reaction temperature and a reaction time are within the temperature range and the time range described in Production Example 1.

### (Production Example 6)

A compound (1-1-7) in accordance with the present invention can be produced by, for example, the following production method.

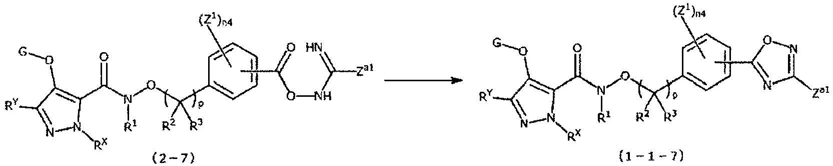

In the formula, Z^{a1} represents C₁-C₆ alkyl, C₃-C₁₀ cycloalkyl or C₁-C₆ haloalkyl, and G, R^{X}, R^{Y}, R¹, R², R³, Z¹, n4 and p have the same meanings as above.

The compound (1-1-7) in accordance with the present invention can be produced by causing a compound (2-7) to react in a solvent or without a solvent and optionally in the presence of a base.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-7).

A reaction temperature and a reaction time are within the temperature range and the time range described in Production Example 1.

### (Production Example 7)

A compound (1-1-9) in accordance with the present invention can be produced by, for example, the following production method.

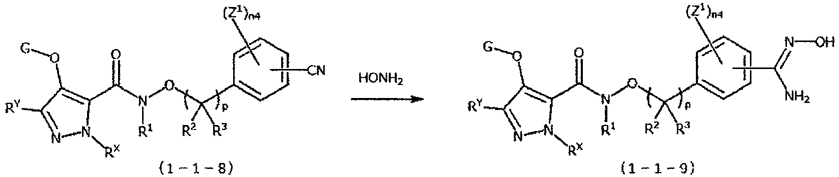

In the formula, G, R^{X}, R^{Y}, R¹, R², R³, Z¹, n4 and p have the same meanings as above.

The compound (1-1-9) in accordance with the present invention can be produced by causing a compound (1-1-8) in accordance with the present invention and hydroxylamine or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of a base.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-1-8) in accordance with the present invention.

A reaction temperature and a reaction time are within the temperature range and the time range described in Production Example 1.

The compound (1-1-8) in accordance with the present invention can be synthesized in accordance with the method of production method 1.

### (Production Example 8)

A compound (1-1-10) in accordance with the present invention can be produced by, for example, the following production method.

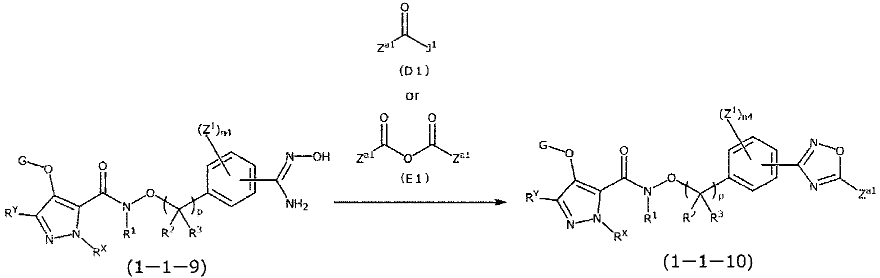

In the formula, G, R^{X}, R^{Y}, R¹, R², R³, Z¹, Za¹, n4 and p have the same meanings as above.

The compound (1-1-10) in accordance with the present invention can be produced by causing the compound (1-1-9) in accordance with the present invention and a compound (D1) or compound (E1) to react with each other in a solvent or without a solvent and optionally in the presence of a base.

Some of compounds (D1) and compounds (E1) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-1-8) in accordance with the present invention.

A reaction temperature and a reaction time are within the temperature range and the time range described in Production Example 1.

### (Production Example 9)

A compound (1-1-11) in accordance with the present invention can be produced by, for example, the following production method.

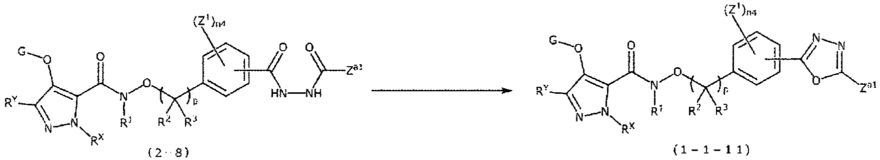

In the formula, G, R^{X}, R^{Y}, R¹, R2, R³, Z¹, Z^{a1} n4 and p have the same meanings as above.

The compound (1-1-11) in accordance with the present invention can be produced by causing a compound (2-8) and a dehydration agent to react with each other in a solvent or without a solvent.

Examples of the dehydration agent that can be used include methyl N-(triethylammoniosulfonyl)carbamate and the like. A use amount (equivalent) of the dehydration agent can be an equivalent of 0.1 to 100 with respect to an equivalent of 1 of the compound (2-8).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

### (Production Example 10)

Among compounds (1) in accordance with the present invention, a compound (1-2-2) in accordance with the present invention in which W is -N(R^{W})- and R^{w} is a hydrogen atom can be produced by, for example, the following production method.

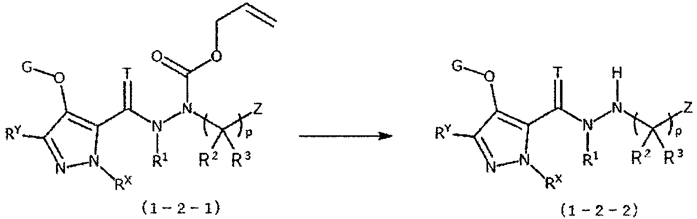

In the formula, G, T, R^{X}, R^{Y}, R¹, R², R³, Z and p have the same meanings as above.

The compound (1-2-2) in accordance with the present invention can be produced by causing a compound (1-2-1) in accordance with the present invention and a nucleophile to react with each other in a solvent or without a solvent and in the presence of a palladium catalyst.

An equivalent of the nucleophile used can be an equivalent of 0.5 to 50 with respect to an equivalent of 1 of the compound (1-2-1) in accordance with the present invention.

Examples of the nucleophile include morpholine, 1,3-dimethylbarbituric acid and the like.

Examples of the palladium catalyst that can be used include tetrakis(triphenylphosphine)palladium(0) and the like. A use amount (equivalent) of the palladium catalyst can be an equivalent of 0.001 to 50 with respect to an equivalent of 1 of the compound (1-2-1) in accordance with the present invention.

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound (1-2-1) in accordance with the present invention can be synthesized in accordance with the method of production method 1.

### (Production Example 11)

A compound (1-2-4) in accordance with the present invention can be produced by, for example, the following production method.

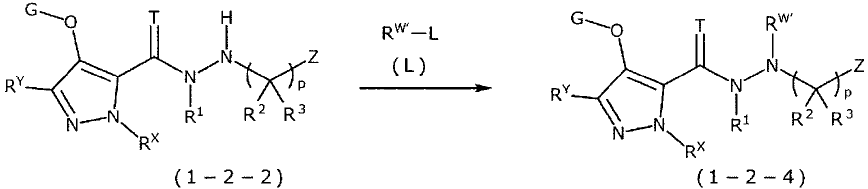

In the formula, R^{W'} represents C₁-C₆ alkyl, C₁-C₆ haloalkyl, C₁-C₆ alkyl substituted by R^{a}, C₂-C₆ alkenyl, C₁-C₆ alkylcarbonyl, C₂-C₆ alkenylcarbonyl, C₃-C₁₀ cycloalkylcarbonyl, C₁-C₆ alkoxycarbonyl, C₁-C₆ alkoxycarbonyl substituted by R^{a}, C₂-C₆ alkenyloxycarbonyl, di(C₁-C₆)alkylaminocarbonyl, di(C₁-C₆)alkylaminothiocarbonyl, C₁-C₆ alkylsulfonyl or -C(O)OR^{d}, and L, G, T, R^{X}, R^{Y}, R¹, R², R³, R^{a}, R^{d}, Z and p have the same meanings as above.

The compound (1-2-4) in accordance with the present invention can be produced by causing the compound (1-2-2) in accordance with the present invention and a compound (L) to react with each other in a solvent or without a solvent and optionally in the presence of one of or both of a base and an additive.

An equivalent of the compound (L) used can be an equivalent of 0.5 to 50 with respect to an equivalent of 1 of the compound (1-2-2) in accordance with the present invention.

Some of compounds (L) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-2-2) in accordance with the present invention.

Examples of the additive that can be used in the reaction include potassium iodide and the like. A use amount (equivalent) of the additive can be an equivalent of 0.005 to 100 with respect to an equivalent of 1 of the compound (1-2-2) in accordance with the present invention.

A reaction temperature and a reaction time are within the temperature range and the time range described in Production Example 1.

### (Production Example 12)

Among compounds (1) in accordance with the present invention, a compound (1-2-3) in accordance with the present invention in which W is -N(R^{W})- and R^{W} is - C(W¹)NHR^{Wa} can be produced by, for example, the following production method.

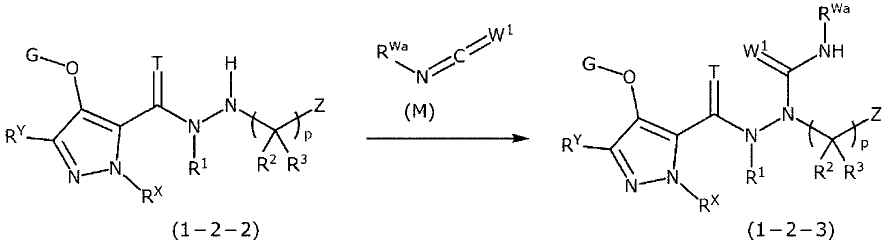

In the formula, W¹ represents an oxygen atom or a sulfur atom, R^{Wa} represents C₁-C₆ alkyl, and G, T, R^{X}, R^{Y}, R¹, R², R³, Z and p have the same meanings as above.

The compound (1-2-3) in accordance with the present invention can be produced by causing the compound (1-2-2) in accordance with the present invention and a compound (M) to react with each other in a solvent or without a solvent and optionally in the presence of a base.

An equivalent of the compound (M) used can be an equivalent of 0.5 to 50 with respect to an equivalent of 1 of the compound (1-2-2) in accordance with the present invention.

Some of compounds (M) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-2-2) in accordance with the present invention.

A reaction temperature and a reaction time are within the temperature range and the time range described in Production Example 1.

### (Production Example 13)

A compound (1-2-5) in accordance with the present invention can be produced by, for example, the following production method.

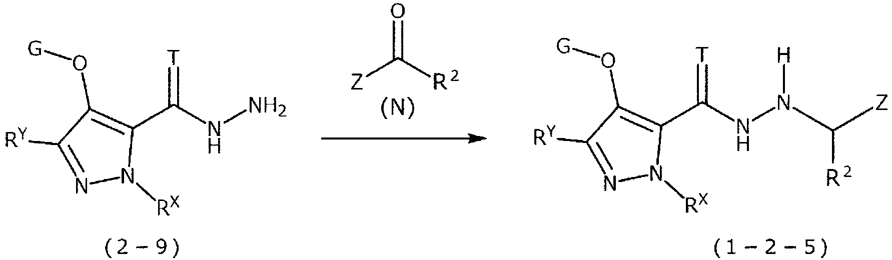

In the formula, G, T, R^{X}, R^{Y}, R² and Z have the same meanings as above.

The compound (1-2-5) in accordance with the present invention can be produced by causing a compound (2-9), a compound (N) and a reducing agent to react with each other in a solvent or without a solvent and in the presence of an acid.

An equivalent of the compound (N) used can be an equivalent of 0.5 to 50 with respect to an equivalent of 1 of the compound (2-9).

Some of compounds (N) are known compounds, and can be obtained as commercially available products. The other compounds can also be produced in accordance with a method for synthesizing a general known compound disclosed in a literature.

An equivalent of the reducing agent used can be an equivalent of 0.5 to 50 with respect to an equivalent of 1 of the compound (2-9).

Examples of the reducing agent include 2-picolineborane and the like.

In a case where a solvent is used, the solvent used only needs to be inert with respect to reaction. Examples of the solvent include the solvents exemplified in Production Example 1.

An equivalent of the acid used can be an equivalent of 0.1 to 100 with respect to an equivalent of 1 of the compound (2-9).

Examples of the acid include hydrochloric acid, acetic acid and the like.

A reaction temperature and a reaction time are within the temperature range and the time range described in Production Example 1.

### (Production Example 14)

A compound (1-2-7) in accordance with the present invention can be produced by, for example, the following production method.

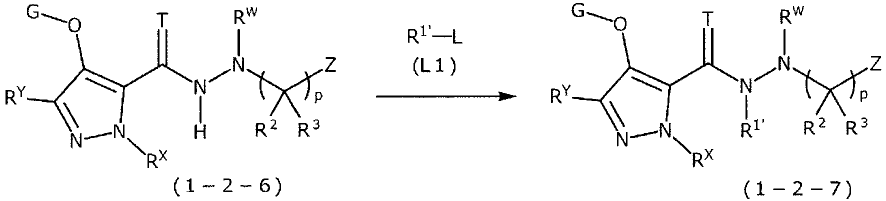

In the formula, R^{1'} represents C₁-C₆ alkyl or C₁-C₆ alkyl substituted by Rⁱ, and L, G, T, R^{X}, R^{Y}, Rⁱ, R², R², R³, R^{W}, Z and p have the same meanings as above.

The compound (1-2-7) in accordance with the present invention can be produced by causing a compound (1-2-6) in accordance with the present invention and a compound (L1) to react with each other in a solvent or without a solvent and in the presence of a base.

An equivalent of the compound (L1) used can be an equivalent of 0.5 to 50 with respect to an equivalent of 1 of the compound (1-2-6) in accordance with the present invention.

Some of compounds (L1) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (1-2-6) in accordance with the present invention.

A reaction temperature and a reaction time are within the temperature range and the time range described in Production Example 1.

The compound (1-2-6) in accordance with the present invention can be synthesized in accordance with the method of production method 1.

The compound (2) used in Production Example 1 can be produced in accordance with, for example, a production route (reaction path) indicated by the following reaction formula 1.

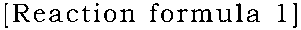

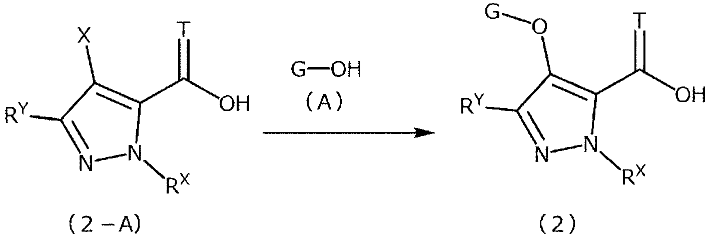

In the formula, X represents a halogen atom, and G, T, R^{X}, and R^{Y} have the same meanings as above.

The compound (2) can be obtained by causing a compound (2-A) and a compound (A) to react with each other in a solvent or without a solvent and in the presence of a base, and optionally in the presence of one of or both of a copper catalyst and an additive. Alternatively, the compound (2) can be obtained through a reaction in the presence of a base, and optionally in the presence of a palladium catalyst and a ligand.

Some of compounds (2-A) are known compounds, and can be obtained as commercially available products. The other compounds can also be produced in accordance with a method for synthesizing a general known compound disclosed in a literature.

An equivalent of the compound (A) used can be an equivalent of 0.5 to 50 with respect to an equivalent of 1 of the compound (2-A).

Some of compounds (A) are known compounds, and can be obtained as commercially available products. The other compounds can also be produced in accordance with a method for synthesizing a general known compound disclosed in a literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-A).

Examples of the copper catalyst that can be used in the reaction include copper(I) iodide, a copper(I) trifluoromethanesulfonate benzene complex, a copper(I) trifluoromethanesulfonate toluene complex and the like. An equivalent of the copper catalyst can be an equivalent of 0.001 to 50 with respect to an equivalent of 1 of the compound (2-A).

Examples of the additive that can be used in the reaction include N,N-dimethylglycine, 4-(dimethylamino)pyridine and the like. A use amount (equivalent) of the additive can be an equivalent of 0.001 to 100 with respect to an equivalent of 1 of the compound (2-A).

Examples of the palladium catalyst that can be used in the reaction include palladium(II) (n-cinnamyl) chloride (dimer), allylpalladium (II) chloride (dimer), tris(dibenzylideneacetone)dipalladium(0) and the like. A use amount (equivalent) of the palladium catalyst can be an equivalent of 0.001 to 50 with respect to an equivalent of 1 of the compound (2-A).

Examples of the ligand that can be used in the reaction include 2-di-tert-butylphosphino-2',4',6'-triisopropylbiphenyl (tert-butyl-XPhos), tetramethyl-di-tert-butylphosphino-2',4',6'-triisopropylbiphenyl (tetramethyl ditert-butyl-XPhos), 2-di-(tert-butyl)phosphino-2',4',6'-triisopropyl-3-methoxy-6-methylbiphenyl (RockPhos), 2-(dicyclohexylphosphino)-3,6-dimethoxy-2',4',6'-triisopropyl-1, 1'-biphenyl (BrettPhos), 2-dicyclohexylphosphino-2',6'-dimethoxybiphenyl (SPhos) and the like. A use amount (equivalent) of the ligand can be an equivalent of 0.001 to 50 with respect to an equivalent of 1 of the compound (2-A).

A reaction temperature and a reaction time are within the temperature range and the time range described in Production Example 1.

The compound (2-1) used in Production Example 1 can be produced in accordance with, for example, a production route (reaction path) indicated by the following reaction formula 2.

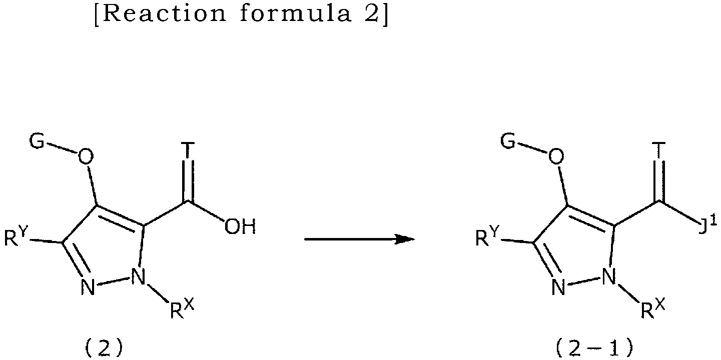

In the formula, J¹, G, T, R^{X}, and R^{Y} have the same meanings as above.

The compound (2-1) can be obtained by causing the compound (2) to react with a halogenating agent such as thionyl chloride, phosphorous pentachloride or oxalyl chloride in accordance with a known method disclosed in a literature, e.g., a method disclosed in Journal of Medicinal Chemistry [J.Med.Chem.], 1991, vol. 34, p. 1630 and the like. Alternatively, the compound (2-1) can be obtained through a reaction with an organic acid halide such as pivaloyl chloride or isobutyl chloroformate optionally in the presence of a base in accordance with a method disclosed in Tetrahedron Letters [Tetrahedron Lett.], 2003, vol. 44, p. 4819, Journal of Medicinal Chemistry [J.Med.Chem.], 1991, vol. 34, p. 222 or the like.

Among the compounds (2), a compound (2-2) in which R^{Y} is a hydrogen atom can be produced in accordance with, for example, a production route (reaction path) indicated by the following reaction formula 3.

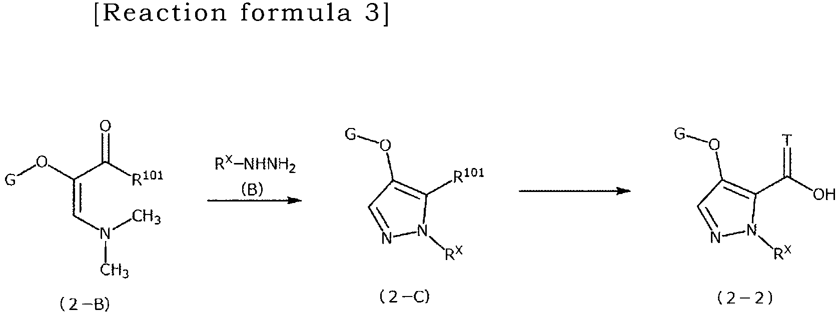

In the formula, R¹⁰¹ represents C₁-C₆ alkyl, and G and R^{X} have the same meanings as above.

Step 1: A compound (2-C) can be obtained by causing a compound (2-B) and a compound (B) or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of a base.

Some of compounds (B) are known compounds, and can be obtained as commercially available products. The other compounds can also be produced in accordance with a method for synthesizing a general known compound disclosed in a literature.

An equivalent of the compound (B) or a salt thereof used can be an equivalent of 0.5 to 50 with respect to an equivalent of 1 of the compound (2-B).

Some of compounds (2-B) are known compounds described in Bioorganic & Medicinal Chemistry Letters, 2015, Vol. 25, p. 4481. The other compounds can also be synthesized, as with the known compounds, in accordance with a method disclosed in the literature.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-B).

A reaction temperature and a reaction time are within the temperature range and the time range described in Production Example 1.

Step 2: The compound (2-2) can be obtained by causing the compound (2-C) and an oxidant to react with each other in a solvent or without a solvent.

Examples of the oxidant that can be used include potassium permanganate and the like. A use amount (equivalent) of the oxidant can be an equivalent of 0.1 to 100 with respect to an equivalent of 1 of the compound (2-C).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound (2-2) can be produced in accordance with, for example, a production route (reaction path) indicated by the following reaction formula 4.

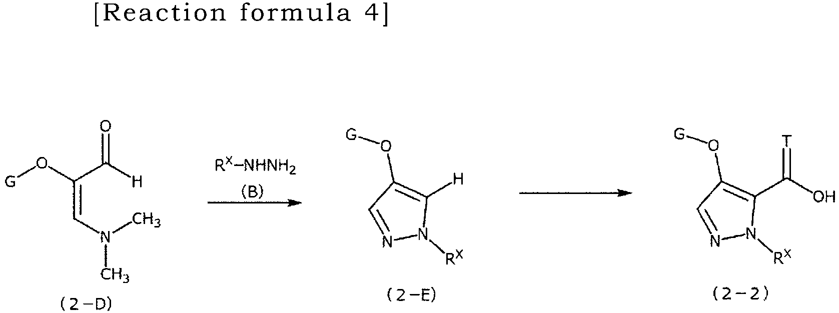

In the formula, G and R^{X} have the same meanings as above.

Step 1: A compound (2-E) can be obtained by causing a compound (2-D) to react in the same condition as step 1 in the reaction formula 3.

Some of compounds (2-D) are known compounds described in International Publication No. WO2003/016275. The other compounds can also be synthesized, as with the known compounds, in accordance with a method disclosed in the literature.

Step 2: The compound (2-2) can be obtained by causing the compound (2-E) and carbon dioxide to react with each other in a solvent or without a solvent and in the presence of a base.

Examples of the base that can be used include n-butyllithium and the like. A use amount (equivalent) of the base can be an equivalent of 0.1 to 100 with respect to an equivalent of 1 of the compound (2-E).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

Among the compounds (3) or a salt thereof (e.g., hydrochloride, hydrobromide, hydroiodide, sulfate, trifluoroacetate, oxalate, para-toluenesulfonate or the like) used in Production Example 1, a compound (3-1) in which R¹ is a hydrogen atom, W is -N(R^{W})-, and p is an integer of 1 can be produced in accordance with, for example, a production route (reaction path) indicated by the following reaction formula 5.

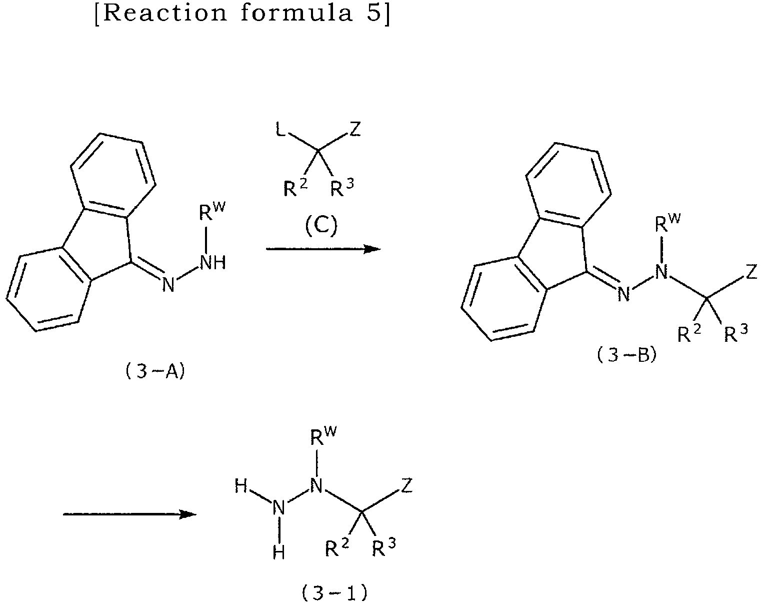

In the formula, L, R^{W}, R², R³ and Z have the same meanings as above.

Step 1: A compound (3-B) can be produced by causing a compound (3-A) and the compound (C) to react in a solvent or without a solvent and optionally in the presence of one of or both of a base and an additive.

Some of compounds (3-A) are known compounds described in International Publication No. WO2016/0129324. The other compounds can also be synthesized, as with the known compounds, in accordance with a method disclosed in the literature.

An equivalent of the compound (C) used can be an equivalent of 0.5 to 50 with respect to an equivalent of 1 of the compound (3-A).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (3-A).

Examples of the additive that can be used in the reaction include potassium iodide and the like. A use amount (equivalent) of the additive can be an equivalent of 0.005 to 100 with respect to an equivalent of 1 of the compound (3-A).

A reaction temperature and a reaction time are within the temperature range and the time range described in Production Example 1.

Step 2: The compound (3-1) or a salt thereof (e.g., hydrochloride, hydrobromide, hydroiodide, sulfate, trifluoroacetate, oxalate, para-toluenesulfonate or the like) can be produced by causing the compound (3-B) and hydroxylamine or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of one of or both of a base and an additive.

Examples of the base that can be used in the reaction include the bases exemplified in Production Example 1. A use amount (equivalent) of the base can be an equivalent of 0.1 to 100 with respect to an equivalent of 1 of the compound (3-B).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound (2-4) can be produced in accordance with, for example, a production route (reaction path) indicated by the following reaction formula 6.

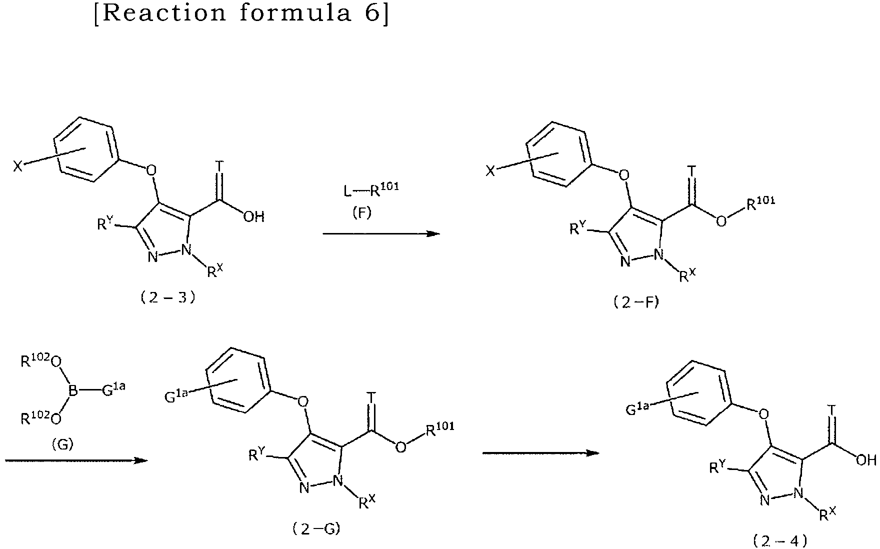

In the formula, G^{1a} represents C₁-C₆ alkyl or C₃-C₁₀ cycloalkyl, R¹⁰² represents a hydrogen atom or C₁-C₆ alkyl, T, X, R^{X}, R^{Y} and R¹⁰¹ have the same meanings as above.

Step 1: A compound (2-F) can be obtained by causing a compound (2-3) and a compound (F) to react with each other in a solvent or without a solvent and in the presence of a base.

Some of compounds (F) are known compounds, and can be obtained as commercially available products. The other compounds can also be produced in accordance with a method for synthesizing a general known compound disclosed in a literature.

An equivalent of the compound (F) used can be an equivalent of 0.5 to 50 with respect to an equivalent of 1 of the compound (2-3).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-3).

A reaction temperature and a reaction time are within the temperature range and the time range described in Production Example 1.

The compound (2-3) can be synthesized in accordance with the method of the reaction formula 3.

Step 2: A compound (2-G) can be obtained by causing the compound (2-F) and a compound (G) to react with each other in a solvent or without a solvent and in the presence of a base, and optionally in the presence of a palladium catalyst and a ligand.

Some of compounds (G) are known compounds, and can be obtained as commercially available products. The other compounds can also be produced in accordance with a method for synthesizing a general known compound disclosed in a literature.

An equivalent of the compound (G) used can be an equivalent of 0.5 to 50 with respect to an equivalent of 1 of the compound (2-F).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-F).

Examples of the palladium catalyst that can be used in the reaction include the palladium catalysts exemplified in the reaction formula 1. A use amount (equivalent) of the palladium catalyst can be an equivalent of 0.001 to 50 with respect to an equivalent of 1 of the compound (2-F).

Examples of the ligand that can be used in the reaction include the ligands exemplified in the reaction formula 1. A use amount (equivalent) of the ligand can be an equivalent of 0.001 to 50 with respect to an equivalent of 1 of the compound (2-F).

A reaction temperature and a reaction time are within the temperature range and the time range described in Production Example 1.

Step 3: The compound (2-4) can be obtained by causing the compound (2-G) to be reacted in a solvent or without a solvent and optionally in the presence of a base.

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-G).

A reaction temperature and a reaction time are within the temperature range and the time range described in Production Example 1.

The compound (2-5) used in Production Example 2 can be produced in accordance with, for example, a production route (reaction path) indicated by the following reaction formula 7.

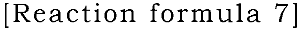

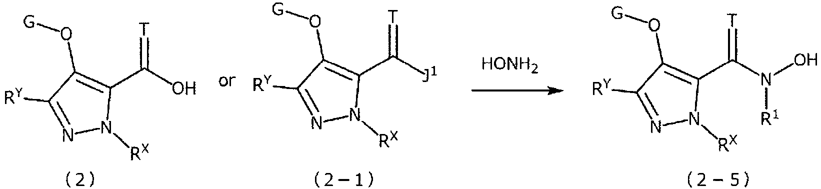

In the formula, J¹, G, T, R¹, R^{X} and R^{Y} have the same meanings as above.

The compound (2-5) can be produced by causing the compound (2), hydroxylamine or a salt thereof (e.g., hydrochloride, hydrobromide, hydroiodide, sulfate, trifluoroacetate, oxalate, para-toluenesulfonate or the like) and a dehydration condensation agent to react with each other in a solvent or without a solvent and optionally in the presence of one of or both of a base and an additive.

Alternatively, the compound (2-5) can be produced by causing the compound (2-1) and hydroxylamine or a salt thereof (e.g., hydrochloride, hydrobromide, hydroiodide, sulfate, trifluoroacetate, oxalate, para-toluenesulfonate or the like) to react with each other in a solvent or without a solvent and optionally in the presence of one of or both of a base and an additive.

An equivalent of the hydroxylamine or a salt thereof used can be an equivalent of 0.5 to 50 with respect to an equivalent of 1 of the compound (2) or compound (2-1).

Examples of the dehydration condensation agent that can be used in the reaction include the dehydration condensation agents exemplified in Production Example 1. A use amount (equivalent) of the dehydration condensation agent can be an equivalent of 0.1 to 100 with respect to an equivalent of 1 of the compound (2).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2) or compound (2-1).

Examples of the additive that can be used in the reaction include the additives exemplified in Production Example 1. A use amount (equivalent) of the additive can be an equivalent of 0.005 to 100 with respect to an equivalent of 1 of the compound (2) or compound (2-1).

A reaction temperature and a reaction time are within the temperature range and the time range described in Production Example 1.

The compound (2-9) can be produced in accordance with, for example, a production route (reaction path) indicated by the following reaction formula 8.

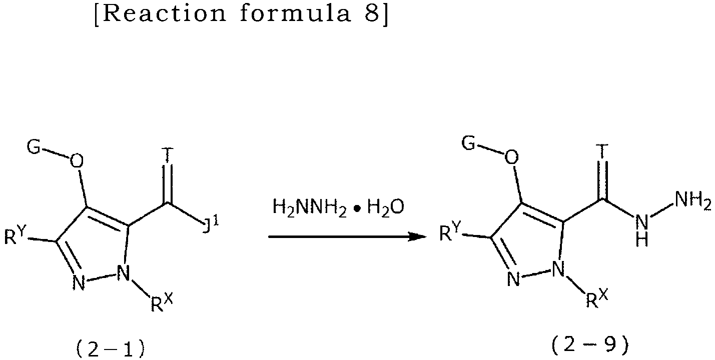

In the formula, J¹, G, T, R^{X} and R^{Y} have the same meanings as above.

The compound (2-9) can be obtained by causing the compound (2-1) and a hydrazine monohydrate to react with each other in a solvent or without a solvent and in the presence of a base.

An equivalent of the hydrazine monohydrate used can be an equivalent of 0.5 to 50 with respect to an equivalent of 1 of the compound (2-1).

In a case where a solvent is used, the solvent used only needs to be inert with respect to reaction. Examples of the solvent include the solvents exemplified in Production Example 1.

Examples of the base that can be used in the reaction include the bases exemplified in Production Example 1. A use amount (equivalent) of the base can be an equivalent of 0.1 to 100 with respect to an equivalent of 1 of the compound (2-1).

A reaction temperature and a reaction time are within the temperature range and the time range described in Production Example 1.

A compound (2-6) can be produced in accordance with, for example, a production route (reaction path) indicated by the following reaction formula 9.

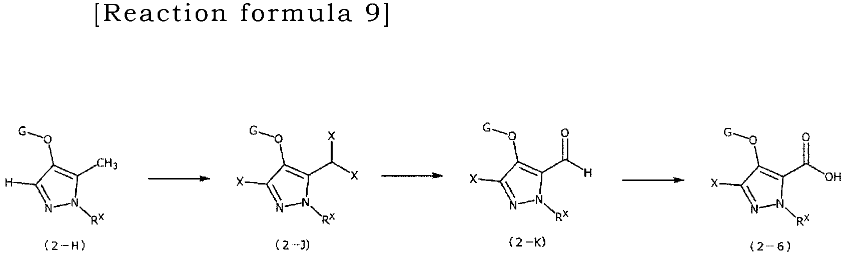

In the formula, G, X and R^{X} have the same meanings as above.

Step 1: The compound (2-J) can be obtained by causing a compound (2-H) and a halogenating agent to react with each other in a solvent or without a solvent.

Examples of the halogenating agent that can be used include chlorine, N-chlorosuccinimide, 1,3-dichloro-5,5-dimethylhydantoin, bromine, N-bromosuccinimide, N-iodosuccinimide and the like. A use amount (equivalent) of the halogenating agent can be an equivalent of 0.1 to 100 with respect to an equivalent of 1 of the compound (2-H).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound (2-H) can be synthesized in accordance with the method of the reaction formula 3.

Step 2: A compound (2-K) can be obtained by hydrolyzing the compound (2-J) in accordance with a known method disclosed in a literature, e.g., a method disclosed in Japanese Patent Application Publication, No. 2014/214118, Journal of Heterocyclic Chemistry, 1992, vol. 29, p. 691 or the like in the presence of an acid such as hydrochloric acid or sulfuric acid or a base such as calcium carbonate or sodium acetate.

Step 3: The compound (2-6) can be obtained by oxidizing the compound (2-K) in accordance with a known method disclosed in a literature, e.g., a method disclosed in International Publication No. WO2008/028688.

The compound (2-7) used in Production Example 6 can be produced in accordance with, for example, a production route (reaction path) indicated by the following reaction formula 10.

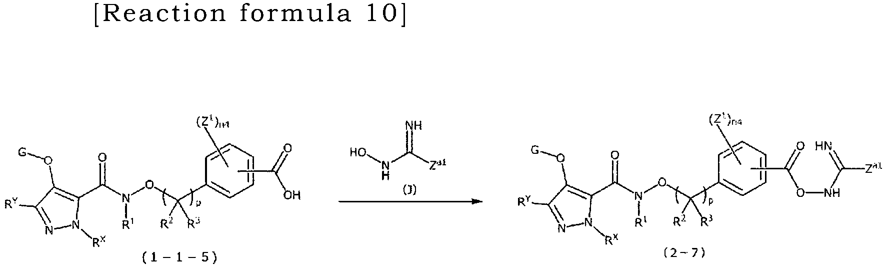

In the formula, G, Z¹, Z^{a1}, n4, p, R¹, R², R³, R^{X} and R^{Y} have the same meanings as above.

The compound (2-7) can be obtained by causing the compound (1-1-5) in accordance with the present invention and a compound (J) to react with each other in accordance with a known method disclosed in a literature, e.g., a method disclosed in International Publication No. WO2008/156721.

Some of compounds (J) are known compounds, and can be obtained as commercially available products. The other compounds can also be produced in accordance with a method for synthesizing a general known compound disclosed in a literature.

The compound (2-8) used in Production Example 9 can be produced in accordance with, for example, a production route (reaction path) indicated by the following reaction formula 11.

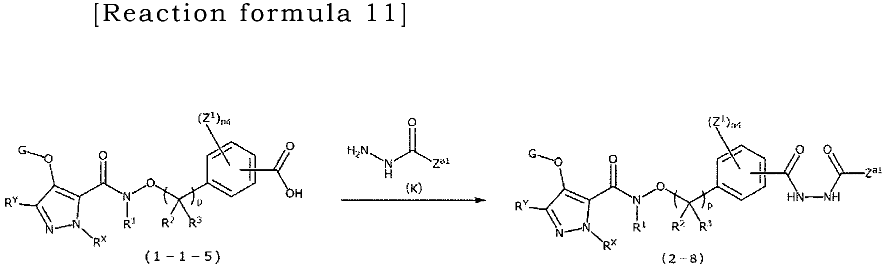

In the formula, G, Z¹, Zal, n4, p, R¹, R², R³, R^{X} and R^{Y} have the same meanings as above.

The compound (2-8) can be obtained by causing the compound (1-1-5) in accordance with the present invention and a compound (K) to react with each other in accordance with a known method disclosed in a literature, e.g., a method disclosed in International Publication No. WO2008/016123.

Some of compounds (K) are known compounds, and can be obtained as commercially available products. The other compounds can also be produced in accordance with a method for synthesizing a general known compound disclosed in a literature.

The compound (2) can be produced in accordance with, for example, a production route (reaction path) indicated by the following reaction formula 11.

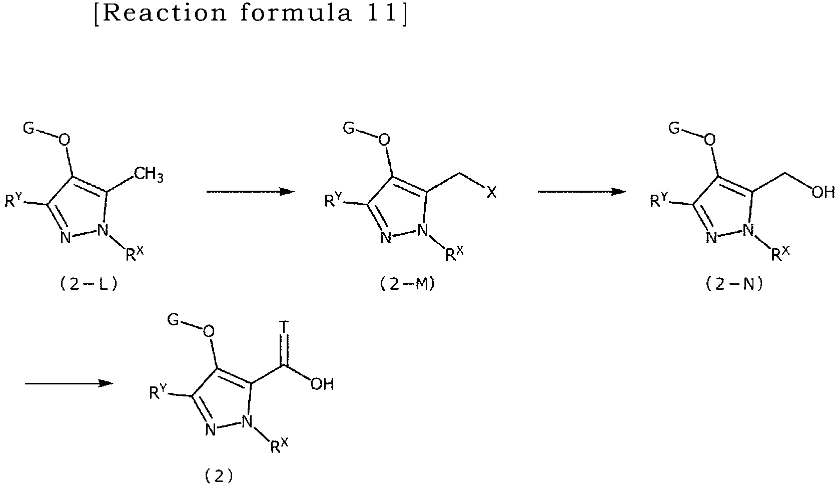

In the formula, G, T, R^{X}, R^{Y} and X have the same meanings as above.

Step 1: A compound (2-M) can be produced by causing a compound (2-L) and a halogenating agent to react with each other in a solvent or without a solvent.

Examples of the halogenating agent that can be used include the halogenating agents exemplified in step 1 in the reaction formula 9. A use amount (equivalent) of the halogenating agent can be an equivalent of 0.1 to 100 with respect to an equivalent of 1 of the compound (2-L).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound (2-L) can be synthesized in accordance with the method of reaction formula 3.

Step 2: A compound (2-N) can be obtained by hydrolyzing the compound (2-M) in accordance with a known method disclosed in a literature, e.g., a method disclosed in International Publication No. WO2006/067443, International Publication No. WO2023/033142 or the like in the presence of water and a base such as sodium hydroxide, potassium hydroxide, or lithium hydroxide.

Step 3: The compound (2) can be obtained by causing the compound (2-N) and an oxidant to react with each other in a solvent or without a solvent and in the presence of a catalyst, and optionally in the presence of one of or both of a buffer and an additive.

Examples of the oxidant that can be used in the reaction include sodium chlorite, a sodium chlorite aqueous solution, sodium hypochlorite, a sodium hypochlorite aqueous solution, sodium hypochlorite pentahydrate and the like. A use amount of the oxidant can be an equivalent of 0.001 to 100 with respect to an equivalent of 1 of the compound (2-N).

Examples of the catalyst that can be used in the reaction include 2,2,6,6-tetramethylpiperidine-1-oxyl (TEMPO), 2-azaadamantane-N-oxyl (AZADO) and the like. A use amount of the catalyst can be an equivalent of 0.001 to 100 with respect to an equivalent of 1 of the compound (2-N).

Examples of the buffer that can be used in the reaction include sodium dihydrogen phosphate, sodium dihydrogen phosphate dihydrate, acetic acid and the like. A use amount of the buffer can be an equivalent of 0.5 to 100 with respect to an equivalent of 1 of the compound (2-N).

Examples of the additive that can be used in the reaction include potassium bromide, tetrabutylammonium bromide and the like. A use amount of the catalyst can be an equivalent of 0.001 to 100 with respect to an equivalent of 1 of the compound (2-N).

In a case where a solvent is used, the solvent used only needs to be inert with respect to reaction. Examples of the solvent include the solvents exemplified in Production Example 1.

A reaction temperature and a reaction time are within the temperature range and the time range described in Production Example 1.

The compound in accordance with the present invention can be used, typically as fungicides for agriculture and horticulture, against a variety of diseases caused by Plasmodiophoromycetes, Oomycetes, Zygomycetes, Ascomycetes, Basidiomycetes, Deuteromycetes, bacteria, and viruses.

The term "pathogens" means a microorganism which serves as a pathogen of a plant disease. Specific examples include, but not limited to, the following microorganisms.

Fungi of Ascomycota such as *Taphrina* spp. (e.g., *Taphrina deformans, T. pruni* and the like), *Pneumocystis* spp., *Geotrichum* spp., *Candida* spp. (e.g., *Candida albicans, C. sorbosa* and the like), *Pichia* spp. (e.g., *Pichia kluyveri* and the like), *Capnodium* spp., *Fumago* spp., *Hypocapnodium* spp., *Cercospora* spp. (e.g., *Cercospora apii, C. asparagi, C. beticola, C. capsici, C. carotae, C. kaki, C. kikuchii, C. zonata* and the like), *Cercosporidium* spp., *Cladosporium* spp. (e.g., *Cladosporium colocasiae, C. cucumerinum, C. variabile* and the like), *Davidiella* spp., *Didymosporium* spp., *Heterosporium* spp. (e.g., *Heterosporium allii* and the like), *Mycosphaerella* spp. (e.g., *Mycosphaerella arachidis, M. berkeleyi, M. cerasella, M. fijiensis, M. fragariae, M. graminicola, M*. *nawae, M. pinodes, M. pomi, M. zingiberis* and the like), *Mycovellosiella* spp. (e.g., *Mycovellosiella fulva, M. nattrassii* and the like), *Paracercospora* spp. (e.g., *Paracercospora egenula* and the like), *Phaeoisariopsis* spp., *Phaeoramularia* spp., *Pseudocercospora* spp. (e.g., *Pseudocercospora abelmoschi, P. fuligena, P. vitis* and the like), *Pseudocercosporella* spp. (e.g., *Pseudocercosporella capsellae* and the like), *Ramichloridium* spp., *Ramularia* spp., *Septogloeum* spp., *Septoria* spp. (e.g., *Septoria albopunctata, S. apiicola, S. chrysanthemella, S*. *helianthi, S. obesa* and the like), *Sphaerulina* spp., *Aureobasidium* spp., *Kabatiella* spp., *Plowrightia* spp., *Stigmina* spp., *Elsinoe* spp. (e.g., *Elsinoe ampelina, E. araliae, E. fawcettii* and the like), *Sphaceloma* spp. (e.g., *Sphaceloma caricae* and the like), *Ascochyta* spp. (e.g., *Ascochyta pisi* and the like), *Corynespora* spp. (e.g., *Corynespora cassiicola* and the like), *Leptosphaeria* spp. (e.g., *Leptosphaeria coniothyrium, L. maculans* and the like), *Saccharicola* spp., *Phaeosphaeria* spp. (e.g., *Phaeosphaeria nodorum* and the like), *Ophiosphaerella* spp., *Setophoma* spp., *Helminthosporium* spp., *Alternaria* spp. (e.g., *Alternaria alternata, A. brassicae, A. brassicicola, A. citri, A. dauci, A. helianthi, A. japonica, A. kikuchiana, A. mali, A. panax, A. porri, A. radicina, A. solani* and the like), *Bipolaris* spp. (e.g., *Bipolaris sorghicola* and the like), *Cochliobolus* spp. (e.g., *Cochliobolus heterostrophus, C. lunatus, C*. *miyabeanus* and the like), *Curvularia* spp. (e.g., *Curvularia geniculata, C. verruculosa* and the like), *Drechslera* spp., *Pleospora* spp. (e.g., *Pleospora herbarum* and the like), *Pyrenophora* spp. (e.g., *Pyrenophora graminea, P. teres* and the like), *Setosphaeria* spp. (e.g., *Setosphaeria turcica* and the like), *Stemphylium* spp. (e.g., *Stemphylium botryosum, S. lycopersici, S. solani, S. vesicarium* and the like), *Fusicladium* spp., *Venturia* spp. (e.g., *Venturia carpophila, V. Inaequalis, V. nashicola, V. pirina* and the like), *Didymella* spp. (e.g., *Didymella bryoniae, D. fabae* and the like), *Hendersonia* spp., *Phoma* spp. (e.g., *Phoma erratica* var. *mikan, P. exigua* var. *exigua, P. wasabiae* and the like), *Pyrenochaeta* spp. (e.g., *Pyrenochaeta lycopersici* and the like), *Stagonospora* spp. (e.g., *Stagonospora sacchari* and the like), *Botryosphaeria* spp. (e.g., *Botryosphaeria berengeriana* f. sp. *piricola, B. dothidea* and the like), *Dothiorella* spp., *Fusicoccum* spp., *Guignardia* spp., *Lasiodiplodia* spp. (e.g., *Lasiodiplodia theobromae* and the like), *Macrophoma* spp., *Macrophomina* spp., *Neofusicoccum* spp., *Phyllosticta* spp. (e.g., *Phyllosticta zingiberis* and the like), *Schizothyrium* spp. (e.g., *Schizothyrium pomi* and the like), *Acrospermum* spp., *Leptosphaerulina* spp., *Aspergillus* spp., *Penicillium* spp. (e.g., *Penicillium digitatum, P. italicum, P. sclerotigenum* and the like), *Microsporum* spp., *Trichophyton* spp. (e.g., *Trichophyton mentagrophytes, T. rubrum* and the like), *Histoplasma* spp., *Blumeria* spp. (e.g., *Blumeria graminis* f. sp. *hordei, B. g. f.* sp. *tritici* and the like), *Erysiphe* spp. (e.g., *Erysiphe betae, E. cichoracearum,* E. c. var. *cichoracearum, E. heraclei, E. pisi* and the like), *Golovinomyces* spp. (e.g., *Golovinomyces cichoracearum* var. *latisporus* and the like), *Leveillula* spp. (e.g., *Leveillula taurica* and the like), *Microsphaera* spp., *Oidium* spp. (e.g., *Oidium neolycopersici* and the like), *Phyllactinia* spp. (e.g., *Phyllactinia kakicola, P. mali, P. moricola* and the like), *Podosphaera* spp. (e.g., *Podosphaera fusca, P. leucotricha, P. pannosa, P. tridactyla* var. *tridactyla, P. xanthii* and the like), *Sphaerotheca* spp. (e.g., *Sphaerotheca aphanis* var. *aphanis, S. fuliginea* and the like), *Uncinula* spp. (e.g., *Uncinula necator,* U. n. var. *necator* and the like), *Uncinuliella* spp. (e.g., *Uncinuliella simulans* var. *simulans,* U. s. var. *tandae* and the like), *Blumeriella* spp. (e.g., *Blumeriella jaapii* and the like), *Cylindrosporium* spp., *Diplocarpon* spp. (e.g., *Diplocarpon mali, D*. *mespili, D. rosae* and the like), *Gloeosporium* spp. (e.g., *Gloeosporium minus* and the like), *Marssonina* spp., *Tapesia* spp. (e.g., *Tapesia acuformis, T. yallundae* and the like), *Lachnum* spp., *Scleromitrula* spp., *Botryotinia* spp. (e.g., *Botryotinia fuckeliana* and the like), *Botrytis* spp. (e.g., *Botrytis allii, B. byssoidea, B. cinerea, B. elliptica, B. fabae, B. squamosa* and the like), *Ciborinia* spp., *Grovesinia* spp., *Monilia mumecola, Monilinia* spp. (e.g., *Monilinia fructicola, M. fructigena, M. laxa, M. mali, M*. *vaccinii-corymbosi* and the like), *Sclerotinia* spp. (e.g., *Sclerotinia borealis, S. homoeocarpa, S*. *minor, S*. *sclerotiorum* and the like), *Valdensia* spp. (e.g., *Valdensia heterodoxa* and the like), *Claviceps* spp. (e.g., *Claviceps sorghi, C. sorghicola* and the like), *Epichloe* spp., *Ephelis japonica, Villosiclava virens, Hypomyces* spp. (e.g., *Hypomyces solani* f. sp. *mori, H.* s. f. sp. *pisi* and the like), *Trichoderma* spp. (e.g., *Trichoderma viride* and the like), *Calonectria* spp. (e.g., *Calonectria ilicicola* and the like), *Candelospora* spp., *Cylindrocarpon* spp., *Cylindrocladium* spp., *Fusarium* spp. (e.g., *Fusarium arthrosporioides, F. crookwellense, F. culmorum, F. cuneirostrum, F. oxysporum,* F. o. f. sp. *adzukicola,* F. o. f. sp. *allii,* F. o. f. sp. *asparagi,* F. o. f. sp. *batatas,* F. o. f. sp. *cepae,* F. o. f. sp. *colocasiae,* F. o. f. sp. *conglutinans,* F. o. f. sp. *cubense,* F. o. f. sp. *cucumerinum,* F. o. f. sp. *fabae,* F. o. f. sp. *fragariae,* F. o. f. sp. *lactucae,* F. o. f. sp. *lagenariae,* F. o. f. sp. *lycopersici,* F. o. f. sp. *melongenae,* F. o. f. sp. *melonis,* F. o. f. sp. *nelumbinicola,* F. o. f. sp. *niveum,* F. o. f. sp. *radicislycopersici,* F. o. f. sp. *raphani,* F. o. f. sp. *spinaciae, F. sporotrichioides, F. solani,* F. s. f. sp. *cucurbitae,* F. s. f. sp. *eumartii,* F. s. f. sp. *glycines,* F. s. f. sp. *pisi,* F. s. f. sp. *Radicicola, F. virguliforme* and the like), *Gibberella* spp. (e.g., *Gibberella avenacea, G. baccata, G. fujikuroi, G. zeae* and the like), *Haematonectria* spp., *Nectria* spp., *Ophionectria* spp., *Caldariomyces* spp., *Myrothecium* spp., *Trichothecium* spp., *Verticillium* spp. (e.g., *Verticillium albo-atrum, V. dahliae, V. longisporum* and the like), *Ceratocystis* spp. (e.g., *Ceratocystis ficicola, C. fimbriata* and the like), *Thielaviopsis* spp. (e.g., *Thielaviopsis basicola* and the like), *Adisciso* spp., *Monochaetia* spp., *Pestalotia* spp. (e.g., *Pestalotia eriobotrifolia* and the like), *Pestalotiopsis* spp. (e.g., *Pestalotiopsis funerea, P. longiseta, P. neglecta, P. theae* and the like), *Physalospora* spp., *Nemania* spp., *Nodulisporium* spp., *Rosellinia* spp. (e.g., *Rosellinia necatrix* and the like), *Monographella* spp. (e.g., *Monographella nivalis* and the like), *Ophiostoma* spp., *Cryphonectria* spp. (e.g., *Cryphonectria parasitica* and the like), *Diaporthe* spp. (e.g., *Diaporthe citri,* D. *kyushuensis,* D. *nomurai, D*. *tanakae* and the like), *Diaporthopsis* spp., *Phomopsis* spp. (e.g., *Phomopsis asparagi, P. fukushii, P. obscurans, P. vexans* and the like), *Cryptosporella* spp., *Discula* spp. (e.g., *Discula theaesinensis* and the like), *Gnomonia* spp., *Coniella* spp., *Coryneum* spp., *Greeneria* spp., *Melanconis* spp., *Cytospora* spp., *Leucostoma* spp., *Valsa* spp. (e.g., *Valsa ceratosperma* and the like), *Tubakia* spp., *Monosporascus* spp., *Clasterosporium* spp., *Gaeumannomyces* spp. (e.g., *Gaeumannomyces graminis* and the like), *Magnaporthe* spp. (e.g., *Magnaporthe grisea* and the like), *Pyricularia* spp. (e.g., *Pyricularia zingiberis* and the like), *Monilochaetes infuscans, Colletotrichum* spp. (e.g., *Colletotrichum acutatum, C. capsici, C. cereale, C. destructivum, C. fragariae, C*. *lindemuthianum, C*. *nigrum, C*. *orbiculare, C. spinaciae* and the like), *Glomerella* spp. (e.g., *Glomerella cingulata* and the like), *Khuskia oryzae, Phyllachora* spp. (e.g., *Phyllachora pomigena* and the like), *Ellisembia* spp., *Briosia* spp., *Cephalosporium* spp. (e.g., *Cephalosporium gramineum* and the like), *Epicoccum* spp., *Gloeocercospora sorghi, Mycocentrospora* spp., *Peltaster* spp. (e.g., *Peltaster fructicola* and the like), *Phaeocytostroma* spp., *Phialophora* spp. (e.g., *Phialophora gregata* and the like), *Pseudophloeosporella dioscoreae, Pseudoseptoria* spp., *Rhynchosporium* spp. (e.g., *Rhynchosporium secalis* and the like), *Sarocladium* spp., *Coleophoma* spp., *Helicoceras oryzae.* Fungi of Basidiomycota such as *Septobasidium* spp. (e.g., *Septobasidium bogoriense,* S. *tanakae* and the like), *Helicobasidium* spp. (e.g., *Helicobasidium longisporum* and the like), *Coleosporium* spp. (e.g., *Coleosporium plectranthi* and the like), *Cronartium* spp., *Phakopsora* spp. (e.g., *Phakopsora artemisiae, P. nishidana, P. pachyrhizi* and the like), *Physopella* spp. (e.g., *Physopella ampelopsidis* and the like), *Kuehneola* spp. (e.g., *Kuehneola japonica* and the like), *Phragmidium* spp. (e.g., *Phragmidium fusiforme, P. mucronatum, P. rosae-multiflorae* and the like), *Gymnosporangium* spp. (e.g., *Gymnosporangium asiaticum, G*. *yamadae* and the like), *Puccinia* spp. (e.g., *Puccinia allii, P. brachypodii* var. *poae-nemoralis, P. coronata,* P. c. var. *coronata, P. cynodontis, P. graminis,* P. g. subsp. *graminicola, P. hordei, P. horiana, P. kuehnii, P. melanocephala, P. recondita, P. striiformis* var. *striiformis, P. tanaceti* var. *tanaceti, P. tokyensis, P. zoysiae* and the like), *Uromyces* spp. (e.g., *Uromyces phaseoli* var. *azukicola,* U. p. var. *phaseoli, Uromyces viciae-fabae* var. *viciae-fabae* and the like), *Naohidemyces vaccinii, Nyssopsora* spp., *Leucotelium* spp., *Tranzschelia* spp. (e.g., *Tranzschelia discolor* and the like), *Aecidium* spp., *Blastospora* spp. (e.g., *Blastospora smilacis* and the like), *Uredo* spp., *Sphacelotheca* spp., *Urocystis* spp., *Sporisorium* spp. (e.g., *Sporisorium scitamineum* and the like), *Ustilago* spp. (e.g., *Ustilago maydis, U. nuda* and the like), *Entyloma* spp., *Exobasidium* spp. (e.g., *Exobasidium reticulatum, E. vexans* and the like), *Microstroma* spp., *Tilletia* spp. (e.g., *Tilletia caries, T. controversa, T. laevis* and the like), *Itersonilia* spp. (e.g., *Itersonilia perplexans* and the like), *Cryptococcus* spp., *Bovista* spp. (e.g., *Bovista dermoxantha* and the like), *Lycoperdon* spp. (e.g., *Lycoperdon curtisii, L. perlatum* and the like), *Conocybe* spp. (e.g., *Conocybe apala* and the like), *Marasmius* spp. (e.g., *Marasmius oreades* and the like), *Armillaria* spp., *Helotium* spp., *Lepista* spp. (e.g., *Lepista subnuda* and the like), *Sclerotium* spp. (e.g., *Sclerotium cepivorum* and the like), *Typhula* spp. (e.g., *Typhula incarnata, T. ishikariensis* var. *ishikariensis* and the like), *Athelia* spp. (e.g., *Athelia rolfsii* and the like), *Ceratobasidium* spp. (e.g., *Ceratobasidium cornigerum* and the like), *Ceratorhiza* spp., *Rhizoctonia* spp. (e.g., *Rhizoctonia solani* and the like), *Thanatephorus* spp. (e.g., *Thanatephorus cucumeris* and the like), *Laetisaria* spp., *Waitea* spp., *Fomitiporia* spp., *Ganoderma* spp., *Chondrostereum purpureum, Phanerochaete* spp. Fungi of Chitridiomycota such as *Olpidium* spp. Fungi of Blastocladiomycota such as *Physoderma* spp. Fungi of Mucoromycotina such as *Choanephora* spp., *Choanephoroidea cucurbitae, Mucor* spp. (e.g., *Mucor fragilis* and the like), *Rhizopus* spp. (e.g., *Rhizopus arrhizus, R. chinensis, R. oryzae, R. stolonifer* var. *stolonifer* and the like). Protists of Cercozoa such as *Plasmodiophora* spp. (e.g., *Plasmodiophora brassicae* and the like), *Spongospora subterranea* f. sp. *Subterranea.* Oomycetes of Heterokontophyta such as *Aphanomyces* spp. (e.g., *Aphanomyces cochlioides, A. raphani* and the like), *Albugo* spp. (e.g., *Albugo macrospora, A. wasabiae* and the like), *Bremia* spp. (e.g., *Bremia lactucae* and the like), *Hyaloperonospora* spp., *Peronosclerospora* spp., *Peronospora* spp. (e.g., *Peronospora alliariae-wasabi, P. chrysanthemicoronarii, P. destructor, P. farinosa* f. sp. *spinaciae, P. manshurica, P. parasitica, P. sparsa* and the like), *Plasmopara* spp. (e.g., *Plasmopara halstedii, P. nivea, P. viticola* and the like), *Pseudoperonospora* spp. (e.g., *Pseudoperonospora cubensis* and the like), *Sclerophthora* spp., *Phytophthora* spp. (e.g., *Phytophthora cactorum, P. capsici, P. citricola, P. citrophthora, P. cryptogea, P. fragariae, P. infestans, P. melonis, P. nicotianae, P. palmivora, P. porri, P. sojae, P. syringae, P. vignae* f. sp. *adzukicola* and the like), *Pythium* spp. (e.g., *Pythium afertile, P. aphanidermatum, P. apleroticum, P. aristosporum, P. arrhenomanes, P. buismaniae, P. debaryanum, P. graminicola, P. horinouchiense, P. irregulare, P. iwayamai, P. myriotylum, P. okanoganense, P. paddicum, P. paroecandrum, P. periplocum, P. spinosum, P. sulcatum, P. sylvaticum, P. ultimum* var. *ultimum, P. vanterpoolii, P. vexans, P. volutum* and the like). Gram-positive fungi of Actinobacteria such as *Clavibacter* spp. (e.g., *Clavibacter michiganensis* subsp. *michiganensis* and the like), *Curtobacterium* spp., *Leifsonia* spp. (e.g., *Leifsonia xyli* subsp. *xyli* and the like), *Streptomyces* spp. (e.g., *Streptomyces ipomoeae* and the like). Gram-positive fungi of Firmicutes such as *Clostridium* sp. Gram-positive fungi of Tenericutes such as *Phytoplasma.* Gram-negative fungi of Proteobacteria such as *Rhizobium* spp. (e.g., *Rhizobium radiobacter* and the like), *Acetobacter* spp., *Burkholderia* spp. (e.g., *Burkholderia andropogonis, B. cepacia, B. gladioli, B. glumae, B. plantarii* and the like), *Acidovorax* spp. (e.g., *Acidovorax avenae* subsp. *avenae,* A. a. subsp. *citrulli, A. konjaci* and the like), *Herbaspirillum* spp., *Ralstonia* spp. (e.g., *Ralstonia solanacearum* and the like), *Xanthomonas* spp. (e.g., *Xanthomonas albilineans, X. arboricola* pv. *pruni, X. axonopodis* pv. *vitians, X. campestris* pv. *campestris,* X. c. pv. *cucurbitae,* X. c. pv. *glycines,* X. c. pv. *mangiferaeindicae,* X. c. pv. *nigromaculans,* X. c. pv. *vesicatoria, X. citri* subsp. *citri, X. oryzae* pv. *oryzae* and the like), *Pseudomonas* spp. (e.g., *Pseudomonas cichorii, P. fluorescens, P. marginalis,* P. m. pv. *marginalis, P. savastanoi* pv. *glycinea, P. syringae,* P. s. pv. *actinidiae,* P. s. pv. *eriobotryae,* P. s. pv. *helianthi,* P. s. pv. *lachrymans,* P. s. pv. *maculicola,* P. s. pv. *mori,* P. s. pv. *morsprunorum,* P. s. pv. *spinaciae,* P. s. pv. *syringae,* P. s. pv. *theae, P. viridiflava* and the like), *Rhizobacter* spp., *Brenneria* spp. (e.g., *Brenneria nigrifluens* and the like), *Dickeya* spp. (e.g., *Dickeya dianthicola, D. zeae* and the like), *Erwinia* spp. (e.g., *Erwinia amylovora, E. rhapontici* and the like), *Pantoea* spp., *Pectobacterium* spp. (e.g., *Pectobacterium atrosepticum, P. carotovorum, P. wasabiae* and the like).

Specific examples of plant diseases caused by infection and growth of these pathogens include, but not limited to, the following plant diseases.

Leaf curl *(Taphrina deformans),* Plum pockets *(Taphrina pruni),* Leaf spot *(Cercospora asparagi),* Cercospora leaf spot *(Cercospora beticola),* Frogeye leaf spot *(Cercospora capsici),* Angular leaf spot *(Cercospora kaki),* Purple stain *(Cercospora kikuchii),* Brown Leaf spot *(Mycosphaerella arachidis),* Cylindrosporium leaf spot *(Mycosphaerella cerasella, Blumeriella jaapii),* Black sigatoka *(Mycosphaerella fijiensis*), Yellow sigatoka (*Mycosphaerell musicola*), Speckled leaf blotch (*Mycosphaerella graminicola*), Circular leaf spot (*Mycosphaerella nawae*), Mycosphaerella blight (*Mycosphaerella pinodes*), Leaf spot (*Mycosphaerella zingiberis),* Leaf mold (*Mycovellosiella fulva*), Leaf mold (*Mycovellosiella nattrassii*), Cercospora leaf mold (*Pseudocercospora fuligena*)*,* Isariopsis leaf spot (*Pseudocercospora vitis*)*,* Leaf spot (*Pseudocercosporella capsellae*)*,* Leaf spot (*Septoria chrysanthemella),* Leaf blight (*Septoria obesa*)*,* Anthracnose (*Elsinoe ampelina*)*,* Spot anthracnose (*Elsinoe araliae*)*,* Scab (*Elsinoe fawcettii*)*,* Leaf spot (*Ascochyta pisi*)*,* Corynespora leaf spot (*Corynespora cassiicola*)*,* Stem canker (*Leptosphaeria coniothyrium),* Glume blotch (*Leptosphaeria nodorum*)*,* Leaf spot (*Alternaria alternata*)*,* Alternaria leaf spot (*Alternaria brassicae*)*,* Leaf blight (*Alternaria dauci),* Black spot (*Alternaria kikuchiana*)*,* Alternaria blotch (*Alternaria mali*)*,* Alternaria leaf spot (*Alternaria porri*)*,* Target spot (*Bipolaris sorghicola*), Southern leaf blight *(Cochliobolus heterostrophus),* Brown spot (*Cochliobolus miyabeanus*), Tip blight (*Pleospora herbarum*)*,* Stripe (*Pyrenophora graminea*)*,* Net blotch (*Pyrenophora teres),* Leaf blight (*Setosphaeria turcica*)*,* Northern leaf blight (*Setosphaeria turcica*)*,* Leaf spot (*Stemphylium botryosum),* Scab (*Venturia carpophila*)*,* Scab (*Venturia Inaequalis*)*,* Scab (*Venturia nashicola*)*,* Gummy stem blight (*Didymella bryoniae*)*,* Leaf spot (*Phoma exigua* var. *exigua*)*,* Streak (*Phoma wasabiae*)*,* Ring rot (*Botryosphaeria berengeriana* f. sp. *piricola*)*,* Soft rot (*Botryosphaeria dothidea, Lasiodiplodia theobromae, Diaporthe* sp.), Common green mold (*Penicillium digitatum*)*,* Blue mold (*Penicillium italicum*)*,* Powdery mildew caused in various kinds of crops, Powdery mildew (*Blumeria graminis* f. sp. *hordei),* Powdery mildew *(Blumeria graminis* f. sp. *tritici*)*,* Powdery mildew (*Erysiphe betae, Leveillula taurica, Oidium* sp., *Podosphaera xanthii*)*,* Powdery mildew (*Erysiphe cichoracearum, Leveillula taurica, Sphaerotheca fuliginea*)*,* Powdery mildew (*Erysiphe heraclei*)*,* Powdery mildew (*Erysiphe pisi*)*, Powdery mildew* (*Leveillula taurica, Oidium neolycopersici, Oidium* sp.), Powdery mildew (*Leveillula taurica*)*,* Powdery mildew (*Oidium* sp., *Podosphaera xanthii),* Powdery mildew (*Oidium* sp.), Powdery mildew (*Phyllactinia kakicola*)*,* Powdery mildew *(Podosphaera fusca*), Powdery mildew (*Podosphaera leucotricha),* Powdery mildew (*Podosphaera pannosa, Uncinuliella simulans* var. *simulans,* U. s. var. *tandae*)*,* Powdery mildew (*Podosphaera xanthii*), Powdery mildew (*Sphaerotheca aphanis* var. *aphanis*)*,* Powdery mildew (*Sphaerotheca fuliginea*)*,* Powdery mildew (*Uncinula necator, U. n. var. necator*)*, Blotch* (*Diplocarpon mali),* Black spot (*Diplocarpon rosae*)*,* Gray mold neck rot (*Botrytis allii*)*,* Gray mold, Botrytis blight (*Botrytis cinerea*)*,* Leaf blight (*Botrytis cinerea, B. byssoidea, B. squamosa*)*,* Chocolate spot (*Botrytis cinerea, B. elliptica, B. fabae*)*,* Brown rot (*Monilinia fructicola, M. fructigena, M. laxa*)*,* Blossom blight *(Monilinia mali*)*,* Dollar spot (*Sclerotinia homoeocarpa*)*,* Cottony rot, Sclerotinia rot, Stem rot (*Sclerotinia sclerotiorum),* False smut (*Villosiclava virens*)*,* Root necrosis (*Calonectria ilicicola*)*,* Fusarium blight (*Fusarium crookwellense, F. culmorum, Gibberella avenacea, G. zeae, Monographella nivalis*)*,* Fusarium blight (*Fusarium culmorum, Gibberella avenacea, G. zeae*)*,* Dry rot (*Fusarium oxysporum, F. solani* f. sp. *radicicola*)*,* Brown rot (*Fusarium oxysporum, F. solani* f. sp. *pisi,* F. s. f. sp. *radicicola*)*,* Fusarium wilt (*Fusarium oxysporum* f. sp. *adzukicola*)*,* Fusarium basal rot (*Fusarium oxysporum* f. sp. *allii, F. solani* f. sp. *radicicola),* Stem rot (*Fusarium oxysporum* f. sp. *batatas, F. solani),* Dry rot (*Fusarium oxysporum* f. sp. *colocasiae*)*,* Yellows (*Fusarium oxysporum* f. sp. *conglutinans*), Panama disease (*Fusarium oxysporum* f. sp. *cubense*), Fusarium wilt (*Fusarium oxysporum* f. sp. *fragariae*), Root rot (*Fusarium oxysporum* f. sp. *lactucae*)*,* Fusarium wilt (*Fusarium oxysporum* f. sp. *lagenariae,* F. o. f. sp. *niveum*)*,* Fusarium wilt (*Fusarium oxysporum* f. sp. *lycopersici*)*,* Fusarium wilt (*Fusarium oxysporum* f. sp. *melonis),* Yellows (*Fusarium oxysporum* f. sp. *raphani*)*,* Fusarium wilt (*Fusarium oxysporum* f. sp. *spinaciae*)*,* Soybean Sudden Death Syndrome (*Fusarium solani* f. sp. *Glycines, Fusarium virguliforme*)*,* "Bakanae" disease (*Gibberella fujikuroi*), Verticillium black spot (*Verticillium albo-atrum, V. dahliae*)*,* Verticillium wilt (*Verticillium dahliae*)*,* Ceratocystis canker (*Ceratocystis ficicola),* Black rot (*Ceratocystis fimbriata*)*,* Gray blight (*Pestalotiopsis longiseta, P. theae*)*,* Endothia canker (*Cryphonectria parasitica*)*,* Melanose (*Diaporthe citri*)*,* Stem blight (*Phomopsis asparagi*)*,* Phomopsis canker (*Phomopsis fukushii*)*,* Brown spot (*Phomopsis vexans),* Anthracnose (*Discula theae-sinensis*)*,* Valsa canker (*Valsa ceratosperma),* Blast (*Magnaporthe grisea*)*,* Crown rot (*Colletotrichum acutatum,* C. *fragariae, Glomerella cingulata*), Bitter rot (*Colletotrichum acutatum, Glomerella cingulata*)*,* Anthracnose (*Colletotrichum acutatum, Glomerella cingulata*)*,* Anthracnose (*Colletotrichum acutatum*)*,* Ripe rot (*Colletotrichum acutatum, Glomerella cingulata*)*,* Anthracnose (*Colletotrichum acutatum),* Anthracnose (*Colletotrichum lindemuthianum*)*,* Anthracnose (*Colletotrichum orbiculare),* Anthracnose (*Glomerella cingulata*)*,* Anthracnose (*Glomerella cingulata*)*,* Anthracnose (*Glomerella cingulata*)*,* Brown stem rot (*Phialophora gregata*)*,* Leaf spot (*Pseudophloeosporella dioscoreae*)*,* Scald (*Rhynchosporium secalis*)*,* Brown rust (*Puccinia recondita*)*,* Stripe rust (*Puccinia striiformis*)*,* Rust caused in various kinds of crops, Rust (*Phakopsora nishidana*)*,* Rust (*Phakopsora pachyrhizi*)*,* Rust (*Kuehneola japonica, Phragmidium fusiforme, P. mucronatum, P. rosae-multiflorae*)*,* Rust (*Gymnosporangium asiaticum*)*,* Rust (*Gymnosporangium yamadae*)*,* Rust (*Puccinia allii*), Rust (*Puccinia horiana),* Rust (*Puccinia tanaceti* var. *tanaceti),* Rust (*Uromyces viciae-fabae* var. *viciae-fabae*)*,* Smut (*Sporisorium scitamineum*)*,* Smut (*Ustilago maydis*)*,* Loose smut (*Ustilago nuda*)*,* Net blister blight (*Exobasidium reticulatum*)*,* Blister blight (*Exobasidium vexans*)*,* Stem rot, Southern blight (*Athelia rolfsii*)*,* Root and stem rot (*Ceratobasidium cornigerum, Rhizoctonia solani*)*,* ginger sheath blight (*Rhizoctonia solani*)*,* Damping-off (*Rhizoctonia solani),* Damping-off (*Rhizoctonia solani*)*,* Bottom rot (*Rhizoctonia solani*)*,* Brown patch, Large patch (*Rhizoctonia solani),* Sheath blight (*Thanatephorus cucumeris*)*,* Root rot (*Thanatephorus cucumeris*)*,* Leaf blight (*Thanatephorus cucumeris*)*,* Rhizopus rot (*Rhizopus stolonifer* var. *stolonifer*)*,* Clubroot (*Plasmodiophora brassicae*)*,* Aphanomyces root rot (*Aphanomyces cochlioides),* White rust (*Albugo macrospora*)*,* Downy mildew caused in various kinds of crops, Downy mildew (*Bremia lactucae*)*,* Downy mildew *(Peronospora chrysanthemi-coronarii*)*,* Downy mildew (*Peronospora destructor*)*,* Downy mildew (*Peronospora farinosa* f. sp. *spinaciae*)*,* Downy mildew (*Peronospora manshurica*)*,* Downy mildew (*Peronospora parasitica*)*,* Downy mildew (*Peronospora sparsa*)*,* Downy mildew (*Plasmopara halstedii*)*,* Downy mildew (*Plasmopara nivea*)*,* Downy mildew (*Plasmopara viticola*)*,* Downy mildew (*Pseudoperonospora cubensis*)*,* Phytophthora root rot (*Phytophthora cactorum*)*,* Brown rot (*Phytophthora capsici*)*,* Phytophthora rot (*Phytophthora capsici*)*,* Phytophthora blight (*Phytophthora capsici*)*,* Phytophthora rot (*Phytophthora cryptogea*)*,* Late blight (*Phytophthora infestans),* White powdery rot (*Phytophthora palmivora),* Leaf blight (*Phytophthora porri*)*,* Phytophthora root and stem rot (*Phytophthora sojae*)*,* Phytophthora stem rot (*Phytophthora vignae* f. sp. *adzukicola*)*,* Damping-off (*Pythium aphanidermatum, P. myriotylum, P. paroecandrum, P. ultimum* var. *ultimum*)*,* Root rot (*Pythium aristosporum*)*,* Browning root rot (*Pythium arrhenomanes, P. graminicola),* Damping-off (*Pythium buismaniae, P. myriotylum*)*,* Root rot (*Pythium myriotylum),* Root rot (*Pythium myriotylum, P. ultimum* var. *ultimum),* Brown blotted root rot (*Pythium sulcatum),* Bacterial canker (*Clavibacter michiganensis* subsp. *michiganensis),* Scab (*Streptomyces* spp.), Crown gall (*Rhizobium radiobacter*)*,* Bacterial stripe (*Burkholderia andropogonis*)*,* Soft rot (*Burkholderia cepacia, Pseudomonas marginalis* pv. *marginalis, Erwinia rhapontici*)*,* Bacterial grain rot (*Burkholderia gladioli, B. glumae*)*,* Bacterial fruit blotch (*Acidovorax avenae* subsp. *citrulli*)*,* Bacterial leaf blight (*Acidovorax konjaci*)*,* Bacterial wilt (*Ralstonia solanacearum),* Bacterial shot hole (*Xanthomonas arboricola* pv. *pruni, Pseudomonas syringae* pv. *syringae, Brenneria nigrifluens*)*,* Bacterial leaf spot (*Xanthomonas arboricola* pv. *pruni*)*,* Bacterial spot (*Xanthomonas axonopodis* pv. *vitians*)*,* Black rot (*Xanthomonas campestris* pv. *campestris*)*,* Bacterial pustule (*Xanthomonas campestris* pv. *glycines*)*,* Bacterial spot (*Xanthomonas campestris* pv. *nigromaculans*)*,* Bacterial spot (*Xanthomonas campestris* pv. *vesicatoria*), Citrus canker (*Xanthomonas citri* subsp. *citri*)*,* garlic spring rot (*Pseudomonas cichorii, P. marginalis* pv. *marginalis, Erwinia* sp.), Bacterial rot (*Pseudomonas cichorii, P. marginalis* pv. *marginalis, P. viridiflava*)*,* Bacterial blossom blight (*Pseudomonas marginalis* pv. *marginalis, P. syringae* pv. *syringae, P. viridiflava*)*,* Bacterial canker (*Pseudomonas syringae* pv. *actinidiae*)*,* Canker (*Pseudomonas syringae* pv. *eriobotryae*)*,* Bacterial spot (*Pseudomonas syringae* pv. *lachrymans*)*,* Bacterial black spot (*Pseudomonas syringae* pv. *maculicola*)*,* Bacterial canker (*Pseudomonas syringae* pv. *morsprunorum, Erwinia* sp.), Bacterial shoot blight (*Pseudomonas syringae* pv. *theae*)*,* Bacterial soft rot (*Dickeya* sp., *Pectobacterium carotovorum*)*,* Fire blight (*Erwinia amylovora*)*,* Soft rot (*Pectobacterium carotovorum*)*,* Bacterial soft rot (*Pectobacterium carotovorum*)*.*

Development of a control agent against diseases in agricultural-horticultural crops has advanced, and a wide variety of chemical agents have been provided for practical use to date. However, due to long-standing use of such chemical agents, an increasing number of situations have been seen in recent years in which pathogens acquire chemical resistance, and control by existing fungicides that have been conventionally used is difficult. Some of the existing chemical agents are highly toxic, and a problem of disturbing the ecosystem is also becoming evident because some chemical agents remain in the environment for a long time. In such circumstances, the compound in accordance with the present invention has excellent control activity against many pathogens and has high safety with respect to crops of interest. The compound in accordance with the present invention can exert a sufficient control effect also against pathogens that have acquired resistance to existing fungicides. Furthermore, the compound in accordance with the present invention does not cause chemical damage to crops of interest, has little adverse effect on mammals, fish, and beneficial insects, and has low persistence and a low load on the environment.

In addition to use as an agricultural-horticultural fungicide, the compound in accordance with the present invention can be used as medical antibacterial agents and animal antibacterial agents for use as antimycotic agents or endoparasite control agents; antifungal agents for use in wood, paper and pulp, adhesives and paints, fibers, leathers and the like; and industrial fungicides for use in cooling water channel in a production plant and the like.

Examples of pathogens targeted as the medical antibacterial agent or the animal antibacterial agent include, but not limited to, Trichophyton fungi such as *Trichophyton rubrum* and *Trichophyton mentagrophytes,* Candida fungi such as *Candida albicans,* Aspergillus fungi such as *Aspergillus fumigatus,* Cryptococcus fungi such as *Cryptococcus neoformas,* gram-negative bacteria such as *Escherichia coli, Pseudomonas aeruginosa,* and *Haemophilus influenzae,* gram-positive bacteria such as *Staphylococcus aureus* and *Streptococcus pyogenes* and the like.

Examples of bacterial strains targeted as the antifungal agent include, but not limited to, wood-rotting fungi such as *Tyromyces palustris* and *Coriolus versicolor,* material deteriorating microorganisms such as *Aspergillus niger, Aspergillus terreus, Eurotium tonophilum, Penicillium citrinum, Penicillium funiculosum, Rhizopus oryzae, Cladosporium cladosporioides, Aureobasidium pullulans, Gliocladium virens, Chaetomium globosum, Fusarium moniliforme,* and *Myrothecium verrucaria* and the like.

Examples of bacterial strain targeted as the industrial fungicide include, but not limited to, slime fungi such as *Sphaerotilis natans* and *Zoogloea ramigera.*

In addition to use as an agricultural-horticultural fungicide, the compound in accordance with the present invention can be used as an endoparasite control agent for domestic animals, domestic fowls, and pet animals.

Specific examples of target endoparasite include, but not limited to, the following.

Nematodes such as *Haemonchus, Trichostrongylus, Ostertagia, Nematodirus, Cooperia, Ascaris, Bunostomum, Oesophagostomum, Chabertia, Trichuris, Storongylus, Trichonema, Dictyocaulus, Capillaria, Heterakis, Toxocara, Ascaridia, Oxyuris, Ancylostoma, Uncinaria, Toxascaris,* and *Parascaris;* Filariidae nematodes such as *Wuchereria, Brugia, Onchoceca, Dirofilaria,* and *Loa;* Dracunculidae nematodes such as *Deacunculus;* cestodes such as *Dipylidium caninum, Taenia taeniaeformis, Taenia solium, Taenia saginata, Hymenolepis diminuta, Moniezia benedeni, Diphyllobothrium latum, Diphyllobothrium erinacei, Echinococcus granulosus,* and *Echinococcus multilocularis;* trematodes such as *Fasciola hepatica, F. gigantica, Paragonimus westermanii, Fasciolopsic bruski, Eurytrema pancreaticum, E. coelomaticum, Clonorchis sinensis, Schistosoma japonicum, Schistosoma haematobium,* and *Schistosoma mansoni; Eimeria* spp. such as *Eimeria tenella, Eimeria acervulina, Eimeria brunetti, Eimeria maxima, Eimeria necatrix, Eimeria bovis,* and *Eimeria ovinoidalis; Trypanosomsa cruzi, Leishmania* spp., *Plasmodium* spp., *Babesis* spp., *Trichomonadidae* spp., *Histomanas* spp., *Giardia* spp., *Toxoplasma* spp., *Entamoeba histolytica, Theileria* spp. and the like.

In addition to use as an agricultural-horticultural fungicide, the compound in accordance with the present invention can be used as an antimycotic agent.

Specific examples of pathogens targeted as the antimycotic agent include, but not limited to, the following. Trichophyton fungi such as *Trichophyton rubrum* and *Trichophyton mentagrophytes,* Candida fungi such as *Candida albicans,* Aspergillus fungi such as *Aspergillus fumigatus,* Cryptococcus fungi such as *Cryptococcus neoformas* and the like.

In a case where the compound in accordance with the present invention is applied as a plant pathogen and pest control agent, the compound in accordance with the present invention is typically mixed with an appropriate solid carrier or liquid carrier, and optionally a surfactant, a penetrating agent, a spreading agent, a thickener, an antifreezing agent, a binder, an anti-caking agent, a disintegrant, a stabilizing agent or the like may be added thereto. Thus, the compound in accordance with the present invention can be practically used as a formulation in an arbitrary form such as a soluble concentrate, an emulsifiable concentrate, a wettable powder, a water soluble powder, a water dispersible granule, a water soluble granule, a suspension concentrate, a concentrated emulsion, a suspoemulsion, a microemulsion, a dustable powder, a granule, a gel or the like. From the viewpoint of laborsaving and improvement in safety, the above formulation in an arbitrary form may be provided while being sealed in a water-soluble package.

Examples of the solid carrier include: naturally-occurring mineral matters such as quartz, kaolinite, pyrophyllite, sericite, talc, bentonite, acid clay, attapulgite, zeolite, and diatomaceous earth; inorganic salts such as calcium carbonate, ammonium sulfate, sodium sulfate, and potassium chloride; synthetic silicic acid; synthetic silicate; and the like.

Examples of the liquid carrier include: alcohols such as ethylene glycol, propylene glycol, isopropanol and the like; aromatic hydrocarbons such as xylene, alkylbenzene, alkylnaphthalene and the like; ethers such as butyl cellosolve; ketones such as cyclohexanone; esters such as y-butyrolactone; acid amides such as N-methylpyrrolidone and N-octylpyrrolidone; vegetable oils such as soybean oil, rapeseed oil, cottonseed oil, and castor oil; water; and the like. These solid and liquid carriers may be used alone or can be used in combination of two or more of these.

Examples of the surfactant include: nonionic surfactants such as polyoxyethylene alkyl ether, polyoxyethylene alkylaryl ether, polyoxyethylene styrylphenyl ether, a polyoxyethylene polyoxypropylene block copolymer, polyoxyethylene fatty acid ester, sorbitan fatty acid ester, and polyoxyethylene sorbitan fatty acid ester; anionic surfactants such as alkyl sulfate, alkylbenzene sulfonate, lignin sulfonate, alkyl sulfosuccinate, naphthalene sulfonate, alkylnaphthalene sulfonate, a salt of formalin condensate of naphthalene sulfonic acid, a salt of formalin condensate of alkylnaphthalene sulfonic acid, polyoxyethylene alkylaryl ether sulfate or phosphate, polyoxyethylene styrylphenyl ether sulfate or phosphate, polycarboxylate and polystyrene sulfonate; cationic surfactants such as an alkylamine salt and an alkyl quaternary ammonium salt; amphoteric surfactants such as of amino acid type and betaine type; and the like.

A content of the surfactant is not particularly limited, and is typically preferably in a range of 0.05 parts by weight to 20 parts by weight, relative to 100 parts by weight of the formulation in accordance with the present invention containing the surfactant. Those surfactants may be used alone or may be used in combination of two or more of those.

In a case where the compound in accordance with the present invention is used as a pesticide, the compound in accordance with the present invention may be applied while being mixed, in formulation or spraying, with other types of herbicides, various insecticides, acaricides, nematicides, fungicides, plant growth regulators, synergists, fertilizers, soil amendments or the like, as necessary.

In particular, by being mixed with other pesticides or phytohormones, it is possible to expect cost reduction by a decrease in an application amount, expansion of a fungicidal-insecticidal spectrum by synergy of the mixed chemical agent, or a higher pest control effect. In this case, it is possible to simultaneously combine the compound with a plurality of known pesticides.

Examples of types of pesticides to be used and mixed with the compound in accordance with the present invention in accordance with an aspect include compounds disclosed in The Pesticide Manual, 18th edition, 2018 and the like. Specific examples of generic names thereof include the following. Note, however, that the pesticide to be used in the mixture is not necessarily limited only to those.

Fungicides: acibenzolar-S-methyl, acypetacs, aldimorph, allyl alcohol, ametoctradin, aminopyrifen, amisulbrom, amobam, ampropylfos, anilazine, azaconazole, azithiram, azoxystrobin, barium polysulfide, benalaxyl, benalaxyl-M, benodanil, benomyl, benquinox, bentaluron, benthiavalicarb-isopropyl, benthiazole, benzamacril, benzamorf, benzovindiflupyr, binapacryl, biphenyl, bitertanol, bixafen, blasticidin-S, bordeaux mixture, boscalid, bromoconazole, bupirimate, buthiobate, butylamine, calcium polysulfide, captafol, captan, carbamorph, carbendazim, carboxin, carpropamid, carvone, cheshunt mixture, chinomethionat, chlobenthiazone, chloraniformethane, chloranil, chlorfenazole, chloroneb, chloropicrin, chlorothalonil, chlorquinox, chlozolinate, climbazole, copper acetate, basic copper carbonate, copper hydroxide, copper naphthenate, copper oleate, copper oxychloride, copper sulfate, basic copper sulfate, copper zinc chromate, coumoxystrobin, cresol, cufraneb, cuprobam, cyazofamid, cyclafuramid, cycloheximide, cyflufenamid, cymoxanil, cypendazole, cyproconazole, cyprodinil, cyprofuram, dazomet, debacarb, decafentin, dehydroacetic acid, dichlobentiazox, dichlofluanid, dichlone, dichlorophen, dichlozoline, diclobutrazol, diclocymet, diclomezine, dicloran, diethofencarb, difenoconazole, diflumetorim, dimethirimol, dimethomorph, dimoxystrobin, diniconazole, diniconazole-M, dinobuton, dinocap, dinocap-4, dinocap-6, dinocton, dinosulfon, dinoterbon, diphenylamine, dipymetitrone, dipyrithione, disulfiram, ditalimfos, dithianon, DNOC, dodemorph, dodine, drazoxolon, edifenphos, enestrobin, enoxastrobin, epoxiconazole, ethaboxam, etaconazole, etem, ethirimol, ethoxyquin, etridiazole, famoxadone, fenamidone, fenaminosulf, fenaminstrobin, fenapanil, fenarimol, fenbuconazole, fenfuram, fenhexamid, fenitropan, fenoxanil, fenpiclonil, fenpicoxamid, fenpropidin, fenpropimorph, fenpyrazamine, fentin, ferbam, ferimzone, florylpicoxamid, fluazinam, fludioxonil, flufenoxystrobin, fluindapyr, flumetover, flumorph, fluopicolide, fluopimomide, fluopyram, fluoroimide, fluotrimazole, fluoxapiprolin, fluoxastrobin, fluquinconazole, flusilazole, flusulfamide, flutolanil, flutianil, flutriafol, fluxapyroxad, folpet, fosetyl-aluminium, fthalide, fuberidazole, furalaxyl, furametpyr, furcarbanil, furconazole, furconazole-cis, furmecyclox, furophanate, glyodin, griseofulvin, guazatine, halacrinate, hexachlorobenzene, hexaconazole, hexylthiofos, 8-hydroxyquinoline sulfate, hymexazol, imazalil, imibenconazole, iminoctadine-albesilate, iminoctadine-triacetate, inpyrfluxam, iodocarb, ipconazole, ipfentrifluconazole, ipflufenoquin, iprobenfos, iprodione, iprovalicarb, isofetamid, isoflucypram, isotianil, isoprothiolane, isopyrazam, isovaledione, kasugamycin, kresoxim-methyl, laminarin, mancopper, mancozeb, mandestrobin, mandipropamid, maneb, mebenil, mecarbinzid, mefentrifluconazole, mepanipyrim, mepronil, meptyldinocap, metalaxyl, metalaxyl-M, metam, metazoxolon, metconazole, methasulfocarb, methfuroxam, metyltetraprole, metiram, metominostrobin, metrafenone, metsulfovax, milneb, myclobutanil, myclozolin, nabam, naftifine, natamycin, organic nickel (nickel bis(dimethyldithiocarbamate)), nitrostyrene, nitrothal-isopropyl, nuarimol, octhilinone, ofurace, orysastrobin, oxadixyl, oxathiapiprolin, oxine copper, oxpoconazole fumarate, oxycarboxin, pefurazoate, penconazole, pencycuron, penflufen, pentachlorophenol, penthiopyrad, 2-phenylphenol, phosdiphen, phthalide, picarbutrazox, picoxystrobin, piperalin, polycarbamate, polyoxins, polyoxorim, potassium azide, potassium hydrogen carbonate, probenazole, prochloraz, procymidone, propamocarb hydrochloride, propiconazole, propineb, proquinazid, prothiocarb, pyrazophos, pyribencarb, pyrifenox, pyrimethanil, pyriminostrobin, pyroquilon, prothiocarb, prothioconazole, pydiflumetofen, pyracarbolid, pyraclostrobin, pyrametostrobin, pyraoxystrobin, pyrapropoyne, pyraziflumid, pyridachlometyl, pyridinitril, pyriofenone, pyrisoxazole, pyroxychlor, pyroxyfur, quinacetol-sulfate, quinazamid, quinconazole, quinoxyfen, quinofumelin, quintozene, rabenzazole, salicylanilide, sedaxane, silthiofam, simeconazole, sodium hydrogen carbonate, sodium hypochlorite, spiroxamine, sulfur, tebuconazole, tebufloquin, tecloftalam, tecnazene, tecoram, tetraconazole, thiabendazole, thiadifluor, thicyofen, thifluzamide, thiochlorfenphim, thiophanate, thiophanate-methyl, thiram, tiadinil, tioxymid, tolclofos-methyl, tolprocarb, tolylfluanid, triadimefon, triadimenol, triamiphos, triarimol, triazbutil, triazoxide, tributyltin oxide, trichlamide, triclopyricarb, tricyclazole, tridemorph, trifloxystrobin, triflumizole, triforine, triticonazole, validamycin, valifenalate, vinclozolin, zarilamid, zinc naphthenate, zinc sulfate, zineb, ziram, zoxamide, a shiitake mycelium extract, a shiitake fruiting body extract and the like.

Insecticide: abamectin, acephate, acequinocyl, acetamiprid, acrinathrin, acynonapyr, afidopyropen, afoxolaner, alanycarb, aldicarb, allethrin, alpha-cypermethrin, alpha-endosulfan, amidoflumet, amitraz, azamethiphos, azinphos-ethyl, azinphos-methyl, azocyclotin, bacillus thuringiensis, bendiocarb, benfluthrin, benfuracarb, bensultap, benzoximate, benzpyrimoxan, beta-cyfluthrin, beta-cypermethrin, bifenazate, bifenthrin, bioallethrin, bioresmethrin, bistrifluron, broflanilide, bromopropylate, buprofezin, butocarboxim, carbaryl, carbofuran, carbosulfan, cartap, chinomethionat, chlorantraniliprole, chlorethxyfos, chlorfenapyr, chlorfenvinphos, chlorfluazuron, chlormephos, chlorobezilate, chloroprallethrin, chlorpyrifos, chlorpyrifos-methyl, chromafenozide, clofentezine, clothianidin, cyanophos, cyan traniliprole, cyclaniliprole, cycloprothrin, cyenopyrafen, cyetpyrafen, cyflumetofen, cyfluthrin, cyhalodiamide, cyhalothrin, cyhexatine, cypermethrin, cyphenothrin, cyproflanilide, cyromazine, deltamethrin, diacloden, diafenthiuron, diazinon, dichlorvos, dicloromezotiaz, dicofol, dienochlor, diflovidazin, diflubenzuron, dimefluthrin, dimethoate, dimethylvinphos, dimpropyridaz, dinotefuran, diofenolan, disulfoton, DNOC, d-tetramethrin, emamectin-benzoate, empenthrin, endosulfan, EPN, epsilon-metofluthrin, epsilon-momfluorothrin, esfenvalerate, ethiofencarb, ethiprole, etofenprox, etoxazole, etrimfos, Febantel, fenazaquin, fenbutatin oxide, fenitrothion, fenmezoditiaz, fenobucarb, fenothiocarb, fenoxycarb, fenpropathrin, fenpyroximate, fenthion, fenvalerate, fipronil, flometoquin, flonicamid, fluacrypyrim, fluazuron, flubendiamide, fluchlorodiniliprole, flucycloxuron, flucythrinate, flufenerim, flufenoxuron, flufenprox, flufiprole, fluhexafon, flumethrin, flupentiofenox, flupyradifurone, flupyrimin, fluralaner, fluvalinate, fluxametamide, fonophos, formetanate, formothion, furathiocarb, gamma-cyhalothrin, halfenprox, halofenozide, heptafluthrin, hexaflumuron, hexythiazox, hydramethylnon, imidacloprid, imiprothrin, indazapyroxamet, indoxacarb, indoxacarb-MP, isocycloseram, isofenphos, isoprocarb, isoxathion, kappa-bifenthrin, kappa-tefluthrin, lambda-cyhalothrin, lepimectin, lufenuron, malathion, meperfluthrin, metaflumizone, metalcarb, metaldehyde, methacrifos, methamidophos, methidathion, methomyl, methoprene, methoxychlor, methoxyfenozide, methyl bromide, metofluthrin, milbemectin, momfluorothrin, monocrotophos, muscalure, nicofluprole, nitenpyram, novaluron, noviflumuron, omethoate, oxazosulfyl, oxydemeton-methyl, oxydeprofos, parathion, parathion-methyl, pentachlorophenol, permethrin, phenothrin, phenthoate, phorate, phosalone, phosmet, phosphamidon, phoxim, pirimicarb, pirimiphos-methyl, Praziquantel, profenofos, profluthrin, propaphos, propargite, prothiofos, protrifenbute, pyflubumide, pymetrozine, pyraclofos, pyrafluprole, pyrethrins, pyridaben, pyridalyl, pyrifluquinazon, pyrimidifen, pyriprole, pyriproxyfen, resmethrin, rotenone, silafluofen, spidoxamat, spinetoram, spinosad, spirodiclofen, spiromesifen, spiropidion, spirotetramat, spyromesifen, sulfotep, sulfoxaflor, sulprofos, tau-fluvalinate, tebfenozide, tebufenpyrad, teflubenzuron, tefluthorin, terbufos, tetrachlorantraniliprole, tetrachlorvinphos, tetramethrin, tetramethylfluthrin, tetraniliprole, thiacloprid, thiamethoxam, thiocyclam, thiodicarb, thiofanox, thiometon, tolfenpyrad, tralomethrin, transfluthrin, triazamate, triazuron, trichlorfon, triflumezopyrim, triflumuron, tyclopyrazoflor, vamidothion, zeta-cypermethrin and the like.

Parasiticides: esfenvalerate, fenpropathrin, fenvalerate, cypermethrin, bifenthrin, cypermethrin, deltamethrin, etofenprox, lambda-cyhalothrin, permethrin, tefluthrin, zeta-cypermethrin, acetamiprid, clothianidin, dinotefuran, imidacloprid, nitenpyram, thiamethoxam, chromafenozide, fenoxycarb, lufenuron, methoprene, pyriproxyfen, triflumuron, chlorpyrifos, chlorpyrifos-methyl, diazinon, dichlorvos, fenitrothion, fenthion, malathion, pirimiphos-methyl, tetrachlorvinphos, ethiprole, fipronil, propoxur, carbaryl, bendiocarb, metoxadiazone, fenobucarb, carbofuran, afoxolaner, fluralaner, fluxametamide, sarolaner, lotilaner, tigolaner, esafoxolaner, modoflaner, umifoxolaner, mivorilaner, avermectin, ivermectin, doramectin, eprinomectin, maduramycin, milbemycin, milbemycin oxime, moxidectin, selamectin, indoxacarb, amitraz, bistrifluron, spinosad, albendazole, atovaquone, bithionol, cambendazole, carnidazole, chloroquine, clazuril, clorsulon, closantel, coumaphos, dichlorophen, diethylcarbamazine, diminazene, dinitolmide, dithiazanine iodide, emodepside, epsiprantel, febantel, fenbendazole, flubendazole, glycalpyramide, imidocarb, levamisole, mebendazole, mefloquine hydrochloride, melarsamine hydrochloride, metronidazole, metyridine, monepantel, morantel tartrate, niclosamide, oxantel pamoate, oxantel tartrate, oxibendazole, oxyclozanide, piperazine adipate, piperazine citrate, piperazine phosphate, praziquantel, pyrantel pamoate, rafoxanide, tetramisole hydrochloride, thiabendazole, triclabendazole and the like.

Antimycotic agents: ketoconazole, miconazole nitrate and the like.

Antimicrobial agents: amoxicillin, ampicillin, bethoxazin, bithionol, bronopol, cefapirin, cefazolin, cefquinome, ceftiofur, chlortetracycline, clavulanic acid, danofloxacin, difloxacin, dinitolmide, enrofloxacin, florfenicol, lincomycin, lomefloxacin, marbofloxacin, miloxacin, mirosamycin, nitrapyrin, norfloxacin, octhilinone, ofloxacin, orbifloxacin, oxolinic acid, oxytetracycline, penicillin, streptomycin, thiamphenicol, tiamulin fumarate, tilmicosin phosphate, acetylisovaleryltylosin, tylosin phosphate, tulathromycin, valnemulin, calcinated shell calcium (calcium oxide), Talaromyces fungi, Trichoderma fungi, and Coniothyrium fungi and the like.

An application amount of the compound in accordance with the present invention varies in accordance with an application situation, application time, application method, cultivated crop or the like, and typically, as an amount of active ingredient, is suitably 0.005 kg to 50 kg, preferably 0.01 kg to 1 kg, per hectare (ha).

The following description will discuss a blending example of the formulation in a case where the compound in accordance with the present invention is used. Note, however, that the present invention is not limited only to those blending examples. In the blending examples below, the term "parts" means parts by weight.

### [Wettable powder]

| | |
|---|---|
| Present compound: | 0.1 parts to 80 parts |
| Solid carrier: | 5 parts to 98.9 parts |
| Surfactant: | 1 part to 10 parts |
| Others: | 0 parts to 5 parts |

Examples of the above others include an anti-caking agent, a stabilizing agent and the like.

### [Emulsifiable concentrate]

| | |
|---|---|
| Present compound: | 0.1 parts to 30 parts |
| Liquid carrier: | 45 parts to 95 parts |
| Surfactant: | 4.9 parts to 15 parts |
| Others: | 0 parts to 10 parts |

Examples of the above others include a spreading agent, a stabilizing agent and the like.

### [Suspension concentrate]

| | |
|---|---|
| Present compound: | 0.1 parts to 70 parts |
| Liquid carrier: | 15 parts to 98.89 parts |
| Surfactant: | 1 part to 12 parts |
| Others: | 0.01 parts to 30 parts |

Examples of the above others include an antifreezing agent, a thickener and the like.

### [Water dispersible granule]

| | |
|---|---|
| Present compound: | 0.1 parts to 90 parts |
| Solid carrier: | 0 parts to 98.9 parts |
| Surfactant: | 1 part to 20 parts |
| Others: | 0 parts to 10 parts |

Examples of the above others include a binder, a stabilizing agent and the like.

### [Soluble concentrate]

| | |
|---|---|
| Present compound: | 0.01 parts to 70 parts |
| Liquid carrier: | 20 parts to 99.99 parts |
| Others: | 0 parts to 10 parts |

Examples of the above others include an antifreezing agent, a spreading agent and the like.

### [Granule]

| | |
|---|---|
| Present compound: | 0.01 parts to 80 parts |
| Solid carrier: | 10 parts to 99.99 parts |
| Others: | 0 parts to 10 parts |

Examples of the above others include a binder, a stabilizing agent and the like.

### [Dustable powder]

| | |
|---|---|
| Present compound: | 0.01 parts to 30 parts |
| Solid carrier: | 65 parts to 99.99 parts |
| Others: | 0 parts to 5 parts |

Examples of the above others include a drift reduction agent, a stabilizing agent and the like.

In use, the formulation is diluted 1 time to 10000 times, preferably 100 times to 10000 times with water and sprayed, or is sprayed without dilution.

Formulation examples of an agricultural-horticultural fungicide which contains the compound in accordance with the present invention as an active ingredient will be specifically described below. The formulation, including the compound in accordance with the present invention, is not limited only to those examples. In the formulation examples below, the term "parts" means parts by weight.

### [Formulation Example 1] Emulsifiable concentrate

| | |
|---|---|
| Present compound No. 1-001: | 20 parts |
| Methylnaphthalene: | 55 parts |
| Cyclohexanone: | 20 parts |
| SORPOL 2680: | 5 parts |

(a mixture of a nonionic surfactant and an anionic surfactant available from TOHO Chemical Industry Co., Ltd., product name)

The above components are uniformly mixed to obtain an emulsifiable concentrate. In use, the emulsifiable concentrate is diluted 50 times to 20000 times with water, and is sprayed such that an active ingredient amount is 0.005 kg to 50 kg per hectare.

### [Formulation Example 2] Wettable powder

| | |
|---|---|
| Present compound No. 1-001: | 25 parts |
| Pyrophyllite: | 66 parts |
| SORPOL 5039: | 4 parts |

(an anionic surfactant available from TOHO Chemical Industry Co., Ltd., product name)

| | |
|---|---|
| CARPLEX #80D: | 3 parts |

(white carbon available from Shionogi & Co., Ltd., product name)

| | |
|---|---|
| Calcium ligninsulfonate: | 2 parts |

The above components are uniformly mixed and pulverized to obtain a wettable powder. In use, the wettable powder is diluted 50 times to 20000 times with water, and is sprayed such that an active ingredient amount is 0.005 kg to 50 kg per hectare.

### [Formulation Example 3] Dustable powder

| | |
|---|---|
| Present compound No. 1-001: | 3 parts |
| CARPLEX #80D: | 0.5 parts |

(white carbon available from Shionogi & Co., Ltd., product name)

| | |
|---|---|
| Kaolinite: | 95 parts |
| Diisopropyl phosphite: | 1.5 parts |

The above components are uniformly mixed and pulverized to obtain a dustable powder. In use, the dustable powder is sprayed such that an active ingredient amount of the dustable powder is 0.005 kg to 50 kg per hectare.

### [Formulation Example 4] Granule

| | |
|---|---|
| Present compound No. 1-001: | 5 parts |
| Bentonite: | 30 parts |
| Talc: | 64 parts |
| Calcium ligninsulfonate: | 1 part |

The above components are uniformly mixed and pulverized, and a small amount of water is added and kneaded. Then a resultant mixture is granulated by an extrusion granulator, and dried to obtain a granule. In use, the granule is sprayed such that an active ingredient amount of the granule is 0.005 kg to 50 kg per hectare.

### [Formulation Example 5] Flowable agent

| | |
|---|---|
| Present compound No. 1-001: | 25 parts |
| SORPOL 3353: | 5 parts |

(a nonionic surfactant available from TOHO Chemical Industry Co., Ltd., product name)

| | |
|---|---|
| RUNOX 1000C: | 0.5 parts |

(an anionic surfactant available from TOHO Chemical Industry Co., Ltd., product name)

| | |
|---|---|
| Xanthan gum (naturally-occurring polymer): | 0.2 parts |
| Sodium benzoate: | 0.4 parts |
| Propylene glycol: | 10 parts |
| Water: | 58.9 parts |

The above components except for the active ingredient (the compound in accordance with the present invention) are uniformly dissolved, and then the compound in accordance with the present invention is added and stirred well. Then, a resultant mixture is wet-pulverized with a sand mill to obtain a flowable agent. In use, the flowable agent is diluted 50 times to 20000 times with water, and is sprayed such that an active ingredient amount is 0.005 kg to 50 kg per hectare.

### [Formulation Example 6] Dry flowable agent

| | |
|---|---|
| Present compound No. 1-001: | 75 parts |
| HITENOL NE- 15: | 5 parts |

(an anionic surfactant available from Dai-ichi Kogyo Seiyaku Co., Ltd., product name)

| | |
|---|---|
| VANILLEX N: | 10 parts |

(an anionic surfactant available from Nippon Paper Industries, Co., Ltd., product name)

| | |
|---|---|
| CARPLEX #80D: | 10 parts |

(white carbon available from Shionogi & Co., Ltd., product name)

The above components are uniformly mixed and finely pulverized, and a small amount of water is added and kneaded. Then, a resultant mixture is granulated by an extrusion granulator, and dried to obtain a dry flowable agent. In use, the dry flowable agent is diluted 50 times to 20000 times with water, and is sprayed such that an active ingredient is 0.005 kg to 50 kg per hectare.

Examples of an application method of the compound in accordance with the present invention include foliage application, soil treatment, seed disinfection and the like. The compound in accordance with the present invention is also effective in a general method which is typically used by a person skilled in the art.

Aspects of the present invention can also be expressed as follows:
As described above, the present invention relates to <1> through <65> below.

<1> A pyrazole compound represented by a formula (1) or a salt thereof: 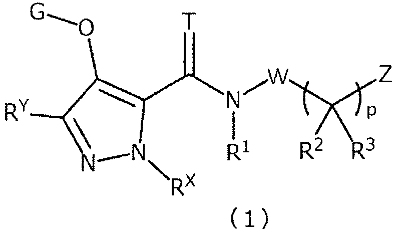
   where G represents G-1,
   G-1 represents a structure indicated by a structural formula below, 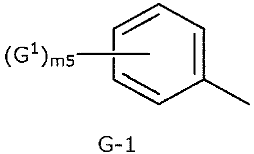
   G¹ represents hydroxy, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   T represents an oxygen atom,
   W represents -O- or -N(R^{W})-,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   R^{X}-1 through R^{X}-4 represent structures indicated by structural formulae below, 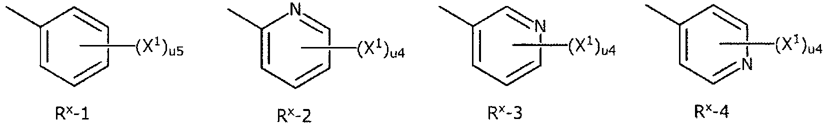
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom, C₁-C₆ alkyl or C₁-C₆ alkyl substituted by Rⁱ,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, di(C₁-C₆ alkyl)aminothiocarbonyl, C₁-C₆ alkylsulfonyl, - C(O)OR^{d}, -C(O)C(O)R^{e}, -C(O)SR^{f}, -C(O)R^{g}, Q-1 to Q-53 or Q-54,
   R² represents a hydrogen atom, C₁-C₆ alkyl, C₁-C₆ haloalkyl or C₁-C₆ alkyl substituted by R^{h},
   R³ represents a hydrogen atom, C₁-C₆ alkyl or C₁-C₆ haloalkyl,
   R^{a} represents hydroxy, cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, -C(N=OR^{b})R^{c}, Q-1 to Q-53 or Q-54,
   R^{d} represents C₁-C₆ alkyl,
   R^{c} represents C₁-C₆ alkyl,
   R^{d} represents a structure indicated by a structural formula below, 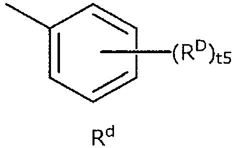
   R^{D} represents a halogen atom, nitro or C₁-C₆ alkyl,
   in a case where t5 represents an integer of 2, 3, 4 or 5 in relationship to R^{D}, the respective R^{D} may be identical with each other or may be different from each other,
   R^{e} represents C₁-C₆ alkyl or C₁-C₆ alkoxy,
   R^{f} represents C₁-C₆ alkyl,
   R^{e} represents Q-1 to Q-53 or Q-54,
   R^{h} represents cyano or C₁-C₆ alkoxy,
   Rⁱ represents cyano or C₁-C₆ alkoxy,
   Z represents Z-1 to Z-49 or Z-50,
   Z-1 through Z-50 respectively represent structures indicated by structural formulae below, 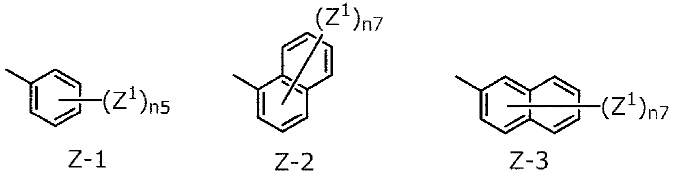 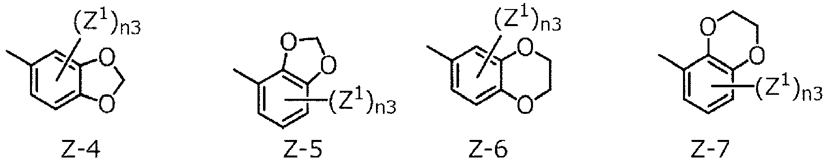 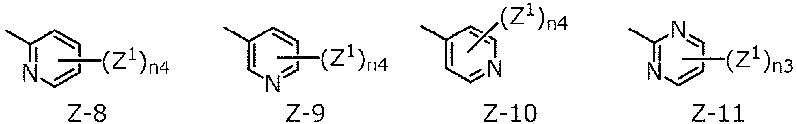 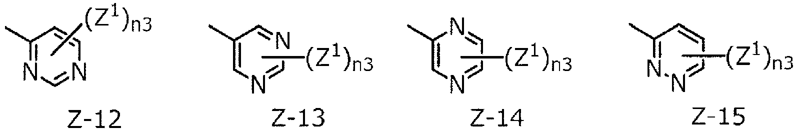 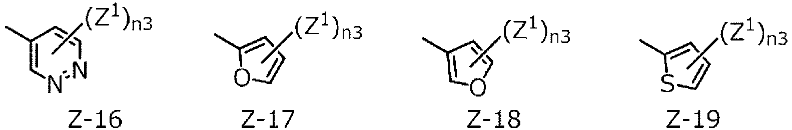 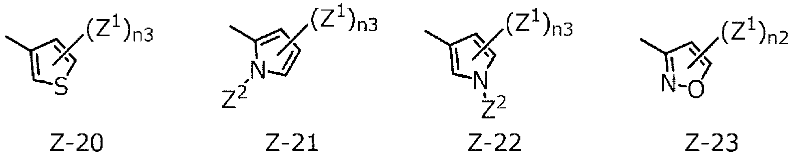 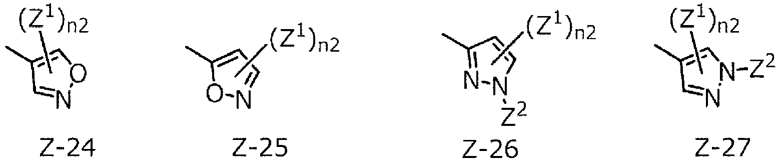 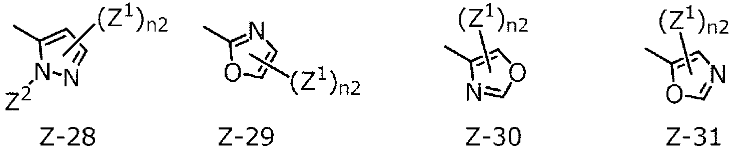 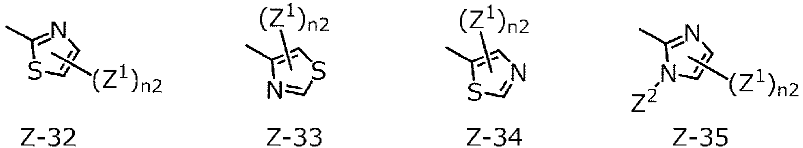 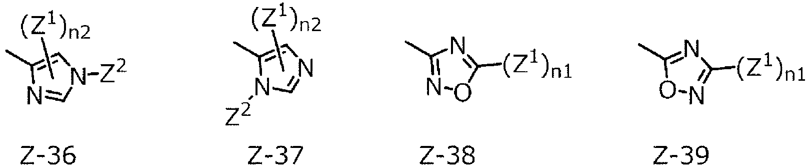 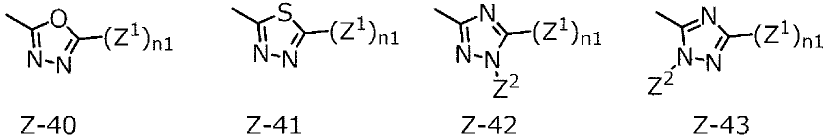 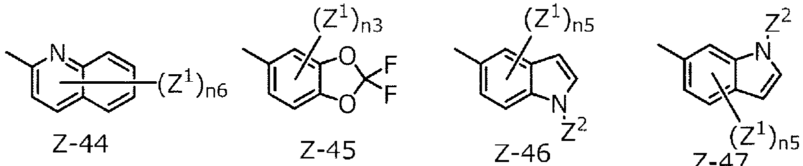 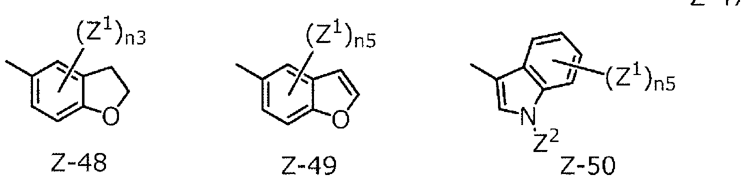
   a substitution position of Z¹ indicates that the substitution occurs on an aromatic ring of each of Z-1 through Z-50,
   Z¹ represents hydroxy, carboxy, amino, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ haloalkylthio, C₁-C₆ alkylsulfinyl, C₁-C₆ alkylsulfonyl, C₁-C₆ alkylcarbonyl, C₁-C₆ alkoxycarbonyl, - OR^{j}, -NHC(O)R^{k}, -C(O)NHR^{m}, -C(=NOR)R', -C(=NOH)NH₂, E-1 to E-54 or E-55,
   in a case where n2 represents an integer of 2 in relationship to Z¹, the respective Z¹ may be identical with each other or may be different from each other,
   in a case where n3 represents an integer of 2 or 3 in relationship to Z¹, the respective Z¹ may be identical with each other or may be different from each other,
   in a case where n4 represents an integer of 2, 3 or 4 in relationship to Z¹, the respective Z¹ may be identical with each other or may be different from each other,
   in a case where n5 represents an integer of 2, 3, 4 or 5 in relationship to Z¹, the respective Z¹ may be identical with each other or may be different from each other,
   in a case where n6 represents an integer of 2, 3, 4, 5 or 6 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   Z² represents C₁-C₆ alkyl, phenyl or pyridin-2-yl,
   R represents a hydrogen atom or C₁-C₆ alkyl,
   R' represents a hydrogen atom or C₁-C₆ alkyl,
   Rⁱ represents C₂-C₆ alkenyl, C₂-C₆ alkynyl, C₁-C₆ alkyl substituted by R⁴, C₁-C₆ alkylcarbonyl, C₁-C₆ alkoxycarbonyl, C₁-C₆ alkylaminocarbonyl, E-1 to E-54 or E-55,
   R⁴ represents cyano, C₁-C₆ alkoxy, C₃-C₁₀ cycloalkyl, C₁-C₆ alkylcarbonyl, C₁-C₆ alkoxycarbonyl, di(C₁-C₆ alkyl)aminocarbonyl, E-1 to E-54 or E-55,
   R^{k} represents C₁-C₆ alkyl, C₃-C₁₀ cycloalkyl, C₁-C₆ haloalkyl, C₁-C₆ alkyl substituted by R⁵, C₁-C₆ alkoxy, C₂-C₆ alkenyloxy, C ₁- C₆ alkylamino, di(C₁-C₆ alkyl)amino, benzyloxy, E-1 to E-54 or E-55,
   R⁵ represents cyano, C₁-C₆ alkoxy, E-1 to E-53 or E-54,
   R^{m} represents C₁-C₆ alkyl, C₃-C₁₀ cycloalkyl, C₂-C₆ alkenyl, C₁-C₆ alkoxy, benzyl, E-1 to E-54 or E-55,
   E-1 through E-55 respectively represent structures indicated by structural formulae below, 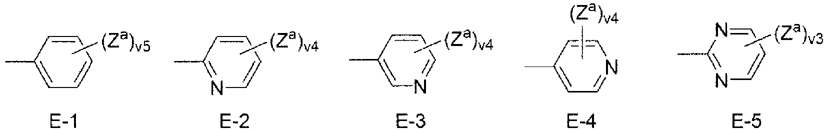 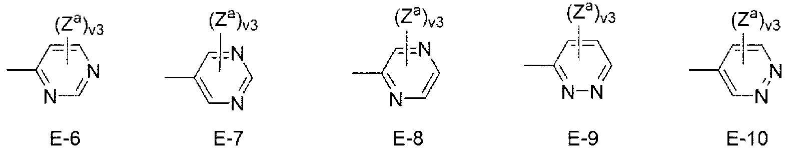 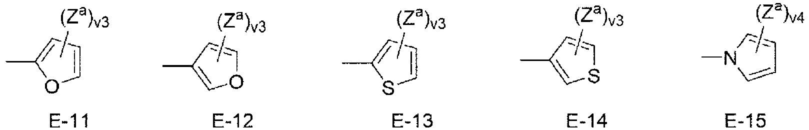 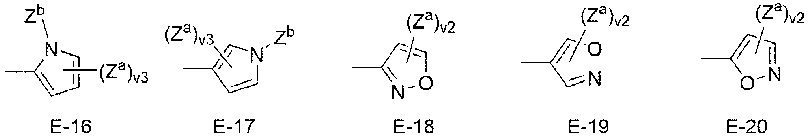 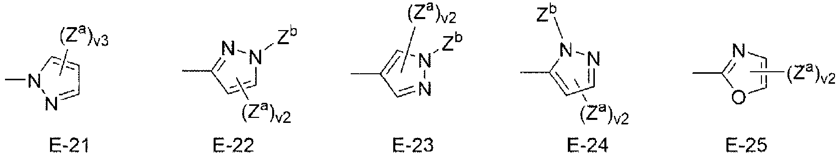 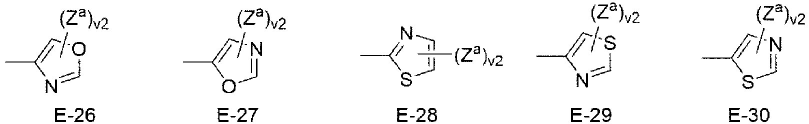 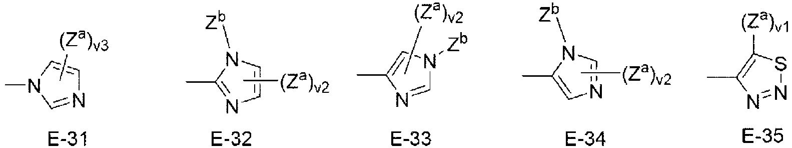 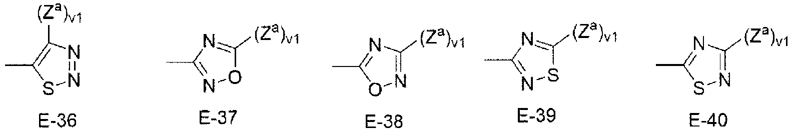 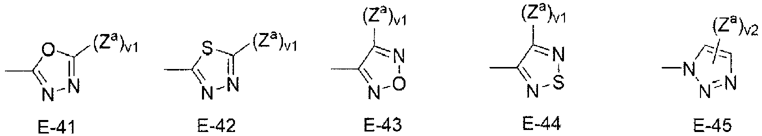 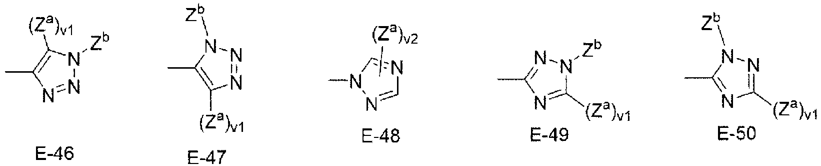 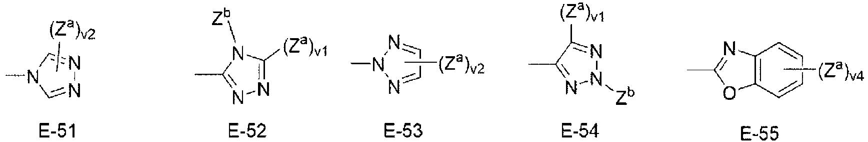
   Z^{a} represents a halogen atom, cyano, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ haloalkoxy, C₁-C₆ alkylcarbonyl, C₁-C₆ alkoxycarbonyl, phenyl, S-1 to S-5 or S-6,
   in a case where v5 represents an integer of 2, 3, 4 or 5 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   in a case where v3 represents an integer of 2 or 3 in relationship to Z^{a}, the respective Z^{a} may be identical with each other or may be different from each other,
   in a case where v2 represents an integer of 2 in relationship to Z^{a}, the respective Z^{a} may be identical with each other or may be different from each other,
   R⁶ represents hydroxy, C₁-C₆ alkoxy or C₁-C₆ alkoxycarbonyl,
   Z^{b} represents C₁-C₆ alkyl,
   S-1 through S-6 respectively represent structures indicated by structural formulae below, 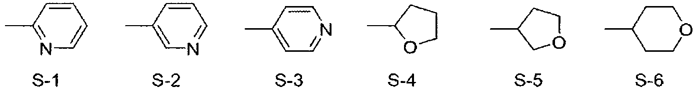
   Q-1 through Q-54 respectively represent structures indicated by structural formulae below, 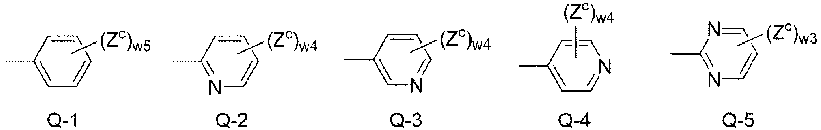 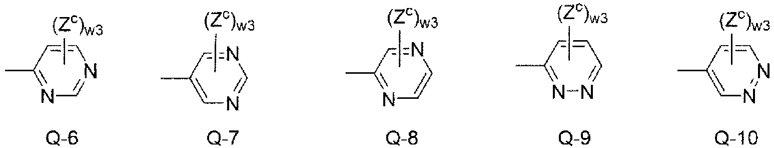 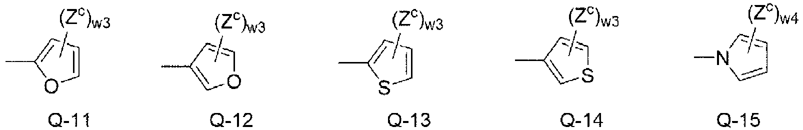 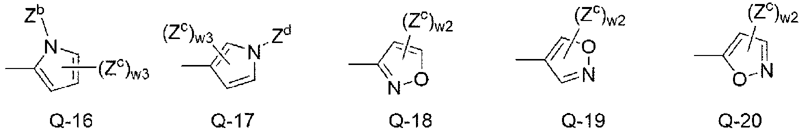 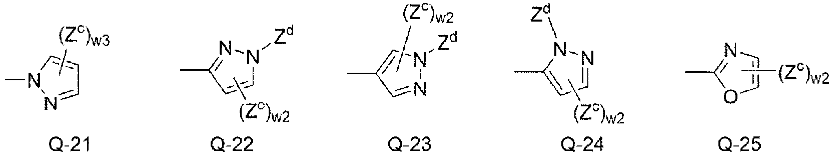 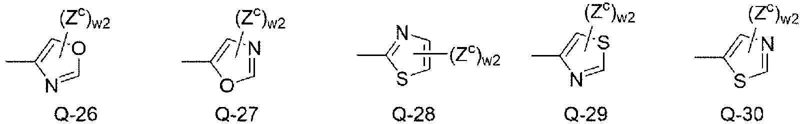 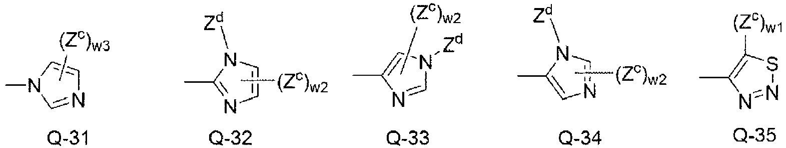 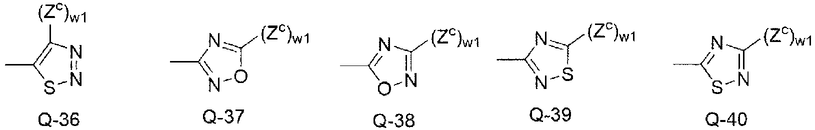 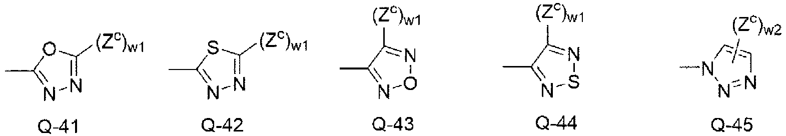 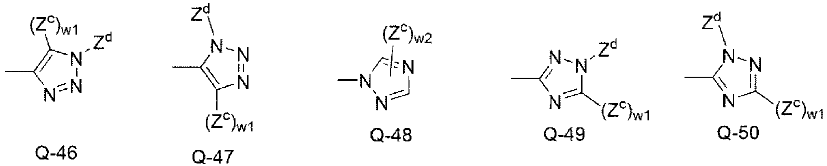 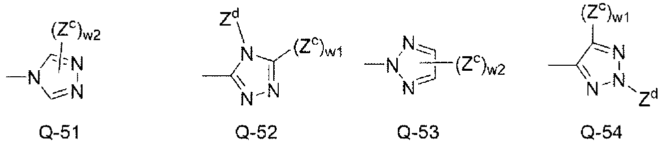
   Z^{c} represents a halogen atom, cyano, C₁-C₆ alkyl, C₁-C₆ haloalkyl, C₁-C₆ alkoxy, C ₁- C₆ haloalkoxy, C₁- C₆ alkylcarbonyl or C₁-C₆ alkoxycarbonyl,
   in a case where w5 represents an integer of 2, 3, 4 or 5 in relationship to Z^{c}, the respective Z^{c} may be identical with each other or may be different from each other,
   in a case where w4 represents an integer of 2, 3 or 4 in relationship to Z^{c}, the respective Z^{c} may be identical with each other or may be different from each other,
   in a case where w3 represents an integer of 2 or 3 in relationship to Z^{c}, the respective Z^{c} may be identical with each other or may be different from each other,
   in a case where w2 represents an integer of 2 in relationship to Z^{c}, the respective Z^{c} may be identical with each other or may be different from each other,
   Z^{d} represents C₁-C₆ alkyl,
   m5 represents an integer of 0, 1, 2, 3, 4 or 5,
   n2 represents an integer of 0, 1 or 2,
   n3 represents an integer of 0, 1, 2 or 3,
   n4 represents an integer of 0, 1, 2, 3 or 4,
   n5 represents an integer of 0, 1, 2, 3, 4 or 5,
   n6 represents an integer of 0, 1, 2, 3, 4, 5 or 6,
   n7 represents an integer of 0, 1, 2, 3, 4, 5, 6 or 7,
   u4 represents an integer of 0, 1, 2, 3 or 4,
   u5 represents an integer of 0, 1, 2, 3, 4 or 5,
   t5 represents an integer of 0, 1, 2, 3, 4 or 5,
   p represents an integer of 0 or 1,
   v1 represents an integer of 0 or 1,
   v2 represents an integer of 0, 1 or 2,
   v3 represents an integer of 0, 1, 2 or 3,
   v4 represents an integer of 0, 1, 2, 3 or 4,
   v5 represents an integer of 0, 1, 2, 3, 4 or 5,
   w1 represents an integer of 0 or 1,
   w2 represents an integer of 0, 1 or 2,
   w3 represents an integer of 0, 1, 2 or 3,
   w4 represents an integer of 0, 1, 2, 3 or 4, and
   w5 represents an integer of 0, 1, 2, 3, 4 or 5.
<2> The pyrazole compound described in < 1> or a salt thereof, in which:
   G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1, R^{X}-2, R^{X}-3 or R^{X}-4,
   R^{Y} represents a hydrogen atom or a halogen atom,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
   R² represents a hydrogen atom, C₁-C₆ alkyl or C₁-C₆ alkyl substituted by R^{h},
   R³ represents a hydrogen atom,
   R^{a} represents hydroxy, cyano, C₃-C₁₀ cycloalkyl, C₁-C₆ alkoxy, C ₁- C₆ alkylsulfonyl, di(C₁-C₆ alkyl)amino, C₁- C₆ alkylcarbonyl, C₁-C₆ alkoxycarbonyl, tri(C₁-C₆ alkyl)silyl, - C(N=OR^{b})R^{c} or Q-1,
   R^{D} represents a halogen atom or nitro,
   R^{e} represents C₁-C₆ alkoxy,
   R^{g} represents Q-1, Q-2, Q-3, Q-4 or Q-11,
   R^{h} represents C₁-C₆ alkoxy,
   Rⁱ represents cyano,
   Z represents Z-1, Z-2, Z-3, Z-4, Z-6, Z-8, Z-9, Z-17, Z-19, Z-25, Z-27, Z-28, Z-34, Z-44, Z-45, Z-46, Z-47, Z-48, Z-49 or Z-50,
   in a case where W is -O-, Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -NHC(O)R^{k}, -C(O)NHR^{m}, -C(=NOR)R', - C(=NOH)NH₂, E-1 to E-4, E-13, E-14, E-25, E-28, E-37, E-38, E-41 or E-55,
   in a case where W is -N(R^{W})-, Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, - OR^{j} or E-1,
   R represents C₁-C₆ alkyl,
   R' represents C₁-C₆ alkyl,
   Rⁱ represents C₂-C₆ alkenyl, C₂-C₆ alkynyl, C₁-C₆ alkyl substituted by R⁴ C₁-C₆ alkoxycarbonyl or E-1,
   R⁴ represents C₁-C₆ alkoxy, C₃-C₁₀ cycloalkyl, E-1, E-2 or E-3,
   R⁵ represents C₁-C₆ alkoxy,
   R^{m} represents C₁-C₆ alkyl, C₃-C₁₀ cycloalkyl, C₂-C₆ alkenyl, C₁-C₆ alkoxy, benzyl, E-1 or E-28,
   Z^{a} represents a halogen atom, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ alkoxycarbonyl, phenyl, S-1, S-4 or S-6,
   m5 represents an integer of 0, 1 or 2,
   n3 represents an integer of 0 or 1,
   n4 represents an integer of 1 or 2,
   n5 represents an integer of 0, 1, 2 or 3,
   n6 represents an integer of 0,
   n7 represents an integer of 0 or 1,
   u4 represents an integer of 0,
   u5 represents an integer of 0,
   t5 represents an integer of 1,
   v2 represents an integer of 1,
   v3 represents an integer of 0,
   v4 represents an integer of 0 or 1,
   v5 represents an integer of 0 or 1,
   w4 represents an integer of 0, and
   w5 represents an integer of 0.
<3> The pyrazole compound described in <2> or a salt thereof, in which:
   R¹ represents a hydrogen atom,
   R^{W} represents C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl or C₁-C₆ alkylaminothiocarbonyl,
   in a case where W is -O-, Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -C(=NOH)NH₂, E-1 to E-4, E-13, E-14, E-25, E-28, E-37, E-38, E-41 or E-55,
   in a case where W is -N(R^{W})-, Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, - ORⱼ or E-1,
   Rⁱ represents C₂-C₆ alkenyl, C₂-C₆ alkynyl, C₁-C₆ alkyl substituted by R⁴ or E-1, and p represents an integer of 1.
<4> The pyrazole compound described in <1> or a salt thereof, in which: W represents -O-.
<5> The pyrazole compound described in <4> or a salt thereof, in which:
   G¹ represents a halogen atom, C₁-C₆ alkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1, R^{X}-2 or R^{X}-3,
   R^{Y} represents a hydrogen atom or a halogen atom,
   R¹ represents a hydrogen atom,
   R² represents a hydrogen atom or C₁-C₆ alkyl,
   R³ represents a hydrogen atom,
   Z represents Z-1, Z-2, Z-3, Z-4, Z-6, Z-8, Z-9, Z-17, Z-19, Z-25, Z-27, Z-28, Z-34, Z-44, Z-45, Z-46, Z-47, Z-48, Z-49 or Z-50,
   Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -NHC(O)R^{k},-C(O)NHR^{m}, -C(=NOR)R', -C(=NOH)NH₂, E-1 to E-4, E-13, E-14, E-25, E-28, E-37, E-38, E-41 or E-55,
   R represents C₁-C₆ alkyl,
   R' represents C₁-C₆ alkyl,
   Rⁱ represents C₂-C₆ alkenyl, C₂-C₆ alkynyl, C₁-C₆ alkyl substituted by R⁴ or E-1,
   R⁴ represents C₁-C₆ alkoxy, C₃-C₁₀ cycloalkyl, E-1, E-2 or E-3,
   R^{k} represents C₃-C₁₀ cycloalkyl, C₁-C₆ haloalkyl, C₁-C₆ alkyl substituted by R⁵, C₁-C₆ alkoxy, E-1, E-2 or E-3,
   R⁵ represents C₁-C₆ alkoxy,
   R^{m} represents C₁-C₆ alkyl, C₃-C₁₀ cycloalkyl, C₂-C₆ alkenyl, C₁-C₆ alkoxy, benzyl, E-1 or E-28,
   Z^{a} represents a halogen atom, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ alkoxycarbonyl, phenyl, S-1, S-4 or S-6,
   m5 represents an integer of 0, 1 or 2,
   n2 represents an integer of 0 or 1,
   n3 represents an integer of 0 or 1,
   n4 represents an integer of 1 or 2,
   n5 represents an integer of 0, 1 or 2,
   n6 represents an integer of 0,
   n7 represents an integer of 0 or 1,
   u4 represents an integer of 0,
   u5 represents an integer of 0,
   v2 represents an integer of 1,
   v3 represents an integer of 0,
   v4 represents an integer of 0 or 1, and
   v5 represents an integer of 0 or 1.
<6> The pyrazole compound described in <5> or a salt thereof, in which:
   Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylcarbonyl, C₁-C₆ alkoxycarbonyl, -OR^{j}, -C(=NOH)NH₂, E-1 to E-4, E-13, E-14, E-25, E-28, E-37, E-38, E-41 or E-55, and
   p represents an integer of 1.
<7> The pyrazole compound described in <1> or a salt thereof, in which: W represents -N(R^{W})-.
<8> The pyrazole compound described in <7> or a salt thereof, in which:
   G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1, R^{X}-2, R^{X}-3 or R^{X}-4,
   R^{Y} represents a hydrogen atom or a halogen atom,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
   R² represents a hydrogen atom, C₁-C₆ alkyl or C₁-C₆ alkyl substituted by R^{h},
   R³ represents a hydrogen atom,
   R^{a} represents hydroxy, cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, - C(N=OR^{b})R^{c} or Q-¹,
   R^{D} represents a halogen atom or nitro,
   R^{e} represents C₁-C₆ alkoxy,
   R^{g} represents Q-1, Q-2, Q-3, Q-4 or Q-11,
   R^{h} represents C₁-C₆ alkoxy,
   Rⁱ represents cyano,
   Z represents Z-1, Z-4, Z-8, Z-9, Z-17, Z-19 or Z-27,
   Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, -OR^{j} or E-1,
   Z² represents C₁-C₆ alkyl,
   R^{j} represents C₁-C₆ alkyl substituted by R⁴ or C₁-C₆ alkoxycarbonyl,
   R⁴ represents E-2,
   Z^{a} represents C₁-C₆ alkyl or C₁-C₆ haloalkyl,
   m5 represents an integer of 0, 1 or 2,
   n2 represents an integer of 0 or 2,
   n3 represents an integer of 1,
   n4 represents an integer of 1,
   n5 represents an integer of 1, 2 or 3,
   u4 represents an integer of 0,
   u5 represents an integer of 0,
   t5 represents an integer of 1,
   v4 represents an integer of 1,
   v5 represents an integer of 0,
   w4 represents an integer of 0, and
   w5 represents an integer of 0.
<9> The pyrazole compound described in <8> or a salt thereof, in which:
   R¹ represents a hydrogen atom,
   R^{W} represents C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl or C₁-C₆ alkylaminothiocarbonyl, and
   p represents an integer of 1.
<10> The pyrazole compound described in any one of <1> through <9> or a salt thereof, in which: R¹, R² and R³ each represent a hydrogen atom.
<11> The pyrazole compound described in any one of <1> through <3> and <7> through <10> or a salt thereof, in which:
   W represents -N(R^{W})-,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₃-C₁₀ cycloalkylcarbonyl, C₁-C₆ alkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl, C₁-C₆ alkylaminothiocarbonyl, C₁-C₆ alkylsulfonyl, -C(O)OR^{d} or -C(O)R^{g},
   R^{a} represents cyano, C₃-C₁₀ cycloalkyl, C₁-C₆ alkoxy, C₁-C₆ alkylcarbonyl, C₁-C₆ alkoxycarbonyl, -C(N=OR^{b})R^{c} or Q-1,
   R^{D} represents a halogen atom, and
   R^{g} represents Q-1, Q-2, Q-3, Q-4 or Q-11.
<12> The pyrazole compound described in <11> or a salt thereof, in which:
   R^{W} represents a hydrogen atom, C₁-C₆ alkyl, C₁-C₆ haloalkyl, C₂-C₆ alkenyl, C₁-C₆ alkylcarbonyl, C₁-C₆ haloalkylcarbonyl, C₃-C₁₀ cycloalkylcarbonyl, C₁-C₆ alkoxycarbonyl, C₁-C₆ alkoxycarbonyl substituted by R^{a}, C₂-C₆ alkenyloxycarbonyl or -C(O)R^{g},
   R^{a} represents cyano, C₃-C₁₀ cycloalkyl or Q-1, and
   R^{g} represents Q-1 or Q-11.
<13> The pyrazole compound described in <12> or a salt thereof, in which: R^{W} represents a hydrogen atom, C₁-C₆ alkyl, C₁-C₆ alkoxycarbonyl or C₂-C₆ alkenyloxycarbonyl.
<14> The pyrazole compound described in <13> or a salt thereof, in which: R^{W} represents a hydrogen atom, C₁-C₆ alkoxycarbonyl or C₂-C₆ alkenyloxycarbonyl.
<15> The pyrazole compound described in any one of <1> through <14> or a salt thereof, in which: R^{X} represents C₁-C₆ alkyl.
<16> The pyrazole compound described in <15> or a salt thereof, in which: R^{X} represents C₄ alkyl.
<17> The pyrazole compound described in any one of <1> through <14> or a salt thereof, in which: R^{X} represents R^{X}-1, R^{X}-2, R^{X}-3 or R^{X}-4,
<18> The pyrazole compound described in any one of <1> through <17> or a salt thereof, in which: R^{Y} represents a hydrogen atom.
<19> The pyrazole compound described in any one of <1> through <17> or a salt thereof, in which: R^{Y} represents a halogen atom.
<20> The pyrazole compound described in any one of <1> through <19> or a salt thereof, in which:
   G¹ represents a halogen atom, C₁-C₆ alkyl, C₃-C₆ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino, and
   m5 represents an integer of 0, 1 or 2.
<21> The pyrazole compound described in <20> or a salt thereof, in which:
   G¹ represents a halogen atom, C₁-C₆ alkyl, C₁-C₆ haloalkyl, C₃-C₆ cycloalkyl or C₁-C₆ alkoxy, and
   m5 represents an integer of 0 or 1.
<22> The pyrazole compound described in <21> or a salt thereof, in which: G¹ represents a halogen atom, C₁-C₆ alkyl or C₁-C₆ haloalkyl.
<23> The pyrazole compound described in <22> or a salt thereof, in which: G¹ represents C₁-C₆ alkyl or C₁-C₆ haloalkyl.
<24> The pyrazole compound described in any one of <1> through <19> or a salt thereof, in which: m5 represents an integer of 0.
<25> The pyrazole compound described in any one of <1> through <23> or a salt thereof, in which:
   G¹ represents C₁-C₆ haloalkyl, and
   m5 represents an integer of 1.
<26> The pyrazole compound described in any one of <1> through <22> or a salt thereof, in which:
   G¹ represents a halogen atom, and
   m5 represents an integer of 1 or 2.
<27> The pyrazole compound described in any one of <1> through <23> or a salt thereof, in which:
   G¹ represents C₁-C₆ alkyl, and
   m5 represents an integer of 1.
<28> The pyrazole compound described in <20> or a salt thereof, in which:
   G¹ represents a halogen atom, C₃-C₆ cycloalkyl or C₁-C₆ alkoxy, and
   m5 represents an integer of 2.
<29> The pyrazole compound described in any one of <1> through <28> or a salt thereof, in which: Z represents Z-1, Z-3, Z-4, Z-8, Z-9, Z-17, Z-19, Z-25, Z-27, Z-46, Z-47, Z-49 or Z-50.
<30> The pyrazole compound described in <29> or a salt thereof, in which: Z represents Z-1, Z-8 or Z-25.
<31> The pyrazole compound described in <30> or a salt thereof, in which: Z represents Z-1.
<32> The pyrazole compound described in any one of <1> through <31> or a salt thereof, in which: Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, E-1, E-37 or E-38.
<33> The pyrazole compound described in <32> or a salt thereof, in which: Z¹ represents cyano, a halogen atom, C₁-C₆ alkyl, C₁-C₆ alkoxy or C₁-C₆ haloalkoxy.
<34> The pyrazole compound described in <33> or a salt thereof, in which: Z¹ represents a halogen atom, C₁-C₆ alkyl or C₁-C₆ alkoxy.
<35> The pyrazole compound described in any one of <1> through <28> or a salt thereof, in which:
   W represents -O-,
   Z represents Z-1,
   Z¹ represents C₁-C₆ haloalkoxy, -OR^{j}, -C(=NOH)NH₂, E-1, E-2, E-3, E-4, E-13, E-14, E-25, E-28, E-37, E-38, E-41 or E-55,
   R^{j} represents C₁-C₆ alkyl substituted by R⁴,
   R⁴ represents E-2 or E-3,
   Z^{a} represents a halogen atom, C₁-C₆ alkyl, C₁-C₆ alkyl substituted by R⁶, C₃-C₁₀ cycloalkyl or C₁-C₆ haloalkyl, and
   R⁶ represents C₁-C₆ alkoxy.
<36> The pyrazole compound described in <35> or a salt thereof, in which: Z¹ represents C₁-C₆ haloalkoxy, -OR^{j}, - C(=NOH)NH₂, E-28, E-37, E-38 or E-41.
<37> The pyrazole compound described in <36> or a salt thereof, in which: Z¹ represents C₁-C₆ haloalkoxy, -OR^{j} or - C(=NOH)NH₂.
<38> The pyrazole compound described in <36> or a salt thereof, in which: Z¹ represents E-37, E-38 or E-41.
<39> The pyrazole compound described in any one of <1> through <28> or a salt thereof, in which:
   Z represents Z-3, Z-4, Z-8, Z-9, Z-17, Z-19, Z-25, Z-27, Z-46, Z-47, Z-49 or Z-50, and
   Z¹ represents cyano, a halogen atom, C₁-C₆ alkyl, C₁-C₆ alkoxy, E-1, E-2, E-3, E-4, E-13 or E-14.
<40> The pyrazole compound described in <39> or a salt thereof, in which:
   Z represents Z-9, Z-25, Z-46, Z-47, Z-49 or Z-50, and
   Z¹ represents a halogen atom, C₁-C₆ alkyl, C₁-C₆ alkoxy, E-1, E-2, E-3, E-4, E-13 or E-14.
<41> The pyrazole compound described in <40> or a salt thereof, in which:
   Z represents Z-25, and
   Z¹ represents a halogen atom, C₁-C₆ alkyl, C₁-C₆ alkoxy, E-1, E-2, E-3, E-4, E-13 or E-14.
<42> The pyrazole compound described in any one of <1> through <28> or a salt thereof, in which:
   Z represents Z-1, Z-2, Z-3, Z-4, Z-6, Z-8, Z-9, Z-17, Z-19, Z-25, Z-27, Z-28, Z-34, Z-44, Z-45, Z-46, Z-47, Z-48, Z-49 or Z-50, and
   Z¹ represents E-1, E-2, E-3, E-4, E-13, E-14, E-25, E-28, E-37, E-38, E-41 or E-55.
<43> The pyrazole compound described in any one of <1> through <28> or a salt thereof, in which:
   W represents -N(R^{W})-,
   Z represents Z-1,
   Z¹ represents hydroxy, nitro, cyano, a halogen atom, C₁-C₆ alkyl, C₁-C₆ haloalkyl, C₁-C₆ haloalkoxy, C₁-C₆ alkylthio, C₁-C₆ haloalkylthio, C₁-C₆ alkoxycarbonyl or E-1.
<44> The pyrazole compound described in <43> or a salt thereof, in which: Z¹ represents cyano, a halogen atom, C₁-C₆ alkyl, C₁-C₆ haloalkyl or C₁-C₆ haloalkoxy.
<45> The pyrazole compound described in <44> or a salt thereof, in which: Z¹ represents a halogen atom, C₁-C₆ alkyl or C₁-C₆ haloalkyl.
<46> The pyrazole compound described in any one of <1> through <44> or a salt thereof, in which: Z² represents C₁-C₆ alkyl or phenyl.
<47> The pyrazole compound described in <46> or a salt thereof, in which: Z² represents C₁-C₆ alkyl.
<48> The pyrazole compound described in <1> or a salt thereof, in which:
   W represents -O-,
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1 to R^{X}-3 or R^{X}-4,
   X¹ represents a halogen atom, C₁-C₆ alkyl, C₁-C₆ haloalkyl or C₁-C₆ alkoxy,
   in a case where u4 represents an integer of 2, 3 or 4 in relationship to X¹, the respective X¹ may be identical with each other or may be different from each other,
   in a case where u5 represents an integer of 2, 3, 4 or 5 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   Z represents Z-1 to Z-6 or Z-7,
   Z¹ represents hydroxy, amino, nitro, cyano, a halogen atom, C₁-C₆ alkyl, C₃-C₁₀ cycloalkyl, C₁-C₆ haloalkyl, C₁-C₆ alkoxy, C₁-C₆ alkylthio, C₁-C₆ alkylsulfinyl, C₁-C₆ alkylsulfonyl or C₁-C₆ alkoxycarbonyl,
   in a case where n5 represents an integer of 2, 3, 4 or 5 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   m5, n5 and u5 each represent an integer of 0, 1, 2, 3, 4 or 5,
   u4 represents an integer of 0, 1, 2, 3 or 4,
   n7 represents an integer of 0, 1, 2, 3, 4, 5, 6 or 7, and
   p represents an integer of 0 or 1.
<49> The pyrazole compound described in <48> or a salt thereof, in which:
   G¹ represents a halogen atom, C₁-C₆ haloalkyl, C₁-C₆ alkoxy or C₁-C₆ haloalkoxy,
   R^{X} represents C₁-C₆ alkyl,
   R^{Y} represents a hydrogen atom,
   R¹ represents a hydrogen atom,
   R² represents a hydrogen atom,
   R³ represents a hydrogen atom,
   Z represents Z-1,
   Z¹ represents a halogen atom, C₁-C₆ alkyl or C₁-C₆ haloalkyl,
   m5 represents an integer of 0, 1 or 2,
   n5 represents an integer of 0, 1 or 2, and
   p represents an integer of 1.
<50> The pyrazole compound described in <1> or a salt thereof, in which:
   W represents -O-,
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1 to R^{X}-3 or R^{X}-4,
   X¹ represents a halogen atom, C₁-C₆ alkyl, C₁-C₆ haloalkyl or C₁-C₆ alkoxy,
   in a case where u4 represents an integer of 2, 3 or 4 in relationship to X¹, the respective X¹ may be identical with each other or may be different from each other,
   in a case where u5 represents an integer of 2, 3, 4 or 5 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   Z represents Z-1 to Z-6 or Z-7,
   Z¹ represents hydroxy, amino, nitro, cyano, a halogen atom, C₁-C₆ alkyl, C₃-C₁₀ cycloalkyl, C₁-C₆ haloalkyl, C₁-C₆ alkoxy, C₁-C₆ alkylthio, C₁-C₆ alkylsulfinyl, C₁-C₆ alkylsulfonyl or C₁-C₆ alkoxycarbonyl,
   in a case where n5 represents an integer of 2, 3, 4 or 5 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   m5, n5 and u5 each represent an integer of 0, 1, 2, 3, 4 or 5,
   u4 represents an integer of 0, 1, 2, 3 or 4,
   n7 represents an integer of 0, 1, 2, 3, 4, 5, 6 or 7, and
   p represents an integer of 0 or 1.
<51> The pyrazole compound described in <50> or a salt thereof, in which:
   G¹ represents a halogen atom, C₁-C₆ alkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1, R^{X}-2 or R^{X}-3,
   R^{Y} represents a hydrogen atom,
   R¹ represents a hydrogen atom,
   R² represents a hydrogen atom,
   R³ represents a hydrogen atom,
   Z represents Z-1, Z-3 or Z-4,
   Z¹ represents nitro, cyano, a halogen atom, C₁-C₆ alkyl, C₁-C₆ haloalkyl, C₁-C₆ alkoxy or C₁-C₆ alkoxycarbonyl,
   m5 represents an integer of 0, 1 or 2,
   n5 represents an integer of 0, 1 or 2,
   u5 represents an integer of 0,
   u4 represents an integer of 0,
   n7 represents an integer of 0, and
   p represents an integer of 1.
<52> The pyrazole compound described in <1> or a salt thereof, in which:
   W represents -O-,
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1 to R^{X}-3 or R^{X}-4,
   X¹ represents a halogen atom, C₁-C₆ alkyl, C₁-C₆ haloalkyl or C₁-C₆ alkoxy,
   in a case where u4 represents an integer of 2, 3 or 4 in relationship to X¹, the respective X¹ may be identical with each other or may be different from each other,
   in a case where u5 represents an integer of 2, 3, 4 or 5 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R² represents a hydrogen atom or C₁-C₆ alkyl,
   R³ represents a hydrogen atom or C₁-C₆ alkyl,
   Z represents Z-1 to Z-44 or Z-45,
   Z¹ represents hydroxy, carboxy, amino, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl, C₁-C₆ alkylcarbonyl, C₁-C₆ alkoxycarbonyl, -OR^{j}, -NHC(O)R^{k}, - C(O)NHR^{m}, -C(=NOR)R', -C(=NOH)NH₂, E-1 to E-53 or E-54,
   in a case where n2 represents an integer of 2 in relationship to Z¹, the respective Z¹ may be identical with each other or may be different from each other,
   in a case where n3 represents an integer of 2 or 3 in relationship to Z¹, the respective Z¹ may be identical with each other or may be different from each other,
   in a case where n4 represents an integer of 2, 3 or 4 in relationship to Z¹, the respective Z¹ may be identical with each other or may be different from each other,
   in a case where n5 represents an integer of 2, 3, 4 or 5 in relationship to Z¹, the respective Z¹ may be identical with each other or may be different from each other,
   in a case where n6 represents an integer of 2, 3, 4, 5 or 6 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   Z² represents C₁-C₆ alkyl or phenyl,
   R represents a hydrogen atom or C₁-C₆ alkyl,
   R' represents a hydrogen atom or C₁-C₆ alkyl,
   R^{j} represents C₁-C₆ alkyl substituted by R⁴, C₂-C₆ alkenyl, C₂-C₆ alkynyl, E-1 to E-53 or E-54,
   R⁴ represents cyano, C₁-C₆ alkoxy, E-1 to E-53 or E-54,
   R^{k} represents C₁-C₆ alkyl, C₃-C₁₀ cycloalkyl, C₁-C₆ haloalkyl, C₁-C₆ alkyl substituted by R⁵, C₁-C₆ alkoxy, C₂-C₆ alkenyloxy, C₁-C₆ alkylamino, di(C₁-C₆ alkyl)amino, benzyloxy, E-1 to E-53 or E-54,
   R⁵ represents cyano, C₁-C₆ alkoxy, E-1 to E-53 or E-54,
   R^{m} represents C₁-C₆ alkyl, C₃-C₁₀ cycloalkyl, C₂-C₆ alkenyl, C₁-C₆ alkoxy, benzyl, E-1 to E-53 or E-54,
   Z^{a} represents a halogen atom, cyano, C₁-C₆ alkyl, C₃-C₁₀ cycloalkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where v5 represents an integer of 2, 3, 4 or 5 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   in a case where v3 represents an integer of 2 or 3 in relationship to Z^{a}, the respective Z^{a} may be identical with each other or may be different from each other,
   in a case where v2 represents an integer of 2 in relationship to Z^{a}, the respective Z^{a} may be identical with each other or may be different from each other,
   Z^{b} represents C₁-C₆ alkyl,
   m5 represents an integer of 0, 1, 2, 3, 4 or 5,
   n6 represents an integer of 0, 1, 2, 3, 4, 5 or 6,
   n5 represents an integer of 0, 1, 2, 3, 4 or 5,
   n4 represents an integer of 0, 1, 2, 3 or 4,
   n3 represents an integer of 0, 1, 2 or 3,
   n2 represents an integer of 0, 1 or 2,
   u5 represents an integer of 0, 1, 2, 3, 4 or 5,
   u4 represents an integer of 0, 1, 2, 3 or 4,
   n7 represents an integer of 0, 1, 2, 3, 4, 5, 6 or 7,
   p represents an integer of 0 or 1,
   v5 represents an integer of 0, 1, 2, 3, 4 or 5,
   v4 represents an integer of 0, 1, 2, 3 or 4,
   v3 represents an **integer** of 0, 1, 2 or 3,
   v2 represents an **integer** of 0, 1 or 2, and
   v1 represents an integer of 0 or 1.
<53> The pyrazole compound described **in** <52> or a salt thereof, **in** which:
   G¹ represents a halogen atom, C₁-C₆ alkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1, R^{X}-2 or R^{X}-3,
   R^{Y} represents a hydrogen atom or a halogen atom,
   R¹ represents a hydrogen atom,
   R³ represents a hydrogen atom,
   Z represents Z-1, Z-2, Z-3, Z-4, Z-6, Z-8, Z-9, Z-17, Z-19, Z-25, Z-27, Z-34, Z-44 or Z-45,
   Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylcarbonyl, C₁-C₆ alkoxycarbonyl, -OR^{j}, -NHC(O)R^{k}, -C(O)NHR^{m}, -C(=NOR)R', - C(=NOH)NH₂, E-1 to E-4, E-13, E-14, E-37, E-38 or E-41,
   Z² represents phenyl,
   R represents C₁-C₆ alkyl,
   R' represents C₁-C₆ alkyl,
   R^{j} represents C₁-C₆ alkyl substituted by R⁴,
   R^{k} represents C₃-C₁₀ cycloalkyl, C₁-C₆ haloalkyl, C₁-C₆ alkyl substituted by R⁵, C₁-C₆ alkoxy, E-1, E-2 or E-3,
   R⁴ represents E-2 or E-3,
   R⁵ represents C₁-C₆ alkoxy,
   R^{m} represents C₁-C₆ alkyl, C₃-C₁₀ cycloalkyl, C₂-C₆ alkenyl, C₁-C₆ alkoxy, benzyl, E-1 or E-28,
   Z^{a} represents a halogen atom, C₁-C₆ alkyl, C₃-C₁₀ cycloalkyl, C₁-C₆ haloalkyl or C₁-C₆ alkoxy,
   m5 represents an integer of 0, 1 or 2,
   n6 represents an integer of 0,
   n5 represents an integer of 0, 1 or 2,
   n4 represents an integer of 1 or 2,
   n3 represents an integer of 0 or 1,
   n2 represents an integer of 0 or 1,
   u5 represents an integer of 0,
   u4 represents an integer of 0,
   n7 represents an integer of 0 or 1,
   v5 represents an integer of 0 or 1,
   v4 represents an integer of 0 or 1,
   v3 represents an integer of 0,
   v2 represents an integer of 1, and
   v1 represents an integer of 1.
<54> The pyrazole compound described in <1> or a salt thereof, in which:
   W represents -N(R^{W})-,
   G¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   X¹ represents a halogen atom or C₁-C₆ alkyl,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom or C₁-C₆ alkyl,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, di(C₁-C₆ alkyl)aminothiocarbonyl, C₁-C₆ alkylsulfonyl, - C(O)OR^{d} or -C(O)C(O)R^{e} or -C(O)SR^{f},
   R² represents a hydrogen atom, C₁-C₆ alkyl or C₁-C₆ haloalkyl,
   R³ represents a hydrogen atom, C₁-C₆ alkyl or C₁-C₆ haloalkyl,
   R^{a} represents cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, phenyl or -C(N=OR^{b})R^{c},
   R^{b} represents C₁-C₆ alkyl,
   R^{c} represents C₁-C₆ alkyl,
   R^{D} represents a halogen atom, nitro or C₁-C₆ alkyl,
   **in** a case where t5 represents an **integer** of 2, 3, 4 or 5 **in** relationship to R^{D}, the respective R^{D} may be identical with each other or may be different from each other,
   R^{e} represents C₁-C₆ alkyl or C₁-C₆ alkoxy,
   R^{f} represents C₁-C₆ alkyl,
   Z represents Z-1 to Z-6 or Z-7,
   Z¹ represents hydroxy, amino, nitro, cyano, a halogen atom, C₁-C₆ alkyl, C₃-C₁₀ cycloalkyl, C₁-C₆ haloalkyl, C₁-C₆ alkoxy, C₁-C₆ alkylthio, C₁-C₆ alkylsulfinyl, C₁-C₆ alkylsulfonyl or C₁-C₆ alkoxycarbonyl,
   in a case where n5 represents an integer of 2, 3, 4 or 5 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   m5, n5 and u5 each represent an integer of 0, 1, 2, 3, 4 or 5,
   u4 represents an integer of 0, 1, 2, 3 or 4,
   n7 represents an integer of 0, 1, 2, 3, 4, 5, 6 or 7,
   t5 represents an integer of 0, 1, 2, 3, 4 or 5, and
   p represents an integer of 0 or 1.
<55> The pyrazole compound described in <54> or a salt thereof, in which:
   G¹ represents a halogen atom, C₁-C₆ alkyl, C₁-C₆ haloalkyl, C₁-C₆ alkoxy or C₁-C₆ haloalkoxy,
   R^{X} represents C₁-C₆ alkyl,
   R^{Y} represents a hydrogen atom,
   R¹ represents a hydrogen atom,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, C₁-C₆ alkylsulfonyl, -C(O)OR^{d}, -C(O)C(O)R^{e} or -C(O)SR^{f},
   R² represents a hydrogen atom,
   R³ represents a hydrogen atom,
   R^{a} represents cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, phenyl or -C(N=OR^{b})R^{c},
   R^{D} represents a halogen atom or nitro,
   R^{e} represents C₁-C₆ alkoxy,
   Z represents Z-1,
   Z¹ represents a halogen atom, C₁-C₆ alkyl or C₁-C₆ alkoxy,
   m5 represents an integer of 0, 1 or 2,
   n5 represents an integer of 1 or 2, and
   t5 represents an integer of 1.
<56> The pyrazole compound described in <1> or a salt thereof, in which:
   W represents -N(R^{W})-,
   G¹ represents hydroxy, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom, C₁-C₆ alkyl or C₁-C₆ alkyl substituted by Rⁱ,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, di(C₁-C₆ alkyl)aminothiocarbonyl, C₁-C₆ alkylsulfonyl, - C(O)OR^{d} or -C(O)C(O)R^{e}, -C(O)SR^{f}, -C(O)R^{g}, Q-1 through Q-53 or Q-54,
   R² represents a hydrogen atom, C₁-C₆ alkyl, C₁-C₆ haloalkyl or C₁-C₆ alkyl substituted by R^{h},
   R³ represents 3, a hydrogen atom, C₁-C₆ alkyl or C₁-C₆ haloalkyl,
   R^{a} represents cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, -C(N=OR^{b})R^{c}, Q-1 through Q-53 or Q-54,
   R^{b} represents C₁-C₆ alkyl,
   R^{c} represents C₁-C₆ alkyl,
   R^{D} represents a halogen atom, nitro or C₁-C₆ alkyl,
   **in** a case where t5 represents an **integer** of 2, 3, 4 or 5 **in** relationship to R^{D}, the respective R^{D} may be identical with each other or may be different from each other,
   R^{e} represents C₁-C₆ alkyl or C₁-C₆ alkoxy,
   R^{f} represents C₁-C₆ alkyl,
   R^{e} represents Q-1 to Q-53 or Q-54,
   R^{h} represents cyano or C₁-C₆ alkoxy,
   Rⁱ represents cyano or C₁-C₆ alkoxy,
   Z represents Z-1 to Z-6 or Z-7,
   Z¹ represents hydroxy, amino, nitro, cyano, a halogen atom, C₁-C₆ alkyl, C₃-C₁₀ cycloalkyl, C₁-C₆ haloalkyl, C₁-C₆ alkoxy, C₁-C₆ alkylthio, C₁-C₆ haloalkylthio, C₁-C₆ alkylsulfinyl, C₁-C₆ alkylsulfonyl, C₁-C₆ alkoxycarbonyl, - OR^{j}, E-1 to E-53 or E-54,
   in a case where n3 represents an integer of 2 or 3 in relationship to Z¹, the respective Z¹ may be identical with each other or may be different from each other,
   in a case where n5 represents an integer of 2, 3, 4 or 5 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   R^{j} represents C₂-C₆ alkenyl, C₂-C₆ alkynyl, C₁-C₆ haloalkyl, C₁-C₆ alkyl substituted by R⁴, C₁-C₆ alkylcarbonyl, C₁-C₆ alkoxycarbonyl, C₁-C₆ alkylaminocarbonyl, E-1 to E-53 or E-54,
   R⁴ represents C₃-C₁₀ cycloalkyl, C₁-C₆ alkylcarbonyl, C₁-C₆ alkoxycarbonyl, di(C₁-C₆ alkyl)aminocarbonyl, E-1 to E-53 or E-54,
   Z^{a} represents a halogen atom, cyano, C₁-C₆ alkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where v5 represents an integer of 2, 3, 4 or 5 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   in a case where v3 represents an integer of 2 or 3 in relationship to Z^{a}, the respective Z^{a} may be identical with each other or may be different from each other,
   in a case where v2 represents an integer of 2 in relationship to Z^{a}, the respective Z^{a} may be identical with each other or may be different from each other,
   Z^{b} represents C₁-C₆ alkyl,
   Z^{c} represents a halogen atom, cyano, C₁-C₆ alkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where w5 represents an integer of 2, 3, 4 or 5 in relationship to Z^{c}, the respective Z^{c} may be identical with each other or may be different from each other,
   in a case where w4 represents an integer of 2, 3 or 4 in relationship to Z^{c}, the respective Z^{c} may be identical with each other or may be different from each other,
   in a case where w3 represents an integer of 2 or 3 in relationship to Z^{c}, the respective Z^{c} may be identical with each other or may be different from each other,
   in a case where w2 represents an integer of 2 in relationship to Z^{c}, the respective Z^{c} may be identical with each other or may be different from each other,
   Z^{d} represents C₁-C₆ alkyl,
   n3 represents an integer of 0, 1, 2 or 3,
   m5, n5 and u5 each represent an integer of 0, 1, 2, 3, 4 or 5,
   u4 represents an integer of 0, 1, 2, 3 or 4,
   n7 represents an integer of 0, 1, 2, 3, 4, 5, 6 or 7,
   t5 represents an integer of 0, 1, 2, 3, 4 or 5,
   p represents an integer of 0 or 1,
   v1 represents an integer of 0 or 1,
   v2 represents an integer of 0, 1 or 2,
   v3 represents an integer of 0, 1, 2 or 3,
   v4 represents an integer of 0, 1, 2, 3 or 4,
   v5 represents an integer of 0, 1, 2, 3, 4 or 5,
   w1 represents an integer of 0 or 1,
   w2 represents an integer of 0, 1 or 2,
   w3 represents an integer of 0, 1, 2 or 3,
   w4 represents an integer of 0, 1, 2, 3 or 4, and
   w5 represents an integer of 0, 1, 2, 3, 4 or 5.
<57> The pyrazole compound described in <56> or a salt thereof, in which:
   G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1 to R^{X}-3 or R^{X}-4,
   R^{Y} represents a hydrogen atom,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
   R² represents a hydrogen atom, C₁-C₆ alkyl or C₁-C₆ alkyl substituted by R^{h},
   R³ represents a hydrogen atom,
   R^{a} represents cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, - C(N=OR^{b})R^{c} or Q-1,
   R^{D} represents a halogen atom or nitro,
   R^{e} represents C₁-C₆ alkoxy,
   R^{g} represents Q-2, Q-3 or Q-4,
   R^{h} represents C₁-C₆ alkoxy,
   Rⁱ represents cyano,
   Z represents Z-1 or Z-4,
   Z¹ represents hydroxy, nitro, cyano, a halogen atom, C₁-C₆ alkyl, C₁-C₆ haloalkyl, C₁-C₆ alkoxy, C₁-C₆ alkylthio, C₁-C₆ haloalkylthio, -OR^{j} or E-1,
   R^{j} represents C₁-C₆ haloalkyl, C₁-C₆ alkyl substituted by R⁴ or C₁-C₆ alkoxycarbonyl,
   R⁴ represents E-2,
   Z^{a} represents C₁-C₆ alkyl,
   m5 represents an integer of 0, 1 or 2,
   n3 represents an integer of 1,
   n5 represents an integer of 1, 2 or 3,
   u5 represents an integer of 0,
   u4 represents an integer of 0,
   t5 represents an integer of 1,
   v4 represents an integer of 1,
   v5 represents an integer of 0,
   w4 represents an integer of 0, and
   w5 represents an integer of 0.
<58> The pyrazole compound described in <1> or a salt thereof, in which:
   W represents -N(R^{W})-,
   G¹ represents hydroxy, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   in a case where m5 represents an integer of 2, 3, 4 or 5 in relationship to G¹, the respective G¹ may be identical with each other or may be different from each other,
   R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
   X¹ represents a halogen atom, C₁-C₆ alkyl, C₁-C₆ haloalkyl or C₁-C₆ alkoxy,
   in a case where u5 represents an integer of 2, 3, 4 or 5 in relationship to X¹, the respective X¹ may be identical with each other or may be different from each other,
   in a case where u4 represents an integer of 2, 3 or 4 in relationship to X¹, the respective X¹ may be identical with each other or may be different from each other,
   R^{Y} represents a hydrogen atom, a halogen atom or C₁-C₆ alkyl,
   R¹ represents a hydrogen atom, C₁-C₆ alkyl or C₁-C₆ alkyl substituted by Rⁱ,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, di(C₁-C₆ alkyl)aminothiocarbonyl, C₁-C₆ alkylsulfonyl, - C(O)OR^{d} or -C(O)C(O)R^{e}, -C(O)SR^{f}, -C(O)R^{g}, Q-1 through Q-53 or Q-54,
   R² represents a hydrogen atom, C₁-C₆ alkyl, C₁-C₆ haloalkyl or C₁-C₆ alkyl substituted by R^{h},
   R³ represents a hydrogen atom, C₁-C₆ alkyl or C₁-C₆ haloalkyl,
   R^{a} represents cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, -C(N=OR^{b})R^{c}, Q-1 through Q-53 or Q-54,
   R^{b} represents C₁-C₆ alkyl,
   R^{c} represents C₁-C₆ alkyl,
   R^{D} represents a halogen atom, nitro or C₁-C₆ alkyl,
   in a case where t5 represents an integer of 2, 3, 4 or 5 in relationship to R^{D}, the respective R^{D} may be identical with each other or may be different from each other,
   R^{e} represents C₁-C₆ alkyl or C₁-C₆ alkoxy,
   R^{f} represents C₁-C₆ alkyl,
   R^{g} represents Q-1 to Q-53 or Q-54,
   R^{h} represents cyano or C₁-C₆ alkoxy,
   Rⁱ represents cyano or C₁-C₆ alkoxy,
   Z represents Z-1 to Z-44 or Z-45,
   Z¹ represents hydroxy, carboxy, amino, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ alkylthio, C₁-C₆ haloalkylthio, C₁-C₆ alkylsulfinyl, C₁-C₆ alkylsulfonyl, C₁-C₆ alkylcarbonyl, C₁-C₆ alkoxycarbonyl, -OR^{j}, E-1 to E-53 or E-54,
   in a case where n2 represents an integer of 2 in relationship to Z¹, the respective Z¹ may be identical with each other or may be different from each other,
   in a case where n3 represents an integer of 2 or 3 in relationship to Z¹, the respective Z¹ may be identical with each other or may be different from each other,
   in a case where n4 represents an integer of 2, 3 or 4 in relationship to Z¹, the respective Z¹ may be identical with each other or may be different from each other,
   in a case where n5 represents an integer of 2, 3, 4 or 5 in relationship to Z¹, the respective Z¹ may be identical with each other or may be different from each other,
   in a case where n6 represents an integer of 2, 3, 4, 5 or 6 in relationship to Z¹, the respective Z¹ may be identical with each other or may be different from each other,
   in a case where n7 represents an integer of 2, 3, 4, 5, 6 or 7 in relationship to Z¹, the respective Z¹ may be identical with each other or may be different from each other,
   Z² represents C₁-C₆ alkyl or phenyl,
   R^{j} represents C₂-C₆ alkenyl, C₂-C₆ alkynyl, C₁-C₆ haloalkyl, C₁-C₆ alkyl substituted by R⁴, C₁-C₆ alkylcarbonyl, C₁-C₆ alkoxycarbonyl, C₁-C₆ alkylaminocarbonyl, E-1 to E-53 or E-54,
   R⁴ represents C₃-C₁₀ cycloalkyl, C₁-C₆ alkylcarbonyl, C₁-C₆ alkoxycarbonyl, di(C₁-C₆ alkyl)aminocarbonyl, E-1 to E-53 or E-54,
   Z^{a} represents a halogen atom, cyano, C₁-C₆ alkyl, C₃-C₁₀ cycloalkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where v5 represents an integer of 2, 3, 4 or 5 in relationship to Z^{a}, the respective Z^{a} may be identical with each other or may be different from each other,
   in a case where v4 represents an integer of 2, 3 or 4 in relationship to Z^{a}, the respective Z^{a} may be identical with each other or may be different from each other,
   in a case where v3 represents an integer of 2 or 3 in relationship to Z^{a}, the respective Z^{a} may be identical with each other or may be different from each other,
   in a case where v2 represents an integer of 2 in relationship to Z^{a}, the respective Z^{a} may be identical with each other or may be different from each other,
   Z^{b} represents C₁-C₆ alkyl,
   Z^{c} represents a halogen atom, cyano, C₁-C₆ alkyl, C₁-C₆ haloalkyl, C₁-C₆ alkoxy, C₁-C₆ haloalkoxy, C₁-C₆ alkylcarbonyl or C₁-C₆ alkoxycarbonyl,
   in a case where w5 represents an integer of 2, 3, 4 or 5 in relationship to Z^{c}, the respective Z^{c} may be identical with each other or may be different from each other,
   in a case where w4 represents an integer of 2, 3 or 4 in relationship to Z^{c}, the respective Z^{c} may be identical with each other or may be different from each other,
   in a case where w3 represents an integer of 2 or 3 in relationship to Z^{c}, the respective Z^{c} may be identical with each other or may be different from each other,
   in a case where w2 represents an integer of 2 in relationship to Z^{c}, the respective Z^{c} may be identical with each other or may be different from each other,
   Z^{d} represents C₁-C₆ alkyl,
   m5 represents an integer of 0, 1, 2, 3, 4 or 5,
   n2 represents an integer of 0, 1 or 2,
   n3 represents an integer of 0, 1, 2 or 3,
   n4 represents an integer of 0, 1, 2, 3 or 4,
   n5 represents an integer of 0, 1, 2, 3, 4 or 5,
   n6 represents an integer of 0, 1, 2, 3, 4, 5 or 6,
   u4 represents an integer of 0, 1, 2, 3 or 4,
   u5 represents an integer of 0, 1, 2, 3, 4 or 5,
   n7 represents an integer of 0, 1, 2, 3, 4, 5, 6 or 7,
   t5 represents an integer of 0, 1, 2, 3, 4 or 5,
   p represents an integer of 0 or 1,
   v1 represents an integer of 0 or 1,
   v2 represents an integer of 0, 1 or 2,
   v3 represents an integer of 0, 1, 2 or 3,
   v4 represents an integer of 0, 1, 2, 3 or 4,
   v5 represents an integer of 0, 1, 2, 3, 4 or 5,
   w1 represents an integer of 0 or 1,
   w2 represents an integer of 0, 1 or 2,
   w3 represents an integer of 0, 1, 2 or 3,
   w4 represents an integer of 0, 1, 2, 3 or 4, and
   w5 represents an integer of 0, 1, 2, 3, 4 or 5.
<59> The pyrazole compound described in <58> or a salt thereof, in which:
   G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
   R^{X} represents C₁-C₆ alkyl, R^{X}-1 to R^{X}-3 or R^{X}-4,
   R^{Y} represents a hydrogen atom or a halogen atom,
   R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
   R² represents a hydrogen atom, C₁-C₆ alkyl or C₁-C₆ alkyl substituted by R^{h},
   R³ represents a hydrogen atom,
   R^{a} represents cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, - C(N=OR^{b})R^{c} or Q-1,
   R^{D} represents a halogen atom or nitro,
   R^{e} represents C₁-C₆ alkoxy,
   R^{e} represents Q-2, Q-3 or Q-4,
   R^{h} represents C₁-C₆ alkoxy,
   Rⁱ represents cyano,
   Z represents Z-1, Z-4, Z-9 or Z-27,
   Z¹ represents hydroxy, nitro, cyano, a halogen atom, C₁-C₆ alkyl, C₃-C₁₀ cycloalkyl, C₁-C₆ haloalkyl, C₁-C₆ alkoxy, C₁-C₆ alkylthio, C₁-C₆ haloalkylthio, -OR^{j} or E-1,
   Z² represents C₁-C₆ alkyl,
   R^{j} represents C₁-C₆ haloalkyl, C₁-C₆ alkyl substituted by R⁴ or C₁-C₆ alkoxycarbonyl,
   R⁴ represents E-2,
   Z^{a} represents C₁-C₆ alkyl or C₁-C₆ haloalkyl,
   m5 represents an integer of 0, 1 or 2,
   n2 represents an integer of 2,
   n3 represents an integer of 1,
   n4 represents an integer of 1,
   n5 represents an integer of 1, 2 or 3,
   u5 represents an integer of 0,
   u4 represents an integer of 0,
   t5 represents an integer of 1,
   v4 represents an integer of 1,
   v5 represents an integer of 0,
   w4 represents an integer of 0, and
   w5 represents an integer of 0.
<60> A pyrazole compound represented by formula (1a) or a salt thereof: 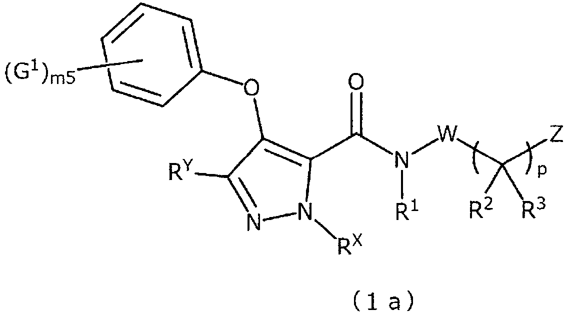 where R^{X}, R^{Y}, G¹, R¹, R², R³, W, Z, p and m5 have the same meanings as those in <1> through <59>.
<61> The pyrazole compound described in any one of <1>, <2>, <4>, <5>, <7>, <8>, <10> through <48>, <50> and <52> through <60> or a salt thereof, in which: p represents an integer of 0.
<62> The pyrazole compound described in any one of <1>, <2>, <4>, <5>, <7>, <8>, <10> through <48>, <50> and <52> through <60> or a salt thereof, in which: p represents an integer of 1.
<63> A pesticide which contains, as an active ingredient, at least one selected from the pyrazole compound described in any one of <1> through <62> and a salt thereof.
<64> A fungicide which contains, as an active ingredient, at least one selected from the pyrazole compound described in any one of <1> through <62> and a salt thereof.
<65> An agricultural-horticultural fungicide which contains, as an active ingredient, at least one selected from the pyrazole compound described in any one of <1> through <62> and a salt thereof.
<66> An antimycotic agent which contains, as an active ingredient, at least one selected from the pyrazole compound described in any one of <1> through <62> and a salt thereof.
<67> An endoparasite control agent which contains, as an active ingredient, at least one selected from the pyrazole compound described in any one of <1> through <62> and a salt thereof.

### Examples

The following description will discuss, as Examples, synthetic examples and test examples of the compound in accordance with the present invention to describe further details of the present invention. Note, however, that the present invention is not limited to those examples.

For medium-pressure preparative liquid chromatography described in the synthetic examples, a medium-pressure preparative apparatus (YFLC-Wprep (flow rate: 18 ml/min, column of 40 µm of silica gel)) available from Yamazen Corporation was used.

A chemical shift value of proton nuclear magnetic resonance spectrum (hereinafter referred to as ¹H-NMR) described below was measured using Me₄Si (tetramethylsilane) as a reference substance at 300 MHz (model: JNM-ECX300 or JNM-ECP300 available from JEOL Ltd.) or at 400 MHz (model: JNM-ECZ400S available from JEOL Ltd.). Note that symbols in the chemical shift value of ¹H-NMR have the following meanings.
s: singlet, d: doublet, dd: double doublet, t: triplet, dt: double triplet, q: quartet, dq: double quartet, sep: septet, m: multiplet, br: broad singlet.

### [Synthesis Examples]

### Synthesis Example 1: Synthesis of 1-(tert-butyl)-N-((4-chlorobenzyl)oxy)-4-(3-iodophenoxy)-1H-pyrazole-5-carboxamide (compound No. 1-006)

To a mixed solution containing 101 mg of 1-(tert-butyl)-4-(3-iodophenoxy)-1H-pyrazole-5-carboxylic acid and 2 ml of N,N-dimethylformamide, 176 mg of 1-[bis(dimethylamino)methylene]-1H-1,2,3-triazolo[4,5-b]pyridinium 3-oxide hexafluorophosphate, 165 µl of N,N-diisopropylethylamine, and 60 mg of O-(4-chlorobenzyl)hydroxylamine hydrochloride were added at room temperature, and a resultant mixture was stirred at the same temperature for 17 hours. After completion of reaction, 2 ml of water was added to the reaction mixture, and extraction was carried out with a mixed solution (3 ml × 1 time) of n-hexane:ethyl acetate = 1:1 (volume ratio, the same applies hereinafter). The obtained organic layer was washed with water and 1 mol/l hydrochloric acid, and then dehydrated and dried with anhydrous sodium sulfate, and then the solvent was evaporated under reduced pressure. The obtained residue was purified by silica gel column chromatography using n-hexane:ethyl acetate [gradient from 9:1 to 1:9 (volume ratio, the same applies hereinafter)] as an eluant, and thus 49 mg of the target compound was obtained as an oil substance.
¹H-NMR (CDCl₃, Me₄Si, 300 MHz): δ8.87 (br, 1H), 7.50-7.40 (m, 1H), 7.33-7.21 (m, 5H), 7.13 (s, 1H), 7.08-6.97 (m, 1H), 6.92-6.82 (m, 1H), 4.89 (s, 2H), 1.71 (s, 9H).

### Synthesis Example 2: Synthesis of 1-(tert-butyl)-N-((4-methylbenzyl)oxy)-4-phenoxy-1H-pyrazole-5-carboxamide (compound No. 1-011)

To a mixed solution containing 50 mg of 1-(tert-butyl)-N-hydroxy-4-phenoxy-1H-pyrazole-5-carboxamide, 34 mg of 1-(bromomethyl)-4-methylbenzene, 27 mg of sodium iodide, and 90 µl of N,N-dimethylformamide, 47 mg of N,N-diisopropylethylamine was added under ice cooling, and a resultant mixture was stirred at the same temperature for 3 hours and then stirred at room temperature for 12 hours. After completion of reaction, 2 ml of water was added to the reaction mixture, and extraction was carried out with a mixed solution (2 ml × 1 time) of n-hexane:ethyl acetate = 1:1. The obtained organic layer was washed with water, and then dehydrated and dried with saturated brine and then with anhydrous sodium sulfate, and then the solvent was evaporated under reduced pressure. The obtained residue was purified by silica gel column chromatography using n-hexane:ethyl acetate (gradient from 9:1 to 1:9) as an eluant, and thus 36 mg of the target compound was obtained as a white solid.
Melting point: 59°C to 61°C.

### Synthesis Example 3: Synthesis of N-(4-(((1-(tert-butyl)-4-(3-(trifluoromethyl)phenoxy)-1H-pyrazole-5-carboxamide)oxy)methyl)phenylpicolinamide (compound No. 2-001)

To a mixed solution containing 110 mg of N-((4-aminobenzyl)oxy)-1-(tert-butyl)-4-(3-(trifluoromethyl)phenoxy)-1H-pyrazole-5-carboxamide, 59 µl of pyridine and 1 ml of dichloromethane, 52 mg of picolinoyl chloride hydrochloride was added under ice cooling, and a resultant mixture was stirred at room temperature for 15 hours. After completion of reaction, 1 mol/l hydrochloric acid was added to the reaction mixture, and extraction was carried out with chloroform (2 ml × 1 time). The obtained organic layer was washed with saturated sodium bicarbonate water, and then dehydrated and dried with anhydrous sodium sulfate, and then the solvent was evaporated under reduced pressure. The obtained residue was purified by silica gel column chromatography using n-hexane:ethyl acetate [gradient from 9:1 to 1:9 (volume ratio, the same applies hereinafter)] as an eluant, and thus 50 mg of the target compound was obtained as a white solid.
Melting point: 166°C to 168°C.

### Synthesis Example 4: Synthesis of 4-(((1-(tert-butyl)-4-(3-(trifluoromethyl)phenoxy)-1H-pyrazole-5-carboxamide)oxy)methyl)benzoic acid (compound No. 1-088)

To a mixed solution containing 6.20 g of methyl 4-(((1-(tert-butyl)-4-(3-(trifluoromethyl)phenoxy)-1H-pyrazole-5-carboxamide)oxy)methyl)benzoate and 40 ml of ethanol, a 1 mol/l sodium hydroxide aqueous solution was added at room temperature, and a resultant mixture was stirred at the same temperature for 18 hours. After completion of reaction, the solvent was evaporated under reduced pressure. To the obtained residue, 50 ml of water was added, and the water layer was washed with dichloromethane and then with hexane. To the obtained water layer, 35% by mass hydrochloric acid was added until the pH reached 1, and a precipitated solid was collected by filtration. The obtained solid was dried under reduced pressure, and thus 5.86 g of the target compound was obtained as a white solid.
Melting point: 82°C to 84°C.

### Synthesis Example 5: Synthesis of 1-(tert-butyl)-N-((4-(phenylcarbamoyl)benzyl)oxy)-4-(3-(trifluoromethyl)phenoxy)-1H-pyrazole-5-carboxamide (compound No. 2-012)

To a mixed solution containing 100 mg of 4-(((1-(tert-butyl)-4-(3-(trifluoromethyl)phenoxy)-1H-pyrazole-5-carboxamide)oxy)methyl)benzoic acid and 2 ml of dichloromethane, 60 mg of 1-ethyl-3-(3-dimethylaminopropyl)carbodiimide hydrochloride, 23 mg of aniline, 32 mg of triethylamine and 3 mg of 1-hydroxy-7-azabenzotriazole were sequentially added at room temperature, and a resultant mixture was stirred at the same temperature for 24 hours. After completion of reaction, 1 mol/l hydrochloric acid was added to the reaction mixture, and extraction was carried out with chloroform (2 ml × 1 time). The obtained organic layer was dehydrated and dried with anhydrous sodium sulfate, and the solvent was evaporated under reduced pressure. The obtained residue was purified by silica gel column chromatography using n-hexane:ethyl acetate (1:1) as an eluant, and thus 47 mg of the target compound was obtained as a white solid.
Melting point: 186°C to 189°C.

### Synthesis Example 6: Synthesis of 1-(tert-butyl)-N-((4-(3-methyl-1,2,4-oxadiazol-5-yl)benzyl)oxy)-4-(3-(trifluoromethyl)phenoxy)-1H-pyrazole-5-carboxamide (compound No. 2-016)

### Step 1: Synthesis of N-((4-((acetimidoamidooxy)carbonyl)benzyl)oxy)-1-(tert-butyl)-4-(3-(trifluoromethyl)phenoxy)-1H-pyrazole-5-carboxamide

To a mixed solution containing 150 mg of 4-(((1-(tert-butyl)-4-(3-(trifluoromethyl)phenoxy)-1H-pyrazole-5-carboxamide)oxy)methyl)benzoic acid and 2 ml of dichloromethane, 60 mg of 1-ethyl-3-(3-dimethylaminopropyl)carbodiimide hydrochloride, 28 mg of acetamide oxime, 47 mg of triethylamine and 4 mg of 1-hydroxy-7-azabenzotriazole were sequentially added at room temperature, and a resultant mixture was stirred at the same temperature for 18 hours. After completion of reaction, 2 ml of water was added to the reaction mixture, and extraction was carried out with chloroform (2 ml × 1 time). The obtained organic layer was dehydrated and dried with anhydrous sodium sulfate, and then the solvent was evaporated under reduced pressure. Thus, the target compound was obtained as an oil substance.

### Step 2: Synthesis of 1-(tert-butyl)-N-((4-(3-methyl-1,2,4-oxadiazol-5-yl)benzyl)oxy)-4-(3-(trifluoromethyl)phenoxy)-1H-pyrazole-5-carboxamide

A mixed solution containing the N-((4-((acetimidoamidooxy)carbonyl)benzyl)oxy)-1-(tert-butyl)-4-(3-(trifluoromethyl)phenoxy)-1H-pyrazole-5-carboxamide obtained in step 1 and 3 ml of pyridine was stirred at 80°C for 6 hours. After completion of stirring, stirring was carried out at room temperature for 16 hours, and then stirring was carried out at 80°C for 8 hours. After completion of reaction, 1 mol/l hydrochloric acid was added to the reaction mixture, and extraction was carried out with ethyl acetate (5 ml × 1 time). The obtained organic layer was dehydrated and dried with anhydrous sodium sulfate, and the solvent was evaporated under reduced pressure. The obtained residue was purified by silica gel column chromatography using n-hexane:ethyl acetate (gradient from 9:1 to 1:9) as an eluant, and thus 12 mg of the target compound was obtained as a white solid.
Melting point: 122°C to 124°C.

### Synthesis Example 7: Synthesis of 1-(tert-butyl)-N-((4-(N'-hydroxycarbamimidoyl)benzyl)oxy)-4-(3-(trifluoromethyl)phenoxy)-1H-pyrazole-5-carboxamide (compound No. 2-046)

To a mixed solution containing 2.30 g of 1-(tert-butyl)-N-((4-cyanobenzyl)oxy)-4-(3-(trifluoromethyl)phenoxy)-1H-pyrazole-5-carboxamide and 25 ml of ethanol, 0.66 g of a 50% by mass hydroxylamine aqueous solution was added at room temperature, and a resultant mixture was stirred at 80°C for 4 hours. After completion of reaction, 50 ml of water was added to the reaction mixture, and extraction was carried out with ethyl acetate (50 ml × 1 time). The obtained organic layer was dehydrated and dried with saturated brine and then with anhydrous sodium sulfate, and then the solvent was evaporated under reduced pressure. The obtained solid was washed with 20 ml of diisopropyl ether, and 2.26 g of the target compound was obtained as a white solid.
Melting point: 137°C to 139°C.

### Synthesis Example 8: Synthesis of 1-(tert-butyl)-N-((4-(5-methyl-1,2,4-oxadiazol-3-yl)benzyl)oxy)-4-(3-(trifluoromethyl)phenoxy)-1H-pyrazole-5-carboxamide (compound No. 2-050)

To a mixed solution containing 120 mg of 1-(tert-butyl)-N-((4-(N'-hydroxycarbamimidoyl)benzyl)oxy)-4-(3-(trifluoromethyl)phenoxy)-1H-pyrazole-5-carboxamide and 2 ml of N,N-dimethylformamide, 200 mg of potassium carbonate and 74 mg of acetic anhydride were sequentially added at room temperature, and a resultant mixture was stirred at 100°C for 2 hours. After completion of reaction, 5 ml of water was added to the reaction mixture, and extraction was carried out with a mixed solution (5 ml × 1 time) of n-hexane:ethyl acetate = 1:1. The obtained organic layer was dehydrated and dried with anhydrous sodium sulfate, and then the solvent was evaporated under reduced pressure. The obtained residue was purified by silica gel column chromatography using n-hexane:ethyl acetate (gradient from 9:1 to 1:9) as an eluant, and thus 82 mg of the target compound was obtained as an oil substance.
¹H-NMR (CDCl₃, Me₄Si, 300 MHz): δ8.95 (br, 1H), 7.97 (d, J = 8.1 Hz, 2H), 7.50-7.31 (m, 4H), 7.20 (s, 1H), 7.13 (s, 1H), 7.08 (d, J = 7.8 Hz, 1H), 4.98 (s, 2H), 2.65 (s, 3H), 1.72 (s, 9H).

### Synthesis Example 9: Synthesis of 1-(tert-butyl)-N-((4-(5-methyl-1,3,4-oxadiazol-2-yl)benzyl)oxy)-4-(3-(trifluoromethyl)phenoxy)-1H-pyrazole-5-carboxamide (compound No. 2-049)

### Step 1: Synthesis of N-((4-(2-acetylhydrazine-1-carbonyl)benzyl)oxy)-1-(tert-butyl)-4-(3-(trifluoromethyl)phenoxy)-1H-pyrazole-5-carboxamide

To a mixed solution containing 100 mg of 4-(((1-(tert-butyl)-4-(3-(trifluoromethyl)phenoxy)-1H-pyrazole-5-carboxamide)oxy)methyl)benzoic acid and 3 ml of tetrahydrofuran, 68 mg of 1,1'-carbonyldiimidazole was added under ice cooling, and a resultant mixture was stirred at room temperature for 4 hours. After completion of stirring, 78 mg of acetohydrazide was added to the reaction mixture at room temperature, and a resultant mixture was stirred at 60°C for 2 hours. After completion of reaction, 5 ml of water was added to the reaction mixture, and extraction was carried out with ethyl acetate (5 ml × 1 time). The obtained organic layer was washed with 1 mol/l hydrochloric acid, then dehydrated and dried with anhydrous sodium sulfate, and then the solvent was evaporated under reduced pressure. Thus, 126 mg of the target compound was obtained as an oil substance.

### Step 2: Synthesis of 1-(tert-butyl)-N-((4-(5-methyl-1,3,4-oxadiazol-2-yl)benzyl)oxy)-4-(3-(trifluoromethyl)phenoxy)-1H-pyrazole-5-carboxamide

To a mixed solution containing 126 mg of N-((4-(2-acetylhydrazine-1-carbonyl)benzyl)oxy)-1-(tert-butyl)-4-(3-(trifluoromethyl)phenoxy)-1H-pyrazole-5-carboxamide and 4 ml of 1,2-dichloroethane, 100 mg of methyl N-(triethylammoniosulfonyl)carbamate was added at room temperature, and a resultant mixture was stirred for 3 hours under a reflux condition. After completion of reaction, 5 ml of water was added to the reaction mixture, and extraction was carried out with chloroform (3 ml × 1 time). The obtained organic layer was dehydrated and dried with anhydrous sodium sulfate, and the solvent was evaporated under reduced pressure. The obtained residue was purified by silica gel column chromatography using n-hexane:ethyl acetate (1:2) as an eluant, and thus 55 mg of the target compound was obtained as an oil substance.
¹H-NMR (CDCl₃, Me₄Si, 300 MHz): δ9.05 (s, 1H), 7.96-7.89 (m, 2H), 7.52-7.45 (m, 2H), 7.44-7.30 (m, 2H), 7.23-7.18 (m, 1H), 7.14 (s, 1H), 7.12-7.06 (m, 1H), 5.00 (s, 2H), 2.62 (s, 3H), 1.72 (s, 9H).

### Synthesis Example 10: Synthesis of allyl 2-(1-(tert-butyl)-4-(3-(trifluoromethyl)phenoxy)-1H-pyrazole-5-carbonyl)-1-(2,4-dimethylbenzyl)hydrazine-1-carboxylate (compound No. 10-005)

To a mixed solution containing 200 mg of 1-(tert-butyl)-4-(3-(trifluoromethyl)phenoxy)-1H-pyrazole-5-carboxylic acid and 3 ml of dichloromethane, 78 µl of oxalyl chloride and 19 mg of N,N-dimethylformamide were sequentially added at room temperature, and a resultant mixture was stirred at the same temperature for 10 minutes. After completion of reaction, the solvent was evaporated under reduced pressure. To the obtained residue, 1 ml of dichloromethane was added. The reaction mixture was added to a mixed solution containing 172 mg of allyl 1-(2,4-dimethylbenzyl)hydrazine-1-carboxylate, 169 µl of triethylamine and 2 ml of dichloromethane under ice cooling, and a resultant mixture was stirred at the same temperature for 1 hour and 30 minutes. After completion of reaction, 3 ml of 1 mol/l hydrochloric acid was added to the reaction mixture, and extraction was carried out with chloroform (3 ml × 2 times). The obtained organic layer was dehydrated and dried with anhydrous sodium sulfate, and then the solvent was evaporated under reduced pressure. The obtained residue was purified by silica gel column chromatography using n-hexane:ethyl acetate [5:1 (volume ratio, the same applies hereinafter)] as an eluant, and thus 318 mg of the target compound was obtained as a light yellow solid.
Melting point: 110°C to 113°C.

### Synthesis Example 11: Synthesis of 1-(tert-butyl)-N'-(2,4-dimethylbenzyl)-4-(3-(trifluoromethyl)phenoxy)-1H-pyrazole-5-carbohydrazide (compound No. 10-006)

To a mixed solution containing 281 mg of allyl 2-(1-(tert-butyl)-4-(3-(trifluoromethyl)phenoxy)-1H-pyrazole-5-carbonyl)-1-(2,4-dimethylbenzyl)hydrazine-1-carboxylate, 1.2 g of 1,3-dimethylbarbituric acid and 5 ml of dichloromethane, 58 mg of tetrakis(triphenylphosphine)palladium(0) was added under a nitrogen atmosphere at room temperature, and a resultant mixture was stirred at the same temperature for 18 hours. After completion of reaction, 5 ml of a saturated sodium hydrogen carbonate aqueous solution was added to the reaction mixture, and extraction was carried out with chloroform (5 ml × 2 times). The obtained organic layer was dehydrated and dried with anhydrous sodium sulfate, and then the solvent was evaporated under reduced pressure. The obtained residue was purified by silica gel column chromatography using n-hexane:ethyl acetate (gradient from 9:1 to 8:2) as an eluant, and thus 172 mg of the target compound was obtained as a colorless oil substance.
¹H-NMR (CDCl₃, Me₄Si, 400 MHz): δ7.80-7.68 (m, 1H), 7.46-7.38 (m, 1H), 7.38-7.32 (m, 1H), 7.25-7.21 (m, 1H), 7.19 (s, 1H), 7.15-7.06 (m, 2H), 6.98-6.92 (m, 1H), 6.90-6.82 (m, 1H), 5.00-4.88 (m, 1H), 3.97-3.84 (m, 2H), 2.29 (s, 3H), 2.26 (s, 3H), 1.73 (s, 9H).

### Synthesis Example 12: Synthesis of 1-(tert-butyl)-N'-(cyanomethyl)-N'-(2,4-dimethylbenzyl)-4-(3-(trifluoromethyl)phenoxy)-1H-pyrazole-5-carbohydrazide (compound No. 10-007)

To a mixed solution containing 100 mg of 1-(tert-butyl)-N'-(2,4-dimethylbenzyl)-4-(3-(trifluoromethyl)phenoxy)-1H-pyrazole-5-carbohydrazide, 53 mg of bromoacetonitrile, 36 mg of potassium iodide, and 1 ml of acetonitrile, 67 mg of 1,8-diazabicyclo[5.4.0]-7-undecene was added at room temperature, and a resultant mixture was stirred at the same temperature for 1 hour and 30 minutes. After completion of reaction, 1 ml of 1 mol/l hydrochloric acid was added to the reaction mixture, and extraction was carried out with chloroform (3 ml × 2 times). The obtained organic layer was dehydrated and dried with anhydrous sodium sulfate, and then the solvent was evaporated under reduced pressure. The obtained residue was purified by silica gel column chromatography using n-hexane:ethyl acetate (gradient from 7:3 to 6:4) as an eluant, and thus 67 mg of the target compound was obtained as a resinous substance.
¹H-NMR (CDCl₃, Me₄Si, 400 MHz): δ7.58-7.50 (m, 1H), 7.48-7.40 (m, 1H), 7.40-7.33 (m, 1H), 7.32-7.27 (m, 1H), 7.21 (s, 1H), 7.19-7.13 (m, 1H), 7.13-7.05 (m, 1H), 6.99-6.90 (m, 1H), 6.89-6.81 (m, 1H), 3.96 (s, 2H), 3.77 (s, 2H), 2.33 (s, 3H), 2.24 (s, 3H), 1.67 (s, 9H).

### Synthesis Example 13: Synthesis of 2-(1-(tert-butyl)-4-phenoxy-1H-pyrazole-5-carbonyl)-1-(2,4-dimethylbenzyl)-N-ethylhydrazine-1-carboxamide (compound No. 10-015)

To a mixed solution containing 150 mg of 1-(tert-butyl)-N'-(2,4-dimethylbenzyl)-4-phenoxy-1H-pyrazole-5-carbohydrazide and 3 ml of dichloromethane, 29 mg of ethyl isocyanate was added under ice cooling, and a resultant mixture was stirred for 3 hours at room temperature. After completion of reaction, 3 ml of water was added to the reaction mixture, and extraction was carried out with chloroform (3 ml × 1 time). The obtained organic layer was dehydrated and dried with anhydrous sodium sulfate, and then the solvent was evaporated under reduced pressure. The obtained solid was washed with 2 ml of diisopropyl ether, and 108 mg of the target compound was obtained as a white solid.
Melting point: 129°C to 132°C.

### Synthesis Example 14: Synthesis of N'-(4-bromo-2-chlorobenzyl)-1-(tert-butyl)-4-phenoxy-1H-pyrazole-5-carbohydrazide (compound No. 10-165)

To a mixed solution containing 350 mg of 1-(tert-butyl)-4-phenoxy-1H-pyrazole-5-carbohydrazide and 12 ml of methanol, 280 mg of 4-bromo-2-chlorobenzaldehyde was added at room temperature, and a resultant mixture was stirred at the same temperature for 30 minutes. To the reaction mixture, 1.2 ml of acetic acid, 137 mg of 2-picoline-borane, and 1.3 ml of 3 mol/l hydrochloric acid were added, and a resultant mixture was stirred at the same temperature for 15 hours. To the reaction mixture, 27 mg of 2-picoline-borane was added at room temperature, and a resultant mixture was stirred at the same temperature for 3 hours. After completion of reaction, the reaction mixture was added to 20 ml of saturated sodium bicarbonate water, and extraction was carried out with a mixed solution (30 ml × 1 time) of n-hexane:ethyl acetate = 1:1 (volume ratio, the same applies hereinafter). The obtained organic layer was dehydrated and dried with anhydrous sodium sulfate, and then the solvent was evaporated under reduced pressure. The obtained residue was purified by silica gel column chromatography using n-hexane:ethyl acetate (1:1) as an eluant, and thus 600 mg of the target compound was obtained as a light yellow oil substance.
¹H-NMR (CDCl₃, Me₄Si, 300 MHz): δ8.00-7.90 (m, 1H), 7.29-7.27 (m, 3H), 7.24-7.20 (m, 4H), 6.96-6.89 (m, 2H), 5.21-5.12 (m, 1H), 4.02 (d, J = 5.4 Hz, 2H), 1.70 (s, 9H).

### Synthesis Example 15: Synthesis of methyl 2-(1-(tert-butyl)-4-phenoxy-1H-pyrazole-5-carbonyl)-1-(2-chloro-4-hydroxybenzyl)-2-ethylhydrazine-1-carboxylate (compound No. 13-001)

To a mixed solution containing 100 mg of methyl 2-(1-(tert-butyl)-4-phenoxy-1H-pyrazole-5-carbonyl)-1-(2-chloro-4-hydroxybenzyl)hydrazine-1-carboxylate and 2 ml of N,N-dimethylformamide, 44 mg of potassium carbonate and 25 µl of ethyl iodide were added at room temperature, and a resultant mixture was stirred at the same temperature for 2 hours. After completion of reaction, 3 ml of a saturated ammonium chloride aqueous solution was added to the reaction mixture, and extraction was carried out with a mixed solution (3 ml × 2 times) of n-hexane:ethyl acetate = 1:1. The obtained organic layer was dehydrated and dried with anhydrous sodium sulfate, and then the solvent was evaporated under reduced pressure. The obtained residue was purified by silica gel column chromatography using n-hexane:ethyl acetate (gradient from 9:1 to 1:9) as an eluant, and thus 12 mg of the target compound was obtained as a colorless oil substance.
¹H-NMR (CDCl₃, Me₄Si, 400 MHz): δ7.41-7.23 (m, 3H), 7.19-6.91 (m, 5H), 6.83-6.74 (m, 1H), 6.34-6.21 (m, 1H), 5.39-4.99 (m, 2H), 3.98-3.05 (m, 5H), 1.76-1.60 (m, 9H), 1.26 (t, J = 7.2 Hz, 3H).

### [Reference Examples]

The following description will discuss reference examples of a method for producing a production intermediate of the compound in accordance with the present invention.

### Reference Example 1: Synthesis of 4-iodo-1-phenyl-1H-pyrazole-5-carboxylic acid

To a mixed solution containing 2.0 g of 1-phenyl-1H-pyrazole-5-carboxylic acid and 10 ml of acetic acid, 2.5 g of N-iodosuccinimide was added at room temperature, and a resultant mixture was stirred at 80°C for 30 minutes. After completion of reaction, 50 ml of water was added at room temperature, and a precipitated solid was taken by filtration. The obtained solid was dried under reduced pressure, and thus 2.69 g of the target compound was obtained as a pink solid.
Melting point: 191°C to 193°C.

### Reference Example 2: Synthesis of 1-(tert-butyl)-4-(3-(trifluoromethyl)phenoxy)-1H-pyrazole-5-carboxylic acid

### Step 1: Synthesis of 4-(dimethylamino)-3-(3-(trifluoromethyl)phenoxy)but-3-en-2-one

A mixed solution containing 4.7 g of 1-(3-(trifluoromethyl)phenoxy)propan-2-one and 2.9 g of N,N-dimethylformamide dimethyl acetal was stirred at 80°C for 16 hours. After completion of reaction, the solvent was evaporated under reduced pressure. The obtained residue was purified by silica gel column chromatography using ethyl acetate as an eluant. To the obtained oil substance, 10 ml of diisopropyl ether and 10 ml of n-hexane were added, and a precipitated solid was taken by filtration. The obtained solid was dried under reduced pressure, and thus 2.36 g of the target compound was obtained as a light brown solid.
Melting point: 112°C to 114°C.

### Step 2: Synthesis of 1-(tert-butyl)-5-methyl-4-(3-(trifluoromethyl)phenoxy)-1H-pyrazole

A mixed solution containing 2.4 g of 4-(dimethylamino)-3-(3-(trifluoromethyl)phenoxy)but-3-en-2-one, 1.64 g of tert-butylhydrazine hydrochloride, and 25 ml of 1,4-dioxane was stirred at 100°C for 3 hours. After completion of reaction, the solvent was evaporated under reduced pressure. To the obtained residue, 20 ml of water was added, and extraction was carried out with n-hexane (20 ml × 2 times). The obtained organic layer was dehydrated and dried with saturated brine and then with anhydrous sodium sulfate, and then the solvent was evaporated under reduced pressure. The obtained residue was purified by silica gel column chromatography using n-hexane:ethyl acetate (5:1) as an eluant, and thus 2.51 g of the target compound was obtained as a light yellow oil substance.
¹H-NMR (CDCl₃, Me₄Si, 300 MHz): δ7.42-7.32 (m, 1H), 7.28 (s, 1H), 7.28-7.22 (m, 1H), 7.21-7.14 (m, 1H), 7.12-7.04 (m, 1H), 2.29 (s, 3H), 1.66 (s, 9H).

### Step 3: Synthesis of 1-(tert-butyl)-4-(3-(trifluoromethyl)phenoxy)-1H-pyrazole-5-carboxylic acid

To a mixed solution containing 930 mg of 1-(tert-butyl)-5-methyl-4-(3-(trifluoromethyl)phenoxy)-1H-pyrazole, 8.5 ml of tert-butyl alcohol, and 4.3 ml of water, 1.48 g of potassium permanganate was added at room temperature, and a resultant mixture was stirred at 100°C for 2 hours. After completion of stirring, 1.48 g of potassium permanganate was added to the reaction mixture at the same temperature, and a resultant mixture was stirred at the same temperature for 2 hours. After completion of reaction, the reaction mixture was filtrated with celite, and the celite was washed with 20 ml of water. A water layer of the obtained filtrate was washed with chloroform (20 ml × 2 times). Then, 1 mol/l hydrochloric acid was added until the pH reached 1, and extraction was carried out with chloroform (20 ml × 2 times). The obtained organic layer was washed with water, and then dehydrated and dried with saturated brine and then with anhydrous sodium sulfate, and then the solvent was evaporated under reduced pressure. The obtained solid was washed with 5 ml of n-hexane, and then dried under reduced pressure, and thus 209 mg of the target compound was obtained as a white solid.
Melting point: 120°C to 124°C.

### Reference Example 3: Synthesis of 1-benzyl-4-(3-(trifluoromethyl)phenoxy)-1H-pyrazole-5-carboxylic acid

### Step 1: Synthesis of 3-(dimethylamino)-2-(3-(trifluoromethyl)phenoxy)acrylaldehyde

To 15 ml of N,N-dimethylformamide, 5.6 ml of phosphorus oxychloride was added under ice cooling, and a resultant mixture was stirred at the same temperature for 15 minutes. After completion of stirring, a solution containing 5.0 g of 1-(2,2-dimethoxyethoxy)-3-(trifluoromethyl)benzene and 5 ml of N,N-dimethylformamide was added to the reaction mixture under ice cooling, and a resultant mixture was stirred at 70°C for 30 minutes. After completion of reaction, the reaction mixture was added to a mixed solution containing 28 g of potassium carbonate, 50 ml of water, and 5 ml of ethanol, and a resultant mixture was stirred at room temperature for 1 hour. After completion of stirring, the reaction mixture was extracted with toluene (40 ml × 2 times). The obtained organic layer was washed with water and then dehydrated and dried with saturated brine and then with anhydrous sodium sulfate. The solvent was evaporated under reduced pressure, and thus 4.40 g of the target compound was obtained as a brown oil substance.
¹H-NMR (CDCl₃, Me₄Si, 400 MHz): δ8.82 (s, 1H), 7.52-7.03 (m, 3H), 7.03-6.95 (m, 1H), 6.62 (s, 1H), 3.12 (br, 6H).

### Step 2: Synthesis of 1-benzyl-4-(3-(trifluoromethyl)phenoxy)-1H-pyrazole

A mixed solution containing 2.0 g of 3-(dimethylamino)-2-(3-(trifluoromethyl)phenoxy)acrylaldehyde, 2.4 g of benzylhydrazine hydrochloride, 2.1 ml of triethylamine, and 25 ml of 1,4-dioxane was stirred at 100°C for 3 hours. After completion of reaction, 20 ml of 1 mol/l hydrochloric acid was added to the reaction mixture at room temperature, and extraction was carried out with chloroform (30 ml × 2 times). The obtained organic layer was washed with water, and then dehydrated and dried with saturated brine and then with anhydrous sodium sulfate, and then the solvent was evaporated under reduced pressure. The obtained residue was purified by silica gel column chromatography using n-hexane:ethyl acetate (gradient from 9:1 to 1:9) as an eluant, and thus 400 mg of the target compound was obtained as a red oil substance.
¹H-NMR (CDCl₃, Me₄Si, 400 MHz): δ7.46-7.20 (m, 7H), 7.20-7.14 (m, 2H), 7.10-7.06 (m, 1H), 7.03-6.96 (m, 1H), 5.29 (s, 2H)

### Step 3: Synthesis of 1-benzyl-4-(3-(trifluoromethyl)phenoxy)-1H-pyrazole-5-carboxylic acid

Under a nitrogen atmosphere, 0.85 ml of an n-hexane solution of 1.6 mol/l n-butyllithium was added dropwise at -78°C to a solution containing 360 mg of 1-benzyl-4-(3-(trifluoromethyl)phenoxy)-1H-pyrazole and 10 ml of tetrahydrofuran, and a resultant mixture was stirred at the same temperature for 10 minutes. After completion of stirring, 1.0 g of dry ice was added at -78°C to the reaction mixture, and a resultant mixture was stirred at the same temperature for 5 minutes. After completion of reaction, 10 ml of 1 mol/l hydrochloric acid was added to the reaction mixture, and extraction was carried out with ethyl acetate (10 ml × 2 times). The obtained organic layer was dehydrated and dried with anhydrous sodium sulfate, and the solvent was evaporated under reduced pressure. The obtained residue was purified by silica gel column chromatography using n-hexane:ethyl acetate:acetic acid (gradient from 74:25:1 to 39:60:1) as an eluant, and thus 45 mg of the target compound was obtained as a colorless solid.
Melting point: 124°C to 126°C.

### Reference Example 4: Synthesis of 1-(tert-butyl)-N-hydroxy-4-phenoxy-1H-pyrazole-5-carboxamide

To a mixed solution containing 1.00 g of 1-(tert-butyl)-4-phenoxy-1H-pyrazole-5-carboxylic acid and 10 ml of dichloromethane, 0.98 g of oxalyl chloride and 11 mg of N,N-dimethylformamide were sequentially added under ice cooling, and a resultant mixture was stirred at room temperature for 1 hour. After completion of reaction, the solvent was evaporated under reduced pressure. To the obtained residue, 70 ml of dichloromethane was added. The reaction mixture was added to a mixed solution containing 1.06 g of hydroxylamine hydrochloride, 1.96 g of N,N-diisopropylethylamine and 30 ml of dichloromethane under ice cooling, and a resultant mixture was stirred at room temperature for 2 hours. After completion of reaction, 1 mol/l hydrochloric acid was added to the reaction mixture, and extraction was carried out with dichloromethane (10 ml × 1 time). The obtained organic layer was dehydrated and dried with saturated brine and then with anhydrous sodium sulfate. Subsequently, the solvent was evaporated under reduced pressure, and thus 1.08 g of the target compound was obtained as a light yellow solid.
Melting point: 122°C to 124°C.

### Reference Example 5: Synthesis of allyl 1-(2,4-dimethylbenzyl)hydrazine-1-carboxylate

### Step 1: Synthesis of allyl 2-(9H-fluoren-9-ylidene)hydrazine-1-carboxylate

To a mixed solution containing 8.19 g of (9H-fluoren-9-ylidene)hydrazine, 6.68 g of pyridine and 85 ml of dichloromethane, a solution containing 5.59 g of allyl chloroformate and 10 ml of dichloromethane was added under ice cooling, and a resultant mixture was stirred for 2 hours at room temperature. After completion of reaction, 50 ml of a saturated sodium hydrogen carbonate aqueous solution was added to the reaction mixture at room temperature, and extraction was carried out with dichloromethane (50 ml × 1 time). The obtained organic layer was dehydrated and dried with anhydrous sodium sulfate, and then the solvent was evaporated under reduced pressure. Thus, 11.6 g of the target compound was obtained as a yellow solid.
Melting point: 111°C to 113°C.

### Step 2: Synthesis of allyl 1-(2,4-dimethylbenzyl)-2-(9H-fluoren-9-ylidene)hydrazine-1-carboxylate

To a mixed solution containing 9.71 g of allyl 2-(9H-fluoren-9-ylidene)hydrazine-1-carboxylate, 22.0 g of a 35% by mass tetraethylammonium hydroxide aqueous solution, 579 mg of potassium iodide and 70 ml of tetrahydrofuran, 6.20 g of 1-(chloromethyl)-2,4-dimethylbenzene was added at room temperature, and a resultant mixture was stirred at 60°C for 1 hour. After completion of reaction, the solvent was evaporated under reduced pressure. To the obtained residue, 50 ml of water was added, and extraction was carried out with a mixed solution (100 ml × 1 time) of n-hexane:ethyl acetate = 1:1. The obtained organic layer was dehydrated and dried with anhydrous sodium sulfate, and then the solvent was evaporated under reduced pressure. The obtained residue was purified by silica gel column chromatography using n-hexane:ethyl acetate (1:1) as an eluant, and thus 14.5 g of the target compound was obtained as a brown oil substance.
¹H-NMR (CDCl₃, Me₄Si, 300 MHz) δ7.90-7.81 (m, 1H), 7.75-7.66 (m, 1H), 7.60-7.50 (m, 2H), 7.46-7.35 (m, 2H), 7.33-7.24 (m, 2H), 7.24-7.14 (m, 1H), 6.97-6.83 (m, 2H), 5.91-5.57 (m, 1H), 5.22-4.98 (m, 4H), 4.66-4.50 (m, 2H), 2.37 (s, 3H), 2.24 (s, 3H).

### Step 3: Synthesis of allyl 1-(2,4-dimethylbenzyl)hydrazine-1-carboxylate

To a mixed solution containing 14.5 g of allyl 1-(2,4-dimethylbenzyl)-2-(9H-fluoren-9-ylidene)hydrazine-1-carboxylate and 70 ml of pyridine, 9.70 g of hydroxylamine hydrochloride was added at room temperature, and a resultant mixture was stirred at 60°C for 1 hour. After completion of reaction, the solvent was evaporated under reduced pressure. To the obtained residue, 50 ml of water was added, and extraction was carried out with diisopropyl ether (100 ml × 1 time). The obtained organic layer was washed with water (50 ml × 2 times), and backward extraction was carried out with 3 mol/l hydrochloric acid (50 ml × 3 times). To the obtained water layer, a 10 mol/l sodium hydroxide aqueous solution was added under ice cooling until the pH reached 8, and extraction was carried out with chloroform (50 ml × 3 times). The obtained organic layer was dehydrated and dried with anhydrous sodium sulfate, and then the solvent was evaporated under reduced pressure. Thus, 7.04 g of the target compound was obtained as a yellow oil substance.
¹H-NMR (CDCl₃, Me₄Si, 300 MHz) δ7.15-7.05 (m, 1H), 7.05-6.95 (m, 2H), 6.06-5.86 (m, 1H), 5.41-5.18 (m, 2H), 4.72-4.65 (m, 2H), 4.62 (s, 2H), 4.00 (br, 2H), 2.32 (s, 6H).

### Reference Example 6: Synthesis of 1-(tert-butyl)-4-phenoxy-1H-pyrazole-5-carbohydrazide

To a mixed solution containing 5.0 g of 1-(tert-butyl)-4-phenoxy-1H-pyrazole-5-carboxylic acid and 80 ml of dichloromethane, 50 µl of N,N-dimethylformamide and 2.47 ml of oxalyl chloride were sequentially added at room temperature, and a resultant mixture was stirred at the same temperature for 1 hour. After completion of reaction, the solvent was evaporated under reduced pressure. To the obtained residue, 100 ml of dichloromethane was added. The reaction mixture was added dropwise to a mixed solution containing 9.6 ml of hydrazine monohydrate and 100 ml of dichloromethane under ice cooling, and a resultant mixture was stirred at the same temperature for 1 hour. After completion of reaction, 100 ml of water was added to the reaction mixture, and extraction was carried out with chloroform (100 ml × 2 times). The obtained organic layer was washed with water, and then dehydrated and dried with saturated brine and then with anhydrous sodium sulfate, and then the solvent was evaporated under reduced pressure. The obtained solid was washed with 20 ml of n-hexane, and then dried under reduced pressure, and thus 3.69 g of the target compound was obtained as a white solid.
Melting point: 81°C to 83°C.

### Reference Example 7: Synthesis of 1-(tert-butyl)-4-(3-methylphenoxy)-1H-pyrazole-5-carboxylic acid (compound No. i-036)

### Step 1: Synthesis of methyl 4-(3-bromophenoxy)-1-(tert-butyl)-1H-pyrazole-5-carboxylate (compound No. ii-004)

To a mixed solution containing 1.00 g of (4-(3-bromophenoxy)-1-(tert-butyl)-1H-pyrazole-5-carboxylic acid and 10 ml of acetone, 0.49 g of potassium carbonate and 0.67 g of dimethyl sulfate were sequentially added at room temperature, and a resultant mixture was stirred at the same temperature for 3 hours. After completion of reaction, the solvent was evaporated under reduced pressure. To the obtained residue, 20 ml of water was added, and extraction was carried out with ethyl acetate (20 ml × 2 times). The obtained organic layer was dehydrated and dried with anhydrous sodium sulfate, and then the solvent was evaporated under reduced pressure. Thus, 0.90 g of the target compound was obtained as a yellow solid.
Melting point: 69°C to 71°C.

### Step 2: Synthesis of methyl 1-(tert-butyl)-4-(3-methylphenoxy)-1H-pyrazole-5-carboxylate (compound No. ii-005)

Under a nitrogen atmosphere, to a mixed solution containing 0.50 g of methyl (4-(3-bromophenoxy)-1-(tert-butyl)-1H-pyrazole-5-carboxylate, 0.89 g of trimethylboroxine, 0.92 g of cesium carbonate, and 10 ml of 1,4-dioxane, 0.10 g of [1,1'-bis(diphenylphosphino)ferrocene]palladium(II) dichloride was added at room temperature, and a resultant mixture was stirred at 100°C for 16 hours. After completion of reaction, 20 ml of water was added to the reaction mixture, and extraction was carried out with ethyl acetate (20 ml × 3 times). Dehydration and drying were carried out with saturated brine and then with anhydrous sodium sulfate, and then the solvent was evaporated under reduced pressure. The obtained residue was purified by silica gel column chromatography using n-hexane:ethyl acetate (gradient from 10:0 to 9:1) as an eluant, and thus 0.30 g of the target compound was obtained as a yellow oil substance.
¹H-NMR (CDCl₃, Me₄Si, 400 MHz) δ7.24 (s, 1H), 7.20-7.13 (m, 1H), 6.90-6.85 (m, 1H), 6.84-6.76 (m, 2H), 3.77 (s, 3H), 2.33 (s, 3H), 1.72 (s, 3H).

### Step 3: Synthesis of 1-(tert-butyl)-4-(3-methylphenoxy)-1H-pyrazole-5-carboxylic acid (compound No. i-036)

To a mixed solution containing 39.0 g of methyl (1-(tert-butyl)-4-(3-methylphenoxy)-1H-pyrazole-5-carboxylate and 100 ml of methanol, a mixed solution containing 27.1 g of sodium hydroxide and 100 ml of water was added at room temperature, and a resultant mixture was stirred at 45°C for 16 hours. After completion of reaction, the solvent was evaporated under reduced pressure. After 50 ml of water was added to the obtained residue, 35% by mass hydrochloric acid was added until the pH reached 2, and a precipitated solid was taken by filtration. The obtained solid was dried under reduced pressure and then washed with 100 ml of n-hexane. Thus, 37.0 g of the target compound was obtained as a white solid.
Melting point: 116°C to 118°C.

### Reference Example 8: Synthesis of 1-(tert-butyl)-3-chloro-4-(3-(trifluoromethyl)jphenoxy)-1H-pyrazole-5-carboxylic acid

### Step 1: Synthesis of 1-(tert-butyl)-3-chloro-5-(dichloromethyl)-4-(3-(trifluoromethyl)phenoxy)-1H-pyrazole

To a mixed solution containing 5.00 g of 1-(tert-butyl)-5-methyl-4-(3-(trifluoromethyl)phenoxy)-1H-pyrazole and 17 ml of N,N-dimethylformamide, 11.4 g of 1,3-dichloro-5,5-dimethylhydantoin was added in portions at 70°C, and a resultant mixture was stirred at 100°C for 1 hour. After completion of reaction, a saturated sodium thiosulfate aqueous solution was added to the reaction mixture under ice cooling, and extraction was carried out with a mixed solution (30 ml × 1 time) of n-hexane:ethyl acetate = 1:1. The obtained organic layer was washed with water and saturated sodium bicarbonate water, and then dehydrated and dried with saturated brine and then with anhydrous sodium sulfate. The solvent was evaporated under reduced pressure, and thus 7.29 g of the target compound was obtained as a brown oil substance.
¹H-NMR (CDCl₃, Me₄Si, 300 MHz): δ7.46-7.37 (m, 1H), 7.35-7.29 (m, 1H), 7.25-7.21 (m, 1H), 7.15-7.06 (m, 2H), 1.71 (s, 9H).

### Step 2: Synthesis of 1-(tert-butyl)-3-chloro-4-(3-(trifluoromethyl)phenoxy)-1H-pyrazole-5-carbaldehyde

To a mixed solution containing 7.29 g of 1-(tert-butyl)-3-chloro-5-(dichloromethyl)-4-(3-(trifluoromethyl)phenoxy)-1H-pyrazole, 16 ml of N,N-dimethylformamide and 8 ml of water, 5.04 g of calcium carbonate was added at room temperature, and a resultant mixture was stirred at 100°C for 5 hours. After completion of stirring, 1.68 g of calcium carbonate and 4 ml of water were added at 100°C to the reaction mixture, and a resultant mixture was stirred at the same temperature for 19 hours. After completion of stirring, 1.68 g of calcium carbonate was added at 100°C to the reaction mixture, and a resultant mixture was stirred at the same temperature for 24 hours. After completion of reaction, the reaction mixture was filtrated with celite, and the celite was washed with 20 ml of ethyl acetate. With respect to the obtained filtrate, extraction was carried out with a mixed solution (20 ml × 1 time) of n-hexane:ethyl acetate = 1:1. The obtained organic layer was washed with water and then dehydrated and dried with anhydrous sodium sulfate, and then the solvent was evaporated under reduced pressure. Thus, 5.56 g of the target compound was obtained as a brown oil substance.
¹H-NMR (CDCl₃, Me₄Si, 300 MHz): δ9.74 (s, 1H), 7.49-7.42 (m, 1H), 7.40-7.33 (m, 1H), 7.25-7.21 (m, 1H), 7.14-7.06 (m, 1H), 1.70 (s, 9H).

### Step 3: Synthesis of 1-(tert-butyl)-3-chloro-4-(3-(trifluoromethyl)phenoxy)-1H-pyrazole-5-carboxylic acid

To a mixed solution containing 4.00 g of 1-(tert-butyl)-3-chloro-4-(3-(trifluoromethyl)phenoxy)-1H-pyrazole-5-carbaldehyde, 8.06 g of 2-methyl-2-butene, 30 ml of tert-butanol and 10 ml of water, 3.60 g of sodium dihydrogen phosphate dihydrate and 2.61 g of sodium chlorite (purity: 80% by mass) were added under ice cooling, and a resultant mixture was stirred at room temperature for 1 hour. After completion of reaction, the solvent was evaporated under reduced pressure. To the obtained residue, 20 ml of diisopropyl ether was added, and backward extraction was carried out with a 1 mol/l sodium hydroxide aqueous solution (20 ml × 1 time). To the obtained water layer, 35% by mass hydrochloric acid was added until the pH reached 1, and extraction was carried out with chloroform (20 ml × 1 time). The obtained organic layer was dehydrated and dried with anhydrous sodium sulfate, and the solvent was evaporated under reduced pressure. The obtained solid was washed with 10 ml of n-hexane, and then dried under reduced pressure, and thus 2.38 g of the target compound was obtained as a light yellow solid.
Melting point: 105°C to 107°C.

### Reference Example 9: Synthesis of 1-(tert-butyl)-4-(3-(trifluoromethyl)phenoxy)-1H-pyrazole-5-carboxylic acid

### Step 1: Synthesis of 1-(tert-butyl)-5-(chloromethyl)-4-(3-(trifluoromethyl)phenoxy)-1H-pyrazole

To a mixed solution containing 1.00 g of 1-(tert-butyl)-5-methyl-4-(3-(trifluoromethyl)phenoxy)-1H-pyrazole and 7 ml of acetonitrile, 448 mg of N-chlorosuccinimide was added at room temperature, and a resultant mixture was stirred at 80°C for 1 hour. After completion of reaction, 10 ml of a saturated sodium thiosulfate aqueous solution was added to the reaction mixture at room temperature, and extraction was carried out with chloroform (10 ml × 1 time). The obtained organic layer was washed with saturated sodium bicarbonate water, and then dehydrated and dried with anhydrous sodium sulfate, and then the solvent was evaporated under reduced pressure, and thus 1.17 g of the target compound was obtained as a brown oil substance. ¹H-NMR (CDCl₃, Me₄Si, 300 MHz): δ7.45--7.36 (m, 1H), 7.34-7.24 (m, 3H), 7.20-7.13 (m, 1H), 4.72 (s, 2H), 1.74 (s, 9H).

### Step 2: Synthesis of (1-(tert-butyl)-4-(3-(trifluoromethyl)phenoxy)-1H-pyrazol-5-yl)methanol

To a mixed solution containing 100 mg of 1-(tert-butyl)-5-(chloromethyl)-4-(3-(trifluoromethyl)phenoxy)-1H-pyrazole and 2 ml of acetonitrile, 2 ml of a 1 mol/l sodium hydroxide aqueous solution was added at room temperature, and a resultant mixture was stirred at 80°C for 1.5 hours. After completion of reaction, 5 ml of water was added at room temperature, and extraction was carried out with dichloromethane (5 ml × 1 time). The obtained organic layer was dehydrated and dried with anhydrous sodium sulfate, and then the solvent was evaporated under reduced pressure. Thus, 113 mg of the target compound was obtained as a yellow oil substance.
¹H-NMR (CDCl₃, Me₄Si, 400 MHz) δ 7.47-7.38 (m, 1H), 7.37-7.23 (m, 3H), 7.20-7.12 (m, 1H), 4.80-4.70 (m, 2H), 1.72 (s, 9H). A peak of an OH proton was not observed.

### Step 3: Synthesis of 1-(tert-butyl)-4-(3-(trifluoromethyl)phenoxy)-1H-pyrazole-5-carboxylic acid

To a mixed solution containing 395 mg of (1-(tert-butyl)-4-(3-(trifluoromethyl)phenoxy)-1H-pyrazol-5-yl)methanol, 500 mg of sodium dihydrogen phosphate dihydrate, 6 ml of acetonitrile and 4.8 ml of water, 20 mg of 2,2,6,6-tetramethylpiperidine-1-oxyl, 40 µl of a sodium hypochlorite aqueous solution (effective chlorine concentration: not less than 8% by mass) and a solution containing 284 mg of sodium chlorite (purity: 80% by mass) and 1.2 ml of water were sequentially added at room temperature, and a resultant mixture was stirred at the same temperature for 19 hours. After completion of stirring, 5 ml of water, 5 ml of a 1 mol/l sodium hydroxide aqueous solution, and 5 ml of a saturated sodium thiosulfate aqueous solution were sequentially added to the reaction mixture, and washing was carried out with 15 ml of dichloromethane. To the obtained water layer, 1 mol/l hydrochloric acid was added until the pH reached 1, and then extraction was carried out with ethyl acetate (20 ml × 1 time). The obtained organic layer was dehydrated and dried with anhydrous sodium sulfate, and then the solvent was evaporated under reduced pressure. Thus, 362 mg of the target compound was obtained as a white solid.
Melting point: 120°C to 124°C.

The compound in accordance with the present invention can be synthesized in accordance with the synthetic examples and reference examples above. Examples of compounds in accordance with the present invention which were produced in a manner similar to Synthesis Examples 1 through 15 are shown in Table 1 through Table 14. Examples of production intermediates produced in a manner similar to Reference Examples 1 through 9 are shown in Table 15 through Table 18. The compounds in accordance with the present invention and the production intermediates are not limited only to those examples.

In the tables, a substituent described as "Me" represents "methyl". Similarly, "Et" represents "ethyl", "nBu" represents "normal butyl", "iBu" represents "isobutyl", "tBu" represents "tertiary butyl", "nPr" represents "normal propyl", "iPr" represents "isopropyl", "cPr" represents "cyclopropyl", "nPen" represents "normal pentyl", "cPen" represents "cyclopentyl", "nHx" represents "normal hexyl", "cHx" represents "cyclohexyl", "Ph" represents "phenyl", and "Bn" represents "benzyl". The symbol "*" indicates that the compound is an oil or resinous compound having no melting point, and "m.p." represents a melting point (unit: °C).

For example, the term "4-" in "4-Me-Ph" in the tables indicates a substitution position on a phenyl group as described below. For example, "4-Me-Ph" represents 4-methylphenyl, and "2,6-F₂-Ph" represents 2,6-difluorophenyl.

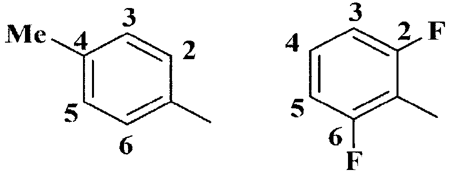

Substituents indicated by F-2a, F-3a, F-4a, F-11a, L-1, L-2, L-3, L-4, L-5, Z-1a, Z-1b, Z-1c, Z-1d, Z-2a, Z-3a, Z-4a, Z-4b, Z-6a, Z-8a, Z-8b, Z-8c, Z-9a, Z-17a, Z-19a, Z-27a, Z-27b, Z-27c, Z-27d, Z-28a, Z-34a, Z-44a, Z-45a, Z-46a, Z-47a, Z-48a, Z-49a, Z-50a, E-2a, E-3a, E-4a, E-13a, E-14a, E-25a, E-28a, E-37a, E-38a, E-41a, E-55a, S-1a, S-4a and S-6a represent the following structures, respectively.

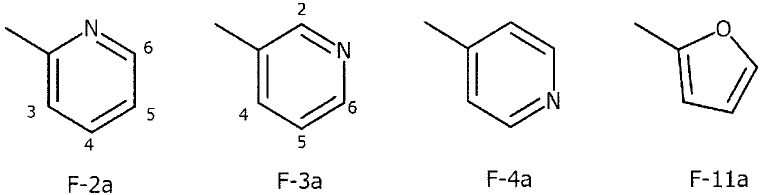

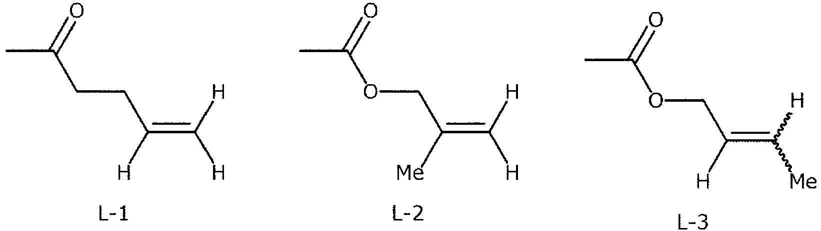

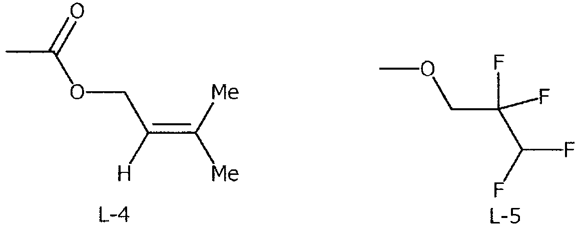

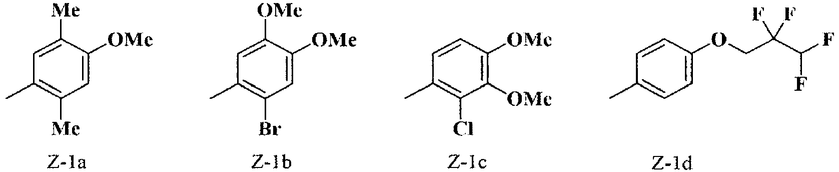

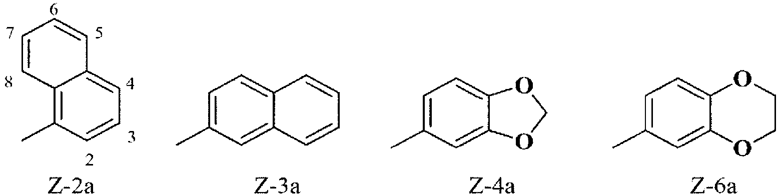

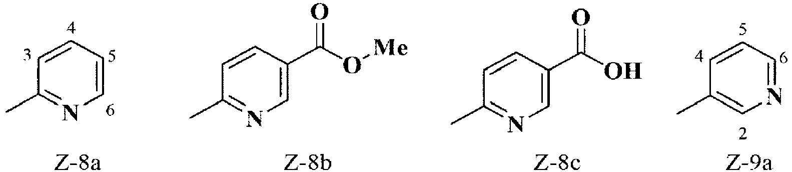

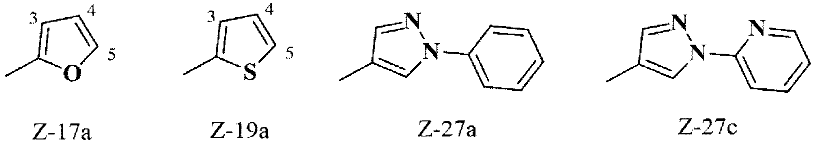

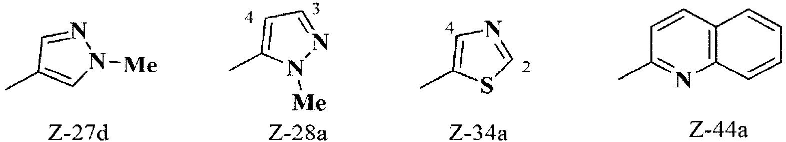

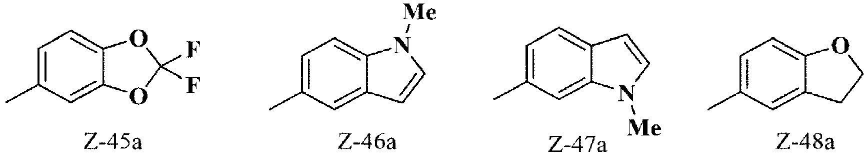

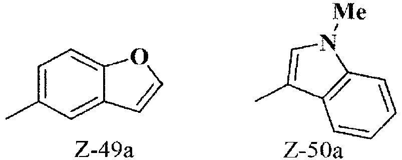

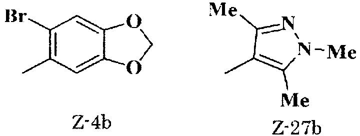

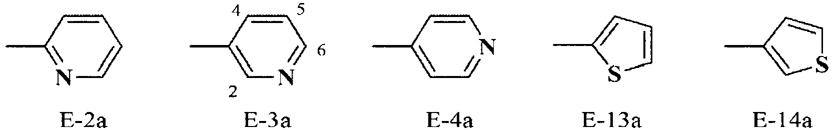

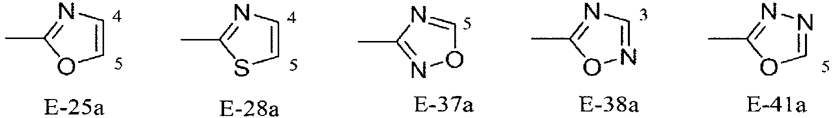

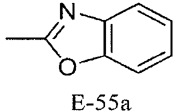

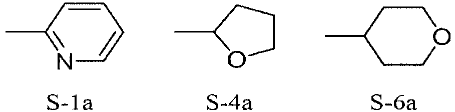

Note that the numerals indicated in the structural formulae above are each a numeral that represents a substitution position. For example, the term "6-OMe-(F-3a)" in the tables indicates a structure below. A structural formula in which a substituent is not described indicates that no substitution is included.

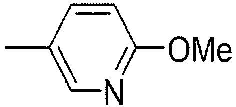

**[Table 1]**

| 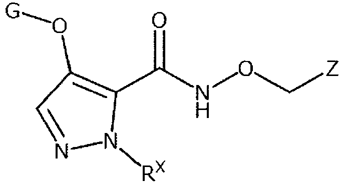 | | | | |
|---|---|---|---|---|
| No. | G | Z | R^{X} | m.p. (°C) |
| 1-001 | Ph | 4-Cl-Ph | tBu | * |
| 1-002 | Ph | Ph | tBu | * |
| 1-003 | 2-Cl-Ph | 4-Cl-Ph | tBu | * |
| 1-004 | 4-Cl-Ph | 4-Cl-Ph | tBu | * |
| 1-005 | 3,4-Cl₂-Ph | 4-Cl-Ph | tBu | * |
| 1-006 | 3-1-Ph | 4-Cl-Ph | tBu | * |
| 1-007 | 3-Cl-Ph | 4-Cl-Ph | tBu | * |
| 1-008 | 3-F-Ph | 4-Cl-Ph | tBu | * |
| 1-009 | 3-OMe-Ph | 4-Cl-Ph | tBu | * |
| 1-010 | 3-OCF₃-Ph | 4-Cl-Ph | tBu | * |
| 1-011 | Ph | 4-Me-Ph | tBu | 59-61 |
| 1-012 | Ph | 2,4-Me₂-Ph | tBu | * |
| 1-013 | Ph | 4-CF₃-Ph | tBu | * |
| 1-014 | 2,4-Cl₂-Ph | 4-Cl-Ph | tBu | * |
| 1-015 | 2,3-Cl₂-Ph | 4-Cl-Ph | tBu | * |
| 1-016 | 2-F-3-Cl-Ph | 4-Cl-Ph | tBu | * |
| 1-017 | Ph | 2,4-Cl₂-Ph | tBu | * |
| 1-018 | 3-CF₃-Ph | 4-Me-Ph | tBu | * |
| 1-019 | 3-CF₃-Ph | 2,4-Me₂-Ph | tBu | * |
| 1-020 | 3-CF₃-Ph | 4-Cl-Ph | tBu | * |
| 1-021 | 3-CF₃-Ph | 2,4-Cl₂-Ph | tBu | 62-64 |
| 1-022 | 3-Cl-Ph | 4-Me-Ph | tBu | 57-59 |
| 1-023 | 3-F-Ph | 4-Me-Ph | tBu | 52-54 |
| 1-024 | 3-Cl-Ph | 2,4-Me₂-Ph | tBu | * |
| 1-025 | 3-Cl-Ph | 2,4-Cl₂-Ph | tBu | * |
| 1-026 | 3-F-Ph | 2,4-Me₂-Ph | tBu | * |
| 1-027 | 3-F-Ph | 2,4-Cl₂-Ph | tBu | * |
| 1-028 | Ph | 4-OMe-Ph | tBu | * |
| 1-029 | Ph | 4-Br-Ph | tBu | * |
| 1-030 | Ph | 2-Br-Ph | tBu | * |
| 1-031 | Ph | 4-CN-Ph | tBu | * |
| 1-032 | Ph | 3,4-Cl₂-Ph | tBu | * |
| 1-033 | Ph | 4-NO₂-Ph | tBu | * |
| 1-034 | 3-CF₃-Ph | 4-Cl-Ph | iPr | 104-106 |
| 1-035 | 3-CF₃-Ph | 4-Cl-Ph | Ph | 117-119 |
| 1-036 | Ph | 4-Cl-Ph | iPr | * |
| 1-037 | Ph | 4-Cl-Ph | Ph | 144-146 |
| 1-038 | 3-CF₃-Ph | 4-OMe-Ph | tBu | 70-72 |
| 1-039 | 3-CF₃-Ph | 4-Br-Ph | tBu | * |
| 1-040 | 3-CF₃-Ph | 2-Br-Ph | tBu | * |
| 1-041 | 3-CF₃-Ph | 3,4-Cl₂-Ph | tBu | * |
| 1-042 | Ph | 3-Me-Ph | tBu | * |
| 1-043 | Ph | 2-Me-Ph | tBu | * |
| 1-044 | Ph | Z-3a | tBu | 92-94 |
| 1-045 | Ph | 4-C(O)OMe-Ph | tBu | * |
| 1-046 | 3-CF₃-Ph | 3-Me-Ph | tBu | * |
| 1-047 | 3-CF₃-Ph | 2-Me-Ph | tBu | * |
| 1-048 | 3-CF₃-Ph | Z-3a | tBu | * |
| 1-049 | 3-CF₃-Ph | 4-C(O)OMe-Ph | tBu | * |
| 1-050 | Ph | Z-4a | tBu | * |
| 1-051 | 3-CF₃-Ph | 4-Cl-Ph | F-2a | 131-133 |
| 1-052 | 3-Me-Ph | 4-Me-Ph | tBu | * |
| 1-053 | 3-Me-Ph | 2,4-Me₂-Ph | tBu | * |
| 1-054 | 3-Me-Ph | 2,4-Cl₂-Ph | tBu | * |
| 1-055 | 3-Me-Ph | 4-Cl-Ph | tBu | * |
| 1-056 | Ph | 2-Cl-4-OMe-Ph | tBu | * |
| 1-057 | 3-CF₃-Ph | 2-Cl-4-OMe-Ph | tBu | * |
| 1-058 | 3-Me-Ph | 2-Cl-4-OMe-Ph | tBu | * |
| 1-059 | 3-NMe₂-Ph | 4-Me-Ph | tBu | * |
| 1-060 | 3-SMe-Ph | 4-Me-Ph | tBu | * |
| 1-061 | 3-CF₃-Ph | 4-Cl-Ph | F-3a | * |
| 1-062 | Ph | 4-Cl-Ph | F-2a | * |
| 1-063 | Ph | 4-Cl-Ph | F-3a | 118-120 |
| 1-064 | 3-SOMe-Ph | 4-Me-Ph | tBu | * |
| 1-065 | 3-SO₂Me-Ph | 4-Me-Ph | tBu | * |
| 1-066 | 3-Me-Ph | 4-OMe-Ph | tBu | * |
| 1-067 | Ph | 2-F-4-OMe-Ph | tBu | * |
| 1-068 | 3-Me-Ph | 2-F-4-OMe-Ph | tBu | * |
| 1-069 | 3-CF₃-Ph | 2-F-4-OMe-Ph | tBu | * |
| 1-070 | 3-CF₃-Ph | 4-NO₂-Ph | tBu | 127-129 |
| 1-071 | 3-CF₃-Ph | 6-Cl-(Z-9a) | tBu | * |
| 1-072 | 3-CF₃-Ph | 4-NH₂-Ph | tBu | * |
| 1-073 | 2-F-Ph | 4-Me-Ph | tBu | * |
| 1-074 | 2-CF₃-Ph | Z-44a | tBu | * |
| 1-075 | 3-CF₃-Ph | 4-CF₃-Ph | tBu | * |
| 1-076 | 3-CF₃-Ph | 4-F-Ph | tBu | * |
| 1-077 | 3-CF₃-Ph | 4-OCF₃-Ph | tBu | * |
| 1-078 | 3-CF₃-Ph | Z-2a | tBu | 127-128 |
| 1-079 | 3-CF₃-Ph | 4-Br-(Z-2a) | tBu | * |
| 1-080 | 3-CF₃-Ph | 5-Cl-(Z-19a) | tBu | * |
| 1-081 | 2,3-F₂-Ph | 4-Me-Ph | tBu | * |
| 1-082 | 2-F-3-OMe-Ph | 4-Me-Ph | tBu | * |
| 1-083 | 2-F-3-Br-Ph | 4-Me-Ph | tBu | * |
| 1-084 | 3-CF₃-Ph | 4-Et-Ph | tBu | * |
| 1-085 | Ph | 4-Et-Ph | tBu | * |
| 1-086 | 3-Me-Ph | 4-Et-Ph | tBu | * |
| 1-087 | 3-CF₃-Ph | 4-OCF₂H-Ph | tBu | 59-61 |
| 1-088 | 3-CF₃-Ph | 4-C(O)OH-Ph | tBu | 82-84 |
| 1-089 | 3-CF₃-Ph | 4-CN-Ph | tBu | 142-144 |
| 1-090 | 3-Me-Ph | 4-Br-Ph | tBu | * |
| 1-091 | 3-CF₃-Ph | 2-Br-5-OMe-Ph | tBu | 108-109 |
| 1-092 | 3-CF₃-Ph | 2-Cl-(Z-34a) | tBu | * |
| 1-093 | 3-CF₃-Ph | Z-27a | tBu | * |
| 1-094 | Ph | 4-OEt-Ph | tBu | * |
| 1-095 | 3-Me-Ph | 4-OEt-Ph | tBu | * |
| 1-096 | 3-CF₃-Ph | 4-OEt-Ph | tBu | * |
| 1-097 | 3-CF₃-Ph | 6-OMe-(Z-9a) | tBu | * |
| 1-098 | 3-CF₃-Ph | 6-OEt-(Z-9a) | tBu | * |
| 1-099 | 3-CF₃-Ph | 2-OMe-4-Me-Ph | tBu | * |
| 1-100 | Ph | 2-OMe-4-Me-Ph | tBu | * |
| 1-101 | 3-CF₃-Ph | 5-Cl-(Z-8a) | tBu | * |
| 1-102 | Ph | 2-Me-4-OMe-Ph | tBu | * |
| 1-103 | 3-Me-Ph | 2-Me-4-OMe-Ph | tBu | * |
| 1-104 | 3-CF₃-Ph | 2-Me-4-OMe-Ph | tBu | * |
| 1-105 | 3-Me-Ph | 2-OMe-4-Me-Ph | tBu | * |
| 1-106 | 3-CF₃-Ph | 5-Me-(Z-8a) | tBu | * |
| 1-107 | 3-CF₃-Ph | 3-F-4-OMe-Ph | tBu | * |
| 1-108 | Ph | 2-OMe-4-Cl-Ph | tBu | * |
| 1-109 | 3-Me-Ph | 2-OMe-4-Cl-Ph | tBu | * |
| 1-110 | 3-CF₃-Ph | 2-OMe-4-Cl-Ph | tBu | * |
| 1-111 | 3-Cl-Ph | 2-OMe-4-Cl-Ph | tBu | * |
| 1-112 | Ph | 2,4-(OMe)₂-Ph | tBu | * |
| 1-113 | 3-Me-Ph | 2,4-(OMe)₂-Ph | tBu | * |
| 1-114 | 3-CF₃-Ph | 2,4-(OMe)₂-Ph | tBu | * |
| 1-115 | 3-Cl-Ph | 2,4-(OMe)₂-Ph | tBu | * |
| 1-116 | 3-CF₃-Ph | 2-Br-4-OMe-Ph | tBu | * |
| 1-117 | Ph | 2-Br-4-OMe-Ph | tBu | * |
| 1-118 | 3-Me-Ph | 2-Br-4-OMe-Ph | tBu | * |
| 1-119 | 3-CF₃-Ph | 4-CH=CH₂-Ph | tBu | * |
| 1-120 | 3-Cl-Ph | 2-F-4-OMe-Ph 2-Cl-4-OMe- | tBu | * |
| 1-121 | 3-Cl-Ph | Ph | tBu | * |
| 1-122 | 3-Cl-Ph | 4-OMe-Ph | tBu | 98-100 |
| 1-123 | 3-Cl-Ph | 4-OEt-Ph | tBu | * |
| 1-124 | 3-Cl-Ph | 4-Br-Ph | tBu | * |
| 1-125 | 3-Cl-Ph | 2-Me-4-OMe-Ph | tBu | * |
| 1-126 | 3-CF₃-Ph | 4-I-Ph | tBu | * |
| 1-127 | Ph | 4-OCF₂H-Ph | tBu | * |
| 1-128 | 3-CF₃-Ph | 4-C(O)Me-Ph | tBu | * |
| 1-129 | 3-CF₃-Ph | 5-Me-(Z-17a) | tBu | * |
| 1-130 | 3-CF₃-Ph | Z-6a | tBu | * |
| 1-131 | 3-CF₃-Ph | 2-OMe-4-F-Ph | tBu | * |
| 1-132 | 3-CF₃-Ph | 2-Cl-4-Me-Ph | tBu | * |
| 1-133 | 3-Me-Ph | 2-Cl-4-Me-Ph | tBu | * |
| 1-134 | 3-Cl-Ph | 2-Cl-4-Me-Ph | tBu | * |
| 1-135 | Ph | 2-Cl-4-Me-Ph | tBu | * |
| 1-136 | 3-CF₃-Ph | 2-Me-4-Cl-Ph | tBu | * |
| 1-137 | 3-Me-Ph | 2-Me-4-Cl-Ph | tBu | * |
| 1-138 | 3-Cl-Ph | 2-Me-4-Cl-Ph | tBu | * |
| 1-139 | Ph | 2-Me-4-Cl-Ph | tBu | * |
| 1-140 | 3-Me-Ph | 4-OCF₂H-Ph | tBu | * |
| 1-141 | 3-Cl-Ph | 2-OMe-4-Me-Ph | tBu | * |
| 1-142 | 3-CF₃-Ph | 2-OMe-4-Br-Ph | tBu | * |
| 1-143 | Ph | 2-OMe-4-Br-Ph | tBu | * |
| 1-144 | 3-Me-Ph | 2-OMe-4-Br-Ph 2-OMe-4-Br- | tBu | * |
| 1-145 | 3-Cl-Ph | Ph | tBu | * |
| 1-146 | 3-CF₃-Ph | 4-iPr-Ph | tBu | * |
| 1-147 | Ph | 4-iPr-Ph | tBu | * |
| 1-148 | 3-Me-Ph | 4-iPr-Ph | tBu | * |
| 1-149 | 3-Cl-Ph | 4-iPr-Ph | tBu | * |
| 1-150 | 3-Cl-Ph | 2-Br-4-OMe-Ph | tBu | * |
| 1-151 | 3-Me-Ph | 4-OH-Ph | tBu | * |
| 1-152 | 3-CF₃-Ph | 4-OH-Ph | tBu | 149-151 |
| 1-153 | 3-CF₃-Ph | 6-CN-(Z-9a) | tBu | 113-117 |
| 1-154 | 3-CF₃-Ph | 5-CN-(Z-8a) | tBu | 134-136 |
| 1-155 | Ph | 6-Me-(Z-9a) | tBu | * |
| 1-156 | 3-Me-Ph | 6-Me-(Z-9a) | tBu | 123-124 |
| 1-157 | 3-CF₃-Ph | 6-Me-(Z-9a) | tBu | * |
| 1-158 | 3-Cl-Ph | 6-Me-(Z-9a) | tBu | 114-116 |
| 1-159 | 3-CF₃-Ph | 5-OMe-(Z-8a) | tBu | * |
| 1-160 | 3-CF₃-Ph | 3-Cl-5-Br-(Z-8a) | tBu | * |
| 1-161 | 3-CF₃-Ph | 3,5-Cl₂-(Z-8a) | tBu | * |
| 1-162 | 3-CF₃-Ph | 3-Br-5-Cl-(Z-8a) | tBu | * |
| 1-163 | 3-CF₃-Ph | Z-45a | tBu | * |
| 1-164 | 3-Cl-Ph | 4-Et-Ph | tBu | * |
| 1-165 | 3-CF₃-Ph | 3-Me-(Z-28a) | tBu | 134-136 |
| 1-166 | 3-CF₃-Ph | 2-Br-4-Cl-Ph | tBu | * |
| 1-167 | 3-Me-Ph | 2-Br-4-Cl-Ph | tBu | * |
| 1-168 | 3-Cl-Ph | 2-Br-4-Cl-Ph | tBu | * |
| 1-169 | Ph | 2-Br-4-Cl-Ph | tBu | * |
| 1-170 | 3-CF₃-Ph | Z-27c | tBu | * |
| 1-171 | 3-CF₃-Ph | 2-Cl-4-Br-Ph | tBu | * |
| 1-172 | Ph | 2-Cl-4-Br-Ph | tBu | * |
| 1-173 | 3-Me-Ph | 2-Cl-4-Br-Ph | tBu | * |
| 1-174 | 3-Cl-Ph | 2-Cl-4-Br-Ph | tBu | * |
| 1-175 | Ph | 2-F-4-Br-Ph | tBu | 94-97 |
| 1-176 | 3-Me-Ph | 2-F-4-Br-Ph | tBu | 71-75 |
| 1-177 | 3-CF₃-Ph | 2-F-4-Br-Ph | tBu | * |
| 1-178 | 3-Cl-Ph | 2-F-4-Br-Ph | tBu | * |
| 1-179 | 3-Me-Ph | 4-CN-Ph | tBu | 107-109 |
| 1-180 | 3-Cl-Ph | 4-CN-Ph | tBu | 129-131 |
| 1-181 | Ph | 4-C(O)OH-Ph | tBu | * |
| 1-182 | 3-Me-Ph | 4-C(O)OH-Ph | tBu | * |
| 1-183 | 3-Cl-Ph | 2,5-Me₂-Ph | tBu | * |
| 1-184 | 3-CF₃-Ph | 2-Br-4-Me-Ph | tBu | * |
| 1-185 | 3-CF₃-Ph | 2-Me-4-Br-Ph | tBu | * |
| 1-186 | 3-CF₃-Ph | 2,4-Br₂-Ph | tBu | * |
| 1-187 | 3-CF₃-Ph | 2-OEt-4-Me-Ph | tBu | * |
| 1-188 | 3-CF₃-Ph | 2-Me-4-OEt-Ph | tBu | * |
| 1-189 | 3-CF₃-Ph | 4-OnPr-Ph | tBu | * |
| 1-190 | Ph | 2-Br-4-Me-Ph | tBu | * |
| 1-191 | Ph | 2-Me-4-Br-Ph | tBu | * |
| 1-192 | Ph | 2,4-Br₂-Ph | tBu | * |
| 1-193 | Ph | 2-OEt-4-Me-Ph | tBu | * |
| 1-194 | Ph | 2-Me-4-OEt-Ph | tBu | * |
| 1-195 | Ph | 4-OnPr-Ph | tBu | * |
| 1-196 | 3-Me-Ph | 2-Me-4-Br-Ph | tBu | * |
| 1-197 | 3-Me-Ph | 2,4-Br₂-Ph | tBu | * |
| 1-198 | 3-Me-Ph | 2-OEt-4-Me-Ph | tBu | * |
| 1-199 | 3-Me-Ph | 2-Me-4-OEt-Ph | tBu | * |
| 1-200 | 3-Me-Ph | 4-OnPr-Ph | tBu | * |
| 1-201 | 3-CF₃-Ph | 4-SMe-Ph | tBu | * |
| 1-202 | 3-CF₃-Ph | 3,4-Me₂-Ph | tBu | * |
| 1-203 | 3-Me-Ph | 3,4-Me₂-Ph | tBu | * |
| 1-204 | 3-Cl-Ph | 3,4-Me₂-Ph | tBu | * |
| 1-205 | Ph | 3,4-Me₂-Ph | tBu | * |
| 1-206 | 3-CF₃-Ph | 3-F-4-Me-Ph | tBu | * |
| 1-207 | 3-Me-Ph | 3-F-4-Me-Ph | tBu | * |
| 1-208 | 3-Cl-Ph | 3-F-4-Me-Ph | tBu | * |
| 1-209 | Ph | 3-F-4-Me-Ph | tBu | * |
| 1-210 | 3-CF₃-Ph | Z-46a | tBu | * |
| 1-211 | 3-Me-Ph | Z-46a | tBu | * |
| 1-212 | 3-Me-Ph | 3,4-(OMe)₂-Ph | tBu | * |
| 1-213 | 3-CF₃-Ph | Z-47a | tBu | * |
| 1-214 | 3-Me-Ph | Z-47a | tBu | * |
| 1-215 | Ph | Z-47a | tBu | * |
| 1-216 | 3-Cl-Ph | Z-47a | tBu | * |
| 1-217 | 3-Me-Ph | 2-Br-4-F-Ph | tBu | * |
| 1-218 | 3-Me-Ph | 2-Me-4-F-Ph | tBu | * |
| 1-219 | 3-Me-Ph | 2-Cl-4-F-Ph | tBu | * |
| 1-220 | 3-CF₃-Ph | 2-Br-4-F-Ph | tBu | * |
| 1-221 | 3-CF₃-Ph | 2-Me-4-F-Ph | tBu | 89-91 |
| 1-222 | 3-CF₃-Ph | 2-Cl-4-F-Ph | tBu | * |
| 1-223 | Ph | Z-46a | tBu | * |
| 1-224 | 3-Cl-Ph | Z-46a | tBu | * |
| 1-225 | 3-CF₃-Ph | 2-(5-Me-(E-37a))-Ph | tBu | * |
| 1-226 | 3-CF₃-Ph | 3-(5-Me-(E-37a))-Ph | tBu | * |
| 1-227 | 3-Me-Ph | Z-4a | tBu | * |
| 1-228 | 3-Me-Ph | Z-48a | tBu | * |
| 1-229 | 3-Me-Ph | 3-Cl-4-Me-Ph | tBu | * |
| 1-230 | 3-Me-Ph | Z-49a | tBu | * |
| 1-231 | 3-CF₃-Ph | 3-Cl-4-Me-Ph | tBu | * |
| 1-232 | 3-CF₃-Ph | Z-49a | tBu | * |
| 1-233 | Ph | 3-Cl-4-Me-Ph | tBu | * |
| 1-234 | Ph | Z-49a | tBu | * |
| 1-235 | 3-CF₃-Ph | Z-50a | tBu | * |
| 1-236 | 3-Me-Ph | Z-50a | tBu | * |
| 1-237 | Ph | Z-50a | tBu | * |
| 1-238 | 3-Cl-Ph | Z-50a | tBu | * |
| 1-239 | 3-Me-Ph | 2-Me-4-OnPr-Ph | tBu | * |
| 1-240 | 3-CF₃-Ph | 2-Me-4-OnPr-Ph | tBu | * |

**[Table 2]**

| 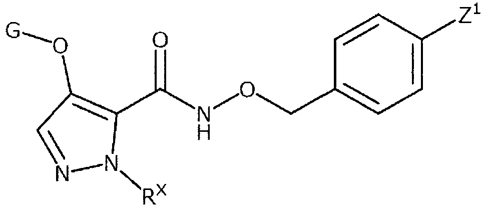 | | | | |
|---|---|---|---|---|
| No. | G | Z¹ | R^{X} | m.p. (°C) |
| 2-001 | 3-CF₃-Ph | NHC(O)(E-2a) NHC(O) (E-2a) | tBu | 166-168 |
| 2-002 | 3-CF₃-Ph | NHC(O)CH₂CF₃ | tBu | * |
| 2-003 | 3-CF₃-Ph | NHC(O)OMe | tBu | * |
| 2-004 | 3-CF₃-Ph | NHC(O)(2-F-Ph) | tBu | 178-179 |
| 2-005 | 3-CF₃-Ph | NHC(O)cPr | tBu | 204-206 |
| 2-006 | 3-CF₃-Ph | NHC(O)CH₂OMe | tBu | * |
| 2-007 | 3-CF₃-Ph | NHC(O) (2-Me-Ph) | tBu | 135-137 |
| 2-008 | 3-CF₃-Ph | NHC(O)(2-OMe-Ph) | tBu | * |
| 2-009 | 3-CF₃-Ph | NHC(O)(4-F-Ph) | tBu | 210-212 |
| 2-010 | 3-CF₃-Ph | NHC(O)CH₂CH₂OMe | tBu | 124-126 |
| 2-011 | 3-CF₃-Ph | Ph | tBu | * |
| 2-012 | 3-CF₃-Ph | C(O)NHPh | tBu | 186-189 |
| 2-013 | 3-CF₃-Ph | 5-CF₃-(E-37a) | tBu | * |
| 2-014 | 3-CF₃-Ph | E-3a | tBu | * |
| 2-015 | 3-CF₃-Ph | 6-OMe-(E-3a) | tBu | * |
| 2-016 | 3-CF₃-Ph | 3-Me-(E-38) | tBu | 122-124 |
| 2-017 | 3-CF₃-Ph | C(O)NHEt | tBu | 149-151 |
| 2-018 | 3-CF₃-Ph | C(O)NHOMe | tBu | * |
| 2-019 | 3-CF₃-Ph | C(O)NHCH₂CH=CH₂ | tBu | * |
| 2-020 | 3-CF₃-Ph | C(O)NHcPen | tBu | 179-181 |
| 2-021 | 3-CF₃-Ph | C(O)NHBn | tBu | 114-116 |
| 2-022 | 3-CF₃-Ph | NHC(O) (E-3a) | tBu | * |
| 2-023 | 3-CF₃-Ph | C(O)NH(5-Me-(E-28a)) | tBu | 176-178 |
| 2-024 | 3-CF₃-Ph | C(O)NHMe | tBu | 59-61 |
| 2-025 | 3-CF₃-Ph | E-14a | tBu | * |
| 2-026 | Ph | OCH₂CF₃ | tBu | 89-91 |
| 2-027 | Ph | OCH₂(6-Cl-(E-3a)) | tBu | 110-112 |
| 2-028 | Ph | OCH₂(6-Cl-(E-2a)) | tBu | * |
| 2-029 | 3-Me-Ph | OCH₂CF₃ | tBu | * |
| 2-030 | 3-Me-Ph | OCH₂(6-Cl-(E-3a)) | tBu | * |
| 2-031 | 3-Me-Ph | OCH₂(6-Cl-(E-2a)) | tBu | * |
| 2-032 | 3-CF₃-Ph | OCH₂CF₃ | tBu | * |
| 2-033 | 3-CF₃-Ph | OCH₂(6-Cl-(E-3a)) | tBu | * |
| 2-034 | 3-CF₃-Ph | OCH₂(6-Cl-(E-2a)) | tBu | 117-119 |
| 2-035 | Ph | OCH₂CF₂H | tBu | * |
| 2-036 | 3-Me-Ph | OCH₂CF₂H | tBu | * |
| 2-037 | 3-CF₃-Ph | OCH₂CF₂H | tBu | * 127-129 |
| 2-038 | 3-Cl-Ph | OCH₂CF₂H | tBu | * |
| 2-039 | 3-Cl-Ph | OCH₂(6-Cl-(E-2a)) | tBu | |
| 2-040 | 3-Cl-Ph | OCH₂(6-Cl-(E-3a)) | tBu | * |
| 2-041 | 3-Cl-Ph | OCH₂CF₃ | tBu | * |
| 2-042 | Ph | OCH₂CF₂CF₂H | tBu | * |
| 2-043 | 3-Me-Ph | OCH₂CF₂CF₂H | tBu | * |
| 2-044 | 3-CF₃-Ph | OCH₂CF₂CF₂H | tBu | * |
| 2-045 | 3-Cl-Ph | OCH₂CF₂CF₂H | tBu | * |
| 2-046 | 3-CF₃-Ph | C(=NOH)NH₂ | tBu | 137-139 |
| 2-047 | 3-CF₃-Ph | 3-Et-(E-38a) | tBu | 126-129 |
| 2-048 | 3-CF₃-Ph | 3-CF₃-(E-38a) | tBu | * |
| 2-049 | 3-CF₃-Ph | 5-Me-(E-41a) | tBu | * |
| 2-050 | 3-CF₃-Ph | 5-Me-(E-37a) | tBu | * |
| 2-051 | 3-CF₃-Ph | C(=NOMe)Me | tBu | * |
| 2-052 | 3-CF₃-Ph | 5-cPr-(E-37a) | tBu | * |
| 2-053 | 3-CF₃-Ph | 5-CF₂H-(E-37a) | tBu | * |
| 2-054 | 3-Me-Ph | 5-CF₂H-(E-37a) | tBu | 94-96 |
| 2-055 | 3-Me-Ph | 5-Et-(E-37a) | tBu | 77-79 |
| 2-056 | 3-Me-Ph | 5-iPr-(E-37a) | tBu | 92-94 |
| 2-057 | 3-Me-Ph | 5-Ph-(E-37a) | tBu | * |
| 2-058 | 3-CF₃-Ph | 5-Et-(E-37a) | tBu | 107-109 |
| 2-059 | 3-CF₃-Ph | 5-iPr-(E-37a) | tBu | * |
| 2-060 | 3-Me-Ph | 5-Me-(E-37a) | tBu | 69-71 |
| 2-061 | 3-Me-Ph | 5-cPr-(E-37a) | tBu | 94-96 |
| 2-062 | 3-CF₃-Ph | OPh | tBu | * |
| 2-063 | 3-CF₃-Ph | 4-Me-(E-25a) | tBu | * |
| 2-064 | 3-Me-Ph | C(=NOH)NH₂ | tBu | 161-163 |
| 2-065 | 3-CF₃-Ph | OBn | tBu | * |
| 2-066 | 3-CF₃-Ph | 5-cPr-(E-41a) | tBu | * |
| 2-067 | 3-CF₃-Ph | 5-CF₂H-(E-41a) | tBu | * |
| 2-068 | Ph | C(=NOH)NH₂ | tBu | 164-166 |
| 2-069 | 3-Cl-Ph | C(=NOH)NH₂ | tBu | 149-151 |
| 2-070 | 3-Me-Ph | 3-Me-(E-38a) | tBu | * |
| 2-071 | 3-CF₃-Ph | 5-nPr-(E-37a) | tBu | * |
| 2-072 | 3-CF₃-Ph | 5-CH₂OMe-(E-37a) | tBu | 84-87 |
| 2-073 | 3-CF₃-Ph | 5-iBu-(E-37a) | tBu | * |
| 2-074 | 3-CF₃-Ph | 5-CH₂CF₃-(E-37a) | tBu | * |
| 2-075 | 3-CF₃-Ph | 5-CCl₃-(E-37a) | tBu | * |
| 2-076 | Ph | 5-Me-(E-37a) | tBu | * |
| 2-077 | Ph | 5-CF₂H-(E-37a) | tBu | 122-124 |
| 2-078 | Ph | 5-cPr-(E-37a) | tBu | 136-138 |
| 2-079 | Ph | 3-Me-(E-38a) | tBu | * |
| 2-080 | Ph | 5-Me-(E-41a) | tBu | * |
| 2-081 | 3-Me-Ph | 5-Et-(E-41a) | tBu | * |
| 2-082 | 3-Me-Ph | 5-nPr-(E-41a) | tBu | * |
| 2-083 | 3-Me-Ph | 5-CH₂OMe-(E-41a) | tBu | * |
| 2-084 | 3-Me-Ph | 5-cPr-(E-41a) | tBu | * |
| 2-085 | 3-Me-Ph | 3-Et-(E-38a) | tBu | |
| 2-086 | 3-Me-Ph | 3-nPr-(E-38a) | tBu | * |
| 2-087 | 3-Me-Ph | 3-cPr-(E-38a) | tBu | * |
| 2-088 | 3-Me-Ph | 5-CF₃-(E-41a) | tBu | * |
| 2-089 | 3-Me-Ph | 5-CF₂H-(E-41a) | tBu | * |
| 2-090 | 3-Me-Ph | 5-Me-(E-41a) | tBu | 95-97 * |
| 2-091 | 3-CF₃-Ph | E-55a | tBu | 134-136 |
| 2-092 | 3-Me-Ph | 5-nPr-(E-37a) | tBu | * |
| 2-093 | 3-Me-Ph | 5-CH₂OMe-(E-37a) | tBu | * |
| 2-094 | 3-Me-Ph | 5-iBu-(E-37a) | tBu | * |
| 2-095 | 3-Me-Ph | 5-CH₂CF₃-(E-37a) | tBu | * |
| 2-096 | 3-Me-Ph | 5-CCl₃-(E-37a) | tBu | * |
| 2-097 | 3-Me-Ph | 5-CF₃-(E-37a) | tBu | * |
| 2-098 | 3-CF₃-Ph | 3-nPr-(E-38a) | tBu | * |
| 2-099 | 3-CF₃-Ph | 3-cPr-(E-38a) | tBu | * |
| 2-100 | 3-CF₃-Ph | OCH₂cPr | tBu | * |
| 2-101 | 3-CF₃-Ph | OCH₂(5-Cl-(E-2a)) | tBu | 99-101 |
| 2-102 | 3-CF₃-Ph | OCH₂(6-Me-(E-2a)) | tBu | 118-120 |
| 2-103 | Ph | OCH₂cPr | tBu | * |
| 2-104 | Ph | OCH₂(5-Cl-(E-2a)) | tBu | * |
| 2-105 | Ph | OCH₂(6-Me-(E-2a)) | tBu | * |
| 2-106 | 3-CF₃-Ph | 5-Et-(E-41a) | tBu | |
| 2-107 | 3-CF₃-Ph | 5-nPr-(E-41a) | tBu | * |
| 2-108 | 3-CF₃-Ph | 5-CH₂OMe-(E-41a) | tBu | * |
| 2-109 | 3-CF₃-Ph | 5-iBu-(E-41a) | tBu | * * |
| 2-110 | 3-CF₃-Ph | 5-CF₃-(E-41a) | tBu | * |
| 2-111 | 3-Me-Ph | OCH₂(5-Cl-(E-2a)) | tBu | * |
| 2-112 | 3-Me-Ph | OCH₂(6-Me-(E-2a)) | tBu | * |
| 2-113 | 3-CF₃-Ph | OCH₂CH=CH₂ | tBu | |
| 2-114 | 3-CF₃-Ph | OCH₂C≡CH | tBu | * * |
| 2-115 | 3-CF₃-Ph | 5-nBu-(E-37a) | tBu | * |
| 2-116 | 3-CF₃-Ph | 5-tBu-(E-37a) | tBu | * |
| 2-117 | 3-Me-Ph | 5-nBu-(E-37a) | tBu | |
| 2-118 | 3-Me-Ph | 5-tBu-(E-37a) | tBu | * |
| 2-119 | Ph | E-37a | tBu | * * |
| 2-120 | 3-CF₃-Ph | E-37a | tBu | * |
| 2-121 | 3-Me-Ph | E-37a | tBu | * |
| 2-122 | 3-Me-Ph | 5-CH(OH)Me-(E-37a) | tBu | * |
| 2-123 | 3-Me-Ph | 5-CHClMe-(E-37a) | tBu | * |
| 2-124 | 3-CF₃-Ph | 4-Me-(E-28a) | tBu | * |
| 2-125 | 3-Me-Ph | 5-CH(OMe)Me-(E-37a) | tBu | * |
| 2-126 | Ph | 5-nPr-(E-37a) | tBu | * |
| 2-127 | Ph | 5-iPr-(E-37a) | tBu | * |
| 2-128 | Ph | 5-nBu-(E-37a) | tBu | * |
| 2-129 | Ph | 5-CH₂OMe-(E-37a) | tBu | * |
| 2-130 | 3-Me-Ph | 5-(S-1a)-(E-37a) | tBu | * |
| 2-131 | 3-Me-Ph | 5-cHx-(E-37a) | tBu | * |
| 2-132 | 3-Me-Ph | 5-(S-6a)-(E-37a) | tBu | * |
| 2-133 | 3-Me-Ph | 5-cPen-(E-37a) | tBu | * |
| 2-134 | 3-Me-Ph | 5-CH₂tBu-(E-37a) | tBu | * |
| 2-135 | 3-Me-Ph | 5-nPen-(E-37a) | tBu | * |
| 2-136 | 3-Me-Ph | 5-nHx-(E-37a) | tBu | * |
| 2-137 | 3-Me-Ph | 5-CH₂C(O)OMe-(E-37a) | tBu | * |
| 2-138 | 3-Me-Ph | 5-C(O)OMe-(E-37a) | tBu | * |
| 2-139 | 3-Cl-Ph | 5-Me-(E-37a) | tBu | * |
| 2-140 | 3-Cl-Ph | 5-CF₂H-(E-37a) | tBu | * |
| 2-141 | 3-Cl-Ph | 5-Et-(E-37a) | tBu | * |
| 2-142 | 3-Cl-Ph | 5-cPr-(E-37a) | tBu | * |
| 2-143 | 3-CF₃-Ph | 3-iPr-(E-38a) | tBu | * |
| 2-144 | 3-Me-Ph | 3-iPr-(E-38a) | tBu | * |
| 2-145 | 3-Cl-Ph | 5-nPr-(E-37a) | tBu | * |
| 2-146 | 3-Cl-Ph | 5-CH₂OMe-(E-37a) | tBu | * |
| 2-147 | 3-Cl-Ph | 5-iBu-(E-37a) | tBu | * |
| 2-148 | 3-Cl-Ph | 5-CF₃-(E-37a) | tBu | * |
| 2-149 | 3-CF₃-Ph | 5-cPen-(E-37a) | tBu | * |
| 2-150 | 3-CF₃-Ph | 5-cHx-(E-37a) | tBu | * |
| 2-151 | 3-CF₃-Ph | 5-(S-6a)-(E-37a) | tBu | * |
| 2-152 | 3-CF₃-Ph | 5-CH₂tBu-(E-37a) | tBu | * |
| 2-153 | 3-CF₃-Ph | 5-nPen-(E-37a) | tBu | * |
| 2-154 | 3-CF₃-Ph | 5-nHx-(E-37a) | tBu | * |
| 2-155 | 3-Me-Ph | 5-(S-4a)-(E-37a) | tBu | * |
| 2-156 | 3-CF₃-Ph | 5-(S-4a)-(E-37a) | tBu | * |
| 2-157 | 3-Me-Ph | 5-Me-(E-28a) | tBu | * |
| 2-158 | 3-CF₃-Ph | 5-Me-(E-28a) | tBu | * |

**[Table 3]**

| 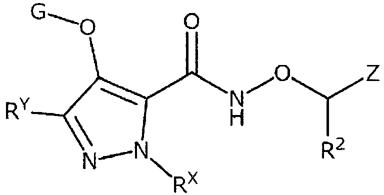 | | | | | | |
|---|---|---|---|---|---|---|
| No. | G | Z | R^{X} | R^{Y} | R² | m.p. (°C) |
| 3-001 | 3-CF₃-Ph | 4-Me-Ph | tBu | Br | H | * |
| 3-002 | 3-CF₃-Ph | 2,4-Me₂-Ph | tBu | Br | H | * |
| 3-003 | 3-CF₃-Ph | 4-Cl-Ph | tBu | Br | H | * |
| 3-004 | 3-CF₃-Ph | 2,4-Cl₂-Ph | tBu | Br | H | * |
| 3-005 | 3-CF₃-Ph | 4-OMe-Ph | tBu | Br | H | * |
| 3-006 | 3-CF₃-Ph | 2-F-4-OMe-Ph | tBu | Br | H | * |
| 3-007 | 3-CF₃-Ph | 4-Br-Ph | tBu | H | | * |
| 3-008 | 3-CF₃-Ph | 4-Me-Ph | tBu | Cl | Me H | * |
| 3-009 | 3-CF₃-Ph | 4-OMe-Ph | tBu | Cl | H | * |
| 3-010 | 3-CF₃-Ph | 4-OEt-Ph | tBu | Cl | H | * |
| 3-011 | 3-CF₃-Ph | 2-F-4-OMe-Ph | tBu | Cl | H | * |
| 3-012 | 3-CF₃-Ph | 2-Me-4-OMe-Ph | tBu | Cl | H | * |
| 3-013 | 3-CF₃-Ph | 2-Cl-4-OMe-Ph | tBu | Cl | H | * |
| 3-014 | 3-CF₃-Ph | 2,4-(OMe)₂-Ph | tBu | Cl | H | * |
| 3-015 | 3-CF₃-Ph | 2-OMe-4-Cl-Ph | tBu | Cl | H | * |
| 3-016 | 3-CF₃-Ph | 4-OCH₂CF₃-Ph | tBu | Cl | H | * |
| 3-017 | 3-CF₃-Ph | 4-Cl-Ph | tBu | H | Me | * |
| 3-018 | 3-CF₃-Ph | 4-Me-Ph | tBu | H | Me | * |
| 3-019 | 3-CF₃-Ph | 6-Cl-(Z-9a) | tBu | Cl | H | 48-50 |
| 3-020 | 3-CF₃-Ph | 6-OMe-(Z-9a) | tBu | Cl | H | * |
| 3-021 | 3-CF₃-Ph | 6-OEt-(Z-9a) | tBu | Cl | H | * |
| 3-022 | 3-CF₃-Ph | 4-OMe-Ph | tBu | H | Me | * |
| 3-023 | 3-CF₃-Ph | 4-CN-Ph | tBu | H | Me | * |
| 3-024 | 3-CF₃-Ph | 4-Cl-Ph | tBu | H | Et | * |
| 3-025 | 3-CF₃-Ph | 4-(5-Et-(E-37a))-Ph | tBu | H | Me | * |
| 3-026 | 3-CF₃-Ph | 4-(5-cPr-(E-37a))-Ph | tBu | H | Me | * |
| 3-027 | 3-CF₃-Ph | 4-(5-Me-(E-37a))-Ph | tBu | H | Me | * |
| 3-028 | 3-Me-Ph | 4-(5-Et-(E-37a))-Ph | tBu | H | Me | * |
| 3-029 | 3-Me-Ph | 4-(5-cPr-(E-37a))-Ph | tBu | H | Me | * |
| 3-030 | 3-Me-Ph | 4-(5-Me-(E-37a))-Ph | tBu | H | Me | * |
| 3-031 | 3-CF₃-Ph | 4-(5-Me-(E-37a))-Ph | tBu | Cl | H | * |
| 3-032 | 3-Me-Ph | 4-(5-cPr-(E-41a))-Ph | tBu | H | Me | * |

**[Table 4]**

| 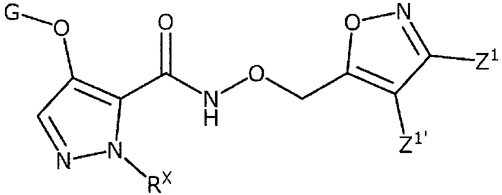 | | | | | |
|---|---|---|---|---|---|
| No. | G | Z¹ | Z^{1'} | R^{X} | m.p. (°C) |
| 4-001 | 3-CF₃-Ph | Me | H | tBu | * |
| 4-002 | 3-CF₃-Ph | Ph | H | tBu | * |
| 4-003 | 3-CF₃-Ph | 2-Cl-Ph | H | tBu | * |
| 4-004 | 3-CF₃-Ph | 3-Cl-Ph | H | tBu | * |
| 4-005 | 3-CF₃-Ph | 4-Cl-Ph | H | tBu | 144-146 |
| 4-006 | 3-CF₃-Ph | 2-Me-Ph | H | tBu | * |
| 4-007 | 3-CF₃-Ph | 3-Me-Ph | H | tBu | * |
| 4-008 | 3-CF₃-Ph | 4-Me-Ph | H | tBu | 138-140 |
| 4-009 | 3-CF₃-Ph | E-2a | H | tBu | 101-103 |
| 4-010 | 3-CF₃-Ph | E-4a | H | tBu | 135-137 |
| 4-011 | 3-CF₃-Ph | E-3a | H | tBu | * |
| 4-012 | 3-CF₃-Ph | 2-OMe-Ph | H | tBu | * |
| 4-013 | 3-CF₃-Ph | 2-F-Ph | H | tBu | * |
| 4-014 | 3-CF₃-Ph | E-13a | H | tBu | * |
| 4-015 | 3-CF₃-Ph | E-14a | H | tBu | * |

**[Table 5]**

| 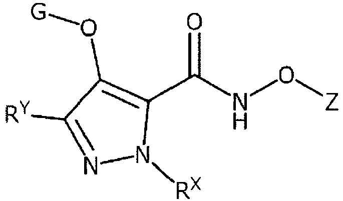 | | | | | |
|---|---|---|---|---|---|
| No. | G | z | R^{X} | R^{Y} | m.p. (°C) |
| 5-001 | 3-CF₃-Ph | 4-Me-Ph | tBu | H | 131-133 |
| 5-002 | Ph | 4-Me-Ph | tBu | H | * |

**[Table 6]**

| 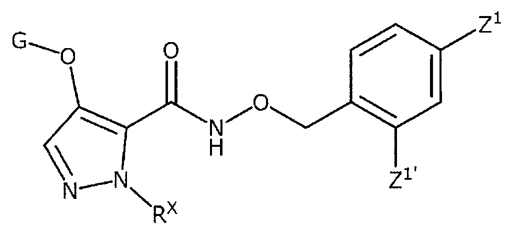 | | | | | |
|---|---|---|---|---|---|
| No. | G | Z¹ | Z¹' | R^{X} | m.p. (°C) |
| 6-001 | 3-CF₃-Ph | 3-Me-(E-38) | OMe | tBu | * |
| 6-002 | 3-CF₃-Ph | C(O)OMe | OMe | tBu | * |
| 6-003 | 3-CF₃-Ph | C(O)OH | OMe | tBu | 64-66 |
| 6-004 | 3-CF₃-Ph | OCH₂CF₂H | Me | tBu | * |
| 6-005 | Ph | OCH₂CF₂H | Me | tBu | * |
| 6-006 | 3-Me-Ph | OCH₂CF₂H | Me | tBu | * |
| 6-007 | 3-Me-Ph | OCH₂CH₂OMe | Me | tBu | * |
| 6-008 | 3-CF₃-Ph | OCH₂CH₂OMe | Me | tBu | * |

**[Table 7]**

| 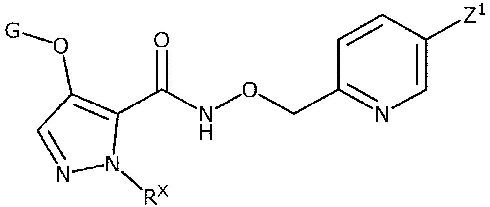 | | | | |
|---|---|---|---|---|
| No. | G | Z¹ | R^{X} | m.p. (°C) |
| 7-001 | 3-CF₃-Ph | 5-Me-(E-37) | tBu | * |

**[Table 8]**

| 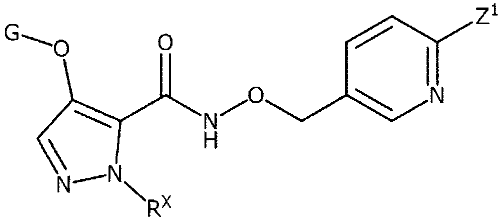 | | | | |
|---|---|---|---|---|
| No. | G | Z¹ | R^{X} | m.p. (°C) |
| 8-001 | 3-CF₃-Ph | 5-Me-(E-37) | tBu | * |

**[Table 9]**

| | | | | | |
|---|---|---|---|---|---|
| 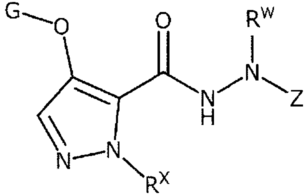 | | | | | |

| No. | G | Z | R^{X} | R^{W} | m.p. (°C) |
|---|---|---|---|---|---|
| 9-001 | 3-CF₃-Ph | 2,4-Me₂-Ph | tBu | H | 97-99 |
| 9-002 | Ph | 2,4-Me₂-Ph | tBu | Me | * |
| 9-003 | 3-Br-Ph | 2,4-Me₂-Ph | tBu | Me | * |
| 9-004 | 2,4-Cl₂-Ph | 2,4-Me₂-Ph | tBu | H | * |
| 9-005 | 2,4-Cl₂-Ph | 2,4-Me₂-Ph | tBu | Me | * |
| 9-006 | 3-Cl-Ph | 2,4-Me₂-Ph | tBu | H | 139-141 |
| 9-007 | 3-Cl-Ph | 2,4-Me₂-Ph | tBu | Me | * |
| 9-008 | 4-Cl-Ph | 2,4-Me₂-Ph | tBu | H | * |
| 9-009 | 4-Cl-Ph | 2,4-Me₂-Ph | tBu | Me | 114-116 |
| 9-010 | 2,3-Cl₂-Ph | 2,4-Me₂-Ph | tBu | Me | * |
| 9-011 | 3-F-Ph | 2,4-Me₂-Ph | tBu | H | 134-136 |
| 9-012 | 3-F-Ph | 2,4-Me₂-Ph | tBu | Me | * |
| 9-013 | 2-Cl-Ph | 2,4-Me₂-Ph | tBu | H | * |
| 9-014 | 2-Cl-Ph | 2,4-Me₂-Ph | tBu | Me | * |
| 9-015 | 3,4-Cl₂-Ph | 2,4-Me₂-Ph | tBu | H | 136-138 |
| 9-016 | 3,4-Cl₂-Ph | 2,4-Me₂-Ph | tBu | Me | * |
| 9-017 | Ph | 2,4-Me₂-Ph | tBu | C(O)OMe | 191-193 |

**[Table 10]**

| 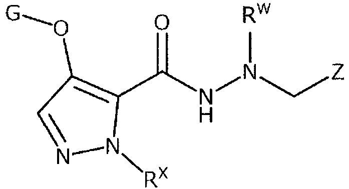 | | | | | |
|---|---|---|---|---|---|
| No. | G | Z | R^{X} | R^{W} | m.p. (°C) |
| 10-001 | 3-CF₃-Ph | 2,4-Me₂-Ph | tBu | C(O)OtBu | * |
| 10-002 | Ph | 2,4-Me₂-Ph | tBu | H | * |
| 10-003 | Ph | 2,4-Me₂-Ph | tBu | C(O)OCH₂CH=CH 2 | * |
| 10-004 | Ph | 2,4-Me₂-Ph | tBu | CH₂CN | * |
| 10-005 | 3-CF₃-Ph | 2,4-Me₂-Ph | tBu | C(O)OCH₂CH=CH 2 | * |
| 10-006 | 3-CF₃-Ph | 2,4-Me₂-Ph | tBu | H | * |
| 10-007 | 3-CF₃-Ph | 2,4-Me₂-Ph | tBu | CH₂CN | * |
| 10-008 | 3-Br-Ph | 2,4-Me₂-Ph | tBu | C(O)OCH₂CH=CH 2 | 109-111 |
| 10-009 | 2-F-3-Cl-Ph | 2,4-Me₂-Ph | tBu | C(O)OCH₂CH=CH ₂ | 107-109 |
| 10-010 | 3-CF₃-Ph | 2,4-Me₂-Ph | tBu | C(O)OMe | 127-129 |
| 10-011 | 3-CF₃-Ph | 2,4-Me₂-Ph | tBu | C(O)OEt | 114-116 |
| 10-012 | 3-CF₃-Ph | 2,4-Me₂-Ph | tBu | C(O)NHEt | 176-178 |
| 10-013 | Ph | 2,4-Me₂-Ph | tBu | C(O)OMe | 157-159 |
| 10-014 | Ph | 2,4-Me₂-Ph | tBu | C(O)OEt | * |
| 10-015 | Ph | 2,4-Me₂-Ph | tBu | C(O)NHEt | 129-132 |
| 10-016 | Ph | 4-Br-Ph | tBu | C(O)OtBu | * |
| 10-017 | 2-F-3-Cl-Ph | 2,4-Me₂-Ph | tBu | H | * |
| 10-018 | Ph | 2,4-Me₂-Ph | tBu | C(O)Me | 157-159 |
| 10-019 | Ph | 2,4-Me₂-Ph | tBu | C(O)Et | 94-96 |
| 10-020 | Ph | 2,4-Me₂-Ph | tBu | C(S)NHEt | 121-123 |
| 10-021 | 3-CF₃-Ph | 2,4-Me₂-Ph | tBu | C(S)NHEt | 132-134 |
| 10-022 | 3-CF₃-Ph | 2,4-Me₂-Ph | tBu | SO₂Et | 147-149 |
| 10-023 | 3-CF₃-Ph | 2,4-Me₂-Ph | tBu | CH₂C(=NOMe)Me | 137-139 |
| 10-024 | 3-CF₃-Ph | 2,4-Me₂-Ph | tBu | C(O)Me | * |
| 10-025 | 3-CF₃-Ph | 2,4-Me₂-Ph | tBu | CH₂C(O)OMe | * |
| 10-026 | 3-CF₃-Ph | 2,4-Me₂-Ph | tBu | CH₂C(O)Me | * |
| 10-027 | 3-CF₃-Ph | 2,4-Me₂-Ph | tBu | C(O)Et | 129-131 |
| 10-028 | 3-CF₃-Ph | 2,4-Me₂-Ph | tBu | Me | 114-116 |
| 10-029 | Ph | 2,4-Me₂-Ph | tBu | C(O)NMe₂ | 120-123 |
| 10-030 | Ph | 2,4-Me₂-Ph | tBu | SO₂Et | 127-130 |
| 10-031 | Ph | 2,4-Me₂-Ph | tBu | CH₂C(O)Me | 112-114 |
| 10-032 | Ph | 2,4-Me₂-Ph | tBu | CH₂CH=CH₂ | * |
| 10-033 | Ph | 2,4-Me₂-Ph | tBu | CH₂C(=NOMe)Me | 85-88 |
| 10-034 | Ph | 2,4-Me₂-Ph | tBu | C(O)OiPr | 100-103 |
| 10-035 | Ph | 2,4-Me₂-Ph | tBu | C(O)OCH₂CH₂OM e | * |
| 10-036 | 3-Br-Ph | 2,4-Me₂-Ph | tBu | H | * |
| 10-037 | 3-CF₃-Ph | 2,4-Me₂-Ph | tBu | C(O)NMe₂ | 132-134 |
| 10-038 | 3-CF₃-Ph | 2,4-Cl₂-Ph | tBu | C(O)OCH₂CH=CH ₂ | * |
| 10-039 | 3-CF₃-Ph | 4-Me-Ph | tBu | C(O)OCH₂CH=CH ₂ | 124-126 |
| 10-040 | 3-CF₃-Ph | 4-Me-Ph | tBu | H | * |
| 10-041 | Ph | 2,4-Cl₂-Ph | tBu | C(O)OCH₂CH=CH ₂ | * |
| 10-042 | Ph | 4-Me-Ph | tBu | C(O)OCH₂CH=CH ₂ | * |
| 10-043 | 3-CF₃-Ph | 2,4-Me₂-Ph | tBu | C(S)NHMe | 179-181 |
| 10-044 | Ph | 2,4-Me₂-Ph | tBu | C(S)NHMe | 127-129 |
| 10-045 | 3-CF₃-Ph | 4-Me-Ph | tBu | C(O)OMe | 110-112 |
| 10-046 | 3-CF₃-Ph | 4-Me-Ph | tBu | C(S)NHMe | 177-179 |
| 10-047 | 3-CF₃-Ph | 4-Me-Ph | tBu | C(O)NHEt | 154-156 |
| 10-048 | 3-Cl-Ph | 2,4-Me₂-Ph | tBu | H | * |
| 10-049 | 4-Cl-Ph | 2,4-Me₂-Ph | tBu | H | * |
| 10-050 | 3-F-Ph | 2,4-Me₂-Ph | tBu | C(O)OCH₂CH=CH ₂ | 77-79 |
| 10-051 | 3-F-Ph | 2,4-Me₂-Ph | tBu | H | * |
| 10-052 | 3-F-Ph | 2,4-Me₂-Ph | tBu | C(O)OMe | 162-164 |
| 10-053 | Ph | 2,4-Cl₂-Ph | tBu | H | * |
| 10-054 | Ph | 4-Me-Ph | tBu | H | * |
| 10-055 | 3-CF₃-Ph | 2,4-Cl₂-Ph | tBu | H | * |
| 10-056 | 3-CF₃-Ph | 2,4-Me₂-Ph | tBu | L-1 | 154-156 |
| 10-057 | Ph | 2,4-Me₂-Ph | tBu | L-1 | 128-130 |
| 10-058 | Ph | 2,4-Cl₂-Ph | tBu | C(O)OMe | 142-144 |
| 10-059 | Ph | 4-Me-Ph | tBu | C(O)OMe | 117-119 |
| 10-060 | Ph | 2,4-Cl₂-Ph | tBu | C(O)OEt | * |
| 10-061 | Ph | 4-Me-Ph | tBu | C(O)OEt | * |
| 10-062 | 3-Cl-Ph | 2,4-Me₂-Ph | tBu | C(O)OMe | 144-146 |
| 10-063 | 4-Cl-Ph | 2,4-Me₂-Ph | tBu | C(O)OMe | 122-124 |
| 10-064 | 4-Cl-Ph | 2,4-Me₂-Ph | tBu | C(O)OCH₂CH=CH ₂ | 87-89 |
| 10-065 | Ph | 2,4-Me₂-Ph | tBu | C(O)O(4-Cl-Ph) | * |
| 10-066 | Ph | 2,4-Me₂-Ph | tBu | C(O)O(4-NO₂-Ph) | * |
| 10-067 | 3-CF₃-Ph | 2,4-Me₂-Ph | iPr | C(O)OCH₂CH=CH ₂ | 79-81 |
| 10-068 | 3-CF₃-Ph | 2,4-Me₂-Ph | iPr | H | * |
| 10-069 | 3-CF₃-Ph | 2,4-Me₂-Ph | iPr | C(O)OMe | 69-71 |
| 10-070 | 3-CF₃-Ph | 2,4-Me₂-Ph | Ph | C(O)OCH₂CH=CH ₂ | 126-128 |
| 10-071 | 3-CF₃-Ph | 2,4-Me₂-Ph | Ph | H | 102-104 |
| 10-072 | 3-CF₃-Ph | 2,4-Me₂-Ph | Ph | C(O)OMe | 54-57 |
| 10-073 | Ph | 2,4-Me₂-Ph | iPr | C(O)OCH₂CH=CH ₂ | * |
| 10-074 | Ph | 2,4-Me₂-Ph | iPr | H | 62-64 |
| 10-075 | Ph | 2,4-Me₂-Ph | iPr | C(O)OMe | 92-94 |
| 10-076 | Ph | 2,4-Me₂-Ph | Ph | C(O)OCH₂CH=CH ₂ | 43-45 |
| 10-077 | Ph | 2,4-Me₂-Ph | Ph | H | 79-81 |
| 10-078 | Ph | 2,4-Me₂-Ph | Ph | C(O)OMe | 119-121 |
| 10-079 | 2-F-3-Br-Ph | 2,4-Me₂-Ph | tBu | C(O)OCH₂CH=CH ₂ | |
| 10-080 | 2-F-3-Br-Ph | 2,4-Me₂-Ph | tBu | H | |
| 10-081 | 2-F-3-Br-Ph | 2,4-Me₂-Ph | tBu | C(O)OMe | |
| 10-082 | 2-F-3-cPr-Ph | 2,4-Me₂-Ph | tBu | C(O)OCH₂CH=CH ₂ | |
| 10-083 | 2-F-3-cPr-Ph | 2,4-Me₂-Ph | tBu | H | |
| 10-084 | 2-F-3-cPr-Ph | 2,4-Me₂-Ph | tBu | C(O)OMe | |
| 10-085 | 3-CF₃-Ph | 2,4-Me₂-Ph | F-2a | C(O)OCH₂CH=CH ₂ | 117-119 |
| 10-086 | 3-CF₃-Ph | 2,4-Me₂-Ph | F-2a | H | 113-115 |
| 10-087 | 3-CF₃-Ph | 2,4-Me₂-Ph | F-2a | C(O)OMe | 105-107 |
| 10-088 | 3-CF₃-Ph | 2,4-Me₂-Ph | F-3a | C(O)OCH₂CH=CH ₂ | 149-151 |
| 10-089 | 3-CF₃-Ph | 2,4-Me₂-Ph | F-3a | H | 50-52 |
| 10-090 | 3-CF₃-Ph | 2,4-Me₂-Ph | F-3a | C(O)OMe | 137-139 |
| 10-091 | 3-CF₃-Ph | 2,4-Me₂-Ph | F-4a | C(O)OCH₂CH=CH ₂ | * |
| 10-092 | 3-CF₃-Ph | 2,4-Me₂-Ph | F-4a | H | 87-89 |
| 10-093 | 3-CF₃-Ph | 2,4-Me₂-Ph | F-4a | C(O)OMe | |
| 10-094 | Ph | 2,4-Me₂-Ph | F-2a | C(O)OCH₂CH=CH ₂ | 51-53 |
| 10-095 | Ph | 2,4-Me₂-Ph | F-2a | H | 50-52 |
| 10-096 | Ph | 2,4-Me₂-Ph | F-2a | C(O)OMe | 55-57 |
| 10-097 | Ph | 2,4-Me₂-Ph | F-3a | C(O)OCH₂CH=CH ₂ | 97-99 |
| 10-098 | Ph | 2,4-Me₂-Ph | F-3a | H | * |
| 10-099 | Ph | 2,4-Me₂-Ph | F-3a | C(O)OMe | 126-128 |
| 10-100 | Ph | 2,4-Me₂-Ph | F-4a | C(O)OCH₂CH=CH ₂ | 134-136 |
| 10-101 | Ph | 2,4-Me₂-Ph | F-4a | H | 62-64 |
| 10-102 | Ph | 2,4-Me₂-Ph | F-4a | C(O)OMe | |
| 10-103 | 3-CF₃-Ph | 2,4-Me₂-Ph | F-2a | C(O)OEt | 139-141 |
| 10-104 | 3-CF₃-Ph | 2,4-Me₂-Ph | F-3a | C(O)OEt | 147-149 |
| 10-105 | 3-CF₃-Ph | 2,4-Me₂-Ph | F-4a | C(O)OEt | |
| 10-106 | Ph | 2,4-Me₂-Ph | F-2a | C(O)OEt | 107-109 |
| 10-107 | Ph | 2,4-Me₂-Ph | F-3a | C(O)OEt | 54-56 |
| 10-108 | Ph | 2,4-Me₂-Ph | F-4a | C(O)OEt | |
| 10-109 | 3-Br-Ph | 2,4-Me₂-Ph | tBu | C(O)OMe | 122-124 |
| 10-110 | 3-Br-Ph | 2,4-Me₂-Ph | tBu | C(O)OEt | 107-109 |
| 10-111 | 3-Me-Ph | 2,4-Me₂-Ph | tBu | C(O)OCH₂CH=CH ₂ | 92-94 |
| 10-112 | 3-Me-Ph | 2,4-Me₂-Ph | tBu | H | * |
| 10-113 | 3-Me-Ph | 2,4-Me₂-Ph | tBu | C(O)OMe | 137-139 |
| 10-114 | 3-Me-Ph | 2,4-Me₂-Ph | tBu | C(O)OEt | 114-116 |
| 10-115 | 3-CF₃-Ph | 2,4-Me₂-Ph | tBu | C(O)OCH₂CH₂OM e | 114-116 |
| 10-116 | 3-CF₃-Ph | 2,4-Cl₂-Ph | tBu | C(O)OCH₂CH₂OM e | * |
| 10-117 | 3-CF₃-Ph | 4-Me-Ph | tBu | C(O)OCH₂CH₂OM e | 119-121 |
| 10-118 | Ph | 2,4-Cl₂-Ph | tBu | C(O)OCH₂CH₂OM e | * |
| 10-119 | Ph | 4-Me-Ph | tBu | C(O)OCH₂CH₂OM e | * |
| 10-120 | Ph | 2,4-Me₂-Ph | tBu | C(O)OBn | * |
| 10-121 | Ph | 2,4-Me₂-Ph | tBu | C(O)OCH₂CCl₃ | 97-99 |
| 10-122 | 2-F-3-Cl-Ph | 2,4-Me₂-Ph | tBu | C(O)OMe | 145-147 |
| 10-123 | 2-Cl-Ph | 2,4-Me₂-Ph | tBu | C(O)OMe | 122-124 |
| 10-124 | 3,4-Cl₂-Ph | 2,4-Me₂-Ph | tBu | C(O)OMe | 120-122 |
| 10-125 | 2,4-C1₂-Ph | 2,4-Me₂-Ph | tBu | C(O)OMe | 125-127 |
| 10-126 | 3-I-Ph | 2,4-Me₂-Ph | tBu | C(O)OMe | 119-121 |
| 10-127 | 3-CF₃-Ph | 2,4-Cl₂-Ph | tBu | CH₂C(O)Me | * |
| 10-128 | 3-CF₃-Ph | 2,4-Cl₂-Ph | tBu | CH₂C(O)OMe | 97-99 |
| 10-129 | 3-CF₃-Ph | 2,4-Cl₂-Ph | tBu | CH₂C(=NOMe)Me | 112-114 |
| 10-130 | 3-CF₃-Ph | 2,4-Cl₂-Ph | tBu | Me | 94-96 |
| 10-131 | 3-CF₃-Ph | 2,4-Cl₂-Ph | tBu | C(O)Me | 81-83 |
| 10-132 | 3-CF₃-Ph | 2,4-Cl₂-Ph | tBu | C(O)Et | 99-101 |
| 10-133 | Ph | 2,4-Me₂-Ph | tBu | L-2 | * |
| 10-134 | 3-CF₃-Ph | 2,4-Cl₂-Ph | tBu | C(O)NHEt | 172-174 |
| 10-135 | 3-CF₃-Ph | 2,4-Cl₂-Ph | tBu | C(S)NHEt | * |
| 10-136 | 3-CF₃-Ph | 2,4-Cl₂-Ph | tBu | C(O)OMe | * |
| 10-137 | 3-CF₃-Ph | 2,4-Cl₂-Ph | tBu | C(O)OEt | * |
| 10-138 | 2,3-Cl₂-Ph | 2,4-Me₂-Ph | tBu | C(O)OMe | 105-107 |
| 10-139 | 3-OCF₃-Ph | 2,4-Me₂-Ph | tBu | C(O)OMe | 120-122 |
| 10-140 | Ph | 2,4-Me₂-Ph | tBu | L-3 | * |
| 10-141 | Ph | 2,4-Me₂-Ph | tBu | L-4 | * |
| 10-142 | 3-OMe-Ph | 2,4-Me₂-Ph | tBu | C(O)OMe | 125-127 |
| 10-143 | Ph | 2,4-Me₂-Ph | tBu | C(O)StBu | * |
| 10-144 | 3-Cl-Ph | 2,4-Me₂-Ph | tBu | C(O)OEt | 112-114 |
| 10-145 | 3-F-Ph | 2,4-Me₂-Ph | tBu | C(O)OEt | 122-124 |
| 10-146 | Ph | 4-Br-Ph | tBu | H | * |
| 10-147 | Ph | 4-Br-Ph | tBu | C(O)OMe | 125-127 |
| 10-148 | Ph | 4-Br-Ph | tBu | C(O)OEt | 101-103 |
| 10-149 | Ph | 4-Br-Ph | tBu | C(O)OCH₂CH=CH ₂ | * |
| 10-150 | 3-CF₃-Ph | 4-Br-Ph | tBu | C(O)OMe | 48-50 |
| 10-151 | 3-CF₃-Ph | 4-Br-Ph | tBu | C(O)OEt | 132-134 |
| 10-152 | 3-CF₃-Ph | 4-Br-Ph | tBu | C(O)OCH₂CH=CH ₂ | 127-129 |
| 10-153 | Ph | 4-OMe-Ph | tBu | C(O)OMe | * |
| 10-154 | Ph | 4-OMe-Ph | tBu | C(O)OEt | * |
| 10-155 | Ph | 4-OMe-Ph | tBu | C(O)OCH₂CH=CH ₂ | * |
| 10-156 | Ph | 4-OEt-Ph | tBu | C(O)OMe | * |
| 10-157 | Ph | 4-OEt-Ph | tBu | C(O)OEt | 85-87 |
| 10-158 | Ph | 4-OEt-Ph | tBu | C(O)OCH₂CH=CH ₂ | * |
| 10-159 | Ph | 4-OEt-Ph | tBu | H | * |
| 10-160 | 3-CF₃-Ph | 2-Me-4-Br-Ph | tBu | H | * |
| 10-161 | Ph | 2-Me-4-Br-Ph | tBu | H | * |
| 10-162 | 3-CF₃-Ph | 4-OMe-Ph | tBu | C(O)OMe | 118-121 |
| 10-163 | 3-CF₃-Ph | 4-OMe-Ph | tBu | C(O)OEt | 135-138 |
| 10-164 | 3-CF₃-Ph | 4-OMe-Ph | tBu | C(O)OCH₂CH=CH ₂ | 109-111 |
| 10-165 | Ph | 2-Cl-4-Br-Ph | tBu | H | * |
| 10-166 | Ph | 2-Cl-4-Br-Ph | tBu | C(O)OMe | 135-136 |
| 10-167 | Ph | 2-Cl-4-Br-Ph | tBu | C(O)OEt | 98-99 |
| 10-168 | Ph | 2-Cl-4-Br-Ph | tBu | C(O)OCH₂CH=CH ₂ | * |
| 10-169 | Ph | 2,4-Me₂-Ph | tBu | C(O)OCH₂CH=CH Et | * |
| 10-170 | 3-CF₃-Ph | 4-OEt-Ph | tBu | H | * |
| 10-171 | Ph | 4-OMe-Ph | tBu | H | * |
| 10-172 | 3-CF₃-Ph | 4-OMe-Ph | tBu | H | * |
| 10-173 | Ph | 4-Cl-Ph | tBu | H | * |
| 10-174 | Ph | 4-Cl-Ph | tBu | C(O)OMe | * |
| 10-175 | Ph | 4-Cl-Ph | tBu | C(O)OEt | 97-100 |
| 10-176 | Ph | 4-Cl-Ph | tBu | C(O)OCH₂CH=CH ₂ | * |
| 10-177 | Ph | 2,4-Me₂-Ph | tBu | C(O)CH₂C(O)OEt | * |
| 10-178 | Ph | 2,4-Me₂-Ph | tBu | C(O)CH₂OMe | * |
| 10-179 | Ph | 2,4-Me₂-Ph | tBu | C(O)CH=CHOEt | * |
| 10-180 | Ph | 2,4-Me₂-Ph | tBu | C(O)C(O)OEt | * |
| 10-181 | Ph | 2,4-Me₂-Ph | tBu | C(O)OtBu | * |
| 10-182 | Ph | 2-Cl-4-Br-Ph | tBu | C(O)CF₃ | * |
| 10-183 | Ph | 2-Cl-4-Br-Ph | tBu | C(O)cPr | 136-138 |
| 10-184 | 3-CF₃-Ph | 2-Me-4-Br-Ph | tBu | C(O)OMe | 127-129 |
| 10-185 | 3-CF₃-Ph | 2-Me-4-Br-Ph | tBu | C(O)OEt | 102-104 |
| 10-186 | 3-CF₃-Ph | 2-Me-4-Br-Ph | tBu | C(O)OCH₂CH=CH ₂ | 107-109 |
| 10-187 | Ph | 2-Me-4-Br-Ph | tBu | C(O)OMe | 156-158 |
| 10-188 | Ph | 2-Me-4-Br-Ph | tBu | C(O)OEt | 124-126 |
| 10-189 | Ph | 2-Me-4-Br-Ph | tBu | C(O)OCH₂CH=CH ₂ | 85-87 |
| 10-190 | 3-Cl-Ph | 2,4-Me₂-Ph | tBu | CH₂cPr | 94-96 |
| 10-191 | Ph | 2-Me-4-Cl-Ph | tBu | C(O)OMe | 159-161 |
| 10-192 | Ph | 2-Me-4-Cl-Ph | tBu | C(O)OEt | 127-129 |
| 10-193 | Ph | 2-Me-4-Cl-Ph | tBu | C(O)OCH₂CH=CH ₂ | * |
| 10-194 | 3-CF₃-Ph | 4-Cl-Ph | tBu | H | * |
| 10-195 | 3-CF₃-Ph | 4-Cl-Ph | tBu | C(O)OMe | * |
| 10-196 | Ph | 2-Cl-4-OMe-Ph | tBu | C(O)OMe | 90-92 |
| 10-197 | Ph | 2-Cl-4-OMe-Ph | tBu | C(O)OEt | * |
| 10-198 | Ph | 2-Cl-4-OMe-Ph | tBu | C(O)OCH₂CH=CH ₂ | * |
| 10-199 | 3-CF₃-Ph | 2-Cl-4-OMe-Ph | tBu | C(O)OMe | * |
| 10-200 | 3-CF₃-Ph | 2-Cl-4-OMe-Ph | tBu | C(O)OEt | * |
| 10-201 | 3-CF₃-Ph | 2-Cl-4-OMe-Ph | tBu | C(O)OCH₂CH=CH ₂ | * |
| 10-202 | Ph | 2-Cl-4-OMe-Ph | tBu | H | * |
| 10-203 | 3-CF₃-Ph | 2-Cl-4-OMe-Ph | tBu | H | * |
| 10-204 | 3-CF₃-Ph | 4-Cl-Ph | tBu | C(O)OEt | 131-133 |
| 10-205 | 3-CF₃-Ph | 4-Cl-Ph | tBu | C(O)OCH₂CH=CH ₂ | 129-131 |
| 10-206 | 3-CF₃-Ph | 4-Br-Ph | tBu | H | * |
| 10-207 | Ph | 2-Me-4-Cl-Ph | tBu | H | * |
| 10-208 | 3-CF₃-Ph | 4-OEt-Ph | tBu | C(O)OMe | * |
| 10-209 | 3-CF₃-Ph | 4-OEt-Ph | tBu | C(O)OEt | * |
| 10-210 | 3-CF₃-Ph | 4-OEt-Ph | tBu | C(O)OCH₂CH=CH ₂ | * |
| 10-211 | 3-CF₃-Ph | 2-Cl-4-Br-Ph | tBu | H | * |
| 10-212 | 3-CF₃-Ph | 2-Cl-4-Br-Ph | tBu | C(O)OMe | * |
| 10-213 | 3-CF₃-Ph | 2-Cl-4-Br-Ph | tBu | C(O)OEt | * |
| 10-214 | 3-CF₃-Ph | 2-Cl-4-Br-Ph | tBu | C(O)OCH₂CH=CH ₂ | * |
| 10-215 | 3-CF₃-Ph | 2-Me-4-Cl-Ph | tBu | C(O)OMe | * |
| 10-216 | 3-CF₃-Ph | 2-Me-4-Cl-Ph | tBu | C(O)OEt | * |
| 10-217 | 3-CF₃-Ph | 2-Me-4-Cl-Ph | tBu | C(O)OCH₂CH=CH ₂ | * |
| 10-218 | 3-CF₃-Ph | 2-Me-4-Cl-Ph | tBu | H | * |
| 10-219 | Ph | 2-Cl-4-Me-Ph | tBu | H | * |
| 10-220 | 3-CF₃-Ph | 2-Cl-4-Me-Ph | tBu | H | * |
| 10-221 | Ph | 2-Cl-4-Me-Ph | tBu | C(O)OMe | 130-133 |
| 10-222 | Ph | 2-Cl-4-Me-Ph | tBu | C(O)OEt | 63-66 |
| 10-223 | Ph | 2-Cl-4-Me-Ph | tBu | C(O)OCH₂CH=CH ₂ | * |
| 10-224 | 3-CF₃-Ph | 2-Cl-4-Me-Ph | tBu | C(O)OMe | 132-135 |
| 10-225 | 3-CF₃-Ph | 2-Cl-4-Me-Ph | tBu | C(O)OEt | 85-88 |
| 10-226 | 3-CF₃-Ph | 2-Cl-4-Me-Ph | tBu | C(O)OCH₂CH=CH ₂ | 100-103 |
| 10-227 | 3-Cl-Ph | 2,4-Me₂-Ph | tBu | CH₂CF₃ | 89-91 |
| 10-228 | 3-Cl-Ph | 2,4-Me₂-Ph | tBu | C(O)cPr | 137-139 |
| 10-229 | 3-Cl-Ph | 2,4-Me₂-Ph | tBu | C(O)CF₃ | * |
| 10-230 | 3-F-Ph | 2,4-Me₂-Ph | tBu | CH₂SiMe₃ | * |
| 10-231 | 4-Cl-Ph | 2,4-Me₂-Ph | tBu | C(O)CH₂OMe | 57-59 |
| 10-232 | 4-Cl-Ph | 2,4-Me₂-Ph | tBu | C(O)CH₂SO₂Me | 67-69 |
| 10-233 | 4-Cl-Ph | 2,4-Me₂-Ph | tBu | C(O)CH₂NMe₂ | 59-61 |
| 10-234 | 4-CF₃-Ph | 2,4-Me₂-Ph | tBu | C(O)OMe | 98-100 |
| 10-235 | 2-CF₃-Ph | 2,4-Me₂-Ph | tBu | C(O)OMe | * |
| 10-236 | Ph | 2-Br-4-Me-Ph | tBu | C(O)OMe | 127-129 |
| 10-237 | Ph | 2-Br-4-Cl-Ph | tBu | C(O)OMe | 136-138 |
| 10-238 | Ph | 2,4-Br₂-Ph | tBu | C(O)OMe | 122-124 |
| 10-239 | Ph | 2,4-(OMe)₂-Ph | tBu | C(O)OMe | * |
| 10-240 | Ph | 2-F-4-OMe-Ph | tBu | C(O)OMe | * |
| 10-241 | Ph | 2-OMe-4-F-Ph | tBu | C(O)OMe | * |
| 10-242 | Ph | 2-F-4-Cl-Ph | tBu | C(O)OMe | 119-121 |
| 10-243 | Ph | 2-Me-4-F-Ph | tBu | C(O)OMe | 149-151 |
| 10-244 | Ph | 2-F-4-Me-Ph | tBu | C(O)OMe | * |
| 10-245 | Ph | 2-Br-4-Me-Ph | tBu | C(O)OEt | * |
| 10-246 | Ph | 2-Br-4-Cl-Ph | tBu | C(O)OEt | * |
| 10-247 | Ph | 2,4-Br₂-Ph | tBu | C(O)OEt | * |
| 10-248 | Ph | 2,4-(OMe)₂-Ph | tBu | C(O)OEt | * |
| 10-249 | Ph | 2-F-4-OMe-Ph | tBu | C(O)OEt | * |
| 10-250 | Ph | 2-OMe-4-F-Ph | tBu | C(O)OEt | * |
| 10-251 | Ph | 2-F-4-Cl-Ph | tBu | C(O)OEt | * |
| 10-252 | Ph | 2-Me-4-F-Ph | tBu | C(O)OEt | 109-111 |
| 10-253 | Ph | 2-F-4-Me-Ph | tBu | C(O)OEt | * |
| 10-254 | Ph | 2-Br-4-Me-Ph | tBu | C(O)OCH₂CH=CH ₂ | * |
| 10-255 | Ph | 2-Br-4-Cl-Ph | tBu | C(O)OCH₂CH=CH ₂ | * |
| 10-256 | Ph | 2,4-Br₂-Ph | tBu | C(O)OCH₂CH=CH ₂ | * |
| 10-257 | Ph | 2,4-(OMe)₂-Ph | tBu | C(O)OCH₂CH=CH ₂ | * |
| 10-258 | Ph | 2-F-4-OMe-Ph | tBu | C(O)OCH₂CH=CH ₂ | * |
| 10-259 | Ph | 2-OMe-4-F-Ph | tBu | C(O)OCH₂CH=CH ₂ | * |
| 10-260 | Ph | 2-F-4-Cl-Ph | tBu | C(O)OCH₂CH=CH ₂ | * |
| 10-261 | Ph | 2-Me-4-F-Ph | tBu | C(O)OCH₂CH=CH ₂ | * |
| 10-262 | Ph | 2-F-4-Me-Ph | tBu | C(O)OCH₂CH=CH ₂ | * |
| 10-263 | Ph | 2-Br-4-Me-Ph | tBu | H | * |
| 10-264 | Ph | 2-Br-4-Cl-Ph | tBu | H | * |
| 10-265 | Ph | 2,4-Br₂-Ph | tBu | H | * |
| 10-266 | Ph | 2,4-(OMe)₂-Ph | tBu | H | * |
| 10-267 | Ph | 2-F-4-OMe-Ph | tBu | H | * |
| 10-268 | Ph | 2-OMe-4-F-Ph | tBu | H | * |
| 10-269 | Ph | 2-F-4-Cl-Ph | tBu | H | * |
| 10-270 | Ph | 2-Me-4-F-Ph | tBu | H | * |
| 10-271 | Ph | 2-F-4-Me-Ph | tBu | H | * |
| 10-272 | Ph | 4-Et-Ph | tBu | C(O)OMe | 62-64 |
| 10-273 | Ph | 4-iPr-Ph | tBu | C(O)OMe | 114-116 |
| 10-274 | Ph | 4-SMe-Ph | tBu | C(O)OMe | * |
| 10-275 | Ph | 4-NO₂-Ph | tBu | C(O)OMe | 109-111 |
| 10-276 | Ph | 4-Ph-Ph | tBu | C(O)OMe | * |
| 10-277 | Ph | 4-OCF₃-Ph | tBu | C(O)OMe | * |
| 10-278 | Ph | 4-SCF₃-Ph | tBu | C(O)OMe | * |
| 10-279 | Ph | 4-I-Ph | tBu | C(O)OMe | 129-131 |
| 10-280 | Ph | 4-CF₃-Ph | tBu | C(O)OMe | * |
| 10-281 | Ph | 4-CN-Ph | tBu | C(O)OMe | 126-128 |
| 10-282 | Ph | 4-Et-Ph | tBu | C(O)OEt | * |
| 10-283 | Ph | 4-iPr-Ph | tBu | C(O)OEt | * |
| 10-284 | Ph | 4-SMe-Ph | tBu | C(O)OEt | 84-86 |
| 10-285 | Ph | 4-NO₂-Ph | tBu | C(O)OEt | * |
| 10-286 | Ph | 4-Ph-Ph | tBu | C(O)OEt | * |
| 10-287 | Ph | 4-OCF₃-Ph | tBu | C(O)OEt | * |
| 10-288 | Ph | 4-SCF₃-Ph | tBu | C(O)OEt | * |
| 10-289 | Ph | 4-I-Ph | tBu | C(O)OEt | * |
| 10-290 | Ph | 4-CF₃-Ph | tBu | C(O)OEt | * |
| 10-291 | Ph | 4-CN-Ph | tBu | C(O)OEt | * |
| 10-292 | Ph | 4-Et-Ph | tBu | C(O)OCH₂CH=CH ₂ | * |
| 10-293 | Ph | 4-iPr-Ph | tBu | C(O)OCH₂CH=CH ₂ | * |
| 10-294 | Ph | 4-SMe-Ph | tBu | C(O)OCH₂CH=CH ₂ | * |
| 10-295 | Ph | 4-NO₂-Ph | tBu | C(O)OCH₂CH=CH ₂ | * |
| 10-296 | Ph | 4-Ph-Ph | tBu | C(O)OCH₂CH=CH ₂ | * |
| 10-297 | Ph | 4-OCF₃-Ph | tBu | C(O)OCH₂CH=CH ₂ | * |
| 10-298 | Ph | 4-SCF₃-Ph | tBu | C(O)OCH₂CH=CH ₂ | * |
| 10-299 | Ph | 4-I-Ph | tBu | C(O)OCH₂CH=CH ₂ | * |
| 10-300 | Ph | 4-CF₃-Ph | tBu | C(O)OCH₂CH=CH ₂ | * |
| 10-301 | Ph | 4-CN-Ph | tBu | C(O)OCH₂CH=CH ₂ | * |
| 10-302 | Ph | 4-Et-Ph | tBu | H | * |
| 10-303 | Ph | 4-iPr-Ph | tBu | | * |
| 10-304 | Ph | 4-SMe-Ph | tBu | H | * |
| 10-305 | Ph | 4-NO₂-Ph | tBu | H | * |
| 10-306 | Ph | 4-Ph-Ph | tBu | H H | * |
| 10-307 | Ph | 4-OCF₃-Ph | tBu | H | * |
| 10-308 | Ph | 4-SCF₃-Ph | tBu | H | * |
| 10-309 | Ph | 4-I-Ph | tBu | H | * |
| 10-310 | Ph | 4-CF₃-Ph | tBu | H | * |
| 10-311 | Ph | 4-CN-Ph | tBu | H | * |
| 10-312 | 3-F-Ph | 4-OMe-Ph | tBu | C(O)OMe | 95-97 |
| 10-313 | 3-Cl-Ph | 4-OMe-Ph | tBu | C(O)OMe | 100-102 |
| 10-314 | 3-OMe-Ph | 4-OMe-Ph | tBu | C(O)OMe | * |
| 10-315 | 3-F-Ph | 4-OEt-Ph | tBu | C(O)OMe | 118-120 |
| 10-316 | 3-Cl-Ph | 4-OEt-Ph | tBu | C(O)OMe | * |
| 10-317 | 3-OMe-Ph | 4-OEt-Ph | tBu | C(O)OMe | * |
| 10-318 | Ph | 2,4-Me₂-Ph | tBu | C(O)CH₂CF₃ | 129-132 |
| 10-319 | Ph | 2,4-Me₂-Ph | tBu | C(O)CMe=CH₂ | * |
| 10-320 | Ph | 3-Me-Ph | tBu | C(O)OMe | * |
| 10-321 | Ph | 2-Me-Ph | tBu | C(O)OMe | 106-108 |
| 10-322 | Ph | 3-Cl-Ph | tBu | C(O)OMe | * |
| 10-323 | Ph | 2-Cl-Ph | tBu | C(O)OMe | * |
| 10-324 | Ph | 3-Br-Ph | tBu | C(O)OMe | * |
| 10-325 | Ph | 2-Br-Ph | tBu | C(O)OMe | * |
| 10-326 | Ph | 3-OMe-Ph | tBu | C(O)OMe | * |
| 10-327 | Ph | 2-OMe-Ph | tBu | C(O)OMe | * |
| 10-328 | Ph | 3-F-4-Me-Ph | tBu | C(O)OMe | 92-94 |
| 10-329 | Ph | Z-1a | tBu | C(O)OMe | * |
| 10-330 | Ph | 3,4-(OMe)₂-Ph | tBu | C(O)OMe | * |
| 10-331 | Ph | 3-Cl-4-Me-Ph | tBu | C(O)OMe | * |
| 10-332 | Ph | 3,4-Cl₂-Ph | tBu | C(O)OMe | * |
| 10-333 | Ph | 2,3-Cl₂-Ph | tBu | C(O)OMe | * |
| 10-334 | Ph | 2,5-Me₂-Ph | tBu | C(O)OMe | * |
| 10-335 | Ph | 3-Me-Ph | tBu | H | * |
| 10-336 | Ph | 2-Me-Ph | tBu | H | * |
| 10-337 | Ph | 3-Cl-Ph | tBu | H | * |
| 10-338 | Ph | 2-Cl-Ph | tBu | H | * |
| 10-339 | Ph | 3-Br-Ph | tBu | H | * |
| 10-340 | Ph | 2-Br-Ph | tBu | H | * |
| 10-341 | Ph | 3-OMe-Ph | tBu | H | * |
| 10-342 | Ph | 2-OMe-Ph | tBu | H | * |
| 10-343 | Ph | 3-F-4-Me-Ph | tBu | H | * |
| 10-344 | Ph | Z-1a | tBu | H | * |
| 10-345 | Ph | 3,4-(OMe)₂-Ph | tBu | H | * |
| 10-346 | Ph | 3-Cl-4-Me-Ph | tBu | H | * |
| 10-347 | Ph | 3,4-Cl₂-Ph | tBu | H | * |
| 10-348 | Ph | 2,3-Cl₂-Ph | tBu | H | * |
| 10-349 | Ph | 2,5-Me₂-Ph | tBu | H | * |
| 10-350 | Ph | 2,4-Me₂-Ph | tBu | C(O)cPr | * |
| 10-351 | Ph | 2,4-Me₂-Ph | tBu | C(O)CF₃ | * |
| 10-352 | Ph | 2,4-Me₂-Ph | tBu | Ph | * |
| 10-353 | Ph | 2-F-4-Br-Ph | tBu | H | * |
| 10-354 | Ph | 2-Br-4-F-Ph | tBu | H | * |
| 10-355 | Ph | 2-F-4-CF₃-Ph | tBu | H | * |
| 10-356 | Ph | 2-OH-4-Me-Ph | tBu | H | * |
| 10-357 | Ph | 2-Me-4-OMe-Ph | tBu | H | * |
| 10-358 | Ph | 2,4,5-Me₃-Ph | tBu | H | * |
| 10-359 | Ph | Z-1b | tBu | H | * |
| 10-360 | Ph | 2-Br-4-OMe-Ph | tBu | H | * |
| 10-361 | Ph | 2-F-4-Br-Ph | tBu | C(O)OMe | * |
| 10-362 | Ph | 2-Br-4-F-Ph | tBu | C(O)OMe | 129-131 |
| 10-363 | Ph | 2-F-4-CF₃-Ph | tBu | C(O)OMe | 112-114 |
| 10-364 | Ph | 2-OH-4-Me-Ph | tBu | C(O)OMe | * |
| 10-365 | Ph | 2-Me-4-OMe-Ph | tBu | C(O)OMe | * |
| 10-366 | Ph | 2,4,5-Me₃-Ph | tBu | C(O)OMe | 148-150 |
| 10-367 | Ph | Z-1b | tBu | C(O)OMe | * |
| 10-368 | Ph | 2-Br-4-OMe-Ph | tBu | C(O)OMe | * |
| 10-369 | Ph | 2-F-4-Br-Ph | tBu | C(O)OEt | * |
| 10-370 | Ph | 2-Br-4-F-Ph | tBu | C(O)OEt | * |
| 10-371 | Ph | 2-F-4-CF₃-Ph | tBu | C(O)OEt | * |
| 10-372 | Ph | 2-OH-4-Me-Ph | tBu | C(O)OEt | * |
| 10-373 | Ph | 2-Me-4-OMe-Ph | tBu | C(O)OEt | * |
| 10-374 | Ph | 2,4,5-Me₃-Ph | tBu | C(O)OEt | 122-124 |
| 10-375 | Ph | Z-1b | tBu | C(O)OEt | * |
| 10-376 | Ph | 2-Br-4-OMe-Ph | tBu | C(O)OEt | * |
| 10-377 | Ph | 2-F-4-Br-Ph | tBu | C(O)OCH2CH=CH ₂ | ^{*} |
| 10-378 | Ph | 2-Br-4-F-Ph | tBu | C(O)OCH2CH=CH ₂ | * |
| 10-379 | Ph | 2-F-4-CF₃-Ph | tBu | C(O)OCH₂CH=CH ₂ | * |
| 10-380 | Ph | 2-OH-4-Me-Ph | tBu | C(O)OCH₂CH=CH ₂ | * |
| 10-381 | Ph | 2-Me-4-OMe-Ph | tBu | C(O)OCH₂CH=CH ₂ | * |
| 10-382 | Ph | 2,4,5-Me₃-Ph | tBu | C(O)OCH₂CH=CH ₂ | * |
| 10-383 | Ph | Z-1b | tBu | C(O)OCH₂CH=CH ₂ | * |
| 10-384 | Ph | 2-Br-4-OMe-Ph | tBu | C(O)OCH₂CH=CH ₂ | * |
| 10-385 | 3-CF₃-Ph | 2,4-(OMe)₂-Ph | tBu | H | * |
| 10-386 | 3-CF₃-Ph | 2-F-4-OMe-Ph | tBu | H | * |
| 10-387 | 3-CF₃-Ph | 2-Br-4-OMe-Ph | tBu | H | * |
| 10-388 | 3-CF₃-Ph | 2-Me-4-OMe-Ph | tBu | H | * |
| 10-389 | 3-CF₃-Ph | 4-OCF₃-Ph | tBu | H | 82-84 |
| 10-390 | 3-CF₃-Ph | 2,4,5-Me₃-Ph | tBu | H | * |
| 10-391 | 3-CF₃-Ph | Z-4b | tBu | H | * |
| 10-392 | 3-CF₃-Ph | Z-1b | tBu | H | * |
| 10-393 | 3-CF₃-Ph | Z-1c | tBu | H | * |
| 10-394 | 3-CF₃-Ph | 2,4-F₂-Ph | tBu | H | * |
| 10-395 | Ph | 2,4-Me₂-Ph | tBu | CH₂tBu | 118-121 |
| 10-396 | Ph | 2-Cl-4-OMe-Ph | tBu | C(O)CH₂Cl | * |
| 10-397 | 3-CF₃-Ph | 2,4-(OMe)₂-Ph | tBu | C(O)OMe | * |
| 10-398 | 3-CF₃-Ph | 2-F-4-OMe-Ph | tBu | C(O)OMe | 101-103 |
| 10-399 | 3-CF₃-Ph | 2-Br-4-OMe-Ph | tBu | C(O)OMe | * |
| 10-400 | 3-CF₃-Ph | 2-Me-4-OMe-Ph | tBu | C(O)OMe | 92-94 |
| 10-401 | 3-CF₃-Ph | 4-OCF₃-Ph | tBu | C(O)OMe | 131-133 |
| 10-402 | 3-CF₃-Ph | 2,4,5-Me₃-Ph | tBu | C(O)OMe | 99-101 |
| 10-403 | 3-CF₃-Ph | Z-4b | tBu | C(O)OMe | 112-114 |
| 10-404 | 3-CF₃-Ph | Z-1b | tBu | C(O)OMe | 101-103 |
| 10-405 | 3-CF₃-Ph | Z-1c | tBu | C(O)OMe | * |
| 10-406 | 3-CF₃-Ph | 2,4-(OMe)₂-Ph | tBu | C(O)OEt | * |
| 10-407 | 3-CF₃-Ph | 2-F-4-OMe-Ph | tBu | C(O)OEt | 94-96 |
| 10-408 | 3-CF₃-Ph | 2-Br-4-OMe-Ph | tBu | C(O)OEt | * |
| 10-409 | 3-CF₃-Ph | 2-Me-4-OMe-Ph | tBu | C(O)OEt | 96-98 |
| 10-410 | 3-CF₃-Ph | 4-OCF₃-Ph | tBu | C(O)OEt | 127-129 |
| 10-411 | 3-CF₃-Ph | 2,4,5-Me₃-Ph | tBu | C(O)OEt | 72-74 |
| 10-412 | 3-CF₃-Ph | Z-4b | tBu | C(O)OEt | * |
| 10-413 | 3-CF₃-Ph | Z-1b | tBu | C(O)OEt | 96-98 |
| 10-414 | 3-CF₃-Ph | Z-1c | tBu | C(O)OEt | * |
| 10-415 | 3-CF₃-Ph | 2,4-(OMe)₂-Ph | tBu | C(O)OCH₂CH=CH ₂ | * |
| 10-416 | 3-CF₃-Ph | 2-F-4-OMe-Ph | tBu | C(O)OCH₂CH=CH ₂ | * |
| 10-417 | 3-CF₃-Ph | 2-Br-4-OMe-Ph | tBu | C(O)OCH₂CH=CH ₂ | * |
| 10-418 | 3-CF₃-Ph | 2-Me-4-OMe-Ph | tBu | C(O)OCH₂CH=CH ₂ | 99-101 |
| 10-419 | 3-CF₃-Ph | 4-OCF₃-Ph | tBu | C(O)OCH₂CH=CH ₂ | * |
| 10-420 | 3-CF₃-Ph | 2,4,5-Me₃-Ph | tBu | C(O)OCH₂CH=CH ₂ | * |
| 10-421 | 3-CF₃-Ph | Z-4b | tBu | C(O)OCH₂CH=CH ₂ | * |
| 10-422 | 3-CF₃-Ph | Z-1b | tBu | C(O)OCH₂CH=CH ₂ | 99-101 |
| 10-423 | 3-CF₃-Ph | Z-1c | tBu | C(O)OCH₂CH=CH ₂ | * |
| 10-424 | Ph | 2,4-Me₂-Ph | tBu | CH₂cHx | * |
| 10-425 | Ph | 2-Cl-4-OH-Ph | tBu | H | * |
| 10-426 | Ph | 2-Cl-4-OEt-Ph | tBu | C(O)OMe | * |
| 10-427 | Ph | 2-Cl-4-OMe-Ph | tBu | C(O)(F-2a) | 116-118 |
| 10-428 | Ph | 2-Cl-4-OMe-Ph | tBu | C(O)(F-3a) | 161-163 |
| 10-429 | Ph | 2-Cl-4-OMe-Ph | tBu | C(O)(F-4a) | 154-156 |
| 10-430 | 3-NMe₂-Ph | 2,4-Me₂-Ph | tBu | C(O)OMe | 134-136 |
| 10-431 | 3-SMe-Ph | 2,4-Me₂-Ph | tBu | C(O)OMe | * |
| 10-432 | 3-CF₃-Ph | 2-Cl-4-Br-Ph | tBu | C(O)(F-2a) | * |
| 10-433 | 3-CF₃-Ph | 2-Cl-4-Br-Ph | tBu | C(O)(F-3a) | * |
| 10-434 | 3-CF₃-Ph | 2-Cl-4-Br-Ph | tBu | C(O)(F-4a) | 71-73 |
| 10-435 | 3-CH=CH₂ -Ph | 2,4-Me₂-Ph | tBu | C(O)OMe | 122-124 |
| 10-436 | 3-cPr-Ph | 2,4-Me₂-Ph | tBu | C(O)OMe | 117-119 |
| 10-437 | 3-SOMe-Ph | 2,4-Me₂-Ph | tBu | C(O)OMe | * |
| 10-438 | 3-SO₂Me-Ph | 2,4-Me₂-Ph | tBu | C(O)OMe | * |
| 10-439 | Ph | 2-Cl-4-OH-Ph | tBu | C(O)OMe | * |
| 10-440 | 3-Me-Ph | 2-F-4-OMe-Ph | tBu | C(O)OMe | * |
| 10-441 | 3-Me-Ph | 2-Cl-4-OMe-Ph | tBu | C(O)OMe | * |
| 10-442 | 3-CF₃-Ph | 2-F-4-OMe-Ph | Ph | C(O)OMe | 114-116 |
| 10-443 | Ph | 2-F-4-OMe-Ph | Ph | C(O)OMe | 134-136 |
| 10-444 | Ph | 2-F-4-OMe-Ph | F-2a | C(O)OMe | * |
| 10-445 | 3-CF₃-Ph | 2-OMe-4-Me-Ph | tBu | H | * |
| 10-446 | Ph | 2-OMe-4-Me-Ph | tBu | H | * |
| 10-447 | 3-CF₃-Ph | 4-OMe-Ph | Ph | C(O)OMe | * |
| 10-448 | Ph | 4-OMe-Ph | Ph | C(O)OMe | 130-132 |
| 10-449 | Ph | 4-OMe-Ph | F-2a | C(O)OMe | * |
| 10-450 | 3-Me-Ph | 4-OMe-Ph | tBu | C(O)OMe | * |
| 10-451 | 3-CF₃-Ph | 2-OMe-4-Me-Ph | tBu | C(O)OMe | * |
| 10-452 | 3-CF₃-Ph | 2-OMe-4-Me-Ph | tBu | C(O)OEt | * |
| 10-453 | 3-CF₃-Ph | 2-OMe-4-Me-Ph | tBu | C(O)OCH₂CH=CH ₂ | * |
| 10-454 | Ph | 2-OMe-4-Me-Ph | tBu | C(O)OMe | * |
| 10-455 | Ph | 2-OMe-4-Me-Ph | tBu | C(O)OEt | * |
| 10-456 | Ph | 2-OMe-4-Me-Ph | tBu | C(O)OCH₂CH=CH 2 | * |
| 10-457 | 3-CF₃-Ph | 4-OMe-Ph | F-2a | C(O)OMe | * |
| 10-458 | 3-CF₃-Ph | 2-F-4-OMe-Ph | F-2a | C(O)OMe | * |
| 10-459 | 2-F-Ph | 2,4-Me₂-Ph | tBu | C(O)OMe | 155-157 |
| 10-460 | 2-F-Ph | 2-F-4-OMe-Ph | tBu | C(O)OMe | * |
| 10-461 | 2-F-Ph | 4-OMe-Ph | tBu | C(O)OMe | * |
| 10-462 | 2,3-F₂-Ph | 2,4-Me₂-Ph | tBu | C(O)OMe | 164-166 |
| 10-463 | 2,3-F₂-Ph | 2-F-4-OMe-Ph | tBu | C(O)OMe | * |
| 10-464 | 2,3-F₂-Ph | 4-OMe-Ph | tBu | C(O)OMe | * |
| 10-465 | 2-F-3-OMe-Ph | 2,4-Me₂-Ph | tBu | C(O)OMe | * |
| 10-466 | 2-F-3-OMe-Ph | 2-F-4-OMe-Ph | tBu | C(O)OMe | * |
| 10-467 | 2-F-3-OMe-Ph | 4-OMe-Ph | tBu | C(O)OMe | * |
| 10-468 | 3-CF₃-Ph | 6-OMe-(Z-9a) | tBu | C(O)OMe | * |
| 10-469 | 3-CF₃-Ph | 6-OMe-(Z-9a) | tBu | C(O)OEt | * |
| 10-470 | 3-CF₃-Ph | 6-OMe-(Z-9a) | tBu | C(O)OCH₂CH=CH ₂ | * |
| 10-471 | 3-Me-Ph | 2-OMe-4-Me-Ph | tBu | C(O)OMe | 97-99 |
| 10-472 | 3-Me-Ph | 2-Me-4-OMe-Ph | tBu | C(O)OMe | * |
| 10-473 | 3-Me-Ph | 4-OEt-Ph | tBu | C(O)OMe | * |
| 10-474 | 3-Me-Ph | 4-Cl-Ph | tBu | C(O)OMe | 99-101 |
| 10-475 | 3-Me-Ph | 4-OCF₂H-Ph | tBu | C(O)OMe | * |
| 10-476 | 3-Me-Ph | Z-1d | tBu | C(O)OMe | 79-81 |
| 10-477 | 3-Me-Ph | 2-OMe-4-Me-Ph | tBu | C(O)OEt | * |
| 10-478 | 3-Me-Ph | 2-F-4-OMe-Ph | tBu | C(O)OEt | * |
| 10-479 | 3-Me-Ph | 2-Cl-4-OMe-Ph | tBu | C(O)OEt | * |
| 10-480 | 3-Me-Ph | 2-Me-4-OMe-Ph | tBu | C(O)OEt | * |
| 10-481 | 3-Me-Ph | 4-OMe-Ph | tBu | C(O)OEt | * |
| 10-482 | 3-Me-Ph | 4-OEt-Ph | tBu | C(O)OEt | * |
| 10-483 | 3-Me-Ph | 4-Cl-Ph | tBu | C(O)OEt | * |
| 10-484 | 3-Me-Ph | 4-OCF₂H-Ph | tBu | C(O)OEt | * |
| 10-485 | 3-Me-Ph | Z-1d | tBu | C(O)OEt | 106-108 |
| 10-486 | 3-CF₃-Ph | 2-F-4-OMe-Ph | tBu | C(O)OiPr | * |
| 10-487 | 3-CF₃-Ph | 2-Cl-4-OMe-Ph | tBu | C(O)OiPr | * |
| 10-488 | 3-CF₃-Ph | 2-Me-4-OMe-Ph | tBu | C(O)OiPr | * |
| 10-489 | 3-CF₃-Ph | 2-F-4-OMe-Ph | tBu | CH₂cPr | 100-102 |
| 10-490 | 3-CF₃-Ph | 2-Cl-4-OMe-Ph | tBu | CH₂cPr | 96-98 |
| 10-491 | 3-CF₃-Ph | 2-Me-4-OMe-Ph | tBu | CH₂cPr | 96-98 |
| 10-492 | 3-CF₃-Ph | 6-Cl-(Z-9a) | tBu | C(O)OMe | * |
| 10-493 | Ph | 6-Me-(Z-9a) | tBu | C(O)OMe | 182-183 |
| 10-494 | 3-CF₃-Ph | Z-27b | tBu | H | * |
| 10-495 | 3-CF₃-Ph | Z-27b | tBu | C(O)OMe | 172-174 |
| 10-496 | 3-CF₃-Ph | Z-27b | tBu | C(O)OEt | 157-159 |
| 10-497 | 3-CF₃-Ph | Z-27b | tBu | C(O)OCH₂CH=CH ₂ | 167-169 |
| 10-498 | Ph | 6-OMe-(Z-9a) | tBu | C(O)OMe | * |
| 10-499 | 3-Me-Ph | 6-Cl-(Z-9a) | tBu | C(O)OMe | * |
| 10-500 | Ph | 6-Cl-(Z-9a) | tBu | C(O)OMe | 136-138 |
| 10-501 | Ph | 6-Ph-(Z-9a) | tBu | C(O)OMe | * |
| 10-502 | Ph | 6-cPr-(Z-9a) | tBu | C(O)OMe | * |
| 10-503 | 3-CF₃-Ph | 2-Me-4-OMe-Ph | tBu | C(O)OnPr | * |
| 10-504 | 3-CF₃-Ph | 2-Me-4-OMe-Ph | tBu | C(O)OiBu | * |
| 10-505 | 3-CF₃-Ph | 2-Me-4-OMe-Ph | tBu | C(O)OBn | * |
| 10-506 | 3-CF₃-Ph | 2-Me-4-OMe-Ph | tBu | C(O)O(4-Cl-Ph) | * |
| 10-507 | 3-CF₃-Ph | 2-Me-4-OMe-Ph | tBu | C(O)cPr | * |
| 10-508 | 3-CF₃-Ph | 2-Me-4-OMe-Ph | tBu | C(O)(F-11a) | 169-171 |
| 10-509 | 3-CF₃-Ph | 2-Me-4-OMe-Ph | tBu | C(O)Ph | 147-149 |
| 10-510 | 3-CF₃-Ph | 2-Cl-4-OMe-Ph | tBu | C(O)OnPr | * |
| 10-511 | 3-CF₃-Ph | 2-Cl-4-OMe-Ph | tBu | C(O)OiBu | * |
| 10-512 | 3-CF₃-Ph | 2-Cl-4-OMe-Ph | tBu | C(O)OBn | * |
| 10-513 | 3-CF₃-Ph | 2-Cl-4-OMe-Ph | tBu | C(O)O(4-Cl-Ph) | * |
| 10-514 | 3-CF₃-Ph | 2-Cl-4-OMe-Ph | tBu | C(O)cPr | * |
| 10-515 | 3-CF₃-Ph | 2-Cl-4-OMe-Ph | tBu | C(O)(F-11a) | 157-159 |
| 10-516 | 3-CF₃-Ph | 2-Cl-4-OMe-Ph | tBu | C(O)Ph | 144-146 |
| 10-517 | 3-CF₃-Ph | 2-F-4-OMe-Ph | tBu | CH₂CF₃ | 98-101 |
| 10-518 | 3-CF₃-Ph | 2-F-4-OMe-Ph | tBu | C(O)Me | 106-108 |
| 10-519 | 3-CF₃-Ph | 2-F-4-OMe-Ph | tBu | C(O)CF₃ | * |
| 10-520 | 3-CF₃-Ph | 2-F-4-OMe-Ph | tBu | C(O)CF₂H | * |
| 10-521 | 3-CF₃-Ph | 2-Cl-4-OMe-Ph | tBu | C(O)Me | 63-65 |
| 10-522 | 3-CF₃-Ph | 2-Cl-4-OMe-Ph | tBu | C(O)CF₃ | * |
| 10-523 | 3-CF₃-Ph | 2-Cl-4-OMe-Ph | tBu | C(O)CF₂H | * |
| 10-524 | 3-CF₃-Ph | 2-Me-4-OMe-Ph | tBu | C(O)Me | 59-61 |
| 10-525 | 3-CF₃-Ph | 2-Me-4-OMe-Ph | tBu | C(O)CF₃ | * |
| 10-526 | 3-CF₃-Ph | 2-Me-4-OMe-Ph | tBu | C(O)CF₂H | * |
| 10-527 | Ph | 2-F-4-OMe-Ph | tBu | C(O)Me | 137-139 |
| 10-528 | Ph | 2-F-4-OMe-Ph | tBu | C(O)CF₃ | * |
| 10-529 | Ph | 2-F-4-OMe-Ph | tBu | C(O)CF₂H | 126-127 |
| 10-530 | Ph | 2-Cl-4-OMe-Ph | tBu | C(O)Me | 147-148 |
| 10-531 | Ph | 2-Cl-4-OMe-Ph | tBu | C(O)CF₃ | * |
| 10-532 | Ph | 2-Cl-4-OMe-Ph | tBu | C(O)CF₂H | * |
| 10-533 | Ph | 2-Me-4-OMe-Ph | tBu | C(O)Me | 47-49 |
| 10-534 | Ph | 2-Me-4-OMe-Ph | tBu | C(O)CF₃ | * |
| 10-535 | Ph | 2-Me-4-OMe-Ph | tBu | C(O)CF₂H | * |
| 10-536 | 3-CF₃-Ph | 2-F-4-OMe-Ph | tBu | C(O)OnPr | * |
| 10-537 | 3-CF₃-Ph | 2-F-4-OMe-Ph | tBu | C(O)OiBu | * |
| 10-538 | 3-CF₃-Ph | 2-F-4-OMe-Ph | tBu | C(O)OBn | * |
| 10-539 | 3-CF₃-Ph | 2-F-4-OMe-Ph | tBu | C(O)O(4-Cl-Ph) | * |
| 10-540 | 3-CF₃-Ph | 2-F-4-OMe-Ph | tBu | C(O)cPr | * |
| 10-541 | 3-CF₃-Ph | 2-F-4-OMe-Ph | tBu | C(O)(F-11a) | 121-123 |
| 10-542 | 3-CF₃-Ph | 2-F-4-OMe-Ph | tBu | C(O)Ph | 89-91 |
| 10-543 | Ph | Z-8b | tBu | C(O)OMe | 156-158 |
| 10-544 | Ph | Z-8c | tBu | C(O)OMe | 186-188 |
| 10-545 | Ph | 4-OCF₂H-Ph | tBu | C(O)OMe | * |
| 10-546 | Ph | 4-OCF₂H-Ph | tBu | C(O)OEt | * |
| 10-547 | Ph | 4-OCH₂CF₃-Ph | tBu | C(O)OMe | * |
| 10-548 | Ph | 4-OCH₂CF₃-Ph | tBu | C(O)OEt | 108-110 |
| 10-549 | Ph | 4-OCH₂CF₂ H-Ph | tBu | C(O)OMe | * |
| 10-550 | Ph | 4-OCH₂CF₂ H-Ph | tBu | C(O)OEt | * |
| 10-551 | Ph | 4-(L-5)-Ph | tBu | C(O)OMe | * |
| 10-552 | Ph | 4-(L-5)-Ph | tBu | C(O)OEt | 82-84 |
| 10-553 | 3-CF₃-Ph | 4-OCF₂H-Ph | tBu | C(O)OMe | 124-126 |
| 10-554 | 3-CF₃-Ph | 4-OCF₂H-Ph | tBu | C(O)OEt | 119-121 |
| 10-555 | 3-CF₃-Ph | 4-OCH₂CF₃-Ph | tBu | C(O)OMe | * |
| 10-556 | 3-CF₃-Ph | 4-OCH₂CF₃-Ph | tBu | C(O)OEt | * |
| 10-557 | 3-CF₃-Ph | 4-OCH₂CF₂ H-Ph | tBu | C(O)OMe | 107-109 |
| 10-558 | 3-CF₃-Ph | 4-OCH₂CF₂ H-Ph | tBu | C(O)OEt | * |
| 10-559 | 3-CF₃-Ph | 4-(L-5)-Ph | tBu | C(O)OMe | * |
| 10-560 | 3-CF₃-Ph | 4-(L-5)-Ph | tBu | C(O)OEt | * |
| 10-561 | 3-CF₃-Ph | 2-F-4-OMe-Ph | tBu | Me | * |
| 10-562 | 3-CF₃-Ph | 2-F-4-OMe-Ph | tBu | CH₂CH=CH₂ | * |
| 10-563 | 3-CF₃-Ph | 2-F-4-OMe-Ph | tBu | CH₂CN | * |
| 10-564 | 3-CF₃-Ph | 2-F-4-OMe-Ph | tBu | CH₂CF₂H | * |
| 10-565 | 3-Me-Ph | 4-CN-Ph | tBu | C(O)OMe | * |
| 10-566 | 3-Cl-Ph | 2-OMe-4-Me-Ph | tBu | C(O)OEt | * |
| 10-567 | 3-Cl-Ph | 2-F-4-OMe-Ph | tBu | C(O)OEt | * |
| 10-568 | 3-Cl-Ph | 2-Cl-4-OMe-Ph | tBu | C(O)OEt | * |
| 10-569 | 3-Cl-Ph | 2-Me-4-OMe-Ph | tBu | C(O)OEt | 102-104 |
| 10-570 | 3-Cl-Ph | 4-OMe-Ph | tBu | C(O)OEt | 101-103 |
| 10-571 | 3-Cl-Ph | 4-OEt-Ph | tBu | C(O)OEt | * |
| 10-572 | 3-Cl-Ph | 4-Cl-Ph | tBu | C(O)OEt | * |
| 10-573 | 3-Cl-Ph | 4-OCF₂H-Ph | tBu | C(O)OEt | * |
| 10-574 | 3-Cl-Ph | 4-OCH₂CF₂ H-Ph | tBu | C(O)OEt | * |
| 10-575 | 3-Cl-Ph | 4-(L-5)-Ph | tBu | C(O)OEt | * |
| 10-576 | 3-CF₃-Ph | 2-Me-4-OMe-Ph | tBu | CH₂CH₂OH | 123-125 |
| 10-577 | 3-CF₃-Ph | 2-Cl-4-OMe-Ph | tBu | Me | * |
| 10-578 | 3-CF₃-Ph | 2-Cl-4-OMe-Ph | tBu | CH₂CH=CH₂ | * |
| 10-579 | 3-CF₃-Ph | 2-Cl-4-OMe-Ph | tBu | CH₂CN | * |
| 10-580 | 3-CF₃-Ph | 2-Cl-4-OMe-Ph | tBu | CH₂CF₃ | * |
| 10-581 | 3-CF₃-Ph | 2-Cl-4-OMe-Ph | tBu | CH₂CF₂H | * |
| 10-582 | 3-CF₃-Ph | 2-Me-4-OMe-Ph | tBu | Me | * |
| 10-583 | 3-CF₃-Ph | 2-Me-4-OMe-Ph | tBu | CH₂CH=CH₂ | * |
| 10-584 | 3-CF₃-Ph | 2-Me-4-OMe-Ph | tBu | CH₂CN | * |
| 10-585 | 3-CF₃-Ph | 2-Me-4-OMe-Ph | tBu | CH₂CF₃ | * |
| 10-586 | 3-CF₃-Ph | 2-Me-4-OMe-Ph | tBu | CH₂CF₂H | * |
| 10-587 | Ph | 2-F-4-OMe-Ph | tBu | Me | * |
| 10-588 | Ph | 2-F-4-OMe-Ph | tBu | CH₂CH=CH₂ | * |
| 10-589 | Ph | 2-F-4-OMe-Ph | tBu | CH₂CN | * |
| 10-590 | Ph | 2-F-4-OMe-Ph | tBu | CH₂CF₃ | * |
| 10-591 | Ph | 2-F-4-OMe-Ph | tBu | CH₂CF₂H | * |
| 10-592 | Ph | 2-Cl-4-OMe-Ph | tBu | Me | * |
| 10-593 | Ph | 2-Cl-4-OMe-Ph | tBu | CH₂CH=CH₂ | * |
| 10-594 | Ph | 2-Cl-4-OMe-Ph | tBu | CH₂CN | 111-113 |
| 10-595 | Ph | 2-Cl-4-OMe-Ph | tBu | CH₂CF₃ | 108-110 |
| 10-596 | Ph | 2-Cl-4-OMe-Ph | tBu | CH₂CF₂H | 95-97 |
| 10-597 | Ph | 2-Me-4-OMe-Ph | tBu | Me | * |
| 10-598 | Ph | 2-Me-4-OMe-Ph | tBu | CH₂CH=CH₂ | * |
| 10-599 | Ph | 2-Me-4-OMe-Ph | tBu | CH₂CN | * |
| 10-600 | Ph | 2-Me-4-OMe-Ph | tBu | CH₂CF₃ | * |
| 10-601 | Ph | 2-Me-4-OMe-Ph | tBu | CH₂CF₂H | * |
| 10-602 | 3-Cl-Ph | 2-OMe-4-Me-Ph | tBu | C(O)OMe | 131-133 |
| 10-603 | 3-Cl-Ph | 2-F-4-OMe-Ph | tBu | C(O)OMe | * |
| 10-604 | 3-Cl-Ph | 2-Cl-4-OMe-Ph | tBu | C(O)OMe | 112-114 |
| 10-605 | 3-Cl-Ph | 2-Me-4-OMe-Ph | tBu | C(O)OMe | 117-119 |
| 10-606 | 3-Cl-Ph | 4-Cl-Ph | tBu | C(O)OMe | * |
| 10-607 | 3-Cl-Ph | 4-OCF₂H-Ph | tBu | C(O)OMe | 106-108 |
| 10-608 | 3-Cl-Ph | 4-OCH₂CF₂ H-Ph | tBu | C(O)OMe | * |
| 10-609 | 3-Cl-Ph | 4-(L-5)-Ph | tBu | C(O)OMe | * |
| 10-610 | 3-Me-Ph | 6-Cl-(Z-9a) | tBu | C(O)OEt | * |
| 10-611 | 3-Me-Ph | 6-OMe-(Z-9a) | tBu | C(O)OEt | * |
| 10-612 | 3-Me-Ph | 5-Me-(Z-17a) | tBu | C(O)OEt | * |
| 10-613 | 3-Me-Ph | 5-Me-(Z-17a) | tBu | C(O)OMe | * |
| 10-614 | Ph | 5-OMe-(Z-8a) | tBu | C(O)OMe | * |
| 10-615 | Ph | 5-OMe-(Z-8a) | tBu | C(O)OEt | * |
| 10-616 | Ph | 5-Cl-(Z-8a) | tBu | C(O)OMe | * |
| 10-617 | Ph | 5-Cl-(Z-8a) | tBu | C(O)OEt | * |
| 10-618 | 3-CF₃-Ph | 5-OMe-(Z-8a) | tBu | C(O)OMe | 112-114 |
| 10-619 | 3-CF₃-Ph | 5-OMe-(Z-8a) | tBu | C(O)OEt | 114-116 |
| 10-620 | 3-CF₃-Ph | 5-Cl-(Z-8a) | tBu | C(O)OMe | * |
| 10-621 | 3-CF₃-Ph | 5-Cl-(Z-8a) | tBu | C(O)OEt | 94-96 |
| 10-622 | 3-Cl-Ph | 5-Cl-(Z-8a) | tBu | C(O)OMe | * |
| 10-623 | 3-Cl-Ph | 5-OMe-(Z-8a) | tBu | C(O)OMe | 105-107 |
| 10-624 | 3-Cl-Ph | 5-Me-(Z-17a) | tBu | C(O)OMe | * |
| 10-625 | 3-Cl-Ph | 5-Br-(Z-19a) | tBu | C(O)OMe | * |
| 10-626 | 3-Cl-Ph | Z-27d | tBu | C(O)OMe | * |
| 10-627 | 3-Cl-Ph | 5-Cl-(Z-8a) | tBu | C(O)OEt | * |
| 10-628 | 3-Cl-Ph | 5-OMe-(Z-8a) | tBu | C(O)OEt | 99-101 |
| 10-629 | 3-Cl-Ph | 5-Me-(Z-17a) | tBu | C(O)OEt | * |
| 10-630 | 3-Cl-Ph | 5-Br-(Z-19a) | tBu | C(O)OEt | * |
| 10-631 | 3-Cl-Ph | Z-27d | tBu | C(O)OEt | * |
| 10-632 | 3-Me-Ph | 2-OMe-4-Me-Ph | tBu | | * |
| 10-633 | 3-Me-Ph | 2-F-4-OMe-Ph | tBu | H | * |
| 10-634 | 3-Me-Ph | 2-Cl-4-OMe-Ph | tBu | H H | * |
| 10-635 | 3-Me-Ph | 2-Me-4-OMe-Ph | tBu | H | * |
| 10-636 | 3-Me-Ph | 4-OMe-Ph | tBu | H | * |
| 10-637 | 3-Me-Ph | 5-Br-(Z-19a) | tBu | C(O)OEt | * |
| 10-638 | 3-Me-Ph | Z-27d | tBu | C(O)OEt | 97-99 |
| 10-639 | 3-Me-Ph | 5-Br-(Z-19a) | tBu | C(O)OMe | 109-111 |
| 10-640 | 3-Me-Ph | Z-27d | tBu | C(O)OMe | 121-123 |
| 10-641 | Ph | 2-Me-4-OMe-Ph | tBu | C(O)OnPr | * |
| 10-642 | Ph | 2-Me-4-OMe-Ph | tBu | C(O)OiBu | * |
| 10-643 | Ph | 2-Me-4-OMe-Ph | tBu | C(O)OBn | 79-81 |
| 10-644 | Ph | 2-Me-4-OMe-Ph | tBu | C(O)O(4-Cl-Ph) | 112-114 |
| 10-645 | Ph | 2-Me-4-OMe-Ph | tBu | C(O)cPr | * |
| 10-646 | Ph | 2-Me-4-OMe-Ph | tBu | C(O)(F-11a) | 147-149 |
| 10-647 | Ph | 2-Me-4-OMe-Ph | tBu | C(O)Ph | 107-109 |
| 10-648 | Ph | 2-F-4-OMe-Ph | tBu | C(O)OnPr | * |
| 10-649 | Ph | 2-F-4-OMe-Ph | tBu | C(O)OiBu | * |
| 10-650 | Ph | 2-F-4-OMe-Ph | tBu | C(O)OBn | * |
| 10-651 | Ph | 2-F-4-OMe-Ph | tBu | C(O)cPr | * |
| 10-652 | Ph | 2-F-4-OMe-Ph | tBu | C(O)(F-11a) | 107-109 |
| 10-653 | Ph | 2-F-4-OMe-Ph | tBu | C(O)Ph | 104-106 |
| 10-654 | Ph | 2-Cl-4-OMe-Ph | tBu | C(O)OnPr | * |
| 10-655 | Ph | 2-Cl-4-OMe-Ph | tBu | C(O)OiBu | * |
| 10-656 | Ph | 2-Cl-4-OMe-Ph | tBu | C(O)OBn | * |
| 10-657 | Ph | 2-Cl-4-OMe-Ph | tBu | C(O)cPr | * |
| 10-658 | Ph | 2-Cl-4-OMe-Ph | tBu | C(O)(F-11a) | 137-139 |
| 10-659 | Ph | 2-Cl-4-OMe-Ph | tBu | C(O)Ph | 132-134 |
| 10-660 | 3-Me-Ph | 4-Cl-Ph | tBu | H | * |
| 10-661 | 3-Me-Ph | 4-OCF₂H-Ph | tBu | H | * |
| 10-662 | 3-Me-Ph | 4-(L-5)-Ph | tBu | H | * |
| 10-663 | 3-CF₃-Ph | 6-OMe-(Z-9a) | tBu | H H | * |
| 10-664 | Ph | Z-8b | tBu | | * |
| 10-665 | Ph | 4-OCF₂H-Ph | tBu | H | * |
| 10-666 | Ph | 4-OCH₂CF₃-Ph | tBu | H | * |
| 10-667 | Ph | 4-OCH₂CF₂ H-Ph | tBu | H | * |
| 10-668 | Ph | 4-(L-5)-Ph | tBu | H | * |
| 10-669 | 3-CF₃-Ph | 4-OCF₂H-Ph | tBu | H | * |
| 10-670 | 3-CF₃-Ph | 4-OCH₂CF₃-Ph | tBu | H | * |
| 10-671 | 3-CF₃-Ph | 4-OCH₂CF₂ H-Ph | tBu | H | * |
| 10-672 | 3-CF₃-Ph | 4-(L-5)-Ph | tBu | H | * |
| 10-673 | 3-Me-Ph | 4-CN-Ph | tBu | H | * |
| 10-674 | 3-Cl-Ph | 2-OMe-4-Me-Ph | tBu | H | 102-104 |
| 10-675 | 3-Cl-Ph | 2-F-4-OMe-Ph | tBu | H | * |
| 10-676 | 3-Cl-Ph | 2-Cl-4-OMe-Ph | tBu | H | * |
| 10-677 | 3-Cl-Ph | 2-Me-4-OMe-Ph | tBu | H | * |
| 10-678 | 3-Cl-Ph | 4-OMe-Ph | tBu | H | * |
| 10-679 | 3-Cl-Ph | 4-OEt-Ph | tBu | H | * |
| 10-680 | 3-Cl-Ph | 4-Cl-Ph | tBu | H | * |
| 10-681 | 3-Cl-Ph | 4-OCF₂H-Ph | tBu | H | * |
| 10-682 | 3-Cl-Ph | 4-OCH₂CF₂ H-Ph | tBu | H | * |
| 10-683 | 3-Cl-Ph | 4-(L-5)-Ph | tBu | H | * |
| 10-684 | 3-CF₃-Ph | 5-OMe-(Z-8a) | tBu | H | * |
| 10-685 | Ph | 5-OMe-(Z-8a) | tBu | H | * |
| 10-686 | 3-CF₃-Ph | 5-Cl-(Z-8a) | tBu | H | 126-129 |
| 10-687 | Ph | 5-Cl-(Z-8a) | tBu | H | 112-114 |
| 10-688 | 3-Me-Ph | 6-Cl-(Z-9a) | tBu | H | * |
| 10-689 | 3-Me-Ph | 6-OMe-(Z-9a) | tBu | H | * |
| 10-690 | 3-Me-Ph | 5-Me-(Z-17a) | tBu | H | * |
| 10-691 | 3-Me-Ph | 5-Br-(Z-19a) | tBu | H | * |
| 10-692 | 3-Me-Ph | Z-27d | tBu | H | 127-129 |
| 10-693 | 3-Cl-Ph | 5-Cl-(Z-8a) | tBu | H | * |
| 10-694 | 3-Cl-Ph | 5-OMe-(Z-8a) | tBu | H | * |
| 10-695 | 3-Cl-Ph | 5-Me-(Z-17a) | tBu | H | * |
| 10-696 | 3-Cl-Ph | 5-Br-(Z-19a) | tBu | H | * |
| 10-697 | 3-Cl-Ph | Z-27d | tBu | H | 112-114 |
| 10-698 | 3-Me-Ph | 4-C(O)OMe-Ph | tBu | H | * |
| 10-699 | 3-Me-Ph | 4-C(O)OMe-Ph | tBu | C(O)OMe | * |

**[Table 11]**

| 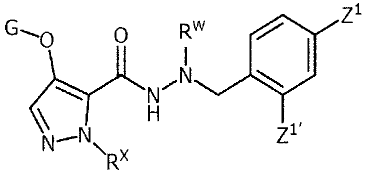 | | | | | | |
|---|---|---|---|---|---|---|
| No. | G | Z¹ | Z^{1'} | R^{X} | R^{W} | m.p. (°C) |
| 11-001 | Ph | OC(O)OMe | Cl | tBu | C(O)OMe | * |
| 11-002 | Ph | OCH₂(6-Me-(F-2a)) | Cl | tBu | C(O)OMe | * |

**[Table 12]**

| 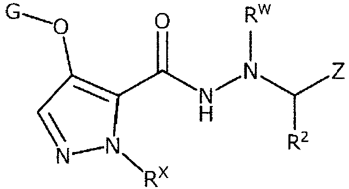 | | | | | | |
|---|---|---|---|---|---|---|
| No. | G | Z | R^{X} | R^{W} | R² | m.p. (°C) |
| 12-001 | 3-CF₃-Ph | 2,4-Me₂-Ph | tBu | H | Me | * |
| 12-002 | Ph | 2,4-Me₂-Ph | tBu | H | Me | * |
| 12-003 | Ph | 2,4-Me₂-Ph | tBu | H | Et | * |
| 12-004 | Ph | 4-Me-Ph | tBu | H | Me | * |
| 12-005 | Ph | 2,4-Me₂-Ph | tBu | C(O)OMe | Me | 101-104 |
| 12-006 | Ph | 2,4-Me₂-Ph | tBu | C(O)OEt | Me | 79-82 |
| 12-007 | Ph | 2,4-Me₂-Ph | tBu | C(O)OCH₂CH=C H₂ | Me | 69-72 |
| 12-008 | Ph | 2,4-Me₂-Ph | tBu | C(O)OMe | Et | * |
| 12-009 | Ph | 2,4-Me₂-Ph | tBu | C(O)OEt | Et | 106-109 |
| 12-010 | Ph | 2,4-Me₂-Ph | tBu | C(O)OCH₂CH=C H₂ | Et | 127-130 |
| 12-011 | Ph | 4-Me-Ph | tBu | C(O)OMe | Me | 109-111 |
| 12-012 | Ph | 4-Me-Ph | tBu | C(O)OEt | Me | 109-111 |
| 12-013 | Ph | 4-Me-Ph | tBu | C(O)OCH₂CH=C H₂ | Me | 85-87 |
| 12-014 | Ph | 2,4-Me₂-Ph | tBu | C(O)OMe | CH₂OM e | 91-94 |
| 12-015 | Ph | 2,4-Me₂-Ph | tBu | C(O)OEt | CH₂OM e | 131-133 |
| 12-016 | Ph | 2,4-Me₂-Ph | tBu | C(O)OCH₂CH=C H₂ | CH₂OM e | 108-110 |
| 12-017 | 3-CF₃-Ph | 2,4-Me₂-Ph | tBu | C(O)OMe | Me | * |
| 12-018 | 3-CF₃-Ph | 2,4-Me₂-Ph | tBu | C(O)OEt | Me | * |
| 12-019 | 3-CF₃-Ph | 2,4-Me₂-Ph | tBu | C(O)OCH₂CH=C H₂ | Me | * |

**[Table 13]**

| 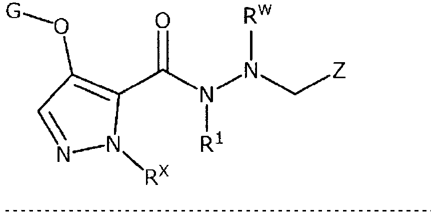 | | | | | | |
|---|---|---|---|---|---|---|
| No. | G | Z | R^{X} | R¹ | R^{W} | m.p. (°C) |
| 13-001 | Ph | 2-Cl-4-OH-Ph | tBu | Et | C(O)OMe | * |
| 13-002 | 3-CF₃-Ph | 2,4-Me₂-Ph | iBu | CH₂CN | C(O)OMe | * |

**[Table 14]**

| 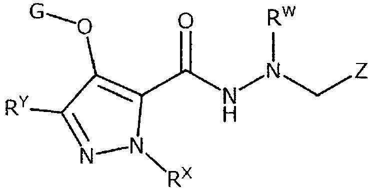 | | | | | | |
|---|---|---|---|---|---|---|
| No. | G | Z | R^{X} | R^{Y} | R^{W} | m.p. (°C) |
| 14-001 | 3-CF₃-Ph | 2,4-Me₂-Ph | tBu | Br | C(O)OMe | 142-144 |
| 14-002 | 3-CF₃-Ph | 2-F-4-OMe-Ph | tBu | Br | C(O)OMe | * |
| 14-003 | 3-CF₃-Ph | 4-OMe-Ph | tBu | Br | C(O)OMe | * |
| 14-004 | 3-CF₃-Ph | 2,4-Me₂-Ph | tBu | Cl | C(O)OMe | * |

**[Table 15]**

| 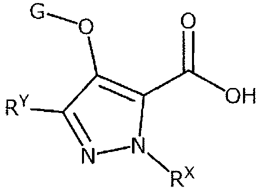 | | | | |
|---|---|---|---|---|
| No. | - G | R^{X} | R^{Y} | |
| i-001 | 3-CF₃-Ph | Ph | H | 177-179 |
| i-002 | 3-CF₃-Ph | tBu | H | 120-124 |
| i-003 | 3-CF₃-Ph | Bn | H | 124-126 |
| i-004 | 3-CF₃-Ph | Me | H | |
| i-005 | 3-CF₃-Ph | 2-Cl-Ph | H | |
| i-006 | 3-CF₃-Ph | 3-Cl-Ph | H | 177-180 |
| i-007 | 3-CF₃-Ph | 4-Cl-Ph | H | |
| i-008 | 2-F-3-cPr-Ph | Ph | H | |
| i-009 | 3-Br-Ph | tBu | H | 95-102 |
| i-010 | 2-F-3-Cl-Ph | tBu | H | 153-155 |
| i-011 | Ph | tBu | H | 142-144 |
| i-012 | 2-CF₃-Ph | tBu | H | 139-141 |
| i-013 | 4-CF₃-Ph | tBu | H | 131-133 |
| i-014 | 3-F-Ph | tBu | H | 154-156 |
| i-015 | 2-Cl-Ph | tBu | H | 166-168 |
| i-016 | 3-Cl-Ph | tBu | H | 122-124 |
| i-017 | 4-Cl-Ph | tBu | H | 119-121 |
| i-018 | 2,3-Cl₂-Ph | tBu | H | 160-162 |
| i-019 | 2,4-Cl₂-Ph | tBu | H | 143-145 |
| i-020 | 3,4-Cl₂-Ph | tBu | H | 143-145 |
| i-021 | 3-I-Ph | tBu | H | 121-123 |
| i-022 | 3-OMe-Ph | tBu | H | 145-147 |
| i-023 | 3-OCF₃-Ph | tBu | H | 112-114 |
| i-024 | 3-NMe₂-Ph | tBu | H | 126-130 |
| i-025 | 3-CF₃-Ph | iPr | H | 177-179 |
| i-026 | Ph | iPr | H | 142-144 |
| i-027 | Ph | Ph | H | 176-178 |
| i-028 | 2-F-3-Br-Ph | tBu | H | 148-151 |
| i-029 | 2-F-3-cPr-Ph | tBu | H | |
| i-030 | 3-CF₃-Ph | F-2a | H | 115-117 |
| i-031 | 3-CF₃-Ph | F-3a | H | 191-193 |
| i-032 | 3-CF₃-Ph | F-4a | H | 166-168 |
| i-033 | Ph | F-2a | H | 121-123 |
| i-034 | Ph | F-3a | H | 187-189 |
| i-035 | Ph | F-4a | H | 153-155 |
| i-036 | 3-Me-Ph | tBu | H | 116-118 |
| i-037 | 3-SMe-Ph | tBu | H | 117-119 |
| i-038 | 3-CF₃-Ph | tBu | Br | 126-128 |
| i-039 | 3-Me-Ph | tBu | Cl | 105-107 |
| i-040 | 2-F-Ph | tBu | H | 118-120 |
| i-041 | 2,3-F₂-Ph | tBu | H | 129-131 |
| i-042 | 2-F-3-OMe-Ph | tBu | H | 171-174 |

**[Table 16]**

| | | | | | |
|---|---|---|---|---|---|
| 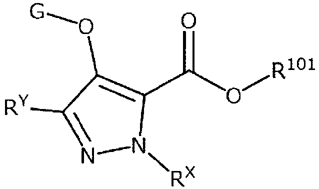 | | | | | |
| - No. | G | R^{X} | R^{Y} | R¹⁰¹ | m.p. (°C) |
| ii-001 | 3-CF₃-Ph | tBu | H | Me | |
| ii-002 | 2-F-3-Br-Ph | tBu | H | Me | |
| ii-003 | 2-F-3-cPr-Ph | tBu | H | Me | |
| ii-004 | 3-Br-Ph | tBu | H | Me | 69-71 |
| ii-005 | 3-Me-Ph | tBu | H | Me | * |

**[Table 17]**

| 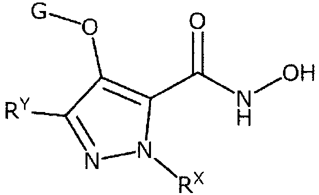 | | | | |
|---|---|---|---|---|
| No. | G | R^{X} | R^{Y} | m.p. (°C) |
| ia-001 | 3-CF₃-Ph | Ph | H | |
| ia-002 | 3-CF₃-Ph | tBu | H | 147-149 |
| ia-003 | 3-CF₃-Ph | Bn | H | |
| ia-004 | 3-CF₃-Ph | Me | H | |
| ia-005 | 3-CF₃-Ph | 2-Cl-Ph | H | |
| ia-006 | 3-CF₃-Ph | 3-Cl-Ph | H | |
| ia-007 | 3-CF₃-Ph | 4-Cl-Ph | H | |
| ia-008 | 2-F-3-cPr-Ph | Ph | H | |
| ia-009 | 3-Br-Ph | tBu | H | |
| ia-010 | 2-F-3-Cl-Ph | tBu | H | |
| ia-011 | Ph | tBu | H | 122-124 |
| ia-012 | 2-CF₃-Ph | tBu | H | |
| ia-013 | 4-CF₃-Ph | tBu | H | |
| ia-014 | 3-F-Ph | tBu | H | |
| ia-015 | 2-Cl-Ph | tBu | H | |
| ia-016 | 3-Cl-Ph | tBu | H | 132-134 |
| ia-017 | 4-Cl-Ph | tBu | H | |
| ia-018 | 2,3-Cl₂-Ph | tBu | H | |
| ia-019 | 2,4-Cl₂-Ph | tBu | H | |
| ia-020 | 3,4-Cl₂-Ph | tBu | H | |
| ia-021 | 3-I-Ph | tBu | H | |
| ia-022 | 3-OMe-Ph | tBu | H | |
| ia-023 | 3-OCF₃-Ph | tBu | H | |
| ia-024 | 3-NMe₂-Ph | tBu | H | |
| ia-025 | 3-CF₃-Ph | iPr | H | |
| ia-026 | Ph | iPr | H | |
| ia-027 | Ph | Ph | H | |
| ia-028 | 2-F-3-Br-Ph | tBu | H | |
| ia-029 | 2-F-3-cPr-Ph | tBu | H | |
| ia-030 | 3-CF₃-Ph | F-2a | H | |
| ia-031 | 3-CF₃-Ph | F-3a | H | |
| ia-032 | 3-CF₃-Ph | F-4a | H | |
| ia-033 | Ph | F-2a | H | |
| ia-034 | Ph | F-3a | H | |
| ia-035 | Ph | F-4a | H | |
| ia-036 | 3-Me-Ph | tBu | H | 136-138 |
| ia-037 | 3-SMe-Ph | tBu | H | |
| ia-038 | 3-CF₃-Ph | tBu | Br | |
| ia-039 | 3-Me-Ph | tBu | Cl | |
| ia-040 | 2-F-Ph | tBu | H | 118-120 |
| ia-041 | 2,3-F₂-Ph | tBu | H | 129-131 |
| ia-042 | 2-F-3-OMe-Ph | tBu | H | 171-174 |

**[Table 18]**

| 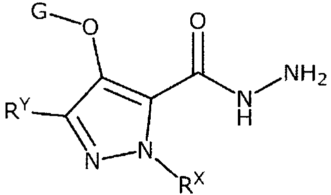 | | | | |
|---|---|---|---|---|
| No. | G | R^{X} | R^{Y} | m.p. (°C) |
| ib-001 | 3-CF₃-Ph | Ph | H | |
| ib-002 | 3-CF₃-Ph | tBu | H | 111-113 |
| ib-003 | 3-CF₃-Ph | Bn | H | |
| ib-004 | 3-CF₃-Ph | Me | H | |
| ib-005 | 3-CF₃-Ph | 2-Cl-Ph | H | |
| ib-006 | 3-CF₃-Ph | 3-Cl-Ph | H | |
| ib-007 | 3-CF₃-Ph | 4-Cl-Ph | H | |
| ib-008 | 2-F-3-cPr-Ph | Ph | H | |
| ib-009 | 3-Br-Ph | tBu | H | |
| ib-010 | 2-F-3-Cl-Ph | tBu | H | |
| ib-011 | Ph | tBu | H | 81-83 |
| ib-012 | 2-CF₃-Ph | tBu | H | |
| ib-013 | 4-CF₃-Ph | tBu | H | |
| ib-014 | 3-F-Ph | tBu | H | |
| ib-015 | 2-Cl-Ph | tBu | H | |
| ib-016 | 3-Cl-Ph | tBu | H | 149-151 |
| ib-017 | 4-Cl-Ph | tBu | H | |
| ib-018 | 2,3-Cl₂-Ph | tBu | H | |
| ib-019 | 2,4-Cl₂-Ph | tBu | H | |
| ib-020 | 3,4-Cl₂-Ph | tBu | H | |
| ib-021 | 3-I-Ph | tBu | H | |
| ib-022 | 3-OMe-Ph | tBu | H | |
| ib-023 | 3-OCF₃-Ph | tBu | H | |
| ib-024 | 3-NMe₂-Ph | tBu | H | |
| ib-025 | 3-CF₃-Ph | iPr | H | |
| ib-026 | Ph | iPr | H | |
| ib-027 | Ph | Ph | H | |
| ib-028 | 2-F-3-Br-Ph | tBu | H | |
| ib-029 | 2-F-3-cPr-Ph | tBu | H | |
| ib-030 | 3-CF₃-Ph | F-2a | H | |
| ib-031 | 3-CF₃-Ph | F-3a | H | |
| ib-032 | 3-CF₃-Ph | F-4a | H | |
| ib-033 | Ph | F-2a | H | |
| ib-034 | Ph | F-3a | H | |
| ib-035 | Ph | F-4a | H | |
| ib-036 | 3-Me-Ph | tBu | H | 120-122 |
| ib-037 | 3-SMe-Ph | tBu | H | |
| ib-038 | 3-CF₃-Ph | tBu | Br | |
| ib-039 | 3-Me-Ph | tBu | Cl | |
| ib-040 | 2-F-Ph | tBu | H | |
| ib-041 | 2,3-F₂-Ph | tBu | H | |
| ib-042 | 2-F-3-OMe-Ph | tBu | H | |

Among the compounds in accordance with the present invention shown in Table 1 through Table 14, ¹H-NMR data of the compounds for which a melting point is not described is shown in Table 19 through Table 21. Note that the data below was measured in deuterated chloroform or deuterated dimethyl sulfoxide. The term "#1" indicates that measurement was carried out at 300 MHz (model: JNM-ECX300 or JNM-ECP300, available from JEOL Ltd.) Similarly, "#2" indicates that measurement was carried out at 400 MHz (model: JNM-ECZ400S, available from JEOL Ltd.)

**[Table 19]**

| | |
|---|---|
| No. | ¹H-NMR (CDCl₃, Me₄Si) |

1-001(#1): δ9.09-8.96 (m, 1H), 7.44-7.03 (m, 8H), 6.99-6.88 (m, 2H), 4.88 (s, 2H), 1.72 (s, 9H).
1-002(#2): δ9.01 (br, 1H), 7.37-7.33 (m, 3H), 7.32-7.28 (m, 4H), 7.13-7.10 (m, 2H), 7.03-6.94 (m, 2H), 4.93 (s, 2H), 1.73 (s, 9H).
1-003(#1): δ9.14 (br, 1H), 7.46-7.39 (m, 1H), 7.32-7.17 (m, 5H), 7.12-7.04 (m, 2H), 7.01-6.92 (m, 1H), 4.90 (s, 2H), 1.71 (s, 9H).
1-004(#1): δ.94 (br, 1H), 7.33-7.21 (m, 6H), 7.10 (s, 1H), 6.89-6.82 (m, 2H), 4.89 (s, 2H), 1.70 (s, 9H).
1-005(#1): δ8.84 (br, 1H), 7.36 (d, J = 9.0 Hz, 1H), 7.29-7.24 (m, 4H), 7.14 (s, 1H), 7.03 (d, J = 2.7 Hz, 1H), 6.76 (dd, J = 9.0, 2.7 Hz, 1H), 4.90 (s, 2H), 1.70 (s, 9H).
1-006(#1): δ8.87 (br, 1H), 7.50-7.40 (m, 1H), 7.33-7.21 (m, 5H), 7.13 (s, 1H), 7.08-6.97 (m, 1H), 6.92-6.82 (m, 1H), 4.89 (s, 2H), 1.71 (s, 9H).
1-007(#2): δ8.93 (br, 1H), 7.35-7.21 (m, 5H), 7.15 (s, 1H), 7.13-7.07 (m, 1H), 6.98-6.92 (m, 1H), 6.86-6.76 (m, 1H), 4.89 (s, 2H), 1.71 (s, 9H).
1-008(#2): δ8.91 (br, 1H), 7.36-7.24 (m, 5H), 7.17 (s, 1H), 6.87-6.79 (m, 1H), 6.74-6.62 (m, 2H), 4.89 (s, 2H), 1.71 (s, 9H).
1-009(#2): δ9.03 (br, 1H), 7.32-7.19 (m, 5H), 7.15 (s, 1H), 6.72-6.64 (m, 1H), 6.55-6.47 (m, 2H), 4.88 (s, 2H), 3.78 (s, 3H), 1.71 (s, 9H).
1-010(#2): δ8.92 (br, 1H), 7.37-7.23 (m, 5H), 7.16 (s, 1H), 7.04-6.96 (m, 1H), 6.88-6.80 (m, 2H), 4.88 (s, 2H), 1.71 (s, 9H).
1-012(#2): δ8.92 (br, 1H), 7.33-7.29 (m, 2H), 7.13-7.09 (m, 3H), 6.97-6.94 (m, 3H), 6.87-6.85 (m, 1H), 4.92 (s, 2H), 2.35 (s, 3H), 2.27 (s, 3H), 1.73 (s, 9H).
1-013(#2): δ9.01 (br, 1H), 7.55-7.46 (m, 4H), 7.35-7.30 (m, 2H), 7.15-7.11 (m, 2H), 6.95-6.92 (m, 2H), 4.98 (m, 2H), 1.72 (s, 9H).
1-014(#2): δ8.91 (br, 1H), 7.41-7.36 (m, 1H), 7.28-7.16 (m, 4H), 7.14-7.10 (m, 1H), 7.04 (s, 1H), 6.87-6.81 (m, 1H), 4.85 (s, 2H), 1.64 (s, 9H).
1-015(#2): δ8.98 (br, 1H), 7.36-7.23 (m, 5H), 7.20-7.11 (m, 2H), 6.91-6.83 (m, 1H), 4.90 (s, 2H), 1.71 (s, 9H).
1-016(#2): δ8.89 (br, 1H), 7.35-7.25 (m, 4H), 7.21-7.10 (m, 2H), 7.06-6.98 (m, 1H), 6.94-6.86 (m, 1H), 4.93 (s, 2H), 1.70 (s, 9H).
1-017(#2): δ9.13 (br, 1H), 7.39-7.31 (m, 4H), 7.16-7.11 (m, 3H), 6.98-6.96 (m, 2H), 5.03 (s, 2H), 1.72 (s, 9H).
1-018(#2): δ8.79 (br, 1H), 7.45-7.36 (m, 2H), 7.23-7.07 (m, 7H), 4.89 (s, 2H), 2.31 (s, 3H), 1.73 (s, 9H).
1-019(#2): δ8.75 (br, 1H), 7.45-7.36 (m, 2H), 7.22 (s, 1H), 7.15-7.07 (m, 3H), 6.98 (s, 1H), 6.86-6.84 (m, 1H), 4.93 (s, 2H), 2.34 (s, 3H), 2.27 (s, 3H), 1.73 (s, 9H).
1-020(#2): δ8.84 (s, 1H), 7.47-7.38 (m, 2H), 7.30-7.23 (m, 5H), 7.15-7.08 (m, 2H), 4.90 (s, 2H), 1.72 (s, 9H).
1-024(#2): δ8.76 (br, 1H), 7.25-7.16 (m, 2H), 7.10-7.07 (m, 2H), 6.80-6.76 (m, 2H), 6.88-6.81 (m, 2H), 4.93 (s, 2H), 2.35 (s, 3H), 2.29 (s, 3H), 1.72 (s, 9H).
1-025(#2): δ8.95 (br, 1H), 7.39-7.34 (m, 2H), 7.25-7.16 (m, 3H), 7.13-7.06 (m, 1H), 7.98-7.97 (m, 1H), 6.85-6.82 (m, 1H), 5.04 (s, 2H), 1.72 (s, 9H).
1-026(#2): δ8.76 (br, 1H), 7.28-7.18 (m, 2H), 7.10-7.09 (m, 1H), 6.98 (s, 1H), 6.89-6.79 (m, 2H), 6.73-6.62 (m, 2H), 4.92 (s, 2H), 2.35 (s, 3H), 2.29 (s, 3H), 1.72 (s, 9H).
1-027(#2): δ8.94 (br, 1H), 7.46-7.34 (m, 3H), 7.19-7.16 (m, 1H), 6.98-6.81 (m, 2H), 6.75-6.67 (m, 2H), 5.03 (s, 2H), 1.72 (s, 9H).
1-028(#2): δ8.96 (br, 1H), 7.35-7.27 (m, 4H), 7.13-7.10 (m, 2H), 6.97-6.95 (m, 2H), 6.81-6.78 (m, 2H), 4.86 (s, 2H), 3.77 (s, 3H), 1.73 (s, 9H).
1-029(#2): δ9.03 (br, 1H), 7.41-7.31 (m, 4H), 7.23-7.22 (m, 2H), 7.15-7.11 (m, 2H), 6.95-6.93 (m, 2H), 4.87 (s, 2H), 1.72 (s, 9H).
1-030(#2): δ9.13 (br, 1H), 7.53-7.43 (m, 2H), 7.34-7.30 (m, 2H), 7.25-7.10 (m, 4H), 6.99-6.97 (m, 2H), 5.06 (s, 2H), 1.73 (s, 9H).
1-031(#2): δ9.12 (br, 1H), 7.57-7.46 (m, 4H), 7.36-7.32 (m, 2H), 7.17-7.12 (m, 2H), 6.96-6.93 (m, 2H), 4.98 (s, 2H), 1.72 (s, 9H).
1-032(#2): δ9.08 (br, 1H), 7.47 (m, 1H), 7.35-7.28 (m, 3H), 7.23-7.11 (m, 3H), 7.03-6.94 (m, 2H), 4.87 (s, 2H), 1.72 (s, 9H).
1-033(#2): δ9.16 (br, 1H), 8.17-8.11 (m, 2H), 7.54-7.52 (m, 2H), 7.34-7.30 (m, 2H), 7.15-7.12 (m, 2H), 6.94-6.92 (m, 2H), 5.03 (s, 2H), 1.72 (s, 9H).
1-036(#2): δ9.32 (s, 1H), 7.38-7.29 (m, 4H), 7.26-7.23 (m, 2H), 7.19-7.15 (m, 2H), 6.97-6.94 (m, 2H), 5.69-5.63 (m, 1H), 4.94 (s, 2H), 1.51-1.49 (m, 6H).
1-039(#2): δ8.85 (s, 1H), 7.47-7.38 (m, 4H), 7.23-7.21 (m, 3H), 7.15-7.08 (m, 2H), 4.88 (s, 2H), 1.72 (s, 9H).
1-040(#2): δ8.94 (s, 1H), 7.51-7.37 (m, 4H), 7.24-7.12 (m, 5H), 5.08 (s, 2H), 1.73 (s, 9H).
1-041(#2): δ8.90 (s, 1H), 7.48-7.35 (m, 4H), 7.25-7.10 (m, 4H), 4.89 (s, 2H), 1.72 (s, 9H).
1-042(#2): δ9.03 (s, 1H), 7.33-7.29 (m, 2H), 7.21-7.09 (m, 6H), 6.96-6.93 (m, 2H), 4.89 (s, 2H), 2.30 (s, 3H), 1.73 (s, 9H).
1-043(#2): δ8.97 (s, 1H), 7.34-7.30 (m, 2H), 7.25-7.06 (m, 6H), 6.97-6.94 (m, 2H), 4.95 (s, 2H), 2.39 (s, 3H), 1.73 (s, 9H).
1-045(#2): δ9.10 (s, 1H), 7.99-7.94 (m, 2H), 7.43-7.41 (m, 2H), 7.33-7.29 (m, 2H), 7.13-7.10 (m, 2H), 6.94-6.92 (m, 2H), 4.98 (s, 2H), 3.92 (s, 3H), 1.72 (s, 9H).
1-046(#2): δ8.85 (s, 1H), 7.45-7.36 (m, 3H), 7.22-7.11 (m, 6H), 4.91 (s, 2H), 2.29 (s, 3H), 1.73 (s, 9H).
1-047(#2): δ8.79 (s, 1H), 7.45-7.36 (m, 3H), 7.22-7.04 (m, 6H), 4.97 (s, 2H), 2.38 (s, 3H), 1.73 (s, 9H).
1-048(#2): δ8.88 (s, 1H), 7.81-7.71 (m, 4H), 7.51-7.46 (m, 3H), 7.29-7.27 (m, 2H), 7.14-7.10 (m, 2H), 6.94 (m, 1H), 5.11 (s, 2H), 1.72 (s, 9H).
1-049(#2): δ8.93 (s, 1H), 7.96-7.94 (m, 2H), 7.44-7.36 (m, 4H), 7.20-7.07 (m, 3H), 4.99 (s, 2H), 3.92 (s, 3H), 1.72 (s, 9H).
1-050(#2): δ9.03 (s, 1H), 7.34-7.30 (m, 2H), 7.13-7.09 (m, 2H), 6.98-6.95 (m, 2H), 6.87-6.78 (m, 2H), 6.69-6.67 (m, 1H), 5.92 (s, 2H), 4.82 (s, 2H), 1.73 (s, 9H).
1-052(#1): δ8.99 (s, 1H), 7.23-7.07 (m, 6H), 6.93-6.90 (m, 1H), 6.75-6.72 (m, 2H), 4.89 (s, 2H), 2.32 (s, 3H), 2.31 (s, 3H), 1.73 (s, 9H).
1-053(#1): δ8.93 (s, 1H), 7.21-7.10 (m, 3H), 6.97-6.85 (m, 3H), 6.76-6.72 (m, 2H), 4.92 (s, 2H), 2.35 (s, 3H), 2.32 (s, 3H), 2.28 (s, 3H), 1.73 (s, 9H).
1-054(#1): δ9.13 (s, 1H), 7.39-7.32 (m, 2H), 7.22-7.12 (m, 3H), 6.95-6.75 (m, 3H), 5.03 (s, 2H), 2.33 (s, 3H), 1.72 (s, 9H).
1-055(#1): δ9.04 (s, 1H), 7.31-7.11 (m, 6H), 6.95-6.92 (m, 1H), 6.75-6.72 (m, 2H), 4.89 (s, 2H), 2.33 (s, 3H), 1.72 (s, 9H).
1-056(#1): δ9.03 (s, 1H), 7.34-7.28 (m, 3H), 7.14-7.08 (m, 2H), 6.70-6.95 (m, 2H), 6.87-6.86 (m, 1H), 6.69-6.66 (m, 1H), 5.00 (s, 2H), 3.76 (s, 3H), 1.73 (s, 9H).
1-057(#1): δ8.84 (s, 1H), 7.48-7.29 (m, 4H), 7.15-7.12 (m, 2H), 6.86-6.85 (m, 1H), 6.70-6.67 (m, 1H), 5.01 (s, 2H), 3.76 (s, 3H), 1.73 (s, 9H).
1-058(#1): δ89.05 (s, 1H), 7.34-7.31 (m, 1H), 7.23-7.12 (m, 2H), 6.93-6.86 (m, 2H), 6.78-6.66 (m, 3H), 5.01 (s, 2H), 3.76 (s, 3H), 2.33 (s, 3H), 1.73 (s, 9H).
1-059(#2): δ9.08 (br, 1H), 7.29-7.22 (m, 2H), 7.19-7.06 (m, 4H), 6.50-6.44 (m, 1H), 6.33-6.30 (m, 1H), 6.29-6.24 (m, 1H), 4.89 (s, 2H), 2.93 (s, 5H), 2.31 (s, 4H), 1.73 (s, 9H).
1-060(#2): δ8.92 (br, 1H), 7.26-7.16 (m, 3H), 7.14-7.06 (m, 3H), 7.00-6.95 (m, 1H), 6.86-6.81 (m, 1H), 6.73-6.65 (m, 1H), 4.89 (s, 2H), 2.46 (s, 3H), 2.32 (s, 3H), 1.72 (s, 9H).
1-061(#2): δ9.27-9.15 (m, 1H), 8.78-8.73 (m, 1H), 8.70-8.59 (m, 1H), 7.85-7.78 (m, 1H), 7.59-7.47 (m, 3H), 7.47-7.39 (m, 1H), 7.35-7.16 (m, 6H), 4.91 (s, 2H).
1-062(#2): δ9.77-9.39 (m, 1H), 8.46-8.30 (m, 1H), 7.93-7.83 (m, 1H), 7.77-7.66 (m, 1H), 7.48-7.09 (m, 9H), 7.05-6.93 (m, 2H), 4.91 (s, 2H).
1-064(#1): δ8.88 (br, 1H), 7.49-7.41 (m, 1H), 7.34-7.01 (m, 8H), 4.87 (s, 2H), 2.71 (s, 3H), 2.32 (s, 3H), 1.71 (s, 9H).
1-065(#1): δ8.69 (br, 1H), 7.71-7.62 (m, 1H), 7.55-7.46 (m, 2H), 7.27-7.16 (m, 4H), 7.14-7.08 (m, 2H), 4.88 (s, 2H), 3.05 (s, 3H), 2.33 (s, 3H), 1.71 (s, 9H).
1-066(#2): δ9.04 (s, 1H), 7.35-7.26 (m, 3H), 7.23-7.16 (m, 1H), 7.11 (s, 1H), 6.96-6.90 (m, 1H), 6.86-6.73 (m, 3H), 4.87 (s, 2H), 3.77 (s, 3H), 2.32 (s, 3H), 1.73 (s, 9H).
1-067(#1): δ9.01 (br, 1H), 7.37-7.23 (m, 3H), 7.15-7.08 (m, 2H), 7.01-6.95 (m, 2H), 6.63-6.49 (m, 2H), 4.94 (d, J = 0.9 Hz, 2H), 3.76 (s, 3H), 1.72 (s, 9H).
1-068(#1): δ9.04 (br, 1H), 7.33-7.24 (m, 1H), 7.23-7.15 (m, 1H), 7.11 (s, 1H), 6.96-6.88 (m, 1H), 6.82-6.74 (m, 2H), 6.62-6.49 (m, 2H), 4.94 (d, J = 0.9 Hz, 2H), 3.76 (s, 3H), 2.32 (s, 3H), 1.72 (s, 9H).
1-069(#1): δ8.84 (br, 1H), 7.48-7.33 (m, 2H), 7.30-7.22 (m, 2H), 7.18-7.09 (m, 2H), 6.63-6.47 (m, 2H), 4.94 (s, 2H), 3.76 (s, 3H), 1.72 (s, 9H).
1-071(#2): δ9.07-8.94 (m, 1H), 8.36 (s, 1H), 7.79-7.72 (m, 1H), 7.51-7.44 (m, 1H), 7.43-7.37 (m, 3H), 7.35-7.26 (m, 1H), 7.20-7.13 (m, 1H), 4.94 (s, 2H), 1.71 (s, 9H).
1-072(#1): δ9.20-8.72 (m, 1H), 7.48-7.29 (m, 2H), 7.24 (s, 1H), 7.17-7.05 (m, 4H), 6.58-6.49 (m, 2H), 4.78 (s, 2H), 3.73 (br, 2H), 1.71 (s, 9H).
1-073(#1): δ9.05-8.92 (m, 1H), 7.30-7.22 (m, 2H), 7.19-6.95 (m, 7H), 4.92 (s, 2H), 2.29 (s, 3H), 1.71 (s, 9H).
1-074(#1): δ10.13 (br, 1H), 8.19-8.04 (m, 2H), 8.01-7.92 (m, 1H), 7.84-7.66 (m, 1H), 7.59-7.49 (m, 2H), 7.41-7.33 (m, 1H), 7.22-7.10 (m, 2H), 6.99-6.82 (m, 2H), 5.24 (s, 2H), 1.73 (s, 9H).
1-075(#2): δ8.97 (br, 1H), 7.59-7.52 (m, 2H), 7.51-7.36 (m, 4H), 7.24 (s, 1H), 7.15 (s, 1H), 7.11-7.06 (m, 1H), 4.98 (s, 2H), 1.71 (s, 9H).
1-076(#2): δ8.83 (br, 1H), 7.47-7.29 (m, 4H), 7.23 (s, 1H), 7.16 (s, 1H), 7.13-7.04 (m, 1H), 6.99-6.93 (m, 2H), 4.89 (s, 2H), 1.72 (s, 9H).
1-077(#2): δ8.96 (br, 1H), 7.48-7.36 (m, 4H), 7.30-7.19 (m, 1H), 7.18-7.08 (m, 4H), 4.92 (s, 2H), 1.71 (s, 9H).
1-079(#2): δ8.88 (br, 1H), 8.39-8.21 (m, 2H), 7.68-7.55 (m, 3H), 7.45-7.33 (m, 2H), 7.24-7.20 (m, 1H), 7.16 (s, 1H), 7.12 (s, 1H), 7.05-6.96 (m, 1H), 5.37 (s, 2H), 1.74 (s, 9H).
1-080(#1): δ8.93 (br, 1H), 7.51-7.34 (m, 3H), 7.18-7.10 (m, 2H), 6.76 (d, J = 3.6 Hz, 1H), 6.67 (d, J = 3.6 Hz, 1H), 4.99 (s, 2H), 1.73 (s, 9H).
1-081(#2): δ8.97-8.81 (m, 1H), 7.31-7.22 (m, 2H), 7.17-7.06 (m, 3H), 7.04-6.88 (m, 2H), 6.80-6.71 (m, 1H), 4.92 (s, 2H), 2.30 (s, 3H), 1.70 (s, 9H).
1-082(#2): δ9.05-8.92 (m, 1H), 7.32-7.21 (m, 2H), 7.13-7.05 (m, 3H), 7.03-6.92 (m, 1H), 6.79-6.71 (m, 1H), 6.65-6.55 (m, 1H), 4.92 (s, 2H), 3.91 (s, 3H), 2.29 (s, 3H), 1.70 (s, 9H).
1-083(#2): δ8.91-8.78 (m, 1H), 7.35-7.20 (m, 3H), 7.14-7.06 (m, 3H), 6.99-6.89 (m, 2H), 4.92 (s, 2H), 2.30 (s, 3H), 1.70 (s, 9H).
1-084(#2): δ8.95-8.81 (m, 1H), 7.48-7.35 (m, 3H), 7.33-7.20 (m, 3H), 7.18-7.08 (m, 3H), 4.89 (s, 2H), 2.61 (q, J = 7.6 Hz, 2H), 1.72 (s, 9H), 1.20 (t, J = 7.6 Hz, 3H).
1-085(#2): δ9.07-8.98 (m, 1H), 7.35-7.25 (m, 4H), 7.16-7.08 (m, 4H), 6.99-6.92 (m, 2H), 4.88 (s, 2H), 2.61 (q, J = 7.6 Hz, 2H), 1.72 (s, 9H), 1.21 (t, J = 7.6 Hz, 3H).
1-086(#2): δ9.10-9.00 (m, 1H), 7.33-7.25 (m, 2H), 7.24-7.10 (m, 4H), 6.96-6.90 (m, 1H), 6.82-6.73 (m, 2H), 4.90 (s, 2H), 2.62 (q, J = 7.6 Hz, 2H), 2.32 (s, 3H), 1.72 (s, 9H), 1.21 (t, J = 7.6 Hz, 3H).
1-090(#1): δ9.04 (s, 1H), 7.43-7.37 (m, 2H), 7.25-7.16 (m, 3H), 7.11 (s, 1H), 6.96-6.91 (m, 1H), 6.77-6.70 (m, 2H), 4.88 (s, 2H), 2.34 (s, 3H), 1.72 (s, 9H).
1-092(#1): δ9.06 (br, 1H), 7.51-7.34 (m, 3H), 7.29 (s, 1H), 7.20-7.14 (m, 2H), 5.06 (s, 2H), 1.72 (s, 9H).
1-093(#1): δ9.04 (br, 1H), 7.95 (s, 1H), 7.77-7.55 (m, 3H), 7.50-7.25 (m, 6H), 7.23-7.08 (m, 2H), 4.93 (s, 2H), 1.72 (s, 9H).
1-094(#1): δ8.98 (br, 1H), 7.36-7.21 (m, 4H), 7.14-7.05 (m, 2H), 6.99-6.91 (m, 2H), 6.81-6.73 (m, 2H), 4.84 (s, 2H), 3.98 (q, J = 6.9 Hz, 2H), 1.72 (s, 9H), 1.40 (t, J = 6.9 Hz, 3H).
1-095(#1): δ9.00 (br, 1H), 7.30-7.23 (m, 2H), 7.21-7.13 (m, 1H), 7.11 (s, 1H), 6.94-6.88 (m, 1H), 6.82-6.71 (m, 4H), 4.85 (s, 2H), 3.98 (q, J = 7.2 Hz, 2H), 2.32 (s, 3H), 1.73 (s, 9H), 1.40 (t, J = 7.2 Hz, 3H).
1-096(#1): δ8.80 (br, 1H), 7.48-7.33 (m, 2H), 7.29-7.20 (m, 3H), 7.17-7.07 (m, 2H), 6.82-6.73 (m, 2H), 4.85 (s, 2H), 3.98 (q, J = 6.9 Hz, 2H), 1.72 (s, 9H), 1.40 (t, J = 6.9 Hz, 3H).
1-097(#1): δ8.86 (br, 1H), 8.14-8.02 (m, 1H), 7.70-7.58 (m, 1H), 7.49-7.33 (m, 2H), 7.30-7.24 (m, 1H), 7.19-7.10 (m, 2H), 6.72-6.63 (m, 1H), 4.86 (s, 2H), 3.90 (s, 3H), 1.71 (s, 9H).
1-098(#1): δ8.89 (br, 1H), 8.07 (s, 1H), 7.69-7.57 (m, 1H), 7.49-7.33 (m, 2H), 7.29-7.25 (m, 1H), 7.17-7.11 (m, 2H), 6.70-6.62 (m, 1H), 4.85 (s, 2H), 4.31 (q, J = 7.2 Hz, 2H), 1.71 (s, 9H), 1.37 (t, J = 7.2 Hz, 3H).
1-099(#2): δ8.84 (br, 1H), 7.46-7.32 (m, 2H), 7.23 (s, 1H), 7.19-7.09 (m, 3H), 6.68-6.62 (m, 2H), 4.97 (s, 2H), 3.77 (s, 3H), 2.32 (s, 3H), 1.73 (s, 9H).
1-100(#2): δ9.02 (br, 1H), 7.34-7.28 (m, 2H), 7.18-7.07 (m, 3H), 6.99-6.94 (m, 2H), 6.67-6.64 (m, 2H), 4.97 (s, 2H), 3.77 (s, 3H), 2.32 (s, 3H), 1.73 (s, 9H).
1-101(#1): δ9.56 (br, 1H), 8.42-8.35 (m, 1H), 7.65-7.57 (m, 1H), 7.47-7.27 (m, 3H), 7.20-7.15 (m, 2H), 7.12-7.06 (m, 1H), 5.02 (s, 2H), 1.72 (s, 9H).
1-102(#1): δ8.93 (br, 1H), 7.35-7.24 (m, 2H), 7.18-7.05 (m, 3H), 7.00-6.92 (m, 2H), 6.72-6.66 (m, 1H), 6.60-6.52 (m, 1H), 4.88 (s, 2H), 3.75 (s, 3H), 2.37 (s, 3H), 1.72 (s, 9H).
1-103(#1): δ8.97 (br, 1H), 7.23-7.10 (m, 3H), 6.95-6.88 (m, 1H), 6.80-6.68 (m, 3H), 6.61-6.54 (m, 1H), 4.90 (s, 2H), 3.75 (s, 3H), 2.38 (s, 3H), 2.32 (s, 3H), 1.73 (s, 9H).
1-104(#1): δ8.78 (br, 1H), 7.49-7.33 (m, 2H), 7.27-7.23 (m, 1H), 7.17-7.08 (m, 3H), 6.72-6.67 (m, 1H), 6.60-6.53 (m, 1H), 4.89 (s, 2H), 3.75 (s, 3H), 2.37 (s, 3H), 1.72 (s, 9H).
1-105(#1): δ9.03 (br, 1H), 7.20-7.10 (m, 3H), 6.94-6.87 (m, 1H), 6.79-6.72 (m, 2H), 6.69-6.62 (m, 2H), 4.97 (s, 2H), 3.77 (s, 3H), 2.34 (s, 3H), 2.32 (s, 3H), 1.73 (s, 9H).
1-106(#1): δ8.17 (br, 1H), 7.46-7.14 (m, 8H), 4.99 (s, 2H), 2.26 (s, 3H), 1.70 (s, 9H).
1-107(#1): δ8.81 (br, 1H), 7.48-7.34 (m, 2H), 7.25-7.22 (m, 1H), 7.16-7.00 (m, 4H), 6.87-6.78 (m, 1H), 4.84 (s, 2H), 3.85 (s, 3H), 1.72 (s, 9H).
1-108(#2): δ9.09 (br, 1H), 7.36-7.29 (m, 2H), 7.24-7.19 (m, 1H), 7.15-7.09 (m, 2H), 6.99-6.93 (m, 2H), 6.84-6.79 (m, 2H), 4.95 (s, 2H), 3.77 (s, 3H), 1.72 (s, 9H).
1-109(#2): δ9.09 (br, 1H), 7.25-7.16 (m, 2H), 7.12 (s, 1H), 6.96-6.91 (m, 1H), 6.84-6.80 (m, 2H), 6.79-6.73 (m, 2H), 4.96 (s, 2H), 3.77 (s, 3H), 2.33 (s, 3H), 1.72 (s, 9H).
1-110(#2): δ8.90 (br, 1H), 7.48-7.35 (m, 2H), 7.30-7.08 (m, 4H), 6.87-6.81 (m, 2H), 4.95 (s, 2H), 3.78 (s, 3H), 1.72 (s, 9H).
1-111(#2): δ8.91 (br, 1H), 7.27-7.19 (m, 2H), 7.17 (s, 1H), 7.12-7.07 (m, 1H), 6.98-6.94 (m, 1H), 6.87-6.80 (m, 3H), 4.95 (s, 2H), 3.79 (s, 3H), 1.72 (s, 9H).
1-112(#1): δ9.00 (br, 1H), 7.35-7.24 (m, 2H), 7.21-7.15 (m, 1H), 7.14-7.05 (m, 2H), 7.01-6.94 (m, 2H), 6.42-6.38 (m, 1H), 6.37-6.30 (m, 1H), 4.93 (s, 2H), 3.77 (s, 3H), 3.76 (s, 3H), 1.72 (s, 9H).
1-113(#1): δ9.03 (br, 1H), 7.24-7.10 (m, 3H), 6.95-6.88 (m, 1H), 6.81-6.73 (m, 2H), 6.42-6.39 (m, 1H), 6.38-6.31 (m, 1H), 4.94 (s, 2H), 3.77 (s, 3H), 3.77 (s, 3H), 2.32 (s, 3H), 1.73 (s, 9H).
1-114(#1): δ8.86 (br, 1H), 7.48-7.31 (m, 2H), 7.29-7.23 (m, 1H), 7.21-7.09 (m, 3H), 6.43-6.39 (m, 1H), 6.38-6.30 (m, 1H), 4.93 (s, 2H), 3.77 (s, 3H), 3.77 (s, 3H), 1.73 (s, 9H).
1-115(#1): δ8.86 (br, 1H), 7.28-7.14 (m, 3H), 7.11-7.04 (m, 1H), 7.00-6.95 (m, 1H), 6.88-6.81 (m, 1H), 6.44-6.40 (m, 1H), 6.38-6.31 (m, 1H), 4.93 (s, 2H), 3.79 (s, 3H), 3.77 (s, 3H), 1.72 (s, 9H).
1-116(#1): δ8.84 (s, 1H), 7.48-7.27 (m, 4H), 7.18-7.09 (m, 2H), 7.06-7.02 (m, 1H), 6.76-6.69 (m, 1H), 5.01 (s, 2H), 3.76 (s, 3H), 1.73 (s, 9H).
1-117(#1): δ9.02 (s, 1H), 7.36-7.27 (m, 3H), 7.15-7.07 (m, 2H), 7.06-7.03 (m, 1H), 7.01-6.94 (m, 2H), 6.75-6.69 (m, 1H), 5.00 (s, 2H), 3.75 (s, 3H), 1.73 (s, 9H).
1-118(#1): δ9.05 (s, 1H), 7.35-7.30 (m, 1H), 7.22-7.15 (m, 1H), 7.12 (s, 1H), 7.07-7.04 (s, 1H), 6.95-6.89 (m, 1H), 6.81-6.70 (m, 3H), 5.01 (s, 2H), 3.76 (s, 3H), 2.33 (s, 3H), 1.73 (s, 9H).
1-119(#2): δ8.88 (s, 1H), 7.45-7.26 (m, 6H), 7.24-7.21 (m, 1H), 7.14 (s, 1H), 7.10-7.05 (m, 1H), 6.71-6.62 (m, 1H), 5.79-5.68 (m, 1H), 5.31-5.23 (m, 1H), 4.91 (s, 2H), 1.72 (s, 9H).
1-120(#1): δ8.92 (br, 1H), 7.31-7.18 (m, 2H), 7.15 (s, 1H), 7.11-7.04 (m, 1H), 7.00-6.95 (m, 1H), 6.90-6.81 (m, 1H), 6.63-6.50 (m, 2H), 4.94 (s, 2H), 3.77 (s, 3H), 1.71 (s, 9H).
1-121(#1): δ8.93 (br, 1H), 7.34-7.18 (m, 2H), 7.15 (s, 1H), 7.11-7.04 (m, 1H), 6.99-6.95 (m, 1H), 6.89-6.81 (m, 2H), 6.72-6.65 (m, 1H), 5.00 (s, 2H), 3.77 (s, 3H), 1.71 (s, 9H).
1-123(#1): δ8.85 (br, 1H), 7.29-7.18 (m, 3H), 7.14 (s, 1H), 7.10-7.04 (m, 1H), 6.97-6.93 (m, 1H), 6.86-6.74 (m, 3H), 4.85 (s, 2H), 3.99 (q, J = 6.9 Hz, 2H), 1.71 (s, 9H), 1.41 (t, J = 6.9 Hz, 3H).
1-124(#1): δ8.90 (br, 1H), 7.46-7.36 (m, 2H), 7.25-7.17 (m, 3H), 7.15 (s, 1H), 7.12-7.06 (m, 1H), 6.97-6.92 (m, 1H), 6.83-6.77 (m, 1H), 4.87 (s, 2H), 1.71 (s, 9H).
1-125(#1): δ8.81 (br, 1H), 7.28-7.18 (m, 1H), 7.17-7.04 (m, 3H), 6.98-6.94 (m, 1H), 6.86-6.79 (m, 1H), 6.72-6.68 (m, 1H), 6.60-6.53 (m, 1H), 4.89 (s, 2H), 3.76 (s, 3H), 2.37 (s, 3H), 1.72 (s, 9H).
1-126(#1): δ8.84 (br, 1H), 7.61 (d, J = 8.4 Hz, 2H), 7.49-7.36 (m, 2H), 7.23 (s, 1H), 7.14 (s, 1H), 7.11-7.04 (m, 3H), 4.86 (s, 2H), 1.71 (s, 9H).
1-127(#2): δ9.04 (br, 1H), 7.38-7.27 (m, 4H), 7.16-7.08 (m, 2H), 7.05-6.92 (m, 4H), 6.45 (t, J = 74.3 Hz, 1H), 4.89 (s, 2H), 1.72 (s, 9H).
1-128(#1): δ9.08 (br, 1H), 7.92-7.80 (m, 2H), 7.49-7.32 (m, 4H), 7.25-7.18 (m, 1H), 7.16-7.05 (m, 2H), 4.98 (s, 2H), 2.56 (s, 3H), 1.71 (s, 9H).
1-129(#1): δ8.96 (br, 1H), 7.49-7.33 (m, 2H), 7.29-7.25 (m, 1H), 7.19-7.10 (m, 2H), 6.19 (d, J = 3.0 Hz, 1H), 5.83 (d, J = 3.0 Hz, 1H), 4.83 (s, 2H), 2.23 (s, 3H), 1.73 (s, 9H).
1-130(#1): δ8.81 (br, 1H), 7.48-7.33 (m, 2H), 7.30-7.24 (m, 1H), 7.17-7.09 (m, 2H), 6.91-6.84 (m, 1H), 6.84-6.70 (m, 2H), 4.80 (s, 2H), 4.26-4.17 (m, 4H), 1.72 (s, 9H).
1-131(#1): δ8.90 (br, 1H), 7.50-7.32 (m, 2H), 7.30-7.07 (m, 4H), 6.61-6.47 (m, 2H), 4.94 (s, 2H), 3.77 (s, 3H), 1.72 (s, 9H).
1-132(#1): δ8.97-8.84 (m, 1H), 7.50-7.33 (m, 2H), 7.33-7.07 (m, 5H), 6.99-6.90 (m, 1H), 5.04 (s, 2H), 2.28 (s, 3H), 1.72 (s, 9H).
1-133(#1): δ9.16-9.05 (m, 1H), 7.35-7.08 (m, 4H), 7.01-6.87 (m, 2H), 6.82-6.70 (m, 2H), 5.03 (s, 2H), 2.32 (s, 3H), 2.28 (s, 3H), 1.72 (s, 9H).
1-134(#1): δ8.98-8.84 (m, 1H), 7.34-7.02 (m, 5H), 7.01-6.91 (m, 2H), 6.87-6.75 (m, 1H), 5.03 (s, 2H), 2.29 (s, 3H), 1.72 (s, 9H).
1-135(#1): δ9.13-9.00 (m, 1H), 7.40-7.19 (m, 3H), 7.17-7.05 (m, 3H), 7.03-6.89 (m, 3H), 5.03 (s, 2H), 2.28 (s, 3H), 1.72 (s, 9H).
1-136(#1): δ8.86-8.76 (m, 1H), 7.51-7.35 (m, 2H), 7.32-6.99 (m, 6H), 4.91 (s, 2H), 2.36 (s, 3H), 1.72 (s, 9H).
1-137(#1): δ9.08-8.98 (m, 1H), 7.29-7.10 (m, 4H), 7.06-6.88 (m, 2H), 6.81-6.70 (m, 2H), 4.91 (s, 2H), 2.36 (s, 3H), 2.33 (s, 3H), 1.72 (s, 9H).
1-138(#1): δ8.90-8.77 (m, 1H), 7.30-6.93 (m, 7H), 6.86-6.77 (m, 1H), 4.91 (s, 2H), 2.36 (s, 3H), 1.71 (s, 9H).
1-139(#1): δ9.04-8.93 (m, 1H), 7.39-7.22 (m, 2H), 7.20-7.07 (m, 4H), 7.06-6.89 (m, 3H), 4.89 (s, 2H), 2.35 (s, 3H), 1.72 (s, 9H).
1-140(#1): δ9.10 (br, 1H), 7.36 (d, J = 8.7 Hz, 2H), 7.19 (t, J = 8.1 Hz, 1H), 7.10 (s, 1H), 7.01 (d, J = 8.4 Hz, 2H), 6.93 (d, J = 8.1 Hz, 1H), 6.78-6.70 (m, 2H), 6.44 (t, J = 74.1 Hz, 1H), 4.90 (s, 2H), 2.32 (s, 3H), 1.72 (s, 9H).
1-141(#2): δ8.86 (br, 1H), 7.25-7.11 (m, 3H), 7.10-7.05 (m, 1H), 6.94 (s, 1H), 6.86-6.80 (m, 1H), 6.70-6.64 (m, 2H), 4.97 (s, 2H), 3.78 (s, 3H), 2.34 (s, 3H), 1.72 (s, 9H).
1-142(#1): δ8.83 (s, 1H), 7.48-7.34 (m, 2H), 7.27-7.23 (m, 1H), 7.20-6.95 (m, 5H), 4.94 (s, 2H), 3.78 (s, 3H), 1.72 (s, 9H).
1-143(#2): δ9.04 (s, 1H), 7.38-7.29 (m, 2H), 7.21-7.09 (m, 3H), 7.00-6.94 (m, 4H), 4.94 (s, 2H), 3.77 (s, 3H), 1.72 (s, 9H).
1-144(#2): δ9.06 (s, 1H), 7.24-7.10 (m, 3H), 7.06-6.91 (m, 3H), 6.86-6.73 (m, 2H), 4.95 (s, 2H), 3.77 (s, 3H), 2.34 (s, 3H), 1.72 (s, 9H).
1-145(#2): δ8.87 (s, 1H), 7.26-7.21 (m, 1H), 7.20-7.08 (m, 3H), 7.06-6.95 (m, 3H), 6.85-6.80 (m, 1H), 4.94 (s, 2H), 3.79 (s, 3H), 1.72 (s, 9H).
1-146(#2): δ8.79 (s, 1H), 7.48-7.35 (m, 3H), 7.31-7.25 (m, 3H), 7.20-7.10 (m, 3H), 4.89 (s, 2H), 2.88 (sep, J = 7.0 Hz, 1H), 1.72 (s, 9H), 1.22 (d, J = 7.0 Hz, 6H).
1-147(#2): δ8.98 (s, 1H), 7.35-7.26 (m, 5H), 7.18-7.09 (m, 3H), 6.99-6.94 (m, 2H), 4.88 (s, 2H), 2.88 (sep, J = 7.0 Hz, 1H), 1.72 (s, 9H), 1.22 (d, J = 7.0 Hz, 6H).
1-148(#2): δ9.01 (s, 1H), 7.33-7.26 (m, 3H), 7.22-7.10 (m, 3H), 6.96-6.91 (m, 1H), 6.82-6.74 (m, 2H), 4.89 (s, 2H), 2.88 (sep, J = 7.0 Hz, 1H), 2.33 (s, 3H), 1.73 (s, 9H), 1.22 (d, J = 7.0 Hz, 6H).
1-149(#2): δ8.80 (s, 1H), 7.31-7.15 (m, 6H), 7.12-7.07 (m, 1H), 6.99-6.96 (m, 1H), 6.86-6.81 (m, 1H), 4.89 (s, 2H), 2.89 (sep, J = 7.0 Hz, 1H), 1.72 (s, 9H), 1.23 (d, J = 7.0 Hz, 6H).
1-150(#2): δ8.87 (s, 1H), 7.33-7.21 (m, 2H), 7.17 (s, 1H), 7.12-7.06 (m, 2H), 6.99-6.97 (m, 1H), 6.88-6.84 (m, 1H), 6.77-6.72 (m, 1H), 5.01 (s, 2H), 3.78 (s, 3H), 1.73 (s, 9H).
1-151(#1): δ9.15-8.98 (m, 1H), 7.32-7.07 (m, 4H), 6.98-6.66 (m, 5H), 4.82 (s, 2H), 2.31 (s, 3H), 1.72 (s, 9H). An OH proton was not observed.
1-155(#1): δ9.47 (br, 1H), 8.40 (s, 1H), 7.66-7.55 (m, 1H), 7.36-7.26 (m, 2H), 7.14-7.02 (m, 3H), 6.99-6.93 (m, 2H), 4.89 (s, 2H), 2.48 (s, 3H), 1.71 (s, 9H).
1-157(#1): δ10.03 (br, 1H), 8.31 (s, 1H), 7.62-7.50 (m, 1H), 7.46-7.30 (m, 2H), 7.21 (s, 1H), 7.18-7.09 (m, 2H), 7.05-6.97 (m, 1H), 4.88 (s, 2H), 2.44-2.33 (m, 3H), 1.70 (s, 9H).
1-159(#1): δ9.62 (s, 1H), 8.15-8.11 (m, 1H), 7.45-7.21 (m, 3H), 7.20-7.15 (m, 2H), 7.14-7.07 (m, 2H), 4.98 (s, 2H), 3.82 (s, 3H), 1.72 (s, 9H).
1-160(#1): δ9.93 (s, 1H), 8.36-8.31 (m, 1H), 7.80-7.76 (m, 1H), 7.43-7.29 (m, 2H), 7.17 (s, 1H), 7.11-7.00 (m, 2H), 5.11 (s, 2H), 1.73 (s, 9H).
1-161(#1): δ9.93 (s, 1H), 8.26-8.23 (m, 1H), 7.64-7.62 (m, 1H), 7.43-7.29 (m, 2H), 7.17 (s, 1H), 7.11-7.00 (m, 2H), 5.13 (s, 2H), 1.73 (s, 9H).
1-162(#1): δ9.97 (s, 1H), 8.29-8.25 (m, 1H), 7.81-7.77 (m, 1H), 7.42-7.29 (m, 2H), 7.17 (s, 1H), 7.09-6.98 (m, 2H), 5.10 (s, 2H), 1.73 (s, 9H).
1-163(#1): δ8.86 (br, 1H), 7.48-7.35 (m, 2H), 7.24 (s, 1H), 7.16-7.08 (m, 3H), 7.06-7.01 (m, 1H), 6.93 (d, J = 8.4 Hz, 1H), 4.88 (s, 2H), 1.72 (s, 9H).
1-164(#1): δ8.81 (br, 1H), 7.29-7.18 (m, 3H), 7.16-7.04 (m, 4H), 6.98-6.91 (m, 1H), 6.85-6.78 (m, 1H), 4.89 (s, 2H), 2.63 (q, J = 7.5 Hz, 2H), 1.72 (s, 9H), 1.22 (t, J = 7.5 Hz, 3H).
1-166(#1): δ9.03-8.87 (m, 1H), 7.55-7.33 (m, 4H), 7.23-7.07 (m, 4H), 5.02 (s, 2H), 1.72 (s, 9H).
1-167(#1): δ9.24-9.09 (m, 1H), 7.53-7.48 (m, 1H), 7.41-7.35 (m, 1H), 7.23-7.10 (m, 3H), 6.99-6.89 (m, 1H), 6.82-6.68 (m, 2H), 5.02 (s, 2H), 2.33 (s, 3H), 1.72 (s, 9H).
1-168(#1): δ9.08-8.88 (m, 1H), 7.55-7.50 (m, 1H), 7.42-7.34 (m, 1H), 7.31-7.06 (m, 4H), 7.01-6.95 (m, 1H), 6.89-6.79 (m, 1H), 5.03 (s, 2H), 1.73 (s, 9H).
1-169(#1): δ9.18-9.04 (m, 1H), 7.54-7.48 (m, 1H), 7.43-7.06 (m, 6H), 7.02-6.91 (m, 2H), 5.01 (s, 2H), 1.72 (s, 9H).
1-170(#1): δ8.86 (s, 1H), 8.59 (s, 1H), 8.40-8.35 (m, 1H), 7.94-7.89 (m, 1H), 7.85-7.76 (m, 1H), 7.74 (s, 1H), 7.40-7.28 (m, 3H), 7.23-7.11 (m, 3H), 4.95 (s, 2H), 1.73 (s, 9H).
1-171(#1): δ8.91 (s, 1H), 7.49-7.34 (m, 3H), 7.33-7.30 (m, 2H), 7.28-7.21 (m, 1H), 7.15 (s, 1H), 7.14-7.09 (m, 1H), 5.02 (s, 2H), 1.72 (s, 9H).
1-172(#1): δ9.09 (s, 1H), 7.49-7.47 (m, 1H), 7.37-7.28 (m, 4H), 7.16-7.09 (m, 2H), 6.99-6.93 (m, 2H), 5.01 (s, 2H), 1.72 (s, 9H).
1-173(#1): δ9.11 (s, 1H), 7.50-7.47 (m, 1H), 7.34-7.26 (m, 2H), 7.23-7.17 (m, 1H), 7.12 (s, 1H), 6.97-6.92 (m, 1H), 6.81-6.73 (m, 2H), 5.02 (s, 2H), 2.34 (s, 3H), 1.72 (s, 9H).
1-174(#1): δ8.91 (s, 1H), 7.51-7.48 (m, 1H), 7.34-7.29 (m, 2H), 7.28-7.18 (m, 1H), 7.16 (s, 1H), 7.13-7.08 (m, 1H), 7.00-6.95 (m, 1H), 6.89-6.81 (m, 1H), 5.02 (s, 2H), 1.72 (s, 9H).
1-177(#1): δ8.87 (br, 1H), 7.50-7.35 (m, 2H), 7.33-7.08 (m, 6H), 5.02-4.91 (m, 2H), 1.71 (s, 9H).
1-178(#1): δ8.89 (br, 1H), 7.33-7.07 (m, 6H), 7.01-6.95 (m, 1H), 6.88-6.80 (m, 1H), 5.04-4.89 (m, 2H), 1.71 (s, 9H).
1-181(#1): δ9.19-9.01 (m, 1H), 8.08-7.96 (m, 2H), 7.52-7.40 (m, 2H), 7.37-7.26 (m, 2H), 7.16-7.07 (m, 2H), 6.98-6.88 (m, 2H), 5.00 (s, 2H), 1.73 (s, 9H). An OH proton was not observed.
1-182(#1): δ9.21-9.06 (m, 1H), 8.07-7.97 (m, 2H), 7.52-7.41 (m, 2H), 7.23-7.08 (m, 2H), 6.97-6.88 (m, 1H), 6.80-6.69 (m, 2H), 5.01 (s, 2H), 2.32 (s, 3H), 1.73 (s, 9H). An OH proton was not observed.
1-183(#1): δ8.80 (br, 1H), 7.23-7.16 (m, 2H), 7.10-7.02 (m, 4H), 6.93-6.92 (m, 1H), 6.85-6.78 (m, 1H), 4.93 (s, 2H), 2.32 (s, 3H), 2.24 (s, 3H), 1.72 (s, 9H).
1-184(#1): δ8.92-8.83 (br, 1H), 7.51-7.31 (m, 4H), 7.30-7.21 (m, 1H), 7.18-7.08 (m, 2H), 7.05-6.94 (m, 1H), 5.03 (s, 2H), 2.27 (s, 3H), 1.73 (s, 9H).
1-185(#1): δ8.77 (br, 1H), 7.52-7.31 (m, 4H), 7.22-7.03 (m, 4H), 4.90 (s, 2H), 2.36 (s, 3H), 1.71 (s, 9H).
1-186(#1): δ8.92 (br, 1H), 7.66 (s, 1H), 7.52-7.30 (m, 5H), 7.20-7.07 (m, 2H), 5.02 (s, 2H), 1.72 (s, 9H).
1-187(#1): δ8.90-8.79 (br, 1H), 7.49-7.30 (m, 2H), 7.23-7.04 (m, 4H), 6.69-6.58 (m, 2H), 4.99 (s, 2H), 4.03-3.90 (m, 2H), 2.30 (s, 3H), 1.72 (s, 9H), 1.41-1.30 (m, 3H).
1-188(#1): δ8.70 (br, 1H), 7.49-7.31 (m, 2H), 7.20-7.02 (m, 4H), 6.75-6.63 (m, 1H), 6.60-6.52 (m, 1H), 4.87 (s, 2H), 4.03-3.88 (m, 2H), 2.36 (s, 3H), 1.72 (s, 9H), 1.45-1.32 (m, 3H).
1-189(#1): δ8.79-8.72 (br, 1H), 7.53-7.31 (m, 3H), 7.24-7.07 (m, 4H), 6.84-6.73 (m, 2H), 4.84 (s, 2H), 3.95-3.80 (m, 2H), 1.86-1.74 (m, 2H), 1.74-1.71 (s, 9H), 1.08-0.92 (m, 3H).
1-190(#1): δ9.11-9.01 (br, 1H), 7.40-7.28 (m, 4H), 7.20-7.05 (m, 2H), 7.05-6.89 (m, 3H), 5.03 (s, 2H), 2.27 (s, 3H), 1.75 (s, 9H).
1-191(#1): δ9.02-8.92 (br, 1H), 7.38-7.29 (m, 3H), 7.22-7.04 (m, 4H), 7.00-6.89 (m, 2H), 4.89 (s, 2H), 2.35 (s, 3H), 1.73 (s, 9H).
1-192(#1): δ9.12 (br, 1H), 7.69-7.61 (m, 1H), 7.41-7.28 (m, 4H), 7.19-7.08 (m, 2H), 7.03-6.92 (m, 2H), 4.99 (s, 2H), 1.73 (s, 9H).
1-193(#1): δ9.00 (br, 1H), 7.37-7.27 (m, 2H), 7.21-7.03 (m, 3H), 7.00-6.90 (m, 2H), 6.69-6.57 (m, 2H), 4.97 (s, 2H), 4.04-3.93 (m, 2H), 2.31 (s, 3H), 1.74 (s, 9H), 1.42-1.29 (m, 3H).
1-194(#1): δ8.90 (br, 1H), 7.38-7.28 (m, 2H), 7.15-7.05 (m, 3H), 7.02-6.89 (m, 2H), 6.73-6.51 (m, 2H), 4.88 (s, 2H), 4.04-3.89 (m, 2H), 2.37 (s, 3H), 1.76 (s, 9H), 1.47-1.32 (m, 3H).
1-195(#1): δ8.95 (br, 1H), 7.35-7.22 (m, 4H), 7.13-7.05 (m, 2H), 7.02-6.91 (m, 2H), 6.85-6.73 (m, 2H), 4.86 (s, 2H), 3.93-3.80 (m, 2H), 1.89-1.75 (m, 2H), 1.72 (s, 9H), 1.06-0.93 (m, 3H).
1-196(#1): δ8.99 (br, 1H), 7.35-7.05 (m, 5H), 6.99-6.90 (m, 1H), 6.79-6.67 (m, 2H), 4.92 (s, 2H), 2.37 (s, 3H), 2.35 (s, 3H), 1.72 (s, 9H).
1-197(#1): δ9.13 (br, 1H), 7.70-7.65 (m, 1H), 7.34-7.30 (m, 2H), 7.23-7.15 (m, 1H), 7.13 (s, 1H), 6.97-6.90 (m, 1H), 6.80-6.72 (m, 2H), 5.00 (s, 2H), 2.33 (s, 3H), 1.73 (s, 9H).
1-198(#1): δ9.04 (br, 1H), 7.22-7.09 (m, 3H), 6.99-6.85 (m, 1H), 6.82-6.58 (m, 4H), 4.98 (s, 2H), 4.05-3.89 (m, 2H), 2.39-2.25 (m, 6H), 1.76 (s, 9H), 1.43-1.30 (m, 3H).
1-199(#1): δ8.92 (br, 1H), 7.22-7.09 (m, 3H), 6.97-6.88 (m, 1H), 6.84-6.51 (m, 4H), 4.89 (s, 2H), 4.03-3.87 (m, 2H), 2.38 (s, 3H), 2.33 (s, 3H), 1.73 (s, 9H), 1.46-1.34 (m, 3H).
1-200(#1): δ8.97 (br, 1H), 7.32-7.08 (m, 4H), 6.98-6.68 (m, 5H), 4.84 (s, 2H), 3.94-3.79 (m, 2H), 2.33 (s, 3H), 1.89-1.75 (m, 2H), 1.72 (s, 9H), 1.08-0.98 (m, 3H).
1-201(#1): δ8.79 (br, 1H), 7.49-7.32 (m, 2H), 7.29-7.21 (m, 3H), 7.22-7.04 (m, 4H), 4.88 (s, 2H), 2.45 (s, 3H), 1.73 (s, 9H).
1-202(#1): δ8.85-8.69 (m, 1H), 7.49-7.31 (m, 2H), 7.24-7.18 (m, 1H), 7.17-6.98 (m, 5H), 4.87 (s, 2H), 2.21 (s, 3H), 2.19 (s, 3H), 1.73 (s, 9H).
1-203(#1): δ9.02-8.93 (m, 1H), 7.21-6.99 (m, 5H), 6.96-6.87 (m, 1H), 6.81-6.68 (m, 2H), 4.87 (s, 2H), 2.32 (s, 3H), 2.22 (s, 3H), 2.20 (s, 3H), 1.73 (s, 9H).
1-204(#1): δ8.85-8.69 (m, 1H), 7.26-7.01 (m, 6H), 6.96-6.88 (m, 1H), 6.86-6.76 (m, 1H), 4.87 (s, 2H), 2.23 (s, 3H), 2.21 (s, 3H), 1.72 (s, 9H).
1-205(#1): δ9.04-8.91 (m, 1H), 7.37-7.22 (m, 2H), 7.18-7.00 (m, 5H), 6.97-6.89 (m, 2H), 4.86 (s, 2H), 2.22 (s, 3H), 2.19 (s, 3H), 1.73 (s, 9H).
1-206(#1): δ8.89-8.76 (m, 1H), 7.49-7.33 (m, 2H), 7.25-7.19 (m, 1H), 7.18-6.94 (m, 5H), 4.87 (s, 2H), 2.26-2.17 (m, 3H), 1.72 (s, 9H).
1-207(#1): δ9.11-8.96 (m, 1H), 7.23-6.88 (m, 6H), 6.80-6.69 (m, 2H), 4.88 (s, 2H), 2.32 (s, 3H), 2.25-2.20 (m, 3H), 1.72 (s, 9H).
1-208(#1): δ8.93-8.77 (m, 1H), 7.29-6.89 (m, 7H), 6.86-6.76 (m, 1H), 4.87 (s, 2H), 2.27-2.22 (m, 3H), 1.72 (s, 9H).
1-209(#1): δ9.07-8.94 (m, 1H), 7.38-7.23 (m, 2H), 7.17-6.88 (m, 7H), 4.87 (s, 2H), 2.25-2.20 (m, 3H), 1.72 (s, 9H).
1-210(#2): δ8.79-8.65 (m, 1H), 7.63-7.54 (m, 1H), 7.40-6.97 (m, 8H), 6.40 (d, J = 3.3 Hz, 1H), 5.05 (s, 2H), 3.77 (s, 3H), 1.73 (s, 9H).
1-211(#2): δ9.00-8.90 (m, 1H), 7.63-7.57 (m, 1H), 7.28-7.21 (m, 2H), 7.19-7.03 (m, 3H), 6.92-6.85 (m, 1H), 6.75-6.63 (m, 2H), 6.42 (d, J = 2.9 Hz, 1H), 5.04 (s, 2H), 3.77 (s, 3H), 2.29 (s, 3H), 1.73 (s, 9H).
1-212(#2): δ9.11-8.97 (br, 1H), 7.24-7.07 (m, 2H), 7.01-6.85 (m, 3H), 6.82-6.67 (m, 3H), 4.90 (s, 2H), 3.84 (s, 6H), 2.31 (s, 3H), 1.75 (s, 9H).
1-213(#1): δ8.94-8.74 (m, 1H), 7.61-6.86 (m, 9H), 6.49-6.39 (m, 1H), 5.06 (s, 2H), 3.70 (s, 3H), 1.72 (s, 9H).
1-214(#1): δ9.13-8.94 (m, 1H), 7.60-7.48 (m, 1H), 7.41-6.77 (m, 6H), 6.72-6.58 (m, 2H), 6.49-6.41 (m, 1H), 5.07 (s, 2H), 3.70 (s, 3H), 2.27 (s, 3H), 1.73 (s, 9H).
1-215(#1): δ9.12-8.93 (m, 1H), 7.60-7.50 (m, 1H), 7.42-6.94 (m, 7H), 6.88-6.74 (m, 2H), 6.50-6.40 (m, 1H), 5.06 (s, 2H), 3.68 (s, 3H), 1.73 (s, 9H).
1-216(#1): δ8.98-8.78 (m, 1H), 7.59-7.48 (m, 1H), 7.37-6.76 (m, 6H), 6.73-6.58 (m, 2H), 6.51-6.41 (m, 1H), 5.07 (s, 2H), 3.71 (s, 3H), 1.72 (s, 9H).
1-217(#1): δ9.14 (br, 1H), 7.47-7.40 (m, 1H), 7.28-7.16 (m, 3H), 6.97-6.87 (m, 2H), 6.81-6.74 (m, 2H), 5.03 (s, 2H), 2.33 (s, 3H), 1.73 (s, 9H).
1-218(#1): δ9.01 (br, 1H), 7.24-7.11 (m, 3H), 6.96-6.81 (m, 2H), 6.78-6.67 (m, 3H), 4.91 (s, 2H), 2.39 (s, 3H), 2.33 (s, 3H), 1.72 (s, 9H).
1-219(#1): δ9.17 (br, 1H), 7.46-7.39 (m, 1H), 7.23-7.03 (m, 3H), 6.96-6.74 (m, 4H), 5.03 (s, 2H), 2.33 (s, 3H), 1.72 (s, 9H).
1-220(#1): δ8.96 (br, 1H), 7.48-7.36 (m, 3H), 7.28-7.21 (m, 2H), 7.18-7.10 (m, 2H), 6.97-6.89 (m, 1H), 5.03 (s, 2H), 1.73 (s, 9H).
1-222(#1): δ9.02 (br, 1H), 7.49-7.35 (m, 3H), 7.28-7.22 (m, 1H), 7.19-7.02 (m, 3H), 6.93-6.84 (m, 1H), 5.03 (s, 2H), 1.72 (s, 9H).
1-223(#1): δ8.97-8.84 (m, 1H), 7.61-7.55 (m, 1H), 7.32-7.18 (m, 3H), 7.14-7.00 (m, 4H), 6.93-6.81 (m, 2H), 6.45-6.38 (m, 1H), 5.03 (s, 2H), 3.77 (s, 3H), 1.73 (s, 9H).
1-224(#1): δ8.77-8.68 (m, 1H), 7.60-7.54 (m, 1H), 7.24-7.00 (m, 6H), 6.87-6.80 (m, 1H), 6.77-6.69 (m, 1H), 6.44-6.39 (m, 1H), 5.04 (s, 2H), 3.78 (s, 3H), 1.72 (s, 9H).
1-225(#1): δ9.34 (br, 1H), 8.08-7.96 (m, 1H), 7.63-7.50 (m, 1H), 7.49-7.27 (m, 5H), 7.20-7.05 (m, 2H), 5.29 (s, 2H), 2.63 (s, 3H), 1.70 (s, 9H).
1-226(#1): δ8.88 (br, 1H), 8.09-7.89 (m, 2H), 7.57-7.29 (m, 4H), 7.23-6.97 (m, 3H), 5.02 (s, 2H), 2.65 (s, 3H), 1.70 (s, 9H).
1-227(#2): δ9.06-8.94 (br, 1H), 7.23-6.64 (m, 8H), 5.93 (s, 2H), 4.83 (s, 2H), 2.32 (s, 3H), 1.77 (s, 9H).
1-228(#1): δ9.12-8.97 (br, 1H), 7.33-7.04 (m, 4H), 6.95-6.87 (m, 1H), 6.81-6.59 (m, 3H), 4.83 (s, 2H), 4.60-4.48 (m, 2H), 3.19-3.00 (m, 2H), 2.32 (s, 3H), 1.71 (s, 9H).
1-229(#1): δ9.11 (br, 1H), 7.36 (br s, 1H), 7.22-7.09 (m, 4H), 6.95-6.89 (m, 1H), 6.79-6.71 (m, 2H), 4.87 (s, 2H), 2.32 (s, 6H), 1.77 (s, 9H).
1-230(#1): δ9.07 (br, 1H), 7.63-7.58 (m, 2H), 7.44-7.38 (m, 1H), 7.33-7.27 (m, 1H), 7.18-7.07 (m, 2H), 6.92-6.87 (m, 1H), 6.72-6.66 (m, 3H), 5.02 (s, 2H), 2.29 (s, 3H), 1.72 (s, 9H).
1-231(#1): δ8.90 (br, 1H), 7.47-7.33 (m, 3H), 7.25-7.07 (m, 5H), 4.87 (s, 2H), 2.32 (s, 3H), 1.72 (s, 9H).
1-232(#1): δ8.91 (br, 1H), 7.64-7.54 (m, 2H), 7.43-7.25 (m, 4H), 7.20-7.09 (m, 2H), 7.06-6.97 (m, 1H), 6.70-6.65 (m, 1H), 5.02 (s, 2H), 1.71 (s, 9H).
1-233(#1): δ9.10 (br, 1H), 7.38-7.25 (m, 3H), 7.18-7.06 (m, 4H), 6.98-6.90 (m, 2H), 4.85 (s, 2H), 2.32 (s, 3H), 1.72 (s, 9H).
1-234(#1): δ9.04 (br, 1H), 7.63-7.57 (m, 2H), 7.43-7.37 (m, 1H), 7.34-7.22 (m, 3H), 7.13-7.05 (m, 2H), 6.92-6.85 (m, 2H), 6.70-6.66 (m, 1H), 5.01 (s, 2H), 1.72 (s, 9H).
1-235(#1): δ8.85-8.70 (m, 1H), 7.86-7.65 (m, 2H), 7.48-6.96 (m, 8H), 5.16 (s, 2H), 3.64 (s, 3H), 1.73 (s, 9H).
1-236(#1): δ8.61-8.38 (m, 1H), 7.44-6.73 (m, 9H), 6.57-6.41 (m, 1H), 5.03 (s, 2H), 3.71 (s, 3H), 2.29 (s, 3H), 1.76-1.64 (m, 9H).
1-237(#1): δ9.00-8.88 (m, 1H), 7.81-7.68 (m, 1H), 7.34-6.95 (m, 8H), 6.92-6.83 (m, 2H), 5.14 (s, 2H), 3.59 (s, 3H), 1.73 (s, 9H).
1-238(#1): δ8.81-8.71 (m, 1H), 7.84-7.68 (m, 2H), 7.33-6.94 (m, 6H), 6.90-6.83 (m, 1H), 6.79-6.69 (m, 1H), 5.16 (s, 2H), 3.65 (s, 3H), 1.73 (s, 9H).
1-239(#2): δ9.12 (br, 1H), 7.20-7.07 (m, 3H), 6.92-6.86 (m, 1H), 6.78-6.67(m, 3H), 6.58-6.51 (m, 1H), 4.87 (s, 2H), 3.87-3.80 (m, 2H), 2.36 (s, 3H), 2.30 (s, 3H), 1.84-1.69 (m, 11H), 1.05-0.97 (m, 3H).
1-240(#2): δ9.01 (br, 1H), 7.46-7.31 (m, 2H), 7.27-7.22 (m, 1H), 7.16-7.06(m, 3H), 6.71-6.67 (m, 1H), 6.58-6.52 (m, 1H), 4.87 (s, 2H), 3.88-3.81 (m, 2H), 2.35 (s, 3H), 1.86-1.62 (m, 11H), 1.06-0.98 (m, 3H).
2-002(#1): δ8.86 (br, 1H), 7.62-7.24 (m, 7H), 7.20 (s, 1H), 7.16-7.06 (m, 2H), 4.88 (s, 2H), 3.23 (q, J = 10.5 Hz, 2H), 1.72 (s, 9H).
2-003(#2): δ8.82 (br, 1H), 7.46-7.33 (m, 2H), 7.32-7.17 (m, 5H), 7.16-7.08 (m, 2H), 6.61 (br, 1H), 4.88 (s, 2H), 3.78 (s, 3H), 1.72 (s, 9H).
2-006(#2): δ8.82 (br, 1H), 8.23 (br, 1H), 7.54-7.48 (m, 2H), 7.47-7.41 (m, 1H), 7.40-7.35 (m, 1H), 7.34-7.29 (m, 2H), 7.24-7.19 (m, 1H), 7.17-7.08 (m, 2H), 4.90 (s, 2H), 4.01 (s, 2H), 3.52 (s, 3H), 1.73 (s, 9H).
2-008(#1): δ9.82 (br, 1H), 8.91 (br, 1H), 8.33-8.21 (m, 1H), 7.64-7.56 (m, 2H), 7.56-7.47 (m, 1H), 7.45-7.36 (m, 1H), 7.34-7.28 (m, 3H), 7.23-7.19 (m, 1H), 7.18-7.02 (m, 4H), 4.91 (s, 2H), 4.07 (s, 3H), 1.73 (s, 9H).
2-011(#2): δ8.92-8.84 (m, 1H), 7.59-7.51 (m, 4H), 7.49-7.31 (m, 7H), 7.28-7.25 (m, 1H), 7.15 (s, 1H), 7.13-7.07 (m, 1H), 4.97 (s, 2H), 1.73 (s, 9H).
2-013(#1): δ8.90 (s, 1H), 8.05-8.00 (m, 2H), 7.54-7.47 (m, 2H), 7.45-7.31 (m, 2H), 7.23-7.17 (m, 1H), 7.13 (s, 1H), 7.12-7.07 (m, 1H), 5.01 (s, 2H), 1.73 (s, 9H).
2-014(#2): δ8.88 (s, 1H), 8.68-8.59 (m, 1H), 8.57-8.46 (m, 1H), 8.03-7.91 (m, 1H), 7.83-7.74 (m, 2H), 7.53-7.31 (m, 5H), 7.24 (s, 1H), 7.16 (s, 1H), 7.14-7.10 (m, 1H), 4.99 (s, 2H), 1.73 (s, 9H).
2-015(#2): δ8.90 (s, 1H), 8.39-8.34 (m, 1H), 7.82-7.72 (m, 2H), 7.52-7.33 (m, 4H), 7.21-7.14 (m, 2H), 7.13-7.08 (m, 1H), 6.86-6.81 (m, 1H), 6.68-6.64 (m, 1H), 4.97 (s, 2H), 3.99 (s, 3H), 1.73 (s, 9H).
2-018(#1): δ8.87 (s, 1H), 8.55 (s, 1H), 7.68-7.62 (m, 2H), 7.48-7.35 (m, 4H), 7.24-7.20 (m, 1H), 7.15 (s, 1H), 7.12-7.07 (m, 1H), 4.97 (s, 2H), 3.89 (s, 3H), 1.72 (s, 9H).
2-019(#1): δ8.94 (s, 1H), 7.73-7.66 (m, 2H), 7.47-7.32 (m, 4H), 7.24-7.20 (m, 1H), 7.14 (s, 1H), 7.12-7.06 (m, 1H), 6.19-6.06 (m, 1H), 6.02-5.86 (m, 1H), 5.32-5.16 (m, 2H), 4.96 (s, 2H), 4.12-4.04 (m, 2H), 1.71 (s, 9H).
2-022(#1): δ9.82-9.61 (m, 1H), 8.55-8.49 (m, 1H), 8.30-8.24 (m, 1H), 8.23-8.15 (m, 1H), 8.07-7.95 (m, 1H), 7.82-7.74 (m, 2H), 7.51-7.18 (m, 6H), 7.16-7.04 (m, 2H), 5.00 (s, 2H), 1.72 (s, 9H).
2-025(#2): δ8.85 (s, 1H), 7.55-7.50 (m, 2H), 7.45-7.43 (m, 1H), 7.42-7.33 (m, 6H), 7.26-7.23 (m, 1H), 7.15 (s, 1H), 7.10-7.06 (m, 1H), 4.95 (s, 2H), 1.73 (s, 9H).
2-028(#1): δ9.07-8.93 (m, 1H), 7.73-7.61 (m, 1H), 7.48-7.39 (m, 1H), 7.36-7.21 (m, 5H), 7.15-7.04 (m, 2H), 7.00-6.90 (m, 2H), 6.90-6.80 (m, 2H), 5.12 (s, 2H), 4.84 (s, 2H), 1.72 (s, 9H).
2-029(#1): δ9.10-8.97 (m, 1H), 7.38-7.28 (m, 2H), 7.24-7.13 (m, 1H), 7.10 (s, 1H), 6.98-6.87 (m, 1H), 6.87-6.70 (m, 4H), 4.88 (s, 2H), 4.35-4.21 (m, 2H), 2.32 (s, 3H), 1.72 (s, 9H).
2-030(#1): δ9.16-9.02 (m, 1H), 8.46-8.38 (m, 1H), 7.78-7.68 (m, 1H), 7.42-7.08 (m, 5H), 7.01-6.70 (m, 5H), 5.00 (s, 2H), 4.87 (s, 2H), 2.31 (s, 3H), 1.72 (s, 9H).
2-031(#1): δ9.13-8.99 (m, 1H), 7.73-7.61 (m, 1H), 7.47-7.39 (m, 1H), 7.34-7.08 (m, 5H), 7.00-6.70 (m, 5H), 5.12 (s, 2H), 4.85 (s, 2H), 2.31 (s, 3H), 1.72 (s, 9H).
2-032(#1): δ8.96-8.81 (m, 1H), 7.51-7.21 (m, 5H), 7.19-7.06 (m, 2H), 6.91-6.78 (m, 2H), 4.87 (s, 2H), 4.38-4.24 (m, 2H), 1.72 (s, 9H).
2-033(#1): δ9.04-8.87 (m, 1H), 8.45-8.36 (m, 1H), 7.77-7.68 (m, 1H), 7.50-7.07 (m, 8H), 6.91-6.79 (m, 2H), 5.00 (s, 2H), 4.86 (s, 2H), 1.72 (s, 9H).
2-035(#1): δ8.99 (br, 1H), 7.39-7.23 (m, 4H), 7.15-7.07 (m, 2H), 6.99-6.92 (m, 2H), 6.83-6.76 (m, 2H), 6.29-5.82 (m, 1H), 4.86 (s, 2H), 4.18-4.04 (m, 2H), 1.72 (s, 9H).
2-036(#1): δ9.03 (br, 1H), 7.34-7.27 (m, 2H), 7.22-7.14 (m, 1H), 7.10 (s, 1H), 6.96-6.89 (m, 1H), 6.83-6.71 (m, 4H), 6.28-5.85 (m, 1H), 4.87 (s, 2H), 4.18-4.05 (m, 2H), 2.32 (s, 3H), 1.72 (s, 9H).
2-037(#1): δ8.85 (br, 1H), 7.48-7.34 (m, 2H), 7.32-7.20 (m, 3H), 7.17-7.07 (m, 2H), 6.84-6.78 (m, 2H), 6.29-5.84 (m, 1H), 4.86 (s, 2H), 4.19-4.06 (m, 2H), 1.72 (s, 9H).
2-038(#1): δ8.90 (br, 1H), 7.34-7.18 (m, 3H), 7.14 (s, 1H), 7.12-7.05 (m, 1H), 6.97-6.92 (m, 1H), 6.85-6.78 (m, 3H), 6.30-5.85 (m, 1H), 4.86 (s, 2H), 4.21-4.06 (m, 2H), 1.71 (s, 9H).
2-040(#1): δ8.91-8.77 (m, 1H), 8.48-8.40 (m, 1H), 7.78-7.69 (m, 1H), 7.43-6.78 (m, 10H), 5.02 (s, 2H), 4.86 (s, 2H), 1.72 (s, 9H).
2-041(#1): δ9.04-8.93 (m, 1H), 7.35-7.05 (m, 5H), 6.97-6.91 (m, 1H), 6.89-6.78 (m, 3H), 4.87 (s, 2H), 4.41-4.25 (m, 2H), 1.74-1.69 (m, 9H).
2-042(#1): δ8.96 (br, 1H), 7.38-7.27 (m, 4H), 7.16-7.08 (m, 2H), 6.99-6.92 (m, 2H), 6.83-6.77 (m, 2H), 6.27-5.81 (m, 1H), 4.87 (s, 2H), 4.35-4.22 (m, 2H), 1.72 (s, 9H).
2-043(#1): δ8.99 (br, 1H), 7.36-7.29 (m, 2H), 7.23-7.15 (m, 1H), 7.10 (s, 1H), 6.95-6.89 (m, 1H), 6.85-6.71 (m, 4H), 6.28-5.82 (m, 1H), 4.88 (s, 2H), 4.35-4.22 (m, 2H), 2.32 (s, 3H), 1.72 (s, 9H).
2-044(#1): δ8.77 (br, 1H), 7.48-7.21 (m, 5H), 7.17-7.07 (m, 2H), 6.86-6.78 (m, 2H), 6.25-5.83 (m, 1H), 4.87 (s, 2H), 4.35-4.24 (m, 2H), 1.72 (s, 9H).
2-045(#1): δ8.79 (br, 1H), 7.35-7.27 (m, 2H), 7.26-7.19 (m, 1H), 7.15 (s, 1H), 7.12-7.06 (m, 1H), 6.97-6.92 (m, 1H), 6.87-6.78 (m, 3H), 6.27-5.82 (m, 1H), 4.87 (s, 2H), 4.37-4.24 (m, 2H), 1.72 (s, 9H).
2-048(#2): δ8.98 (s, 1H), 8.14-8.07 (m, 2H), 7.62-7.55 (m, 2H), 7.46-7.33 (m, 2H), 7.23-7.19 (m, 1H), 7.14 (s, 1H), 7.13-7.09 (m, 1H), 5.04 (s, 2H), 1.73 (s, 9H).
2-049(#1): δ9.05 (s, 1H), 7.96-7.89 (m, 2H), 7.52-7.45 (m, 2H), 7.44-7.30 (m, 2H), 7.23-7.18 (m, 1H), 7.14 (s, 1H), 7.12-7.06 (m, 1H), 5.00 (s, 2H), 2.62 (s, 3H), 1.72 (s, 9H).
2-050(#1): δ8.95 (br, 1H), 7.97 (d, J = 8.1 Hz, 2H), 7.50-7.31 (m, 4H), 7.20 (s, 1H), 7.13 (s, 1H), 7.08 (d, J = 7.8 Hz, 1H), 4.98 (s, 2H), 2.65 (s, 3H), 1.72 (s, 9H).
2-051(#1): δ8.77 (br, 1H), 7.62-7.53 (m, 2H), 7.46-7.29 (m, 4H), 7.24-7.21 (m, 1H), 7.17-7.12 (m, 1H), 7.12-7.05 (m, 1H), 4.98-4.87 (m, 2H), 4.00&3.84 (s, 3H), 2.23-2.14 (m, 3H), 1.72 (s, 9H).
2-052(#1): δ8.94 (br, 1H), 7.95 (d, J = 7.8 Hz, 2H), 7.46-7.31 (m, 4H), 7.20 (s, 1H), 7.13 (s, 1H), 7.07 (d, J = 7.8 Hz, 1H), 4.98 (s, 2H), 2.31-2.21 (m, 1H), 1.72 (s, 9H), 1.36-1.20 (m, 4H).
2-053(#1): δ8.97 (br, 1H), 8.02 (d, J = 8.1 Hz, 2H), 7.49 (d, J = 8.1 Hz, 2H), 7.44-7.28 (m, 2H), 7.20 (s, 1H), 7.16-7.06 (m, 2H), 6.87 (t, J = 52.5 Hz, 1H), 5.00 (s, 2H), 1.72 (s, 9H). 2-057(#1): δ9.12 (br, 1H), 8.25-8.19 (m, 2H), 8.13-8.07 (m, 2H), 7.66-7.43 (m, 5H), 7.17-7.10 (m, 2H), 6.88 (d, J = 7.2 Hz, 1H), 6.75-6.72 (m, 2H), 5.01 (s, 2H), 2.29 (s, 3H), 1.73 (s, 9H).
2-059(#1): δ8.91 (br, 1H), 7.99 (d, J = 7.8 Hz, 2H), 7.45-7.34 (m, 4H), 7.20 (s, 1H), 7.13 (s, 1H), 7.10-7.05 (m, 1H), 4.99 (s, 2H), 3.34-3.24 (sep, 1H), 1.72 (s, 9H), 1.47 (d, J = 6.8 Hz, 6H).
2-062(#1): δ8.81 (s, 1H), 7.50-7.27 (m, 7H), 7.19-7.08 (m, 3H), 7.02-6.95 (m, 2H), 6.94-6.87 (m, 2H), 4.89 (s, 2H), 1.73 (s, 9H).
2-063(#2): δ9.24 (s, 1H), 7.95-7.87 (m, 2H), 7.47-7.29 (m, 5H), 7.22-7.17 (m, 1H), 7.15-7.10 (m, 1H), 7.09-7.03 (m, 1H), 4.97 (s, 2H), 2.26-2.21 (m, 3H), 1.72 (s, 9H).
2-065(#1): δ8.74 (br, 1H), 7.49-7.04 (m, 12H), 6.93-6.83 (m, 2H), 5.02 (s, 2H), 4.85 (s, 2H), 1.72 (s, 9H).
2-066(#1): δ9.05-8.89 (m, 1H), 7.98-7.83 (m, 2H), 7.55-7.03 (m, 7H), 5.00 (s, 2H), 2.33-2.16 (m, 1H), 1.74 (s, 9H), 1.35-1.16 (m, 4H).
2-067(#1): δ9.01-8.84 (m, 1H), 8.07-7.97 (m, 2H), 7.61-6.71 (m, 8H), 5.02 (s, 2H), 1.73 (s, 9H).
2-070(#2): δ9.27-9.16 (m, 1H), 8.05-7.97 (m, 2H), 7.56-7.47 (m, 2H), 7.21-7.07 (m, 2H), 6.94-6.85 (m, 1H), 6.78-6.67 (m, 2H), 5.02 (s, 2H), 2.48 (s, 3H), 2.29 (s, 3H), 1.72 (s, 9H).
2-071(#1): δ9.09-8.90 (m, 1H), 8.05-7.94 (m, 2H), 7.55-7.03 (m, 7H), 5.00 (s, 2H), 2.94 (t, J = 7.5 Hz, 2H), 2.02-1.86 (m, 2H), 1.73 (s, 9H), 0.99 (t, J = 7.3 Hz, 3H).
2-073(#1): δ8.08-7.94 (m, 2H), 7.53-6.98 (m, 7H), 4.98 (s, 2H), 2.84 (d, J = 7.2 Hz, 2H), 2.31-2.02 (m, 1H), 1.72 (s, 9H), 0.98 (d, J = 6.5 Hz, 6H). An NH proton was not observed.
2-074(#1): δ9.13-8.83 (m, 1H), 8.03-7.93 (m, 2H), 7.55-7.00 (m, 7H), 5.00 (s, 2H), 3.95-3.79 (m, 2H), 1.72 (s, 9H).
2-075(#1): δ9.07-8.90 (m, 1H), 8.05-7.97 (m, 2H), 7.56-7.00 (m, 7H), 5.01 (s, 2H), 1.72 (s, 9H).
2-076(#1): δ9.17-9.04 (m, 1H), 8.03-7.94 (m, 2H), 7.51-7.41 (m, 2H), 7.35-7.23 (m, 2H), 7.15-7.03 (m, 2H), 6.97-6.87 (m, 2H), 4.97 (s, 2H), 2.66 (s, 3H), 1.72 (s, 9H).
2-079(#1): δ9.20-9.08 (m, 1H), 8.06-7.94 (m, 2H), 7.57-7.43 (m, 2H), 7.34-7.19 (m, 2H), 7.15-7.01 (m, 2H), 6.97-6.83 (m, 2H), 5.00 (s, 2H), 2.48 (s, 3H), 1.72 (s, 9H).
2-080(#1): δ9.23-9.09 (m, 1H), 8.02-7.89 (m, 2H), 7.54-7.44 (m, 2H), 7.33-7.22 (m, 2H), 7.14-7.02 (m, 2H), 6.98-6.86 (m, 2H), 4.98 (s, 2H), 2.62 (s, 3H), 1.72 (s, 9H).
2-081(#1): δ9.18-9.08 (m, 1H), 7.99-7.90 (m, 2H), 7.54-7.45 (m, 2H), 7.21-7.07 (m, 2H), 6.92-6.84 (m, 1H), 6.82-6.68 (m, 2H), 5.00 (s, 2H), 2.97 (q, J = 7.7 Hz, 2H), 2.29 (s, 3H), 1.73 (s, 9H), 1.45 (t, J = 7.7 Hz, 3H).
2-082(#1): δ9.22-9.08 (m, 1H), 7.99-7.90 (m, 2H), 7.53-7.45 (m, 2H), 7.21-7.07 (m, 2H), 6.95-6.85 (m, 1H), 6.76-6.65 (m, 2H), 5.00 (s, 2H), 2.91 (t, J = 7.5 Hz, 2H), 2.29 (s, 3H), 1.99-1.81 (m, 2H), 1.72 (s, 9H), 1.08 (t, J = 7.3 Hz, 3H).
2-083(#1): δ9.23-9.13 (m, 1H), 8.03-7.93 (m, 2H), 7.57-7.46 (m, 2H), 7.21-7.07 (m, 2H), 6.93-6.84 (m, 1H), 6.79-6.68 (m, 2H), 5.01 (s, 2H), 4.72 (s, 2H), 3.51 (s, 3H), 2.29 (s, 3H), 1.73 (s, 9H).
2-084(#1): δ9.23-9.08 (m, 1H), 7.95-7.84 (m, 2H), 7.52-7.43 (m, 2H), 7.21-7.06 (m, 2H), 6.95-6.85 (m, 1H), 6.79-6.68 (m, 2H), 4.99 (s, 2H), 2.29 (s, 3H), 2.28-2.16 (m, 1H), 1.73 (s, 9H), 1.26-1.14 (m, 4H).
2-086(#1): δ9.20-9.10 (m, 1H), 8.06-7.96 (m, 2H), 7.55-7.45 (m, 2H), 7.19-7.06 (m, 2H), 6.92-6.83 (m, 1H), 6.78-6.67 (m, 2H), 5.01 (s, 2H), 2.78 (t, J = 7.5 Hz, 2H), 2.28 (s, 3H), 1.93-1.78 (m, 2H), 1.73 (s, 9H), 1.04 (t, J = 7.5 Hz, 3H).
2-087(#1): δ9.21-9.07 (m, 1H), 8.03-7.93 (m, 2H), 7.54-7.43 (m, 2H), 7.20-7.06 (m, 2H), 6.92-6.84 (m, 1H), 6.79-6.67 (m, 2H), 5.00 (s, 2H), 2.28 (s, 3H), 2.23-2.08 (m, 1H), 1.73 (s, 9H), 1.18-1.04 (m, 4H).
2-088(#1): δ9.23-9.13 (m, 1H), 8.05-7.96 (m, 2H), 7.59-7.51 (m, 2H), 7.21-7.10 (m, 1H), 7.08 (s, 1H), 6.91-6.84 (m, 1H), 6.77-6.67 (m, 2H), 5.04 (s, 2H), 2.29 (s, 3H), 1.73 (s, 9H).
2-089(#1): δ9.23-9.12 (m, 1H), 8.04-7.96 (m, 2H), 7.59-7.50 (m, 2H), 7.19-6.67 (m, 6H), 5.03 (s, 2H), 2.28 (s, 3H), 1.73 (s, 9H).
2-090(#1): δ9.21-9.12 (m, 1H), 7.97-7.90 (m, 2H), 7.54-7.44 (m, 2H), 7.21-7.07 (m, 2H), 6.94-6.85 (m, 1H), 6.80-6.68 (m, 2H), 5.00 (s, 2H), 2.63 (s, 3H), 2.29 (s, 3H), 1.73 (s, 9H).
2-092(#1): δ9.15-9.02 (m, 1H), 8.05-7.96 (m, 2H), 7.53-7.42 (m, 2H), 7.21-7.07 (m, 2H), 6.93-6.84 (m, 1H), 6.79-6.68 (m, 2H), 4.99 (s, 2H), 2.93 (t, J = 7.5 Hz, 2H), 2.29 (s, 3H), 2.01-1.84 (m, 2H), 1.73 (s, 9H), 1.07 (t, J = 7.5 Hz, 3H).
2-093(#1): δ9.15-9.06 (m, 1H), 8.08-7.99 (m, 2H), 7.53-7.44 (m, 2H), 7.21-7.08 (m, 2H), 6.94-6.85 (m, 1H), 6.80-6.68 (m, 2H), 4.99 (s, 2H), 4.76 (s, 2H), 3.57 (s, 3H), 2.29 (s, 3H), 1.73 (s, 9H).
2-094(#1): δ9.13-9.04 (m, 1H), 8.04-7.95 (m, 2H), 7.51-7.42 (m, 2H), 7.21-7.08 (m, 2H), 6.94-6.84 (m, 1H), 6.78-6.68 (m, 2H), 4.99 (s, 2H), 2.84 (d, J = 7.2 Hz, 2H), 2.32-2.24 (m, 4H), 1.73 (s, 9H), 1.06 (d, J = 6.8 Hz, 6H).
2-095(#1): δ9.17-9.05 (m, 1H), 8.04-7.96 (m, 2H), 7.53-7.44 (m, 2H), 7.18-7.07 (m, 2H), 6.94-6.84 (m, 1H), 6.78-6.67 (m, 2H), 5.00 (s, 2H), 3.97-3.79 (m, 2H), 2.28 (s, 3H), 1.73 (s, 9H).
2-096(#1): δ9.15-9.06 (m, 1H), 8.07-7.98 (m, 2H), 7.55-7.45 (m, 2H), 7.21-7.06 (m, 2H), 6.92-6.84 (m, 1H), 6.79-6.67 (m, 2H), 5.02 (s, 2H), 2.29 (s, 3H), 1.73 (s, 9H).
2-097(#1): δ9.17-9.06 (m, 1H), 8.07-7.99 (m, 2H), 7.57-7.46 (m, 2H), 7.20-7.07 (m, 2H), 6.94-6.83 (m, 1H), 6.79-6.68 (m, 2H), 5.02 (s, 2H), 2.29 (s, 3H), 1.73 (s, 9H).
2-098(#1): δ9.06-8.93 (m, 1H), 8.09-7.97 (m, 2H), 7.58-7.28 (m, 4H), 7.25-7.03 (m, 3H), 5.01 (s, 2H), 2.78 (t, J = 7.5 Hz, 2H), 1.95-1.75 (m, 2H), 1.72 (s, 9H), 1.04 (t, J = 7.5 Hz, 3H). 2-099(#1): δ9.07-8.92 (m, 1H), 8.03-7.92 (m, 2H), 7.54-7.29 (m, 4H), 7.25-7.02 (m, 3H), 5.00 (s, 2H), 2.21-2.08 (m, 1H), 1.72 (s, 9H), 1.19-1.05 (m, 4H).
2-100(#1): δ8.75 (br, 1H), 7.52-7.30 (m, 3H), 7.25-7.07 (m, 4H), 6.85-6.74 (m, 2H), 4.84 (s, 2H), 3.79-3.68 (m, 2H), 1.73 (s, 9H), 1.29-1.17 (m, 1H), 0.72-0.57 (m, 2H), 0.39-0.22 (m, 2H).
2-103(#1): δ8.95 (br, 1H), 7.37-7.23 (m, 4H), 7.18-7.10 (m, 2H), 7.02-6.90 (m, 2H), 6.86-6.76 (m, 2H), 4.87 (s, 2H), 3.81-3.70 (m, 2H), 1.75 (s, 9H), 1.31-1.21 (m, 1H), 0.71-0.59 (m, 2H), 0.41-0.29 (m, 2H).
2-104(#1): δ9.02-8.95 (br, 1H), 8.57-8.52 (m, 1H), 7.72-7.66 (m, 1H), 7.50-7.45 (m, 1H), 7.35-7.28 (m, 4H), 7.17-7.07 (m, 2H), 6.99-6.92 (m, 2H), 6.89-6.83 (m, 2H), 5.13 (s, 2H), 4.85 (s, 2H), 1.74 (s, 9H).
2-105(#1): δ8.96 (br, 1H), 7.64-7.54 (m, 1H), 7.38-7.25 (m, 5H), 7.18-7.04 (m, 3H), 6.99-6.82 (m, 4H), 5.14 (s, 2H), 4.85 (s, 2H), 2.61 (s, 3H), 1.75 (s, 9H).
2-106(#1): δ9.08-8.93 (m, 1H), 7.97-7.89 (m, 2H), 7.52-7.28 (m, 4H), 7.23-7.05 (m, 3H), 5.00 (s, 2H), 2.96 (q, J = 7.6 Hz, 2H), 1.72 (s, 9H), 1.45 (t, J = 7.6 Hz, 3H).
2-107(#1): δ9.06-8.91 (m, 1H), 7.98-7.90 (m, 2H), 7.54-7.29 (m, 4H), 7.24-7.04 (m, 3H), 4.99 (s, 2H), 2.91 (t, J = 7.5 Hz, 2H), 1.99-1.80 (m, 2H), 1.72 (s, 9H), 1.08 (t, J = 7.3 Hz, 3H). 2-108(#1): δ9.00-8.87 (m, 1H), 8.03-7.92 (m, 2H), 7.56-7.46 (m, 2H), 7.45-7.29 (m, 2H), 7.23-7.03 (m, 3H), 5.00 (s, 2H), 4.72 (s, 2H), 3.51 (s, 3H), 1.73 (s, 9H).
2-109(#1): δ9.01-8.87 (m, 1H), 7.99-7.89 (m, 2H), 7.54-7.00 (m, 7H), 5.00 (s, 2H), 2.82 (d, J = 7.0 Hz, 2H), 2.32-2.15 (m, 1H), 1.72 (s, 9H), 1.06 (d, J = 7.0 Hz, 6H).
2-110(#1): δ8.99-8.87 (m, 1H), 8.05-7.98 (m, 2H), 7.60-7.50 (m, 2H), 7.49-7.30 (m, 2H), 7.22-7.04 (m, 3H), 5.03 (s, 2H), 1.73 (s, 9H).
2-111(#1): δ9.00 (br, 1H), 8.58-8.53 (m, 1H), 7.73-7.64 (m, 1H), 7.49-7.42 (m, 1H), 7.32-7.24 (m, 2H), 7.22-7.08 (m, 2H), 6.95-6.71 (m, 5H), 5.13 (s, 2H), 4.86 (s, 2H), 2.31 (s, 3H), 1.72 (s, 9H).
2-112(#1): δ8.97 (br, 1H), 7.63-7.54 (m, 1H), 7.36-7.23 (m, 3H), 7.21-7.02 (m, 3H), 6.95-6.70 (m, 5H), 5.13 (s, 2H), 4.86 (s, 2H), 2.59 (s, 3H), 2.33 (s, 3H), 1.71 (s, 9H).
2-113(#2): δ8.73 (br, 1H), 7.48-7.34 (m, 2H), 7.30-7.22 (m, 3H), 7.18-7.08(m, 2H), 6.85-6.78 (m, 2H), 6.12-5.96 (m, 1H), 5.45-5.26 (m, 2H), 4.86 (s, 2H), 4.52-4.47 (m, 2H), 1.73 (s, 9H).
2-114(#2): δ8.74 (br, 1H), 7.48-7.35 (m, 2H), 7.33-7.23 (m, 3H), 7.18-7.08(m, 2H), 6.92-6.86 (m, 2H), 4.86 (s, 2H), 4.68-4.65 (m, 2H), 2.53-2.50 (m, 1H), 1.73 (s, 9H).
2-115(#1): δ9.10-8.93 (m, 1H), 8.04-7.93 (m, 2H), 7.52-7.02 (m, 7H), 4.98 (s, 2H), 2.95 (t, J = 7.5 Hz, 2H), 1.98-1.79 (m, 2H), 1.72 (s, 9H), 1.54-1.37 (m, 2H), 0.98 (t, J = 7.3 Hz, 3H). 2-116(#1): δ9.11-8.86 (m, 1H), 8.03-7.93 (m, 2H), 7.50-7.03 (m, 7H), 4.99 (s, 2H), 1.72 (s, 9H), 1.50 (s, 9H).
2-117(#1): δ9.19-9.05 (m, 1H), 8.05-7.95 (m, 2H), 7.51-7.42 (m, 2H), 7.21-7.08 (m, 2H), 6.95-6.84 (m, 1H), 6.77-6.66 (m, 2H), 4.99 (s, 2H), 2.95 (t, J = 7.7 Hz, 2H), 2.29 (s, 3H), 1.94-1.79 (m, 2H), 1.73 (s, 9H), 1.56-1.40 (m, 2H), 0.99 (t, J = 7.3 Hz, 3H).
2-118(#1): δ9.28-9.02 (m, 1H), 8.05-7.96 (m, 2H), 7.50-7.39 (m, 2H), 7.21-7.08 (m, 2H), 6.93-6.83 (m, 1H), 6.80-6.66 (m, 2H), 4.99 (s, 2H), 2.28 (s, 3H), 1.73 (s, 9H), 1.50 (s, 9H).
2-119(#1): δ9.18-9.04 (m, 1H), 8.76 (s, 1H), 8.08-7.99 (m, 2H), 7.52-7.44 (m, 2H), 7.35-7.22 (m, 2H), 7.14-7.03 (m, 2H), 6.97-6.88 (m, 2H), 4.99 (s, 2H), 1.72 (s, 9H).
2-120(#1): δ9.01-8.86 (m, 1H), 8.76 (s, 1H), 8.08-7.98 (m, 2H), 7.54-6.96 (m, 7H), 5.00 (s, 2H), 1.72 (s, 9H).
2-121(#1): δ9.25-9.10 (m, 1H), 8.76 (s, 1H), 8.09-7.98 (m, 2H), 7.53-7.43 (m, 2H), 7.21-7.06 (m, 2H), 6.93-6.83 (m, 1H), 6.79-6.67 (m, 2H), 5.00 (s, 2H), 2.28 (s, 3H), 1.73 (s, 9H).
2-122(#2): δ9.13 (br, 1H), 7.67-7.60 (m, 2H), 7.51-7.44 (m, 2H), 7.24-7.17 (m, 1H), 7.11 (s, 1H), 6.98-6.92 (m, 1H), 6.81-6.72 (m, 2H), 4.98 (s, 2H), 4.43-4.32 (m, 1H), 2.33 (s, 3H), 2.29 (br, 1H), 1.72 (s, 9H), 1.61-1.56 (m, 3H).
2-123(#2): δ.13 (br, 1H), 8.04-7.98 (m, 2H), 7.51-7.44 (m, 2H), 7.20-7.09 (m, 1H), 7.10 (s, 1H), 6.92-6.86 (m, 1H), 6.75-6.70 (m, 2H), 5.28-5.19 (m, 1H), 5.00 (s, 2H), 2.29 (s, 3H), 2.05-2.00 (m, 3H), 1.73 (s, 9H).
2-124(#1): δ9.01 (br, 1H), 7.87-7.74 (m, 2H), 7.58-7.30 (m, 4H), 7.24-6.98 (m, 3H), 6.93-6.84 (m, 1H), 4.94 (s, 2H), 2.49 (s, 3H), 1.70 (s, 9H).
2-125(#2): δ9.12 (br, 1H), 7.59-7.44 (m, 4H), 7.23-7.15 (m, 1H), 7.11 (s, 1H), 7.00-6.94 (m, 1H), 6.78-6.70 (m, 2H), 5.00 (s, 2H), 4.77-4.67 (m, 1H), 3.48 (s, 3H), 2.29 (s, 3H), 1.73 (s, 9H), 1.70-1.66 (m, 3H).
2-126(#1): δ9.21-9.06 (m, 1H), 8.04-7.92 (m, 2H), 7.52-7.40 (m, 2H), 7.35-7.20 (m, 2H), 7.15-7.00 (m, 2H), 6.99-6.84 (m, 2H), 4.97 (s, 2H), 2.93 (t, J = 7.5 Hz, 2H), 1.99-1.85 (m, 2H), 1.72 (s, 9H), 1.07 (t, J = 7.3 Hz, 3H).
2-127(#1): δ9.28-9.05 (m, 1H), 8.07-7.93 (m, 2H), 7.52-7.37 (m, 2H), 7.35-7.19 (m, 2H), 7.17-7.00 (m, 2H), 6.98-6.86 (m, 2H), 4.97 (s, 2H), 3.38-3.20 (m, 1H), 1.72 (s, 9H), 1.47 (d, J = 6.8 Hz, 6H).
2-128(#1): δ9.21-9.03 (m, 1H), 8.06-7.94 (m, 2H), 7.51-7.40 (m, 2H), 7.34-7.21 (m, 2H), 7.15-7.01 (m, 2H), 6.98-6.86 (m, 2H), 4.97 (s, 2H), 2.95 (t, J = 7.5 Hz, 2H), 1.95-1.78 (m, 2H), 1.72 (s, 9H), 1.56-1.39 (m, 2H), 0.99 (t, J = 7.3 Hz, 3H).
2-129(#1): δ9.26-9.07 (m, 1H), 8.09-7.96 (m, 2H), 7.53-7.39 (m, 2H), 7.35-7.21 (m, 2H), 7.15-7.02 (m, 2H), 6.98-6.87 (m, 2H), 4.98 (s, 2H), 4.75 (s, 2H), 3.57 (s, 3H), 1.72 (s, 9H).
2-130(#2): δ9.14 (br, 1H), 8.91-8.87 (m, 1H), 8.35-8.29 (m, 1H), 8.19-8.13(m, 2H), 8.00-7.92 (m, 1H), 7.59-7.48 (m, 3H) 7.20-7.15 (m, 1H), 7.11 (s, 1H), 6.92-6.86 (m, 1H), 6.75-6.70 (m, 2H), 5.02 (s, 2H), 2.29 (s, 3H), 1.73 (s, 9H).
2-131(#2): δ9.14 (br, 1H), 8.03-7.97 (m, 2H), 7.49-7.43 (m, 2H), 7.20-7.15 (m, 1H), 7.11 (s, 1H), 6.92-6.86 (m, 1H), 6.75-6.70 (m, 2H), 4.99 (s, 2H), 3.08-2.95 (m, 1H), 2.29 (s, 3H), 2.20-2.10 (m, 2H), 1.95-1.80 (m, 2H), 1.73 (s, 9H), 1.67-1.22 (m, 6H).
2-132(#2): δ9.12 (br, 1H), 8.03-7.97 (m, 2H), 7.50-7.44 (m, 2H), 7.20-7.15 (m, 1H), 7.11 (s, 1H), 6.92-6.86 (m, 1H), 6.77-6.71 (m, 2H), 5.00 (s, 2H), 4.12-4.03 (m, 2H), 3.65-3.53 (m, 2H), 3.34-3.20 (m, 1H), 2.29 (s, 3H), 2.13-2.01 (m, 4H), 1.73 (s, 9H).
2-133(#2): δ9.10 (br, 1H), 8.03-7.97 (m, 2H), 7.48-7.42 (m, 2H), 7.20-7.15 (m, 1H), 7.11 (s, 1H), 6.92-6.86 (m, 1H), 6.75-6.70 (m, 2H), 4.99 (s, 2H), 3.48-3.34 (m, 1H), 2.29 (s, 3H), 2.25-1.22 (m, 17H).
2-134(#2): δ9.11 (br, 1H), 8.04-7.99 (m, 2H), 7.49-7.43 (m, 2H), 7.19-7.12 (m, 1H), 7.11 (s, 1H), 6.92-6.86 (m, 1H), 6.75-6.70 (m, 2H), 5.00 (s, 2H), 2.86 (s, 2H), 2.29 (s, 3H), 1.73 (s, 9H), 1.10 (s, 9H).
2-135(#2): δ9.10 (br, 1H), 8.03-7.98 (m, 2H), 7.49-7.44 (m, 2H), 7.20-7.12 (m, 1H), 7.11 (s, 1H), 6.92-6.86 (m, 1H), 6.76-6.70 (m, 2H), 4.99 (s, 2H), 2.99-2.91 (m, 2H), 2.29 (s, 3H), 1.95-1.83 (m, 2H), 1.73 (s, 9H), 1.60-1.30 (m, 4H), 0.96-0.88 (m, 3H).
2-136(#2): δ9.10 (br, 1H), 8.03-7.98 (m, 2H), 7.49-7.44 (m, 2H) 7.20-7.12 (m, 1H), 7.11 (s, 1H), 6.92-6.86 (m, 1H), 6.76-6.70 (m, 2H), 4.99 (s, 2H), 2.99-2.91 (m, 2H), 2.29 (s, 3H), 1.95-1.83 (m, 2H), 1.73 (s, 9H), 1.50-1.28 (m, 6H), 0.94-0.87 (m, 3H).
2-137(#2): δ9.11 (br, 1H), 8.03-7.98 (m, 2H), 7.50-7.44 (m, 2H) 7.20-7.12 (m, 1H), 7.11 (s, 1H), 6.92-6.86 (m, 1H), 6.76-6.70 (m, 2H), 5.00 (s, 2H), 4.07 (s, 2H), 3.81 (s, 3H), 2.29 (s, 3H), 1.73 (s, 9H).
2-138(#2): δ9.13 (br, 1H), 8.11-8.05 (m, 2H), 7.54-7.48 (m, 2H) 7.20-7.12 (m, 1H), 7.11 (s, 1H), 6.92-6.86 (m, 1H), 6.78-6.70 (m, 2H), 5.01 (s, 2H), 4.13 (s, 3H), 2.30 (s, 3H), 1.73 (s, 9H).
2-139(#1): δ8.93-8.81 (m, 1H), 8.04-7.96 (m, 2H), 7.51-7.42 (m, 2H), 7.24-7.02 (m, 3H), 6.96-6.89 (m, 1H), 6.84-6.74 (m, 1H), 4.99 (s, 2H), 2.67 (s, 3H), 1.72 (s, 9H).
2-140(#1): δ8.96-8.84 (m, 1H), 8.08-7.99 (m, 2H), 7.55-7.44 (m, 2H), 7.24-7.10 (m, 2H), 7.08-6.66 (m, 4H), 5.01 (s, 2H), 1.72 (s, 9H).
2-141(#1): δ8.95-8.82 (m, 1H), 8.05-7.96 (m, 2H), 7.51-7.41 (m, 2H), 7.24-7.10 (m, 2H), 7.08-7.00 (m, 1H), 6.95-6.87 (m, 1H), 6.84-6.75 (m, 1H), 4.99 (s, 2H), 2.99 (q, J = 7.6 Hz, 2H), 1.72 (s, 9H), 1.47 (t, J = 7.6 Hz, 3H).
2-142(#1): δ8.95-8.83 (m, 1H), 8.02-7.93 (m, 2H), 7.48-7.39 (m, 2H), 7.24-7.11 (m, 2H), 7.09-7.01 (m, 1H), 6.96-6.89 (m, 1H), 6.83-6.74 (m, 1H), 4.98 (s, 2H), 2.34-2.20 (m, 1H), 1.72 (s, 9H), 1.38-1.20 (m, 4H).
2-143(#1): δ9.01-8.83 (m, 1H), 8.10-7.95 (m, 2H), 7.59-6.98 (m, 7H), 5.01 (s, 2H), 3.25-3.07 (m, 1H), 1.73 (s, 9H), 1.41 (d, J = 6.8 Hz, 6H).
2-144(#1): δ9.20-9.06 (m, 1H), 8.07-7.95 (m, 2H), 7.55-7.42 (m, 2H), 7.21-7.03 (m, 2H), 6.93-6.80 (m, 1H), 6.77-6.63 (m, 2H), 5.02 (s, 2H), 3.26-3.08 (m, 1H), 2.28 (s, 3H), 1.73 (s, 9H), 1.41 (d, J = 6.8 Hz, 6H).
2-145(#1): δ8.99-8.87 (m, 1H), 8.04-7.95 (m, 2H), 7.51-7.40 (m, 2H), 7.24-7.11 (m, 2H), 7.08-6.99 (m, 1H), 6.95-6.88 (m, 1H), 6.84-6.73 (m, 1H), 4.99 (s, 2H), 2.93 (t, J = 7.4 Hz, 2H), 2.00-1.84 (m, 2H), 1.72 (s, 9H), 1.07 (t, J = 7.4 Hz, 3H).
2-146(#1): δ9.06-8.91 (m, 1H), 8.09-7.98 (m, 2H), 7.54-7.42 (m, 2H), 7.26-7.10 (m, 2H), 7.08-6.99 (m, 1H), 6.94-6.88 (m, 1H), 6.84-6.75 (m, 1H), 4.99 (s, 2H), 4.76 (s, 2H), 3.57 (s, 3H), 1.72 (s, 9H).
2-147(#1): δ9.07-8.96 (m, 1H), 8.05-7.96 (m, 2H), 7.50-7.39 (m, 2H), 7.25-7.12 (m, 2H), 7.08-7.00 (m, 1H), 6.94-6.87 (m, 1H), 6.83-6.75 (m, 1H), 4.98 (s, 2H), 2.84 (d, J = 7.2 Hz, 2H), 2.37-2.21 (m, 1H), 1.72 (s, 9H), 1.06 (d, J = 6.5 Hz, 6H).
2-148(#1): δ8.99-8.89 (m, 1H), 8.09-7.99 (m, 2H), 7.54-7.46 (m, 2H), 7.24-7.12 (m, 2H), 7.10-7.01 (m, 1H), 6.93-6.88 (m, 1H), 6.84-6.76 (m, 1H), 5.01 (s, 2H), 1.72 (s, 9H).
2-149(#1): δ8.96-8.83 (m, 1H), 8.05-7.94 (m, 2H), 7.53-7.02 (m, 7H), 4.99 (s, 2H), 3.48-3.33 (m, 1H), 2.28-1.43 (m, 17H). 2-150(#2): δ8.96-8.85 (m, 1H), 8.05-7.96 (m, 2H), 7.51-7.04 (m, 7H), 4.99 (s, 2H), 3.09-2.95 (m, 1H), 2.22-2.09 (m, 2H), 1.96-1.30 (m, 17H).
2-151(#1): δ8.98-8.84 (m, 1H), 8.04-7.93 (m, 2H), 7.51-6.98 (m, 7H), 4.99 (s, 2H), 4.21-3.97 (m, 4H), 3.70-3.50 (m, 2H), 3.35-3.17 (m, 1H), 2.16-1.46 (m, 11H).
2-152(#1): δ8.93-8.81 (m, 1H), 8.06-7.93 (m, 2H), 7.56-7.00 (m, 7H), 4.99 (s, 2H), 2.86 (s, 2H), 1.87-1.42 (m, 18H).
2-153(#1): δ9.00-8.82 (m, 1H), 8.03-7.91 (m, 2H), 7.57-6.96 (m, 7H), 4.99 (s, 2H), 2.94 (t, J = 7.5 Hz, 2H), 2.06-1.09 (m, 15H), 0.99-0.78 (m, 3H).
2-154(#2): δ9.04-8.83 (m, 1H), 8.03-7.98 (m, 2H), 7.54-7.06 (m, 7H), 4.99 (s, 2H), 2.95 (t, J = 7.7 Hz, 3H), 1.97-1.28 (m, 16H), 0.98-0.86 (m, 3H).
2-155(#2): δ9.22 (br, 1H), 8.05-7.99 (m, 2H), 7.52-7.43 (m, 2H) 7.23-7.08 (m, 2H), 6.98-6.87 (m, 1H), 6.80-6.71 (m, 2H), 5.30-5.23 (m, 1H), 4.99 (s, 2H), 4.20-4.12 (m, 1H), 4.08-4.00 (m, 1H), 2.53-2.42 (m, 1H), 2.37-2.27 (m, 4H), 2.25-2.00 (m, 2H), 1.72 (s, 9H).
2-156(#2): δ9.09 (br, 1H), 8.03-7.97 (m, 2H), 7.50-7.30 (m, 4H) 7.25-6.98 (m, 3H), 5.28-5.22 (m, 1H), 4.98 (s, 2H), 4.19-3.98 (m, 2H), 2.50-2.05 (m, 4H), 1.72 (s, 9H).
2-157(#2): δ9.08 (br, 1H), 7.83-7.78 (m, 2H), 7.52-7.49 (m, 1H), 7.43-7.38(m, 2H), 7.19-7.09 (m, 2H), 6.92-6.86 (m, 1H), 6.77-6.70 (m, 2H), 4.96 (s, 2H), 2.54-2.51 (m, 3H), 2.28 (s, 3H), 1.73 (s, 9H).
2-158(#2): δ8.97 (br, 1H), 7.83-7.77 (m, 2H), 7.52-7.48 (m, 1H), 7.42-7.33(m, 4H), 7.24-7.20 (m, 1H), 7.15-7.12 (m, 1H), 7.10-7.00 (m, 1H), 4.94 (s, 2H), 2.52 (s, 3H), 1.72 (s, 9H).
3-001(#1): δ8.58 (br, 1H), 7.45-7.32 (m, 2H), 7.19-7.13 (m, 3H), 7.12-7.05 (m, 2H), 7.00-6.91 (m, 1H), 4.82 (s, 2H), 2.33 (s, 3H), 1.70 (s, 9H).
3-002(#1): δ8.62 (br, 1H), 7.47-7.30 (m, 2H), 7.17 (s, 1H), 7.06-6.93 (m, 3H), 6.91-6.82 (m, 1H), 4.86 (s, 2H), 2.31 (s, 3H), 2.29 (s, 3H), 1.71 (s, 9H).
3-003(#1): δ8.69 (br, 1H), 7.47-7.21 (m, 6H), 7.16 (s, 1H), 7.01-6.91 (m, 1H), 4.82 (s, 2H), 1.70 (s, 9H).
3-004(#1): δ8.78 (br, 1H), 7.47-7.24 (m, 4H), 7.21-7.11 (m, 2H), 7.03-6.93 (m, 1H), 4.97 (s, 2H), 1.70 (s, 9H).
3-005(#1): δ8.64 (br, 1H), 7.48-7.31 (m, 2H), 7.26-7.14 (m, 3H), 7.04-6.93 (m, 1H), 6.85-6.75 (m, 2H), 4.79 (s, 2H), 3.79 (s, 3H), 1.70 (s, 9H).
3-006(#1): δ8.67 (br, 1H), 7.46-7.32 (m, 2H), 7.24-7.14 (m, 2H), 7.03-6.93 (m, 1H), 6.63-6.48 (m, 2H), 4.88 (s, 2H), 3.78 (s, 3H), 1.70 (s, 9H).
3-007(#1): δ8.68 (br, 1H), 7.47-7.33 (m, 5H), 7.18-7.12 (m, 2H), 7.09 (s, 1H), 7.02-6.94 (m, 1H), 5.00 (q, J = 6.6 Hz, 1H), 1.69 (s, 9H), 1.53 (d, J = 6.6 Hz, 3H).
3-008(#1): δ8.74-8.58 (m, 1H), 7.47-7.30 (m, 2H), 7.22-7.04 (m, 5H), 7.02-6.90 (m, 1H), 4.83 (s, 2H), 2.32 (s, 3H), 1.70 (s, 9H).
3-009(#1): δ8.68 (br, 1H), 7.47-7.31 (m, 2H), 7.24-7.15 (m, 3H), 7.03-6.94 (m, 1H), 6.84-6.74 (m, 2H), 4.80 (s, 2H), 3.78 (s, 3H), 1.69 (s, 9H).
3-010(#1): δ8.63 (br, 1H), 7.47-7.30 (m, 2H), 7.23-7.14 (m, 3H), 7.02-6.93 (m, 1H), 6.82-6.74 (m, 2H), 4.79 (s, 2H), 4.00 (q, J = 6.9 Hz, 2H), 1.70 (s, 9H), 1.41 (t, J = 6.9 Hz, 3H).
3-011(#1): δ8.72 (br, 1H), 7.48-7.31 (m, 2H), 7.24-7.15 (m, 2H), 7.05-6.97 (m, 1H), 6.64-6.48 (m, 2H), 4.88 (s, 2H), 3.77 (s, 3H), 1.69 (s, 9H).
3-012(#1): δ8.64 (br, 1H), 7.47-7.29 (m, 2H), 7.22-7.16 (m, 1H), 7.08-6.96 (m, 2H), 6.72-6.67 (m, 1H), 6.61-6.53 (m, 1H), 4.83 (s, 2H), 3.77 (s, 3H), 2.34 (s, 3H), 1.70 (s, 9H).
3-013(#1): δ8.76 (br, 1H), 7.47-7.31 (m, 2H), 7.27-7.16 (m, 2H), 7.04-6.97 (m, 1H), 6.89-6.84 (m, 1H), 6.73-6.64 (m, 1H), 4.95 (s, 2H), 3.77 (s, 3H), 1.70 (s, 9H).
3-014(#1): δ8.72 (br, 1H), 7.45-7.29 (m, 2H), 7.22-7.16 (m, 1H), 7.14-7.06 (m, 1H), 7.03-6.96 (m, 1H), 6.44-6.39 (m, 1H), 6.38-6.30 (m, 1H), 4.87 (s, 2H), 3.79 (s, 3H), 3.77 (s, 3H), 1.70 (s, 9H).
3-015(#1): δ8.78 (br, 1H), 7.48-7.33 (m, 2H), 7.21-7.11 (m, 2H), 7.03-6.96 (m, 1H), 6.87-6.79 (m, 2H), 4.89 (s, 2H), 3.78 (s, 3H), 1.69 (s, 9H).
3-016(#1): δ8.74 (br, 1H), 7.47-7.29 (m, 2H), 7.28-7.22 (m, 2H), 7.20-7.13 (m, 1H), 7.03-6.96 (m, 1H), 6.87-6.80 (m, 2H), 4.81 (s, 2H), 4.32 (q, J = 7.8 Hz, 2H), 1.69 (s, 9H).
3-017(#1): δ8.61 (br, 1H), 7.47-7.32 (m, 2H), 7.22-7.18 (m, 4H), 7.16-7.12 (m, 1H), 7.09 (s, 1H), 7.02-6.94 (m, 1H), 5.02 (q, J = 6.9 Hz, 1H), 1.69 (s, 9H), 1.53 (d, J = 6.9 Hz, 3H).
3-018(#1): δ8.57 (br, 1H), 7.45-7.29 (m, 2H), 7.19-6.97 (m, 7H), 5.01 (q, J = 6.6 Hz, 1H), 2.27 (s, 3H), 1.70 (s, 9H), 1.57-1.52 (m, 3H).
3-020(#2): δ8.89 (br, 1H), 8.04 (s, 1H), 7.66-7.53 (m, 1H), 7.48-7.33 (m, 2H), 7.22 (s, 1H), 7.06-6.97 (m, 1H), 6.72-6.65 (m, 1H), 4.80 (s, 2H), 3.91 (s, 3H), 1.69 (s, 9H).
3-021(#2): δ8.87-8.71 (m, 1H), 8.03 (s, 1H), 7.64-7.54 (m, 1H), 7.46-7.33 (m, 2H), 7.21 (s, 1H), 7.05-6.98 (m, 1H), 6.69-6.62 (m, 1H), 4.80 (s, 2H), 4.33 (q, J = 7.2 Hz, 2H), 1.69 (s, 9H), 1.39 (t, J = 7.2 Hz, 3H).
3-022(#1): δ8.59 (br, 1H), 7.45-7.32 (m, 2H), 7.23-7.12 (m, 3H), 7.08 (s, 1H), 7.05-6.99 (m, 1H), 6.80-6.72 (m, 2H), 5.00 (q, J = 6.3 Hz, 1H), 3.74 (s, 3H), 1.70 (s, 9H), 1.54 (d, J = 6.3 Hz, 3H).
3-023(#1): δ8.78-8.68 (br, 1H), 7.56-7.47 (m, 2H), 7.47-7.35 (m, 4H), 7.17-7.07 (m, 2H), 7.07-6.93 (m, 1H), 5.14-5.02 (m, 1H), 1.69 (s, 9H), 1.62-1.48 (m, 3H).
3-024(#1): δ8.58 (br, 1H), 7.48-7.31 (m, 2H), 7.23-7.03 (m, 6H), 7.03-6.91 (m, 1H), 4.87-4.66 (m, 1H), 1.68 (s, 9H), 1.31-1.20 (m, 2H), 0.93-0.80 (m, 3H).
3-025(#1): δ8.70 (br, 1H), 8.02-7.88 (m, 2H), 7.45-7.28 (m, 4H), 7.13-6.89 (m, 3H), 5.20-5.03 (m, 1H), 3.07-2.89 (m, 2H), 1.72-1.65 (s, 9H), 1.65-1.53 (m, 3H), 1.53-1.39 (m, 3H).
3-026(#1): δ8.70-8.65 (br, 1H), 7.94-7.88 (m, 2H), 7.41-7.26 (m, 4H), 7.13-7.05 (m, 2H), 6.99-6.87 (m, 1H), 5.17-5.03 (m, 1H), 2.37-2.19 (m, 1H), 1.70 (s, 9H), 1.63-1.54 (m, 3H), 1.40-1.19 (m, 4H).
3-027(#1): δ8.69 (br, 1H), 8.00-7.89 (m, 2H), 7.43-7.27 (m, 4H), 7.13-6.91 (m, 3H), 5.17-5.04 (m, 1H), 2.66 (s, 3H), 1.69 (s, 9H), 1.64-1.53 (m, 3H).
3-028(#1): δ8.95-8.88 (br, 1H), 8.02-7.92 (m, 2H), 7.50-7.34 (m, 2H), 7.16-7.01 (m, 2H), 6.93-6.82 (m, 1H), 6.70-6.57 (m, 2H), 5.20-4.99 (m, 1H), 3.09-2.89 (m, 2H), 2.33-2.22 (m, 3H), 1.71 (s, 9H), 1.65-1.56 (m, 3H), 1.53-1.42 (m, 3H).
3-029(#1): δ8.90 (br, 1H), 7.99-7.85 (m, 2H), 7.43-7.35 (m, 2H), 7.15-7.02 (m, 2H), 6.91-6.82 (m, 1H), 6.69-6.57 (m, 2H), 5.19-5.01 (m, 1H), 2.26 (s, 3H), 1.72 (s, 9H), 1.66-1.56 (m, 4H), 1.35-1.20 (m, 4H).
3-030(#1): δ8.91 (br, 1H), 8.01-7.91 (m, 2H), 7.46-7.35 (m, 2H), 7.18-7.00 (m, 2H), 6.91-6.82 (m, 1H), 6.71-6.58 (m, 2H), 5.20-4.99 (m, 1H), 2.66 (s, 3H), 2.27 (s, 3H), 1.71 (s, 9H), 1.64-1.49 (m, 3H).
3-031(#2): δ9.40 (br, 1H), 7.96-7.89 (m, 2H), 7.42-7.28 (m, 4H) 7.16-7.10 (m, 1H), 7.10-6.93 (m, 1H), 4.91 (s, 2H), 2.64 (s, 3H), 1.67 (s, 9H).
3-032(#2): δ9.04-8.89 (br, 1H), 7.99-7.81 (m, 2H), 7.57-7.38 (m, 2H), 7.20-6.78 (m, 3H), 6.75-6.56 (m, 2H), 5.28-5.04 (m, 1H), 2.27 (s, 3H), 1.76 (s, 9H), 1.66-1.54 (m, 4H), 1.39-1.16 (m, 4H).
4-001(#2): δ9.23 (br, 1H), 7.53-7.25 (m, 3H), 7.23-7.15 (m, 2H), 6.13 (s, 1H), 5.01 (s, 2H), 2.23 (d, J = 1.2 Hz, 3H), 1.73 (s, 9H).
4-002(#2): δ9.16 (br, 1H), 7.81-7.68 (m, 2H), 7.63-7.25 (m, 6H), 7.22-7.11 (m, 2H), 6.63 (s, 1H), 5.13 (s, 2H), 1.73 (s, 9H).
4-003(#1): δ9.19 (br, 1H), 7.72-7.64 (m, 1H), 7.51-7.28 (m, 6H), 7.22-7.12 (m, 2H), 6.81 (s, 1H), 5.13 (s, 2H), 1.73 (s, 9H).
4-004(#1): δ9.16 (br, 1H), 7.74-7.70 (m, 1H), 7.64-7.58 (m, 1H), 7.47-7.27 (m, 5H), 7.21-7.13 (m, 2H), 6.62 (s, 1H), 5.12 (s, 2H), 1.73 (s, 9H).
4-006(#1): δ9.22 (br, 1H), 7.46-7.13 (m, 9H), 6.51 (s, 1H), 5.12 (s, 2H), 2.43 (s, 3H), 1.73 (s, 9H).
4-007(#1): δ9.15 (br, 1H), 7.58 (s, 1H), 7.54-7.47 (m, 1H), 7.42-7.24 (m, 5H), 7.19-7.12 (m, 2H), 6.62 (s, 1H), 5.11 (s, 2H), 2.40 (s, 3H), 1.73 (s, 9H).
4-011(#1): δ9.80 (br, 1H), 8.86 (s, 1H), 8.65-8.57 (m, 1H), 8.07-7.98 (m, 1H), 7.35-7.31 (m, 4H), 7.20-7.11 (m, 2H), 6.66 (s, 1H), 5.13 (s, 2H), 1.72 (s, 9H).
4-012(#1): δ9.18 (br, 1H), 7.88-7.80 (m, 1H), 7.46-7.38 (m, 1H), 7.37-7.27 (m, 3H), 7.18-7.11 (m, 2H), 7.07-6.95 (m, 2H), 6.84 (s, 1H), 5.11 (s, 2H), 3.86 (s, 3H), 1.73 (s, 9H).
4-013(#1): δ9.24 (br, 1H), 7.97-7.87 (m, 1H), 7.30 (d, J = 114.8 Hz, 8H), 6.80-6.73 (m, 1H), 5.12 (s, 2H), 1.72 (s, 9H). 4-014(#1): δ9.14 (br, 1H), 7.45-7.27 (m, 5H), 7.21-7.06 (m, 3H), 6.56 (s, 1H), 5.09 (s, 2H), 1.73 (s, 9H).
4-015(#1): δ9.24 (br, 1H), 7.66-7.60 (m, 1H), 7.47-7.26 (m, 5H), 7.19-7.11 (m, 2H), 6.53 (s, 1H), 5.09 (s, 2H), 1.72 (s, 9H).
5-002(#2): δ9.57 (br, 1H), 7.41-7.34 (m, 2H), 7.20-7.14 (m, 2H), 7.13-7.08 (m, 2H), 7.07-7.02 (m, 2H), 6.97-6.92 (m, 2H), 2.28 (s, 3H), 1.75 (s, 9H).
6-001(#1): δ8.99 (br, 1H), 7.65-7.56 (m, 1H), 7.54-7.27 (m, 4H), 7.25-7.05 (m, 3H), 5.05 (s, 2H), 3.88 (s, 3H), 2.48 (s, 3H), 1.72 (s, 9H).
6-002(#2): δ8.92 (br, 1H), 7.56-7.51 (m, 1H), 7.50-7.46 (m, 1H), 7.45-7.32 (m, 3H), 7.24-7.20 (m, 1H), 7.15 (s, 1H), 7.13-7.05 (m, 1H), 5.03 (s, 2H), 3.91 (s, 3H), 3.83 (s, 3H), 1.72 (s, 9H).
6-004(#1): δ8.78-8.67 (br, 1H), 7.47-7.32 (m, 2H), 7.25-7.06 (m, 4H), 6.76-6.52 (m, 2H), 6.29-5.84 (m, 1H), 4.94-4.87 (m, 2H), 4.19-4.02 (m, 2H), 2.44-2.33 (m, 3H), 1.77-1.70 (s, 9H). 6-005(#1): δ8.93 (br, 1H), 7.40-7.22 (m, 2H), 7.22-7.05 (m, 3H), 7.08-6.93 (m, 2H), 6.74-6.51 (m, 2H), 6.30-5.82 (m, 1H), 4.91 (s, 2H), 4.21-4.01 (m, 2H), 2.44-2.37 (s, 3H), 1.75 (s, 9H).
6-006(#1): δ8.94 (br, 1H), 7.23-7.06 (m, 3H), 6.99-6.87 (m, 1H), 6.84-6.69 (m, 3H), 6.60-6.48 (m, 1H), 6.29-5.79 (m, 1H), 4.90 (s, 2H), 4.20-4.00 (m, 2H), 2.40 (s, 3H), 2.33 (s, 3H), 1.72 (s, 9H).
6-007(#2): δ9.03 (br, 1H), 7.22-7.10 (m, 3H), 6.94-6.89 (m, 1H), 6.80-6.72(m, 3H), 6.63-6.55 (m, 1H), 4.88 (s, 2H), 4.08-4.02 (m, 2H), 3.75-3.69 (m, 2H), 3.44 (s, 3H), 2.37 (s, 3H), 2.32 (s, 3H), 1.72 (s, 9H).
6-008(#2): δ8.93 (br, 1H), 7.45-7.31 (m, 2H), 7.27-7.22 (m, 1H), 7.17-7.08(m, 3H), 6.75-6.71 (m, 1H), 6.62-6.56 (m, 1H), 4.88 (s, 2H), 4.08-4.02 (m, 2H), 3.75-3.69 (m, 2H), 3.43 (s, 3H), 2.36 (s, 3H), 1.71 (s, 9H).
7-001(#1): δ9.93-9.71 (m, 1H), 9.14-9.06 (m, 1H), 8.37-8.23 (m, 1H), 7.49-7.22 (m, 3H), 7.22-7.04 (m, 3H), 5.11 (s, 2H), 2.69 (s, 3H), 1.72 (s, 9H).
8-001(#2): δ9.20-9.03 (m, 1H), 8.78-8.72 (m, 1H), 8.10-8.04 (m, 1H), 7.97-7.84 (m, 1H), 7.51-7.32 (m, 2H), 7.21-7.11 (m, 3H), 5.04 (s, 2H), 2.70 (s, 3H), 1.71 (s, 9H).

**[Table 20]**

| | |
|---|---|
| No. | ¹H-NMR (CDCl₃, Me₄Si) |

9-002(#1): δ7.62 (s, 1H), 7.32-7.23 (m, 3H), 7.11-6.97 (m, 2H), 6.95-6.87 (m, 4H), 2.94 (s, 3H), 2.26 (s, 3H), 2.20 (s, 3H), 1.73 (s, 9H).
9-003(#1): δ7.44 (s, 1H), 7.29-7.00 (m, 5H), 6.99-6.89 (m, 2H), 6.83-6.77 (m, 1H), 2.98 (s, 3H), 2.27 (s, 3H), 2.17 (s, 3H), 1.72 (s, 9H).
9-004(#2): δ8.24 (br, 1H), 7.51-7.47 (m, 1H), 7.24-7.20 (m, 1H), 7.18 (s, 1H), 7.05-6.98 (m, 1H), 6.95-6.89 (m, 1H), 6.84-6.78 (m, 1H), 6.74-6.67 (m, 1H), 6.22-6.00 (m, 1H), 2.25 (s, 3H), 2.22 (s, 3H), 1.72 (s, 9H).
9-005(#2): δ7.61 (br, 1H), 7.41-7.34 (m, 1H), 7.21 (s, 1H), 7.17-7.12 (m, 1H), 7.10-7.05 (m, 1H), 6.99-6.90 (m, 2H), 6.88-6.80 (m, 1H), 2.99 (s, 3H), 2.27 (s, 3H), 2.19 (s, 3H), 1.72 (s, 9H).
9-007(#1): δ7.46 (br, 1H), 7.29-7.11 (m, 2H), 7.11-7.08 (m, 5H), 6.80-6.68 (m, 1H), 2.98 (s, 3H), 2.27 (s, 3H), 2.17 (s, 3H), 1.72 (s, 9H).
9-008(#1): δ8.17 (d, J = 4.8 Hz, 1H), 7.37-7.28 (m, 2H), 7.20 (s, 1H), 7.06-6.98 (m, 2H), 6.91-6.87 (m, 1H), 6.81-6.72 (m, 1H), 6.67-6.60 (m, 1H), 6.08 (d, J = 4.8 Hz, 1H), 2.24 (s, 3H), 2.22 (s, 3H), 1.72 (s, 9H).
9-010(#1): δ7.63 (br, 1H), 7.27 (s, 1H), 7.22-7.17 (m, 1H), 7.14-7.04 (m, 2H), 6.98-6.87 (m, 2H), 6.83-6.78 (m, 1H), 2.98 (s, 3H), 2.28 (s, 3H), 2.14 (s, 3H), 1.74 (s, 9H).
9-012(#2): δ7.45 (br, 1H), 7.28-7.19 (m, 2H), 7.06-7.01 (m, 1H), 6.96-6.90 (m, 2H), 6.82-6.73 (m, 1H), 6.70-6.64 (m, 1H), 6.63-6.57 (m, 1H), 2.97 (s, 3H), 2.27 (s, 3H), 2.18 (s, 3H), 1.73 (s, 9H).
9-013(#1): δ8.37 (d, J = 4.8 Hz, 1H), 7.52-7.42 (m, 1H), 7.30-7.20 (m, 1H), 7.17 (s, 1H), 7.15-7.06 (m, 2H), 6.93-6.86 (m, 1H), 6.82-6.68 (m, 2H), 6.11 (d, J = 4.8 Hz, 1H), 2.25 (s, 3H), 2.21 (s, 3H), 1.72 (s, 9H).
9-014(#1): δ7.75 (br, 1H), 7.42-7.35 (m, 1H), 7.22-7.14 (m, 2H), 7.09-7.00 (m, 2H), 6.95-6.89 (m, 3H), 2.96 (s, 3H), 2.26 (s, 3H), 2.21 (s, 3H), 1.73 (s, 9H).
9-016(#1): δ7.38 (br, 1H), 7.31-7.26 (m, 1H), 7.22 (s, 1H), 7.08-7.01 (m, 1H), 6.98-6.88 (m, 3H), 6.73-6.67 (m, 1H), 3.01 (s, 3H), 2.28 (s, 3H), 2.16 (s, 3H), 1.72 (s, 9H).
10-001(#2): δ7.96-7.89 (m, 1H), 7.43-7.34 (m, 2H), 7.15-7.10 (m, 1H), 7.08 (s, 1H), 7.06-6.95 (m, 1H), 6.95-6.88 (m, 1H), 6.86-6.81 (m, 1H), 6.74-6.64 (m, 1H), 4.69 (br, 2H), 2.22-2.12 (m, 6H), 1.74 (s, 9H), 1.52-1.34 (m, 9H).
10-002(#1): δ7.99-7.85 (m, 1H), 7.36-7.27 (m, 2H), 7.17 (s, 1H), 7.15-7.05 (m, 2H), 7.01-6.94 (m, 3H), 6.93-6.84 (m, 1H), 5.02-4.82 (m, 1H), 4.01-3.76 (m, 2H), 2.32 (s, 3H), 2.29 (s, 3H), 1.75 (s, 9H).
10-003(#1): δ8.24-8.05 (m, 1H), 8.00 (s, 1H), 7.31-7.26 (m, 1H), 7.15-7.04 (m, 2H), 6.97 (d, J = 7.8 Hz, 1H), 6.89-6.81 (m, 3H), 6.73 (d, J = 7.8 Hz, 1H), 6.03-5.60 (m, 1H), 5.40-5.00 (m, 2H), 4.75 (s, 2H), 4.68-4.38 (m, 2H), 2.21 (s, 6H), 1.72 (s, 9H).
10-004(#2): δ7.67 (s, 1H), 7.37-7.29 (m, 2H), 7.20 (s, 1H), 7.15-7.07 (m, 2H), 7.05-6.99 (m, 2H), 6.98-6.94 (m, 1H), 6.92-6.85 (m, 1H), 3.92 (s, 2H), 3.71 (s, 2H), 2.34 (s, 3H), 2.26 (s, 3H), 1.68 (s, 9H).
10-006(#2): δ7.80-7.68 (m, 1H), 7.46-7.38 (m, 1H), 7.38-7.32 (m, 1H), 7.25-7.21 (m, 1H), 7.19 (s, 1H), 7.15-7.06 (m, 2H), 6.98-6.92 (m, 1H), 6.90-6.82 (m, 1H), 5.00-4.88 (m, 1H), 3.97-3.84 (m, 2H), 2.29 (s, 3H), 2.26 (s, 3H), 1.73 (s, 9H).
10-007(#2): δ7.58-7.50 (m, 1H), 7.48-7.40 (m, 1H), 7.40-7.33 (m, 1H), 7.32-7.27 (m, 1H), 7.21 (s, 1H), 7.19-7.13 (m, 1H), 7.13-7.05 (m, 1H), 6.99-6.90 (m, 1H), 6.89-6.81 (m, 1H), 3.96 (s, 2H), 3.77 (s, 2H), 2.33 (s, 3H), 2.24 (s, 3H), 1.67 (s, 9H). 10-014(#2): δ8.11 (s, 1H), 7.34-7.25 (m, 2H), 7.15-7.07 (m, 2H), 7.02-6.95 (m, 1H), 6.92-6.83 (m, 3H), 6.77-6.72 (m, 1H), 4.74 (s, 2H), 4.39-3.97 (m, 2H), 2.21 (s, 6H), 1.73 (s, 9H), 1.37-1.02 (m, 3H).
10-016(#2): δ8.26 (s, 1H), 7.36-7.22 (m, 5H), 7.16-7.09 (m, 1H), 7.05-6.99 (m, 2H), 6.91-6.85 (m, 2H), 4.61 (s, 2H), 1.73 (s, 9H), 1.44 (s, 9H).
10-017(#2): δ7.79 (d, J = 7.2 Hz, 1H), 7.18-7.11 (m, 3H), 7.03-6.96 (m, 1H), 6.95 (s, 1H), 6.92-6.86 (m, 2H), 4.94 (dt, J = 7.2, 5.2 Hz, 1H), 3.94 (d, J = 5.2 Hz, 2H), 2.32 (s, 3H), 2.26 (s, 3H), 1.72 (s, 9H).
10-024(#2): δ8.31 (s, 1H), 7.46-7.42 (m, 2H), 7.15 (s, 1H), 7.07 (s, 1H), 7.03-7.02 (m, 1H), 6.92 (d, J = 7.6 Hz, 1H), 6.83 (s, 1H), 6.72 (d, J = 7.6 Hz, 1H), 5.80-3.92 (m, 2H), 2.16 (s, 6H), 2.06 (s, 3H), 1.73 (s, 9H).
10-025(#2): δ8.31 (s, 1H), 7.42-7.36 (m, 2H), 7.32-7.30 (m, 1H), 7.21 (s, 1H), 7.11-7.07 (m, 2H), 6.93 (s, 1H), 6.86-6.84 (m, 1H), 4.02 (s, 2H), 3.64 (s, 3H), 3.58 (s, 2H), 2.36 (s, 3H), 2.24 (s, 3H), 1.61 (s, 9H).
10-026(#2): δ8.31 (s, 1H), 7.42-7.36 (m, 2H), 7.32-7.30 (m, 1H), 7.21 (s, 1H), 7.11-7.07 (m, 2H), 6.93 (s, 1H), 6.86-6.84 (m, 1H), 4.02 (s, 2H), 3.64 (s, 3H), 3.58 (s, 2H), 2.36 (s, 3H), 2.24 (s, 3H), 1.61 (s, 9H).
10-032(#2): δ7.34-7.25 (m, 2H), 7.17 (s, 1H), 7.14 (s, 1H), 7.12-7.04 (m, 2H), 6.96-6.89 (m, 3H), 6.84-6.79 (m, 1H), 5.91-5.77 (m, 1H), 5.16-4.98 (m, 2H), 3.89 (s, 2H), 3.46 (d, J = 6.8 Hz, 2H), 2.32 (s, 3H), 2.24 (s, 3H), 1.65 (s, 9H).
10-035(#2): δ8.19 (s, 1H), 7.33-7.25 (m, 2H), 7.16-7.08 (m, 1H), 7.06 (s, 1H), 7.01-6.96 (m, 1H), 6.91-6.83 (m, 3H), 6.76-6.69 (m, 1H), 4,75 (s, 2H), 4.44-4.13 (m, 2H), 3.70-3.17 (m, 5H), 2.17 (s, 6H), 1.73 (s, 9H).
10-036(#2): δ7.77-7.72 (br, 1H), 7.24-7.09 (m, 5H), 6.97-6.85 (m, 3H), 4.99-4.90 (br, 1H), 3.93-3.89 (m, 2H), 2.31 (s, 3H), 2.29 (s, 3H), 1.73 (s, 9H).
10-038(#2): δ8.14 (s, 1H), 7.45-7.38 (m, 3H), 7.20-7.18 (m, 2H), 7.13 (s, 1H), 7.06-7.05 (m, 2H), 5.97-5.67 (m, 1H), 5.40-5.05 (m, 2H), 4.84 (s, 2H), 4.75-4.44 (m, 2H), 1.72 (s, 9H).
10-040(#1): δ7.74 (s, 1H), 7.44-7.33 (m, 2H), 7.22-7.15 (m, 4H), 7.10-7.04 (m, 3H), 5.05 (s, 1H), 3.91 (s, 2H), 2.30 (s, 3H), 1.73 (s, 9H).
10-041(#1): δ8.31 (s, 1H), 7.36-7.17 (m, 4H), 7.15-7.08 (m, 2H), 7.07-6.99 (m, 1H), 6.91-6.84 (m, 2H), 6.02-5.66 (m, 1H), 5.37-5.05 (m, 2H), 4.83 (s, 2H), 4.74-4.44 (m, 2H), 1.71 (s, 9H).
10-042(#1): δ8.14 (s, 1H), 7.33-7.24 (m, 2H), 7.18-7.02 (m, 4H), 7.00-6.93 (m, 2H), 6.91-6.45 (m, 2H), 6.05-5.64 (m, 1H), 5.39-5.04 (m, 2H), 4.69 (s, 2H), 4.60-4.45 (m, 2H), 2.26 (s, 3H), 1.72 (s, 9H).
10-048(#1): δ7.74 (d, J = 6.9 Hz, 1H), 7.24-7.15 (m, 2H), 7.14-7.02 (m, 2H), 6.98-6.92 (m, 2H), 6.91-6.84 (m, 1H), 6.84-6.78 (m, 1H), 4.94 (dt, J = 6.9, 5.1 Hz, 1H), 3.90 (d, J = 5.1 Hz, 2H), 2.30 (s, 3H), 2.28 (s, 3H), 1.73 (s, 9H).
10-049(#1): δ7.77 (d, J = 6.6 Hz, 1H), 7.28-7.19 (m, 2H), 7.14 (s, 1H), 7.13-7.08 (m, 1H), 6.94 (s, 1H), 6.90-6.81 (m, 3H), 5.01-4.85 (m, 1H), 3.91 (d, J = 4.5 Hz, 2H), 2.30 (s, 3H), 2.28 (s, 3H), 1.72 (s, 9H).
10-051(#1): δ7.73 (d, J = 7.5 Hz, 1H), 7.29-7.16 (m, 2H), 7.14-7.07 (m, 1H), 6.95 (s, 1H), 6.92-6.84 (m, 1H), 6.83-6.68 (m, 2H), 6.67-6.59 (m, 1H), 5.02-4.84 (m, 1H), 3.89 (d, J = 4.8 Hz, 2H), 2.29 (s, 3H), 2.28 (s, 3H), 1.73 (s, 9H).
10-053(#2): δ8.01-7.92 (m, 1H), 7.37-7.23 (m, 4H), 7.15 (s, 1H), 7.14-7.04 (m, 2H), 6.98-6.92 (m, 2H), 5.21-5.13 (m, 1H), 4.04 (d, J = 5.2 Hz, 2H), 1.70 (s, 9H).
10-054(#2): δ7.98-7.90 (m, 1H), 7.34-7.26 (m, 3H), 7.22-7.17 (m, 2H), 7.15 (s, 1H), 7.11-7.05 (m, 2H), 6.98-6.92 (m, 2H), 5.06-4.98 (m, 1H), 3.91 (d, J = 5.2 Hz, 2H), 2.31 (s, 3H), 1.73 (s, 9H).
10-055(#2): δ7.79 (d, J = 6.4 Hz, 1H), 7.45-7.37 (m, 2H), 7.30-7.23 (m, 3H), 7.17 (s, 1H), 7.11-7.07 (m, 2H), 5.17 (m, 1H), 4.05 (d, J = 5.2 Hz, 2H), 1.70 (s, 9H).
10-060(#2): δ8.28 (s, 1H), 7.35-7.26 (m, 3H), 7.22-7.20 (m, 1H), 7.16-7.09 (m, 2H), 7.07-7.01 (m, 1H), 6.92-6.87 (m, 2H), 4.82 (s, 2H), 4.33-4.02 (m, 2H), 1.73 (s, 9H), 1.35-1.00 (m, 3H).
10-061(#2): δ8.12 (s, 1H), 7.35-7.27 (m, 3H), 7.17-7.03 (m, 3H), 7.02-6.95 (m, 2H), 6.92-6.87 (m, 2H), 4.68 (s, 2H), 4.35-3.99 (m, 2H), 2.26 (s, 3H), 1.73 (s, 9H), 1.39-0.99 (m, 3H).
10-065(#2): δ8.41 (s, 1H), 7.39-7.19 (m, 4H), 7.18-7.01 (m, 4H), 7.00-6.59 (m, 5H), 4.82 (s, 2H), 2.26 (s, 3H), 2.22 (s, 3H), 1.70 (s, 9H).
10-066(#2): δ8.46 (s, 1H), 8.40-8.05 (m, 2H), 7.56-6.77 (m, 11H), 4.84 (s, 2H), 2.28 (s, 3H), 2.23 (s, 3H), 1.70 (s, 9H).
10-068(#2): δ8.13 (br, 1H), 7.51-7.38 (m, 3H), 7.22 (s, 1H), 7.19-7.15 (m, 1H), 7.13-7.09 (m, 1H), 6.95 (s, 1H), 6.87-6.82 (m, 1H), 5.79-5.65 (m, 1H), 4.97 (br, 1H), 3.97 (s, 2H), 2.33 (s, 3H), 2.25 (s, 3H), 1.52 (s, 3H), 1.51 (s, 3H).
10-073(#2): δ8.54-8.27 (m, 1H), 7.37-7.27 (m, 2H), 7.20-7.10 (m, 2H), 7.03-6.96 (m, 1H), 6.93-6.85 (m, 3H), 6.81-6.72 (m, 1H), 6.12-5.54 (m, 2H), 5.46-5.07 (m, 2H), 4.94-4.45 (m, 4H), 2.29-2.15 (m, 6H), 1.49 (s, 3H), 1.47 (s, 3H).
10-091(#2): δ8.78-8.57 (m, 2H), 8.17-8.00 (m, 1H), 7.76-7.33 (m, 5H), 7.25-7.19 (m, 1H), 7.18-7.11 (m, 1H), 6.98-6.91 (m, 1H), 6.90-6.84 (m, 1H), 6.75-6.68 (m, 1H), 6.10-5.68 (m, 1H), 5.45-5.11 (m, 2H), 4.84-4.53 (m, 4H), 2.32-2.07 (m, 6H).
10-098(#2): δ8.76-8.70 (m, 1H), 8.68-8.62 (m, 1H), 8.28-8.20 (m, 1H), 7.80-7.77 (m, 1H), 7.48-7.33 (m, 4H), 7.27-7.17 (m, 1H), 7.15-7.02 (m, 3H), 6.97-6.90 (m, 1H), 6.88-6.82 (m, 1H), 5.00-4.87 (m, 1H), 4.02-3.90 (m, 2H), 2.32 (s, 3H), 2.27 (s, 3H).
10-112(#2): δ7.22-7.12 (m, 3H), 6.97 (s, 1H), 6.93-6.85 (m. 2H), 6.83-6.75 (m, 2H), 3.94 (s, 2H), 2.33 (s, 6H), 2.29 (s, 3H), 1.74 (s, 9H) (NHNH proton peak was not observed).
10-116(#2): δ8.18 (s, 1H), 7.45-7.38 (m, 3H), 7.20-7.17 (m, 2H), 7.12 (s, 1H), 7.07-7.03 (m, 2H), 4.83 (s, 2H), 4.34-4.22 (br, 2H), 3.60-3.46 (br, 2H), 3.35-3.27 (br, 3H), 1.73 (s, 9H). 10-118(#2): δ8.35 (s, 1H), 7.36-7.27 (m, 2H), 7.25-7.08 (m, 4H), 7.06-7.00 (m, 1H), 6.93-6.87 (m, 2H), 4.82 (s, 2H), 4.32-4.26 (m, 2H), 3.65-3.57 (m, 2H), 3.39 (s, 3H), 1.73 (s, 9H).
10-119(#2): δ8.24 (s, 1H), 7.36-7.27 (m, 2H), 7.17-7.06 (m, 4H), 7.00-6.87 (m, 4H), 4.68 (s, 2H), 4.32-4.25 (m, 2H), 3.67-3.58 (m, 2H), 3.38 (s, 3H), 2.25 (s, 3H), 1.72 (s, 9H).
10-120(#2): δ8.19 (s, 1H), 7.45-7.19 (m, 7H), 7.13-7.06 (m, 1H), 7.04 (s, 1H), 7.01-6.91 (m, 1H), 6.89-6.76 (m, 3H), 6.75-6.69 (m, 1H), 5.32-5.00 (m, 2H), 4.77 (s, 2H), 2.20 (s, 6H), 1.66 (s, 9H).
10-127(#2): δ8.30 (s, 1H), 7.56-7.54 (m, 1H), 7.42-7.38 (m, 1H), 7.33-7.31 (m, 1H), 7.25-7.22 (m, 2H), 7.19 (s, 1H), 7.13 -7.11(m, 1H), 7.06-7.03 (m, 1H), 4.20 (s, 2H), 3.80 (s, 2H), 1.99 (s, 3H), 1.62 (s, 9H).
10-133(#2): δ8.51-8.24 (m, 1H), 7.41-7.26 (m, 2H), 7.19-7.08 (m, 1H), 7.05 (s, 1H), 7.03-6.96 (m, 1H), 6.95-6.82 (m, 3H), 6.79-6.72 (m, 1H), 4.94-4.73 (m, 4H), 2.22 (s, 3H), 2.21 (s, 3H), 1.72 (s, 9H).
10-135(#1): δ8.06 (s, 1H), 7.48-7.45 (m, 3H), 7.21 (s, 1H), 7.14-7.05 (m, 4H), 6.52 (t, J = 5.4 Hz, 1H), 5.56 (s, 2H), 3.58 (dq, J = 5.4 Hz, 7.5Hz, 2H), 1.70 (s, 9H), 1.07 (t, J = 7.5 Hz, 3H).
10-136(#1): δ8.09 (s, 1H), 7.45-7.36 (m, 2H), 7.21-7.15 (m, 4H), 7.08-7.03 (m, 2H), 4.83 (s, 2H), 3.77-3.52 (br, 3H), 1.72 (s, 9H).
10-137(#1): δ8.10 (s, 1H), 7.45-7.36 (m, 3H), 7.20-7.18 (m, 2H), 7.13 (s, 1H), 7.06-7.03 (m, 2H), 4.82 (s, 2H), 4.28-4.00 (br, 2H), 1.73 (s, 9H), 1.21-1.02 (br, 3H).
10-140(#2): δ8.12 (s, 1H), 7.31-7.22 (m, 2H), 7.13-7.04 (m, 2H), 7.00-6.93 (m, 1H), 6.88-6.80 (m, 3H), 6.76-6.68 (m, 1H), 5.89-5.29 (m, 2H), 4.85-4.33 (m, 4H), 2.20 (s, 6H), 1.72 (s, 9H), 1.71-1.58 (m, 3H).
10-141(#2): δ8.11 (s, 1H), 7.32-7.21 (m, 2H), 7.14-7.03 (m, 2H), 7.00-6.92 (m, 1H), 6.88-6.80 (m, 3H), 6.76-6.67 (m, 1H), 5.31-5.07 (m, 1H), 4.82-4.45 (m, 4H), 2.20 (s, 6H), 1.80-1.57 (m, 15H).
10-143(#2): δ8.17 (s, 1H), 7.33-7.26 (m, 2H), 7.16-7.09 (m, 1H), 7.06 (s, 1H), 6.96-6.90 (m, 1H), 6.89-6.81 (m, 3H), 6.71-6.66 (m, 1H), 5.59-5.43 (m, 1H), 5.17-5.03 (m, 1H), 2.19 (s, 3H), 2.17 (s, 3H), 1.75 (s, 9H), 1.43 (s, 9H).
10-146(#1): δ7.96-7.87 (m, 1H), 7.40-7.24 (m, 4H), 7.20-7.05 (m, 4H), 6.95-6.87 (m, 2H), 5.13-4.97 (m, 1H), 3.90 (d, J = 4.4 Hz, 2H), 1.72 (s, 9H).
10-149(#2): δ8.25-8.13 (m, 1H), 7.36-7.23 (m, 4H), 7.18-6.98 (m, 4H), 6.89-6.83 (m, 2H), 6.08-5.66 (m, 1H), 5.44-5.07 (m, 2H), 4.80-4.42 (m, 4H), 1.71 (s, 9H).
10-153(#2): δ8.14 (s, 1H), 7.36-7.27 (m, 2H), 7.19-7.07 (m, 4H), 6.95-6.89 (m, 2H), 6.74-6.66 (m, 2H), 4.66 (m, 2H), 3.95-3.39 (m, 6H), 1.72 (s, 9H).
10-154(#2): δ8.13 (s, 1H), 7.39-7.23 (m, 2H), 7.21-7.04 (m, 4H), 6.99-6.88 (m, 2H), 6.77-6.64 (m, 2H), 4.66 (m, 2H), 4.40-3.39 (m, 2H), 3.73 (s, 3H), 1.73 (s, 9H), 1.39-0.99 (m, 3H).
10-155(#2): δ8.18 (s, 1H), 7.35-7.28 (m, 2H), 7.18-7.06 (m, 4H), 6.96-6.89 (m, 2H), 6.71-6.65 (m, 2H), 6.03-5.65 (m, 1H), 5.48-5.04 (m, 2H), 4.85-4.40 (m, 4H), 3.72 (s, 3H), 1.72 (s, 9H).
10-156(#2): δ8.31-8.03 (m, 1H), 7.39-7.27 (m, 2H), 7.18-7.05 (m, 4H), 6.96-6.86 (m, 2H), 6.76-6.63 (m, 2H), 4.76-4.56 (m, 2H), 4.22-3.28 (m, 5H), 1.72 (s, 9H), 1.39 (t, J = 6.8 Hz, 3H). 10-158(#2): δ8.40-8.09 (m, 1H), 7.40-7.27 (m, 2H), 7.24-7.02 (m, 4H), 6.99-6.88 (m, 2H), 6.77-6.63 (m, 2H), 6.45-4.96 (m, 3H), 4.93-4.34 (m, 4H), 3.93 (q, J = 6.8 Hz, 2H), 1.72 (s, 9H), 1.39 (t, J = 6.8 Hz, 3H).
10-159(#2): δ8.01-7.88 (m, 1H), 7.34-7.28 (m, 2H), 7.23-7.18 (m, 2H), 7.15 (s, 1H), 7.12-7.04 (m, 1H), 7.01-6.92 (m, 2H), 6.81-6.75 (m, 2H), 5.06-4.91 (m, 1H), 3.99 (q, J = 7.2 Hz, 2H), 3.91-3.83 (m, 2H), 1.73 (s, 9H), 1.40 (d, J = 7.2 Hz, 3H). 10-160(#1): δ8.03-7.89 (br, 1H), 7.45-7.32 (m, 2H), 7.25-7.20 (m, 2H), 7.18-7.10 (m, 2H), 7.09-7.04 (m, 2H), 4.98 (br, 1H), 3.90-3.82 (m, 2H), 2.29-2.22 (m, 3H), 1.74-1.68 (m, 9H). 10-161(#1): δ8.05 (br, 1H), 7.34-7.22 (m, 2H), 7.14-7.04 (m, 4H), 6.96-6.89 (m, 3H), 4.97 (br, 1H), 3.85 (s, 2H), 2.27 (s, 3H), 1.72 (s, 9H).
10-165(#1): δ8.00-7.90 (m, 1H), 7.29-7.27 (m, 3H), 7.24-7.20 (m, 4H), 6.96-6.89 (m, 2H), 5.21-5.12 (m, 1H), 4.02 (d, J = 5.4 Hz, 2H), 1.70 (s, 9H).
10-168(#1): δ8.31 (s, 1H), 7.45-7.23 (m, 3H), 7.23-7.05 (m, 4H), 6.93-6.77 (m, 2H), 6.00-5.61 (m, 1H), 5.50-5.05 (m, 2H), 4.81 (s, 2H), 4.75-4.45 (m, 2H), 1.72 (s, 9H).
10-169(#2): δ8.10 (s, 1H), 7.34-7.26 (m, 2H), 7.15-7.06 (m, 2H), 7.01-6.94 (m, 1H), 6.91-6.83 (m, 3H), 6.77-6.71 (m, 1H), 5.77-5.19 (m, 2H), 4.89-4.51 (m, 4H), 2.21 (s, 6H), 2.14-1.99 (m, 2H), 1.72 (s, 9H), 1.00-0.87 (m, 3H).
10-170(#2): δ7.82-7.73 (m, 1H), 7.46-7.33 (m, 2H), 7.25-7.16 (m, 4H), 7.13-7.08 (m, 1H), 6.83-6.75 (m, 2H), 5.12-4.93 (m, 1H), 3.98 (q, J = 7.2 Hz, 2H), 3.88 (d, J = 4.0 Hz, 2H), 1.73 (s, 9H), 1.40 (t, J = 7.2 Hz, 3H).
10-171(#2): δ7.98-7.90 (m, 1H), 7.35-7.28 (m, 2H), 7.25-7.19 (m, 2H), 7.18-7.06 (m, 2H), 6.99-6.93 (m, 2H), 6.82-6.76 (m, 2H), 5.05-4.95 (m, 1H), 3.91-3.85 (m, 2H), 3.78 (s, 3H), 1.73 (s, 9H).
10-172(#2): δ7.79-7.69 (m, 1H), 7.46-7.04 (m, 7H), 6.82-6.71 (m, 2H), 5.06-4.96 (m, 1H), 3.92-3.85 (m, 2H), 3.76 (s, 3H), 1.73 (s, 9H).
10-173(#2): δ7.93 (s, 1H), 7.33-7.29 (m, 4H), 7.21-7.20 (m, 3H), 7.15 (s, 1H), 6.93-6.91 (m, 2H), 5.06 (s, 1H), 3.92 (s, 2H), 1.72 (s, 9H).
10-174(#2): δ8.13 (s, 1H), 7.34-7.29 (m, 3H), 7.16-7.11 (m, 5H), 6.88-6.86 (m, 2H), 4.68 (s, 2H), 3.95 -3.45 (br, 3H), 1.72 (s, 9H).
10-176(#2): δ8.18 (s, 1H), 7.34-7.30 (m, 3H), 7.13-7.11 (m, 5H), 6.88-6.86 (m, 2H), 6.04-5.68 (m, 1H), 5.39 -5.10 (m, 2H), 4.69 (s, 2H), 4.62-4.46 (br, 2H), 1.71 (s, 9H).
10-177(#2): δ8.60 (s, 1H), 7.35-7.27 (m, 2H), 7.19-7.12 (m, 1H), 7.05-6.98 (m, 2H), 6.87-6.80 (m, 3H), 6.79-6.74 (m, 1H), 5.63-5.39 (m, 1H), 4.39-4.14 (m, 1H), 4.10-3.86 (m, 2H), 3.72-3.23 (m, 2H), 2.20 (s, 3H), 2.18 (s, 3H), 1.72 (s, 9H), 1.43 (t, J = 7.2 Hz, 3H).
10-178(#2): δ8.29 (s, 1H), 7.38-7.28 (m, 2H), 7.21-7.12 (m, 1H), 7.07 (s, 1H), 6.97-6.91 (m, 1H), 6.90-6.83 (m, 3H), 6.78-6.70 (m, 1H), 5.50-4.10 (m, 2H), 4.05 (s, 2H), 3.32 (s, 3H), 2.19 (s, 6H), 1.72 (s, 9H).
10-179(#2): δ8.20 (s, 1H), 7.73-7.63 (m, 1H), 7.35-7.26 (m, 2H), 7.23-7.10 (m, 1H), 7.07 (s, 1H), 6.98-6.93 (m, 1H), 6.92-6.85 (m, 3H), 6.78-6.73 (m, 1H), 5.73-5.65 (m, 1H), 4.04-3.92 (m, 2H), 3.71-3.59 (m, 2H), 2.20 (s, 3H), 2.19 (s, 3H), 1.72 (s, 9H), 1.19 (t, J = 7.0 Hz, 3H).
10-180(#2): δ8.47 (s, 1H), 7.38-7.27 (m, 2H), 7.21-7.14 (m, 1H), 7.07-7.00 (m, 2H), 6.98-6.92 (m, 1H), 6.88-6.80 (m, 2H), 6.72-6.64 (m, 1H), 5.30-3.85 (m, 4H), 2.20 (s, 3H), 2.14 (s, 3H), 1.72 (s, 9H), 1.23 (t, J = 7.0 Hz, 3H).
10-181(#2): δ8.16 (s, 1H), 7.37-7.24 (m, 2H), 7.20-7.05 (m, 3H), 7.00-6.94 (m, 1H), 6.92-6.83 (m, 2H), 6.79-6.68 (m, 1H), 4.70 (s, 2H), 2.20 (s, 6H), 1.74 (s, 9H), 1.43 (s, 9H).
10-182(#2): δ8.80 (s, 1H), 7.45-7.35 (m, 3H), 7.30-7.18 (m, 2H), 7.16-7.12 (m, 1H), 7.05 (s, 1H), 6.94-6.88 (m, 2H), 5.50-5.26 (m, 1H), 4.70-4.44 (m, 1H), 1.72 (s, 9H).
10-193(#2): δ8.27-8.09 (m, 1H), 7.39-7.24 (m, 2H), 7.20-7.07 (m, 2H), 7.07-6.95 (m, 3H), 6.93-6.79 (m, 2H), 6.13-5.66 (m, 1H), 5.50-5.05 (m, 2H), 4.90-4.38 (m, 4H), 2.29-2.12 (m, 3H), 1.85-1.63 (m, 9H).
10-194(#2): δ7.77 (d, J = 6.8 Hz, 1H), 7.45-7.36 (m, 3H), 7.21 (s, 4H), 7.17 (s, 1H), 7.07-7.05 (m, 1H), 5.06 (dt, J = 6.8 Hz, 4.4 Hz, 1H), 3.93 (d, J = 4.4 Hz, 2H), 1.72 (s, 9H).
10-195(#2): δ7.97 (s, 1H), 7.46-7.38 (m, 3H), 7.18-7.13 (m, 5H), 7.03-7.01 (m, 1H), 4.70 (s, 2H), 3.95-3.45 (br, 3H), 1.72 (s, 9H).
10-197(#2): δ8.28 (s, 1H), 7.32-7.28 (m, 3H), 7.13-7.08 (m, 2H), 6.93-6.91 (m, 2H), 6.75 (m, 1H), 6.63-6.55 (m, 1H), 4.81 (s, 2H), 4.37-4.00 (br, 2H), 3.70 (s, 3H), 1.73 (s, 9H), 1.37-1.00 (br, 3H).
10-198(#2): δ8.38 (s, 1H), 7.31-7.27 (m, 3H), 7.12-7.08 (m, 2H), 6.92-6.90 (m, 2H), 6.74 (s, 1H), 6.62-6.55 (m, 1H), 6.07-5.65 (br, 1H), 5.44-5.04 (br, 2H), 4.82 (s, 2H), 4.75-4.43 (br, 2H), 3.68 (s, 3H), 1.71 (s, 9H).
10-199(#2): δ8.08 (s, 1H), 7.45-7.36 (m, 2H), 7.20-7.07 (m, 4H), 6.75-6.74 (m, 1H), 6.78-6.56 (br, 1H), 4.82 (s, 2H), 3.94-3.39 (br, 3H), 3.71 (s, 3H), 1.73 (s, 9H).
10-200(#2): δ8.13 (s, 1H), 7.46-7.36 (m, 2H), 7.26-7.19 (m, 2H), 7.12-7.08 (m, 2H), 6.74-6.73 (m, 1H), 6.65-6.57 (br, 1H), 4.81 (s, 2H), 4.36-3.96 (br, 2H), 3.70 (s, 3H), 1.74 (s, 9H), 1.18-0.97 (br, 3H).
10-201(#2): δ8.14 (s, 1H), 7.45-7.37 (m, 2H), 7.26-7.19 (m, 2H), 7.11-7.08 (m, 2H), 6.75-6.72 (m, 1H), 6.67-6.55 (br, 1H), 6.04-5.63 (br, 1H), 5.42-5.03 (br, 2H), 4.83 (s, 2H), 4.77-4.39 (br, 2H), 3.70 (s, 3H), 1.72 (s, 9H).
10-202(#2): δ7.98-7.96 (m, 1H), 7.33-7.29 (m, 2H), 7.21-7.19 (m, 1H), 7.15 (s, 1H), 7.12-7.08 (m, 1H), 6.99-6.96 (m, 2H), 6.87-6.86 (m, 1H), 6.64-6.61 (m, 1H), 5.14-5.09 (m, 1H), 4.01-3.99 (m, 2H), 3.73 (s, 3H), 1.70 (s, 9H).
10-203(#2): δ7.79 (s, 1H), 7.45-7.35 (m, 3H), 7.25-7.17 (m, 3H), 6.86 (m, 1H), 6.66-6.63 (m, 1H), 5.14-5.10 (m, 1H), 4.02-4.00 (m, 2H), 3.75 (s, 3H), 1.71 (s, 9H).
10-206(#2): δ7.80-7.71 (m, 1H), 7.48-7.30 (m, 4H), 7.24-7.12 (m, 4H), 7.10-7.02 (m, 1H), 5.17-4.98 (m, 1H), 3.96-3.88 (m, 2H), 1.72 (s, 9H).
10-207(#2): δ7.97-7.89 (m, 1H), 7.36-7.27 (m, 2H), 7.19-7.07 (m, 5H), 6.98-6.90 (m, 2H), 5.23-4.77 (m, 1H), 3.94-3.85 (m, 2H), 2.30 (s, 3H), 1.73 (s, 9H).
10-208(#2): δ8.02 (br, 1H), 7.51-7.35 (m, 2H), 7.21 (s, 1H), 7.16-7.03 (m, 4H), 6.79-6.62 (m, 2H), 4.65 (s, 2H), 4.04-3.28 (m, 5H), 1.72 (s, 9H), 1.43-1.34 (m, 3H).
10-209(#2): δ8.02 (br, 1H), 7.50-7.35 (m, 2H), 7.24-7.19 (m, 1H), 7.15-7.04 (m, 4H), 6.73-6.65 (m, 2H), 4.65 (s, 2H), 4.38-3.97 (m, 2H), 3.92 (q, J = 6.8 Hz, 2H), 1.73 (s, 9H), 1.43-0.95 (m, 3H), 1.39 (t, J = 6.8 Hz, 3H).
10-210(#2): δ8.02 (br, 1H), 7.47-7.35 (m, 2H), 7.24-7.19 (m, 1H), 7.17-7.02 (m, 4H), 6.74-6.64 (m, 2H), 6.12-5.61 (m, 1H), 5.55-4.97 (m, 2H), 4.93-4.34 (m, 4H), 3.92 (q, J = 7.2 Hz, 2H), 1.72 (s, 9H), 1.39 (t, J = 7.2 Hz, 3H).
10-211(#2): δ8.00-7.90 (m, 1H), 7.45-7.27 (m, 3H), 7.21-7.05 (m, 3H), 6.97-6.90 (m, 2H), 5.21-5.12 (m, 1H), 4.02 (d, J = 5.4 Hz, 2H), 1.70 (s, 9H).
10-212(#1): δ8.20-8.06 (m, 1H), 7.50-7.35 (m, 4H), 7.25-7.10 (m, 3H), 7.08-7.03 (m, 1H), 4.82 (s, 2H), 3.95-3.36 (m, 3H), 1.72 (s, 9H).
10-213(#2): δ8.20-8.06 (m, 1H), 7.50-7.35 (m, 4H), 7.25-7.12 (m, 3H), 7.08-7.03 (m, 1H), 4.81 (s, 2H), 4.40-4.00 (m, 2H), 1.73 (s, 9H), 1.40-0.95 (m, 3H).
10-214(#2): δ8.16 (s, 1H), 7.55-7.35 (m, 4H), 7.25-7.10 (m, 3H), 7.10-7.03 (m, 1H), 6.05-5.62 (m, 1H), 5.50-5.05 (m, 2H), 4.87-4.44 (m, 4H), 1.72 (s, 9H).
10-215(#2): δ8.16-7.93 (m, 1H), 7.50-7.33 (m, 2H), 7.22-7.09 (m, 2H), 7.07-6.96 (m, 3H), 6.93-6.87 (m, 1H), 4.80-4.68 (m, 2H), 3.98-3.37 (m, 3H), 2.29-2.14 (m, 3H), 1.71 (s, 9H).
10-216(#2): δ8.08-7.90 (m, 1H), 7.47-7.35 (m, 2H), 7.18-7.08 (m, 2H), 7.05-6.93 (m, 3H), 6.92-6.83 (m, 1H), 4.81-4.64 (m, 2H), 4.39-3.91 (m, 2H), 2.24-2.16 (m, 3H), 1.72 (s, 9H), 1.46-0.88 (m, 3H).
10-217(#2): δ8.19-7.96 (m, 1H), 7.49-7.34 (m, 2H), 7.18-7.07 (m, 2H), 7.06-6.95 (m, 3H), 6.92-6.84 (m, 1H), 6.06-5.57 (m, 1H), 5.47-5.00 (m, 2H), 4.86-4.37 (m, 4H), 2.26-2.15 (m, 3H), 1.71 (s, 9H).
10-218(#2): δ7.77-7.71 (m, 1H), 7.47-7.40 (m, 1H), 7.40-7.34 (m, 1H), 7.24-7.21 (m, 1H), 7.19 (s, 1H), 7.17-7.13 (m, 2H), 7.12-7.05 (m, 1H), 7.04-6.98 (m, 1H), 5.00-4.90 (m, 1H), 3.90 (d, J = 4.8 Hz, 2H), 2.29 (s, 3H), 1.73 (s, 9H).
10-219(#2): δ7.94 (s, 1H), 7.35-7.28 (m, 3H), 7.21-7.08 (m, 3H), 7.00-6.94 (m, 2H), 6.93-6.88 (m, 1H), 5.26-5.04 (m, 1H), 4.03 (s, 2H), 2.27 (s, 3H), 1.70 (s, 9H).
10-220(#2): δ7.77 (s, 1H), 7.48-7.34 (m, 3H), 7.26-7.10 (m, 4H), 6.94-6.88 (m, 1H), 5.35-5.07 (m, 1H), 4.04 (s, 2H), 2.27 (s, 3H), 1.71 (s, 9H).
10-223(#2): δ8.30 (s, 1H), 7.34-7.26 (m, 3H), 7.15-7.07 (m, 3H), 7.05-7.00 (m, 1H), 6.94-6.82 (m, 2H), 6.04-5.67 (m, 1H), 5.43-5.05 (m, 2H), 4.95-4.80 (m, 2H), 4.78-4.40 (m, 2H), 2.21 (s, 3H), 1.72 (s, 9H).
10-229(#2): δ8.36 (s, 1H), 7.28-7.20 (m, 1H), 7.18-7.11 (m, 1H), 7.04 (s, 1H), 6.95-6.88 (m, 1H), 6.86-6.82 (m, 1H), 6.81-6.77 (m, 1H), 6.76-6.70 (m, 2H), 5.73-5.24 (m, 1H), 4.62-4.16 (m, 1H), 2.17 (s, 3H), 2.15 (s, 3H), 1.73 (s, 9H).
10-230(#2): δ7.30-7.21 (m, 1H), 7.18 (s, 1H), 7.13-7.06 (m, 2H), 6.91-6.86 (m, 1H), 6.85-6.75 (m, 2H), 6.72-6.68 (m, 1H), 6.66-6.60 (m, 1H), 3.84 (s, 2H), 2.39 (s, 2H), 2.33 (s, 3H), 2.24 (s, 3H), 1.65 (s, 9H), -0.01 (s, 9H).
10-235(#2): δ8.18-7.91 (m, 1H), 7.73-7.55 (m, 1H), 7.53-7.42 (m, 1H), 7.24-6.62 (m, 6H), 4.98-4.55 (m, 2H), 4.00-3.07 (m, 3H), 2.33-2.13 (m, 6H), 1.71 (s, 9H).
10-239(#2): δ8.50-8.13 (m, 1H), 7.43-7.23 (m, 2H), 7.18-6.98 (m, 3H), 6.97-6.87 (m, 2H), 6.42-6.19 (m, 2H), 4.82-4.55 (m, 2H), 3.97-3.31 (m, 9H), 1.73 (s, 9H).
10-240(#2): δ8.48-8.11 (m, 1H), 7.42-7.24 (m, 2H), 7.23-7.03 (m, 3H), 7.00-6.89 (m, 2H), 6.58-6.35 (m, 2H), 4.83-4.65 (m, 2H), 3.91-3.39 (m, 6H), 1.72 (s, 9H).
10-241(#2): δ8.40-8.14 (m, 1H), 7.37-7.23 (m, 2H), 7.21-6.98 (m, 3H), 6.95-6.83 (m, 2H), 6.56-6.37 (m, 2H), 4.81-4.59 (m, 2H), 3.92-3.42 (m, 6H), 1.72 (s, 9H).
10-244(#2): δ8.47-8.12 (m, 1H), 7.41-7.23 (m, 2H), 7.22-7.01 (m, 3H), 6.98-6.84 (m, 2H), 6.83-6.61 (m, 2H), 4.87-4.67 (m, 2H), 3.94-3.30 (m, 3H), 2.25 (s, 3H), 1.72 (s, 9H).
10-245(#2): δ8.42-8.20 (m, 1H), 7.39-6.99 (m, 5H), 6.96-6.81 (m, 4H), 4.93-4.73 (m, 2H), 4.38-3.95 (m, 2H), 2.25-2.19 (m, 3H), 1.73 (s, 9H), 1.35-1.00 (m, 3H).
10-246(#2): δ8.36-8.24 (m, 1H), 7.43-7.36 (m, 1H), 7.35-7.22 (m, 3H), 7.18-7.04 (m, 3H), 6.93-6.84 (m, 2H), 4.86-4.75 (m, 2H), 4.32-4.01 (m, 2H), 1.73 (s, 9H), 1.33-1.01 (m, 3H).
10-247(#2): δ8.34-8.24 (m, 1H), 7.56-7.52 (m, 1H), 7.37-7.04 (m, 6H), 6.93-6.85 (m, 2H), 4.86-4.72 (m, 2H), 4.37-3.99 (m, 2H), 1.73 (s, 9H), 1.39-1.01 (m, 3H).
10-248(#2): δ8.47-8.13 (m, 1H), 7.38-7.21 (m, 2H), 7.19-6.98 (m, 3H), 6.97-6.83 (m, 2H), 6.42-6.20 (m, 2H), 4.78-4.54 (m, 2H), 4.33-3.94 (m, 2H), 3.74 (s, 3H), 3.66 (s, 3H), 1.73 (s, 9H), 1.43-0.96 (m, 3H).
10-249(#2): δ8.38-8.14 (m, 1H), 7.45-6.89 (m, 7H), 6.57-6.36 (m, 2H), 4.87-4.66 (m, 2H), 4.37-3.97 (m, 2H), 3.80-3.65 (m, 3H), 1.88-1.64 (m, 9H), 1.44-0.91 (m, 3H).
10-250(#2): δ8.40-8.16 (m, 1H), 7.35-7.22 (m, 2H), 7.20-7.00 (m, 3H), 6.96-6.84 (m, 2H), 6.55-6.39 (m, 2H), 4.78-4.58 (m, 2H), 4.34-3.96 (m, 2H), 3.66 (s, 3H), 1.73 (s, 9H), 1.44-0.99 (m, 3H).
10-251(#2): δ8.36-8.14 (m, 1H), 7.38-7.28 (m, 2H), 7.20-7.07 (m, 3H), 7.01-6.83 (m, 4H), 4.83-4.67 (m, 2H), 4.34-3.95 (m, 2H), 1.72 (s, 9H), 1.46-0.98 (m, 3H).
10-253(#2): δ8.38-8.14 (m, 1H), 7.39-7.23 (m, 2H), 7.21-7.04 (m, 3H), 6.99-6.83 (m, 2H), 6.82-6.64 (m, 2H), 4.86-4.66 (m, 2H), 4.38-3.92 (m, 2H), 2.27-2.22 (m, 3H), 1.72 (s, 9H), 1.42-0.95 (m, 3H).
10-254(#2): δ8.50-8.26 (m, 1H), 7.47-7.03 (m, 7H), 6.96-6.84 (m, 2H), 6.06-5.63 (m, 1H), 5.45-5.04 (m, 2H), 4.93-4.42 (m, 4H), 2.26-2.17 (m, 3H), 1.82-1.65 (m, 9H).
10-255(#2): δ8.40-8.30 (m, 1H), 7.41-7.21 (m, 3H), 7.18-7.04 (m, 4H), 6.92-6.84 (m, 2H), 6.04-5.66 (m, 1H), 5.46-5.07 (m, 2H), 4.93-4.43 (m, 4H), 1.72 (s, 9H).
10-256(#2): δ8.39-8.30 (m, 1H), 7.58-7.51 (m, 1H), 7.38-7.03 (m, 6H), 6.95-6.84 (m, 2H), 6.03-5.67 (m, 1H), 5.41-5.07 (m, 2H), 4.89-4.44 (m, 4H), 1.79-1.65 (m, 9H).
10-257(#2): δ8.42-8.23 (m, 1H), 7.34-7.25 (m, 2H), 7.16-7.04 (m, 3H), 6.97-6.88 (m, 2H), 6.36-6.20 (m, 2H), 6.02-5.65 (m, 1H), 5.38-5.03 (m, 2H), 4.76-4.39 (m, 4H), 3.79-3.60 (m, 6H), 1.73 (s, 9H).
10-258(#2): δ8.37-8.20 (m, 1H), 7.36-7.26 (m, 2H), 7.21-7.04 (m, 3H), 6.99-6.91 (m, 2H), 6.57-6.39 (m, 2H), 6.05-5.62 (m, 1H), 5.45-5.02 (m, 2H), 4.87-4.40 (m, 4H), 3.76-3.66 (m, 3H), 1.76-1.68 (m, 9H).
10-259(#2): δ8.37-8.23 (m, 1H), 7.34-7.26 (m, 2H), 7.16-7.05 (m, 3H), 6.95-6.86 (m, 2H), 6.51-6.39 (m, 2H), 6.01-5.67 (m, 1H), 5.41-5.03 (m, 2H), 4.78-4.41 (m, 4H), 3.66 (s, 3H), 1.72 (s, 9H).
10-260(#2): δ8.35-8.24 (m, 1H), 7.42-7.04 (m, 5H), 6.99-6.82 (m, 4H), 6.02-5.63 (m, 1H), 5.44-5.04 (m, 2H), 4.85-4.37 (m, 4H), 1.71 (s, 9H).
10-261(#2): δ8.23-8.07 (m, 1H), 7.34-7.25 (m, 2H), 7.17-6.99 (m, 3H), 6.90-6.82 (m, 2H), 6.77-6.70 (m, 1H), 6.62-6.53 (m, 1H), 6.04-5.66 (m, 1H), 5.40-5.07 (m, 2H), 4.83-4.42 (m, 4H), 2.27-2.18 (m, 3H), 1.71 (s, 9H).
10-262(#2): δ8.37-8.21 (m, 1H), 7.34-7.25 (m, 2H), 7.17-7.05 (m, 3H), 6.98-6.85 (m, 2H), 6.81-6.65 (m, 2H), 6.05-5.61 (m, 1H), 5.44-5.01 (m, 2H), 4.89-4.38 (m, 4H), 2.28-2.22 (m, 3H), 1.77-1.68 (m, 9H).
10-263(#2): δ8.13-7.91 (m, 1H), 7.53-6.90 (m, 9H), 5.27-5.09 (m, 1H), 4.30-3.95 (m, 2H), 2.38-2.15 (m, 3H), 1.91-1.50 (m, 9H).
10-264(#2): δ8.02-7.92 (m, 1H), 7.61-7.04 (m, 7H), 6.98-6.90 (m, 2H), 5.22-5.13 (m, 1H), 4.06-4.00 (m, 2H), 1.73-1.66 (m, 9H).
10-265(#2): δ7.99-7.92 (m, 1H), 7.66-7.61 (m, 1H), 7.36-7.04 (m, 6H), 6.96-6.89 (m, 2H), 5.22-5.13 (m, 1H), 4.07-3.98 (m, 2H), 1.70 (s, 9H).
10-266(#2): δ7.97-7.82 (m, 1H), 7.38-7.26 (m, 2H), 7.20-7.03 (m, 3H), 7.02-6.94 (m, 2H), 6.45-6.40 (m, 1H), 6.34-6.26 (m, 1H), 5.25-5.04 (m, 1H), 3.96-3.71 (m, 8H), 1.72-1.67 (m, 9H). 10-267(#2): δ8.12-7.92 (m, 1H), 7.48-6.93 (m, 7H), 6.73-6.49 (m, 2H), 5.18-4.99 (m, 1H), 4.09-3.68 (m, 5H), 1.86-1.68 (m, 9H).
10-268(#2): δ8.03-7.84 (m, 1H), 7.44-6.92 (m, 7H), 6.66-6.40 (m, 2H), 5.34-5.11 (m, 1H), 4.05-3.76 (m, 5H), 1.86-1.58 (m, 9H).
10-269(#2): δ8.11-7.93 (m, 1H), 7.46-6.88 (m, 9H), 5.19-5.03 (m, 1H), 4.06-3.91 (m, 2H), 1.84-1.62 (m, 9H).
10-270(#2): δ7.99-7.89 (m, 1H), 7.38-7.27 (m, 2H), 7.22-7.06 (m, 3H), 6.99-6.92 (m, 2H), 6.88-6.66 (m, 2H), 4.98-4.87 (m, 1H), 3.94-3.84 (m, 2H), 2.33 (s, 3H), 1.73 (s, 9H).
10-271(#2): δ8.03-7.93 (m, 1H), 7.39-7.24 (m, 3H), 7.20-7.01 (m, 2H), 7.01-6.66 (m, 4H), 5.15-5.02 (m, 1H), 4.02-3.94 (m, 2H), 2.29 (s, 3H), 1.71 (s, 9H).
10-274(#1): δ8.22-8.00 (m, 1H), 7.38-7.21 (m, 2H), 7.18-6.99 (m, 6H), 6.95-6.83 (m, 2H), 4.73-4.63 (m, 2H), 4.01-3.39 (m, 3H), 2.42 (s, 3H), 1.72 (s, 9H).
10-276(#1): δ8.31-8.08 (m, 1H), 7.62-7.00 (m, 13H), 6.97-6.81 (m, 2H), 4.86-4.71 (m, 2H), 4.00-3.42 (m, 3H), 1.84-1.64 (m, 9H).
10-277(#1): δ8.24-8.05 (m, 1H), 7.38-6.96 (m, 8H), 6.93-6.83 (m, 2H), 4.78-4.64 (m, 2H), 3.94-3.41 (m, 3H), 1.78-1.64 (m, 9H).
10-278(#1): δ8.30-8.09 (m, 1H), 7.54-7.39 (m, 2H), 7.36-7.05 (m, 6H), 6.94-6.82 (m, 2H), 4.83-4.65 (m, 2H), 4.01-3.42 (m, 3H), 1.82-1.63 (m, 9H).
10-280(#1): δ8.32-8.05 (m, 1H), 7.53-7.37 (m, 2H), 7.35-7.21 (m, 4H), 7.18-7.03 (m, 2H), 6.92-6.77 (m, 2H), 4.87-4.69 (m, 2H), 4.00-3.44 (m, 3H), 1.85-1.64 (m, 9H).
10-282(#1): δ8.27-8.01 (m, 1H), 7.36-7.23 (m, 2H), 7.17-6.96 (m, 6H), 6.95-6.85 (m, 2H), 4.75-4.62 (m, 2H), 4.35-3.99 (m, 2H), 2.57 (q, J = 7.6 Hz, 2H), 1.73 (s, 9H), 1.33-1.00 (m, 6H). 10-283(#1): δ8.23-8.04 (m, 1H), 7.38-7.22 (m, 2H), 7.18-6.99 (m, 6H), 6.97-6.86 (m, 2H), 4.75-4.60 (m, 2H), 4.36-3.92 (m, 2H), 2.83 (sep, 1H, J = 6.9 Hz), 1.73 (s, 9H), 1.44-0.95 (m, 9H).
10-285(#1): δ8.28-8.11 (m, 1H), 8.03-7.87 (m, 2H), 7.42-7.16 (m, 4H), 7.15-7.01 (m, 2H), 6.89-6.70 (m, 2H), 4.86-4.70 (m, 2H), 4.39-4.01 (m, 2H), 1.83-1.60 (m, 9H), 1.42-1.03 (m, 3H).
10-286(#1): δ8.30-8.10 (m, 1H), 7.61-7.18 (m, 11H), 7.16-7.00 (m, 2H), 6.94-6.83 (m, 2H), 4.84-4.70 (m, 2H), 4.35-4.02 (m, 2H), 1.84-1.68 (m, 9H), 1.43-1.01 (m, 3H).
10-287(#1): δ8.24-8.07 (m, 1H), 7.42-7.06 (m, 7H), 7.05-6.96 (m, 2H), 6.94-6.83 (m, 2H), 4.75-4.65 (m, 2H), 4.30-4.01 (m, 2H), 1.80-1.67 (m, 9H), 1.41-1.02 (m, 2H).
10-288(#1): δ8.27-8.05 (m, 1H), 7.53-6.97 (m, 8H), 6.93-6.75 (m, 2H), 4.81-4.58 (m, 2H), 4.34-3.98 (m, 2H), 1.82-1.56 (m, 9H), 1.40-0.98 (m, 3H).
10-289(#1): δ8.21-8.05 (m, 1H), 7.54-7.42 (m, 2H), 7.41-7.28 (m, 2H), 7.20-7.05 (m, 3H), 6.97-6.79 (m, 3H), 4.70-4.60 (m, 2H), 4.33-3.95 (m, 2H), 1.72 (s, 9H), 1.39-1.03 (m, 3H).
10-290(#1): δ8.24-8.09 (m, 1H), 7.51-7.37 (m, 2H), 7.35-7.22 (m, 4H), 7.17-7.05 (m, 2H), 6.91-6.79 (m, 2H), 4.83-4.71 (m, 2H), 4.32-4.04 (m, 2H), 1.79-1.66 (m, 9H), 1.39-1.04 (m, 3H).
10-291(#1): δ8.25-8.12 (m, 1H), 7.54-7.20 (m, 6H), 7.19-7.08 (m, 2H), 6.88-6.80 (m, 2H), 4.74 (s, 2H), 4.32-4.02 (m, 2H), 1.78-1.66 (m, 9H), 1.40-1.04 (m, 3H).
10-292(#1): δ8.28-8.07 (m, 1H), 7.35-7.21 (m, 3H), 7.17-6.96 (m, 5H), 6.94-6.83 (m, 2H), 6.07-4.99 (m, 3H), 4.82-4.39 (m, 4H), 2.57 (q, J = 7.7 Hz, 2H), 1.72 (s, 9H), 1.18 (t, J = 7.7 Hz, 3H).
10-293(#1): δ8.28-8.08 (m, 1H), 7.39-7.21 (m, 3H), 7.18-6.98 (m, 5H), 6.97-6.85 (m, 2H), 6.01-5.01 (m, 3H), 4.77-4.39 (m, 4H), 2.83 (sep, 1H, J = 7.1 Hz), 1.78-1.64 (m, 9H), 1.20 (d, J = 7.1 Hz, 6H).
10-294(#1): δ8.23-8.09 (m, 1H), 7.39-7.22 (m, 2H), 7.18-6.98 (m, 6H), 6.95-6.83 (m, 2H), 6.01-5.02 (m, 3H), 4.75-4.38 (m, 4H), 2.42 (s, 3H), 1.72 (s, 9H).
10-295(#1): δ8.31-8.19 (m, 1H), 8.03-7.92 (m, 2H), 7.39-7.20 (m, 4H), 7.18-7.04 (m, 2H), 6.86-6.76 (m, 2H), 6.01-5.67 (m, 1H), 5.41-5.10 (m, 2H), 4.87-4.47 (m, 4H), 1.81-1.62 (m, 9H). 10-296(#1): δ8.34-8.16 (m, 1H), 7.63-7.17 (m, 11H), 7.15-6.99 (m, 2H), 6.94-6.81 (m, 2H), 6.08-5.63 (m, 1H), 5.43-5.04 (m, 2H), 4.86-4.42 (m, 4H), 1.85-1.63 (m, 9H).
10-297(#1): δ8.28-8.06 (m, 1H), 7.47-6.74 (m, 10H), 6.05-5.61 (m, 1H), 5.41-5.03 (m, 2H), 4.81-4.35 (m, 4H), 1.82-1.56 (m, 9H).
10-298(#1): δ8.30-8.08 (m, 1H), 7.53-6.97 (m, 8H), 6.95-6.75 (m, 2H), 6.01-5.58 (m, 1H), 5.41-5.02 (m, 2H), 4.85-4.39 (m, 4H), 1.83-1.58 (m, 9H).
10-299(#1): δ8.28-8.07 (m, 1H), 7.54-7.41 (m, 2H), 7.40-7.21 (m, 2H), 7.19-7.00 (m, 2H), 6.96-6.75 (m, 4H), 6.06-5.60 (m, 1H), 5.47-5.03 (m, 2H), 4.78-4.41 (m, 4H), 1.84-1.63 (m, 9H). 10-300(#1): δ8.28-8.09 (m, 1H), 7.52-6.98 (m, 8H), 6.89-6.72 (m, 2H), 6.05-5.62 (m, 1H), 5.42-5.05 (m, 2H), 4.87-4.42 (m, 4H), 1.85-1.58 (m, 9H).
10-301(#1): δ8.30-8.15 (m, 1H), 7.54-7.21 (m, 6H), 7.21-7.07 (m, 2H), 6.90-6.79 (m, 2H), 6.05-5.63 (m, 1H), 5.43-5.06 (m, 2H), 4.83-4.47 (m, 4H), 1.83-1.63 (m, 9H).
10-302(#1): δ8.00-7.87 (m, 1H), 7.38-7.03 (m, 8H), 7.00-6.89 (m, 2H), 5.15-4.92 (m, 1H), 3.99-3.86 (m, 2H), 2.69-2.54 (m, 2H), 1.76-1.69 (m, 9H), 1.32-1.14 (m, 3H).
10-303(#1): δ7.99-7.89 (m, 1H), 7.36-7.18 (m, 4H), 7.18-7.03 (m, 4H), 6.99-6.91 (m, 2H), 5.21-4.87 (m, 1H), 3.96-3.85 (m, 2H), 2.87 (sep, 1H, J = 7.0 Hz), 1.72 (s, 9H), 1.22 (d, J = 7.0 Hz, 6H).
10-304(#1): δ7.97-7.85 (m, 1H), 7.37-7.02 (m, 8H), 6.98-6.89 (m, 2H), 5.12-4.96 (m, 1H), 3.94-3.87 (m, 2H), 2.45 (s, 3H), 1.77-1.69 (m, 9H).
10-305(#1): δ8.07-7.99 (m, 2H), 7.58-7.49 (m, 2H), 7.46-7.39 (m, 2H), 7.33-7.01 (m, 3H), 6.89-6.82 (m, 2H), 5.20-5.06 (m, 1H), 4.11-4.04 (m, 2H), 1.77-1.68 (m, 9H).
10-306(#1): δ8.05-7.91 (m, 1H), 7.63-7.22 (m, 10H), 7.18-7.00 (m, 3H), 6.99-6.88 (m, 2H), 5.20-5.01 (m, 1H), 4.06-3.95 (m, 2H), 1.79-1.69 (m, 9H).
10-307(#1): δ8.01-7.91 (m, 1H), 7.38-7.23 (m, 3H), 7.19-7.01 (m, 5H), 6.98-6.87 (m, 2H), 5.16-4.99 (m, 1H), 4.00-3.90 (m, 2H), 1.75-1.69 (m, 9H).
10-308(#1): δ8.01-7.91 (m, 1H), 7.55-7.48 (m, 2H), 7.38-7.25 (m, 4H), 7.19-7.03 (m, 2H), 6.95-6.86 (m, 2H), 5.20-5.00 (m, 1H), 4.04-3.93 (m, 2H), 1.76-1.69 (m, 9H).
10-309(#1): δ8.01-7.87 (m, 1H), 7.59-7.49 (m, 2H), 7.37-7.26 (m, 2H), 7.19-6.98 (m, 4H), 6.94-6.86 (m, 2H), 5.14-4.98 (m, 1H), 3.93-3.85 (m, 2H), 1.76-1.68 (m, 9H).
10-310(#1): δ8.01-7.90 (m, 1H), 7.53-7.44 (m, 2H), 7.43-7.35 (m, 2H), 7.34-7.22 (m, 2H), 7.19-7.02 (m, 2H), 6.94-6.83 (m, 2H), 5.20-5.03 (m, 1H), 4.07-3.96 (m, 2H), 1.76-1.68 (m, 9H). 10-311(#1): δ8.07-7.90 (m, 1H), 7.54-7.43 (m, 2H), 7.42-7.22 (m, 4H), 7.19-6.97 (m, 2H), 6.93-6.81 (m, 2H), 5.23-5.01 (m, 1H), 4.08-3.95 (m, 2H), 1.81-1.66 (m, 9H).
10-314(#1): δ8.07 (br, 1H), 7.23-7.03 (m, 4H), 6.74-6.61 (m, 3H), 6.51-6.44 (m, 2H), 4.65 (s, 2H), 3.91-3.42 (m, 9H), 1.72 (s, 9H).
10-316(#2): δ7.94 (br, 1H), 7.21-7.18 (m, 1H), 7.17-7.03 (m, 4H), 6.93-6.90 (m, 1H), 6.81-6.76 (m, 1H), 6.75-6.67 (m, 2H), 4.65 (s, 2H), 3.96 (q, J = 7.2 Hz, 2H), 3.91-3.45 (m, 3H), 1.72 (s, 9H), 1.40 (t, J = 7.2 Hz, 3H).
10-317(#2): δ8.07 (br, 1H), 7.23-7.04 (m, 4H), 6.75-6.64 (m, 3H), 6.52-6.45 (m, 2H), 4.65 (s, 2H), 3.94 (q, J = 7.2 Hz, 2H), 3.87-3.46 (m, 6H), 1.72 (s, 9H), 1.39 (t, J = 7.2 Hz, 3H).
10-319(#2): δ8.35 (s, 1H), 7.34-7.26 (m, 2H), 7.17-7.11 (m, 1H), 7.03 (s, 1H), 6.98-6.94 (m, 1H), 6.87-6.85 (m, 1H), 6.84-6.79 (m, 2H), 6.78-6.74 (m, 1H), 6.28-6.23 (m, 1H), 5.88-5.82 (m, 1H), 5.23-4.96 (m, 2H), 2.20 (s, 3H), 2.18 (s, 3H), 1.89 (s, 3H), 1.72 (s, 9H).
10-320(#2): δ8.31-8.06 (m, 1H), 7.35-6.81 (m, 10H), 4.79-4.60 (m, 2H), 4.02-3.39 (m, 3H), 2.24 (s, 3H), 1.73 (s, 9H).
10-322(#2): δ8.36-8.07 (m, 1H), 7.39-7.00 (m, 8H), 6.95-6.86 (m, 2H), 4.79-4.60 (m, 2H), 3.99-3.40 (m, 3H), 1.72 (s, 9H). 10-323(#2): δ8.46-8.22 (m, 1H), 7.46-7.20 (m, 4H), 7.19-7.04 (m, 4H), 6.96-6.84 (m, 2H), 5.00-4.77 (m, 2H), 4.02-3.38 (m, 3H), 1.72 (s, 9H).
10-324(#2): δ8.36-8.10 (m, 1H), 7.43-7.22 (m, 4H), 7.19-6.99 (m, 4H), 6.94-6.86 (m, 2H), 4.77-4.61 (m, 2H), 3.96-3.45 (m, 3H), 1.72 (s, 9H).
10-325(#2): δ8.47-8.21 (m, 1H), 7.49-6.98 (m, 8H), 6.95-6.82 (m, 2H), 4.97-4.74 (m, 2H), 3.99-3.41 (m, 3H), 1.72 (s, 9H). 10-326(#2): δ8.34-8.06 (m, 1H), 7.35-7.22 (m, 2H), 7.19-7.05 (m, 3H), 6.94-6.66 (m, 5H), 4.82-4.60 (m, 2H), 3.98-3.40 (m, 6H), 1.72 (s, 9H).
10-327(#2): δ8.46-8.20 (m, 1H), 7.44-7.04 (m, 5H), 7.00-6.72 (m, 5H), 4.83-4.64 (m, 2H), 3.88-3.41 (m, 6H), 1.73 (s, 9H).
10-329(#2): δ8.23-7.96 (m, 1H), 7.34-7.22 (m, 2H), 7.18-7.02 (m, 3H), 6.92-6.81 (m, 2H), 6.56-6.41 (m, 1H), 4.82-4.63 (m, 2H), 3.91-3.34 (m, 6H), 2.37-2.14 (m, 6H), 1.72 (s, 9H).
10-330(#2): δ8.22-8.04 (m, 1H), 7.36-7.23 (m, 2H), 7.15-7.05 (m, 2H), 6.93-6.55 (m, 5H), 4.73-4.61 (m, 2H), 3.88-3.46 (m, 9H), 1.73 (s, 9H).
10-331(#2): δ8.29-8.06 (m, 1H), 7.37-6.84 (m, 9H), 4.76-4.58 (m, 2H), 3.99-3.45 (m, 3H), 2.30-2.25 (m, 3H), 1.72 (s, 9H). 10-332(#2): δ8.31-8.11 (m, 1H), 7.50-6.92 (m, 7H), 6.91-6.83 (m, 2H), 4.73-4.59 (m, 2H), 3.97-3.42 (m, 3H), 1.72 (s, 9H).
10-333(#2): δ8.42-8.21 (m, 1H), 7.36-7.20 (m, 4H), 7.18-6.97 (m, 3H), 6.94-6.81 (m, 2H), 4.95-4.75 (m, 2H), 3.96-3.42 (m, 3H), 1.72 (s, 9H).
10-334(#2): δ8.22-8.01 (m, 1H), 7.34-7.21 (m, 2H), 7.19-7.04 (m, 2H), 7.02-6.89 (m, 3H), 6.89-6.77 (m, 2H), 4.88-4.65 (m, 2H), 3.97-3.35 (m, 3H), 2.25-2.13 (m, 6H), 1.72 (s, 9H).
10-335(#2): δ8.02-7.92 (m, 1H), 7.36-7.23 (m, 2H), 7.20-6.99 (m, 6H), 6.98-6.91 (m, 2H), 5.10-4.97 (m, 1H), 3.96-3.87 (m, 2H), 2.30 (s, 3H), 1.73 (s, 9H).
10-336(#2): δ7.98-7.89 (m, 1H), 7.38-7.04 (m, 8H), 7.02-6.93 (m, 2H), 5.02-4.92 (m, 1H), 3.98-3.89 (m, 1H), 2.34 (s, 3H), 1.74 (s, 10H).
10-337(#2): δ8.00-7.91 (m, 1H), 7.35-7.25 (m, 3H), 7.24-7.04 (m, 5H), 6.99-6.90 (m, 2H), 5.11-5.02 (m, 1H), 3.97-3.89 (m, 2H), 1.73 (s, 9H).
10-338(#2): δ8.07-7.93 (m, 1H), 7.45-6.91 (m, 10H), 5.27-5.11 (m, 1H), 4.18-4.02 (m, 2H), 1.87-1.63 (m, 9H).
10-339(#2): δ8.02-7.92 (m, 1H), 7.51-7.46 (m, 1H), 7.40-7.18 (m, 5H), 7.17-7.04 (m, 2H), 6.97-6.90 (m, 2H), 5.13-4.99 (m, 1H), 3.98-3.88 (m, 2H), 1.73 (s, 9H).
10-340(#2): δ8.08-7.93 (m, 1H), 7.62-7.45 (m, 1H), 7.43-6.91 (m, 9H), 5.28-5.12 (m, 1H), 4.19-4.02 (m, 2H), 1.90-1.66 (m, 9H).
10-341(#2): δ8.02-7.92 (m, 1H), 7.36-7.22 (m, 3H), 7.21-7.05 (m, 3H), 6.99-6.74 (m, 4H), 5.10-5.02 (m, 1H), 3.97-3.91 (m, 2H), 3.77 (s, 3H), 1.73 (s, 9H).
10-342(#2): δ7.96-7.87 (m, 1H), 7.36-7.04 (m, 6H), 7.03-6.76 (m, 4H), 5.29-5.18 (m, 1H), 4.00-3.94 (m, 2H), 3.83 (s, 3H), 1.69 (s, 9H).
10-343(#2): δ7.99-7.90 (m, 1H), 7.35-7.24 (m, 3H), 7.19-6.90 (m, 6H), 5.08-5.00 (m, 1H), 3.94-3.88 (m, 2H), 2.24-2.20 (m, 3H), 1.73 (s, 9H).
10-344(#2): δ8.00-7.89 (m, 1H), 7.40-7.24 (m, 2H), 7.22-6.92 (m, 5H), 6.69-6.57 (m, 1H), 4.95-4.78 (m, 1H), 3.94-3.77 (m, 5H), 2.17 (s, 3H), 2.11 (s, 3H), 1.82-1.70 (m, 9H).
10-345(#2): δ8.01-7.90 (m, 1H), 7.37-7.25 (m, 3H), 7.19-7.06 (m, 2H), 7.00-6.79 (m, 3H), 6.74-6.68 (m, 1H), 5.09-4.96 (m, 1H), 3.93-3.81 (m, 8H), 1.74 (s, 9H).
10-346(#2): δ8.00-7.92 (m, 1H), 7.41-7.23 (m, 4H), 7.20-7.02 (m, 4H), 6.98-6.90 (m, 1H), 5.10-4.98 (m, 1H), 3.94-3.88 (m, 2H), 2.32 (s, 3H), 1.79-1.70 (m, 9H).
10-347(#2): δ8.02-7.92 (m, 1H), 7.44-7.02 (m, 7H), 6.93-6.87 (m, 2H), 5.12-5.02 (m, 1H), 3.95-3.89 (m, 2H), 1.72 (s, 9H).
10-348(#2): δ8.03-7.95 (m, 1H), 7.38-7.21 (m, 4H), 7.18-7.01 (m, 3H), 6.98-6.90 (m, 2H), 5.23-5.14 (m, 1H), 4.12-4.06 (m, 2H), 1.70 (s, 9H).
10-349(#2): δ7.99-7.90 (m, 1H), 7.35-7.25 (m, 2H), 7.20-7.17 (m, 1H), 7.13-6.93 (m, 6H), 4.97-4.85 (m, 1H), 3.92-3.86 (m, 2H), 2.29 (s, 3H), 2.25 (s, 3H), 1.74 (s, 9H).
10-350(#2): δ8.34 (s, 1H), 7.35-7.26 (m, 2H), 7.16-7.09 (m, 2H), 6.99-6.93 (m, 1H), 6.90-6.81 (m, 3H), 6.79-6.72 (m, 1H), 5.80-4.00 (m, 2H), 2.20 (s, 3H), 2.18 (s, 3H), 1.90-1.82 (m, 1H), 1.73 (s, 9H), 1.01-0.93 (m, 2H), 0.60-0.50 (m, 2H).
10-351(#2): δ8.55 (s, 1H), 7.35-7.28 (m, 2H), 7.20-7.14 (m, 1H), 7.00 (s, 1H), 6.95-6.90 (m, 1H), 6.85-6.79 (m, 3H), 6.74-6.70 (m, 1H), 5.55-5.31 (m, 1H), 4.50-4.29 (m, 1H), 2.16 (s, 3H), 2.14 (s, 3H), 1.73 (s, 9H).
10-352(#2): δ8.37-8.29 (m, 1H), 7.43-6.65 (m, 14H), 5.00-4.92 (m, 1H), 3.94-3.77 (m, 1H), 2.32-2.27 (m, 3H), 2.21-2.12 (m, 3H), 1.72 (s, 9H).
10-353(#2): δ8.03-7.92 (m, 1H), 7.40-7.28 (m, 2H), 7.20-7.07 (m, 5H), 7.00-6.91 (m, 2H), 5.17-5.06 (m, 1H), 4.01-3.94 (m, 2H), 1.70 (s, 9H).
10-354(#2): δ7.98-7.92 (m, 1H), 7.36-7.21 (m, 4H), 7.18-7.02 (m, 2H), 6.99-6.93 (m, 2H), 6.88-6.81 (m, 1H), 5.21-5.09 (m, 1H), 4.06-4.01 (m, 2H), 1.71 (s, 9H).
10-355(#2): δ8.33-8.21 (m, 2H), 8.07-7.94 (m, 1H), 7.70-7.57 (m, 1H), 7.51-7.05 (m, 5H), 6.99-6.90 (m, 1H), 5.27-5.11 (m, 1H), 4.12-4.04 (m, 2H), 1.79 (s, 9H).
10-356(#2): δ8.85 (s, 1H), 8.21-8.09 (m, 1H), 7.36-7.26 (m, 2H), 7.19-7.07 (m, 2H), 6.98-6.90 (m, 2H), 6.85-6.77 (m, 1H), 6.72-6.68 (m, 1H), 6.59-6.53 (m, 1H), 5.07-4.97 (m, 1H), 4.13-4.06 (m, 2H), 2.27 (s, 3H), 1.73 (s, 9H).
10-357(#2): δ7.97-7.85 (m, 1H), 7.35-7.28 (m, 2H), 7.19-7.06 (m, 3H), 7.00-6.95 (m, 2H), 6.71-6.66 (m, 1H), 6.62-6.55 (m, 1H), 4.94-4.82 (m, 1H), 3.89-3.84 (m, 2H), 3.76 (s, 3H), 2.33 (s, 3H), 1.74 (s, 9H).
10-358(#2): δ7.99-7.88 (m, 1H), 7.35-7.25 (m, 2H), 7.17 (s, 1H), 7.12-7.02 (m, 2H), 7.00-6.93 (m, 2H), 6.93-6.89 (m, 1H), 4.95-4.82 (m, 1H), 3.89-3.83 (m, 2H), 2.28 (s, 3H), 2.19 (s, 3H), 2.15 (s, 3H), 1.74 (s, 9H).
10-359(#2): δ8.00-7.93 (m, 1H), 7.35-7.27 (m, 2H), 7.17-7.05 (m, 2H), 7.00-6.90 (m, 4H), 5.15-5.06 (m, 1H), 4.05-3.98 (m, 2H), 3.84 (s, 3H), 3.81 (s, 3H), 1.72 (s, 9H).
10-360(#2): δ7.98-7.87 (m, 1H), 7.36-7.28 (m, 2H), 7.24-7.18 (m, 1H), 7.15 (s, 1H), 7.14-7.04 (m, 2H), 7.01-6.94 (m, 2H), 6.70-6.65 (m, 1H), 5.18-5.04 (m, 1H), 4.03-3.97 (m, 2H), 3.75 (s, 3H), 1.71 (s, 9H).
10-361(#2): δ8.32-8.16 (m, 1H), 7.41-7.28 (m, 2H), 7.24-7.01 (m, 5H), 6.98-6.88 (m, 2H), 4.82-4.66 (m, 2H), 3.98-3.42 (m, 3H), 1.71 (s, 9H).
10-364(#2): δ8.42-7.88 (m, 1H), 7.39-7.32 (m, 1H), 7.32-7.23 (m, 1H), 7.21-6.96 (m, 4H), 6.94-6.88 (m, 1H), 6.87-6.69 (m, 2H), 4.90-4.55 (m, 2H), 3.92-3.39 (m, 4H), 2.35-2.15 (m, 3H), 1.79-1.68 (m, 9H).
10-365(#2): δ8.20-7.99 (m, 1H), 7.34-7.26 (m, 2H), 7.17-6.95 (m, 3H), 6.94-6.83 (m, 2H), 6.67-6.55 (m, 1H), 6.51-6.42 (m, 1H), 4.81-4.63 (m, 2H), 3.84-3.32 (m, 6H), 2.31-2.14 (m, 3H), 1.77-1.65 (m, 9H).
10-367(#2): δ8.40-8.20 (m, 1H), 7.35-7.23 (m, 2H), 7.15-7.04 (m, 2H), 6.98-6.87 (m, 3H), 6.86-6.75 (m, 1H), 4.90-4.73 (m, 2H), 3.93-3.42 (m, 9H), 1.72 (s, 9H).
10-368(#2): δ8.39-8.18 (m, 1H), 7.35-7.25 (m, 2H), 7.19-7.04 (m, 3H), 7.00-6.86 (m, 3H), 6.76-6.55 (m, 1H), 4.92-4.71 (m, 2H), 3.92-3.40 (m, 6H), 1.72 (s, 9H).
10-369(#2): δ8.34-8.17 (m, 1H), 7.41-7.28 (m, 3H), 7.23-7.00 (m, 4H), 6.97-6.88 (m, 2H), 4.81-4.66 (m, 2H), 4.37-3.95 (m, 2H), 1.72 (s, 9H), 1.40-0.98 (m, 3H).
10-370(#2): δ8.36-8.23 (m, 1H), 7.42-7.22 (m, 2H), 7.19-7.03 (m, 4H), 6.95-6.74 (m, 3H), 4.89-4.73 (m, 2H), 4.34-3.99 (m, 2H), 1.76-1.69 (m, 9H), 1.42-0.97 (m, 3H).
10-371(#2): δ8.38-8.19 (m, 2H), 7.50-7.08 (m, 6H), 6.96-6.86 (m, 2H), 4.89-4.75 (m, 2H), 4.40-3.98 (m, 2H), 1.72 (s, 9H), 1.46-0.94 (m, 3H).
10-372(#2): δ8.37-8.15 (m, 1H), 7.40-7.33 (m, 1H), 7.32-7.23 (m, 1H), 7.19-6.96 (m, 4H), 6.94-6.88 (m, 2H), 6.85-6.63 (m, 2H), 4.91-4.53 (m, 2H), 4.35-3.90 (m, 2H), 2.19 (s, 3H), 1.72 (s, 9H), 1.42-0.93 (m, 3H).
10-373(#2): δ8.20-8.00 (m, 1H), 7.36-7.24 (m, 2H), 7.17-7.06 (m, 2H), 7.04-6.97 (m, 1H), 6.93-6.83 (m, 2H), 6.65-6.55 (m, 1H), 6.49-6.41 (m, 1H), 4.82-4.61 (m, 2H), 4.35-3.97 (m, 2H), 3.70 (s, 3H), 2.28-2.18 (m, 3H), 1.73 (s, 9H), 1.42-0.98 (m, 3H).
10-375(#2): δ8.44-8.23 (m, 1H), 7.36-7.23 (m, 2H), 7.18-7.03 (m, 2H), 6.97-6.83 (m, 3H), 6.78 (s, 1H), 4.89-4.75 (m, 2H), 4.37-3.70 (m, 8H), 1.73 (s, 9H), 1.48-0.97 (m, 3H).
10-376(#2): δ8.43-8.21 (m, 1H), 7.36-7.23 (m, 2H), 7.19-7.04 (m, 3H), 6.99-6.88 (m, 3H), 6.75-6.58 (m, 1H), 4.89-4.74 (m, 2H), 4.36-3.99 (m, 2H), 3.69 (s, 3H), 1.73 (s, 9H), 1.44-0.97 (m, 3H).
10-377(#2): δ8.47-8.22 (m, 1H), 7.41-7.24 (m, 3H), 7.23-6.99 (m, 4H), 6.97-6.88 (m, 2H), 6.04-5.63 (m, 1H), 5.44-5.04 (m, 2H), 4.89-4.39 (m, 4H), 1.71 (s, 9H).
10-378(#2): δ8.40-8.29 (m, 1H), 7.41-7.22 (m, 3H), 7.20-6.99 (m, 3H), 6.95-6.74 (m, 3H), 6.03-5.66 (m, 1H), 5.43-5.07 (m, 2H), 4.92-4.43 (m, 4H), 1.72 (s, 9H).
10-379(#2): δ8.45-8.19 (m, 3H), 7.51-7.06 (m, 5H), 6.97-6.85 (m, 2H), 6.02-5.63 (m, 1H), 5.45-5.00 (m, 2H), 4.92-4.41 (m, 4H), 1.86-1.74 (m, 9H).
10-380(#2): δ8.45-8.24 (m, 1H), 7.41-7.24 (m, 3H), 7.21-6.97 (m, 4H), 6.96-6.63 (m, 2H), 6.49-6.39 (m, 1H), 6.01-5.59 (m, 1H), 5.44-5.03 (m, 2H), 4.88-4.36 (m, 4H), 2.25 (s, 3H), 1.76-1.68 (m, 9H).
10-381(#2): δ8.27-8.04 (m, 1H), 7.38-7.24 (m, 2H), 7.19-6.96 (m, 3H), 6.95-6.83 (m, 2H), 6.67-6.55 (m, 1H), 6.50-6.39 (m, 1H), 6.04-5.67 (m, 1H), 5.43-5.04 (m, 2H), 4.85-4.36 (m, 4H), 3.70 (s, 3H), 2.30-2.17 (m, 3H), 1.79-1.67 (m, 9H).
10-382(#2): δ8.34-8.06 (m, 1H), 7.33-7.22 (m, 2H), 7.17-7.03 (m, 2H), 6.95-6.76 (m, 4H), 6.04-5.60 (m, 1H), 5.44-5.03 (m, 2H), 4.85-4.39 (m, 4H), 2.25-2.00 (m, 9H), 1.72 (s, 9H).
10-383(#2): δ8.48-8.30 (m, 1H), 7.35-7.23 (m, 3H), 7.17-7.02 (m, 2H), 6.97-6.85 (m, 2H), 6.78 (s, 1H), 6.02-5.68 (m, 1H), 5.42-5.07 (m, 2H), 4.90-4.44 (m, 4H), 3.85-3.70 (m, 6H), 1.72 (s, 9H).
10-384(#2): δ8.40-8.26 (m, 1H), 7.36-7.02 (m, 6H), 6.97-6.85 (m, 2H), 6.73-6.57 (m, 1H), 6.03-5.65 (m, 1H), 5.42-5.05 (m, 2H), 4.89-4.41 (m, 4H), 3.69 (s, 3H), 1.72 (s, 9H).
10-385(#2): δ7.75-7.68 (m, 1H), 7.46-7.39 (m, 1H), 7.38-7.32 (m, 1H), 7.28-7.24 (m, 1H), 7.18 (s, 1H), 7.15-7.09 (m, 1H), 7.07-7.01 (m, 1H), 6.45-6.40 (m, 1H), 6.33-6.27 (m, 1H), 5.25-5.04 (m, 1H), 3.95-3.69 (m, 8H), 1.70 (s, 9H).
10-386(#2): δ7.86-7.75 (m, 1H), 7.47-7.40 (m, 1H), 7.39-7.34 (m, 1H), 7.28-7.24 (m, 1H), 7.21-7.11 (m, 3H), 6.58-6.50 (m, 2H), 5.11-5.01 (m, 1H), 3.99-3.92 (m, 2H), 3.75 (s, 3H), 1.71 (s, 9H).
10-387(#2): δ7.83-7.73 (m, 1H), 7.47-7.40 (m, 1H), 7.39-7.33 (m, 1H), 7.28-7.09 (m, 4H), 7.06-7.03 (m, 1H), 6.72-6.65 (m, 1H), 5.21-5.05 (m, 1H), 4.05-3.98 (m, 2H), 3.77-3.72 (m, 3H), 1.73-1.68 (m, 9H).
10-388(#2): δ7.84-7.71 (m, 1H), 7.49-7.33 (m, 2H), 7.30-7.24 (m, 1H), 7.23-7.09 (m, 3H), 6.76-6.55 (m, 2H), 5.00-4.82 (m, 1H), 3.91-3.85 (m, 2H), 3.82-3.73 (m, 3H), 2.40-2.30 (m, 3H), 1.81-1.70 (m, 9H).
10-390(#2): δ7.82-7.75 (m, 1H), 7.45-7.38 (m, 1H), 7.37-7.32 (m, 1H), 7.26-7.22 (m, 1H), 7.19 (s, 1H), 7.14-7.08 (m, 1H), 7.05-7.02 (m, 1H), 6.93-6.88 (m, 1H), 4.98-4.83 (m, 1H), 3.91-3.83 (m, 2H), 2.29-2.13 (m, 9H), 1.74 (s, 9H).
10-391(#2): δ7.87-7.74 (m, 1H), 7.47-7.39 (m, 1H), 7.39-7.33 (m, 1H), 7.28-7.24 (m, 1H), 7.19-7.10 (m, 2H), 6.93-6.89 (m, 1H), 6.88-6.84 (m, 1H), 5.95-5.89 (m, 2H), 5.21-5.05 (m, 1H), 4.02-3.93 (m, 2H), 1.71 (s, 9H).
10-392(#2): δ7.83-7.75 (m, 1H), 7.46-7.32 (m, 2H), 7.27-7.23 (m, 1H), 7.19-7.16 (m, 1H), 7.14-7.06 (m, 1H), 6.96-6.90 (m, 2H), 5.16-5.02 (m, 1H), 4.05-3.98 (m, 2H), 3.86-3.79 (m, 6H), 1.76-1.68 (m, 9H).
10-393(#2): δ7.82-7.74 (m, 1H), 7.48-7.40 (m, 1H), 7.40-7.34 (m, 1H), 7.30-7.25 (m, 1H), 7.20-7.11 (m, 2H), 6.99 (d, J = 8.4 Hz, 1H), 6.65 (d, J = 8.4 Hz, 1H), 5.18-5.08 (m, 1H), 4.05-3.98 (m, 2H), 3.90-3.80 (m, 6H), 1.70 (s, 9H).
10-394(#2): δ7.60-7.34 (m, 2H), 7.31-7.07 (m, 3H), 7.02-6.56 (m, 4H), 4.78-4.69 (m, 1H), 4.15-4.08 (m, 2H), 1.79 (s, 9H).
10-396(#2): δ8.71 (s, 1H), 7.38-7.11 (m, 2H), 7.24-7.14 (m, 2H), 7.06 (s, 1H), 6.97-6.92 (m, 2H), 6.70-6.63 (m, 2H), 5.37 (br, 1H), 4.48 (br, 1H), 4.30-3.90 (m, 2H), 3.68 (s, 3H), 1.72 (s, 9H).
10-397(#2): δ8.20-7.98 (m, 1H), 7.47-7.33 (m, 2H), 7.25-6.94 (m, 4H), 6.41-6.21 (m, 2H), 4.77-4.58 (m, 2H), 3.89-3.34 (m, 9H), 1.73 (s, 9H).
10-399(#2): δ8.14-8.03 (m, 1H), 7.49-7.33 (m, 2H), 7.24-7.05 (m, 4H), 6.98-6.87 (m, 1H), 6.74-6.60 (m, 1H), 4.89-4.75 (m, 2H), 3.91-3.40 (m, 6H), 1.73 (s, 9H).
10-405(#2): δ8.22-8.03 (m, 1H), 7.49-7.35 (m, 2H), 7.26-6.95 (m, 4H), 6.73-6.55 (m, 1H), 4.88-4.73 (m, 2H), 3.94-3.37 (m, 9H), 1.76-1.68 (m, 9H).
10-406(#2): δ8.24-7.97 (m, 1H), 7.46-7.31 (m, 2H), 7.25-7.00 (m, 4H), 6.38-6.21 (m, 2H), 4.78-4.56 (m, 2H), 4.30-3.64 (m, 8H), 1.73 (s, 9H), 1.42-0.90 (m, 3H).
10-408(#2): δ8.16-8.05 (m, 1H), 7.49-7.34 (m, 2H), 7.23-7.17 (m, 1H), 7.16-7.05 (m, 3H), 6.94-6.89 (m, 1H), 6.72-6.59 (m, 1H), 4.87-4.75 (m, 2H), 4.40-3.97 (m, 2H), 3.69 (s, 3H), 1.73 (s, 9H), 1.46-0.97 (m, 3H).
10-412(#2): δ8.17-8.04 (m, 1H), 7.49-7.35 (m, 2H), 7.25-7.19 (m, 1H), 7.18-7.07 (m, 2H), 6.89-6.73 (m, 2H), 5.88 (s, 2H), 4.89-4.68 (m, 2H), 4.40-3.94 (m, 2H), 1.74 (s, 9H), 1.45-0.96 (m, 3H).
10-414(#2): δ8.22-8.06 (m, 1H), 7.49-7.33 (m, 2H), 7.27-7.21 (m, 1H), 7.18-7.02 (m, 3H), 6.70-6.52 (m, 1H), 4.87-4.73 (m, 2H), 4.35-3.93 (m, 2H), 3.92-3.73 (m, 6H), 1.78-1.69 (m, 9H), 1.44-0.92 (m, 3H).
10-415(#2): δ8.23-8.03 (m, 1H), 7.46-7.33 (m, 2H), 7.23-7.17 (m, 1H), 7.17-6.92 (m, 3H), 6.39-6.17 (m, 2H), 6.03-5.60 (m, 1H), 5.42-4.99 (m, 2H), 4.82-4.38 (m, 4H), 3.78-3.63 (m, 6H), 1.82-1.63 (m, 9H).
10-416(#2): δ8.21-8.02 (m, 1H), 7.51-7.33 (m, 2H), 7.27-6.99 (m, 4H), 6.59-6.36 (m, 2H), 6.05-5.59 (m, 1H), 5.45-4.99 (m, 2H), 4.85-4.35 (m, 4H), 3.71 (s, 3H), 1.72 (s, 9H).
10-417(#2): δ.20-8.07 (m, 1H), 7.50-7.33 (m, 2H), 7.26-7.03 (m, 4H), 6.97-6.86 (m, 1H), 6.75-6.56 (m, 1H), 6.05-5.62 (m, 1H), 5.44-5.00 (m, 2H), 4.93-4.40 (m, 4H), 3.69 (s, 3H), 1.72 (s, 9H).
10-419(#2): δ8.09-7.98 (m, 1H), 7.49-7.36 (m, 2H), 7.25-7.16 (m, 3H), 7.14 (s, 1H), 7.09-6.98 (m, 3H), 6.07-5.62 (m, 1H), 5.45-5.03 (m, 2H), 4.83-4.37 (m, 4H), 1.71 (s, 9H).
10-420(#2): δ8.11-7.89 (m, 1H), 7.46-7.31 (m, 2H), 7.17-6.93 (m, 3H), 6.92-6.74 (m, 2H), 6.03-5.60 (m, 1H), 5.43-4.99 (m, 2H), 4.88-4.38 (m, 4H), 2.25-2.01 (m, 9H), 1.73 (s, 9H).
10-421(#2): δ8.20-8.08 (m, 1H), 7.50-7.34 (m, 2H), 7.25-7.18 (m, 1H), 7.16-7.05 (m, 2H), 6.90-6.68 (m, 2H), 6.02-5.62 (m, 3H), 5.44-5.01 (m, 2H), 4.87-4.38 (m, 4H), 1.79-1.63 (m, 9H). 10-423(#2): δ8.21-8.11 (m, 1H), 7.50-7.33 (m, 2H), 7.26-7.19 (m, 1H), 7.16-6.84 (m, 3H), 6.72-6.51 (m, 1H), 6.04-5.58 (m, 1H), 5.46-4.99 (m, 2H), 4.90-4.35 (m, 4H), 3.92-3.71 (m, 6H), 1.81-1.63 (m, 9H).
10-424(#2): δ7.33-7.25 (m, 2H), 7.17 (s, 1H), 7.10-7.03 (m, 2H), 6.98-6.91 (m, 4H), 6.86-6.80 (m, 1H), 3.83 (m, 2H), 2.54-2.48 (m, 2H), 2.36 (s, 3H), 2.25 (s, 3H), 1.81-1.20 (m, 20H).
10-425(#2): δ8.01-7.93 (m, 1H), 7.37-7.22 (m, 3H), 7.21-7.06 (m, 4H), 7.03-6.95 (m, 2H), 6.83-6.78 (m, 1H), 6.59-6.50 (m, 1H), 4.01-3.95 (m, 2H), 1.70 (s, 9H).
10-426(#2): δ8.36-8.18 (m, 1H), 7.39-6.88 (m, 7H), 6.82-6.70 (m, 1H), 6.67-6.49 (m, 1H), 4.90-4.73 (m, 2H), 4.06-3.23 (m, 5H), 1.77-1.64 (m, 9H), 1.38 (t, J = 7.0 Hz, 3H).
10-431(#2): δ8.02 (br, 1H), 7.23-7.09 (m, 2H), 7.04-6.94 (m, 2H), 6.90 (s, 1H), 6.82-6.72 (m, 2H), 6.62-6.57 (m, 1H), 4.92-4.55 (m, 2H), 3.94-3.35 (m, 3H), 2.44 (s, 3H), 2.32-2.15 (m, 6H), 1.72 (s, 9H).
10-432(#1): δ9.03 (s, 1H), 8.30-8.22 (m, 1H), 7.88-7.80 (m, 2H), 7.53-7.37 (m, 3H), 7.35-7.19 (m, 4H), 7.13-6.96 (m, 2H), 5.05 (s, 2H), 1.43 (s, 9H).
10-433(#1): δ8.78-8.72 (m, 1H), 8.68-8.60 (m, 1H), 8.54-8.46 (m, 1H), 7.93-7.88 (m, 1H), 7.52-7.40 (m, 2H), 7.34-7.12 (m, 5H), 7.06-6.95 (m, 2H), 4.97 (br, 2H), 1.49 (s, 9H).
10-437(#1): δ7.95 (br, 1H), 7.48-7.39 (m, 1H), 7.37-7.31 (m, 1H), 7.24-7.18 (m, 1H), 7.13 (s, 1H), 7.00-6.87 (m, 3H), 6.81-6.75 (m, 1H), 4.73 (s, 2H), 3.99-3.29 (m, 3H), 2.71 (s, 3H), 2.30-2.16 (m, 6H), 1.72 (s, 9H).
10-438(#1): δ7.87 (br, 1H), 7.71-7.62 (m, 1H), 7.53-7.42 (m, 2H), 7.14 (s, 1H), 7.13-7.06 (m, 1H), 6.99-6.93 (m, 1H), 6.90 (s, 1H), 6.82-6.74 (m, 1H), 4.74 (s, 2H), 3.96-3.42 (m, 3H), 3.05 (s, 3H), 2.28-2.16 (m, 6H), 1.72 (s, 9H).
10-439(#2): δ8.45-8.20 (m, 1H), 7.42-7.23 (m, 4H), 7.19-7.05 (m, 3H), 7.01-6.86 (m, 3H), 4.93-4.72 (m, 2H), 4.00-3.41 (m, 3H), 1.80-1.66 (m, 9H).
10-440(#1): δ8.23 (br, 1H), 7.24-7.04 (m, 3H), 6.95-6.87 (m, 1H), 6.79-6.69 (m, 2H), 6.56-6.39 (m, 2H), 4.74 (s, 2H), 3.81-3.35 (m, 6H), 2.32 (s, 3H), 1.72 (s, 9H).
10-441(#1): δ8.27 (br, 1H), 7.28-7.04 (m, 3H), 6.96-6.87 (m, 1H), 6.80-6.68 (m, 3H), 6.66-6.55 (m, 1H), 4.81 (s, 2H), 3.85-3.45 (m, 6H), 2.31 (s, 3H), 1.72 (s, 9H).
10-444(#1): δ8.74-8.38 (m, 2H), 7.93-7.76 (m, 1H), 7.64-7.55 (m, 1H), 7.45-7.28 (m, 4H), 7.24-6.99 (m, 4H), 6.65-6.37 (m, 2H), 4.74 (s, 2H), 3.97-3.38 (m, 6H).
10-445(#2): δ7.77-7.68 (m, 1H), 7.45-7.32 (m, 2H), 7.29-7.22 (m, 1H), 7.21-7.06 (m, 2H), 7.04-6.99 (m, 1H), 6.68-6.63 (m, 1H), 6.62-6.56 (m, 1H), 5.31-5.06 (m, 1H), 3.96-3.90 (m, 2H), 3.81 (s, 3H), 2.31 (s, 3H), 1.69 (s, 9H).
10-446(#2): δ7.93-7.81 (m, 1H), 7.37-7.22 (m, 2H), 7.21-7.01 (m, 3H), 6.99-6.93 (m, 2H), 6.68-6.64 (m, 1H), 6.63-6.56 (m, 1H), 5.24-5.12 (m, 1H), 3.96-3.90 (m, 2H), 3.82 (s, 3H), 2.31 (s, 3H), 1.69 (s, 9H).
10-447(#2): δ8.14 (br, 1H), 7.53-7.34 (m, 9H), 7.19-7.00 (m, 3H), 6.75-6.60 (m, 2H), 4.63 (s, 2H), 3.90-3.54 (m, 6H).
10-449(#2): δ8.70-8.28 (m, 2H), 7.93-7.79 (m, 1H), 7.68-7.56 (m, 1H), 7.43 (s, 1H), 7.39-7.30 (m, 3H), 7.24-7.13 (m, 3H), 7.04-6.98 (m, 2H), 6.77-6.65 (m, 2H), 4.67 (s, 2H), 3.91-3.48 (m, 6H).
10-450(#2): δ8.16 (br, 1H), 7.24-7.07 (m, 4H), 6.96-6.89 (m, 1H), 6.76-6.64 (m, 4H), 4.66 (s, 2H), 3.86-3.47 (m, 6H), 2.32 (s, 3H), 1.72 (s, 9H).
10-451(#2): δ8.24-8.01 (m, 1H), 7.48-7.31 (m, 2H), 7.23-6.86 (m, 4H), 6.69-6.49 (m, 2H), 4.81-4.59 (m, 2H), 3.91-3.34 (m, 6H), 2.27 (s, 3H), 1.73 (s, 9H).
10-452(#2): δ8.31-8.03 (m, 1H), 7.49-7.31 (m, 2H), 7.23-6.91 (m, 4H), 6.69-6.52 (m, 2H), 4.81-4.59 (m, 2H), 4.39-3.90 (m, 2H), 3.68 (s, 3H), 2.26 (s, 3H), 1.73 (s, 9H), 1.38-0.92 (m, 3H).
10-453(#2): δ8.36-8.04 (m, 1H), 7.49-7.30 (m, 2H), 7.23-6.85 (m, 4H), 6.69-6.47 (m, 2H), 6.11-5.55 (m, 1H), 5.48-4.99 (m, 2H), 4.82-4.22 (m, 4H), 3.68 (s, 3H), 2.26 (s, 3H), 1.73 (s, 9H).
10-454(#2): δ8.46-8.17 (m, 1H), 7.35-7.22 (m, 2H), 7.20-6.85 (m, 5H), 6.69-6.51 (m, 2H), 4.80-4.58 (m, 2H), 3.93-3.37 (m, 6H), 2.28 (s, 3H), 1.73 (s, 9H).
10-455(#2): δ8.45-8.20 (m, 1H), 7.34-7.21 (m, 2H), 7.18-6.85 (m, 5H), 6.68-6.52 (m, 2H), 4.78-4.60 (m, 2H), 4.30-3.95 (m, 2H), 3.68 (s, 3H), 2.28 (s, 3H), 1.73 (s, 9H), 1.39-0.96 (m, 3H).
10-456(#2): δ8.47-8.22 (m, 1H), 7.35-7.23 (m, 2H), 7.18-6.84 (m, 5H), 6.67-6.51 (m, 2H), 6.02-5.63 (m, 1H), 5.42-5.03 (m, 2H), 4.80-4.38 (m, 4H), 3.67 (s, 3H), 2.28 (s, 3H), 1.72 (s, 9H).
10-457(#2): δ9.00-8.55 (m, 1H), 8.52-8.29 (m, 1H), 7.96-7.83 (m, 1H), 7.76-7.64 (m, 1H), 7.55-7.08 (m, 8H), 6.82-6.66 (m, 2H), 4.68 (s, 2H), 3.97-3.42 (m, 3H), 3.74 (s, 3H).
10-458(#2): δ9.21-8.70 (m, 1H), 8.56-8.37 (m, 1H), 8.01-7.83 (m, 1H), 7.80-7.65 (m, 1H), 7.57-7.14 (m, 7H), 6.74-6.38 (m, 2H), 4.75 (s, 2H), 3.99-3.43 (m, 3H), 3.72 (s, 3H).
10-460(#1): δ8.30-8.16 (m, 1H), 7.27-6.90 (m, 6H), 6.58-6.40 (m, 2H), 4.82-4.69 (m, 2H), 3.88-3.43 (m, 6H), 1.78-1.64 (m, 9H).
10-461(#1): δ8.21-8.05 (m, 1H), 7.22-6.90 (m, 7H), 6.74-6.63 (m, 2H), 4.78-4.59 (m, 2H), 3.96-3.48 (m, 6H), 1.76-1.64 (m, 9H).
10-463(#1): δ8.18-7.99 (m, 1H), 7.30-7.06 (m, 2H), 7.05-6.84 (m, 2H), 6.82-6.67 (m, 1H), 6.58-6.37 (m, 2H), 4.87-4.65 (m, 2H), 3.95-3.42 (m, 6H), 1.82-1.65 (m, 9H).
10-464(#1): δ8.04-7.92 (m, 1H), 7.18-7.04 (m, 3H), 7.03-6.86 (m, 2H), 6.78-6.64 (m, 3H), 4.77-4.59 (m, 2H), 3.94-3.48 (m, 6H), 1.75-1.66 (m, 9H).
10-465(#1): δ8.19-8.01 (m, 1H), 7.14-6.40 (m, 7H), 4.86-4.71 (m, 2H), 3.99-3.44 (m, 6H), 2.37-2.11 (m, 6H), 1.75-1.61 (m, 9H).
10-466(#1): δ8.28-8.15 (m, 1H), 7.26-6.34 (m, 7H), 4.85-4.69 (m, 2H), 4.01-3.42 (m, 9H), 1.80-1.64 (m, 9H).
10-467(#1): δ8.20-8.06 (m, 1H), 7.30-6.40 (m, 8H), 4.77-4.61 (m, 2H), 3.99-3.47 (m, 9H), 1.79-1.63 (m, 9H).
10-468(#1): δ8.17-8.00 (m, 1H), 7.99-7.92 (m, 1H), 7.56-7.33 (m, 3H), 7.31-7.05 (m, 3H), 6.66-6.51 (m, 1H), 4.74-4.58 (m, 2H), 3.97-3.28 (m, 6H), 1.85-1.60 (m, 9H).
10-469(#1): δ8.14-7.91 (m, 2H), 7.52-7.31 (m, 3H), 7.29-7.20 (m, 1H), 7.17-7.06 (m, 2H), 6.65-6.52 (m, 1H), 4.76-4.59 (m, 2H), 4.29-3.93 (m, 2H), 3.85 (s, 3H), 1.82-1.66 (m, 9H), 1.43-0.88 (m, 3H).
10-470(#1): δ8.12-7.93 (m, 2H), 7.57-7.36 (m, 3H), 7.30-7.22 (m, 1H), 7.19-7.06 (m, 2H), 6.65-6.52 (m, 1H), 6.04-5.58 (m, 1H), 5.44-5.00 (m, 2H), 4.79-4.35 (m, 4H), 3.87 (s, 3H), 1.82-1.66 (m, 9H).
10-472(#1): δ8.20-8.01 (m, 1H), 7.24-6.83 (m, 4H), 6.78-6.57 (m, 3H), 6.52-6.42 (m, 1H), 4.80-4.63 (m, 2H), 3.89-3.43 (m, 6H), 2.29-2.16 (m, 6H), 1.79-1.66 (m, 9H).
10-473(#1): δ8.24-8.05 (m, 1H), 7.30-7.03 (m, 3H), 7.02-6.83 (m, 2H), 6.79-6.61 (m, 4H), 4.72-4.59 (m, 2H), 3.93 (q, J = 7.1 Hz, 2H), 3.87-3.41 (m, 3H), 2.37-2.28 (m, 3H), 1.78-1.69 (m, 9H), 1.39 (t, J = 7.1 Hz, 3H).
10-475(#1): δ8.23-8.04 (m, 1H), 7.31-7.02 (m, 4H), 7.00-6.83 (m, 2H), 6.79-6.63 (m, 4H), 4.72-4.59 (m, 2H), 3.88-3.45 (m, 3H), 2.32 (s, 3H), 1.79-1.67 (m, 9H).
10-477(#1): δ8.49-8.20 (m, 1H), 7.21-6.83 (m, 4H), 6.78-6.66 (m, 2H), 6.66-6.53 (m, 2H), 4.78-4.61 (m, 2H), 4.33-3.97 (m, 2H), 3.67 (s, 3H), 2.42-2.24 (m, 6H), 1.87-1.68 (m, 9H), 1.44-0.94 (m, 3H).
10-478(#1): δ8.34-8.14 (m, 1H), 7.23-7.02 (m, 3H), 6.98-6.86 (m, 1H), 6.83-6.68 (m, 2H), 6.59-6.37 (m, 2H), 4.81-4.67 (m, 2H), 4.31-3.95 (m, 2H), 3.71 (s, 3H), 2.32 (s, 3H), 1.80-1.64 (m, 9H), 1.41-0.97 (m, 3H).
10-479(#1): δ8.36-8.20 (m, 1H), 7.24-7.03 (m, 3H), 6.99-6.87 (m, 1H), 6.83-6.53 (m, 4H), 4.86-4.76 (m, 2H), 4.39-3.98 (m, 2H), 3.70 (s, 3H), 2.31 (s, 3H), 1.73 (s, 9H), 1.43-0.96 (m, 3H).
10-480(#1): δ8.16-8.05 (m, 1H), 7.24-6.86 (m, 4H), 6.78-6.57 (m, 3H), 6.49-6.40 (m, 1H), 4.78-4.68 (m, 2H), 4.32-3.99 (m, 2H), 3.70 (s, 3H), 2.39-2.17 (m, 6H), 1.73 (s, 9H), 1.46-0.96 (m, 3H).
10-481(#1): δ8.23-8.04 (m, 1H), 7.28-7.03 (m, 4H), 6.98-6.86 (m, 1H), 6.78-6.63 (m, 4H), 4.73-4.58 (m, 2H), 4.32-3.99 (m, 2H), 3.72 (s, 3H), 2.32 (s, 3H), 1.78-1.69 (m, 9H), 1.42-0.96 (m, 3H).
10-482(#1): δ8.20-8.06 (m, 1H), 7.27-7.03 (m, 4H), 6.99-6.83 (m, 1H), 6.78-6.61 (m, 4H), 4.69-4.61 (m, 2H), 4.30-4.01 (m, 2H), 3.93 (q, J = 7.2 Hz, 2H), 2.38-2.27 (m, 3H), 1.78-1.65 (m, 9H), 1.54-0.94 (m, 6H).
10-483(#1): δ8.21-8.10 (m, 1H), 7.34-7.02 (m, 6H), 6.98-6.88 (m, 1H), 6.75-6.58 (m, 2H), 4.72-4.65 (m, 2H), 4.30-4.03 (m, 2H), 2.38-2.27 (m, 3H), 1.79-1.67 (m, 9H), 1.45-1.01 (m, 3H). 10-484(#1): δ8.23-8.08 (m, 1H), 7.50-7.05 (m, 4H), 7.01-6.84 (m, 3H), 6.80-6.08 (m, 3H), 4.76-4.64 (m, 2H), 4.37-3.99 (m, 2H), 2.39-2.27 (m, 3H), 1.80-1.67 (m, 9H), 1.40-0.99 (m, 3H). 10-486(#1): δ8.11-7.99 (m, 1H), 7.49-7.29 (m, 2H), 7.28-7.04 (m, 4H), 6.57-6.36 (m, 2H), 5.04-4.89 (m, 1H), 4.77-4.65 (m, 2H), 3.70 (s, 3H), 1.73 (s, 9H), 1.47-0.96 (m, 6H).
10-487(#1): δ8.14-8.04 (m, 1H), 7.51-7.32 (m, 2H), 7.24-7.05 (m, 4H), 6.76-6.68 (m, 1H), 6.65-6.52 (m, 1H), 5.06-4.90 (m, 1H), 4.85-4.74 (m, 2H), 3.69 (s, 3H), 1.73 (s, 9H), 1.43-1.00 (m, 6H).
10-488(#1): δ7.96-7.88 (m, 1H), 7.46-7.33 (m, 2H), 7.20-7.14 (m, 1H), 7.12-6.94 (m, 3H), 6.62-6.55 (m, 1H), 6.47-6.37 (m, 1H), 5.06-4.90 (m, 1H), 4.80-4.65 (m, 2H), 3.68 (s, 3H), 2.21 (s, 3H), 1.73 (s, 9H), 1.40-1.05 (m, 6H).
10-492(#2): δ8.24-8.20 (m, 1H), 8.12 (br, 1H), 7.63-7.43 (m, 2H), 7.43-7.36 (m, 1H), 7.25-7.13 (m, 3H), 7.12-7.06 (m, 1H), 4.72 (s, 2H), 4.00-3.35 (m, 3H), 1.70 (s, 9H).
10-494(#2): δ7.80 (br, 1H), 7.48-7.41 (m, 1H), 7.39-7.33 (m, 1H), 7.31-7.27 (m, 1H), 7.21 (s, 1H), 7.18-7.14 (m, 1H), 4.62 (br, 1H), 3.72-3.64 (m, 5H), 2.21 (s, 3H), 2.19 (s, 3H), 1.74 (s, 9H).
10-498(#1): δ8.32-8.09 (m, 1H), 8.02-7.94 (m, 1H), 7.54-7.41 (m, 1H), 7.36-7.29 (m, 2H), 7.17-7.08 (m, 2H), 6.97-6.89 (m, 2H), 6.64-6.52 (m, 1H), 4.65 (s, 2H), 3.88 (s, 3H), 3.75-3.24 (m, 3H), 1.71 (s, 9H).
10-499(#1): δ8.28 (br, 1H), 8.23-8.16 (m, 1H), 7.62-7.42 (m, 1H), 7.24-7.16 (m, 1H), 7.15-7.08 (m, 2H), 6.97-6.90 (m, 1H), 6.77-6.66 (m, 2H), 4.69 (s, 2H), 3.99-3.36 (m, 3H), 2.34 (s, 3H), 1.70 (s, 9H).
10-501(#1): δ8.48 (s, 1H), 8.45-8.36 (m, 1H), 7.95-7.86 (m, 2H), 7.65-7.36 (m, 5H), 7.28-7.18 (m, 2H), 7.11 (s, 1H), 7.08-7.00 (m, 1H), 6.93-6.84 (m, 2H), 4.74 (s, 2H), 4.02-3.28 (m, 3H), 1.70 (s, 9H).
10-502(#1): δ8.30-8.24 (m, 1H), 8.19 (br, 1H), 7.48-7.23 (m, 3H), 7.15-7.06 (m, 2H), 6.99-6.85 (m, 3H), 4.66 (s, 2H), 4.04-3.30 (m, 3H), 2.02-1.90 (m, 1H), 1.70 (s, 9H), 1.01-0.92 (m, 4H).
10-503(#1): δ8.00-7.91 (m, 1H), 7.49-7.32 (m, 2H), 7.22-7.14 (m, 1H), 7.12-6.94 (m, 3H), 6.63-6.55 (m, 1H), 6.48-6.40 (m, 1H), 4.81-4.67 (m, 2H), 4.24-3.88 (m, 2H), 3.69 (s, 3H), 2.22 (s, 3H), 1.72 (s, 9H), 1.61-1.55 (m, 2H), 1.07-0.71 (m, 3H).
10-504(#1): δ8.04-7.94 (m, 1H), 7.51-7.33 (m, 2H), 7.21-7.13 (m, 1H), 7.12-6.93 (m, 3H), 6.63-6.55 (m, 1H), 6.50-6.41 (m, 1H), 4.80-4.67 (m, 2H), 4.04-3.77 (m, 2H), 3.69 (s, 3H), 2.22 (s, 3H), 2.02-1.93 (m, 1H), 1.73 (s, 9H), 1.07-0.70 (m, 6H).
10-505(#1): δ8.05-7.93 (m, 1H), 7.53-6.89 (m, 11H), 6.61-6.52 (m, 1H), 6.47-6.35 (m, 1H), 5.31-5.02 (m, 2H), 4.84-4.65 (m, 2H), 3.67 (s, 3H), 2.29-2.07 (m, 3H), 1.90-1.52 (m, 9H).
10-506(#1): δ8.14-8.07 (m, 1H), 7.50-6.78 (m, 10H), 6.68-6.59 (m, 1H), 6.54-6.43 (m, 1H), 4.98-4.73 (m, 2H), 3.71 (s, 3H), 2.28 (s, 3H), 1.82-1.63 (m, 9H).
10-507(#1): δ8.23-8.15 (m, 1H), 7.49-7.34 (m, 2H), 7.20-6.91 (m, 4H), 6.60-6.53 (m, 1H), 6.50-6.40 (m, 1H), 3.86-3.61 (m, 5H), 2.19 (s, 3H), 1.92-1.78 (m, 1H), 1.73 (s, 9H), 1.15-0.77 (m, 4H).
10-510(#1): δ8.13-8.06 (m, 1H), 7.52-7.32 (m, 3H), 7.24-7.16 (m, 1H), 7.14-7.05 (m, 2H), 6.77-6.69 (m, 1H), 6.67-6.53 (m, 1H), 4.86-4.77 (m, 2H), 4.22-3.88 (m, 2H), 3.70 (s, 3H), 1.80-1.68 (m, 11H), 1.07-0.70 (m, 3H).
10-511(#1): δ8.22-8.06 (m, 1H), 7.54-7.33 (m, 3H), 7.23-7.03 (m, 3H), 6.76-6.71 (m, 1H), 6.68-6.52 (m, 1H), 4.89-4.78 (m, 2H), 4.05-3.64 (m, 5H), 2.03-1.90 (m, 1H), 1.81-1.68 (m, 9H), 1.05-0.66 (m, 6H).
10-512(#1): δ8.20-8.08 (m, 1H), 7.54-6.88 (m, 11H), 6.75-6.48 (m, 2H), 5.35-5.03 (m, 2H), 4.95-4.75 (m, 2H), 3.68 (s, 3H), 1.90-1.50 (m, 9H).
10-513(#1): δ8.32-8.23 (m, 1H), 7.50-6.98 (m, 10H), 6.91-6.73 (m, 2H), 5.03-4.81 (m, 2H), 3.71 (s, 3H), 1.82-1.67 (m, 9H).
10-514(#1): δ8.47-8.31 (m, 1H), 7.52-7.04 (m, 6H), 6.76-6.58 (m, 2H), 3.68 (s, 3H), 1.91-1.77 (m, 1H), 1.73 (s, 9H), 1.15-0.75 (m, 4H).
10-519(#1): δ8.62-8.54 (m, 1H), 7.53-7.40 (m, 2H), 7.25-7.03 (m, 4H), 6.58-6.48 (m, 1H), 6.36-6.27 (m, 1H), 5.43-5.20 (m, 1H), 4.55-4.32 (m, 1H), 3.66 (s, 3H), 1.73 (s, 9H).
10-520(#1): δ8.60-8.51 (m, 1H), 7.54-7.38 (m, 2H), 7.30-7.05 (m, 4H), 6.58-6.49 (m, 1H), 6.38-6.28 (m, 1H), 6.25-5.84 (m, 1H), 5.37-5.12 (m, 1H), 4.55-4.32 (m, 1H), 3.67 (s, 3H), 1.73 (s, 9H).
10-522(#1): δ8.58-8.50 (m, 1H), 7.50-7.43 (m, 2H), 7.21-7.01 (m, 4H), 6.65-6.56 (m, 2H), 5.52-5.25 (m, 1H), 4.71-4.39 (m, 1H), 3.64 (s, 3H), 1.73 (s, 9H).
10-523(#1): δ8.58-8.49 (m, 1H), 7.53-7.39 (m, 2H), 7.24-7.03 (m, 4H), 6.66-6.57 (m, 2H), 6.29-5.86 (m, 1H), 5.46-5.23 (m, 1H), 4.69-4.40 (m, 1H), 3.65 (s, 3H), 1.72 (s, 9H).
10-525(#1): δ8.37-8.30 (m, 1H), 7.47-7.39 (m, 2H), 7.14-6.90 (m, 4H), 6.54-6.37 (m, 2H), 5.52-5.27 (m, 1H), 4.51-4.24 (m, 1H), 3.62 (s, 3H), 2.17 (s, 3H), 1.73 (s, 9H).
10-526(#1): δ8.41-8.30 (m, 1H), 7.50-7.39 (m, 2H), 7.21-6.90 (m, 4H), 6.56-6.50 (m, 1H), 6.48-6.36 (m, 1H), 6.30-5.85 (m, 1H), 5.59-5.16 (m, 1H), 4.59-4.19 (m, 1H), 3.64 (s, 3H), 2.18 (s, 3H), 1.72 (s, 9H).
10-528(#1): δ8.80-8.70 (m, 1H), 7.42-7.25 (m, 2H), 7.23-6.87 (m, 5H), 6.57-6.45 (m, 1H), 6.37-6.27 (m, 1H), 5.47-5.18 (m, 1H), 4.60-4.31 (m, 1H), 3.66 (s, 3H), 1.72 (s, 9H).
10-531(#1): δ8.79-8.69 (m, 1H), 7.39-7.25 (m, 2H), 7.23-7.11 (m, 2H), 7.01 (s, 1H), 6.96-6.88 (m, 2H), 6.68-6.56 (m, 2H), 5.54-5.24 (m, 1H), 4.71-4.40 (m, 1H), 3.65 (s, 3H), 1.72 (s, 9H).
10-532(#1): δ8.75-8.66 (m, 1H), 7.41-7.13 (m, 4H), 7.03 (s, 1H), 6.98-6.88 (m, 2H), 6.71-6.56 (m, 2H), 6.29-5.85 (m, 1H), 5.43-5.19 (m, 1H), 4.70-4.39 (m, 1H), 3.66 (s, 3H), 1.71 (s, 9H).
10-534(#2): δ8.59-8.52 (m, 1H), 7.37-7.27 (m, 2H), 7.23-7.13 (m, 1H), 7.03-6.94 (m, 2H), 6.89-6.82 (m, 2H), 6.56-6.51 (m, 1H), 6.48-6.40 (m, 1H), 5.53-5.28 (m, 1H), 4.48-4.28 (m, 1H), 3.65 (s, 3H), 2.17 (s, 3H), 1.73 (s, 9H).
10-535(#2): δ8.55-8.48 (m, 1H), 7.39-7.28 (m, 2H), 7.23-7.14 (m, 1H), 7.02 (s, 1H), 6.99-6.92 (m, 1H), 6.92-6.83 (m, 2H), 6.58-6.52 (m, 1H), 6.49-6.40 (m, 1H), 6.25-5.92 (m, 1H), 5.52-5.15 (m, 1H), 4.51-4.20 (m, 1H), 3.66 (s, 3H), 2.19 (s, 3H), 1.72 (s, 9H).
10-536(#1): δ8.13-8.05 (m, 1H), 7.52-7.30 (m, 3H), 7.28-7.04 (m, 3H), 6.57-6.37 (m, 2H), 4.80-4.69 (m, 2H), 4.28-3.86 (m, 2H), 3.71 (s, 3H), 1.82-1.69 (m, 9H), 1.61-1.31 (m, 2H), 1.08-0.57 (m, 3H).
10-537(#1): δ8.16-8.08 (m, 1H), 7.50-7.31 (m, 2H), 7.27-7.08 (m, 4H), 6.58-6.36 (m, 2H), 4.81-4.70 (m, 2H), 4.03-3.66 (m, 5H), 2.05-1.91 (m, 1H), 1.81-1.69 (m, 9H), 1.10-0.67 (m, 6H). 10-538(#1): δ8.18-8.06 (m, 1H), 7.50-6.94 (m, 11H), 6.53-6.34 (m, 2H), 5.31-5.00 (m, 2H), 4.82-4.67 (m, 2H), 3.70 (s, 3H), 1.84-1.55 (m, 9H).
10-539(#1): δ8.30-8.21 (m, 1H), 7.51-6.97 (m, 8H), 6.89-6.71 (m, 2H), 6.63-6.41 (m, 2H), 4.93-4.74 (m, 2H), 3.73 (s, 3H), 1.82-1.64 (m, 9H).
10-540(#1): δ8.39-8.30 (m, 1H), 7.51-7.34 (m, 2H), 7.25-7.03 (m, 4H), 6.62-6.46 (m, 1H), 6.45-6.32 (m, 1H), 3.70 (s, 3H), 1.89-1.67 (m, 10H), 1.15-0.77 (m, 4H).
10-545(#2): δ8.31-8.12 (m, 1H), 7.43-7.05 (m, 6H), 7.02-6.82 (m, 4H), 6.64-6.20 (m, 1H), 4.76-4.63 (m, 2H), 3.97-3.40 (m, 3H), 1.71 (s, 9H).
10-546(#2): δ8.28-8.06 (m, 1H), 7.40-7.05 (m, 6H), 6.97-6.84 (m, 4H), 6.63-6.19 (m, 1H), 4.77-4.62 (m, 2H), 4.35-3.95 (m, 2H), 1.72 (s, 9H), 1.39-1.00 (m, 3H).
10-547(#2): δ8.28-8.07 (m, 1H), 7.41-7.24 (m, 3H), 7.21-7.04 (m, 3H), 6.96-6.84 (m, 2H), 6.79-6.64 (m, 2H), 4.75-4.59 (m, 2H), 4.30-4.17 (m, 2H), 4.04-3.43 (m, 3H), 1.72 (s, 9H).
10-549(#2): δ8.33-8.02 (m, 1H), 7.40-7.22 (m, 2H), 7.19-7.02 (m, 4H), 6.97-6.86 (m, 2H), 6.74-6.61 (m, 2H), 6.22-5.85 (m, 1H), 4.74-4.59 (m, 2H), 4.23-3.38 (m, 5H), 1.71 (s, 9H).
10-550(#2): δ8.25-8.07 (m, 1H), 7.38-7.24 (m, 2H), 7.18-7.03 (m, 4H), 6.96-6.86 (m, 2H), 6.74-6.61 (m, 2H), 6.22-5.87 (m, 1H), 4.73-4.59 (m, 2H), 4.31-3.95 (m, 4H), 1.72 (s, 9H), 1.45-0.95 (m, 3H).
10-551(#2): δ8.24-8.07 (m, 1H), 7.43-7.03 (m, 6H), 6.97-6.85 (m, 2H), 6.77-6.61 (m, 2H), 6.24-5.88 (m, 1H), 4.77-4.60 (m, 2H), 4.31-4.16 (m, 2H), 3.98-3.41 (m, 3H), 1.72 (s, 9H).
10-555(#2): δ8.10-7.90 (m, 1H), 7.57-7.32 (m, 2H), 7.24-6.99 (m, 5H), 6.82-6.64 (m, 2H), 4.77-4.60 (m, 2H), 4.32-4.19 (m, 2H), 3.98-3.37 (m, 3H), 1.72 (s, 9H).
10-556(#2): δ8.11-7.93 (m, 1H), 7.56-7.01 (m, 7H), 6.79-6.66 (m, 2H), 4.74-4.60 (m, 2H), 4.40-3.96 (m, 4H), 1.73 (s, 9H), 1.46-0.97 (m, 3H).
10-558(#2): δ8.11-7.95 (m, 1H), 7.52-7.33 (m, 2H), 7.24-7.01 (m, 5H), 6.76-6.64 (m, 2H), 6.26-5.87 (m, 1H), 4.76-4.59 (m, 2H), 4.40-3.98 (m, 4H), 1.73 (s, 9H), 1.46-0.98 (m, 3H).
10-559(#2): δ8.11-7.93 (m, 1H), 7.50-7.34 (m, 2H), 7.24-6.99 (m, 5H), 6.80-6.66 (m, 2H), 6.24-5.86 (m, 1H), 4.75-4.62 (m, 2H), 4.31-4.18 (m, 2H), 4.02-3.42 (m, 3H), 1.72 (s, 9H).
10-560(#2): δ8.11-7.92 (m, 1H), 7.51-7.34 (m, 2H), 7.23-7.00 (m, 5H), 6.78-6.65 (m, 2H), 6.24-5.87 (m, 1H), 4.76-4.60 (m, 2H), 4.45-3.94 (m, 4H), 1.73 (s, 9H), 1.45-0.94 (m, 3H).
10-561(#2): δ7.47-7.06 (m, 7H), 6.69-6.42 (m, 2H), 3.95-3.90 (m, 2H), 3.73 (s, 3H), 2.57 (s, 3H), 1.66 (s, 9H).
10-562(#2): δ7.47-6.99 (m, 7H), 6.59-6.50 (m, 1H), 6.49-6.41 (m, 1H), 5.97-5.77 (m, 1H), 5.16-4.95 (m, 2H), 3.98 (s, 2H), 3.72 (s, 3H), 3.51-3.44 (m, 2H), 1.66 (s, 9H).
10-563(#2): δ7.67-7.59 (m, 1H), 7.52-7.14 (m, 6H), 6.59-6.43 (m, 2H), 4.04 (s, 2H), 3.89 (s, 2H), 3.72 (s, 3H), 1.65 (s, 9H). 10-564(#2): δ7.60-7.54 (m, 1H), 7.49-7.05 (m, 6H), 6.58-6.42 (m, 2H), 5.98-5.62 (m, 1H), 4.15 (s, 2H), 3.71 (s, 3H), 3.36-3.24 (m, 2H), 1.70 (s, 9H).
10-565(#2): δ8.26-8.16 (m, 1H), 7.50-7.39 (m, 3H), 7.33-7.08 (m, 2H), 7.00-6.89 (m, 2H), 6.72-6.61 (m, 2H), 4.82-4.71 (m, 2H), 3.92-3.54 (m, 3H), 2.38-2.28 (m, 3H), 1.71 (s, 9H).
10-566(#1): δ8.22-7.98 (m, 1H), 7.24-6.98 (m, 4H), 6.88-6.83 (m, 1H), 6.82-6.73 (m, 1H), 6.66-6.54 (m, 2H), 4.80-4.65 (m, 2H), 4.33-3.94 (m, 2H), 3.68 (s, 3H), 2.29 (s, 3H), 1.73 (s, 9H), 1.37-0.98 (m, 3H).
10-567(#1): δ8.13-7.97 (m, 1H), 7.29-7.02 (m, 4H), 6.97-6.90 (m, 1H), 6.88-6.79 (m, 1H), 6.61-6.37 (m, 2H), 4.79-4.68 (m, 2H), 4.34-3.98 (m, 2H), 3.73 (s, 3H), 1.72 (s, 9H), 1.43-0.83 (m, 3H).
10-568(#1): δ8.14-8.03 (m, 1H), 7.24-7.03 (m, 4H), 6.92-6.87 (m, 1H), 6.82-6.73 (m, 2H), 6.68-6.55 (m, 1H), 4.86-4.74 (m, 2H), 4.34-4.00 (m, 2H), 3.72 (s, 3H), 1.72 (s, 9H), 1.42-0.93 (m, 3H).
10-571(#1): δ8.00-7.90 (m, 1H), 7.28-7.17 (m, 2H), 7.16-7.02 (m, 3H), 6.95-6.88 (m, 1H), 6.84-6.65 (m, 3H), 4.72-4.61 (m, 2H), 4.30-3.89 (m, 4H), 1.72 (s, 9H), 1.40 (t, J = 6.8 Hz, 3H), 1.40-1.00 (m, 3H).
10-572(#1): δ8.02-7.92 (m, 1H), 7.30-7.03 (m, 7H), 6.91-6.84 (m, 1H), 6.79-6.68 (m, 1H), 4.73-4.65 (m, 2H), 4.31-4.03 (m, 2H), 1.72 (s, 9H), 1.41-1.03 (m, 3H).
10-573(#1): δ8.03-7.92 (m, 1H), 7.25-7.06 (m, 5H), 6.98-6.87 (m, 3H), 6.82-6.73 (m, 1H), 6.71-6.17 (m, 1H), 4.73-4.63 (m, 2H), 4.33-4.01 (m, 2H), 1.72 (s, 9H), 1.41-0.99 (m, 3H).
10-574(#1): δ8.00-7.92 (m, 1H), 7.38-7.04 (m, 4H), 6.97-6.66 (m, 6H), 6.29-5.83 (m, 2H), 4.71-4.62 (m, 2H), 4.27-3.96 (m, 2H), 1.72 (s, 9H), 1.46-0.99 (m, 3H).
10-575(#1): δ8.00-7.91 (m, 1H), 7.26-7.06 (m, 5H), 6.92-6.86 (m, 1H), 6.80-6.68 (m, 3H), 6.27-5.83 (m, 1H), 4.73-4.62 (m, 2H), 4.35-4.00 (m, 4H), 1.72 (s, 9H), 1.45-0.99 (m, 3H).
10-577(#1): δ7.50-7.12 (m, 5H), 7.07-6.98 (m, 2H), 6.80-6.74 (m, 1H), 6.69-6.61 (m, 1H), 4.00-3.95 (m, 2H), 3.71 (s, 3H), 2.64 (s, 3H), 1.65 (s, 9H).
10-578(#1): δ7.52-6.97 (m, 7H), 6.80-6.71 (m, 1H), 6.68-6.60 (m, 1H), 5.97-5.73 (m, 1H), 5.19-4.96 (m, 2H), 4.06-4.01 (m, 2H), 3.70 (s, 3H), 3.58-3.51 (m, 2H), 1.65 (s, 9H).
10-579(#1): δ7.70-7.59 (m, 1H), 7.53-7.08 (m, 6H), 6.84-6.76 (m, 1H), 6.71-6.60 (m, 1H), 4.15-4.10 (m, 2H), 3.94-3.90 (m, 2H), 3.71 (s, 3H), 1.66 (s, 9H).
10-580(#1): δ7.88-7.79 (m, 1H), 7.56-7.02 (m, 6H), 6.77-6.71 (m, 1H), 6.65-6.57 (m, 1H), 4.34-4.28 (m, 2H), 3.75-3.59 (m, 5H), 1.71 (s, 9H).
10-581(#1): δ7.65-7.54 (m, 1H), 7.51-7.00 (m, 6H), 6.79-6.73 (m, 1H), 6.67-6.56 (m, 1H), 6.04-5.58 (m, 1H), 4.26-4.21 (m, 2H), 3.70 (s, 3H), 3.49-3.30 (m, 2H), 1.69 (s, 9H).
10-582(#1): δ7.49-6.91 (m, 7H), 6.66-6.60 (m, 1H), 6.58-6.48 (m, 1H), 3.84-3.80 (m, 2H), 3.72 (s, 3H), 2.56 (s, 3H), 2.33 (s, 3H), 1.65 (s, 9H).
10-583(#1): δ7.46-6.95 (m, 7H), 6.64-6.59 (m, 1H), 6.55-6.47 (m, 1H), 5.91-5.73 (m, 1H), 5.15-4.94 (m, 2H), 3.92-3.88 (m, 2H), 3.71 (s, 3H), 3.51-3.43 (m, 2H), 2.33 (s, 3H), 1.65 (s, 9H).
10-584(#1): δ7.58-7.01 (m, 7H), 6.74-6.65 (m, 1H), 6.60-6.52 (m, 1H), 3.96-3.91 (m, 2H), 3.79-3.70 (m, 5H), 2.35 (s, 3H), 1.67 (s, 9H).
10-585(#1): δ7.80-7.71 (m, 1H), 7.47-6.99 (m, 6H), 6.64-6.57 (m, 1H), 6.55-6.44 (m, 1H), 4.24-4.17 (m, 2H), 3.70-3.56 (m, 5H), 2.29 (s, 3H), 1.71 (s, 9H).
10-586(#1): δ7.58-6.99 (m, 7H), 6.66-6.59 (m, 1H), 6.55-6.45 (m, 1H), 5.93-5.45 (m, 1H), 4.12-4.06 (m, 2H), 3.70 (s, 3H), 3.40-3.23 (m, 2H), 2.30 (s, 3H), 1.69 (s, 9H).
10-587(#1): δ7.38-7.00 (m, 6H), 6.98-6.89 (m, 2H), 6.58-6.43 (m, 2H), 3.93-3.87 (m, 2H), 3.73 (s, 3H), 2.54 (s, 3H), 1.66 (s, 9H).
10-588(#1): δ7.38-7.22 (m, 3H), 7.20-7.03 (m, 3H), 6.98-6.87 (m, 2H), 6.60-6.41 (m, 2H), 5.96-5.74 (m, 1H), 5.17-4.98 (m, 2H), 3.98-3.93 (m, 2H), 3.72 (s, 3H), 3.48-3.40 (m, 2H), 1.65 (s, 9H).
10-589(#1): δ7.80-7.71 (m, 1H), 7.40-6.96 (m, 7H), 6.62-6.41 (m, 2H), 4.04-3.98 (m, 2H), 3.86-3.80 (m, 2H), 3.72 (s, 3H), 1.65 (s, 9H).
10-590(#1): δ8.01-7.93 (m, 1H), 7.40-7.02 (m, 5H), 6.98-6.87 (m, 2H), 6.58-6.40 (m, 2H), 4.28-4.20 (m, 2H), 3.70 (s, 3H), 3.69-3.53 (m, 2H), 1.71 (s, 9H).
10-591(#1): δ7.69-7.59 (m, 1H), 7.39-7.00 (m, 5H), 6.98-6.87 (m, 2H), 6.57-6.41 (m, 2H), 5.98-5.50 (m, 1H), 4.17-4.10 (m, 2H), 3.72 (s, 3H), 3.33-3.18 (m, 2H), 1.69 (s, 9H).
10-592(#1): δ7.43-7.23 (m, 3H), 7.18-7.01 (m, 3H), 6.95-6.88 (m, 2H), 6.83-6.77 (m, 1H), 6.68-6.61 (m, 1H), 3.99-3.94 (m, 2H), 3.72 (s, 3H), 2.60 (s, 3H), 1.65 (s, 9H).
10-593(#1): δ7.46-7.38 (m, **1H),** 7.34-7.00 (m, 5H), 6.95-6.88 (m, 2H), 6.82-6.75 (m, **1H),** 6.70-6.61 (m, 1H), 6.01-5.79 (m, 1H), 5.20-4.99 (m, 2H), 4.05-4.00 (m, 2H), 3.72 (s, 3H), 3.55-3.49 (m, 2H), 1.64 (s, 9H).
10-597(#2): δ7.36-7.22 (m, 2H), 7.19 (s, 1H), 7.16-7.01 (m, 3H), 6.98-6.89 (m, 2H), 6.68-6.62 (m, **1H),** 6.59-6.50 (m, 1H), 3.81-3.76 (m, 2H), 3.73 (s, 3H), 2.53 (s, 3H), 2.34 (s, 3H), 1.66 (s, 9H).
10-598(#2): δ7.33-7.24 (m, 2H), 7.18-7.03 (m, 4H), 6.96-6.90 (m, 2H), 6.66-6.61 (m, **1H),** 6.55-6.48 (m, 1H), 5.89-5.76 (m, 1H), 5.16-4.98 (m, 2H), 3.86 (s, 2H), 3.73 (s, 3H), 3.50-3.43 (m, 2H), 2.34 (s, 3H), 1.65 (s, 9H).
10-599(#2): δ7.73-7.63 (m, 1H), 7.41-6.95 (m, 7H), 6.71-6.66 (m, 1H), 6.63-6.53 (m, 1H), 3.92-3.87 (m, 2H), 3.81-3.65 (m, 5H), 2.36 (s, 3H), 1.68 (s, 9H).
10-600(#2): δ7.95-7.87 (m, 1H), 7.33-7.25 (m, 2H), 7.15-7.03 (m, 3H), 6.95-6.86 (m, 2H), 6.65-6.60 (m, 1H), 6.54-6.48 (m, 1H), 4.21-4.16 (m, 2H), 3.71 (s, 3H), 3.65-3.54 (m, 2H), 2.29 (s, 3H), 1.72 (s, 9H).
10-601(#2): δ7.65-7.56 (m, 1H), 7.37-7.23 (m, 2H), 7.15 (s, 1H), 7.14-7.01 (m, 2H), 6.96-6.88 (m, 2H), 6.67-6.63 (m, 1H), 6.57-6.49 (m, 1H), 5.83-5.48 (m, 1H), 4.09-4.06 (m, 2H), 3.73 (s, 3H), 3.34-3.21 (m, 2H), 2.31 (s, 3H), 1.69 (s, 9H).
10-603(#1): δ8.10-7.96 (m, 1H), 7.31-7.03 (m, 4H), 6.97-6.90 (m, 1H), 6.87-6.75 (m, 1H), 6.59-6.40 (m, 2H), 4.78-4.67 (m, 2H), 3.86-3.42 (m, 6H), 1.72 (s, 9H).
10-606(#1): δ8.04-7.89 (m, 1H), 7.29-7.00 (m, 7H), 6.93-6.85 (m, 1H), 6.77-6.68 (m, 1H), 4.75-4.64 (m, 2H), 4.06-3.43 (m, 3H), 1.71 (s, 9H).
10-608(#1): δ8.03-7.89 (m, 1H), 7.27-7.02 (m, 5H), 6.93-6.86 (m, 1H), 6.83-6.66 (m, 3H), 6.29-5.85 (m, 1H), 4.71-4.61 (m, 2H), 4.18-4.04 (m, 2H), 3.95-3.43 (m, 3H), 1.72 (s, 9H).
10-609(#1): δ8.04-7.87 (m, 1H), 7.24-7.01 (m, 5H), 6.94-6.86 (m, 1H), 6.83-6.66 (m, 3H), 6.28-5.81 (m, 1H), 4.75-4.61 (m, 2H), 4.37-4.20 (m, 2H), 4.01-3.39 (m, 3H), 1.72 (s, 9H).
10-610(#1): δ8.37-8.13 (m, 2H), 7.58-7.45 (m, 1H), 7.25-7.06 (m, 3H), 6.99-6.88 (m, 1H), 6.80-6.66 (m, 2H), 4.78-4.63 (m, 2H), 4.28-4.00 (m, 2H), 2.42-2.30 (m, 3H), 1.81-1.64 (m, 9H), 1.49-0.96 (m, 3H).
10-611(#1): δ8.24-8.11 (m, 1H), 8.00-7.93 (m, 1H), 7.52-7.39 (m, 1H), 7.23-7.08 (m, 2H), 6.97-6.89 (m, 1H), 6.82-6.68 (m, 2H), 6.61-6.52 (m, 1H), 4.70-4.61 (m, 2H), 4.29-3.99 (m, 2H), 3.87 (s, 3H), 2.33 (s, 3H), 1.71 (s, 9H), 1.43-1.00 (m, 3H).
10-612(#1): δ8.50-8.22 (m, 1H), 7.23-7.09 (m, 2H), 6.96-6.88 (m, 1H), 6.84-6.69 (m, 2H), 6.03-5.94 (m, 1H), 5.79-5.70 (m, 1H), 4.78-4.58 (m, 2H), 4.41-3.94 (m, 2H), 2.32 (s, 3H), 2.14 (s, 3H), 1.74 (s, 9H), 1.43-1.00 (m, 3H).
10-613(#1): δ8.41-8.20 (m, 1H), 7.23-7.11 (m, 2H), 6.96-6.89 (m, 1H), 6.84-6.69 (m, 2H), 6.08-5.95 (m, 1H), 5.80-5.71 (m, 1H), 4.75-4.59 (m, 2H), 3.91-3.41 (m, 3H), 2.33 (s, 3H), 2.15 (s, 3H), 1.73 (s, 9H).
10-614(#1): δ8.86-8.73 (m, 1H), 8.08-7.94 (m, 1H), 7.28-6.93 (m, 6H), 6.87-6.76 (m, 2H), 4.85-4.64 (m, 2H), 3.92-3.45 (m, 6H), 1.73 (s, 9H).
10-615(#1): δ8.85-8.74 (m, 1H), 8.08-7.98 (m, 1H), 7.30-6.98 (m, 6H), 6.87-6.76 (m, 2H), 4.79-4.66 (m, 2H), 4.32-4.00 (m, 2H), 3.77 (s, 3H), 1.73 (s, 9H), 1.42-1.01 (m, 3H).
10-616(#1): δ8.79-8.67 (m, 1H), 8.32-8.20 (m, 1H), 7.58-7.43 (m, 1H), 7.34-6.99 (m, 5H), 6.88-6.74 (m, 2H), 4.87-4.68 (m, 2H), 3.89-3.48 (m, 3H), 1.73 (s, 9H).
10-617(#1): δ8.80-8.69 (m, 1H), 8.29-8.20 (m, 1H), 7.52-7.43 (m, 1H), 7.32-6.99 (m, 5H), 6.84-6.71 (m, 2H), 4.85-4.69 (m, 2H), 4.30-4.01 (m, 2H), 1.73 (s, 9H), 1.37-0.98 (m, 3H).
10-620(#1): δ8.77-8.63 (m, 1H), 8.32-8.21 (m, 1H), 7.59-7.46 (m, 1H), 7.41-6.94 (m, 6H), 4.88-4.67 (m, 2H), 3.95-3.48 (m, 3H), 1.73 (s, 9H).
10-622(#1): δ8.28-8.02 (m, 2H), 7.62-7.45 (m, 1H), 7.30-7.05 (m, 4H), 6.97-6.87 (m, 1H), 6.83-6.75 (m, 1H), 4.75-4.62 (m, 2H), 3.89-3.46 (m, 3H), 1.69 (s, 9H).
10-624(#1): δ8.21-8.00 (m, 1H), 7.27-7.04 (m, 3H), 7.03-6.95 (m, 1H), 6.92-6.81 (m, 1H), 6.03-5.91 (m, 1H), 5.85-5.73 (m, 1H), 4.78-4.55 (m, 2H), 4.03-3.36 (m, 3H), 2.17 (s, 3H), 1.73 (s, 9H).
10-625(#1): δ8.23-8.04 (m, 1H), 7.34-7.06 (m, 3H), 7.00-6.93 (m, 1H), 6.89-6.81 (m, 1H), 6.78-6.70 (m, 1H), 6.64-6.47 (m, 1H), 4.87-4.71 (m, 2H), 3.97-3.40 (m, 3H), 1.73 (s, 9H).
10-626(#1): δ8.04-7.93 (m, 1H), 7.38-7.07 (m, 5H), 7.07-6.97 (m, 1H), 6.94-6.83 (m, 1H), 4.63-4.53 (m, 2H), 3.90-3.38 (m, 6H), 1.73 (s, 9H).
10-627(#1): δ8.24-8.18 (m, 1H), 8.11-8.02 (m, 1H), 7.60-7.45 (m, 1H), 7.30-7.07 (m, 4H), 6.96-6.90 (m, 1H), 6.84-6.77 (m, 1H), 4.76-4.68 (m, 2H), 4.30-4.02 (m, 2H), 1.70 (s, 9H), 1.51-1.00 (m, 3H).
10-629(#1): δ8.16-8.01 (m, 1H), 7.29-7.06 (m, 3H), 7.04-6.96 (m, 1H), 6.93-6.81 (m, 1H), 6.02-5.89 (m, 1H), 5.80-5.71 (m, 1H), 4.77-4.57 (m, 2H), 4.41-3.87 (m, 2H), 2.17 (s, 3H), 1.73 (s, 9H), 1.43-0.96 (m, 3H).
10-630(#1): δ8.18-8.06 (m, 1H), 7.33-7.05 (m, 3H), 7.00-6.95 (m, 1H), 6.89-6.80 (m, 1H), 6.77-6.70 (m, 1H), 6.57-6.46 (m, 1H), 4.85-4.72 (m, 2H), 4.38-3.99 (m, 2H), 1.73 (s, 9H), 1.42-1.01 (m, 3H).
10-631(#1): δ8.21-7.97 (m, 1H), 7.42-7.08 (m, 5H), 7.05-6.98 (m, 1H), 6.95-6.84 (m, 1H), 4.65-4.50 (m, 2H), 4.38-3.96 (m, 2H), 3.74 (s, 3H), 1.73 (s, 9H), 1.45-0.95 (m, 3H).
10-632(#1): δ7.96-7.83 (m, 1H), 7.22-7.11 (m, 2H), 7.08-7.00 (m, 1H), 6.95-6.86 (m, 1H), 6.81-6.71 (m, 2H), 6.69-6.55 (m, 2H), 5.26-5.10 (m, 1H), 3.99-3.91 (m, 2H), 3.81 (s, 3H), 2.37-2.26 (m, 6H), 1.69 (s, 9H).
10-633(#1): δ8.02-7.92 (m, 1H), 7.23-7.10 (m, 3H), 6.95-6.87 (m, 1H), 6.83-6.73 (m, 2H), 6.59-6.48 (m, 2H), 5.11-5.00 (m, 1H), 3.99-3.91 (m, 2H), 3.75 (s, 3H), 2.32 (s, 3H), 1.71 (s, 9H).
10-634(#1): δ8.03-7.91 (m, 1H), 7.25-7.10 (m, 3H), 6.97-6.71 (m, 3H), 6.68-6.57 (m, 1H), 5.19-5.04 (m, 1H), 4.07-3.95 (m, 3H), 3.80-3.71 (m, 3H), 2.32 (s, 3H), 1.82-1.67 (m, 9H).
10-635(#1): δ7.96-7.86 (m, 1H), 7.22-7.06 (m, 3H), 6.94-6.84 (m, 1H), 6.82-6.66 (m, 3H), 6.64-6.53 (m, 1H), 4.95-4.83 (m, 1H), 3.94-3.84 (m, 2H), 3.81-3.71 (m, 3H), 2.37-2.28 (m, 6H), 1.73 (s, 9H).
10-636(#1): δ8.02-7.90 (m, 1H), 7.26-7.11 (m, 4H), 6.97-6.67 (m, 5H), 5.07-4.94 (m, 1H), 3.94-3.86 (m, 2H), 3.77 (s, 3H), 2.36-2.28 (m, 3H), 1.73 (s, 9H).
10-637(#1): δ8.35-8.17 (m, 2H), 7.59-7.46 (m, 1H), 7.26-7.07 (m, 2H), 7.02-6.89 (m, 1H), 6.80-6.66 (m, 2H), 4.78-4.63 (m, 2H), 4.29-4.00 (m, 2H), 2.40-2.29 (m, 3H), 1.82-1.65 (m, 9H), 1.43-1.00 (m, 3H).
10-641(#1): δ8.20-8.08 (m, 1H), 7.35-7.21 (m, 2H), 7.16-6.97 (m, 3H), 6.93-6.83 (m, 2H), 6.64-6.55 (m, 1H), 6.50-6.39 (m, 1H), 4.80-4.66 (m, 2H), 4.21-3.91 (m, 2H), 3.70 (s, 3H), 2.23 (s, 3H), 1.89-1.40 (m, 11H), 1.15-0.69 (m, 3H).
10-642(#1): δ8.25-8.12 (m, 1H), 7.36-7.22 (m, 2H), 7.15-6.97 (m, 3H), 6.94-6.83 (m, 2H), 6.63-6.54 (m, 1H), 6.52-6.41 (m, 1H), 4.80-4.67 (m, 2H), 3.99-3.66 (m, 5H), 2.23 (s, 3H), 2.06-1.87 (m, 1H), 1.72 (s, 9H), 0.95 (d, J = 6.5 Hz, 6H).
10-645(#1): δ8.40-8.28 (m, 1H), 7.39-7.24 (m, 2H), 7.18-7.07 (m, 2H), 7.04-6.96 (m, 1H), 6.93-6.82 (m, 2H), 6.63-6.58 (m, 1H), 6.53-6.42 (m, 1H), 3.70 (s, 3H), 2.21 (s, 3H), 1.94-1.80 (m, 1H), 1.74 (s, 9H), 1.24-0.89 (m, 4H).
10-648(#1): δ8.32-8.20 (m, 1H), 7.39-7.20 (m, 2H), 7.18-7.05 (m, 3H), 7.00-6.89 (m, 2H), 6.55-6.37 (m, 2H), 4.79-4.68 (m, 2H), 4.23-3.89 (m, 2H), 3.71 (s, 3H), 1.82-1.39 (m, 11H), 1.06-0.72 (m, 3H).
10-649(#1): δ8.39-8.23 (m, 1H), 7.39-7.20 (m, 3H), 7.16-7.05 (m, 2H), 7.02-6.90 (m, 2H), 6.58-6.37 (m, 2H), 4.82-4.67 (m, 2H), 4.04-3.64 (m, 5H), 2.04-1.91 (m, 1H), 1.72 (s, 9H), 0.95 (d, J = 6.8 Hz, 6H).
10-650(#1): δ8.36-8.23 (m, 1H), 7.50-6.78 (m, 12H), 6.61-6.35 (m, 2H), 5.32-4.99 (m, 2H), 4.85-4.68 (m, 2H), 3.69 (s, 3H), 1.86-1.53 (m, 9H).
10-651(#1): δ8.58-8.44 (m, 1H), 7.37-7.07 (m, 5H), 6.99-6.88 (m, 2H), 6.61-6.33 (m, 2H), 3.71 (s, 3H), 1.95-1.77 (m, 1H), 1.74 (s, 9H), 1.23-0.82 (m, 4H).
10-654(#1): δ8.35-8.26 (m, 1H), 7.41-7.21 (m, 3H), 7.17-7.05 (m, 2H), 6.97-6.88 (m, 2H), 6.80-6.72 (m, 1H), 6.69-6.52 (m, 1H), 4.89-4.75 (m, 2H), 4.21-3.91 (m, 2H), 3.70 (s, 3H), 1.87-1.39 (m, 11H), 1.11-0.73 (m, 3H).
10-655(#1): δ8.46-8.26 (m, 1H), 7.39-7.04 (m, 5H), 6.97-6.86 (m, 2H), 6.76-6.72 (m, 1H), 6.71-6.51 (m, 1H), 4.87-4.76 (m, 2H), 4.04-3.66 (m, 5H), 2.03-1.91 (m, 1H), 1.72 (s, 9H), 1.06-0.70 (m, 6H).
10-656(#1): δ8.36-8.27 (m, 1H), 7.45-7.16 (m, 9H), 7.13-6.68 (m, 5H), 5.34-4.98 (m, 2H), 4.89-4.77 (m, 2H), 3.68 (s, 3H), 1.86-1.54 (m, 9H).
10-657(#1): δ8.63-8.49 (m, 1H), 7.36-7.18 (m, 3H), 7.18-7.08 (m, 2H), 7.00-6.87 (m, 2H), 6.77-6.61 (m, 2H), 3.71 (s, 3H), 1.96-1.80 (m, 1H), 1.74 (s, 9H), 1.25-0.78 (m, 4H).
10-660(#1): δ8.00-7.89 (m, 1H), 7.29-7.10 (m, 6H), 6.98-6.87 (m, 1H), 6.77-6.66 (m, 2H), 5.13-4.98 (m, 1H), 3.98-3.89 (m, 2H), 2.33 (s, 3H), 1.72 (s, 9H).
10-661(#1): δ8.01-7.89 (m, 1H), 7.42-7.07 (m, 5H), 7.04-6.83 (m, 3H), 6.81-6.12 (m, 2H), 5.14-5.00 (m, 1H), 4.01-3.91 (m, 2H), 2.40-2.28 (m, 3H), 1.72 (s, 9H).
10-662(#1): δ8.03-7.90 (m, 1H), 7.33-7.09 (m, 4H), 6.97-6.69 (m, 5H), 6.28-5.81 (m, 1H), 5.10-4.96 (m, 1H), 4.36-4.19 (m, 2H), 3.98-3.88 (m, 2H), 2.39-2.26 (m, 3H), 1.73 (s, 9H).
10-663(#1): δ8.05-7.97 (m, 1H), 7.90-7.77 (m, 1H), 7.59-7.07 (m, 6H), 6.69-6.59 (m, 1H), 5.09-4.95 (m, 1H), 3.98-3.82 (m, 5H), 1.72 (s, 9H).
10-664(#2): δ9.06-9.02 (m, 1H), 8.38-8.32 (m, 1H), 8.16-8.11 (m, 1H), 7.41-7.32 (m, 1H), 7.28-7.00 (m, 4H), 6.89-6.82 (m, 2H), 5.38-5.28 (m, 1H), 4.22-4.15 (m, 2H), 3.95 (s, 3H), 1.72 (s, 9H).
10-665(#1): δ8.02-7.90 (m, 1H), 7.42-7.23 (m, 5H), 7.19-6.90 (m, 5H), 6.73-6.15 (m, 1H), 5.16-4.99 (m, 1H), 4.00-3.87 (m, 2H), 1.73 (s, 9H).
10-666(#1): δ8.00-7.87 (m, 1H), 7.41-7.20 (m, 4H), 7.18-7.00 (m, 2H), 6.99-6.88 (m, 2H), 6.86-6.71 (m, 2H), 5.10-4.93 (m, 1H), 4.37-4.20 (m, 2H), 3.96-3.85 (m, 2H), 1.73 (s, 9H).
10-667(#1): δ8.03-7.87 (m, 1H), 7.43-7.01 (m, 6H), 6.99-6.86 (m, 2H), 6.83-6.71 (m, 2H), 6.29-5.84 (m, 1H), 5.17-4.89 (m, 1H), 4.19-4.05 (m, 2H), 3.95-3.85 (m, 2H), 1.73 (s, 9H).
10-668(#1): δ7.99-7.87 (m, 1H), 7.41-7.05 (m, 6H), 7.01-6.88 (m, 2H), 6.85-6.70 (m, 2H), 6.27-5.83 (m, 1H), 5.12-4.91 (m, 1H), 4.36-4.20 (m, 2H), 3.97-3.86 (m, 2H), 1.73 (s, 9H).
10-669(#1): δ7.85-7.69 (m, 1H), 7.51-6.88 (m, 9H), 6.75-6.15 (m, 1H), 5.16-4.96 (m, 1H), 4.00-3.86 (m, 2H), 1.72 (s, 9H). 10-670(#1): δ7.84-7.68 (m, 1H), 7.51-7.02 (m, 7H), 6.86-6.73 (m, 2H), 5.11-4.96 (m, 1H), 4.37-4.21 (m, 2H), 3.97-3.85 (m, 2H), 1.73 (s, 9H).
10-671(#1): δ7.84-7.68 (m, 1H), 7.49-7.05 (m, 7H), 6.86-6.74 (m, 2H), 6.31-5.82 (m, 1H), 5.11-4.96 (m, 1H), 4.20-4.03 (m, 2H), 3.95-3.85 (m, 2H), 1.73 (s, 9H).
10-672(#1): δ7.84-7.69 (m, 1H), 7.53-7.02 (m, 7H), 6.86-6.73 (m, 2H), 6.28-5.83 (m, 1H), 5.11-4.94 (m, 1H), 4.39-4.21 (m, 2H), 3.98-3.84 (m, 2H), 1.72 (s, 9H).
10-673(#2): δ8.01-7.95 (m, 1H), 7.52-7.45 (m, 2H), 7.41-7.35 (m, 2H), 7.23-7.14 (m, 1H), 7.12 (s, 1H), 6.97-6.90 (m, 1H), 6.72-6.66 (m, 2H), 5.20-5.06 (m, 1H), 4.07-3.98 (m, 2H), 2.33 (s, 3H), 1.72 (s, 9H).
10-675(#1): δ7.85-7.73 (m, 1H), 7.25-7.14 (m, 3H), 7.11-7.04 (m, 1H), 7.00-6.95 (m, 1H), 6.88-6.82 (m, 1H), 6.59-6.48 (m, 2H), 5.13-4.98 (m, 1H), 3.99-3.92 (m, 2H), 3.76 (s, 3H), 1.70 (s, 9H).
10-676(#1): δ7.87-7.68 (m, 1H), 7.27-6.76 (m, 7H), 6.70-6.61 (m, 1H), 5.21-5.03 (m, 1H), 4.06-3.96 (m, 2H), 3.76 (s, 3H), 1.70 (s, 9H).
10-677(#1): δ7.77-7.69 (m, 1H), 7.24-7.02 (m, 4H), 6.99-6.94 (m, 1H), 6.87-6.80 (m, 1H), 6.76-6.66 (m, 1H), 6.63-6.55 (m, 1H), 4.96-4.84 (m, 1H), 3.92-3.85 (m, 2H), 3.76 (s, 3H), 2.33 (s, 3H), 1.73 (s, 9H).
10-678(#1): δ7.80-7.69 (m, 1H), 7.26-7.15 (m, 4H), 7.10-7.02 (m, 1H), 6.97-6.93 (m, 1H), 6.85-6.74 (m, 3H), 5.07-4.95 (m, 1H), 3.92-3.86 (m, 2H), 3.78 (s, 3H), 1.72 (s, 9H).
10-679(#1): δ7.81-7.70 (m, 1H), 7.32-6.99 (m, 4H), 6.98-6.68 (m, 5H), 5.08-4.95 (m, 1H), 4.10-3.94 (m, 2H), 3.93-3.84 (m, 2H), 1.72 (s, 9H), 1.40 (t, J = 7.0 Hz, 3H).
10-680(#1): δ7.82-7.69 (m, 1H), 7.34-6.99 (m, 7H), 6.96-6.87 (m, 1H), 6.83-6.72 (m, 1H), 5.14-4.99 (m, 1H), 3.98-3.86 (m, 2H), 1.72 (s, 9H).
10-681(#1): δ7.84-7.72 (m, 1H), 7.34-6.91 (m, 8H), 6.84-6.77 (m, 1H), 6.76-6.18 (m, 1H), 5.10-4.99 (m, 1H), 3.98-3.90 (m, 2H), 1.72 (s, 9H).
10-682(#1): δ7.84-7.67 (m, 1H), 7.43-7.01 (m, 5H), 6.97-6.86 (m, 2H), 6.84-6.74 (m, 2H), 6.35-5.84 (m, 1H), 5.09-4.95 (m, 1H), 4.26-4.07 (m, 2H), 3.95-3.85 (m, 2H), 1.72 (s, 9H).
10-683(#1): δ7.82-7.68 (m, 1H), 7.28-7.14 (m, 4H), 7.10-7.03 (m, 1H), 6.99-6.88 (m, 1H), 6.87-6.75 (m, 3H), 6.27-5.83 (m, 1H), 5.09-4.97 (m, 1H), 4.38-4.24 (m, 2H), 3.95-3.87 (m, 2H), 1.72 (s, 9H).
10-684(#2): δ8.31-8.25 (m, 1H), 8.17-8.14 (m, 1H), 7.43-7.36 (m, 1H), 7.34-7.30 (m, 1H), 7.18 (s, 1H), 7.16-7.13 (m, 1H), 7.10-7.03 (m, 3H), 5.29-5.17 (m, 1H), 4.08-3.99 (m, 2H), 3.81 (s, 3H), 1.73 (s, 9H).
10-685(#2): δ8.34-8.27 (m, 1H), 8.18-8.14 (m, 1H), 7.33-7.27 (m, 2H), 7.16-7.01 (m, 4H), 6.94-6.89 (m, 2H), 5.29-5.18 (m, 1H), 4.07-4.02 (m, 2H), 3.81 (s, 3H), 1.72 (s, 9H).
10-688(#1): δ8.34-8.24 (m, 1H), 8.05-7.93 (m, 1H), 7.64-7.54 (m, 1H), 7.26-7.10 (m, 3H), 6.98-6.89 (m, 1H), 6.79-6.67 (m, 2H), 5.12-5.00 (m, 1H), 4.05-3.94 (m, 2H), 2.33 (s, 3H), 1.72 (s, 9H).
10-689(#1): δ8.08-7.92 (m, 2H), 7.59-7.52 (m, 1H), 7.22-7.11 (m, 2H), 6.94-6.87 (m, 1H), 6.82-6.72 (m, 2H), 6.70-6.57 (m, 1H), 5.07-4.93 (m, 1H), 4.05-3.82 (m, 5H), 2.32 (s, 3H), 1.72 (s, 9H).
10-690(#1): δ8.14-8.00 (m, 1H), 7.23-7.11 (m, 2H), 6.97-6.68 (m, 3H), 6.06-5.99 (m, 1H), 5.86-5.78 (m, 1H), 5.03-4.90 (m, 1H), 3.96-3.88 (m, 2H), 2.32 (s, 3H), 2.23 (s, 3H), 1.73 (s, 9H).
10-691(#2): δ8.15-8.06 (m, 1H), 7.25-7.14 (m, 2H), 6.97-6.90 (m, 1H), 6.82-6.73 (m, 3H), 6.65-6.58 (m, 1H), 5.12-5.04 (m, 1H), 4.14-4.07 (m, 2H), 2.34 (s, 3H), 1.73 (s, 9H).
10-693(#1): δ8.31-8.25 (m, 1H), 8.18-8.11 (m, 1H), 7.87-7.78 (m, 1H), 7.63-7.54 (m, 1H), 7.30-7.01 (m, 3H), 6.96-6.91 (m, 1H), 6.84-6.76 (m, 1H), 5.12-4.98 (m, 1H), 4.05-3.94 (m, 2H), 1.71 (s, 9H).
10-694(#1): δ8.08-7.96 (m, 1H), 7.88-7.69 (m, 1H), 7.61-7.49 (m, 1H), 7.26-7.13 (m, 2H), 7.10-7.01 (m, 1H), 6.99-6.92 (m, 1H), 6.88-6.78 (m, 1H), 6.71-6.60 (m, 1H), 5.10-4.91 (m, 1H), 3.96-3.81 (m, 5H), 1.78-1.65 (m, 9H).
10-695(#1): δ7.91-7.81 (m, 1H), 7.24-7.18 (m, 2H), 7.10-7.05 (m, 1H), 7.01-6.97 (m, 1H), 6.90-6.82 (m, 1H), 6.03-5.98 (m, 1H), 5.84-5.79 (m, 1H), 5.03-4.89 (m, 1H), 3.97-3.87 (m, 2H), 2.24 (s, 3H), 1.73 (s, 9H).
10-696(#1): δ7.97-7.84 (m, 1H), 7.31-7.16 (m, 2H), 7.15-6.99 (m, 1H), 6.99-6.89 (m, 1H), 6.84-6.73 (m, 2H), 6.61-6.53 (m, 1H), 5.17-5.00 (m, 1H), 4.18-3.93 (m, 2H), 1.72 (s, 9H).
10-698(#1): δ8.04-7.88 (m, 3H), 7.42-7.31 (m, 2H), 7.19-7.09 (m, 2H), 6.95-6.86 (m, 1H), 6.75-6.65 (m, 2H), 5.20-5.05 (m, 1H), 4.07-3.99 (m, 2H), 3.91 (s, 3H), 2.30 (s, 3H), 1.72 (s, 9H).
10-699(#1): δ8.30-8.13 (m, 1H), 7.92-7.81 (m, 2H), 7.31-7.05 (m, 4H), 6.96-6.87 (m, 1H), 6.68-6.57 (m, 2H), 4.82-4.69 (m, 2H), 3.98-3.46 (m, 6H), 2.29 (s, 3H), 1.71 (s, 9H).
11-001(#2): δ8.45-8.21 (m, 1H), 7.43-7.23 (m, 3H), 7.19-7.04 (m, 3H), 7.00-6.86 (m, 3H), 4.93-4.70 (m, 2H), 4.02-3.39 (m, 6H), 1.77-1.68 (m, 9H).
11-002(#2): δ8.42-8.16 (m, 1H), 7.66-7.53 (m, 1H), 7.43-6.40 (m, 11H), 5.23-4.59 (m, 4H), 3.94-3.12 (m, 3H), 2.62-2.54 (m, 3H), 1.72 (s, 9H).
12-001(#2): δ7.73-7.62 (m, 1H), 7.48-7.31 (m, 2H), 7.30-7.24 (m, 1H), 7.20-6.93 (m, 4H), 6.86-6.78 (m, 1H), 5.20-4.96 (m, 1H), 4.45-4.32 (m, 1H), 2.25 (s, 3H), 2.11 (s, 3H), 1.71 (s, 9H), 1.30-1.21 (m, 3H).
12-002(#2): δ7.94-7.84 (m, 1H), 7.41-7.35 (m, 1H), 7.32-7.23 (m, 2H), 7.11 (s, 1H), 7.10-7.04 (m, 1H), 7.00-6.94 (m, 1H), 6.89-6.81 (m, 3H), 5.14-4.97 (m, 1H), 4.42-4.33 (m, 1H), 2.26 (s, 3H), 2.16 (s, 3H), 1.71 (s, 9H), 1.30-1.24 (m, 3H).
12-003(#2): δ7.88-7.79 (m, 1H), 7.37-7.22 (m, 4H), 7.10-7.00 (m, 2H), 6.99-6.93 (m, 1H), 6.85-6.78 (m, 2H), 5.15-4.76 (m, 1H), 4.22-4.15 (m, 1H), 2.24 (s, 3H), 2.14 (s, 3H), 1.78-1.71 (m, 2H), 1.70 (s, 9H), 0.98-0.74 (m, 3H).
12-004(#2): δ8.12-7.97 (m, 1H), 7.31-7.19 (m, 3H), 7.18-7.13 (m, 2H), 7.12-6.98 (m, 4H), 6.88-6.81 (m, 1H), 5.05-4.73 (m, 1H), 4.13-4.02 (m, 1H), 2.26 (s, 3H), 1.68 (s, 9H), 1.33-1.25 (m, 3H).
12-008(#2): δ7.79-7.61 (m, 1H), 7.39-7.32 (m, 2H), 7.26-7.22 (m, 1H), 7.17-7.10 (m, 2H), 7.08-6.94 (m, 3H), 6.69-6.62 (m, 1H), 5.56-5.40 (m, 1H), 3.72 (s, 3H), 2.29 (s, 3H), 2.20 (s, 3H), 1.87-1.72 (m, 2H), 1.67 (s, 9H), 0.97-0.86 (m, 3H).
12-017(#2): δ7.55 (s, 1H), 7.50-7.37 (m, 2H), 7.28-7.24 (m, 1H), 7.18-7.11 (m, 2H), 7.08-7.03 (m, 1H), 7.00-6.93 (m, 1H), 6.71-6.66 (m, 1H), 5,81-5.66 (m, 1H), 3.70-3.40 (m, 3H), 2.33 (s, 3H), 2.20 (s, 3H), 1.68 (s, 9H), 1.50 (d, J = 7.2 Hz, 3H).
12-018(#2): δ7.57 (s, 1H), 7.51-7.38 (m, 2H), 7.36-7.24 (m, 1H), 7.20-7.14 (m, 1H), 7.11 (s, 1H), 7.07-7.03 (m, 1H), 6.98-6.93 (m, 1H), 6.68-6.62 (m, 1H), 5,81-5.65 (m, 1H), 4.40-4.00 (m, 2H), 2.33 (s, 3H), 2.19 (s, 3H), 1.69 (s, 9H), 1.49 (d, J = 6.8 Hz, 3H), 1.19-1.06 (m, 3H).
12-019(#2): δ7.61 (s, 1H), 7.51-7.09 (m, 5H), 7.08-7.03 (m, 1H), 7.00-6.93 (m, 1H), 6.68-6.62 (m, 1H), 5,95-5.64 (m, 2H), 5.35-5.07 (m, 2H), 4.75-4.43 (m, 2H), 2.32 (s, 3H), 2.19 (s, 3H), 1.68 (s, 9H), 1.50 (d, J = 6.8 Hz, 3H).
13-001(#2): δ7.41-7.23 (m, 3H), 7.19-6.91 (m, 5H), 6.83-6.74 (m, 1H), 6.34-6.21 (m, 1H), 5.39-4.99 (m, 2H), 3.98-3.05 (m, 5H), 1.76-1.60 (m, 9H), 1.26 (t, J = 7.2 Hz, 3H).
14-002(#1): δ7.91 (s, 1H), 7.46-7.31 (m, 2H), 7.23-7.00 (m, 2H), 6.98-6.92 (m, 1H), 6.63-6.38 (m, 2H), 4.67 (s, 2H), 3.80-3.03 (m, 3H), 3.75 (s, 3H), 1.71 (s, 9H).
14-003(#2): δ7.86 (br, 1H), 7.46-7.34 (m, 2H), 7.15 (s, 1H), 7.12-6.90 (m, 3H), 6.81-6.67 (m, 2H), 4.61 (s, 2H), 3.92-3.16 (m, 3H), 3.76 (s, 3H), 1.71 (s, 9H).
14-004(#1): δ7.94-7.78 (m, 1H), 7.44-7.28 (m, 2H), 7.12-6.69 (m, 5H), 4.79-4.62 (m, 2H), 4.05-3.23 (m, 3H), 2.34-2.07 (m, 6H), 1.80-1.58 (m, 9H).

**[Table 21]**

| | |
|---|---|
| No. | ¹H-NMR (DMSO-d6, Me₄Si) |

13-002(#2): δ7.74-7.46 (m, 3H), 7.41-7.21 (m, 2H), 7.03-6.83 (m, 2H), 6.63-6.36 (m, 1H), 5.03-4.00 (m, 5H), 3.87-3.38 (m, 3H), 2.26-1.96 (m, 6H), 1.48-1.06 (m, 6H).

### [Test Examples]

The following description will specifically discuss usefulness of the compound in accordance with the present invention as a pest control agent with reference to test examples below. Note, however, that the present invention is not limited only to those test examples. Note that "RH" in the test examples represents a relative humidity. For example, a phrase "humidity of 100%RH" indicates that the relative humidity is 100%.

### Preparation of test chemical solution

The compound in accordance with the present invention was dissolved in a solvent for emulsion (a mixture of SORPOL (registered trademark) 3005XL (available from TOHO Chemical Industry Co., Ltd.), N-methylpyrrolidone, and SOLVESSO (registered trademark) 200 (available from Exxon Mobil) at 1:5:28 (weight ratio)), and emulsifiable concentrates having concentrations of 1% by mass, 5% by mass, and 20% by mass, respectively, were prepared. The prepared emulsifiable concentrates were each diluted with water so as to be adjusted to contain 500 ppm of the compound in accordance with the present invention. Each of the diluted emulsifiable concentrates was subjected to Test Examples 1 through 7 below as a test chemical solution.

### Test Example 1: Test of control effect on wheat glume blotch

In a plastic pot of 90 cm³, wheat (variety: Haruyutaka) was planted. In the 1.3 leaf stage, the test chemical solution was sprayed by 5 ml. After 1 day from the spraying, a conidium suspension of a fungus (Septoria nodorum) which causes wheat glume blotch was inoculated onto the wheat by spraying, and the wheat was kept in an inoculation box at temperature of 20°C and humidity of 100%RH for 2 days. After that, the wheat was placed in an air-conditioned greenhouse (temperature: 20°C, humidity: 80%RH) and kept for 8 days. The proportion of formed lesions to the inoculated leaf was measured, and a control value was calculated in accordance with a calculation formula below. 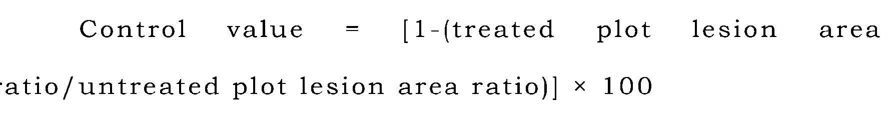 Control value = [1-(treated plot lesion area ratio/untreated plot lesion area ratio)] × 100

As a result, the following compounds exhibited a control value of 70% or more among the tested compounds.

Compounds: No. 1-006, 1-007, 1-008, 1-011, 1-012, 1-013, 1-015, 1-016, 1-017, 1-018, 1-019, 1-020, 1-021, 1-022, 1-023, 1-024, 1-025, 1-026, 1-027, 1-028, 1-029, 1-038, 1-039, 1-040, 1-041, 1-042, 1-043, 1-044, 1-045, 1-046, 1-048, 1-050, 1-051, 1-052, 1-053, 1-054, 1-055, 1-056, 1-057, 1-058, 1-062, 1-066, 1-067, 1-068, 1-069, 1-073, 1-075, 1-076, 1-077, 1-078, 1-079, 1-081, 1-083, 1-084, 1-085, 1-086, 1-087, 1-090, 1-093, 1-094, 1-095, 1-096, 1-102, 1-103, 1-104, 1-105, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-130, 1-135, 1-137, 1-138, 1-139, 1-140, 1-141, 1-144, 1-145, 1-150, 1-160, 1-162, 1-163, 1-164, 1-166, 1-167, 1-168, 1-169, 1-170, 1-171, 1-172, 1-173, 1-174, 1-175, 1-176, 1-177, 1-178, 1-180, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-212, 1-214, 1-215, 1-217, 1-218, 1-219, 1-220, 1-221, 1-222, 1-224, 1-225, 1-226, 1-227, 1-228, 1-231, 1-232, 1-233, 1-234, 1-239, 1-240, 2-001, 2-003, 2-005, 2-008, 2-013, 2-025, 2-026, 2-027, 2-028, 2-029, 2-030, 2-031, 2-032, 2-033, 2-034, 2-035, 2-036, 2-037, 2-039, 2-042, 2-047, 2-050, 2-051, 2-052, 2-053, 2-054, 2-055, 2-056, 2-057, 2-059, 2-060, 2-061, 2-063, 2-064, 2-065, 2-066, 2-067, 2-070, 2-071, 2-072, 2-073, 2-074, 2-076, 2-077, 2-078, 2-079, 2-082, 2-084, 2-085, 2-086, 2-087, 2-088, 2-089, 2-091, 2-092, 2-093, 2-094, 2-095, 2-096, 2-097, 2-098, 2-099, 2-100, 2-101, 2-102, 2-103, 2-104, 2-105, 2-107, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-005, 3-007, 3-009, 3-011, 3-012, 3-013, 3-014, 3-017, 3-018, 3-022, 3-024, 3-025, 3-026, 3-027, 3-028, 3-029, 3-030, 3-031, 3-032, 6-001, 6-002, 6-004, 6-005, 6-006, 6-007, 6-008, 10-002, 10-003, 10-004, 10-006, 10-007, 10-008, 10-009, 10-010, 10-011, 10-012, 10-013, 10-014, 10-015, 10-016, 10-029, 10-032, 10-033, 10-034, 10-035, 10-040, 10-041, 10-044, 10-045, 10-048, 10-050, 10-051, 10-052, 10-058, 10-059, 10-060, 10-061, 10-062, 10-069, 10-072, 10-075, 10-076, 10-077, 10-078, 10-085, 10-087, 10-096, 10-103, 10-106, 10-109, 10-110, 10-111, 10-112, 10-113, 10-114, 10-115, 10-116, 10-117, 10-118, 10-119, 10-120, 10-122, 10-126, 10-133, 10-136, 10-137, 10-140, 10-141, 10-142, 10-144, 10-145, 10-147, 10-148, 10-149, 10-150, 10-151, 10-152, 10-153, 10-154, 10-155, 10-156, 10-157, 10-158, 10-160, 10-161, 10-162, 10-163, 10-164, 10-165, 10-166, 10-167, 10-168, 10-169, 10-174, 10-175, 10-176, 10-180, 10-181, 10-182, 10-183, 10-184, 10-185, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-207, 10-208, 10-209, 10-210, 10-212, 10-213, 10-214, 10-216, 10-217, 10-218, 10-221, 10-222, 10-223, 10-224, 10-225, 10-226, 10-230, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-265, 10-272, 10-273, 10-274, 10-277, 10-279, 10-280, 10-282, 10-283, 10-284, 10-285, 10-286, 10-289, 10-290, 10-292, 10-293, 10-300, 10-312, 10-313, 10-315, 10-316, 10-317, 10-320, 10-321, 10-323, 10-324, 10-325, 10-326, 10-327, 10-328, 10-329, 10-330, 10-331, 10-350, 10-353, 10-357, 10-360, 10-361, 10-362, 10-363, 10-364, 10-365, 10-366, 10-368, 10-369, 10-370, 10-371, 10-372, 10-373, 10-374, 10-376, 10-377, 10-378, 10-380, 10-381, 10-382, 10-384, 10-385, 10-396, 10-397, 10-398, 10-399, 10-400, 10-405, 10-406, 10-407, 10-408, 10-409, 10-411, 10-412, 10-415, 10-416, 10-417, 10-426, 10-427, 10-431, 10-435, 10-436, 10-440, 10-441, 10-442, 10-443, 10-444, 10-446, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-470, 10-471, 10-472, 10-473, 10-474, 10-475, 10-476, 10-477, 10-478, 10-479, 10-480, 10-481, 10-482, 10-483, 10-484, 10-487, 10-488, 10-489, 10-499, 10-500, 10-503, 10-504, 10-505, 10-507, 10-510, 10-511, 10-518, 10-528, 10-529, 10-531, 10-532, 10-534, 10-535, 10-536, 10-540, 10-545, 10-546, 10-547, 10-548, 10-549, 10-550, 10-551, 10-552, 10-557, 10-558, 10-559, 10-562, 10-563, 10-565, 10-566, 10-567, 10-568, 10-569, 10-570, 10-571, 10-572, 10-573, 10-574, 10-575, 10-578, 10-579, 10-582, 10-583, 10-584, 10-587, 10-589, 10-591, 10-592, 10-593, 10-594, 10-597, 10-598, 10-599, 10-600, 10-602, 10-603, 10-604, 10-605, 10-606, 10-607, 10-608, 10-609, 10-610, 10-611, 10-616, 10-618, 10-619, 10-620, 10-623, 10-625, 10-628, 10-630, 10-633, 10-634, 10-635, 10-636, 10-637, 10-639, 10-641, 10-642, 10-643, 10-648, 10-650, 10-651, 10-654, 10-655, 10-660, 10-661, 10-665, 10-668, 10-676, 10-678, 10-682, 10-687, 11-002, 12-004, 12-005, 12-006, 12-007, 12-011, 12-012, 12-014, 13-001, 14-002.

### Test Example 2: Test of control effect on tomato powdery mildew

In a plastic pot of 90 cm³, tomato (variety: Momotaro) was planted. In the two-leaf stage, the test chemical solution was sprayed by 5 ml. After air drying, the tomato was placed in an air-conditioned greenhouse (temperature: 20°C, humidity: 70%RH), and a conidium suspension of a fungus (Leveillula taurica) which causes tomato powdery mildew was inoculated by spraying. After keeping for 14 days, the proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-034, 1-036, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-062, 1-064, 1-066, 1-067, 1-068, 1-069, 1-070, 1-071, 1-072, 1-073, 1-075, 1-076, 1-077, 1-078, 1-079, 1-080, 1-081, 1-082, 1-083, 1-084, 1-085, 1-086, 1-087, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-152, 1-153, 1-154, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-222, 1-223, 1-224, 1-225, 1-226, 1-227, 1-228, 1-229, 1-230, 1-231, 1-232, 1-233, 1-234, 1-236, 1-237, 1-238, 1-239, 1-240, 2-001, 2-002, 2-003, 2-006, 2-007, 2-008, 2-010, 2-011, 2-013, 2-014, 2-015, 2-016, 2-018, 2-019, 2-021, 2-022, 2-023, 2-025, 2-026, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-047, 2-048, 2-049, 2-050, 2-051, 2-052, 2-053, 2-054, 2-055, 2-056, 2-057, 2-058, 2-059, 2-060, 2-061, 2-062, 2-063, 2-065, 2-066, 2-067, 2-068, 2-070, 2-071, 2-072, 2-073, 2-074, 2-075, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 4-001, 4-002, 4-003, 4-004, 4-005, 4-006, 4-007, 4-008, 4-009, 4-012, 4-013, 4-014, 4-015, 6-001, 6-002, 6-004, 6-005, 6-006, 6-007, 6-008, 7-001, 8-001, 9-001, 9-002, 9-003, 9-004, 9-005, 9-006, 9-007, 9-008, 9-010, 9-011, 9-012, 9-013, 9-014, 9-015, 9-016, 10-001, 10-002, 10-003, 10-004, 10-005, 10-006, 10-007, 10-008, 10-009, 10-010, 10-011, 10-012, 10-013, 10-014, 10-015, 10-016, 10-017, 10-018, 10-019, 10-020, 10-021, 10-022, 10-023, 10-024, 10-025, 10-026, 10-027, 10-028, 10-029, 10-030, 10-031, 10-032, 10-033, 10-034, 10-035, 10-036, 10-037, 10-038, 10-039, 10-040, 10-041, 10-042, 10-043, 10-044, 10-045, 10-046, 10-047, 10-048, 10-049, 10-050, 10-051, 10-052, 10-053, 10-054, 10-055, 10-056, 10-057, 10-058, 10-059, 10-060, 10-061, 10-062, 10-063, 10-064, 10-065, 10-066, 10-067, 10-068, 10-069, 10-070, 10-071, 10-072, 10-073, 10-074, 10-075, 10-076, 10-077, 10-078, 10-085, 10-086, 10-087, 10-089, 10-090, 10-094, 10-095, 10-096, 10-097, 10-099, 10-100, 10-103, 10-104, 10-106, 10-107, 10-109, 10-110, 10-111, 10-112, 10-113, 10-114, 10-115, 10-116, 10-117, 10-118, 10-119, 10-120, 10-121, 10-122, 10-123, 10-124, 10-125, 10-126, 10-127, 10-128, 10-129, 10-130, 10-131, 10-132, 10-133, 10-135, 10-136, 10-137, 10-138, 10-139, 10-140, 10-141, 10-142, 10-143, 10-144, 10-145, 10-146, 10-147, 10-148, 10-149, 10-150, 10-151, 10-152, 10-153, 10-154, 10-155, 10-156, 10-157, 10-158, 10-159, 10-160, 10-161, 10-162, 10-163, 10-164, 10-165, 10-166, 10-167, 10-168, 10-169, 10-170, 10-171, 10-172, 10-173, 10-174, 10-175, 10-176, 10-177, 10-178, 10-179, 10-180, 10-181, 10-182, 10-183, 10-184, 10-185, 10-186, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-206, 10-207, 10-208, 10-209, 10-210, 10-211, 10-212, 10-213, 10-214, 10-215, 10-216, 10-217, 10-218, 10-219, 10-220, 10-221, 10-222, 10-223, 10-224, 10-225, 10-226, 10-227, 10-228, 10-229, 10-230, 10-231, 10-232, 10-234, 10-235, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-264, 10-265, 10-266, 10-267, 10-268, 10-269, 10-270, 10-271, 10-272, 10-273, 10-274, 10-275, 10-276, 10-277, 10-278, 10-279, 10-280, 10-281, 10-282, 10-283, 10-284, 10-285, 10-286, 10-287, 10-288, 10-289, 10-290, 10-291, 10-292, 10-293, 10-294, 10-295, 10-296, 10-297, 10-298, 10-299, 10-300, 10-301, 10-302, 10-303, 10-306, 10-307, 10-308, 10-309, 10-310, 10-312, 10-313, 10-314, 10-315, 10-316, 10-317, 10-318, 10-319, 10-320, 10-321, 10-322, 10-323, 10-324, 10-325, 10-326, 10-327, 10-328, 10-329, 10-330, 10-331, 10-332, 10-333, 10-334, 10-335, 10-336, 10-338, 10-339, 10-340, 10-341, 10-342, 10-343, 10-344, 10-346, 10-347, 10-348, 10-349, 10-350, 10-351, 10-353, 10-354, 10-355, 10-356, 10-357, 10-358, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-386, 10-387, 10-388, 10-389, 10-390, 10-391, 10-392, 10-393, 10-394, 10-395, 10-396, 10-397, 10-398, 10-399, 10-400, 10-401, 10-402, 10-403, 10-404, 10-405, 10-406, 10-407, 10-408, 10-409, 10-410, 10-411, 10-412, 10-413, 10-414, 10-415, 10-416, 10-417, 10-418, 10-419, 10-420, 10-421, 10-422, 10-423, 10-424, 10-425, 10-426, 10-427, 10-428, 10-429, 10-430, 10-431, 10-432, 10-433, 10-434, 10-435, 10-436, 10-437, 10-438, 10-439, 10-440, 10-441, 10-442, 10-443, 10-444, 10-445, 10-446, 10-447, 10-449, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-468, 10-469, 10-470, 10-471, 10-472, 10-473, 10-474, 10-475, 10-476, 10-477, 10-478, 10-479, 10-480, 10-481, 10-482, 10-483, 10-484, 10-485, 10-486, 10-487, 10-488, 10-489, 10-490, 10-491, 10-492, 10-493, 10-494, 10-498, 10-499, 10-500, 10-501, 10-502, 10-503, 10-504, 10-505, 10-506, 10-507, 10-508, 10-509, 10-510, 10-511, 10-512, 10-513, 10-514, 10-515, 10-516, 10-517, 10-518, 10-519, 10-520, 10-521, 10-522, 10-523, 10-524, 10-525, 10-526, 10-527, 10-528, 10-529, 10-530, 10-531, 10-532, 10-533, 10-534, 10-535, 10-536, 10-537, 10-538, 10-539, 10-540, 10-541, 10-542, 10-545, 10-546, 10-547, 10-548, 10-549, 10-550, 10-551, 10-552, 10-553, 10-554, 10-555, 10-556, 10-557, 10-558, 10-559, 10-560, 10-561, 10-562, 10-563, 10-564, 10-565, 10-566, 10-567, 10-568, 10-569, 10-570, 10-571, 10-572, 10-573, 10-574, 10-575, 10-576, 10-577, 10-578, 10-579, 10-580, 10-581, 10-582, 10-583, 10-584, 10-585, 10-586, 10-587, 10-588, 10-589, 10-590, 10-591, 10-592, 10-593, 10-594, 10-595, 10-596, 10-597, 10-598, 10-599, 10-600, 10-601, 10-602, 10-603, 10-604, 10-605, 10-606, 10-607, 10-608, 10-609, 10-610, 10-611, 10-614, 10-615, 10-616, 10-617, 10-618, 10-619, 10-620, 10-621, 10-622, 10-623, 10-625, 10-626, 10-627, 10-628, 10-629, 10-630, 10-631, 10-632, 10-633, 10-634, 10-635, 10-636, 10-637, 10-638, 10-639, 10-640, 10-641, 10-642, 10-643, 10-644, 10-645, 10-646, 10-647, 10-648, 10-649, 10-650, 10-651, 10-652, 10-653, 10-654, 10-655, 10-656, 10-657, 10-658, 10-659, 10-660, 10-662, 10-663, 10-665, 10-666, 10-667, 10-668, 10-669, 10 -670, 10-671, 10-672, 10-673, 10-674, 10-675, 10-676, 10-677, 10-678, 10-679, 10-680, 10-681, 10-682, 10-683, 10-684, 10-685, 10-686, 10-687, 10-688, 10-689, 10-691, 10-693, 10-694, 10-696, 10-697, 10-698, 10-699, 11-001, 11-002, 12-001, 12-002, 12-003, 12-004, 12-005, 12-006, 12-007, 12-008, 12-009, 12-010, 12-011, 12-012, 12-013, 12-014, 12-015, 12-016, 12-017, 12-018, 12-019, 13-001, 13-002, 14-001, 14-002, 14-003, 14-004.

### Test Example 3: Test of control effect on cucumber gray mold

In a plastic pot of 90 cm³, cucumber (variety: Sagami Hanjiro) was planted. In the cotyledon stage, the test chemical solution was sprayed by 5 ml and air dried. After that, the treated leaf was cut off and placed in a plastic container. A conidium suspension of a fungus (Botrytis cinerea) which causes cucumber gray mold and a dissolved PDA culture medium were mixed at a ratio of 1:1 (volume ratio), and a resultant mixture was inoculated onto the treated leaf by dripping by 30 pl. After the inoculation, the leaf was kept in conditions of 20°C and high humidity (humidity of 100%RH) for 3 days. After that, the proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-002, 1-003, 1-004, 1-005, 1-006, 1-007, 1-008, 1-009, 1-010, 1-011, 1-012, 1-013, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-062, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-152, 1-154, 1-155, 1-156, 1-157, 1-158, 1-159, 1-160, 1-161, 1-162, 1-164, 1-166, 1-167, 1-168, 1-171, 1-172, 1-173, 1-174, 1-175, 1-176, 1-177, 1-178, 1-179, 1-180, 1-181, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-222, 1-223, 1-224, 1-225, 1-226, 1-227, 1-228, 1-229, 1-230, 1-231, 1-232, 1-233, 1-234, 1-235, 1-236, 1-237, 1-238, 1-239, 1-240, 2-001, 2-002, 2-003, 2-004, 2-006, 2-007, 2-008, 2-009, 2-010, 2-011, 2-012, 2-013, 2-014, 2-015, 2-016, 2-017, 2-018, 2-019, 2-020, 2-021, 2-022, 2-023, 2-024, 2-025, 2-026, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-046, 2-047, 2-048, 2-049, 2-050, 2-054, 2-055, 2-056, 2-057, 2-058, 2-059, 2-060, 2-061, 2-064, 2-065, 2-066, 2-067, 2-068, 2-069, 2-070, 2-071, 2-072, 2-073, 2-074, 2-075, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 4-001, 4-002, 4-003, 4-004, 4-005, 4-006, 4-007, 4-008, 4-009, 4-010, 4-011, 4-012, 4-013, 4-014, 4-015, 6-001, 6-004, 6-005, 6-006, 6-007, 6-008, 8-001, 9-002,9-003, 9-005, 9-007, 9-009, 9-010, 9-012, 10-001, 10-002, 10-003, 10-004, 10-005, 10-006, 10-007, 10-008, 10-009, 10-010, 10-011, 10-013, 10-014, 10-015, 10-016, 10-017, 10-018, 10-019, 10-020, 10-021, 10-024, 10-025, 10-026, 10-027, 10-028, 10-030, 10-031, 10-032, 10-033, 10-034, 10-035, 10-036, 10-038, 10-039, 10-040, 10-041, 10-042, 10-043, 10-044, 10-045, 10-046, 10-047, 10-048, 10-049, 10-050, 10-051, 10-052, 10-053, 10-054, 10-055, 10-057, 10-058, 10-059, 10-060, 10-061, 10-062, 10-063, 10-064, 10-065, 10-066, 10-067, 10-068, 10-069, 10-070, 10-071, 10-072, 10-073, 10-074, 10-075, 10-076, 10-077, 10-078, 10-085, 10-086, 10-087, 10-089, 10-090, 10-092, 10-094, 10-095, 10-096, 10-097, 10-098, 10-099, 10-100, 10-103, 10-104, 10-106, 10-107, 10-109, 10-110, 10-111, 10-112, 10-113, 10-114, 10-115, 10-116, 10-117, 10-118, 10-119, 10-120, 10-121, 10-122, 10-123, 10-124, 10-125, 10-126, 10-127, 10-128, 10-130, 10-131, 10-132, 10-133, 10-134, 10-135, 10-136, 10-137, 10-138, 10-139, 10-140, 10-141, 10-142, 10-143, 10-144, 10-145, 10-146, 10-147, 10-148, 10-149, 10-150, 10-151, 10-152, 10-153, 10-154, 10-155, 10-156, 10-157, 10-158, 10-159, 10-160, 10-161, 10-162, 10-163, 10-164, 10-165, 10-166, 10-167, 10-168, 10-169, 10-170, 10-171, 10-172, 10-173, 10-174, 10-175, 10-176, 10-177, 10-178, 10-179, 10-180, 10-181, 10-182, 10-183, 10-184, 10-185, 10-186, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-206, 10-207, 10-208, 10-209, 10-210, 10-211, 10-212, 10-213, 10-214, 10-215, 10-216, 10-217, 10-218, 10-219, 10-220, 10-221, 10-222, 10-223, 10-224, 10-225, 10-226, 10-227, 10-228, 10-229, 10-230, 10-231, 10-235, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-264, 10-265, 10-266, 10-267, 10-268, 10-269, 10-270, 10-271, 10-272, 10-273, 10-274, 10-275, 10-276, 10-277, 10-278, 10-279, 10-280, 10-281, 10-282, 10-283, 10-284, 10-285, 10-286, 10-287, 10-288, 10-289, 10-290, 10-291, 10-292, 10-293, 10-294, 10-295, 10-296, 10-297, 10-298, 10-299, 10-300, 10-301, 10-302, 10-303, 10-304, 10-305, 10-306, 10-307, 10-308, 10-309, 10-310, 10-311, 10-312, 10-313, 10-314, 10-315, 10-316, 10-317, 10-319, 10-320, 10-321, 10-322, 10-323, 10-324, 10-325, 10-326, 10-327, 10-328, 10-329, 10-330, 10-331, 10-332, 10-333, 10-334, 10-335, 10-336, 10-337, 10-338, 10-339, 10-340, 10-341, 10-342, 10-343, 10-344, 10-345, 10-346, 10-347, 10-348, 10-349, 10-350, 10-351, 10-353, 10-354, 10-355, 10-356, 10-357, 10-358, 10-359, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-386, 10-387, 10-388, 10-389, 10-390, 10-391, 10-392, 10-393, 10-394, 10-396, 10-397, 10-398, 10-399, 10-400, 10-401, 10-402, 10-403, 10-404, 10-405, 10-406, 10-407, 10-408, 10-409, 10-411, 10-412, 10-413, 10-414, 10-415, 10-416, 10-417, 10-418, 10-419, 10-420, 10-421, 10-422, 10-423, 10-424, 10-425, 10-426, 10-427, 10-429, 10-430, 10-431, 10-432, 10-433, 10-434, 10-435, 10-436, 10-437, 10-438, 10-439, 10-440, 10-441, 10-442, 10-443, 10-444, 10-445, 10-446, 10-447, 10-449, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-468, 10-469, 10-470, 10-471, 10-472, 10-473, 10-474, 10-475, 10-476, 10-477, 10-478, 10-479, 10-480, 10-481, 10-482, 10-483, 10-484, 10-485, 10-486, 10-487, 10-488, 10-489, 10-490, 10-491, 10-492, 10-493, 10-494, 10-495, 10-496, 10-497, 10-498, 10-499, 10-500, 10-501, 10-502, 10-607, 10-608, 10-609, 10-610, 10-611, 10-612, 10-613, 10-614, 10-615, 10-616, 10-617, 10-618, 10-619, 10-620, 10-621, 10-622, 10-623, 10-624, 10-625, 10-626, 10-627, 10-628, 10-629, 10-630, 10-631, 10-632, 10-633, 10-634, 10-635, 10-636, 10-637, 10-638, 10-639, 10-640, 10-641, 10-642, 10-643, 10-644, 10-645, 10-646, 10-647, 10-648, 10-649, 10-650, 10-651, 10-652, 10-653, 10-654, 10-655, 10-656, 10-657, 10-658, 10-659, 10-660, 10-661, 10-662, 10-663, 10-664, 10-665, 10-666, 10-667, 10-668, 10-669, 10-670, 10-671, 10-672, 10-673, 10-674, 10-675, 10-676, 10-677, 10-678, 10-679, 10-680, 10-681, 10-682, 10-683, 10-684, 10-685, 10-686, 10-687, 10-688, 10-689, 10-69010-69110-69210-69310-69410-69510-69610-697, 10-698, 10-699, 11-001, 11-002, 12-001, 12-002, 12-003, 12-004, 12-005, 12-006, 12-007, 12-008, 12-009, 12-010, 12-011, 12-012, 12-013, 12-014, 12-015, 12-016, 12-017, 12-018, 12-019, 13-001, 14-002, 14-003, 14-004.

### Test Example 4: Test of control effect on cucumber sclerotinia rot

In a plastic pot of 90 cm³, cucumber (variety: Sagami Hanjiro) was planted. In the cotyledon stage, the test chemical solution was sprayed by 5 ml and air dried. After that, the treated leaf was placed in a plastic container. An agar piece (diameter: 5 mm) containing a fungi (Sclerotinia sclerotiorum) which causes cucumber sclerotinia rot and had been cultured in a PDA culture medium in advance was inoculated onto a cotyledon of cucumber which had been treated with the chemical agent. After inoculation, the plastic container was covered with a plastic sheet and humidified (humidity: 100%RH), and kept at 20°C for 2 days. After that, the proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-002, 1-003, 1-004, 1-005, 1-006, 1-007, 1-008, 1-009, 1-010, 1-011, 1-012, 1-013, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-033, 1-034, 1-035, 1-036, 1-037, 1-038, 1-039, 1-040, 1-041, 1-042, 1-043, 1-044, 1-045, 1-046, 1-047, 1-048, 1-049, 1-050, 1-051, 1-052, 1-053, 1-054, 1-055, 1-056, 1-057, 1-058, 1-059, 1-060, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-152, 1-153, 1-154, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-181, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-222, 1-223, 1-224, 1-225, 1-226, 1-227, 1-228, 1-229, 1-230, 1-231, 1-232, 1-233, 1-234, 1-235, 1-236, 1-237, 1-238, 1-239, 1-240, 2-001, 2-002, 2-003, 2-004, 2-006, 2-007, 2-008, 2-009, 2-011, 2-013, 2-014, 2-015, 2-016, 2-017, 2-018, 2-019, 2-021, 2-022, 2-023, 2-024, 2-025, 2-026, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-046, 2-047, 2-048, 2-049, 2-050, 2-051, 2-052, 2-053, 2-054, 2-055, 2-056, 2-057, 2-058, 2-059, 2-060, 2-061, 2-062, 2-063, 2-064, 2-065, 2-066, 2-067, 2-068, 2-069, 2-070, 2-071, 2-072, 2-073, 2-074, 2-075, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 4-001, 4-002, 4-003, 4-004, 4-005, 4-006, 4-007, 4-008, 4-009, 4-010, 4-011, 4-012, 4-013, 4-014, 4-015, 6-001, 6-002, 6-004, 6-005, 6-006, 6-007, 6-008, 7-001, 8-001, 9-011, 9-012, 10-001, 10-002, 10-003, 10-004, 10-006, 10-007, 10-008, 10-009, 10-010, 10-011, 10-013, 10-014, 10-015, 10-016, 10-017, 10-018, 10-019, 10-020, 10-021, 10-024, 10-025, 10-026, 10-027, 10-028, 10-030, 10-031, 10-032, 10-033, 10-034, 10-035, 10-036, 10-038, 10-039, 10-040, 10-041, 10-042, 10-044, 10-045, 10-048, 10-049, 10-050, 10-051, 10-052, 10-053, 10-054, 10-055, 10-057, 10-058, 10-059, 10-060, 10-061, 10-062, 10-063, 10-064, 10-066, 10-067, 10-068, 10-069, 10-070, 10-071, 10-072, 10-073, 10-074, 10-075, 10-076, 10-077, 10-078, 10-085, 10-086, 10-087, 10-088, 10-089, 10-090, 10-092, 10-094, 10-095, 10-096, 10-097, 10-098, 10-099, 10-100, 10-103, 10-104, 10-106, 10-107, 10-109, 10-110, 10-111, 10-112, 10-113, 10-114, 10-115, 10-116, 10-117, 10-118, 10-119, 10-121, 10-122, 10-123, 10-124, 10-125, 10-126, 10-127, 10-128, 10-130, 10-131, 10-132, 10-133, 10-134, 10-135, 10-136, 10-137, 10-138, 10-139, 10-140, 10-141, 10-142, 10-144, 10-145, 10-146, 10-147, 10-148, 10-149, 10-150, 10-151, 10-152, 10-153, 10-154, 10-155, 10-156, 10-157, 10-158, 10-159, 10-160, 10-161, 10-162, 10-163, 10-164, 10-165, 10-166, 10-167, 10-168, 10-169, 10-170, 10-171, 10-172, 10-173, 10-174, 10-175, 10-176, 10-177, 10-178, 10-179, 10-180, 10-181, 10-182, 10-183, 10-184, 10-185, 10-186, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-206, 10-207, 10-208, 10-209, 10-210, 10-211, 10-212, 10-213, 10-214, 10-215, 10-216, 10-217, 10-218, 10-219, 10-220, 10-221, 10-222, 10-223, 10-224, 10-225, 10-226, 10-227, 10-228, 10-229, 10-230, 10-231, 10-234, 10-235, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-264, 10-265, 10-266, 10-267, 10-268, 10-269, 10-270, 10-271, 10-272, 10-273, 10-274, 10-275, 10-276, 10-277, 10-278, 10-279, 10-280, 10-281, 10-282, 10-283, 10-284, 10-285, 10-286, 10-287, 10-288, 10-289, 10-290, 10-291, 10-292, 10-293, 10-294, 10-295, 10-296, 10-297, 10-298, 10-299, 10-300, 10-301, 10-302, 10-303, 10-304, 10-305, 10-306, 10-307, 10-308, 10-309, 10-310, 10-311, 10-312, 10-313, 10-314, 10-315, 10-316, 10-317, 10-318, 10-319, 10-320, 10-321, 10-322, 10-323, 10-324, 10-325, 10-326, 10-327, 10-328, 10-329, 10-330, 10-331, 10-332, 10-333, 10-334, 10-335, 10-336, 10-337, 10-338, 10-339, 10-340, 10-341, 10-342, 10-343, 10-344, 10-345, 10-346, 10-347, 10-348, 10-349, 10-350, 10-351, 10-353, 10-354, 10-355, 10-356, 10-357, 10-358, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-386, 10-387, 10-388, 10-389, 10-390, 10-391, 10-392, 10-393, 10-394, 10-397, 10-398, 10-399, 10-400, 10-401, 10-402, 10-403, 10-404, 10-405, 10-406, 10-407, 10-408, 10-409, 10-411, 10-412, 10-413, 10-414, 10-415, 10-416, 10-417, 10-418, 10-419, 10-420, 10-421, 10-422, 10-423, 10-424, 10-425, 10-426, 10-427, 10-428, 10-429, 10-430, 10-431, 10-435, 10-436, 10-437, 10-438, 10-439, 10-440, 10-441, 10-442, 10-443, 10-444, 10-445, 10-446, 10-447, 10-448, 10-449, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-468, 10-469, 10-470, 10-471, 10-472, 10-473, 10-474, 10-475, 10-476, 10-477, 10-478, 10-479, 10-480, 10-481, 10-482, 10-483, 10-484, 10-485, 10-486, 10-487, 10-488, 10-489, 10-490, 10-491, 10-492, 10-494, 10-495, 10-496, 10-497, 10-498, 10-499, 10-500, 10-501, 10-502, 10-503, 10-504, 10-507, 10-510, 10-514, 10-518, 10-519, 10-520, 10-521, 10-522, 10-525, 10-526, 10-527, 10-528, 10-529, 10-530, 10-531, 10-532, 10-533, 10-534, 10-535, 10-536, 10-537, 10-538, 10-540, 10-543, 10-544, 10-545, 10-546, 10-547, 10-548, 10-549, 10-550, 10-551, 10-552, 10-553, 10-554, 10-555, 10-556, 10-557, 10-558, 10-559, 10-560, 10-561, 10-562, 10-563, 10-564, 10-565, 10-566, 10-567, 10-568, 10-569, 10-570, 10-571, 10-572, 10-573, 10-574, 10-575, 10-576, 10-577, 10-578, 10-579, 10-580, 10-581, 10-582, 10-583, 10-584, 10-585, 10-586, 10-587, 10-588, 10-589, 10-590, 10-591, 10-592, 10-593, 10-594, 10-595, 10-596, 10-597, 10-598, 10-599, 10-600, 10-601, 10-602, 10-603, 10-604, 10-605, 10-606, 10-607, 10-608, 10-609, 10-610, 10-611, 10-612, 10-613, 10-614, 10-615, 10-616, 10-617, 10-618, 10-619, 10-620, 10-621, 10-622, 10-623, 10-624, 10-625, 10-626, 10-627, 10-628, 10-629, 10-630, 10-631, 10-632, 10-633, 10-634, 10-635, 10-636, 10-637, 10-638, 10-639, 10-640, 10-641, 10-642, 10-643, 10-644, 10-645, 10-646, 10-647, 10-648, 10-649, 10-650, 10-651, 10-652, 10-653, 10-654, 10-655, 10-656, 10-657, 10-659, 10-660, 10-661, 10-662, 10-663, 10-664, 10-665, 10-666, 10-667, 10-668, 10-669, 10-670, 10-671, 10-672, 10-673, 10-674, 10-675, 10-676, 10-677, 10-678, 10-679, 10-680, 10-681, 10-682, 10-683, 10-684, 10-685, 10-686, 10-687, 10-688, 10-689, 10-690, 10-691, 10-692, 10-693, 10-694, 10-695, 10-696, 10-698, 10-699, 11-001, 11-002, 12-001, 12-002, 12-003, 12-004, 12-005, 12-006, 12-007, 12-008, 12-009, 12-010, 12-011, 12-012, 12-013, 12-014, 12-015, 12-016, 12-017, 12-018, 12-019, 13-001, 14-002, 14-003.

### Test Example 5: Test of control effect on cucumber powdery mildew

In a plastic pot of 90 cm³, cucumber (variety: Sagami Hanjiro) was planted. In the cotyledon stage, the test chemical solution was sprayed by 5 ml. After air drying, the cucumber was placed in an air-conditioned greenhouse (temperature: 20°C, humidity: 70%RH), and a conidium suspension of a fungus (Erysiphe polygoni) which causes cucumber powdery mildew was inoculated by spraying. After keeping for 9 days, the proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-002, 1-003, 1-004, 1-006, 1-007, 1-008, 1-009, 1-010, 1-011, 1-012, 1-013, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-033, 1-034, 1-036, 1-038, 1-039, 1-040, 1-041, 1-042, 1-043, 1-044, 1-045, 1-046, 1-047, 1-048, 1-049, 1-050, 1-052, 1-053, 1-054, 1-055, 1-056, 1-057, 1-058, 1-059, 1-060, 1-062, 1-064, 1-066, 1-067, 1-068, 1-069, 1-070, 1-071, 1-073, 1-075, 1-076, 1-077, 1-078, 1-079, 1-080, 1-081, 1-082, 1-083, 1-084, 1-085, 1-086, 1-087, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-209, 1-210, 1-211, 1-212, 1-213, 1-214, 1-215, 1-216, 1-217, 1-218, 1-219, 1-220, 1-221, 1-222, 1-223, 1-224, 1-225, 1-226, 1-227, 1-228, 1-229, 1-230, 1-231, 1-232, 1-233, 1-234, 1-236, 1-237, 1-238, 1-239, 1-240, 2-001, 2-002, 2-003, 2-006, 2-007, 2-008, 2-009, 2-010, 2-011, 2-013, 2-014, 2-015, 2-016, 2-019, 2-022, 2-023, 2-025, 2-026, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-047, 2-048, 2-049, 2-050, 2-051, 2-052, 2-053, 2-054, 2-055, 2-056, 2-057, 2-058, 2-060, 2-061, 2-062, 2-063, 2-065, 2-066, 2-067, 2-068, 2-070, 2-071, 2-072, 2-073, 2-074, 2-075, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 4-003, 4-007, 4-008, 4-009, 4-011, 4-012, 4-013, 4-015, 6-001, 6-002, 6-004, 6-005, 6-006, 6-007, 6-008, 7-001, 8-001, 9-011, 9-012, 9-013, 10-001, 10-002, 10-003, 10-004, 10-005, 10-006, 10-007, 10-008, 10-009, 10-010, 10-011, 10-013, 10-014, 10-016, 10-017, 10-018, 10-019, 10-020, 10-021, 10-024, 10-025, 10-026, 10-027, 10-028, 10-031, 10-032, 10-033, 10-034, 10-035, 10-036, 10-038, 10-039, 10-040, 10-041, 10-042, 10-043, 10-044, 10-045, 10-046, 10-047, 10-048, 10-049, 10-050, 10-051, 10-052, 10-053, 10-054, 10-055, 10-056, 10-057, 10-058, 10-059, 10-060, 10-061, 10-062, 10-063, 10-064, 10-065, 10-068, 10-069, 10-070, 10-071, 10-072, 10-073, 10-075, 10-076, 10-077, 10-078, 10-085, 10-086, 10-087, 10-090, 10-094, 10-095, 10-096, 10-097, 10-098, 10-099, 10-103, 10-104, 10-106, 10-107, 10-109, 10-110, 10-111, 10-112, 10-113, 10-114, 10-115, 10-116, 10-117, 10-118, 10-119, 10-120, 10-121, 10-122, 10-123, 10-124, 10-125, 10-126, 10-127, 10-128, 10-130, 10-131, 10-132, 10-133, 10-134, 10-135, 10-136, 10-137, 10-138, 10-140, 10-141, 10-142, 10-143, 10-144, 10-145, 10-146, 10-147, 10-148, 10-149, 10-150, 10-151, 10-152, 10-153, 10-154, 10-155, 10-156, 10-157, 10-158, 10-159, 10-160, 10-161, 10-162, 10-163, 10-164, 10-165, 10-166, 10-167, 10-168, 10-169, 10-170, 10-171, 10-172, 10-173, 10-174, 10-175, 10-176, 10-177, 10-178, 10-179, 10-180, 10-181, 10-182, 10-183, 10-184, 10-185, 10-186, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-206, 10-207, 10-208, 10-209, 10-210, 10-211, 10-212, 10-213, 10-214, 10-215, 10-216, 10-217, 10-218, 10-219, 10-220, 10-221, 10-222, 10-223, 10-224, 10-225, 10-226, 10-227, 10-228, 10-229, 10-230, 10-234, 10-235, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-264, 10-265, 10-266, 10-267, 10-268, 10-269, 10-270, 10-271, 10-272, 10-273, 10-274, 10-275, 10-276, 10-277, 10-278, 10-279, 10-280, 10-281, 10-282, 10-283, 10-284, 10-285, 10-286, 10-287, 10-288, 10-289, 10-290, 10-291, 10-292, 10-293, 10-294, 10-295, 10-296, 10-297, 10-298, 10-299, 10-300, 10-301, 10-302, 10-303, 10-304, 10-306, 10-307, 10-308, 10-309, 10-310, 10-312, 10-313, 10-314, 10-315, 10-316, 10-317, 10-318, 10-319, 10-320, 10-321, 10-322, 10-323, 10-324, 10-325, 10-326, 10-327, 10-328, 10-329, 10-330, 10-331, 10-332, 10-333, 10-334, 10-335, 10-336, 10-338, 10-340, 10-341, 10-342, 10-343, 10-344, 10-345, 10-346, 10-348, 10-349, 10-350, 10-351, 10-353, 10-354, 10-355, 10-356, 10-357, 10-358, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-386, 10-387, 10-388, 10-389, 10-390, 10-391, 10-397, 10-398, 10-399, 10-400, 10-401, 10-402, 10-403, 10-404, 10-405, 10-406, 10-407, 10-408, 10-409, 10-410, 10-411, 10-412, 10-413, 10-414, 10-415, 10-416, 10-417, 10-418, 10-419, 10-420, 10-421, 10-422, 10-423, 10-424, 10-426, 10-427, 10-430, 10-431, 10-432, 10-435, 10-436, 10-437, 10-438, 10-439, 10-440, 10-441, 10-443, 10-444, 10-445, 10-446, 10-447, 10-449, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-468, 10-469, 10-470, 10-471, 10-472, 10-473, 10-474, 10-475, 10-476, 10-477, 10-478, 10-479, 10-480, 10-481, 10-482, 10-483, 10-484, 10-485, 10-486, 10-487, 10-488, 10-489, 10-490, 10-491, 10-492, 10-493, 10-498, 10-499, 10-500, 10-501, 10-502, 10-503, 10-504, 10-505, 10-507, 10-510, 10-511, 10-512, 10-514, 10-517, 10-518, 10-519, 10-520, 10-521, 10-522, 10-523, 10-525, 10-526, 10-528, 10-529, 10-530, 10-531, 10-532, 10-533, 10-534, 10-535, 10-536, 10-537, 10-538, 10-540, 10-545, 10-546, 10-547, 10-548, 10-549, 10-550, 10-551, 10-552, 10-553, 10-554, 10-555, 10-556, 10-557, 10-558, 10-559, 10-560, 10-561, 10-562, 10-563, 10-564, 10-565, 10-566, 10-567, 10-568, 10-569, 10-570, 10-571, 10-572, 10-573, 10-574, 10-575, 10-576, 10-577, 10-578, 10-579, 10-580, 10-581, 10-582, 10-583, 10-584, 10-585, 10-586, 10-587, 10-588, 10-589, 10-590, 10-591, 10-592, 10-593, 10-594, 10-595, 10-596, 10-597, 10-598, 10-599, 10-600, 10-601, 10-602, 10-603, 10-604, 10-605, 10-606, 10-607, 10-608, 10-609, 10-610, 10-611, 10-612, 10-614, 10-615, 10-616, 10-617, 10-618, 10-619, 10-620, 10-621, 10-622, 10-623, 10-624, 10-625, 10-626, 10-627, 10-628, 10-630, 10-631, 10-632, 10-633, 10-634, 10-635, 10-636, 10-637, 10-638, 10-639, 10-640, 10-641, 10-642, 10-643, 10-644, 10-645, 10-646, 10-648, 10-649, 10-650, 10-651, 10-652, 10-654, 10-655, 10-656, 10-657, 10-660, 10-661, 10-662, 10-663, 10-665, 10-666, 10-667, 10-668, 10-669, 10-670, 10-671, 10-672, 10-674, 10-675, 10-676, 10-677, 10-678, 10-679, 10-680, 10-681, 10-682, 10-683, 10-684, 10-685, 10-686, 10-687, 10-688, 10-689, 10-691, 10-693, 10-694, 10-696, 10-698, 10-699, 11-002, 12-001, 12-002, 12-004, 12-005, 12-006, 12-007, 12-008, 12-009, 12-010, 12-011, 12-012, 12-013, 12-014, 12-015, 12-017, 12-018, 12-019, 14-001, 14-002, 14-003, 14-004.

### Test Example 6: Test of control effect on cucumber anthracnose

In a plastic pot of 90 cm³, cucumber (variety: Sagami Hanjiro) was planted. In the cotyledon stage, the test chemical solution was sprayed by 5 ml. After 1 day from the spraying, a conidium suspension of a fungus (Colletotrichum lagenarium) which causes cucumber anthracnose was inoculated onto the cucumber by spraying, and the cucumber was kept in an inoculation box at temperature of 25°C and humidity of 100%RH for 2 days. After that, the cucumber was placed in an air-conditioned greenhouse (temperature: 23°C, humidity: 60%RH) and kept for 7 days. The proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-021, 1-022, 1-023, 1-024, 1-025, 1-026, 1-027, 1-028, 1-029, 1-030, 1-031, 1-032, 1-033, 1-035, 1-036, 1-037, 1-038, 1-039, 1-040, 1-041, 1-042, 1-043, 1-044, 1-045, 1-046, 1-047, 1-048, 1-049, 1-051, 1-052, 1-053, 1-054, 1-055, 1-056, 1-057, 1-058, 1-059, 1-060, 1-061, 1-062, 1-063, 1-064, 1-065, 1-066, 1-067, 1-068, 1-069, 1-070, 1-071, 1-072, 1-073, 1-074, 1-075, 1-076, 1-077, 1-078, 1-079, 1-080, 1-081, 1-082, 1-083, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-102, 1-103, 1-104, 1-105, 1-106, 1-107, 1-108, 1-109, 1-110, 1-111, 1-112, 1-113, 1-114, 1-115, 1-116, 1-117, 1-118, 1-119, 1-120, 1-121, 1-122, 1-123, 1-124, 1-125, 1-126, 1-127, 1-128, 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-152, 1-154, 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179, 1-180, 1-182, 1-183, 1-184, 1-185, 1-186, 1-187, 1-188, 1-189, 1-190, 1-191, 1-192, 1-193, 1-194, 1-195, 1-196, 1-197, 1-198, 1-199, 1-200, 1-201, 1-202, 1-203, 1-204, 1-205, 1-206, 1-207, 1-208, 1-210, 1-211, 1-212, 1-213, 1-214, 1-215, 1-216, 1-217, 1-218, 1-219, 1-220, 1-221, 1-222, 1-223, 1-224, 1-225, 1-226, 1-227, 1-228, 1-229, 1-230, 1-231, 1-232, 1-233, 1-234, 1-236, 1-238, 1-239, 1-240, 2-001, 2-002, 2-003, 2-004, 2-005, 2-006, 2-007, 2-008, 2-009, 2-010, 2-011, 2-012, 2-013, 2-014, 2-015, 2-016, 2-017, 2-018, 2-019, 2-020, 2-021, 2-022, 2-023, 2-024, 2-025, 2-026, 2-027, 2-028, 2-029, 2-030, 2-031, 2-032, 2-033, 2-034, 2-035, 2-036, 2-037, 2-038, 2-039, 2-040, 2-041, 2-042, 2-043, 2-044, 2-045, 2-046, 2-047, 2-048, 2-049, 2-050, 2-051, 2-052, 2-053, 2-054, 2-055, 2-056, 2-057, 2-058, 2-059, 2-060, 2-061, 2-062, 2-063, 2-064, 2-065, 2-066, 2-067, 2-069, 2-070, 2-071, 2-072, 2-073, 2-074, 2-075, 2-076, 2-077, 2-078, 2-079, 2-080, 2-081, 2-082, 2-083, 2-084, 2-085, 2-086, 2-087, 2-088, 2-089, 2-090, 2-091, 2-092, 2-093, 2-094, 2-095, 2-096, 2-097, 2-098, 2-099, 2-100, 2-101, 2-102, 2-103, 2-104, 2-105, 2-106, 2-107, 2-108, 2-109, 2-110, 2-111, 2-112, 2-113, 2-114, 2-115, 2-116, 2-117, 2-118, 2-119, 2-120, 2-121, 2-122, 2-123, 2-124, 2-125, 2-126, 2-127, 2-128, 2-129, 2-130, 2-131, 2-132, 2-133, 2-134, 2-135, 2-136, 2-137, 2-138, 2-139, 2-140, 2-141, 2-142, 2-143, 2-144, 2-145, 2-146, 2-147, 2-148, 2-149, 2-150, 2-151, 2-152, 2-153, 2-154, 2-155, 2-156, 2-157, 2-158, 3-001, 3-002, 3-003, 3-004, 3-005, 3-006, 3-007, 3-008, 3-009, 3-010, 3-011, 3-012, 3-013, 3-014, 3-015, 3-016, 3-017, 3-018, 3-019, 3-020, 3-021, 3-022, 3-023, 3-024, 3-025, 3-026, 3-027, 3-028, 3-029, 3-030, 3-031, 3-032, 4-001, 4-002, 4-003, 4-004, 4-005, 4-007, 4-008, 4-009, 4-010, 4-011, 4-012, 4-013, 4-015, 6-001, 6-002, 6-004, 6-005, 6-006, 6-007, 6-008, 7-001, 8-001, 9-001, 9-002,9-003, 9-004, 9-005, 9-006, 9-007, 9-008, 9-009, 9-010, 9-011, 9-012, 9-013, 9-014, 9-015, 9-016, 9-017, 10-001, 10-002, 10-003, 10-004, 10-005, 10-006, 10-007, 10-008, 10-009, 10-010, 10-011, 10-012, 10-013, 10-014, 10-015, 10-016, 10-017, 10-018, 10-019, 10-020, 10-021, 10-022, 10-023, 10-024, 10-025, 10-026, 10-027, 10-028, 10-029, 10-030, 10-031, 10-032, 10-033, 10-034, 10-035, 10-036, 10-037, 10-038, 10-039, 10-040, 10-041, 10-042, 10-043, 10-044, 10-045, 10-046, 10-047, 10-048, 10-049, 10-050, 10-051, 10-052, 10-053, 10-054, 10-055, 10-056, 10-057, 10-058, 10-059, 10-060, 10-061, 10-062, 10-063, 10-064, 10-065, 10-066, 10-067, 10-068, 10-069, 10-070, 10-071, 10-072, 10-073, 10-074, 10-075, 10-076, 10-077, 10-078, 10-085, 10-086, 10-087, 10-088, 10-089, 10-090, 10-094, 10-095, 10-096, 10-097, 10-098, 10-099, 10-103, 10-104, 10-106, 10-107, 10-109, 10-110, 10-111, 10-112, 10-113, 10-114, 10-115, 10-116, 10-117, 10-118, 10-119, 10-120, 10-121, 10-122, 10-123, 10-124, 10-126, 10-127, 10-128, 10-129, 10-130, 10-131, 10-132, 10-133, 10-134, 10-135, 10-136, 10-137, 10-138, 10-139, 10-140, 10-141, 10-142, 10-143, 10-144, 10-145, 10-146, 10-147, 10-148, 10-149, 10-151, 10-152, 10-153, 10-154, 10-155, 10-156, 10-157, 10-158, 10-159, 10-160, 10-161, 10-162, 10-163, 10-164, 10-165, 10-166, 10-167, 10-168, 10-169, 10-170, 10-171, 10-172, 10-173, 10-174, 10-175, 10-176, 10-177, 10-178, 10-179, 10-180, 10-181, 10-182, 10-183, 10-184, 10-185, 10-186, 10-187, 10-188, 10-189, 10-190, 10-191, 10-192, 10-193, 10-194, 10-195, 10-196, 10-197, 10-198, 10-199, 10-200, 10-201, 10-202, 10-203, 10-204, 10-205, 10-206, 10-207, 10-208, 10-209, 10-210, 10-211, 10-212, 10-213, 10-214, 10-215, 10-216, 10-217, 10-218, 10-219, 10-220, 10-221, 10-222, 10-223, 10-224, 10-225, 10-226, 10-227, 10-228, 10-229, 10-230, 10-231, 10-232, 10-233, 10-234, 10-235, 10-236, 10-237, 10-238, 10-239, 10-240, 10-241, 10-242, 10-243, 10-244, 10-245, 10-246, 10-247, 10-248, 10-249, 10-250, 10-251, 10-252, 10-253, 10-254, 10-255, 10-256, 10-257, 10-258, 10-259, 10-260, 10-261, 10-262, 10-263, 10-264, 10-265, 10-266, 10-267, 10-268, 10-269, 10-270, 10-271, 10-272, 10-273, 10-274, 10-275, 10-276, 10-277, 10-278, 10-279, 10-280, 10-281, 10-282, 10-283, 10-284, 10-285, 10-286, 10-287, 10-288, 10-289, 10-290, 10-291, 10-292, 10-293, 10-294, 10-295, 10-296, 10-297, 10-298, 10-299, 10-300, 10-301, 10-302, 10-303, 10-304, 10-305, 10-306, 10-307, 10-308, 10-309, 10-310, 10-311, 10-312, 10-313, 10-314, 10-315, 10-316, 10-317, 10-318, 10-319, 10-320, 10-321, 10-322, 10-323, 10-324, 10-325, 10-326, 10-327, 10-328, 10-329, 10-330, 10-331, 10-332, 10-333, 10-334, 10-335, 10-336, 10-337, 10-338, 10-339, 10-340, 10-341, 10-342, 10-343, 10-344, 10-345, 10-346, 10-347, 10-348, 10-349, 10-350, 10-351, 10-352, 10-353, 10-354, 10-355, 10-356, 10-357, 10-358, 10-359, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-386, 10-387, 10-388, 10-389, 10-390, 10-391, 10-393, 10-394, 10-396, 10-397, 10-398, 10-399, 10-400, 10-401, 10-402, 10-403, 10-404, 10-405, 10-406, 10-407, 10-408, 10-409, 10-410, 10-411, 10-412, 10-413, 10-414, 10-415, 10-416, 10-417, 10-418, 10-419, 10-420, 10-421, 10-422, 10-423, 10-424, 10-425, 10-426, 10-427, 10-429, 10-430, 10-431, 10-432, 10-433, 10-434, 10-435, 10-436, 10-437, 10-439, 10-440, 10-441, 10-442, 10-444, 10-445, 10-446, 10-447, 10-449, 10-450, 10-451, 10-452, 10-453, 10-454, 10-455, 10-456, 10-457, 10-458, 10-459, 10-460, 10-461, 10-462, 10-463, 10-464, 10-465, 10-466, 10-467, 10-468, 10-469, 10-470, 10-471, 10-472, 10-473, 10-474, 10-475, 10-476, 10-477, 10-478, 10-479, 10-480, 10-481, 10-482, 10-483, 10-484, 10-485, 10-486, 10-487, 10-488, 10-489, 10-490, 10-491, 10-492, 10-493, 10-494, 10-495, 10-496, 10-497, 10-498, 10-499, 10-500, 10-501, 10-502, 10-503, 10-504, 10-505, 10-507, 10-508, 10-510, 10-511, 10-512, 10-514, 10-517, 10-518, 10-519, 10-520, 10-521, 10-522, 10-523, 10-524, 10-525, 10-526, 10-527, 10-528, 10-530, 10-531, 10-532, 10-533, 10-534, 10-536, 10-537, 10-538, 10-539, 10-540, 10-541, 10-542, 10-545, 10-546, 10-547, 10-548, 10-549, 10-550, 10-551, 10-552, 10-553, 10-554, 10-555, 10-556, 10-557, 10-558, 10-559, 10-560, 10-561, 10-562, 10-563, 10-564, 10-566, 10-567, 10-568, 10-569, 10-570, 10-571, 10-572, 10-573, 10-574, 10-575, 10-577, 10-578, 10-579, 10-580, 10-581, 10-582, 10-583, 10-584, 10-585, 10-586, 10-588, 10-590, 10-591, 10-592, 10-593, 10-594, 10-595, 10-596, 10-597, 10-598, 10-599, 10-600, 10-601, 10-602, 10-603, 10-604, 10-605, 10-606, 10-607, 10-608, 10-609, 10-610, 10-611, 10-612, 10-613, 10-614, 10-615, 10-616, 10-617, 10-618, 10-619, 10-621, 10-622, 10-623, 10-624, 10-625, 10-626, 10-627, 10-628, 10-629, 10-630, 10-631, 10-632, 10-633, 10-634, 10-635, 10-636, 10-637, 10-638, 10-639, 10-640, 10-641, 10-642, 10-643, 10-644, 10-645, 10-646, 10-647, 10-648, 10-649, 10-650, 10-651, 10-652, 10-654, 10-655, 10-656, 10-657, 10-658, 10 -659, 10-660, 10-661, 10-662, 10-663, 10-664, 10-665, 10-666, 10-667, 10-668, 10-669, 10-670, 10-671, 10-672, 10-673, 10-674, 10-675, 10-676, 10-677, 10-678, 10-679, 10-680, 10-681, 10-682, 10-683, 10-684, 10-685, 10-686, 10-687, 10-688, 10-689, 10-690, 10-691, 10-692, 10-693, 10-694, 10-695, 10-696, 10-697, 10-698, 10-699, 11-001, 11-002, 12-001, 12-002, 12-003, 12-004, 12-005, 12-006, 12-007, 12-008, 12-009, 12-010, 12-011, 12-012, 12-013, 12-014, 12-015, 12-016, 12-017, 12-018, 12-019, 13-001, 13-002, 14-001, 14-002, 14-003, 14-004.

### Test Example 7: Test of control effect on soybean rust

In a plastic pot of 90 cm³, soybean (variety: Enrei) was planted. In the unifoliolate leaf stage, the test chemical solution was sprayed by 5 ml. After 1 day from the spraying, a conidium suspension of a fungus (Phakopsora pachyrhizi) which causes soybean rust was inoculated onto the soybean by spraying, and the soybean was kept in an inoculation box at temperature of 20°C and humidity of 100%RH for 2 days. After that, the soybean was placed in an air-conditioned greenhouse (temperature: 20°C, humidity: 60%RH) and kept for 10 days. The proportion of formed lesions to the inoculated leaf was measured, and a control value was calculated in accordance with a calculation formula identical with that of Test Example 1.

As a result, the following compounds exhibited a control value of 70% or more among the tested compounds.

Compounds: No. 1-001, 1-003, 1-007, 1-010, 1-011, 1-015, 1-017, 1-018, 1-019, 1-021, 1-024, 1-025, 1-027, 1-028, 1-029, 1-031, 1-033, 1-034, 1-036, 1-038, 1-041, 1-044, 1-045, 1-046, 1-047, 1-049, 1-051, 1-053, 1-058, 1-059, 1-060, 1-064, 1-068, 1-069, 1-075, 1-076, 1-081, 1-083, 1-084, 1-085, 1-086, 1-088, 1-090, 1-092, 1-093, 1-095, 1-099, 1-108, 1-109, 1-110, 1-111, 1-112, 1-113, 1-114, 1-115, 1-116, 1-117, 1-118, 1-121, 1-122, 1-123, 1-124, 1-125, 1-126, 1-129, 1-131, 1-132, 1-134, 1-135, 1-136, 1-137, 1-139, 1-141, 1-142, 1-143, 1-146, 1-148, 1-149, 1-150, 1-155, 1-159, 1-161, 1-167, 1-169, 1-170, 1-171, 1-172, 1-173, 1-175, 1-176, 1-177, 1-178, 1-182, 1-183, 1-184, 1-185, 1-186, 1-187, 1-188, 1-189, 1-190, 1-191, 1-192, 1-194, 1-195, 1-196, 1-197, 1-198, 1-199, 1-200, 1-204, 1-205, 1-206, 1-207, 1-208, 1-209, 1-210, 1-211, 1-213, 1-214, 1-215, 1-217, 1-219, 1-220, 1-221, 1-222, 1-224, 1-227, 1-228, 1-229, 1-231, 1-232, 1-236, 1-237, 1-239, 2-006, 2-011, 2-012, 2-013, 2-019, 2-020, 2-026, 2-028, 2-030, 2-032, 2-033, 2-034, 2-035, 2-040, 2-041, 2-042, 2-044, 2-045, 2-047, 2-048, 2-049, 2-050, 2-066, 2-067, 2-069, 2-072, 2-077, 2-087, 2-088, 2-093, 2-094, 2-095, 2-096, 2-097, 2-098, 2-099, 2-100, 2-102, 2-103, 2-104, 2-106, 2-107, 2-108, 2-109, 2-110, 2-111, 2-112, 2-113, 2-115, 2-117, 2-118, 2-119, 2-122, 2-125, 2-126, 2-127, 2-128, 2-129, 2-130, 2-131, 2-132, 2-134, 2-135, 2-136, 2-137, 2-138, 2-141, 2-142, 2-143, 2-145, 2-148, 2-152, 2-153, 2-154, 2-156, 2-157, 2-158, 3-001, 3-002, 3-003, 3-005, 3-006, 3-012, 3-014, 3-015, 3-016, 3-017, 3-018, 3-019, 3-021, 3-023, 3-025, 3-026, 3-027, 3-029, 3-030, 4-005, 4-006, 4-009, 4-010, 4-011, 4-012, 4-013, 4-014, 4-015, 6-005, 6-006, 9-001, 9-004, 9-005, 9-007, 9-009, 9-013, 9-015, 9-016, 9-017, 10-001, 10-002, 10-004, 10-005, 10-017, 10-018, 10-019, 10-020, 10-023, 10-030, 10-034, 10-035, 10-039, 10-041, 10-042, 10-044, 10-045, 10-046, 10-048, 10-049, 10-051, 10-052, 10-053, 10-056, 10-057, 10-066, 10-067, 10-070, 10-073, 10-074, 10-075, 10-076, 10-077, 10-088, 10-089, 10-094, 10-095, 10-097, 10-106, 10-109, 10-110, 10-112, 10-115, 10-117, 10-118, 10-119, 10-120, 10-122, 10-130, 10-132, 10-142, 10-143, 10-147, 10-148, 10-151, 10-152, 10-153, 10-154, 10-156, 10-157, 10-160, 10-162, 10-164, 10-166, 10-167, 10-168, 10-169, 10-170, 10-171, 10-172, 10-173, 10-174, 10-176, 10-177, 10-179, 10-180, 10-182, 10-184, 10-187, 10-190, 10-191, 10-193, 10-195, 10-196, 10-197, 10-199, 10-200, 10-201, 10-204, 10-206, 10-207, 10-208, 10-211, 10-212, 10-214, 10-215, 10-216, 10-217, 10-218, 10-219, 10-220, 10-222, 10-223, 10-224, 10-225, 10-226, 10-230, 10-232, 10-234, 10-235, 10-236, 10-237, 10-238, 10-239, 10-240, 10-241, 10-242, 10-247, 10-249, 10-252, 10-254, 10-256, 10-257, 10-258, 10-260, 10-261, 10-262, 10-264, 10-265, 10-267, 10-268, 10-271, 10-272, 10-274, 10-275, 10-276, 10-278, 10-281, 10-282, 10-283, 10-284, 10-285, 10-286, 10-289, 10-290, 10-291, 10-292, 10-294, 10-296, 10-298, 10-300, 10-301, 10-302, 10-305, 10-309, 10-311, 10-313, 10-314, 10-315, 10-320, 10-321, 10-323, 10-324, 10-327, 10-330, 10-338, 10-342, 10-343, 10-344, 10-345, 10-346, 10-347, 10-348, 10-349, 10-350, 10-351, 10-353, 10-355, 10-356, 10-357, 10-359, 10-360, 10-361, 10-362, 10-363, 10-364, 10-365, 10-366, 10-367, 10-368, 10-369, 10-370, 10-371, 10-372, 10-373, 10-374, 10-375, 10-376, 10-377, 10-378, 10-379, 10-380, 10-381, 10-382, 10-383, 10-384, 10-385, 10-396, 10-397, 10-398, 10-400, 10-402, 10-403, 10-404, 10-405, 10-406, 10-407, 10-411, 10-413, 10-419, 10-421, 10-422, 10-424, 10-427, 10-428, 10-429, 10-430, 10-431, 10-433, 10-434, 10-435, 10-436, 10-437, 10-438, 10-440, 10-443, 10-444, 10-450, 10-451, 10-452, 10-453, 10-454, 10-455, 10-456, 10-457, 10-458, 10-463, 10-466, 10-468, 10-469, 10-473, 10-474, 10-480, 10-482, 10-484, 10-485, 10-486, 10-487, 10-489, 10-494, 10-497, 10-498, 10-499, 10-506, 10-557, 10-560, 10-561, 10-562, 10-563, 10-567, 10-568, 10-571, 10-572, 10-573, 10-584, 10-586, 10-589, 10-595, 10-597, 10-599, 10-607, 10-616, 10-619, 10-622, 10-633, 10-636, 10-638, 10-639, 10-640, 10-641, 10-642, 10-643, 10-644, 10-645, 10-647, 10-648, 10-649, 10-650, 10-651, 10-652, 10-653, 10-656, 10-657, 10-659, 10-673, 10-674, 10-675, 10-677, 10-680, 10-681, 10-682, 10-683, 10-684, 10-685, 10-686, 10-687, 10-688, 10-689, 10-691, 10-692, 10-694, 10-695, 10-696, 10-697, 11-002, 12-002, 12-005, 12-006, 12-007, 12-008, 12-009, 12-010, 12-016, 12-018, 12-019, 13-002, 14-001, 14-002, 14-003.

### Industrial Applicability

The pyrazole compound in accordance with the present invention is an extremely useful compound that exhibits excellent pest control activity, in particular, fungicidal activity. Furthermore, the pyrazole compound has little adverse effect on non-target organisms such as mammals, fish, and beneficial insects.

## Claims

1. A pyrazole compound represented by a formula (1) or a salt thereof: 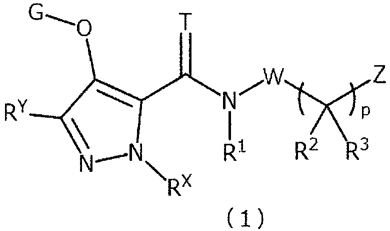
where G represents G-1,
G-1 represents a structure indicated by a structural formula below, 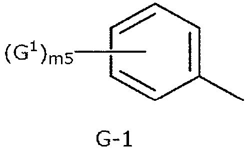
G¹ represents hydroxy, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
in a case where m5 represents an integer of 2, 3, 4 or 5 in relationship to G¹, the respective G¹ may be identical with each other or may be different from each other,
T represents an oxygen atom,
W represents -O- or -N(R^{W})-,
R^{X} represents C₁-C₆ alkyl, C₃-C₁₀ cycloalkyl, C₁-C₆ haloalkyl, benzyl, R^{X}-1, R^{X}-2, R^{X}-3 or R^{X}-4,
R^{X}-1 through R^{X}-4 represent structures indicated by structural formulae below, 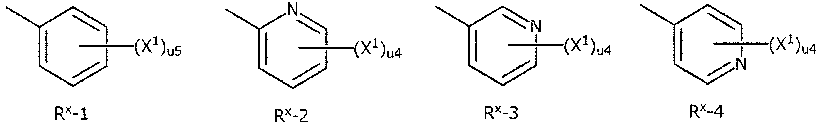
X¹ represents a halogen atom, C₁-C₆ alkyl, C₁-C₆ haloalkyl or C₁-C₆ alkoxy,
in a case where u5 represents an integer of 2, 3, 4 or 5 in relationship to X¹, the respective X¹ may be identical with each other or may be different from each other,
in a case where u4 represents an integer of 2, 3 or 4 in relationship to X¹, the respective X¹ may be identical with each other or may be different from each other,
R^{Y} represents a hydrogen atom, a halogen atom or C₁-C₆ alkyl,
R¹ represents a hydrogen atom, C₁-C₆ alkyl or C₁-C₆ alkyl substituted by Rⁱ,
R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, di(C₁-C₆ alkyl)aminothiocarbonyl, C₁-C₆ alkylsulfonyl, - C(O)OR^{d}, -C(O)C(O)R^{e}, -C(O)SR^{f}, -C(O)R^{g}, Q-1 to Q-53 or Q-54,
R² represents a hydrogen atom, C₁-C₆ alkyl, C₁-C₆ haloalkyl or C₁-C₆ alkyl substituted by R^{h},
R³ represents a hydrogen atom, C₁-C₆ alkyl or C₁-C₆ haloalkyl,
R² represents hydroxy, cyano, C₃-C₁₀ cycloalkyl, C₁-C₆ alkoxy, C₁-C₆ alkylthio, C₁-C₆ alkylsulfinyl, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, -C(N=OR^{b})R^{c}, Q-1 to Q-53 or Q-54,
R^{b} represents C₁-C₆ alkyl,
R^{c} represents C₁-C₆ alkyl,
R^{d} represents a structure indicated by a structural formula below, 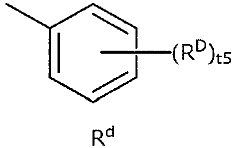
R^{D} represents a halogen atom, nitro or C₁-C₆ alkyl,
in a case where t5 represents an integer of 2, 3, 4 or 5 in relationship to R^{D}, the respective R^{D} may be identical with each other or may be different from each other,
R^{e} represents C₁-C₆ alkyl or C₁-C₆ alkoxy,
R^{f} represents C₁-C₆ alkyl,
R^{g} represents Q-1 to Q-53 or Q-54,
R^{h} represents cyano or C₁-C₆ alkoxy,
Rⁱ represents cyano or C₁-C₆ alkoxy,
Z represents Z-1 to Z-49 or Z-50,
Z-1 through Z-50 respectively represent structures indicated by structural formulae below, 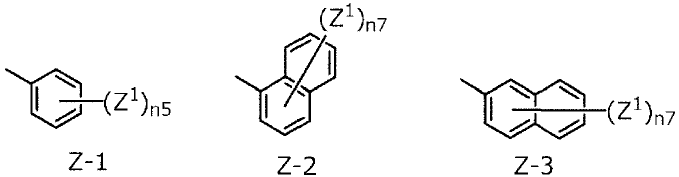 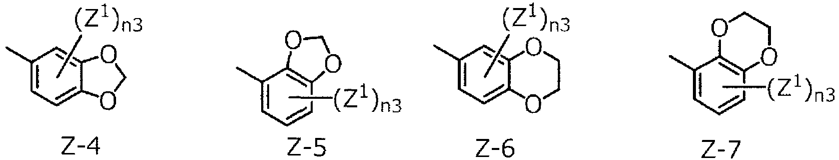 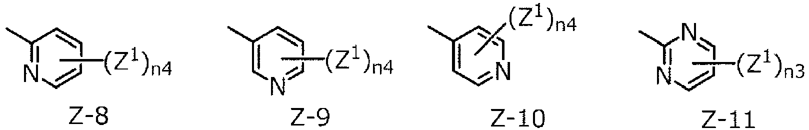 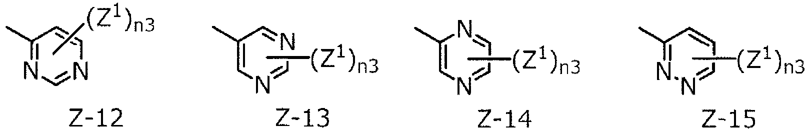 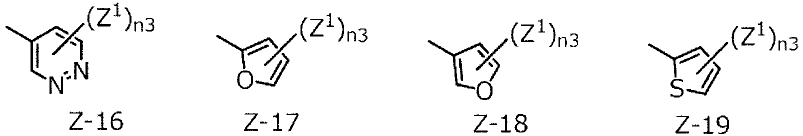 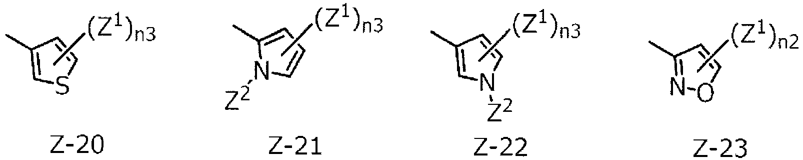 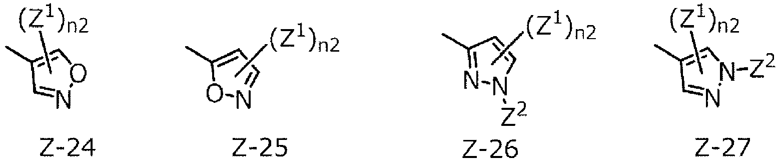 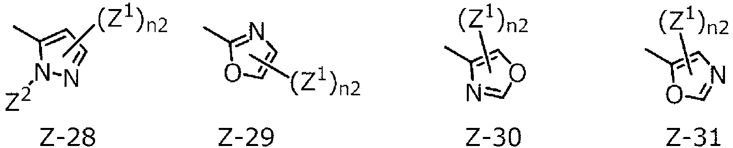 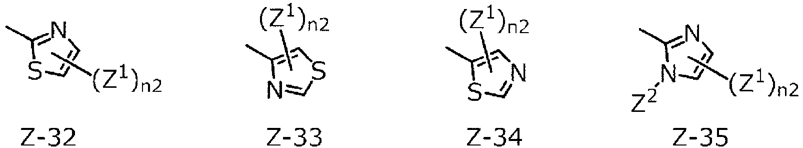 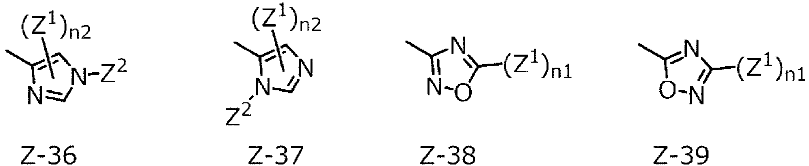 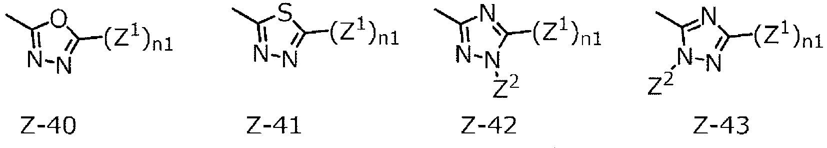 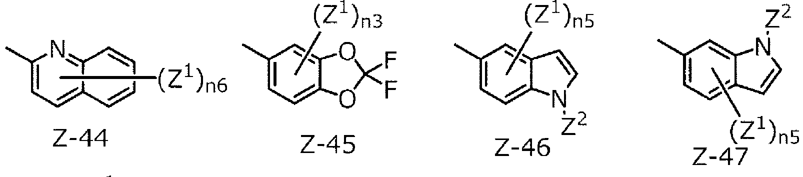 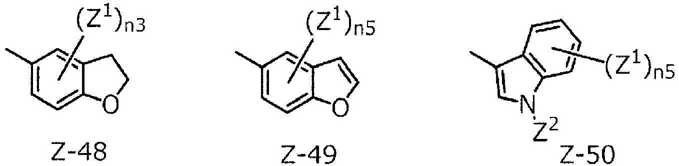
a substitution position of Z¹ indicates that the substitution occurs on an aromatic ring of each of Z-1 through Z-50,
Z¹ represents hydroxy, carboxy, amino, nitro, cyano, a halogen atom, C₁-C₆ alkyl, C₃-C₁₀ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ haloalkylthio, C₁-C₆ alkylsulfinyl, C₁-C₆ alkylsulfonyl, C₁-C₆ alkylcarbonyl, C₁-C₆ alkoxycarbonyl, - OR^{j}, -NHC(O)R^{k}, -C(O)NHR^{m}, -C(=NOR)R', -C(=NOH)NH₂, E-1 to E-54 or E-55,
in a case where n2 represents an integer of 2 in relationship to Z¹, the respective Z¹ may be identical with each other or may be different from each other,
in a case where n3 represents an integer of 2 or 3 in relationship to Z¹, the respective Z¹ may be identical with each other or may be different from each other,
in a case where n4 represents an integer of 2, 3 or 4 in relationship to Z¹, the respective Z¹ may be identical with each other or may be different from each other,
in a case where n5 represents an integer of 2, 3, 4 or 5 in relationship to Z¹, the respective Z¹ may be identical with each other or may be different from each other,
in a case where n6 represents an integer of 2, 3, 4, 5 or 6 in relationship to Z¹, the respective Z¹ may be identical with each other or may be different from each other,
in a case where n7 represents an integer of 2, 3, 4, 5, 6 or 7 in relationship to Z¹, the respective Z¹ may be identical with each other or may be different from each other,
Z² represents C₁-C₆ alkyl, phenyl or pyridin-2-yl,
R represents a hydrogen atom or C₁-C₆ alkyl,
R' represents a hydrogen atom or C₁-C₆ alkyl,
R^{j} represents C₂-C₆ alkenyl, C₂-C₆ alkynyl, C₁-C₆ alkyl substituted by R⁴, C₁-C₆ alkylcarbonyl, C₁-C₆ alkoxycarbonyl, C₁-C₆ alkylaminocarbonyl, E-1 to E-54 or E-55,
R⁴ represents cyano, C₁-C₆ alkoxy, C₃-C₁₀ cycloalkyl, C₁-C₆ alkylcarbonyl, C₁-C₆ alkoxycarbonyl, di(C₁-C₆ alkyl)aminocarbonyl, E-1 to E-54 or E-55,
R^{k} represents C₁-C₆ alkyl, C₃-C₁₀ cycloalkyl, C₁-C₆ haloalkyl, C₁-C₆ alkyl substituted by R⁵, C₁-C₆ alkoxy, C₂-C₆ alkenyloxy, C₁-C₆ alkylamino, di(C₁-C₆ alkyl)amino, benzyloxy, E-1 to E-54 or E-55,
R⁵ represents cyano, C₁-C₆ alkoxy, E-1 to E-53 or E-54,
R^{m} represents C₁-C₆ alkyl, C₃-C₁₀ cycloalkyl, C₂-C₆ alkenyl, C₁-C₆ alkoxy, benzyl, E-1 to E-54 or E-55,
E-1 through E-55 respectively represent structures indicated by structural formulae below, 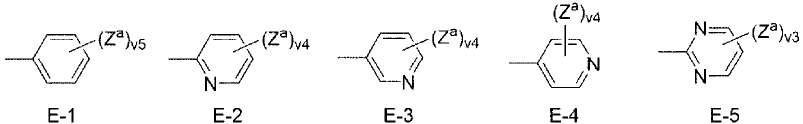 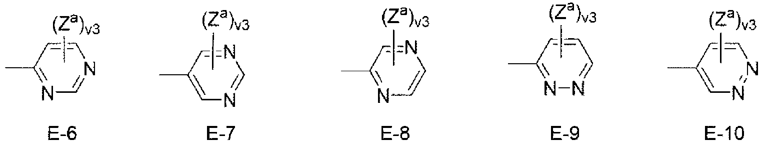 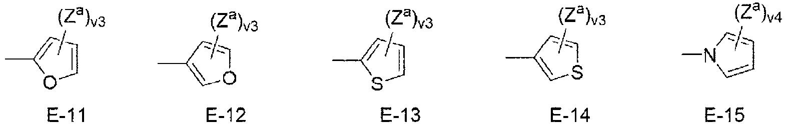 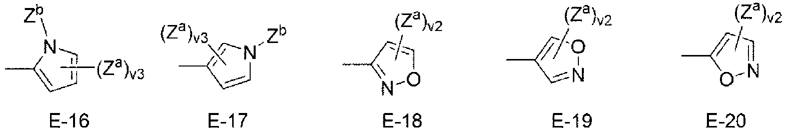 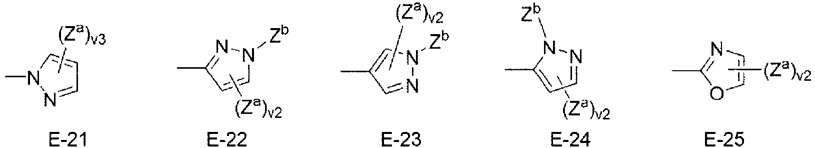 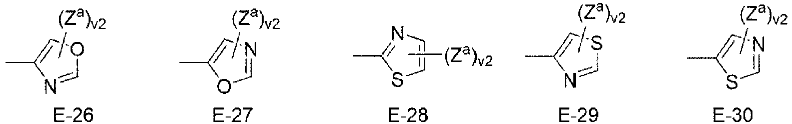 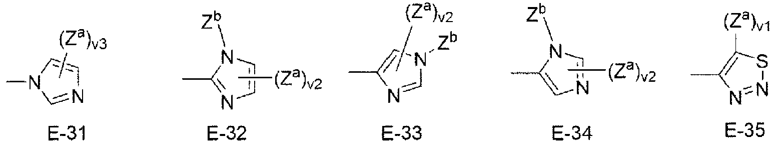 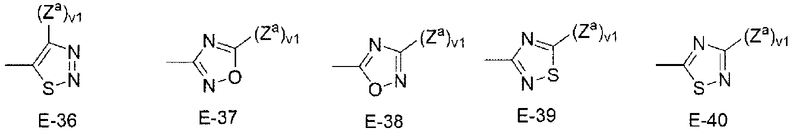 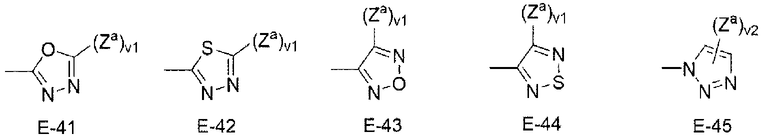 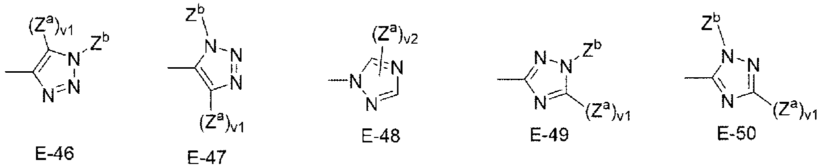 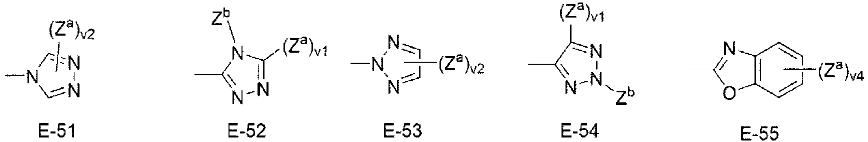
Z^{a} represents a halogen atom, cyano, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ haloalkoxy, C₁-C₆ alkylcarbonyl, C₁-C₆ alkoxycarbonyl, phenyl, S-1 to S-5 or S-6,
in a case where v5 represents an integer of 2, 3, 4 or 5 in relationship to Z^{a}, the respective Z^{a} may be identical with each other or may be different from each other,
in a case where v4 represents an integer of 2, 3 or 4 in relationship to Z^{a}, the respective Z^{a} may be identical with each other or may be different from each other,
in a case where v3 represents an integer of 2 or 3 in relationship to Z^{a}, the respective Z^{a} may be identical with each other or may be different from each other,
in a case where v2 represents an integer of 2 in relationship to Z^{a}, the respective Z^{a} may be identical with each other or may be different from each other,
R⁶ represents hydroxy, C₁-C₆ alkoxy or C₁-C₆ alkoxycarbonyl,
Z^{b} represents C₁-C₆ alkyl,
S-1 through S-6 respectively represent structures indicated by structural formulae below, 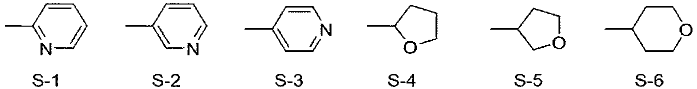
Q-1 through Q-54 respectively represent structures indicated by structural formulae below, 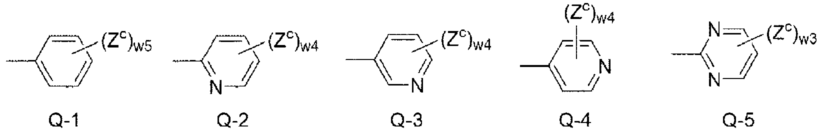 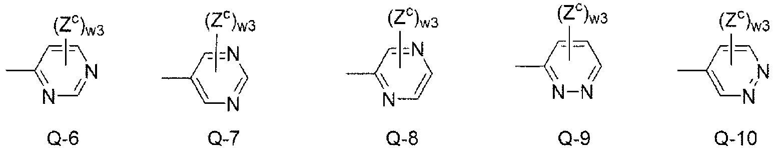 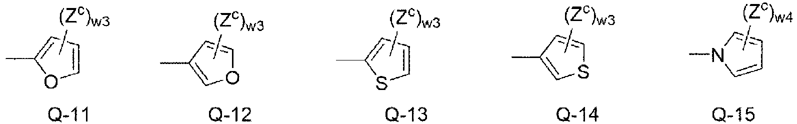 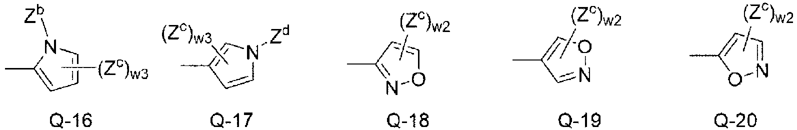 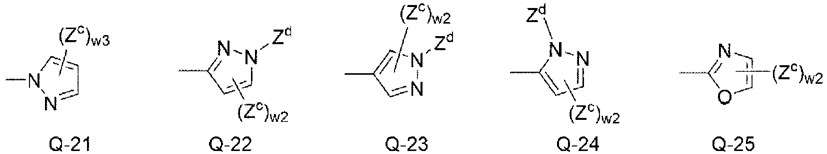 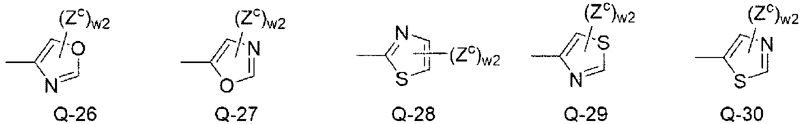 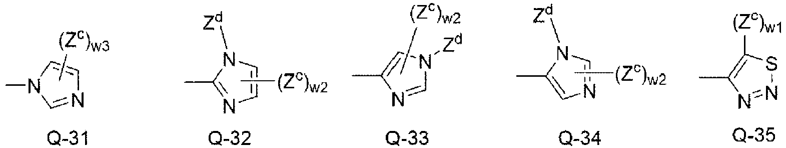 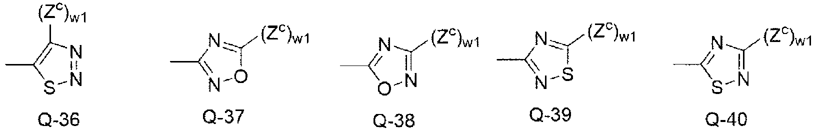 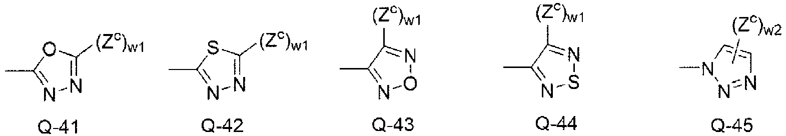 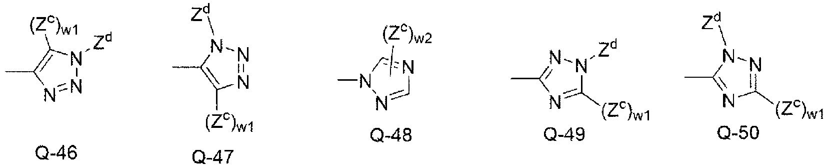 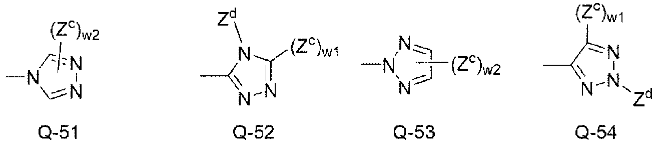
Z^{c} represents a halogen atom, cyano, C₁-C₆ alkyl, C₁-C₆ haloalkyl, C₁-C₆ alkoxy, C₁-C₆ haloalkoxy, C₁-C₆ alkylcarbonyl or C₁-C₆ alkoxycarbonyl,
in a case where w5 represents an integer of 2, 3, 4 or 5 in relationship to Z^{c}, the respective Z^{c} may be identical with each other or may be different from each other,
in a case where w4 represents an integer of 2, 3 or 4 in relationship to Z^{c}, the respective Z^{c} may be identical with each other or may be different from each other,
in a case where w3 represents an integer of 2 or 3 in relationship to Z^{c}, the respective Z^{c} may be identical with each other or may be different from each other,
in a case where w2 represents an integer of 2 in relationship to Z^{c}, the respective Z^{c} may be identical with each other or may be different from each other,
Z^{d} represents C₁-C₆ alkyl,
m5 represents an integer of 0, 1, 2, 3, 4 or 5,
n2 represents an integer of 0, 1 or 2,
n3 represents an integer of 0, 1, 2 or 3,
n4 represents an integer of 0, 1, 2, 3 or 4,
n5 represents an integer of 0, 1, 2, 3, 4 or 5,
n6 represents an integer of 0, 1, 2, 3, 4, 5 or 6,
n7 represents an integer of 0, 1, 2, 3, 4, 5, 6 or 7,
u4 represents an integer of 0, 1, 2, 3 or 4,
u5 represents an integer of 0, 1, 2, 3, 4 or 5,
t5 represents an integer of 0, 1, 2, 3, 4 or 5,
p represents an integer of 0 or 1,
v1 represents an integer of 0 or 1,
v2 represents an integer of 0, 1 or 2,
v3 represents an integer of 0, 1, 2 or 3,
v4 represents an integer of 0, 1, 2, 3 or 4,
v5 represents an integer of 0, 1, 2, 3, 4 or 5,
w1 represents an integer of 0 or 1,
w2 represents an integer of 0, 1 or 2,
w3 represents an integer of 0, 1, 2 or 3,
w4 represents an integer of 0, 1, 2, 3 or 4, and
w5 represents an integer of 0, 1, 2, 3, 4 or 5.

2. The pyrazole compound as set forth in claim 1 or a salt thereof, wherein:
G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
R^{X} represents C₁-C₆ alkyl, R^{X}-1, R^{X}-2, R^{X}-3 or R^{X}-4,
R^{Y} represents a hydrogen atom or a halogen atom,
R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
R² represents a hydrogen atom, C₁-C₆ alkyl or C₁-C₆ alkyl substituted by R^{h},
R³ represents a hydrogen atom,
R^{a} represents hydroxy, cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, - C(N=OR^{b})R^{c} or Q-1,
R^{D} represents a halogen atom or nitro,
R^{e} represents C₁-C₆ alkoxy,
R^{g} represents Q-1, Q-2, Q-3, Q-4 or Q-11,
R^{h} represents C₁-C₆ alkoxy,
Rⁱ represents cyano,
Z represents Z-1, Z-2, Z-3, Z-4, Z-6, Z-8, Z-9, Z-17, Z-19, Z-25, Z-27, Z-28, Z-34, Z-44, Z-45, Z-46, Z-47, Z-48, Z-49 or Z-50,
in a case where W is -O-, Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -NHC(O)R^{k}, -C(O)NHR^{m}, -C(=NOR)R', - C(=NOH)NH₂, E-1 to E-4, E-13, E-14, E-25, E-28, E-37, E-38, E-41 or E-55,
in a case where W is -N(R^{W})-, Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, - OR^{j} or E-1,
R represents C₁-C₆ alkyl,
R' represents C₁-C₆ alkyl,
R^{j} represents C₂-C₆ alkenyl, C₂-C₆ alkynyl, C₁-C₆ alkyl substituted by R⁴ C₁-C₆ alkoxycarbonyl or E-1,
R⁴ represents C₁-C₆ alkoxy, C₃-C₁₀ cycloalkyl, E-1, E-2 or E-3,
R⁵ represents C₁-C₆ alkoxy,
R^{m} represents C₁-C₆ alkyl, C₃-C₁₀ cycloalkyl, C₂-C₆ alkenyl, C₁-C₆ alkoxy, benzyl, E-1 or E-28,
Z^{a} represents a halogen atom, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ alkoxycarbonyl, phenyl, S-1, S-4 or S-6,
m5 represents an integer of 0, 1 or 2,
n3 represents an integer of 0 or 1,
n4 represents an integer of 1 or 2,
n5 represents an integer of 0, 1, 2 or 3,
n6 represents an integer of 0,
n7 represents an integer of 0 or 1,
u4 represents an integer of 0,
u5 represents an integer of 0,
t5 represents an integer of 1,
v2 represents an integer of 1,
v3 represents an integer of 0,
v4 represents an integer of 0 or 1,
v5 represents an integer of 0 or 1,
w4 represents an integer of 0, and
w5 represents an integer of 0.

3. The pyrazole compound as set forth in claim 2 or a salt thereof, wherein:
R¹ represents a hydrogen atom,
R^{W} represents C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl or C₁-C₆ alkylaminothiocarbonyl,
in a case where W is -O-, Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -C(=NOH)NH₂, E-1 to E-4, E-13, E-14, E-25, E-28, E-37, E-38, E-41 or E-55,
in a case where W is -N(R^{W})-, Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, - OR^{j} or E-1,
R^{j} represents C₂-C₆ alkenyl, C₂-C₆ alkynyl, C₁-C₆ alkyl substituted by R⁴ or E-1, and
p represents an integer of 1.

4. The pyrazole compound as set forth in claim 1 or a salt thereof, wherein:
W represents -O-.

5. The pyrazole compound as set forth in claim 4 or a salt thereof, wherein:
G¹ represents a halogen atom, C₁-C₆ alkyl, C₁-C₆ haloalkyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
R^{X} represents C₁-C₆ alkyl, R^{X}-1, R^{X}-2 or R^{X}-3,
R^{Y} represents a hydrogen atom or a halogen atom,
R¹ represents a hydrogen atom,
R² represents a hydrogen atom or C₁-C₆ alkyl,
R³ represents a hydrogen atom,
Z represents Z-1, Z-2, Z-3, Z-4, Z-6, Z-8, Z-9, Z-17, Z-19, Z-25, Z-27, Z-28, Z-34, Z-44, Z-45, Z-46, Z-47, Z-48, Z-49 or Z-50,
Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylthio, C₁-C₆ alkylcarbonyl, C₁-C₆ alkoxycarbonyl, -OR^{j}, -NHC(O)R^{k}, - C(O)NHR^{m}, -C(=NOR)R', -C(=NOH)NH₂, E-1 to E-4, E-13, E-14, E-25, E-28, E-37, E-38, E-41 or E-55,
R represents C₁-C₆ alkyl,
R' represents C₁-C₆ alkyl,
R^{j} represents C₂-C₆ alkenyl, C₂-C₆ alkynyl, C₁-C₆ alkyl substituted by R⁴ or E-1,
R⁴ represents C₁-C₆ alkoxy, C₃-C₁₀ cycloalkyl, E-1, E-2 or E-3,
R^{k} represents C₃-C₁₀ cycloalkyl, C₁-C₆ haloalkyl, C₁-C₆ alkyl substituted by R⁵, C₁-C₆ alkoxy, E-1, E-2 or E-3,
R⁵ represents C₁-C₆ alkoxy,
R^{m} represents C₁-C₆ alkyl, C₃-C₁₀ cycloalkyl, C₂-C₆ alkenyl, C₁-C₆ alkoxy, benzyl, E-1 or E-28,
Z^{a} represents a halogen atom, C₁-C₆ alkyl, C₁-C₆ alkyl substituted by R⁶, C₃-C₁₀ cycloalkyl, C₁-C₆ haloalkyl, C₁-C₆ alkoxy, C₁-C₆ alkoxycarbonyl, phenyl, S-1, S-4 or S-6,
m5 represents an integer of 0, 1 or 2,
n2 represents an integer of 0 or 1,
n3 represents an integer of 0 or 1,
n4 represents an integer of 1 or 2,
n5 represents an integer of 0, 1 or 2,
n6 represents an integer of 0,
n7 represents an integer of 0 or 1,
u4 represents an integer of 0,
u5 represents an integer of 0,
v2 represents an integer of 1,
v3 represents an integer of 0,
v4 represents an integer of 0 or 1, and
v5 represents an integer of 0 or 1.

6. The pyrazole compound as set forth in claim 5 or a salt thereof, wherein:
Z¹ represents hydroxy, carboxy, amino, nitro, cyano, a halogen atom, C₁-C₆ alkyl, C₁-C₆ haloalkyl, C₂-C₆ alkenyl, C₁-C₆ alkoxy, C₁-C₆ haloalkoxy, C₁-C₆ alkylcarbonyl, C₁-C₆ alkoxycarbonyl, -OR^{j}, -C(=NOH)NH₂, E-1 to E-4, E-13, E-14, E-25, E-28, E-37, E-38, E-41 or E-55, and
p represents an integer of 1.

7. The pyrazole compound as set forth in claim 1 or a salt thereof, wherein:
W represents -N(R^{W})-.

8. The pyrazole compound as set forth in claim 7 or a salt thereof, wherein:
G¹ represents a halogen atom, C₁-C₆ alkyl, C₃-C₆ cycloalkyl, C₁-C₆ haloalkyl, C₂-C₆ alkenyl, C₁-C₆ alkoxy, C₁-C₆ haloalkoxy, C₁-C₆ alkylthio, C₁-C₆ alkylsulfinyl, C₁-C₆ alkylsulfonyl or di(C₁-C₆ alkyl)amino,
R^{X} represents C₁-C₆ alkyl, R^{X}-1, R^{X}-2, R^{X}-3 or R^{X}-4,
R^{Y} represents a hydrogen atom or a halogen atom,
R^{W} represents a hydrogen atom, C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl) aminocarbonyl, C₁-C₆ alkylaminothiocarbonyl, C₁-C₆ alkylsulfonyl, -C(O)OR^{d}, -C(O)C(O)R^{e}, -C(O)SR^{f}, -C(O)R^{g} or Q-1,
R² represents a hydrogen atom, C₁-C₆ alkyl or C₁-C₆ alkyl substituted by R^{h},
R³ represents a hydrogen atom,
R^{a} represents hydroxy, cyano, C₃-C₁₀ cycloalkyl, C₁-C₆ alkoxy, C₁-C₆ alkylsulfonyl, di(C₁-C₆ alkyl)amino, C₁-C₆ alkylcarbonyl, C₁-C₆ alkoxycarbonyl, tri(C₁-C₆ alkyl)silyl, - C(N=OR^{b})R^{c} or Q-1,
R^{D} represents a halogen atom or nitro,
R^{e} represents C₁-C₆ alkoxy,
R^{g} represents Q-1, Q-2, Q-3, Q-4 or Q-11,
R^{h} represents C₁-C₆ alkoxy,
Rⁱ represents cyano,
Z represents Z-1, Z-4, Z-8, Z-9, Z-17, Z-19 or Z-27,
Z¹ represents hydroxy, carboxy, nitro, cyano, a halogen atom, C₁-C₆ alkyl, C₃-C₁₀ cycloalkyl, C₁-C₆ haloalkyl, C₁-C₆ alkoxy, C₁-C₆ haloalkoxy, C₁-C₆ alkylthio, C₁-C₆ haloalkylthio, C₁-C₆ alkoxycarbonyl, -OR^{j} or E-1,
Z² represents C₁-C₆ alkyl,
R^{j} represents C₁-C₆ alkyl substituted by R⁴ or C₁-C₆ alkoxycarbonyl,
R⁴ represents E-2,
Z^{a} represents C₁-C₆ alkyl or C₁-C₆ haloalkyl,
m5 represents an integer of 0, 1 or 2,
n2 represents an integer of 0 or 2,
n3 represents an integer of 1,
n4 represents an integer of 1,
n5 represents an integer of 1, 2 or 3,
u4 represents an integer of 0,
u5 represents an integer of 0,
t5 represents an integer of 1,
v4 represents an integer of 1,
v5 represents an integer of 0,
w4 represents an integer of 0, and
w5 represents an integer of 0.

9. The pyrazole compound as set forth in claim 8 or a salt thereof, wherein:
R¹ represents a hydrogen atom,
R^{W} represents C₁-C₆ alkyl, C₁-C₆ haloalkyl, C₁-C₆ alkyl substituted by R^{a}, C₂-C₆ alkenyl, C₁-C₆ alkylcarbonyl, C₁-C₆ haloalkylcarbonyl, C₁-C₆ alkylcarbonyl substituted by R^{a}, C₂-C₆ alkenylcarbonyl, C₂-C₆ alkenylcarbonyl substituted by R^{a}, C₃-C₁₀ cycloalkylcarbonyl, C₁-C₆ alkoxycarbonyl, C₁-C₆ haloalkoxycarbonyl, C₁-C₆ alkoxycarbonyl substituted by R^{a}, C₂-C₆ alkenyloxycarbonyl, C₁-C₆ alkylaminocarbonyl, di(C₁-C₆ alkyl)aminocarbonyl or C₁-C₆ alkylaminothiocarbonyl, and
p represents an integer of 1.

10. A pesticide which contains, as an active ingredient, at least one selected from a pyrazole compound recited in any one of claims 1 through 9 and a salt thereof.

11. A fungicide which contains, as an active ingredient, at least one selected from a pyrazole compound recited in any one of claims 1 through 9 and a salt thereof.

12. An agricultural-horticultural fungicide which contains, as an active ingredient, at least one selected from a pyrazole compound recited in any one of claims 1 through 9 and a salt thereof.

13. An antimycotic agent which contains, as an active ingredient, at least one selected from a pyrazole compound recited in any one of claims 1 through 9 and a salt thereof.

14. An endoparasite control agent which contains, as an active ingredient, at least one selected from a pyrazole compound recited in any one of claims 1 through 9 and a salt thereof.
